# EUROPEAN PATENT APPLICATION

(11) **EP 1 452 590 A1**
(43) Date of publication of application: **01.09.2004**
(21) Application number: 02802719.1
(22) Date of filing: 06.11.2002
(51) Int. Cl.: C12N 15/00, C12Q 1/25, G01N 33/15, G01N 33/50, G06F 17/50

(54) **STEREOSTRUCTURE OF DNA RECOMBINATION REPAIR ENZYME AND UTILIZATION THEREOF**

(30) Priority: 06.11.2001 JP 2001341172
(71) Applicant: Riken, Wako-shi, Saitama 351-0198 (JP)
(72) Inventor: KURUMIZAKA, Hitoshi, c/o RIKEN Yokohama Institute, Yokohama-shi, Kanagawa 230-0045 (JP); KAGAWA, Wataru, c/o RIKEN Yokohama Institute, Yokohama-shi, Kanagawa 230-0045 (JP); YOKOYAMA, Shigeyuki, c/o RIKEN Yokohama Institute, Yokohama-shi, Kanagawa 230-0045 (JP)
(74) Representative: Arends, William Gerrit
(86) International application number: PCT/JP2002/011548
(87) International publication number: WO 2003/040361

(57) **Abstract**

A crystal of DNA recombination/repair protein, Rad52 protein is prepared and the three-dimensional structure thereof is determined by X-ray crystallography with the use of the same. The obtained three-dimensional structure of Rad52 protein can be used for screening a variety of mutant enzymes or compounds which binds to the enzyme complex. The mutant Rad52 protein complex, or the compound that binds to the complex so as to modulate the DNA recombinational repairing activity, which are screened by the method of the present invention, can be used for diagnosis and treatment of diseases induced by DNA damages, and detection of the DNA recombinational repairing activity *in vivo.* Moreover, these substances are usable in drug discovery targeting to Rad52, and gene transfer to a specific site by homologous recombination, etc.

SEQUENCE LISTING

<110>  RIKEN

<120>  STEREOSTRUCTURE OF DNA RECOMBINATION REPAIR ENZYME AND UTILIZATION
THEREOF

<130>  SJW/WA/47165

<140>  EP 02802719.1
<141>  2002-11-06

<150>  JP P2001-341172
<151>  2001-11-06

<160>  2

<170>  PatentIn version 3.1

<210>  1
<211>  418
<212>  PRT
<213>  Homo sapiens

<400>  1
   Met Ser Gly Thr Glu Glu Ala Ile Leu Gly Gly Arg Asp Ser His Pro
   1               5                   10                  15
   Ala Ala Gly Gly Gly Ser Val Leu Cys Phe Gly Gln Cys Gln Tyr Thr
               20                  25                  30
   Ala Glu Glu Tyr Gln Ala Ile Gln Lys Ala Leu Arg Gln Arg Leu Gly
           35                  40                  45
   Pro Glu Tyr Ile Ser Ser Arg Met Ala Gly Gly Gly Gln Lys Val Cys
       50                  55                  60
   Tyr Ile Glu Gly His Arg Val Ile Asn Leu Ala Asn Glu Met Phe Gly
   65                  70                  75                  80
   Tyr Asn Gly Trp Ala His Ser Ile Thr Gln Gln Asn Val Asp Phe Val
                   85                  90                  95
   Asp Leu Asn Asn Gly Lys Phe Tyr Val Gly Val Cys Ala Phe Val Arg
               100                 105                 110
   Val Gln Leu Lys Asp Gly Ser Tyr His Glu Asp Val Gly Tyr Gly Val
           115                 120                 125
   Ser Glu Gly Leu Lys Ser Lys Ala Leu Ser Leu Glu Lys Ala Arg Lys
       130                 135                 140
   Glu Ala Val Thr Asp Gly Leu Lys Arg Ala Leu Arg Ser Phe Gly Asn
   145                 150                 155                 160
   Ala Leu Gly Asn Cys Ile Leu Asp Lys Asp Tyr Leu Arg Ser Leu Asn
                   165                 170                 175
   Lys Leu Pro Arg Gln Leu Pro Leu Glu Val Asp Leu Thr Lys Ala Lys
               180                 185                 190
   Arg Gln Asp Leu Glu Pro Ser Val Glu Glu Ala Arg Tyr Asn Ser Cys
           195                 200                 205
   Arg Pro Asn Met Ala Leu Gly His Pro Gln Leu Gln Gln Val Thr Ser
       210                 215                 220
   Pro Ser Arg Pro Ser His Ala Val Ile Pro Ala Asp Gln Asp Cys Ser
   225                 230                 235                 240
   Ser Arg Ser Leu Ser Ser Ser Ala Val Glu Ser Glu Ala Thr His Gln
                   245                 250                 255
   Arg Lys Leu Arg Gln Lys Gln Leu Gln Gln Gln Phe Arg Glu Arg Met
               260                 265                 270
   Glu Lys Gln Gln Val Arg Val Ser Thr Pro Ser Ala Glu Lys Ser Glu
           275                 280                 285
   Ala Ala Pro Pro Ala Pro Pro Val Thr His Ser Thr Pro Val Thr Val
       290                 295                 300
   Ser Glu Pro Leu Leu Glu Lys Asp Phe Leu Ala Gly Val Thr Gln Glu
   305                 310                 315                 320
   Leu Ile Lys Thr Leu Glu Asp Asn Ser Glu Lys Trp Ala Val Thr Pro
                   325                 330                 335
   Asp Ala Gly Asp Gly Val Val Lys Pro Ser Ser Arg Ala Asp Pro Ala
               340                 345                 350
   Gln Thr Ser Asp Thr Leu Ala Leu Asn Asn Gln Met Val Thr Gln Asn
           355                 360                 365
   Arg Thr Pro His Ser Val Cys His Gln Lys Pro Gln Ala Lys Ser Gly
       370                 375                 380
   Ser Trp Asp Leu Gln Thr Tyr Ser Ala Asp Gln Arg Thr Thr Gly Asn
   385                 390                 395                 400
   Trp Glu Ser His Arg Lys Ser Gln Asp Met Lys Lys Arg Lys Tyr Asp
                   405                 410                 415
   Pro Ser

<210>  2
<211>  50
<212>  DNA
<213>  Artificial Sequence

<220>
<223>  HPLC-purified oligonucleotide SAT-1

<400>  2
atttcatgct agacagaaga attctcagta acttctttgt gctgtgtgta                50

## Description

### BACKGROUND OF THE INVENTION

### Field of the Invention

The present invention relates to the crystallization of DNA recombination/repair protein, the determination of the three-dimensional structure of DNA recombination/repair protein by X-ray crystallography using the crystal, and the use thereof. More specifically, the invention relates to the designing and identification of a mutant enzyme based on the three-dimensional structure, and a rational method for screening a compound binding to the enzyme.

### Description of the Related Art

The ability to repair double-stranded DNA breaks that occur on damaged chromosomal DNA is necessary to maintain the integrity of the genomic DNA. It has been known that the double-stranded DNA breaks can result from ionizing radiation, the administration of DNA-damaging agent, replication errors, and also from specific enzymes that act during meiosis, mating-type switching of yeast, and V(D)J recombination of antibody gene. To repair such chromosomal DNA damage, cells have acquired homologous recombination and non-homologous DNA end joining activities. Homologous recombination plays an important role in the preservation of DNA sequence in chromosomal DNA. The homologous pairing step of the homologous recombination, which is the process of searching for sequence homology in either homologous chromosome or sister chromatids, is an essential step. Thus, the identification of an enzyme promoting homologous pairing is one of the most important steps for understanding homologous recombination.

Two homologous pairing enzymes of RecA and RecT are reported in the case of *Escherichia coli.* RecA and RecT, which have no amino acid sequence homology, catalyze homologous pairing in separate recombinational repair pathways, the RecBCD and RecF pathways for RecA, and the RecE pathway for RecT. In eukaryotes, two RecA homologues, Rad51 and Dmc1, have been identified, and both proteins promote homologous pairing. Although the Rad51-independent recombination pathway has been identified, no proteins with significant sequence homology to RecT have been found in eukaryotes. An electron microscopic visualization showed that RecT forms a heptameric or octameric ring structure. Interestingly, the yeast Rad52 protein (referred to as "SCRad52" hereinafter) and the human Rad52 protein (referred to as "HsRad52" hereinafter) also form ring structures similar to that of RecT. Stasiak *et al.* reported based on their electron microscopicanalysis that HsRad52 shows a heptameric ring structure (Current Biology, 10, (2000), p. 337-340).

*RAD52* genes have been identified in eukaryotes including yeast, chicken, mouse and human. The role of Rad52 in the Rad51-dependent recombination pathway has been extensively studied both *in vivo* and *in vitro.* The ScRad52 and HsRad52 both bind to single-stranded DNA and promote annealing of complementary single-stranded DNAs to each other. Inaddition, it is reported that ScRad52 and HsRad52 together with yeast and human Rad51 proteins, enhance the joint molecule formation between circular single-stranded DNA and linear double-stranded DNA. Therefore, Rad52 protein has been proposed to be a mediator protein that binds single-stranded DNA and facilitates the loading of Rad51 onto the recombination site. In addition to such Rad51-dependent recombination pathway, the role of Rad52 in Rad51-independent recombination pathway has also been studied. However its precise molecular mechanism, particularly the catalytic mechanism of the enzyme including the DNA-binding mode during the homologous pairing step has not yet sufficiently been understood.

According to JP-T-2001-502545, a complex of eukaryotic Rad52 protein with nucleic acid, and a method for screening a biologically active substance involved in the homologous recombination using the complex are disclosed. According to themethod disclosed in the publication, however, the eukaryotic Rad52 protein should be purified and isolated after its expression by recombinant DNA technology. Thus, itisdifficult to directly assay the homologous recombination activity of the Rad52 in eukaryotic cells. Because the assay procedure for the homologous recombination activity is complicated, screening of numerous compounds using the complex is extremely inefficient.

Meanwhile, currently, it is known that certain types of cancer and other genetic diseases are caused by abnormalities in chromosomal DNA. For example, many oncogenes have been identified in humans. Most of oncogenes identified so far have been known to cause amino acid substitution in the resulting expressed protein due to a mutation in the gene in normal cell (proto-oncogene), frequently due to point mutation. The mutated expressed protein exhibits abnormal biological functions to cause carcinogenesis of cells.

For example, Ras is one of the most extensively studied oncogenes. Through the substitution of specific amino acid residues in the Ras protein, the Ras protein is activated to stimulate abnormal proliferation of cells and thereby induce carcinogenesis in cells.

Alternatively, another important oncogene encodes an intracellular protein p53. The protein p53 is believed to be a carcinogenesis-suppressor gene. It is revealed that p53 inactivation via mutagenesis induces carcinogenesis of cells. Thus, attempts have been made for cancer therapy to introduce a gene expressing the normal p53 protein in cancer cells (US Patent No. 6017524).

Because chromosomal DNA damage and the introduction of mutation into DNA due to the damage are closely associated with cellular carcinogenesis through oncogene activation and the inactivation of carcinogenesis-suppressor gene, as described above, the preservation of genomic integrity with DNA recombinational repair, and stable transmission of genomic information may possibly be useful for the prophylaxis and therapeutic treatment of cancer. That is, in cells with significantly reduced DNA recombination/repair protein activity, it is considered that the enhancement of the activity will possibly suppress carcinogenesis by repairing chromosomal DNA damage.

On the other hand, the suppression of DNA recombination/repair protein activity will possibly enhance anticancer therapies, particularly radiotherapy or chemotherapy. Because ionizing radiation and radioactive drugs are generally used to damage DNA to treat cancer cells, it is suggested that the administration of compounds inhibiting DNA recombination/repair protein activity enhance the sensitivity of cancer cells to the radiotherapy and chemotherapeutic agents (see JP-T- 2001-508868) . In addition, because homologous recombination of chromosomal DNA is involved in the integration of virus DNA and the like in gene therapy, such homologous recombination can be used in a manner to modulate DNA recombinational repairing activity to enhance the efficiency of the introduction of foreign gene into a specific site of chromosomal DNA, and to increase the ultimate use of gene therapy.

Although the role of the DNA recombinational repair mechanism and its applications are so significant as described above, it is very difficult to assay the Rad52-specific DNA recombination activity or to elucidate the physiological role thereof, because various DNA recombination/repair proteins exist in cells as described above. Additionally, the relationship between the structure and functions of Rad52 in homologous DNA recombinational repair has not yet been elucidated.

To solve the problems described above, it is an object of the present invention to obtain a single crystal of the Rad52 protein as one of DNA recombination/repair proteins to demonstrate the three-dimensional structure by X-ray crystallography and elucidate the relationship between the structure and the expression mechanism of the DNA recombinational repairing activity.

It is an another objective of the present invention to identify structural characteristics influencing the DNA recombinational repairing activity based on the three-dimensional structure of Rad 52 and to design a mutant enzyme with various modulations for the enhancement, suppression or stabilization of the enzyme activity.

Further, it is a still additional objective of the present invention to provide a method for screening a compound capable of modulating the DNA recombinational repairing activity via binding to Rad52 and also to provide an inhibitor or an activator obtained by using the method.

### SUMMARY OF THE INVENTION

With attention focused on the fact that the N-terminal region of the Rad52 protein is essential for DNA binding and the appearance of homologous pairing activity, the inventors screened the N-terminal region necessary and sufficient for the enzyme to express the function thereof by forming a mul timer complex. Further, the inventors crystallized the protein and carried out the X-ray crystallographic analysis using the crystal. Consequently, the inventors first revealed that the N-terminal region of the Rad52 protein formed an undecameric ring structure and could bind in the periphery of the ring structure to DNAs, and have finally led to the completion of the present invention.

That is, the present invention relates to the crystal of the Rad52 protein as a DNA recombination/repair protein, a method for producing the crystal, the analysis of the three-dimensional structure using the crystal, and a method for screening a compound binding to various mutant enzymes and the enzyme complex using the three-dimensional structure thus obtained. More specifically, the invention is as follows.
(1) A polypeptide fragment comprising the amino acid sequence from position 1 to position 192 in the amino acid sequence defined in SEQ ID NO: 1, wherein the amino acid residues at positions 55, 65, 69, 70, 153 and 169 form a DNA binding site.
(2) Apolypeptide fragment derived from the polypeptide fragmentof (1) aspreparedbydeletion, substitutionoraddition of one or several amino acids, the polypeptide fragment having a DNA binding activity.
(3) A DNA recombination/repair protein complex comprising an assembly of the following protein (a) or (b), wherein said complex forms an undecameric ring structure:
   (a) a protein consisting of the amino acid sequence of SEQ ID NO:1:
   (b) a protein derived from the amino acid sequence of SEQ ID NO:1 as prepared by deletion, addition, insertion or substitution of one or several amino acids, the protein having a DNA recombinational repairing activity.
(4) A mutant DNA recombination/repair protein complex comprising an assembly of theprotein fromposition 1 to position 212 in the amino acid sequence of SEQ ID NO:1, wherein the complex forms an undecameric ring structure.
(5) The mutant DNA recombination/repair protein complex of (4), wherein the complex has a three-dimensional structure substantially defined by the atomic coordinates shown in Table 1.
(6) A crystal of an undecameric mutant DNA recombination/repair protein complex comprising an assembly of the protein comprising the amino acid sequence from position 1 to position 212 in the amino acid sequence of SEQ ID NO:1, wherein the crystal has a space group of P4₃2₁2.
(7) A crystal of an undecameric mutant DNA recombination/repair protein complex comprising an assembly of the protein comprising the amino acid sequence from position 1 to position 212 in the amino acid sequence of SEQ ID NO:1, wherein the crystal has unit cell dimensions of a=b=145.6 Å, and c=247.5 Å.
(8) The crystal of a mutant DNA recombination/repair protein complex of (6) or (7), wherein methionine at position 78 in the amino acid sequence of SEQ ID NO:1 is substituted with selenomethionine.
(9) A method for preparing a crystal of a DNA recombination/repair protein complex or a mutant thereof, comprising the steps of mixing a DNA recombination/repair protein or a mutant thereof at a concentration of 3 to 4 mg/ml and single-stranded DNA at a molar ratio of 14:1 to 11:1, and crystallizing the resulting mixture by vapor diffusion method using a buffer solution, pH 6.0 to 8.0 containing 10 to 20% polyethylene glycol (PEG) or polyethylene glycol monomethyl ester (PEGMME) and 0.05 to 0.2 M ammonium sulfate.
(10) A method for determining the three-dimensional structure of a DNA recombination/repair protein complex or a mutant thereof, comprising the steps of:
   (a) generating a crystal of a protein with DNA recombinational repairing activity,
   (b) obtaining the X-ray diffraction data using said crystal by heavy atom isomorphous replacement or multiwavelength anomalous diffraction, and
   (c) determining a three-dimensional structural coordinate based on said X-ray diffraction data.
(11) A computer system comprising a memory with atomic coordinates defining a DNA recombination/repair protein complex being arranged in the inside thereof and a processor in electric communication with the memory, the processor comprising a process of generating a model with a three-dimensional structure representing at least a part of the DNA recombination/repair protein complex or calculating the conformational energy of the complex.
(12) A method for identifying a mutant DNA recombination/repair protein with modulated DNA recombinational repairing activity, comprising the steps of:
   (a) constructing the three-dimensional structure representing a DNA recombination/repair protein complex, using a computer model,
   (b) identifying a site influencing the DNA recombinational repairing activity on the basis of the three-dimensional structure model,
   (c) producing a mutant enzyme by introducing a mutation in said identified site, and
   (d) measuring the activity of said mutant enzyme.
(13) The method of (12), wherein said three-dimensional structure model is substantially defined by the atomic coordinates shown in Table 1.
(14) The method of (12) or (13), wherein said DNA recombinational repairing activity is DNA binding activity, homologous-pairing activity or multimer-forming activity.
(15) A method for identifying a mutant DNA recombination/repair protein with modulated DNA recombinational repairing activity, comprising the steps of:
   (a) identifying a DNA binding region by calculating the surface charge of all or a part of the DNA recombination/repair protein complex substantially defined by the atomic coordinates shown in Table 1,
   (b) selecting basic and hydrophobic amino acid residues existing in said identified DNA binding region,
   (c) preparing a mutant enzyme by carrying out the substitution, deletion, addition or chemical modification of said selected amino acid residues, and
   (d) assaying the DNA binding activity and/or homologous pairing activity of the mutant enzyme.
(16) The method for identifying a mutant DNA recombination/repair protein of (15), wherein the amino acid residues selected at the step (b) are one or two or more amino acid residues selected from arginine at position 55, tyrosine at position 65, histidine at position 69, arginine at position 70, arginine at position 153 and lysine at position 169 in the amino acid sequence of SEQ ID NO:1.
(17) A method for producing a mutant DNA recombination/repair protein, comprising the steps of identifying a mutant DNA recombination/repair protein by a method of any one of (12) to (16), and expressing said mutant DNA recombination/repair protein using the recombinant DNA encoding the mutant DNA recombination/repair protein.
(18) A mutant DNA recombination/repair protein with modulated DNA recombinational repair activity, as obtained by a method of any one of (12) to (16).
(19) A method for screening a compound capable of modulating the repairing activity of a DNA recombination/repair protein, using a polypeptide fragment or a complex of any one of (1) to (5) to design or select a compound binding thereto.
(20) A method for screening a compound capable of modulating the repairing activity of a DNA recombination/repair protein, using a computer with the memory of the coordinate data of the three-dimensional structure of a polypeptide fragment or a complex of any one of (1) through (5) to design or select a compound binding thereto.
(21) The method of (20) , further comprising contacting said designed or selected compound with a DNA recombination/repair protein, and determining whether said compound binds to the DNA recombination/repair protein.
(22) A compound modulating the activity of a DNA recombination/repair protein as obtained by a method of any one of (19) through (21).
(23) The compound according to (22), wherein said compound modulating the DNA recombinational repairing activity is a natural compound or a synthetic compound.
(24) A method for preparing a pharmaceutical composi tion containing a compound modulating DNA recombinational repairing activity, comprising a step of mixing a compound obtained by a method of any one of (19) through (21) with a pharmaceutically acceptable carrier.

### BRIEF DESCRIPTION OF THE DRAWINGS

Fig. 1 shows the three-dimensional structure of the HsRad52₁₋₂₁₂ complex as a human-derived mutant DNA recombination/repair protein in a ribbon model. Figs. 1A and 1B show the structure of the undecamer of the HsRad52₁₋₂₁₂ as depicted in a different direction.
Fig. 2 shows the three-dimensional structure of the HsRad52₁₋₂₁₂ monomer as a human-derived mutant DNA recombination/repair protein in a ribbon model. Figs. 2A and 2B show the structure of the HsRad52-₁₋₂₁₂ monomer as depicted in a different direction.
Fig. 3 shows the DNA binding site and DNA binding mode of the HsRad52₁₋₂₁₂ monomer as a human-derived mutant DNA recombination/repair protein. Fig. 3A is a stereo representation of the DNA bind ing sites of the HsRad52₁₋₂₁₂ , while Fig. 3B is a steric figure depicting the DNAbinding site between adjacent two HsRad52₁₋₂₁₂ molecules forming the complex and Fig. 3C is a model figure depicting the DNA binding mode of the undecamer of the HsRad52₁₋₂₁₂ complex.
Fig. 4 shows the assay results of the DNAbinding activities of the wild type HsRad52-₁₋₂₁₂ and various mutants thereof by gel shift method. Fig. 4A shows the results of detecting the binding activity to single-stranded DNA by autoradiography. Fig. 4B shows the results of detecting the binding activity to double-stranded DNA by ethidium bromidestaining. Fivelanes for each one protein show the concentration dependency in case of sequentially increasing the protein concentration.
Fig. 5 shows the proton NMR spectrum of a ligand candidate compound AK-968-41170131 (Compound A).
Fig. 6 shows the proton NMR spectrum of a mixture (1:1) of the ligand candidate compound AK-968-41170131 (Compound A) and HsRad52₁₋₂₁₂.
Fig. 7 shows the comparison of the NMR spectra of the ligand candidate compound AK-968-41170131 (Compound A) and a mixture (1:1) of the ligand candidate compound AK-968-41170131 (Compound A) and HsRad52₁₋₂₁₂ around 7.5 ppm.
Fig. 8 shows the proton NMR spectrum of a ligand candidate compound AK-968-41025315 (Compound a).
Fig. 9 shows the proton NMR spectrum of a mixture (1:1) of the ligand candidate compound AK-968-41025315 (Compound a) and HsRad52₁₋₂₁₂.
Fig. 10 shows the comparison of the proton NMR spectra of the ligand candidate compound AK-968-41025315 (Compound a) and a mixture (1:1) of the ligand candidate compound AK-968-41025315 (Compound a) and HsRad52₁₋₂₁₂ around 7.5 ppm.
Fig. 11 shows the gel shift assay results showing that the ligand candidate compound AK-968-41170131 (Compound A) inhibits the binding of HsRad52 to single-stranded DNA (ssDNA) .

### DETAILED DESCRIPTION OF THE INVENTION

In accordance with the invention, "DNA recombinational repairing activity" means an activity for recognizing a site with chromosomal DNA damage and the like, and recognizing, breaking and rejoining homologous nucleotide sequences between DNA molecules via homologous recombination. Because homologous recombination is very complex, the details of the reaction mechanism are not yet elucidated. As described above, it is considered that two pathways, namely Rad51-dependent pathway and Rad51-independent pathway exist in eukaryotes. Therefore, DNA repairing activity through these two homologous recombination reactions is referred to as "DNA recombinational repairing activity".

"DNA recombination/repair protein" means an enzyme having the DNA recombinational repairing activity and examples thereof include HsRad52 with the amino acid sequence of SEQ ID NO:1. HsRad52 is a protein comprising 418 amino acid residues from humans. The cDNA nucleotide sequence has been revealed (for example, GenBank Accession No. XM_052347). The *RAD52* genes in eukaryotes are found in chicken, mouse and yeast (*S. pombe* and *S. cerevisiae).* The primary structures of the Rad52 proteins obtained from these cDNA nucleotide sequences are compared to each other. About 70% homology is known in their N-terminal regions, particularly the regions up to the position 177 from the N terminus in HsRad52 (Muris, DF, *et al.,* 1994, Mutat. Res. 315, 295-305).

Therefore, the "DNA recombination/repair protein" in accordance with the invention means HsRad52 comprising the amino acid sequence of SEQ ID NO:1 or a homolog thereof in eukaryotes.

In accordance with the invention, the "mutant" of the DNA recombination/repair protein means a modified enzyme comprising an amino acid sequence derived from the amino acid sequence of SEQ ID NO:1 as prepared by deletion, addition, insertion or substitution or modification of one or several amino acids and still having at least a part of the activity of the intact enzyme. Herein, the term "at least a part of the activity" typically means a specific activity of at least 10% of the activity of the intact enzyme, preferably at least 50% thereof. If desired, the term sometimes means a specific activity at less than 10% thereof.

According to research works so far made by the present inventors, the limited digestion of HsRad52 protein and the analysis of a mutant enzyme deficient in a C-terminal region show that a core domain responsible for the homologous pairing activity of HsRad52 exists in the N-terminal region of HsRad52. A polypeptide HsRad52₁₋₂₃₇ comprising the amino acids at position 237 from the N terminus is resistant to the limited digestion withproteinase K. Because the analysis of the D-loop formation reaction shows that the mutant enzyme HsRad52₁₋₂₃₇ has a homologous pairing activity at a level almost equal to that of the wild type enzyme, it is considered that the N-terminal region from the N terminus of HsRad52 to position 237 thereof is responsible for the homologous pairing activity. Further, the N-terminal region forms a mul timeric higher-order structure like that of the wild type HsRad52 and has also the DNA binding activity. These indicate that the HsRad52 protein even when the protein is deficient in a part of the C-terminal region forms a complex similar to that formed by the wild type HsRad52 protein and still keeps the DNA recombinational repairing activity and that such C-terminal region-deficient HsRad52 protein is sufficiently useful for the elucidation of the relationship between the structure and function of the HsRad52 protein for the homologous recombination mechanism. As such mutant HsRad52 protein deleted of a part of the C-terminal region, specifically, various mutants can be utilized. For example, plural deficient mutants with serial deletion by every five amino acids from the C terminus of HsRad52₁₋₂₃₇ are exemplified, such as HsRad52-₁₋₂₃₂, HsRad52₁₋₂₂₇, HsRad52₁₋₂₂₂, HsRad52₁₋₂₁₇, HsRad52₁₋₂₁₂, HsRad52₁₋₂₀₇, HsRad52₁₋₂₀₂, HsRad52₁₋₁₉₇, HsRad52₁₋₁₉₂ and HsRad52₁₋₁₈₇. Among them, mutant enzymes with amino acid sequences from the N terminus at least up to position 192 still retain an activity as DNA recombination/repair protein. Thus, selecting amutant enzyme most crystallizable, aprotein crystal for use in X-ray crystallography can be prepared.

### (Preparation of crystal of DNA recombination/repair protein complex)

The most general approach for identifying three-dimensional protein structure is an approach by X-ray crystallographic analysis. That is, the approach includes crystallizing protein, allowing monochromatic X-ray to irradiate the crystal to obtain an X-ray diffraction image, and analyzing the three-dimensional structure of the protein based on the resulting X-ray diffraction image (Blundell, T.L. and Johnson, L. N., PROTEIN CRYSTALLOGRAPHY, p.1-565, (1976) Academic Press, New York).

The crystallization utilizes a protein property of deposition in crystal under a specific condition, by a procedure of adding a precipitation agent to a solution of a target protein or by a procedure of reducing the amount of the solvent via evaporation or the like to turn the solution state of the protein to the non-solution state. For the crystallization, highly purified protein is required. Additionally, physical and chemical factors are involved in the crystallization conditions, such as protein concentration, salt concentration, hydrogen ion concentration (pH), the type of a precipitation agent to be added, and temperature. Further, methods for adding such precipitation agent and for adjusting the amount of the solvent, numerous crystallization methods exist such as batch method, dialysis method and vapor diffusion method (Blundell, T. L. and Johnson, L.N., PROTEIN CRYSTALLOGRAPHY, p. 59-82, (1976) Academic Press, New York) . For example, the vapor diffusion method functions tomobilize volatile solvents or precipitation agentsvia diffusionthrough gasphase. Through vapor diffusion from the protein solution and the solution of a precipitation agent, the protein concentration and the concentration of the precipitation agent both increase, leading to the occurrence of crystallization in a supersaturated zone. Depending on how liquid droplets of the protein solution are made, there are several methods such as hanging drop method, sitting dropmethod, and sandwich drop method. So as to prepare a protein crystal suitable for X-ray crystallography as described above, essentially, an individual protein should be obtained as a protein at high purity and various factors and crystallization methods should be examined for their optimization. The preparation of high-purity protein is an important factor for successful crystallization. Thus, a homogenous protein can be prepared rapidly by expressing a cloned gene in an efficient expression system.

The crystal of the DNA recombination/repair protein complex in accordance with the invention can be prepared as describedbelow. The cDNA sequence encoding the HsRad52 protein has already been known and can be readily expressed in various hosts such as bacteria and animal cells using DNA recombination technology.

In one embodiment of the present invention, the HsRad52 protein can be expressed at a large scale in a system using a recombinant *Escherichia (E.) coli.* various plasmid vectors are commercially available for large-scale expression of exogenous gene in recombinant *E*. *coli.* For example, pET system (Novagen) can be used, which works for introducing a gene to be expressed downstream strong T7 phage promoter for transcription with T7RNA polymerase supplied by the host *E. coli.* In the system, T7 RNA polymerase gene is integrated in the chromosome of the host *E. coli* and is downstream lacUV5 promoter, so that the expression can be controlled by the induction by IPTG addition. Additionally, introducing various tag sequences on the N-terminal side or C-terminal side of the HsRad52 protein enables ready purification us ing a metal chelate resin (histidine tag) and antibodies (T7 tag, CBD tag, and the like). From the fused protein purified, only the moiety of the HsRad52 protein can further be recovered using proteases such as enterokinase and thrombin.

By growing and culturing the recombinant *E. coli* expressing the HsRad52 protein in a culture medium containing selenomethionine or selenocysteine containing selenium as a heavy metal atom, additionally, a mutant enzyme where the methionine or cysteine residue in the HsRad52 protein is substituted with selenomethionine or selenocysteine can be recovered.

For further allowing the resulting enzyme more suitable for crystallization, such protein is purified to higher purity. As the purification method, general methods used by a person skilled in the art forproteinpurification including for example column chromatography (affinity, hydrophobic, ion exchange, gel filtration, etc.), salting out, centrifugation, and electrophoresis can be used singly or in combination.

Then, the crystal of the DNA recombination/repair protein complex is prepared. Essentially, crystallization should be carried out under conditions so that the HsRad52 protein can keep the state of the multimer complex formed intracellularly. Using vapor diffusin process, for example, the crystal of the DNA recombination/repair protein complex can be obtained under conditions of pH = 6 to 8, a protein concentration of 3 to 4 mg/ml, and a temperature of 20°C, in case that 10 to 20% polyethylene glycol (PEG) or polyethylene glycol monomethyl ester (PEGMME) and 0.05 to 0.2 M ammonium sulfate are used as precipitation agents. So as to promote the accumulation of the HsRad52 protein to form the multimer complex, preferably, the HsRad52 protein and single-stranded DNA are preliminarily mixed together at a molar ratio of 14:1 to 11:1.

In addition to the method for introducing the heavy metal atom into protein via culturing in the selenium-containing culture medium, the native type protein crystal is immersed in a solution of a metal salt or an organic metal compound containing gold, platinum, iridium, osmium, mercury, lead, uranium and samarium, to prepare crystals of heavy metal derivatives. The method is utilized for the application of heavy atom isomorphous replacement and multiwavelength anomalous diffraction.

The crystal of the HsRad52₁₋₂₁₂ complex containing the amino acid sequence from position 1 to position 212 of SEQ ID NO:1 belongs to a tetragonal space group P4₃2₁2 and is of unit cell dimensions of 145.6 angstroms in the directions of axes a and b and 247.6 angstroms in the direction of axis c.

### (X-ray crystallography of DNA recombination/repair protein complex)

The structure of the thus obtained crystal of the DNA recombination/repair protein complex is analyzed using X-ray crystallographic analytical technology known to a person skilled in the art. For the determination of three-dimensional structure as in the case of the invention, where the three-dimensional structure of an analogous protein is unknown and the structural analysis by molecular substitution method using the three-dimensional structure is impossible, the heavy atom isomorphous replacement and multiwavelength anomalous diffraction (MAD) are applicable. In one embodiment of the present invention, X-ray crystallographic analysis is MAD. X-ray used therefor is at a wavelength of 0.3 to 3.0 angstroms obtained from synchrotron radiation. The MAD is a process of obtaining X-ray diffraction data at plural wavelength from a heavy atom isomorphous replacement crystal with an absorption end close to the incident X-ray energy. Resonance between X-ray and the electron shell of the heavy atom causes a difference in X-ray diffraction, to thereby overcome the phase problem in the structural analysis of protein. The principle of the method has already been known classically. However, the appearance of synchrotron radiation with a variable wavelength first enabled the use for structural protein determination. Hendrickson, *et al.* first made a success in the structural analysis of protein by the method (see Hendrickson W.A. et al., Proteins 4, 77-88, 1988 and Hendrickson W.A. et al., EMBO J. 5, 1665-1672, 1990).

Synchrotron radiation is a radiation obtained by accelerating electron charged in accumulate ring with a magnetic field, and has an energy far higher than those from general X-ray generators. For example, the third generation Large Synchrotron Apparatus Spring- 8 (8GeV) at RIKEN HARIMA INSTITUTE can be used.

The data of the measured diffraction image are processed into reflection intensity data, using a program DENZO (Mac Science) or a similar image processing program. Based on the reflection intensity data of the native type crystal and the reflection intensity data of the heavy atom replacement crystal at plural wavelengths, the position of the heavy atom bound to the protein in the crystal is determined using the Differential Patterson chart. Using subsequently a program PHASE or CCP4 or a similar diffraction data analysis program, the heavy atom position parameters are refined to determine the initial phase. The determined initial phase is modified into a phase with higher reliability according to a solvent smoothing method using Program DM (CCP4 package) or a similar phase improvement program (electron density improvement program).

The three-dimensional structure model of the DNA recombination/repair protein complex can be constructed from the electron density map displayed on the three-dimensional graphics by the Program O by the following procedures. First, plural regions with characteristic amino acid sequences are screened on the electron density map. With reference to the amino acid sequences starting from the regions found, then, a partial structure of amino acid residues fitting to the electron density is constructed on three-dimensional graphics using the Program O.

By repeating the procedure sequentially, all the amino acid residues of the DNA recombination/repair protein complex are fitted to the corresponding electron densities, so that an initial three-dimensional structure model for the whole complex is constructed. The constructed three-dimensional structure model is used as a starting model structure, to refine the three-dimensional coordinates describing the three-dimensional structure according to the refined protocol of XPLOR as a structure refiningprogram. The status of refining progress and molecular model can be checked and confirmed by the following indicators. Reliability factor (R factor) is an indicator showing the agreement level between the absolute value of the crystal structure factor obtainedby the diffraction intensity measurement and the absolute value of the crystal structure factor as calculated from molecular model. The reliability factor takes a small value as the refining progresses normally. Additionally, a method for confirming that the dihedral angle values of the main chain of a protein are in an acceptable region by calculating the dihedral angles of the main chain of the protein and plotting the angles is also used (Ramachandran Plot).

In such manner, the three-dimensional atomic coordinates (values showing the spatial positional relation among individual atoms) of the DNA recombination/repair protein complex of the invention can first be obtained in such manner. Table 1 shows the resulting atomic coordinates according to the text format of Protein Data Bank as an expression method for general use for a person skilled in the art. Herein, the resulting atomic coordinates has been deposited with the accession code PDB ID: 1KNO at Protein Data Bank (PDB) , Research Collaboratory for Structural Bioinformatics (operated by RCSB) . Table 1 can be referred to within the scope of the claims of the present specification.

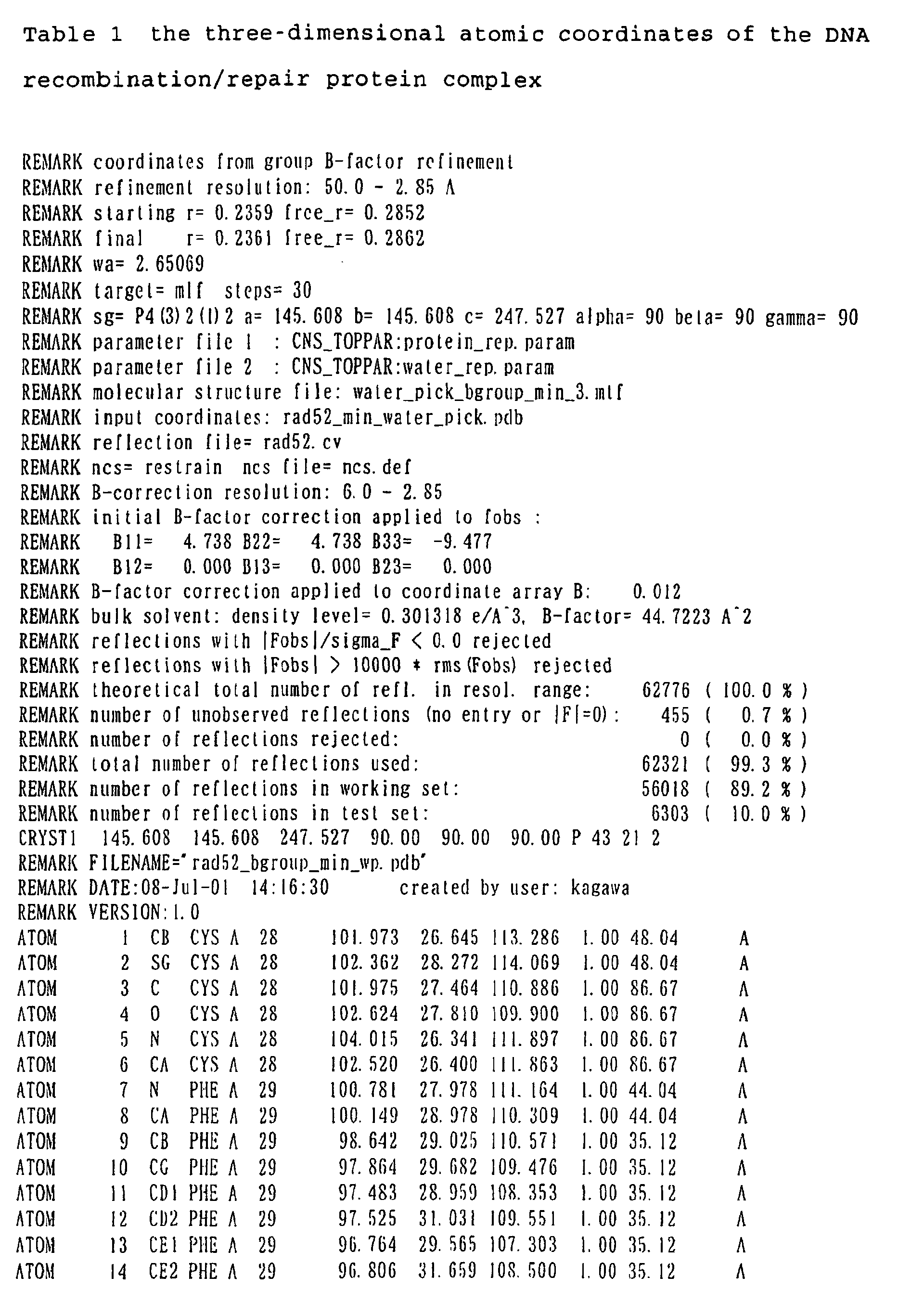

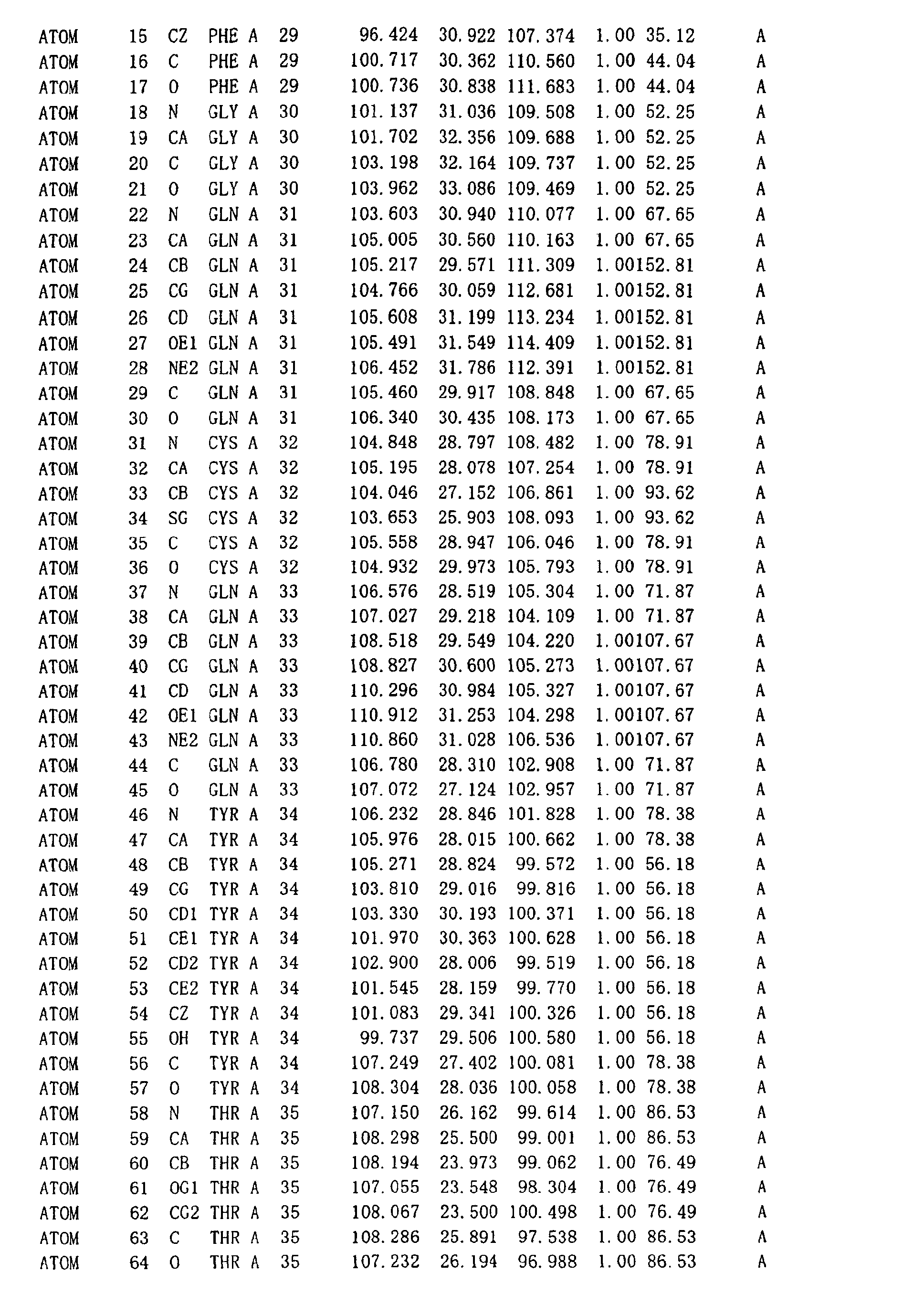

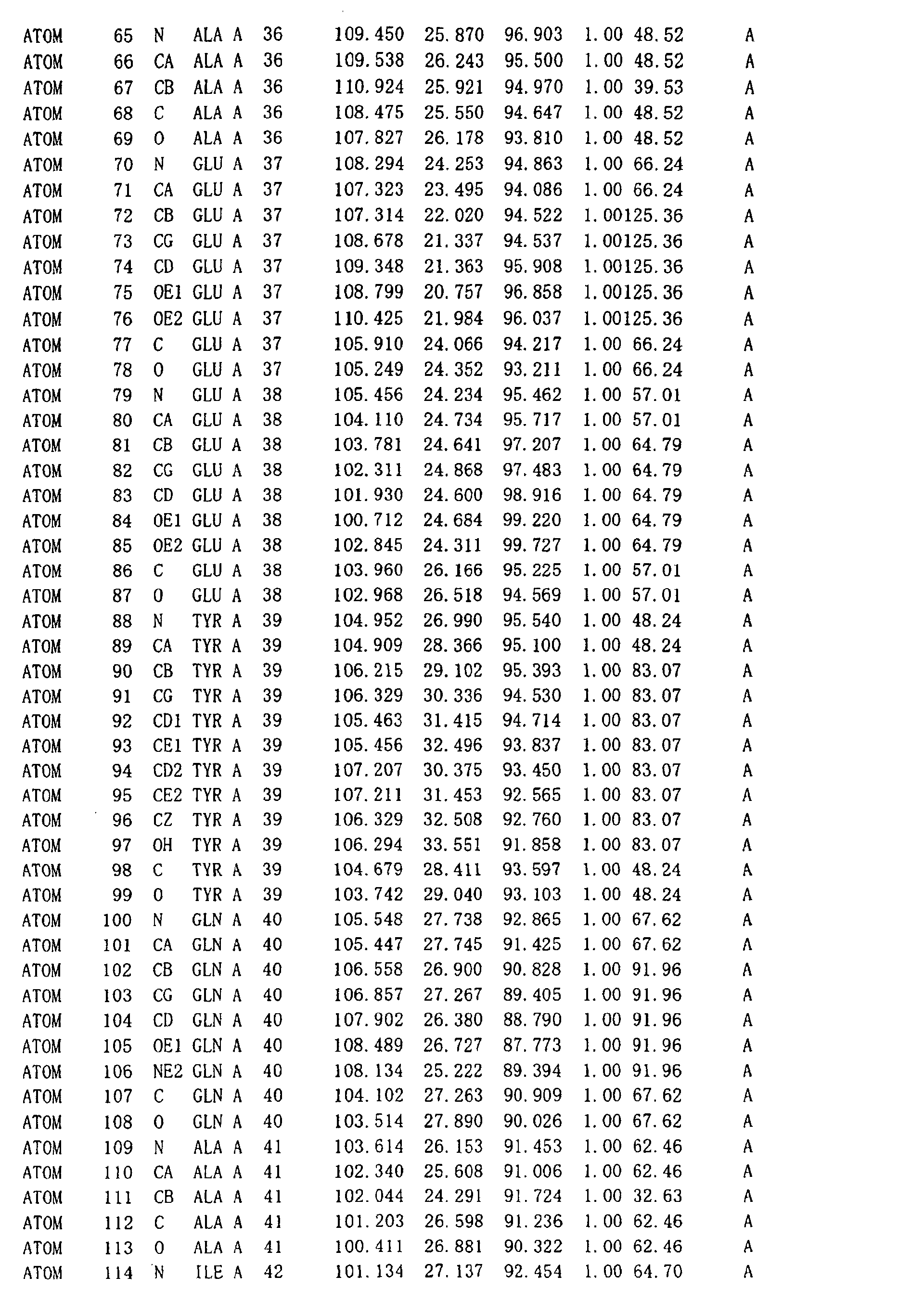

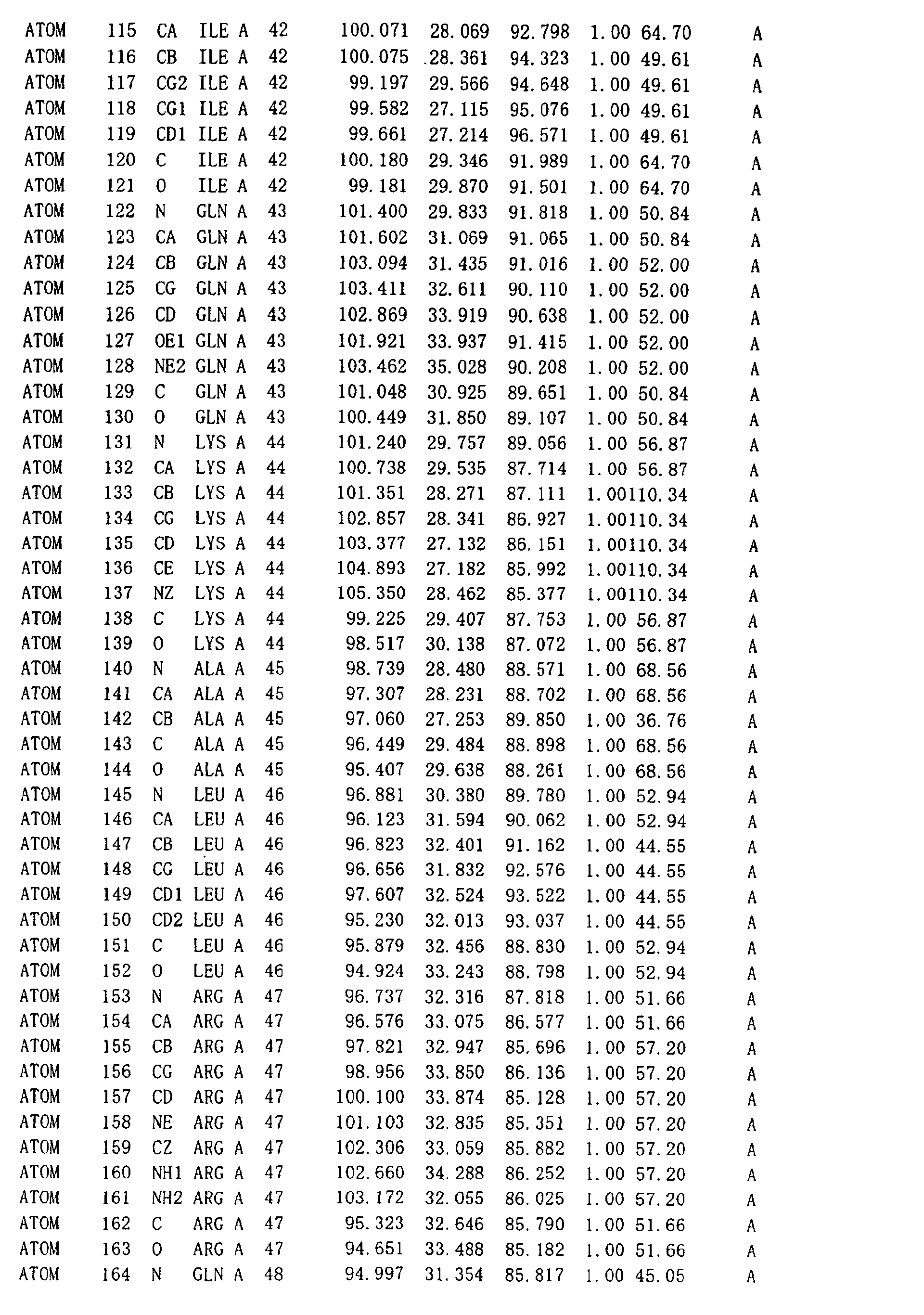

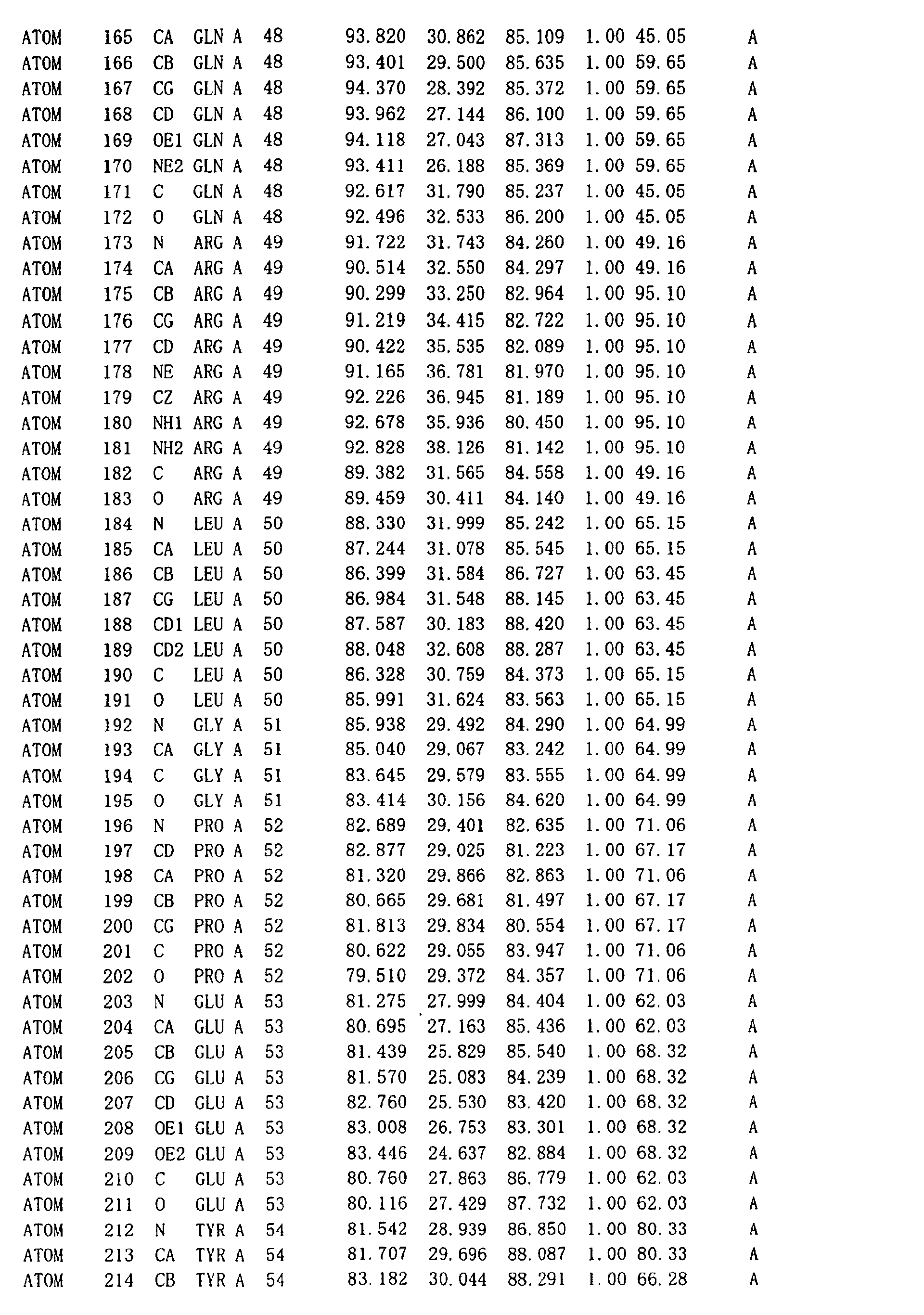

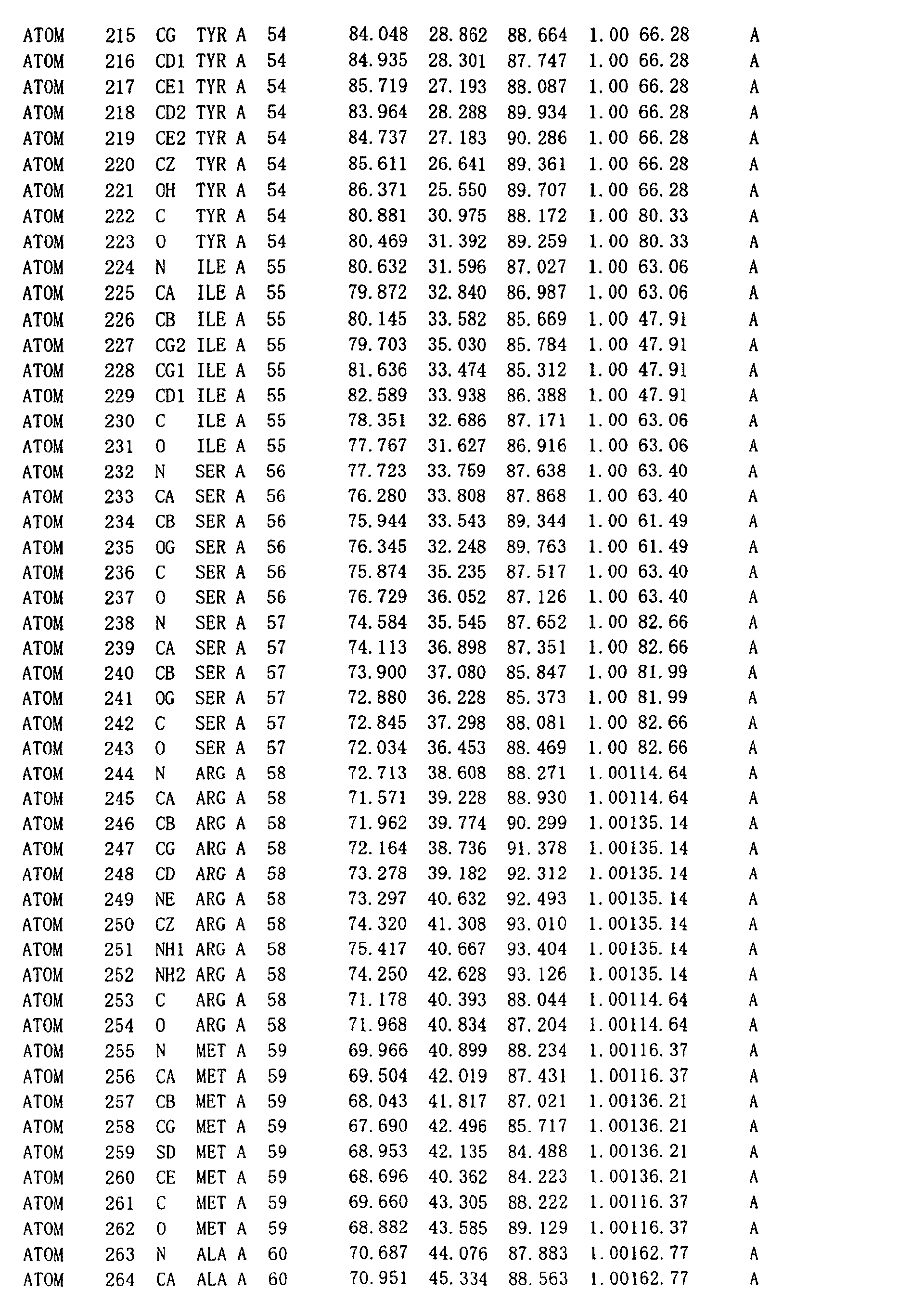

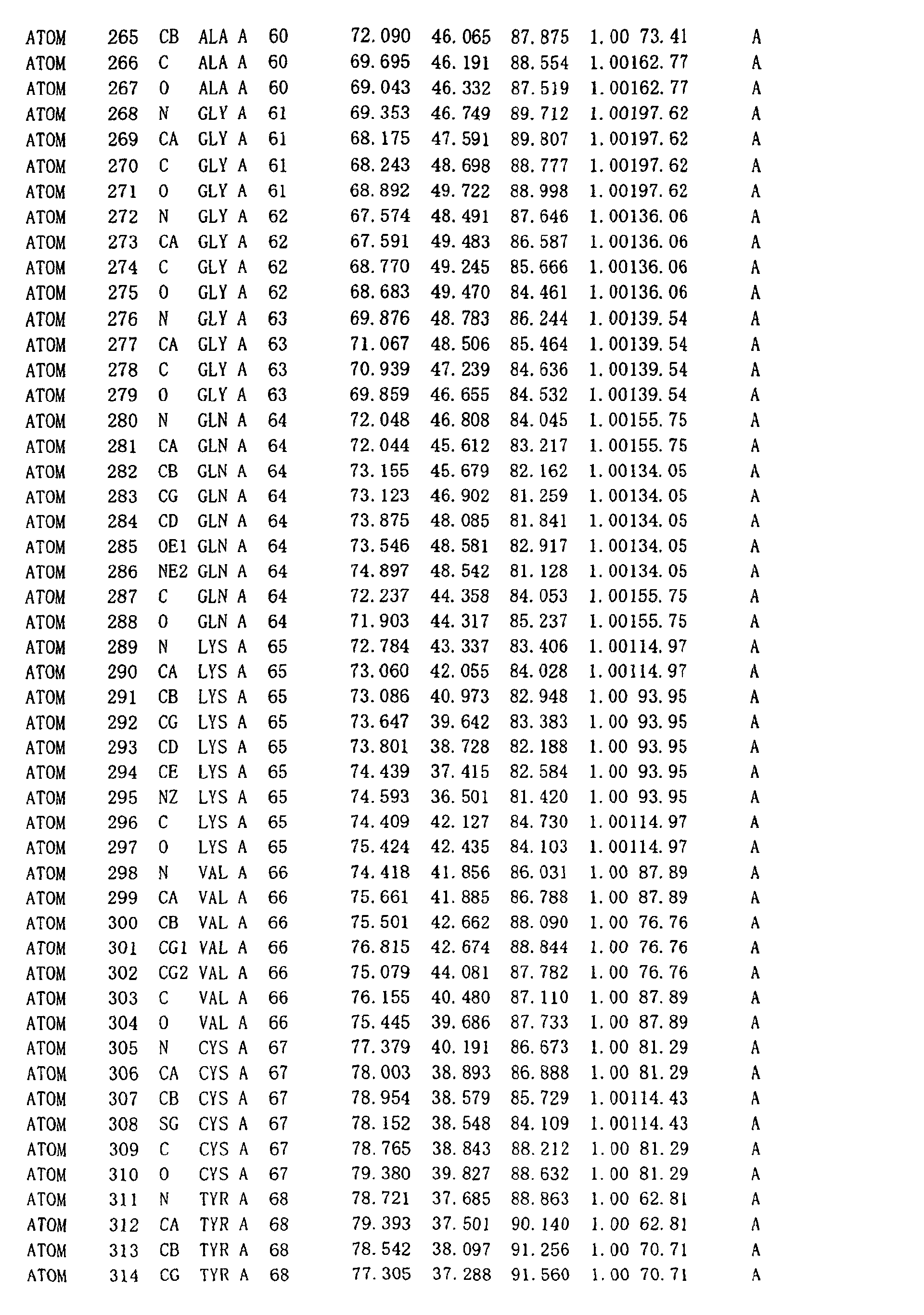

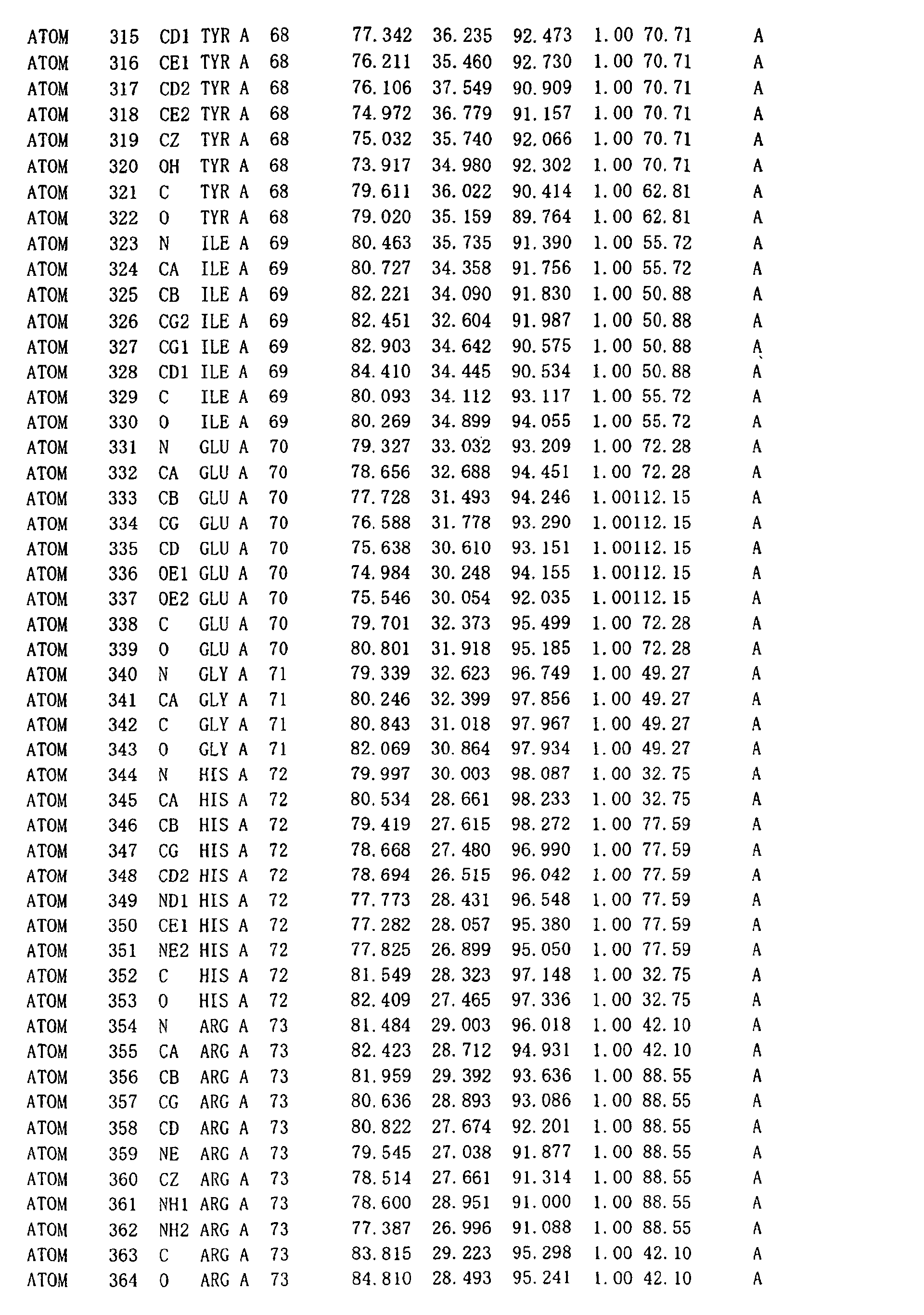

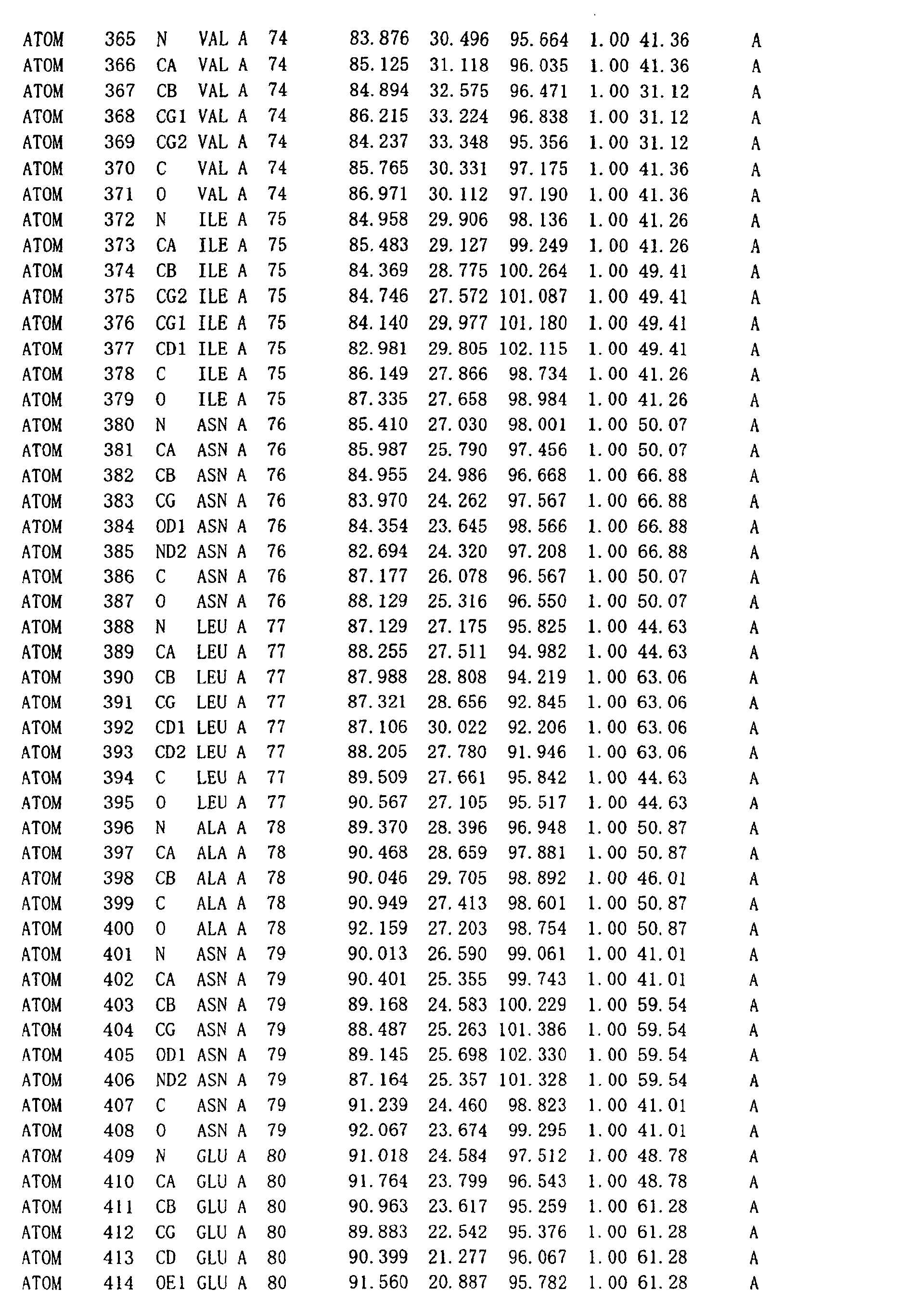

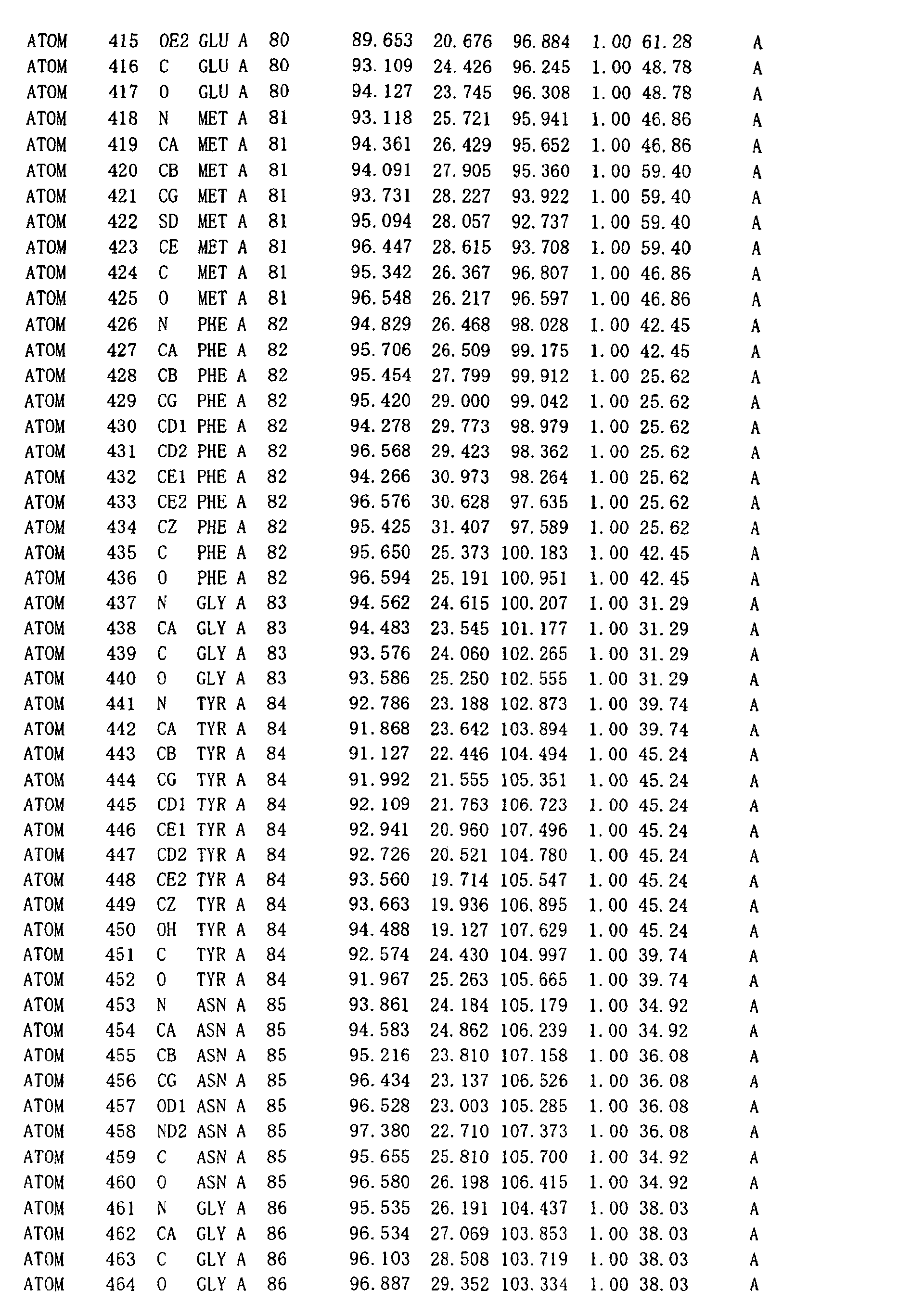

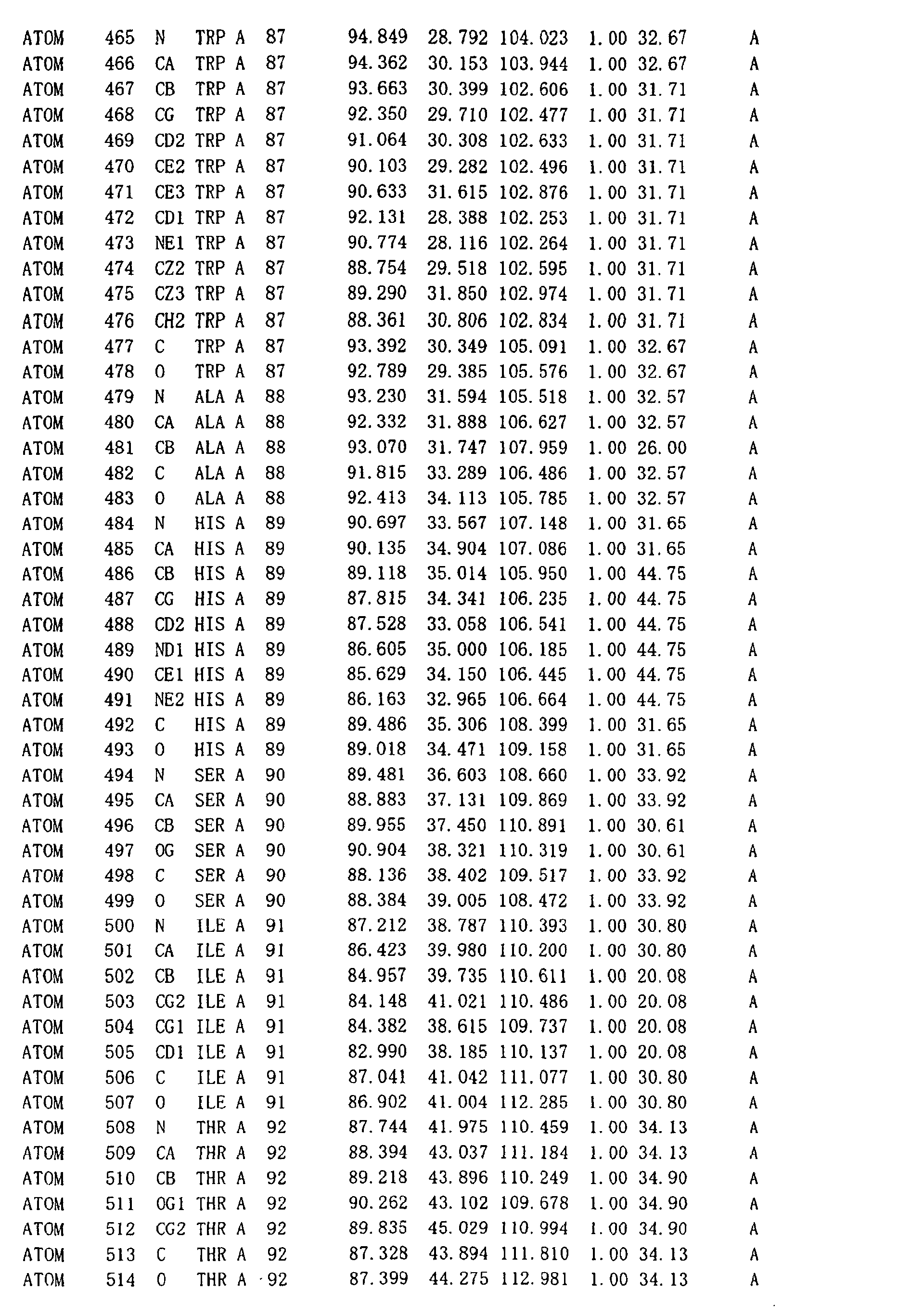

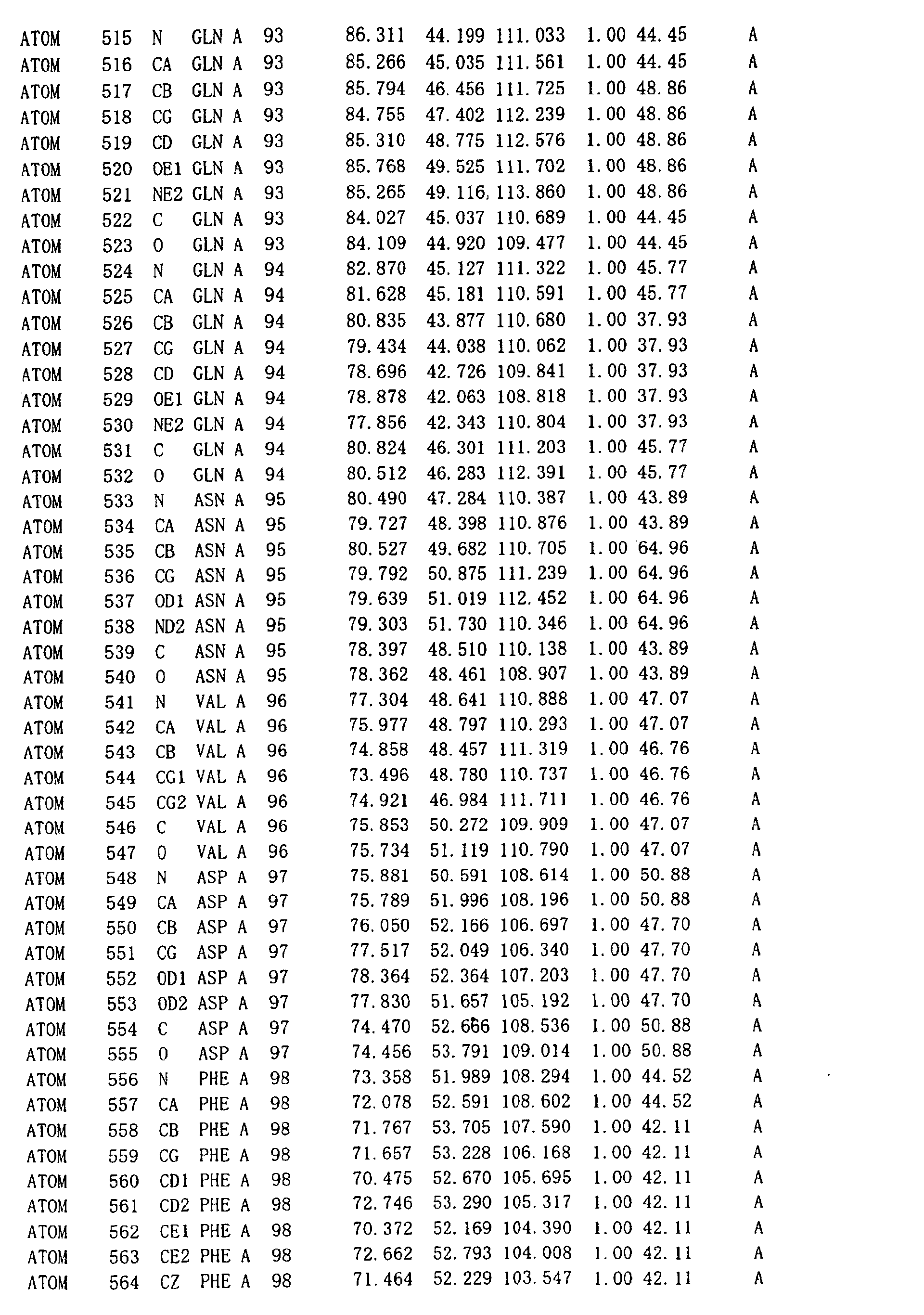

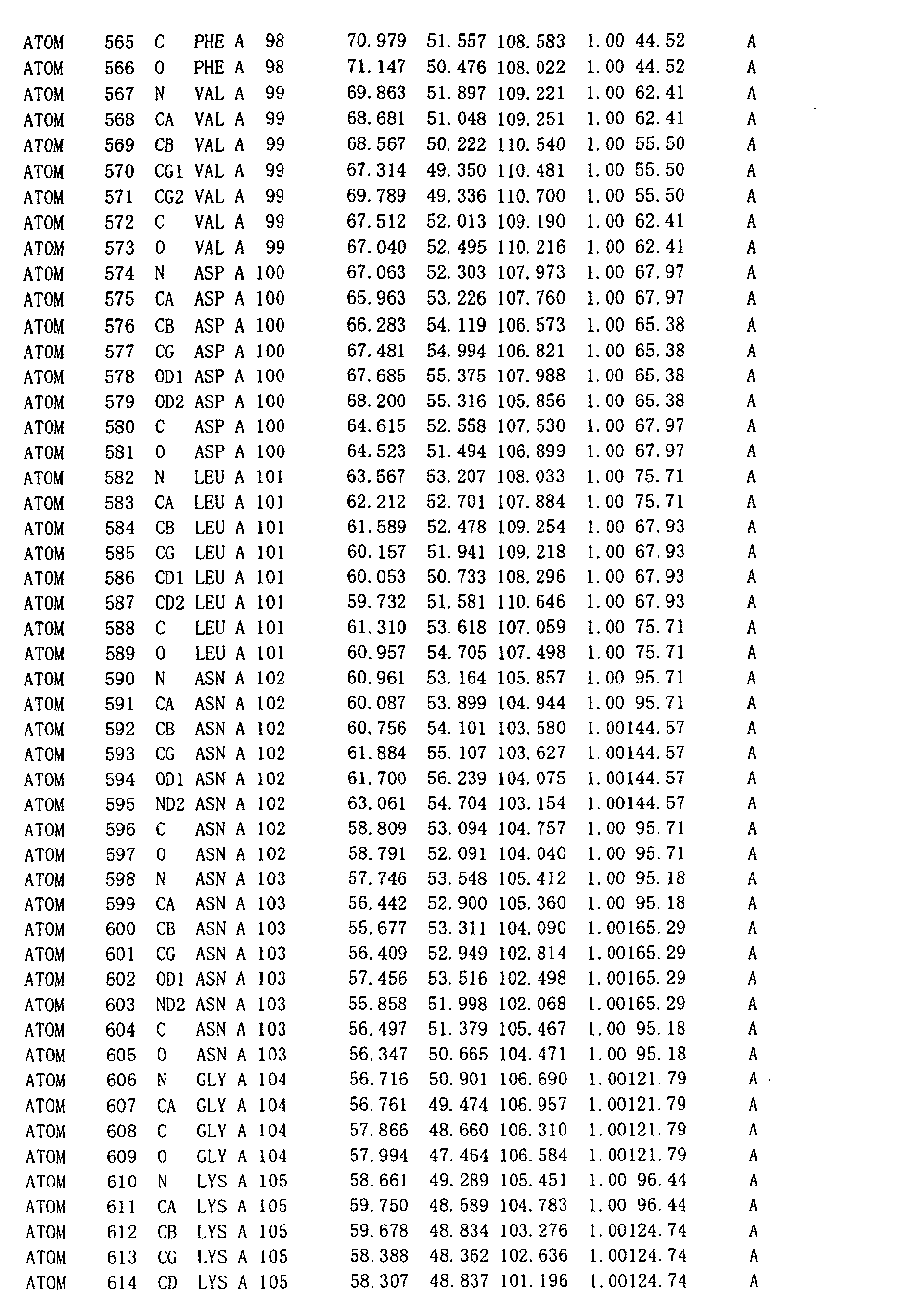

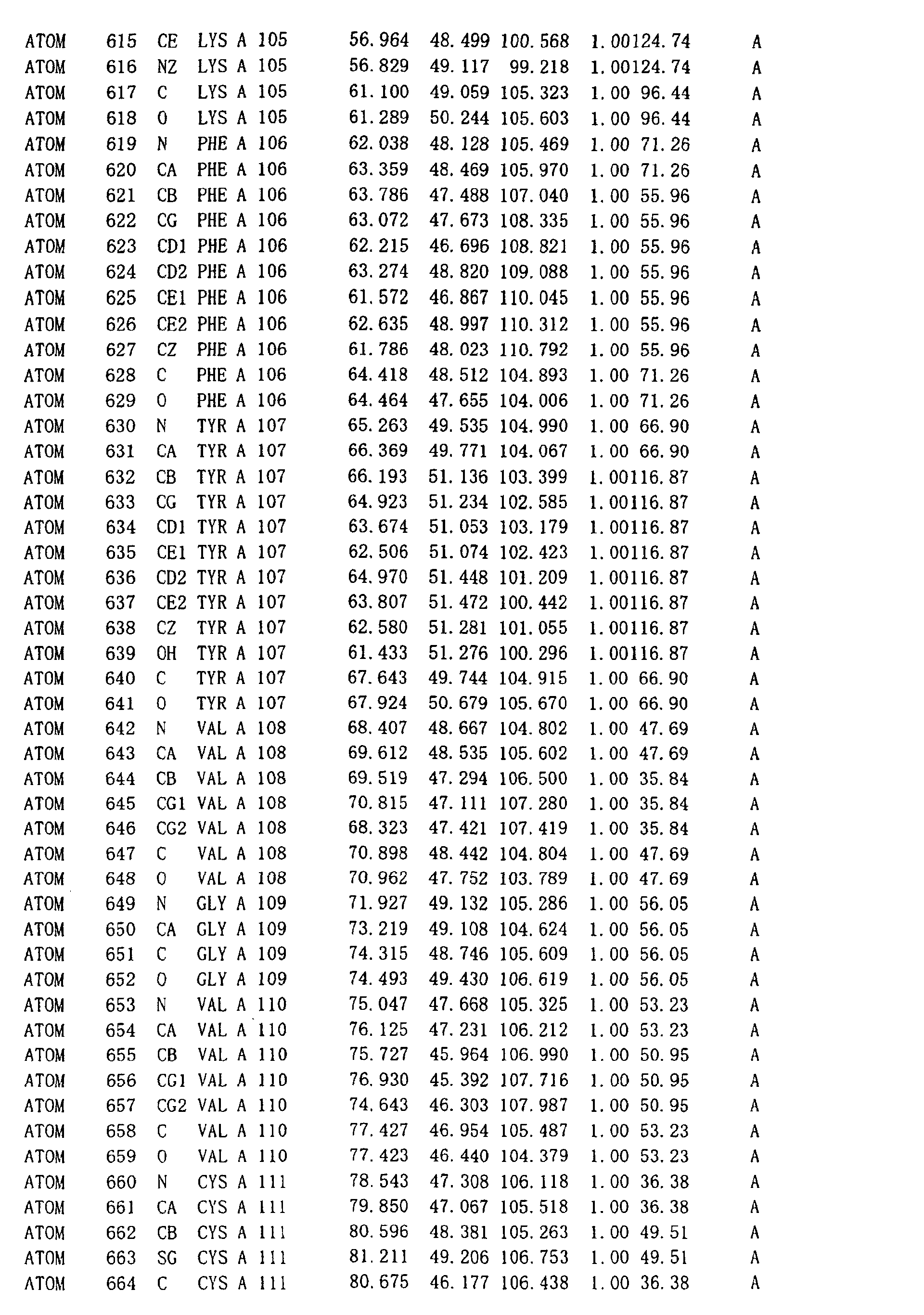

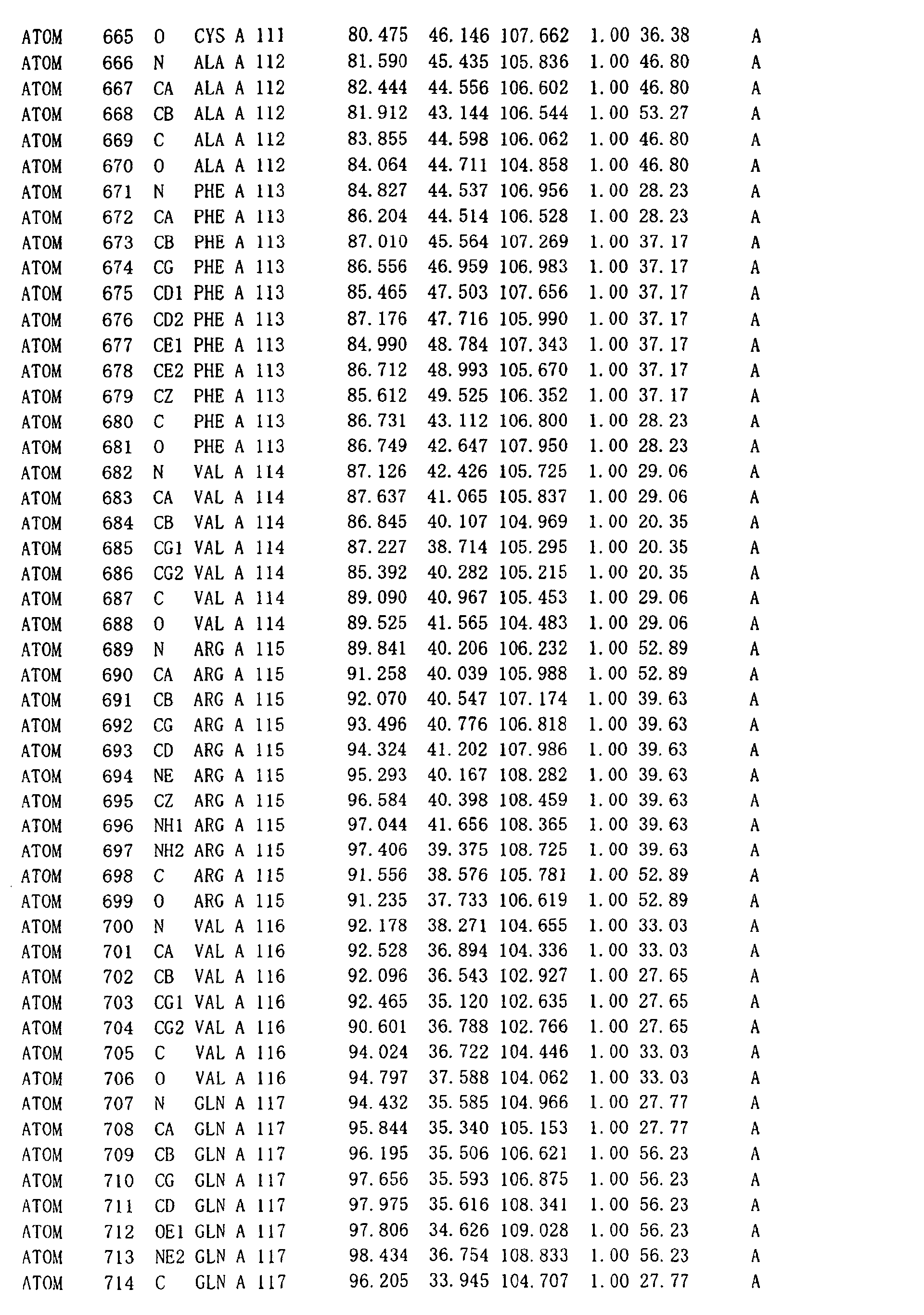

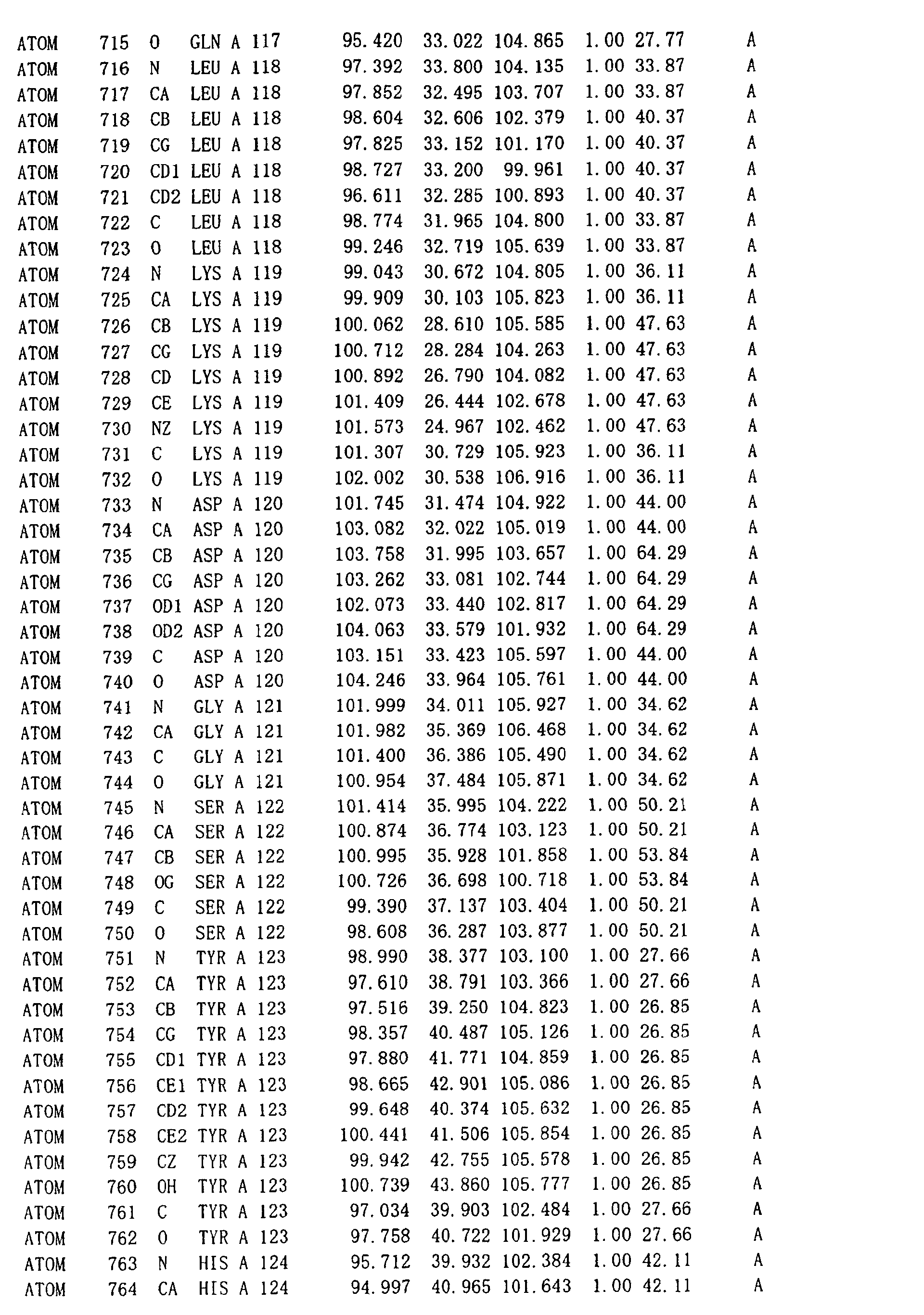

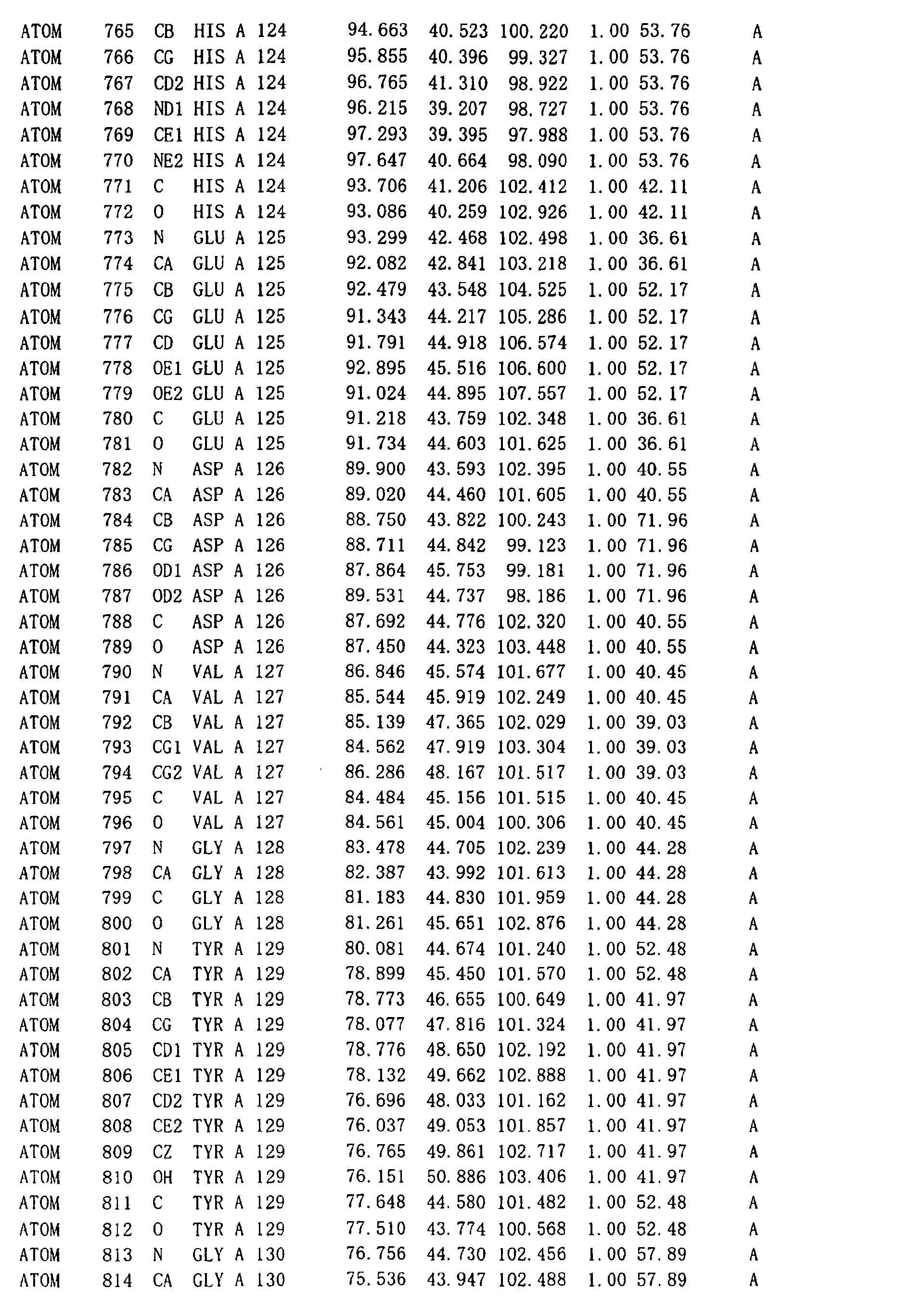

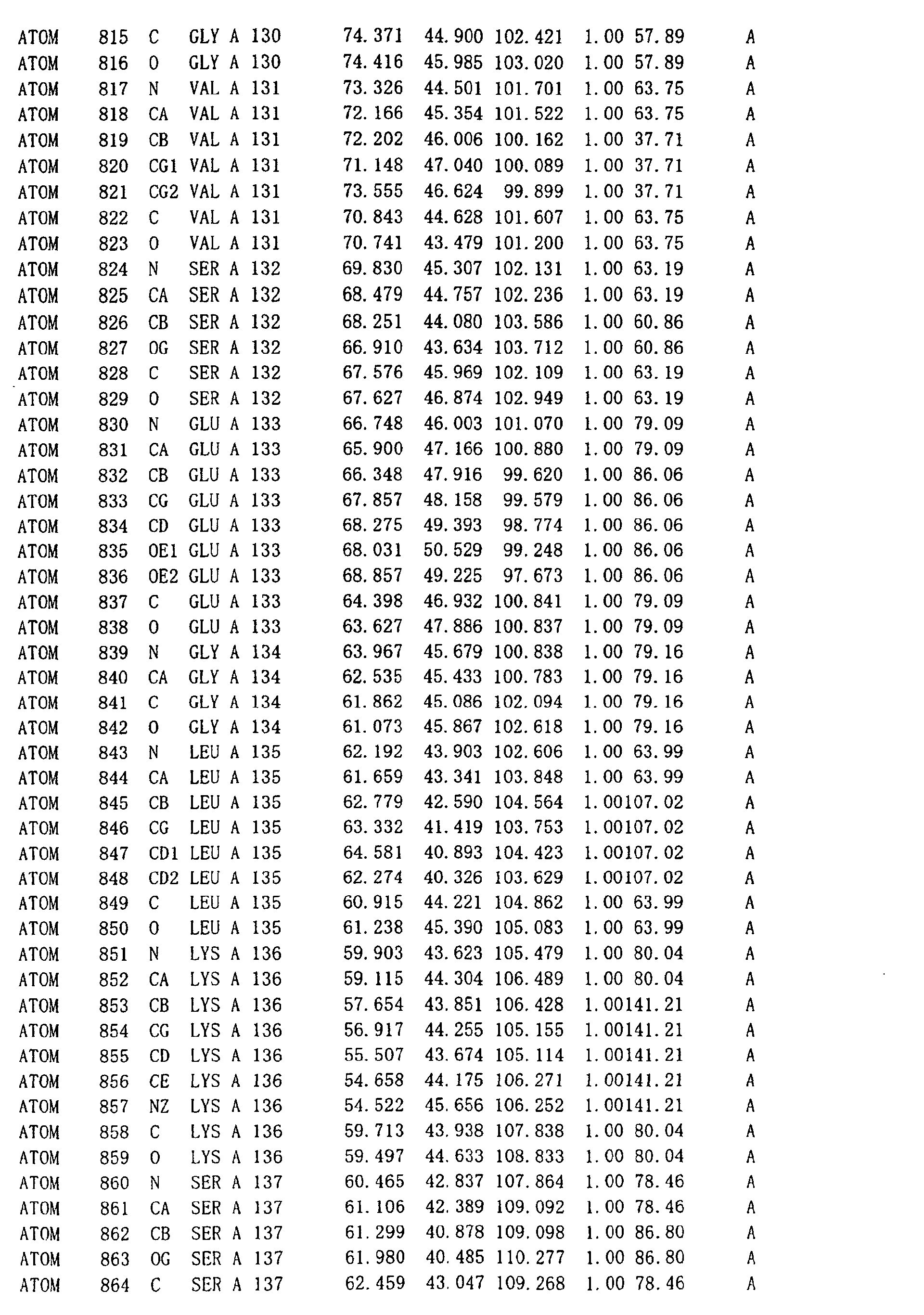

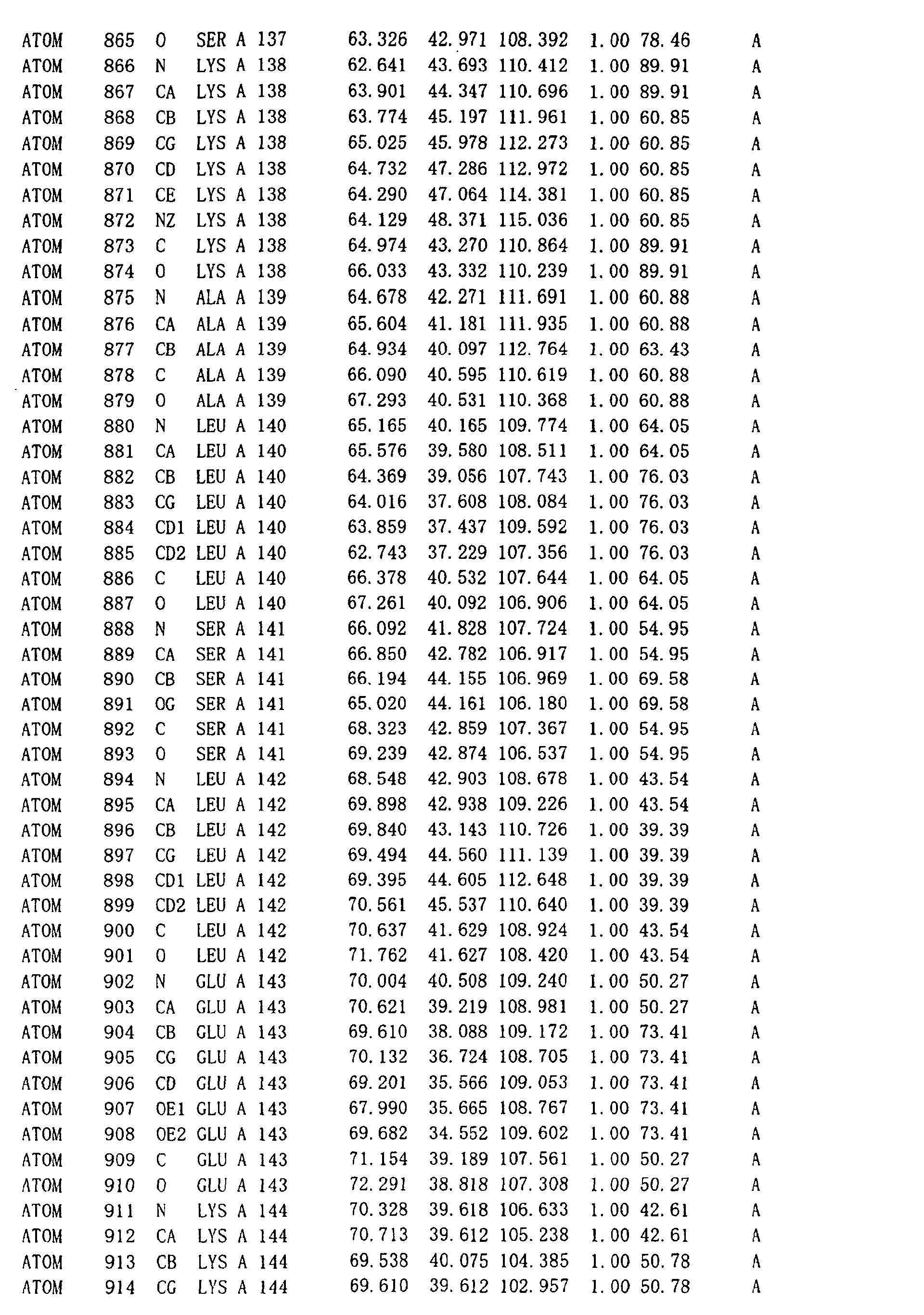

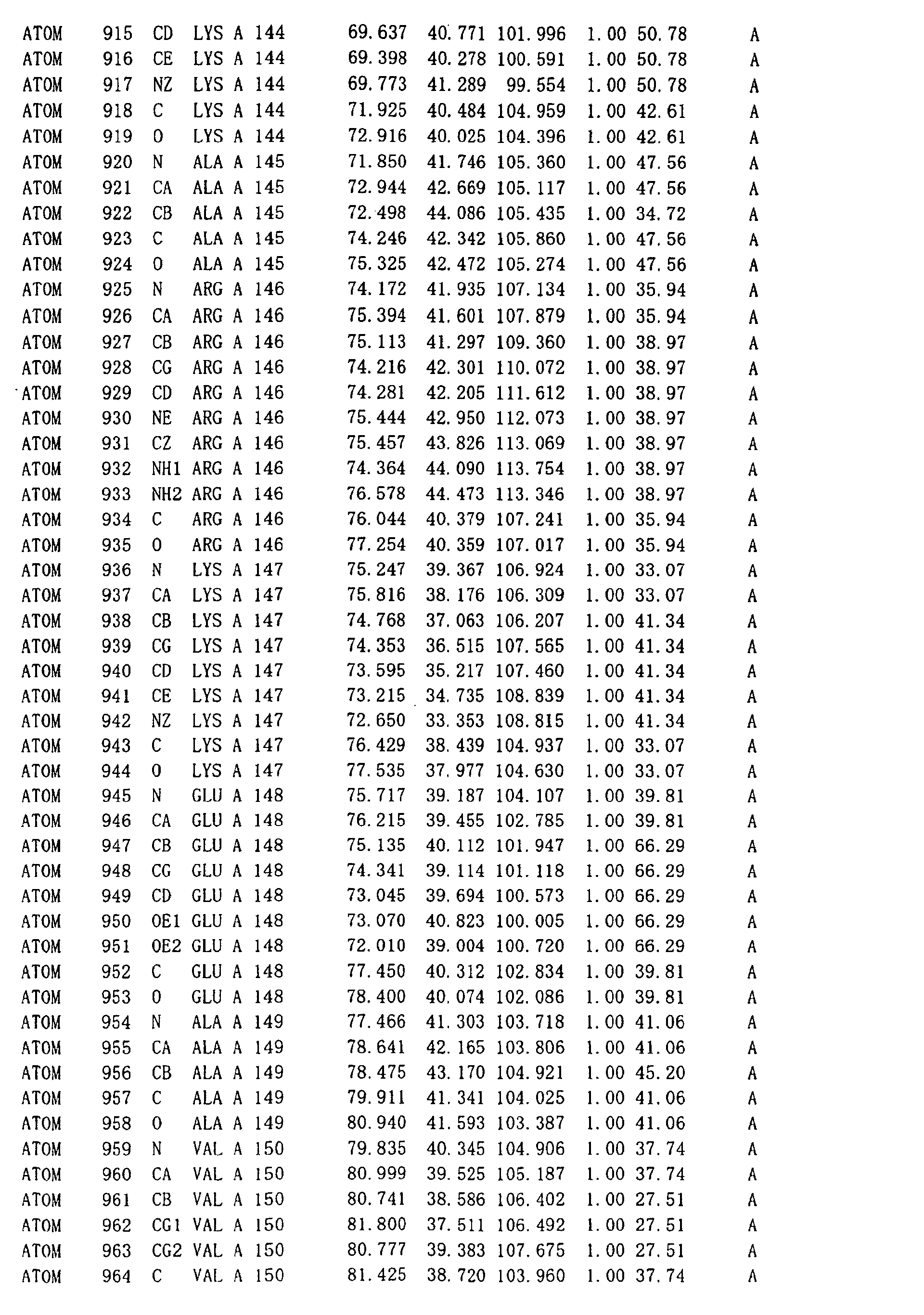

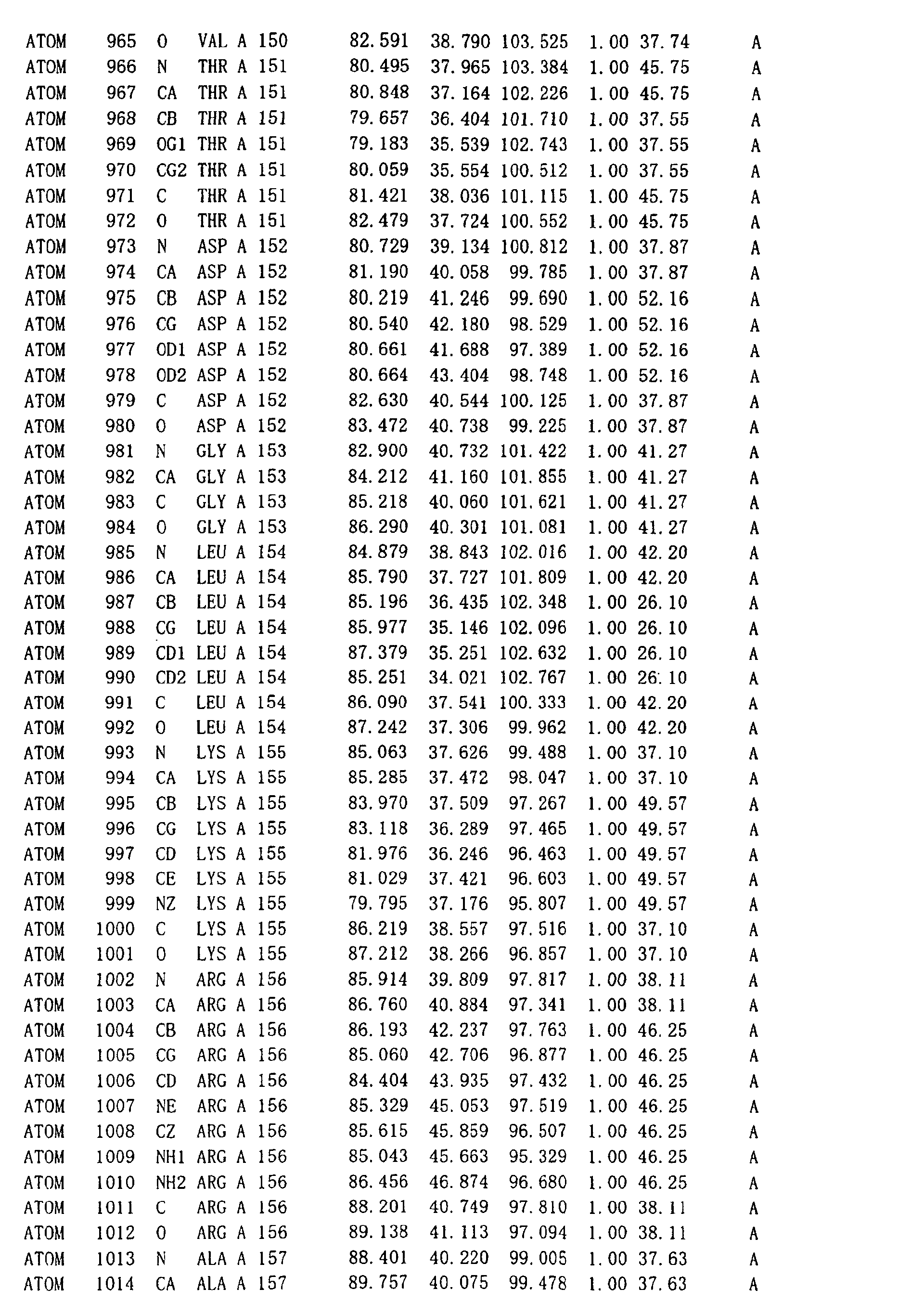

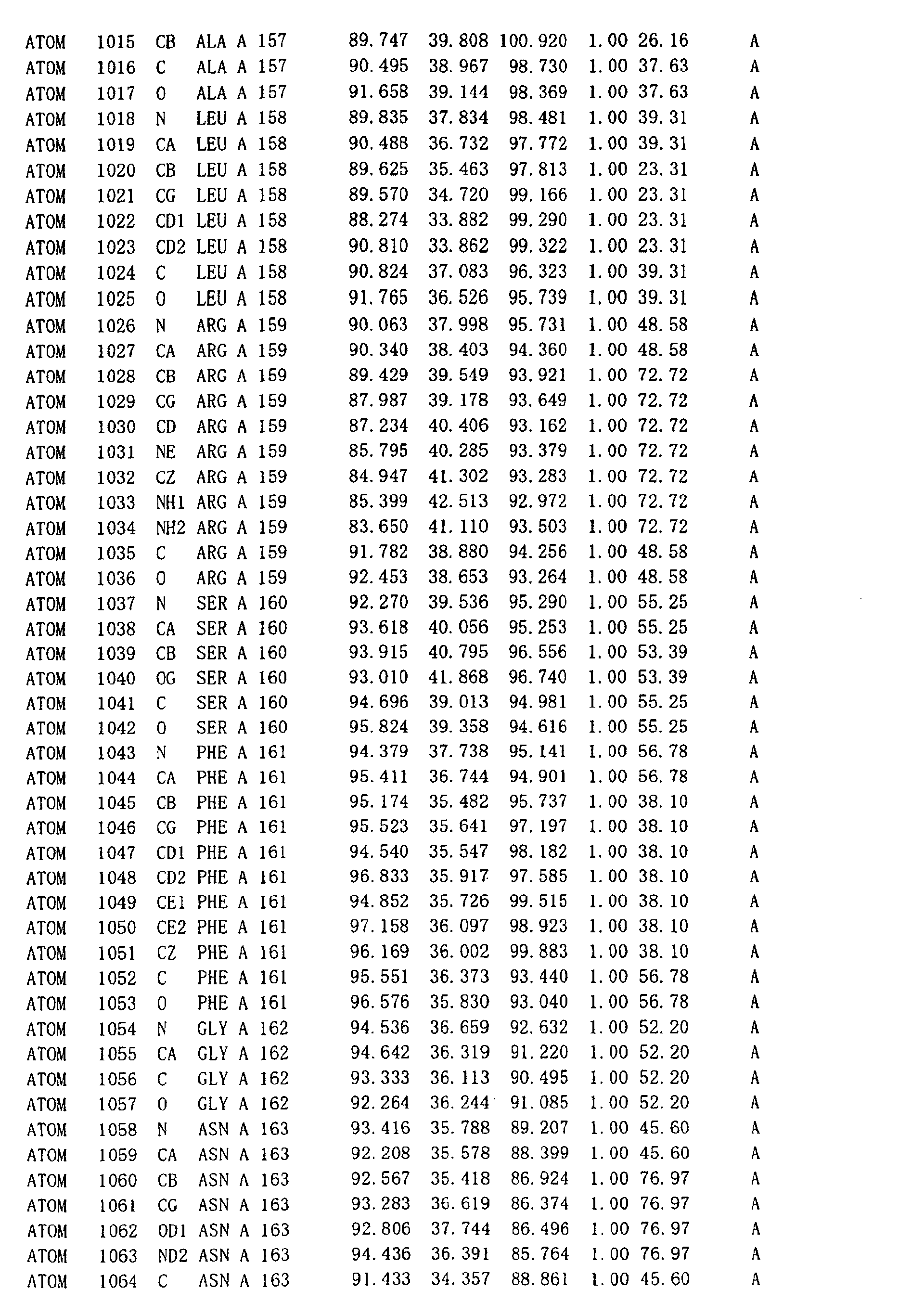

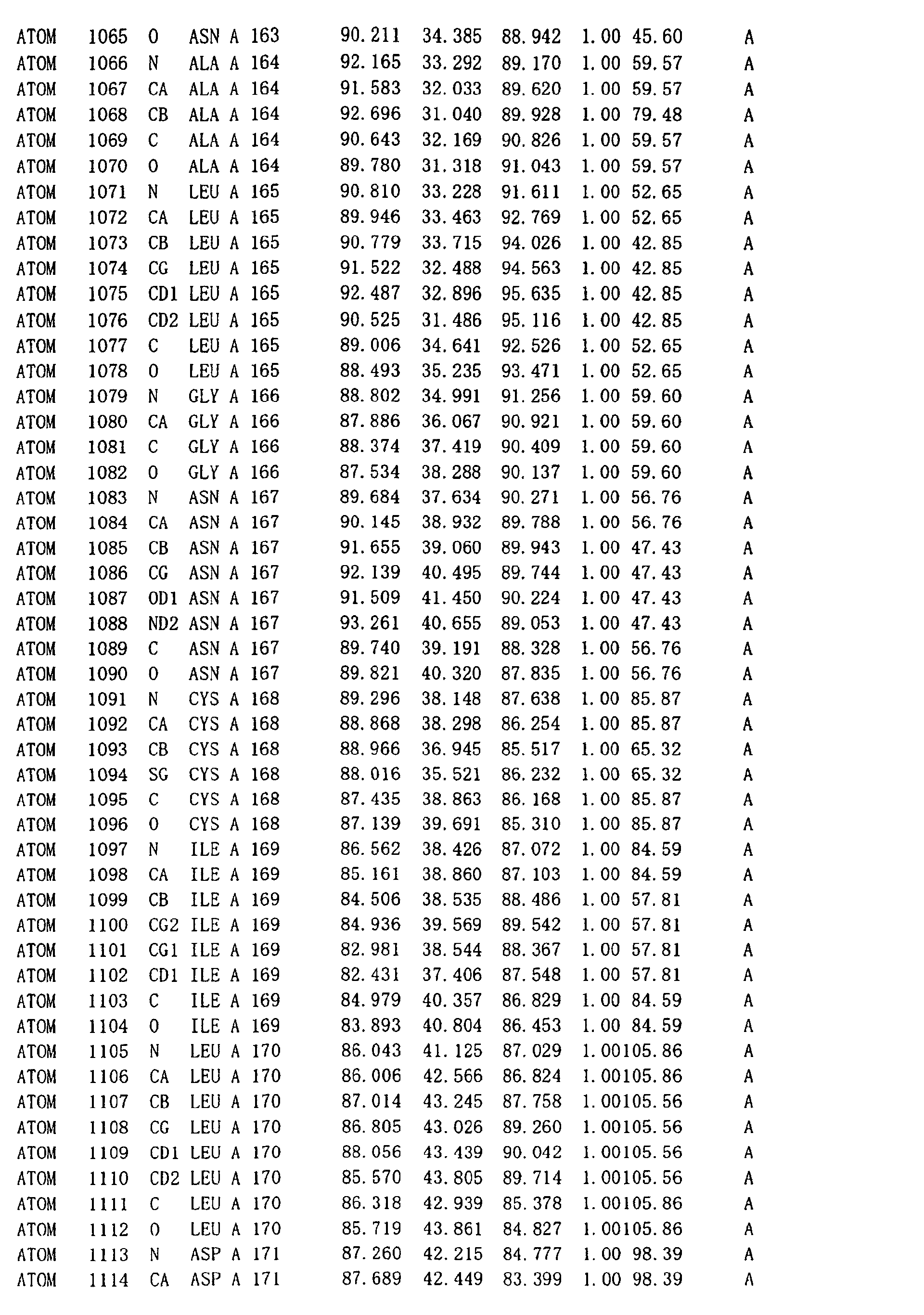

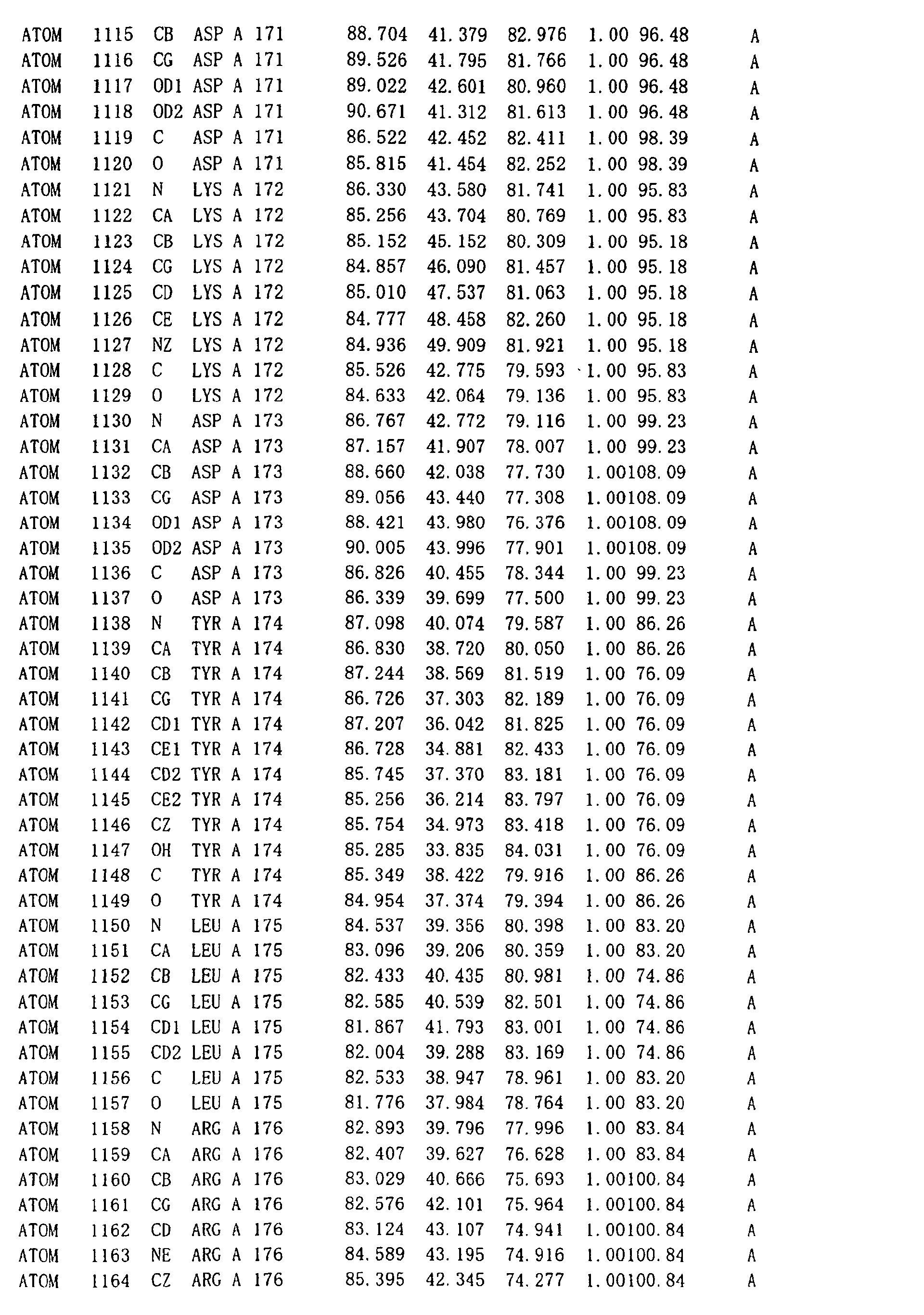

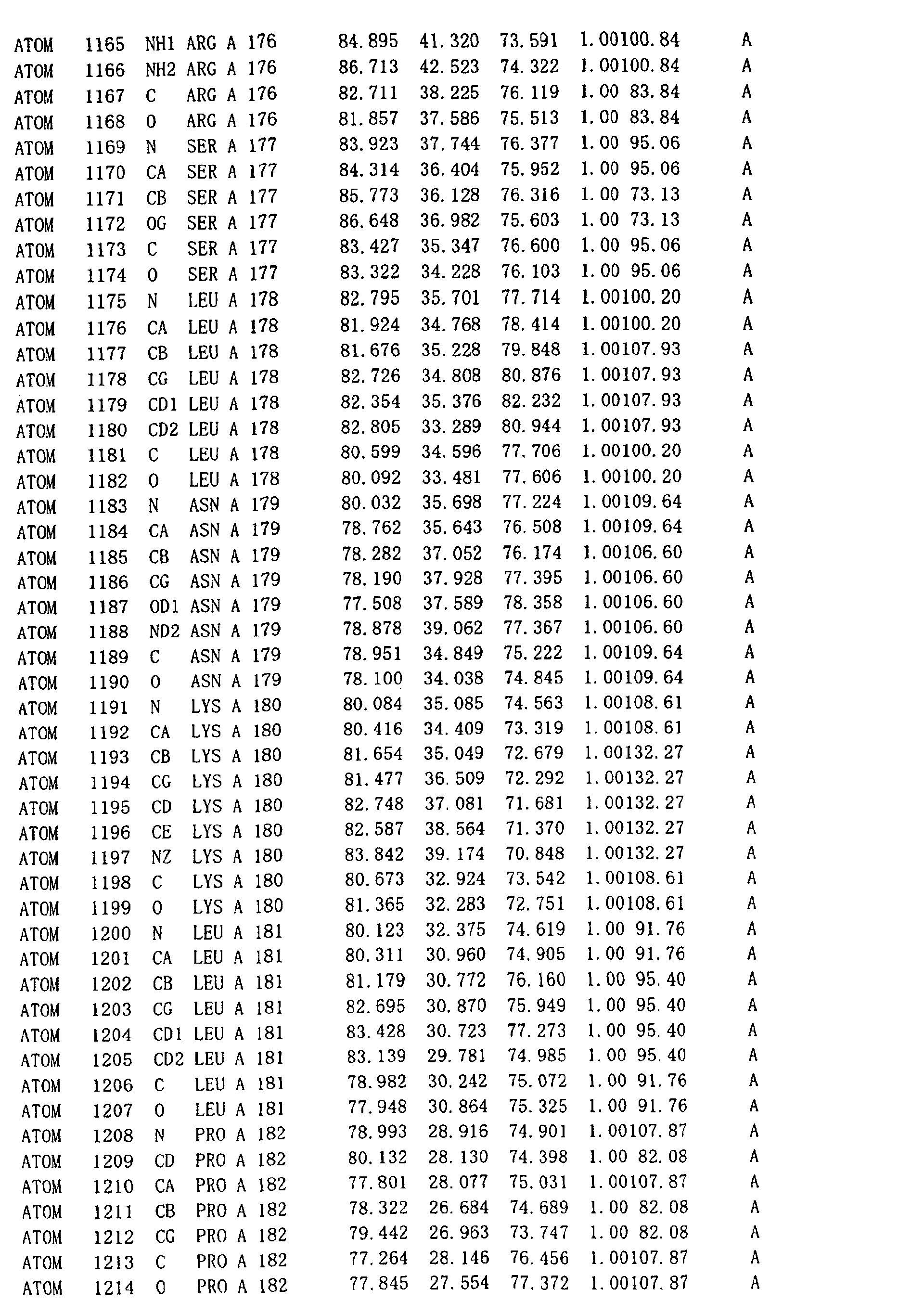

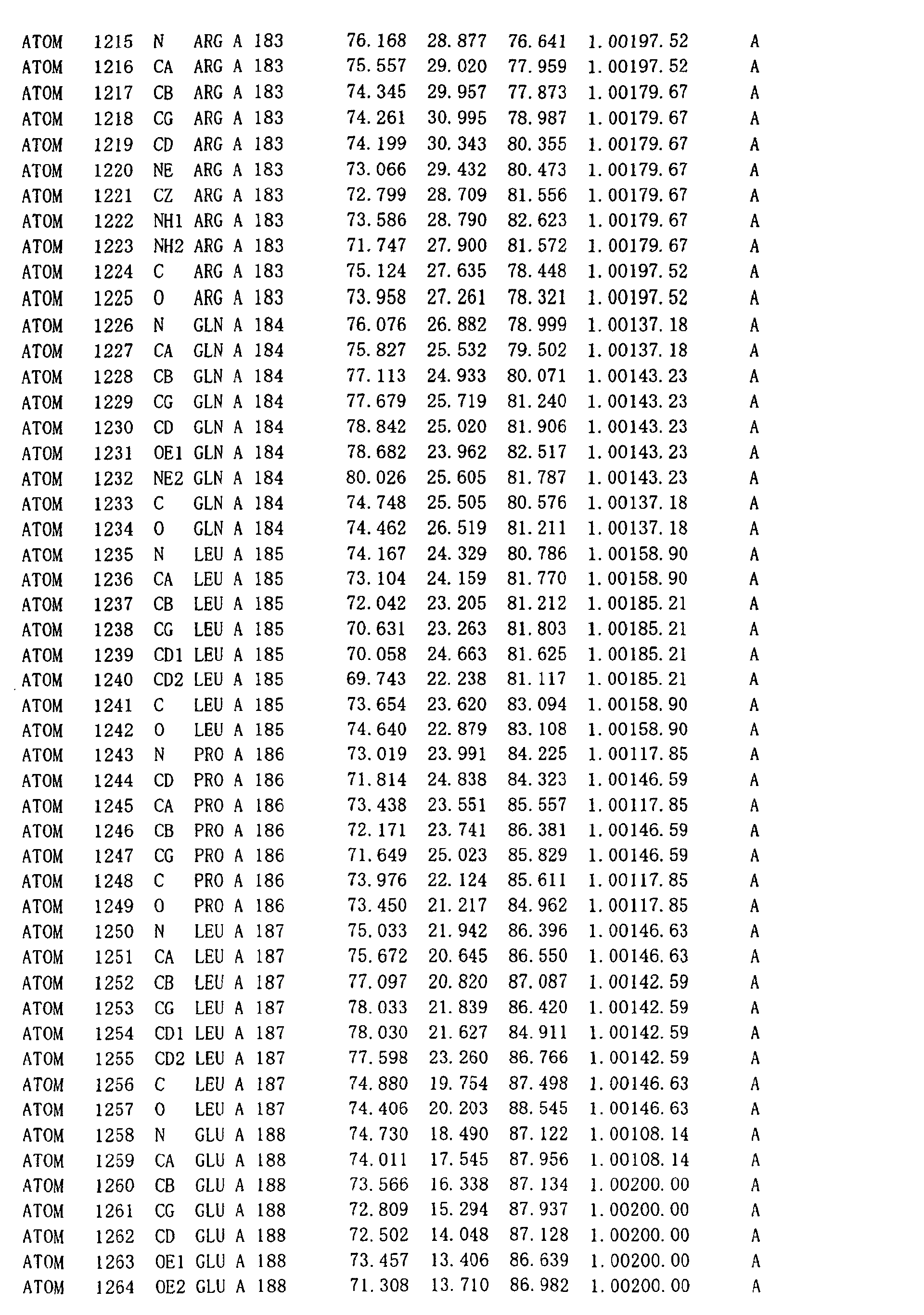

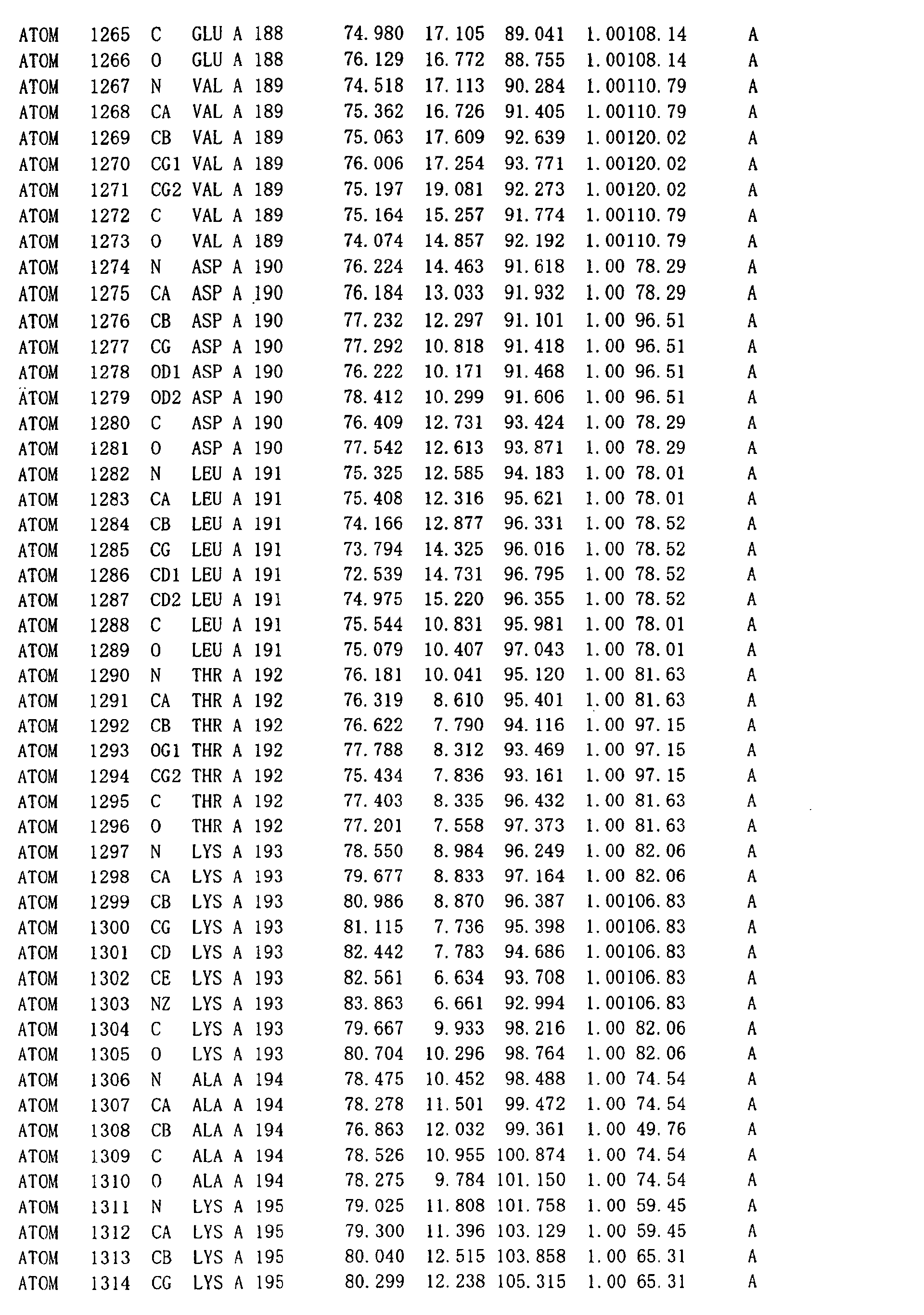

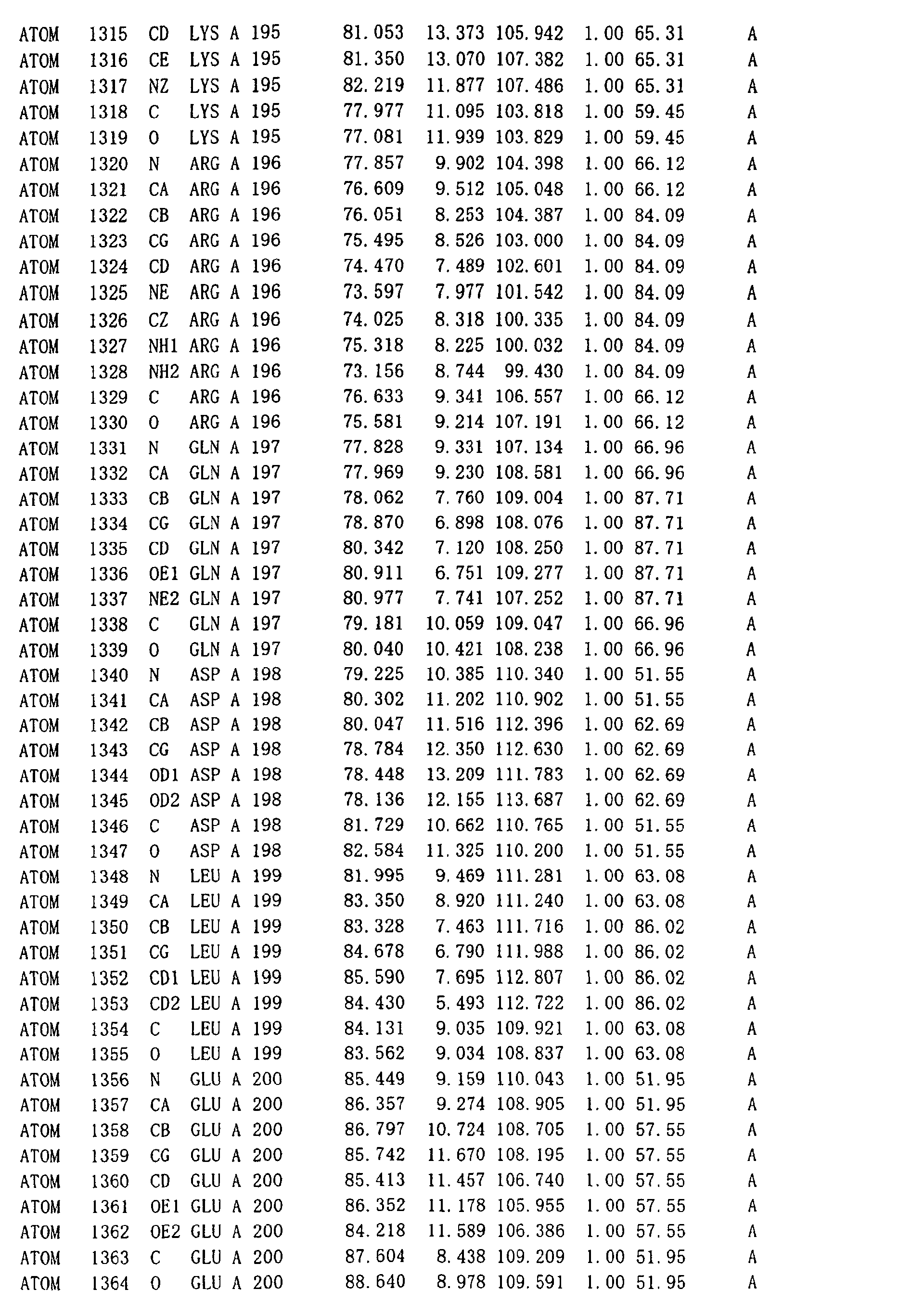

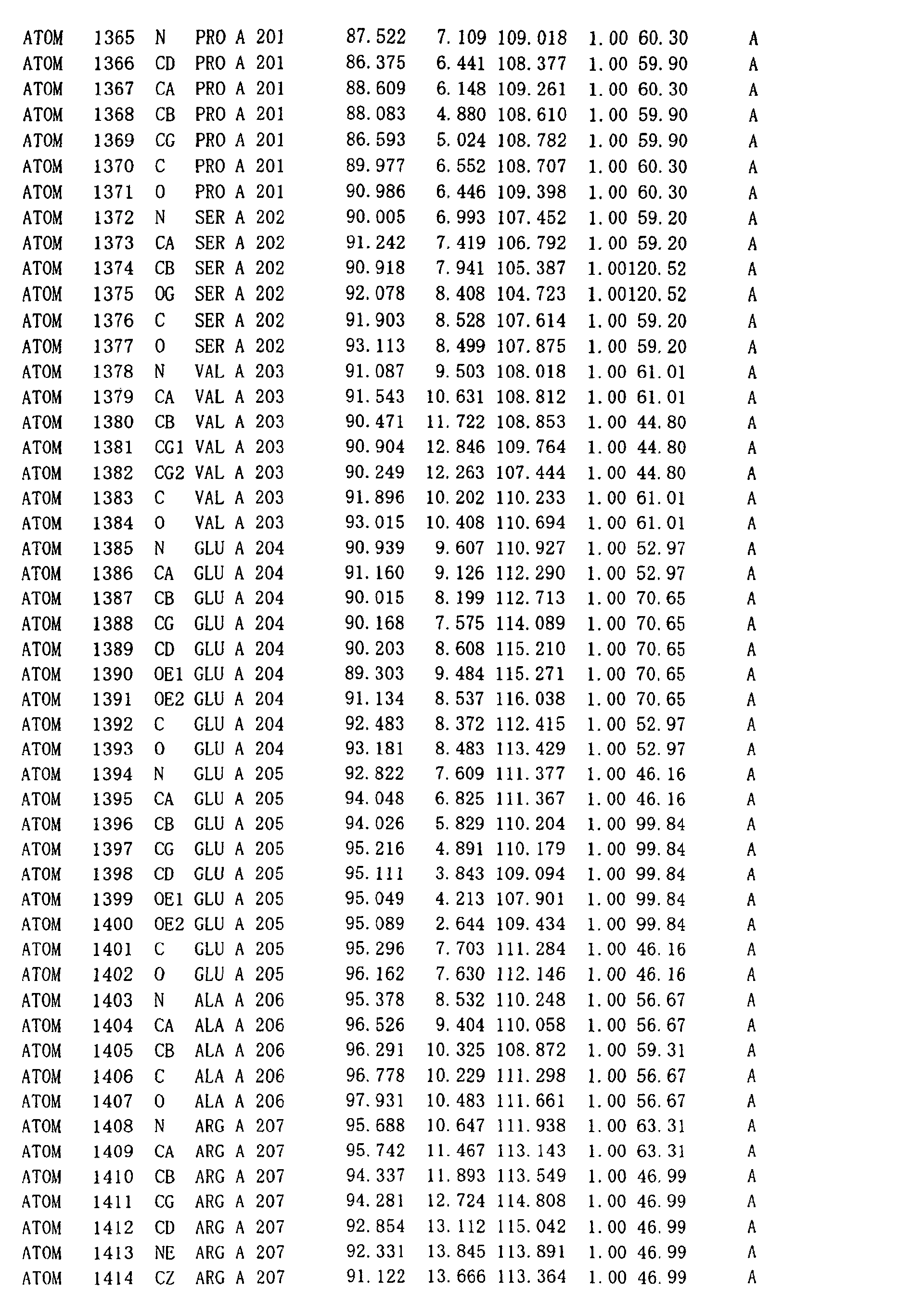

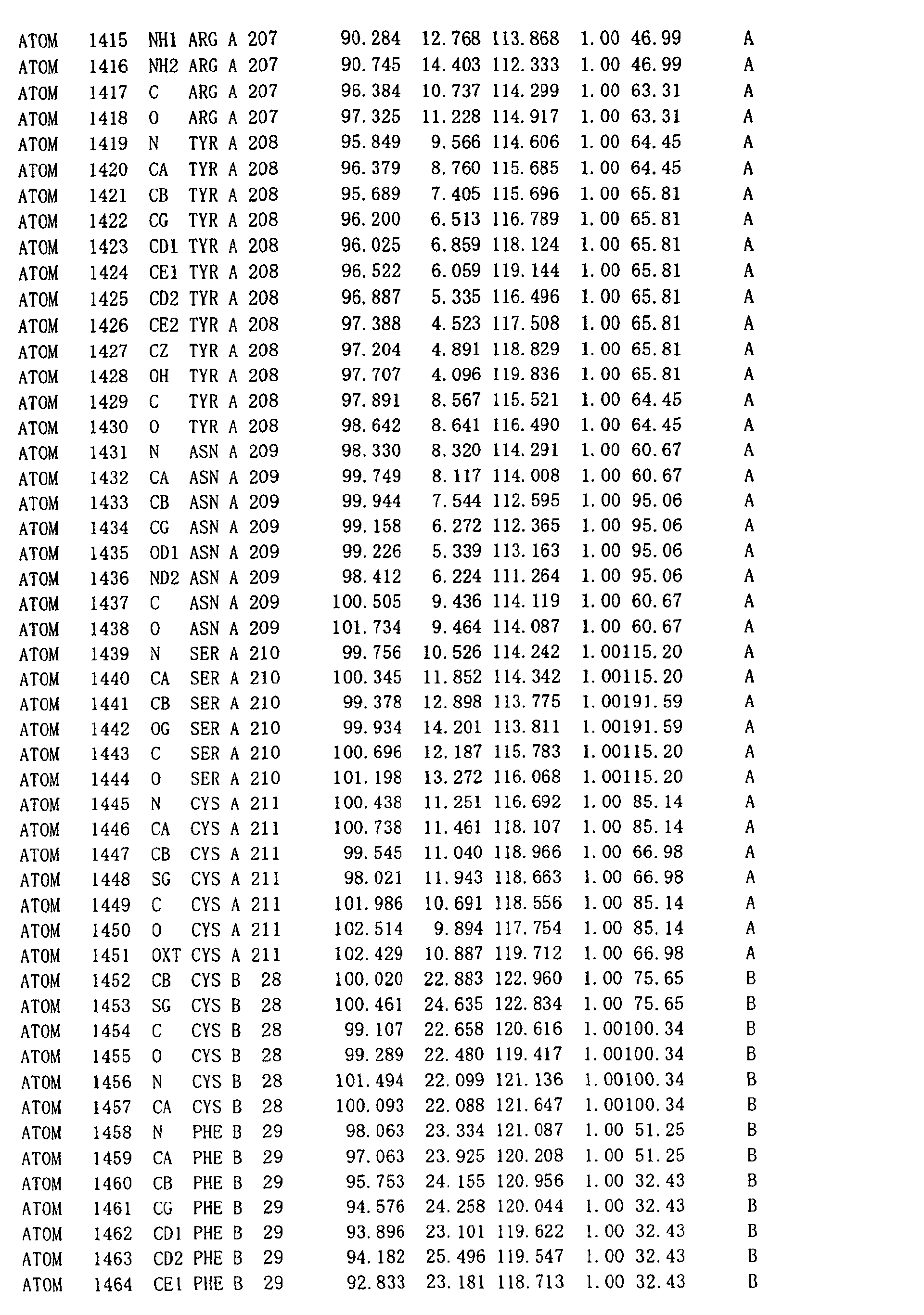

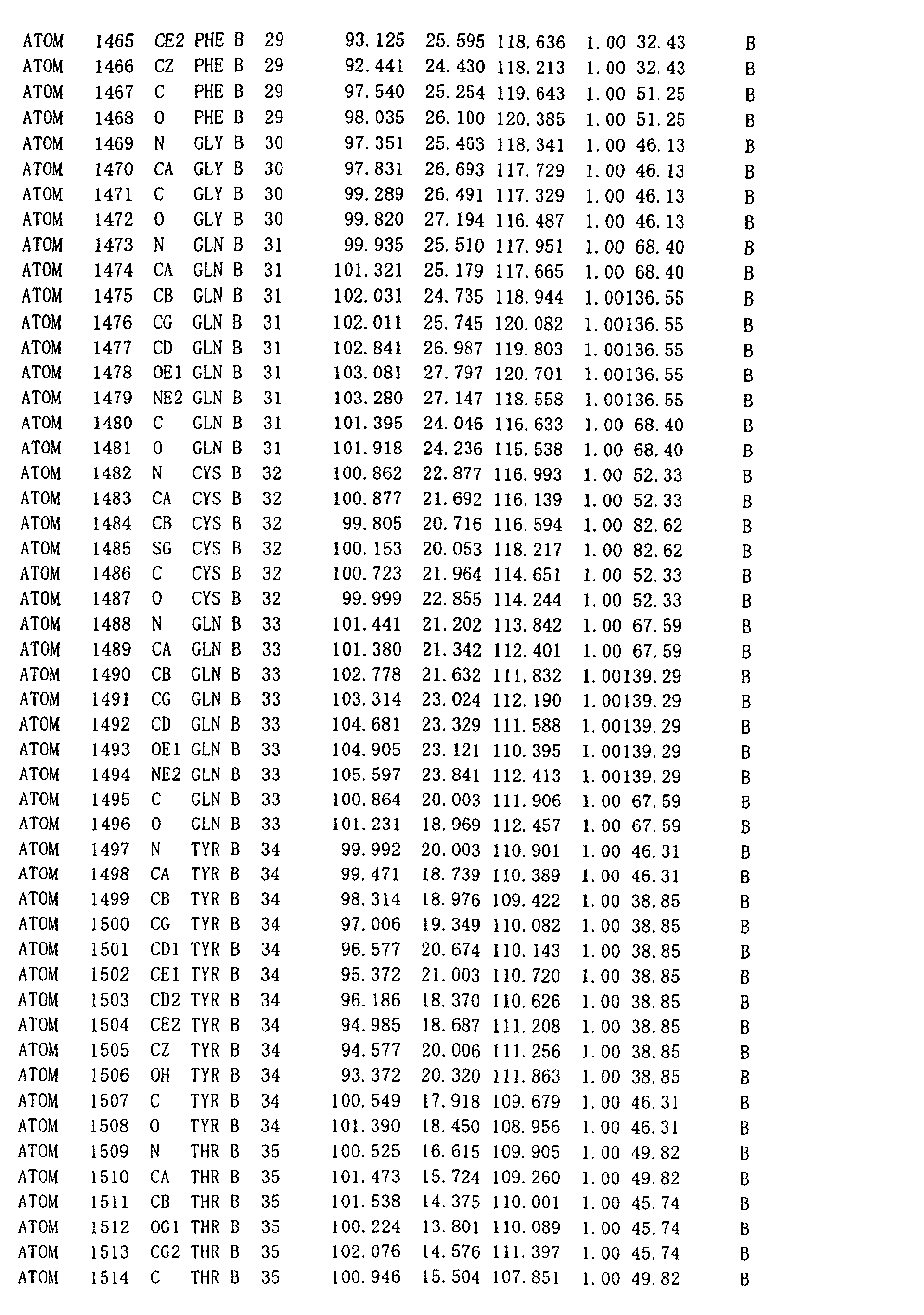

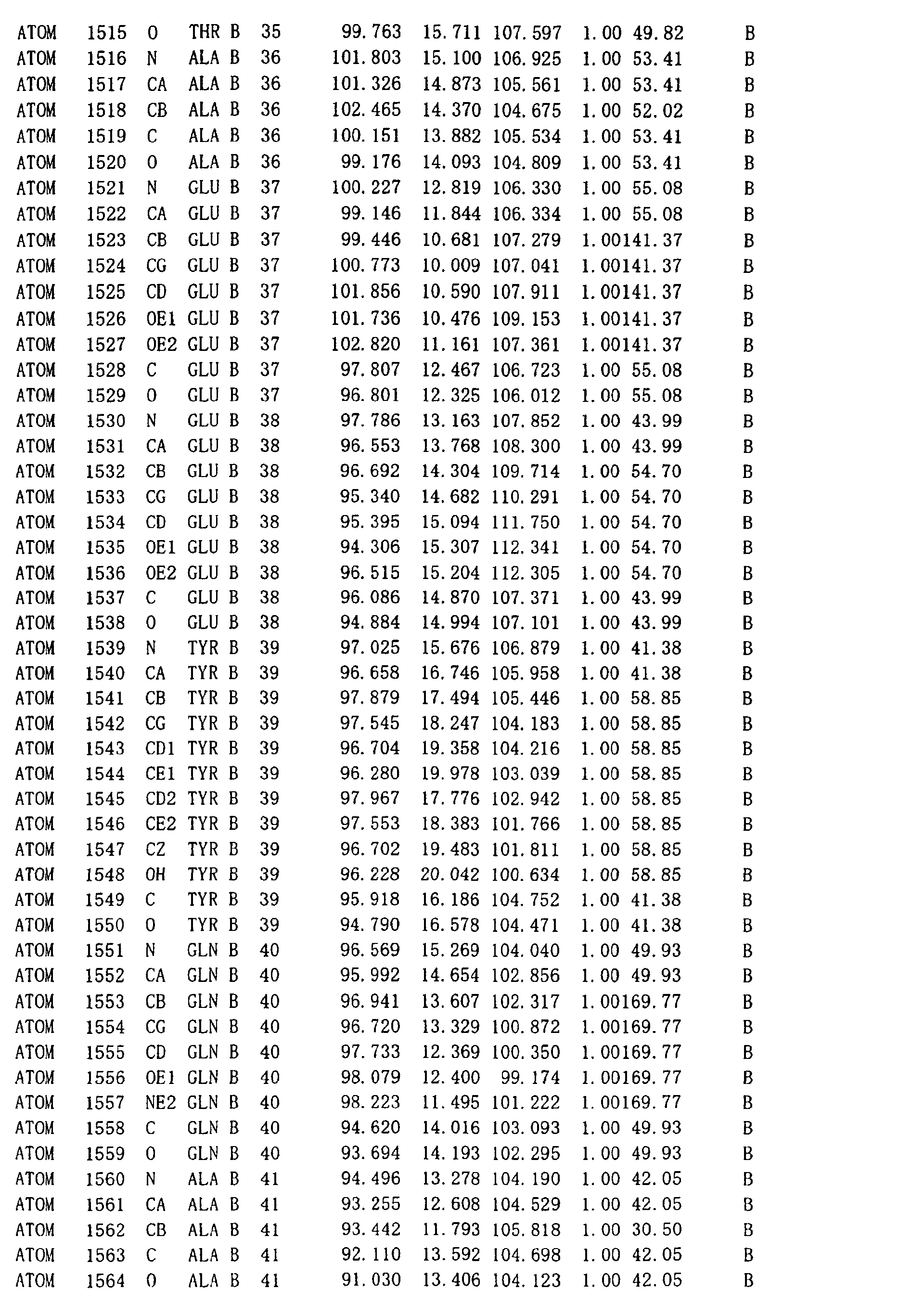

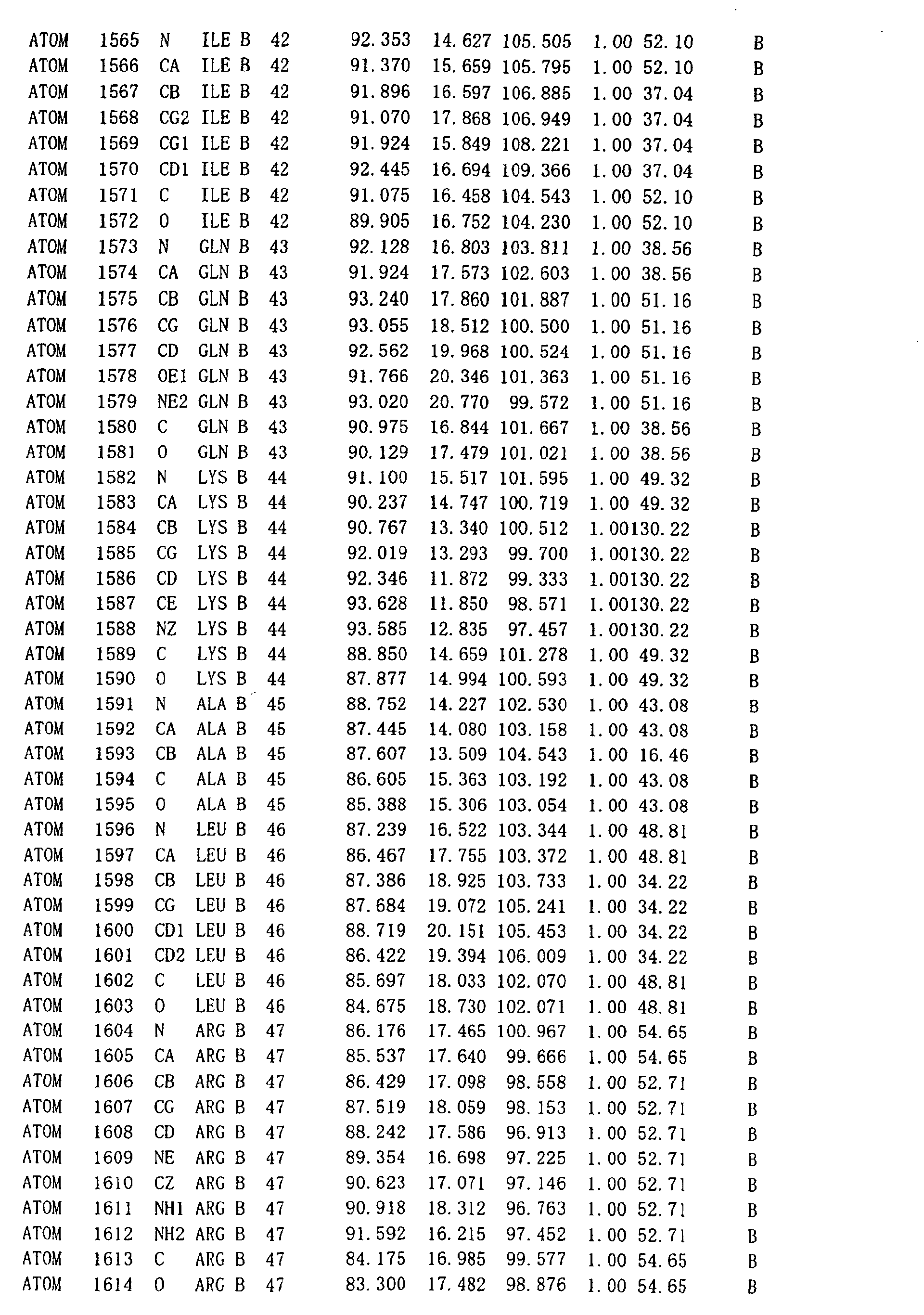

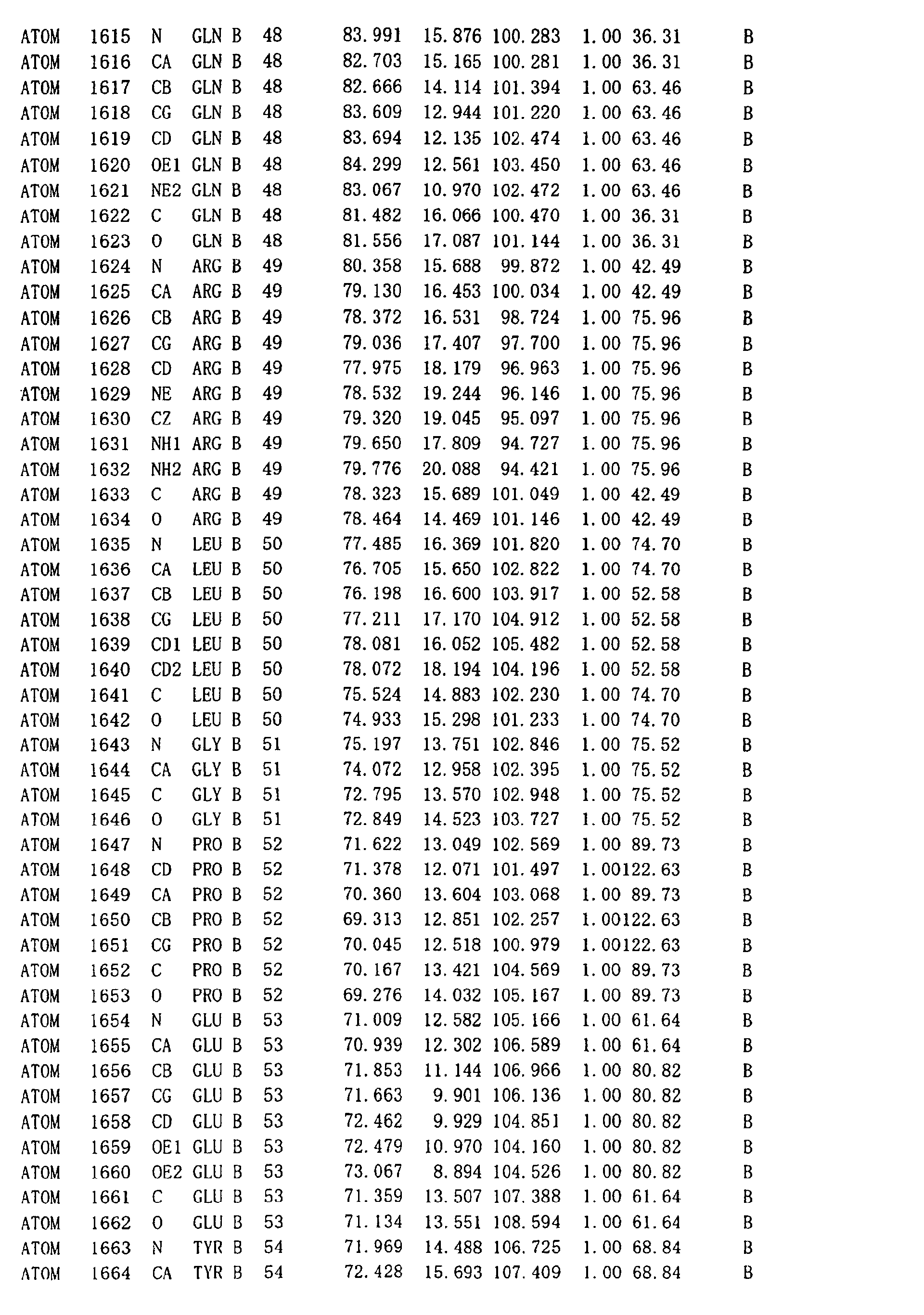

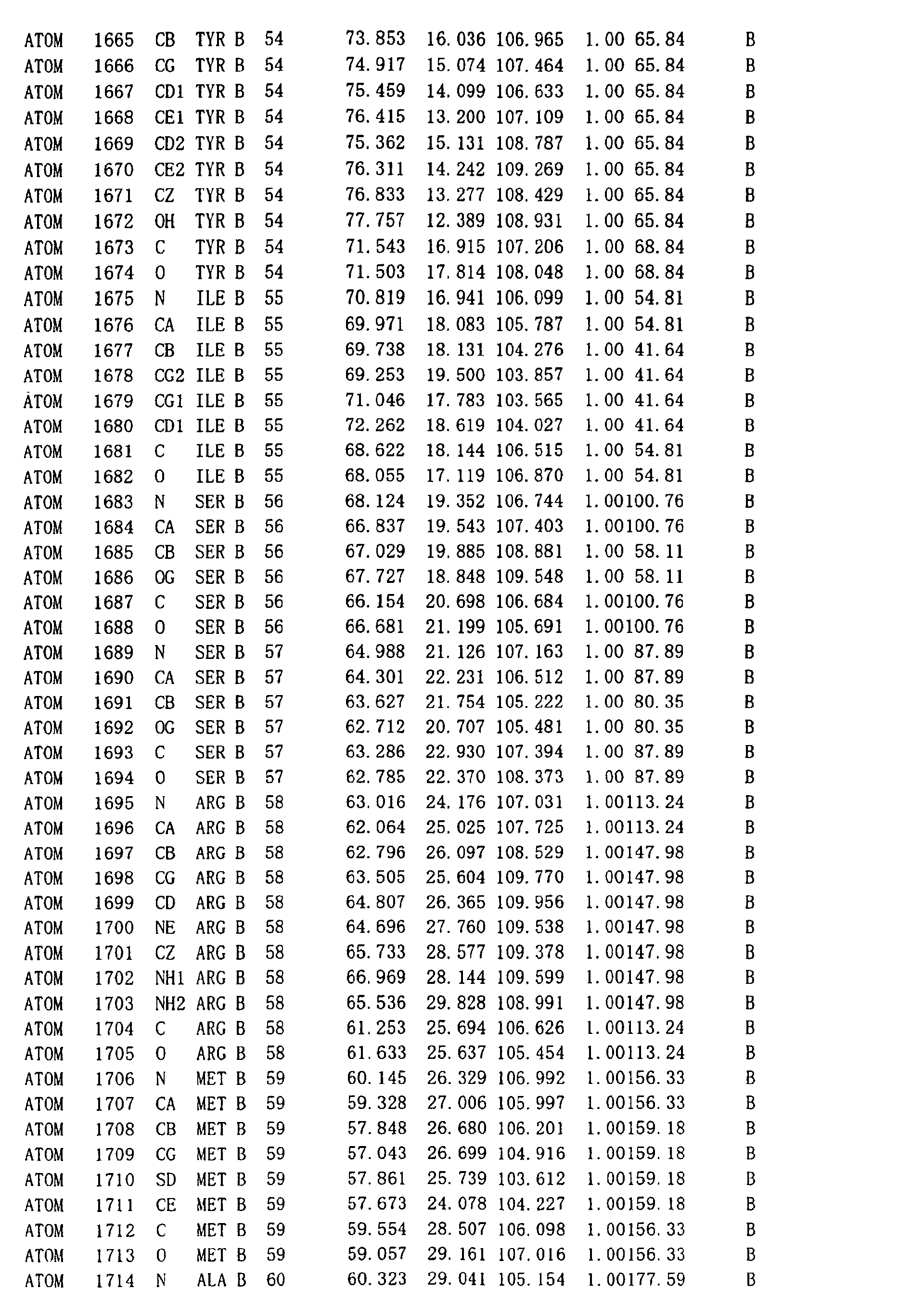

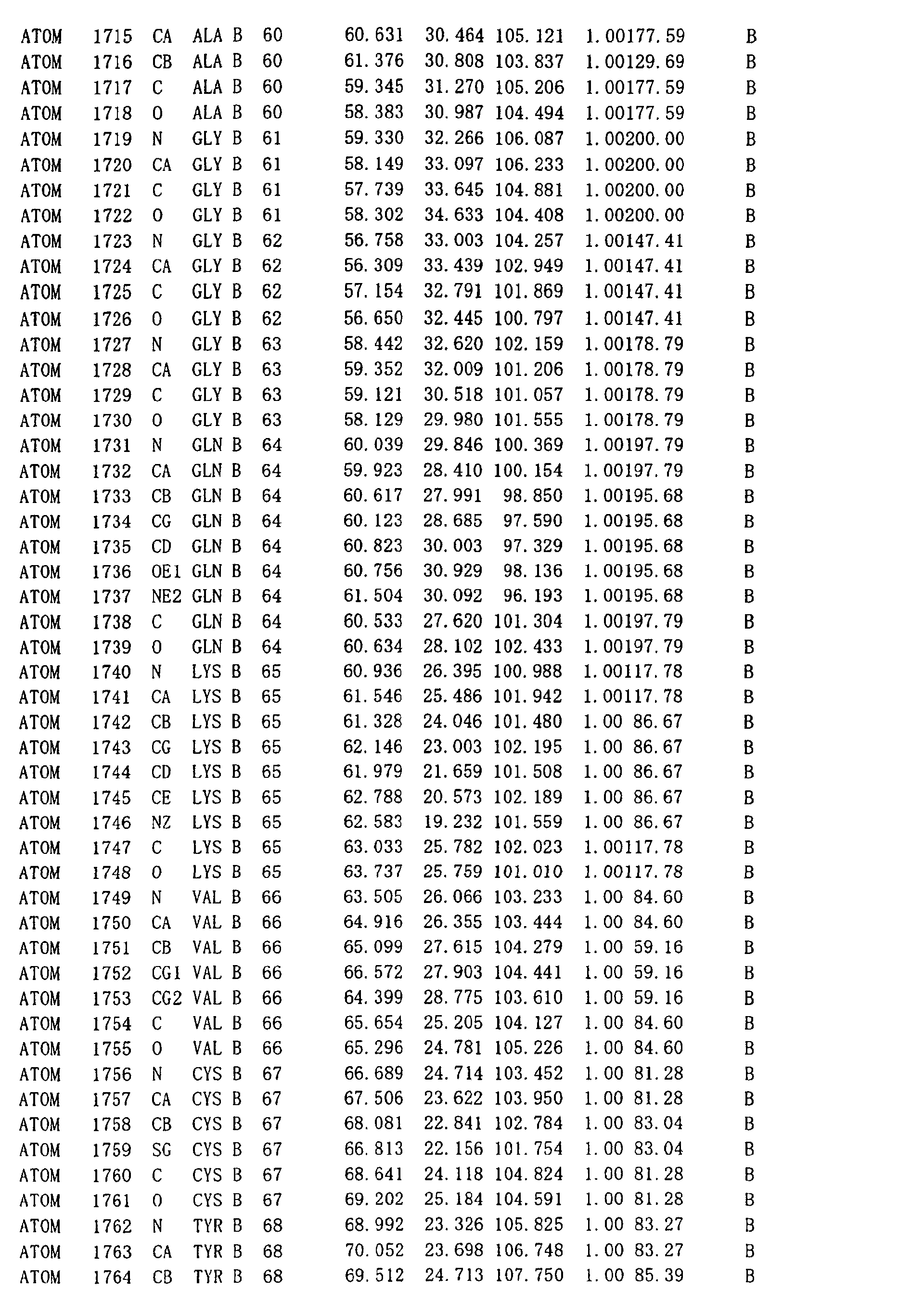

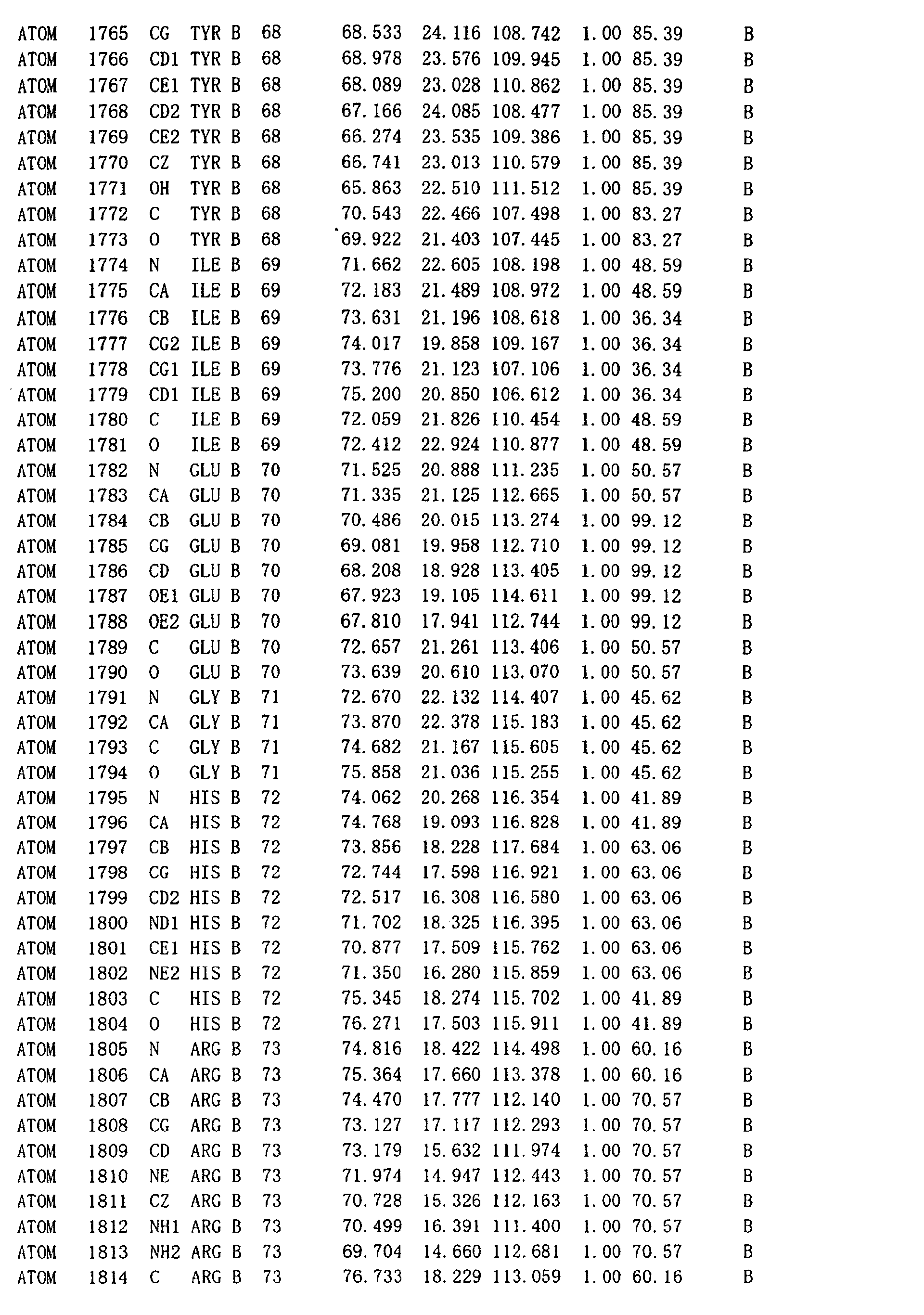

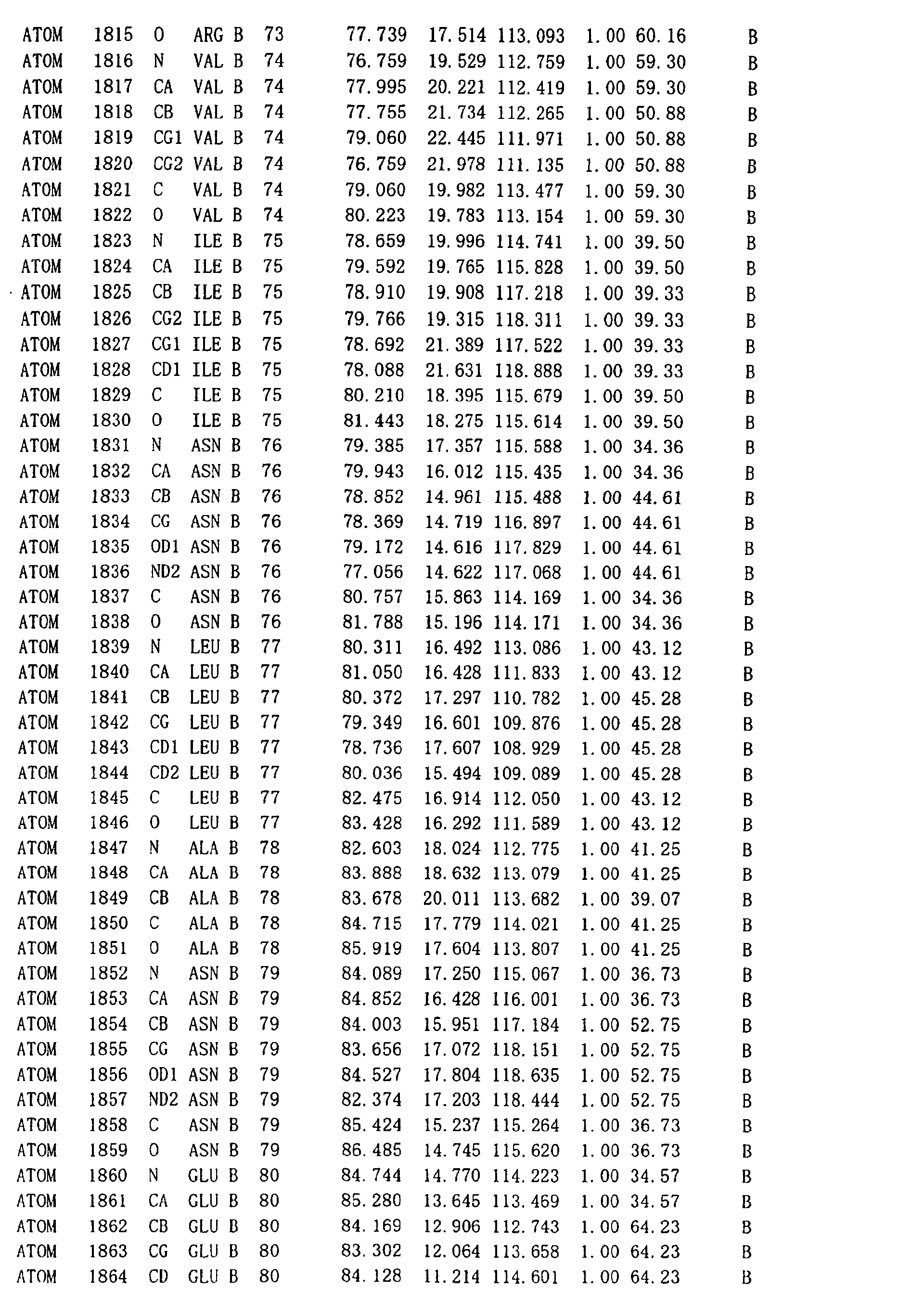

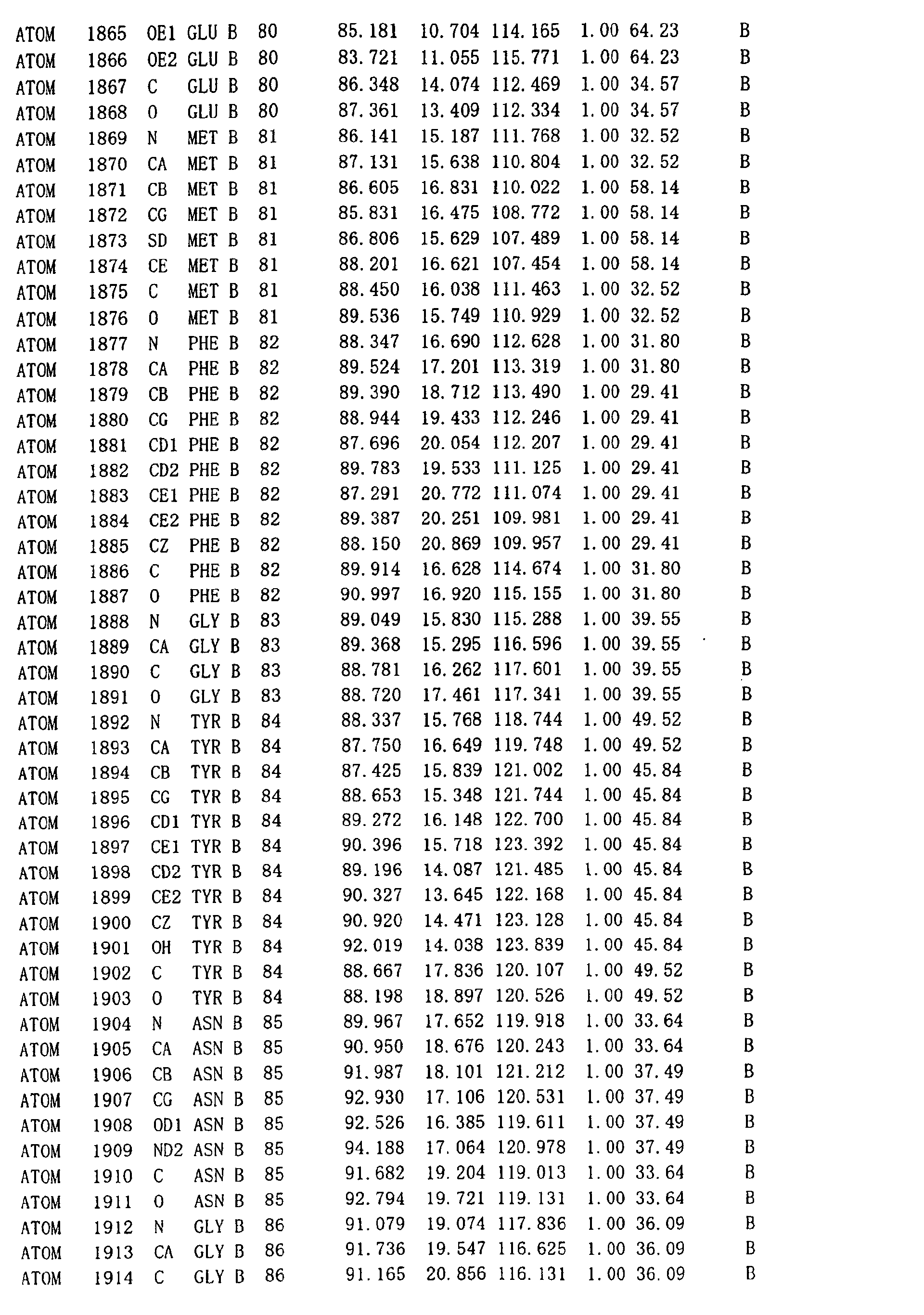

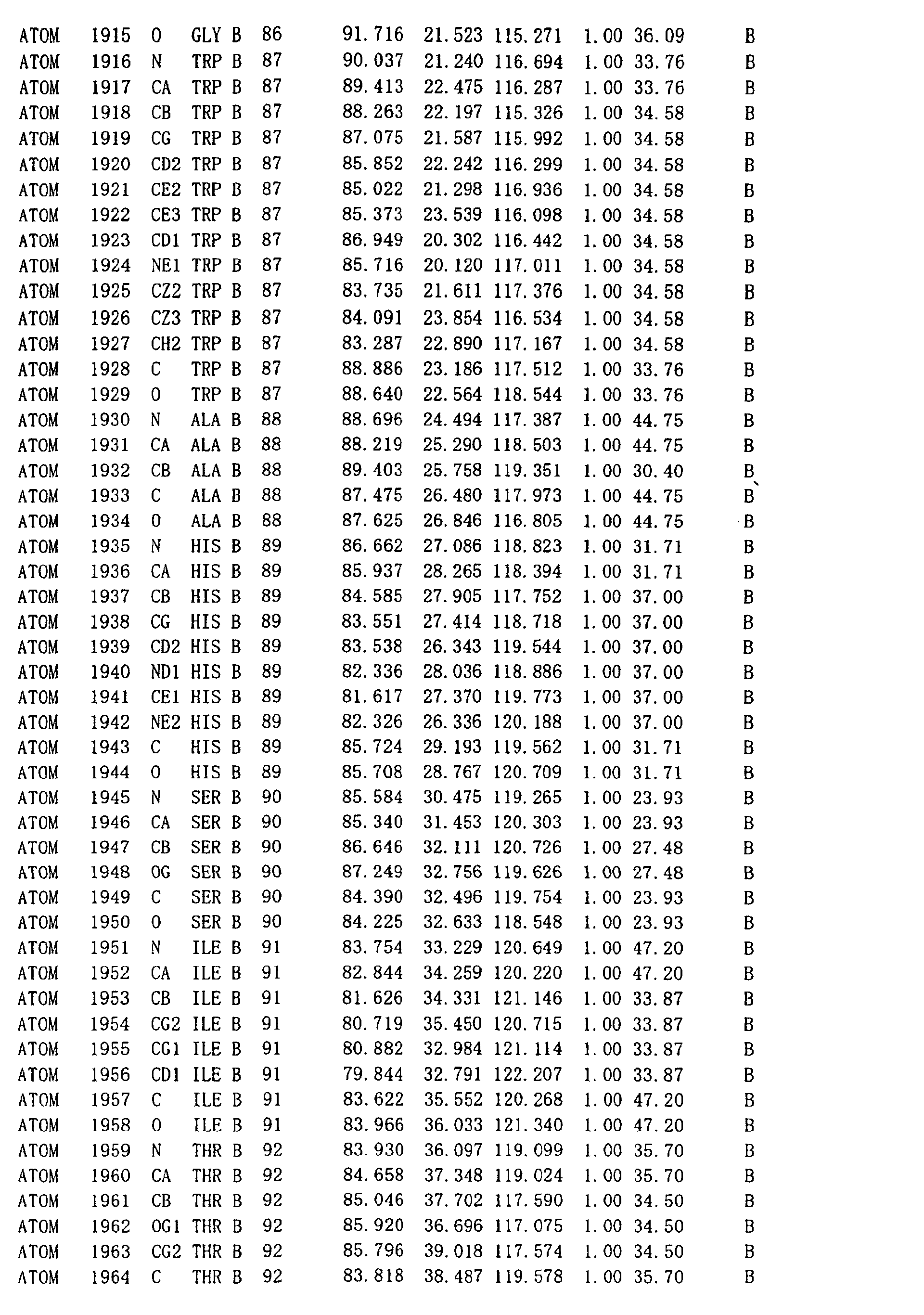

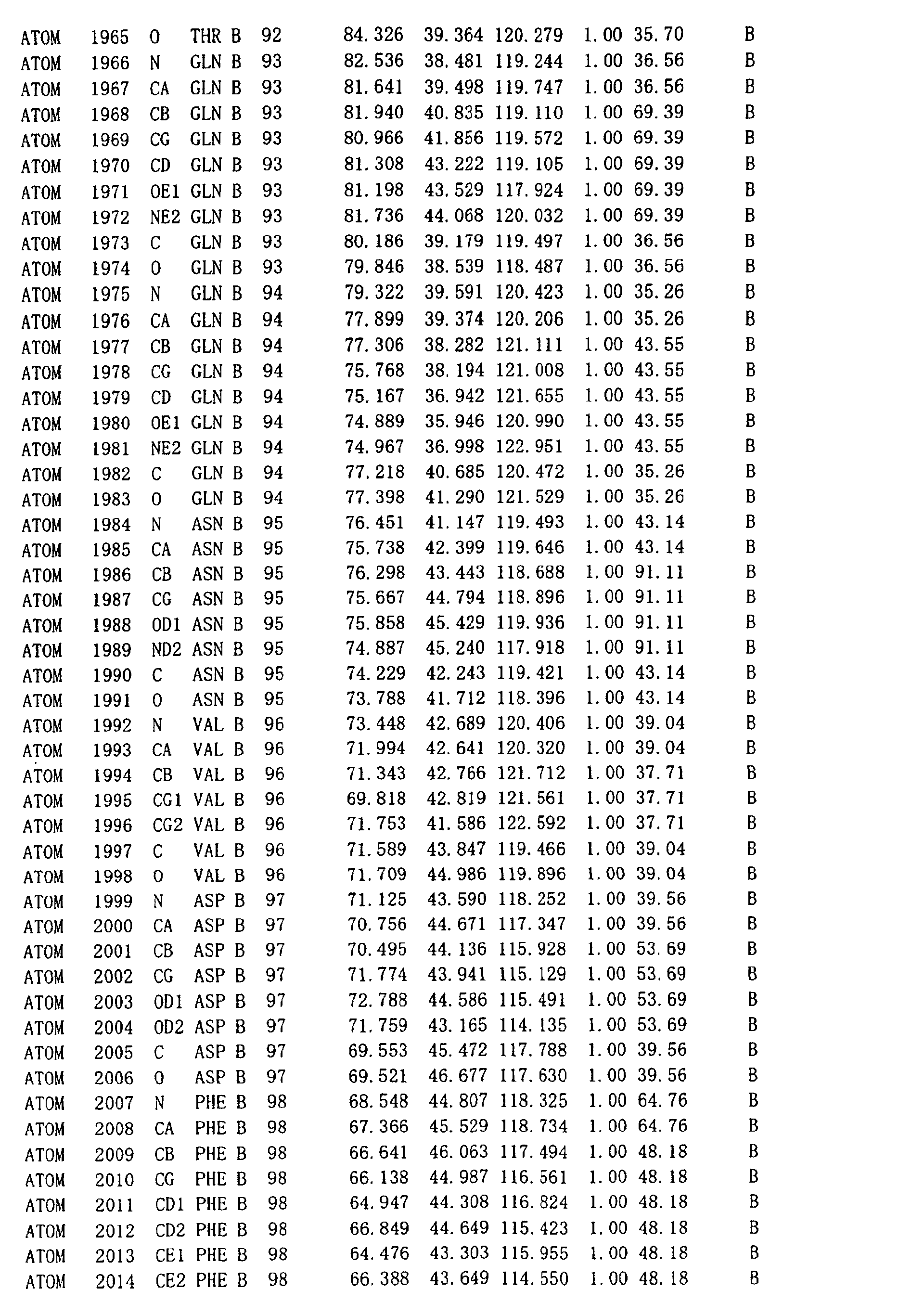

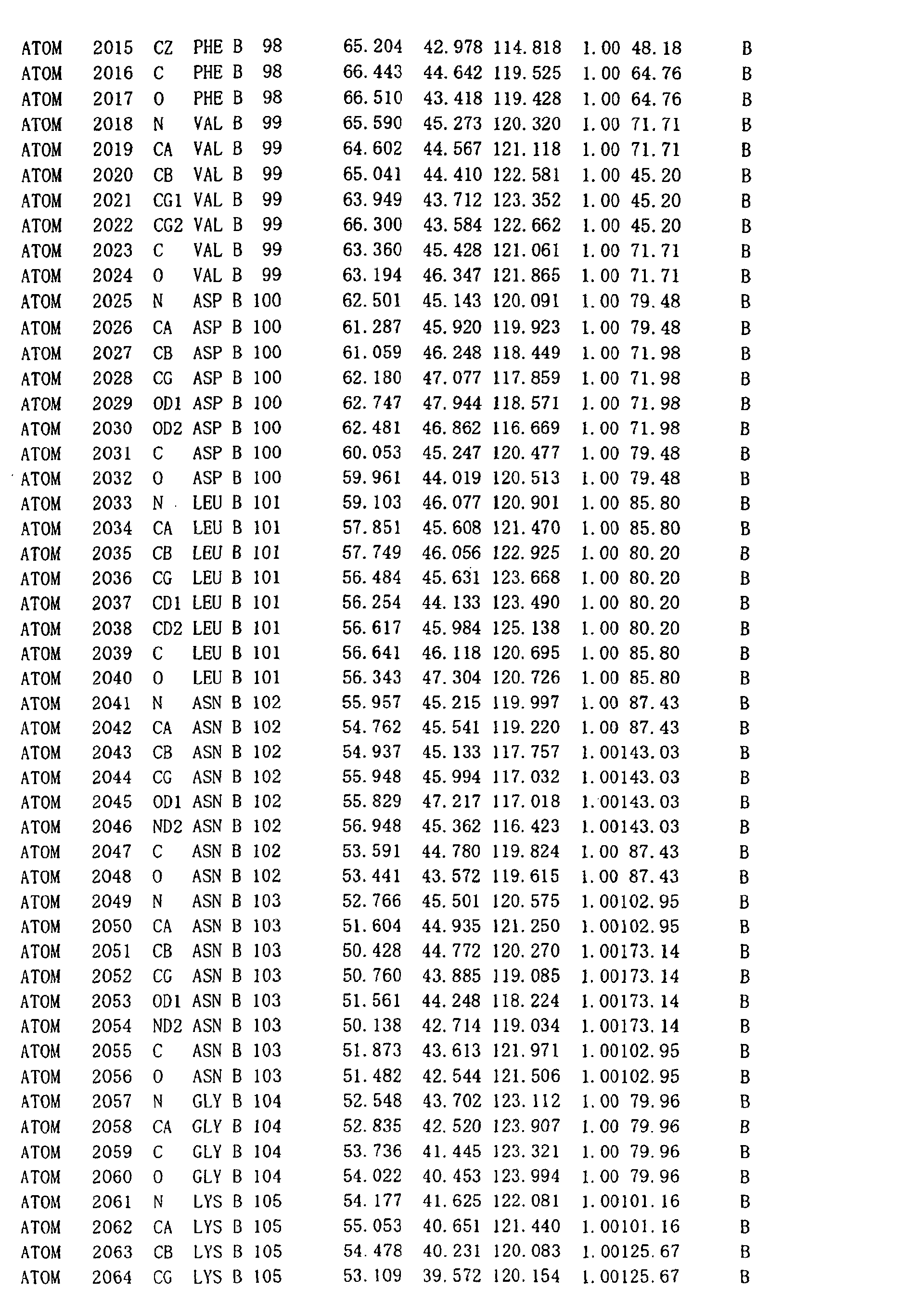

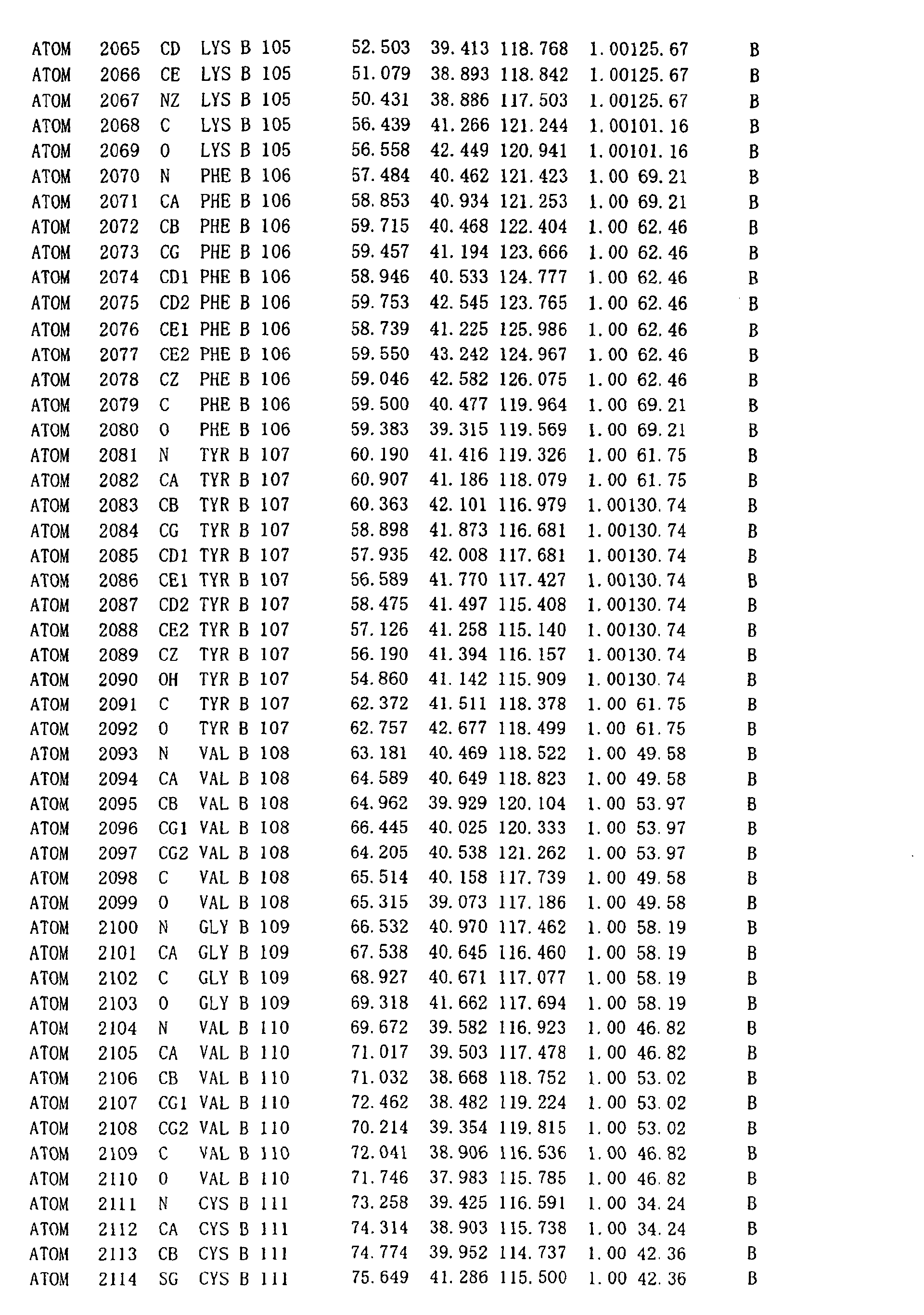

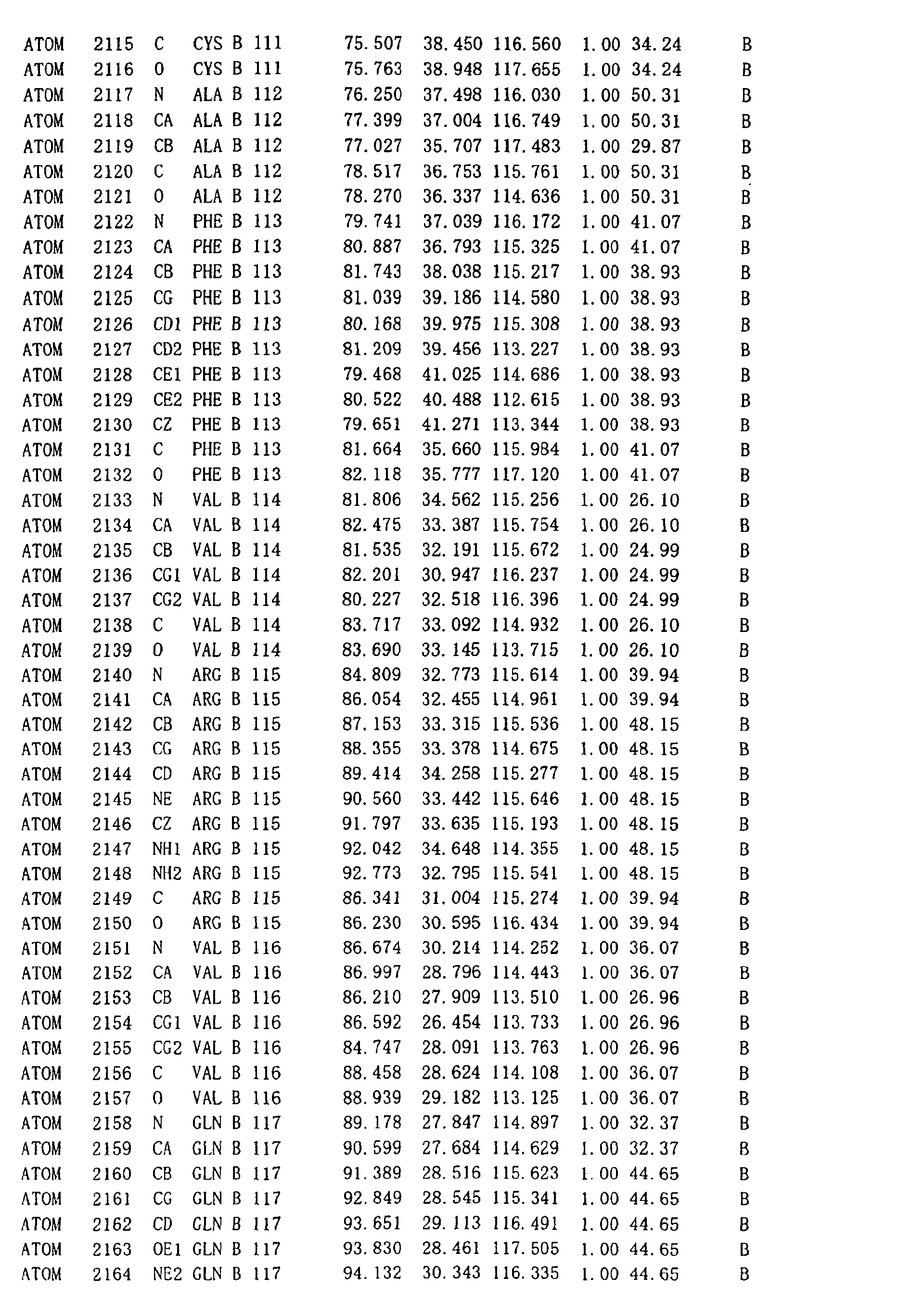

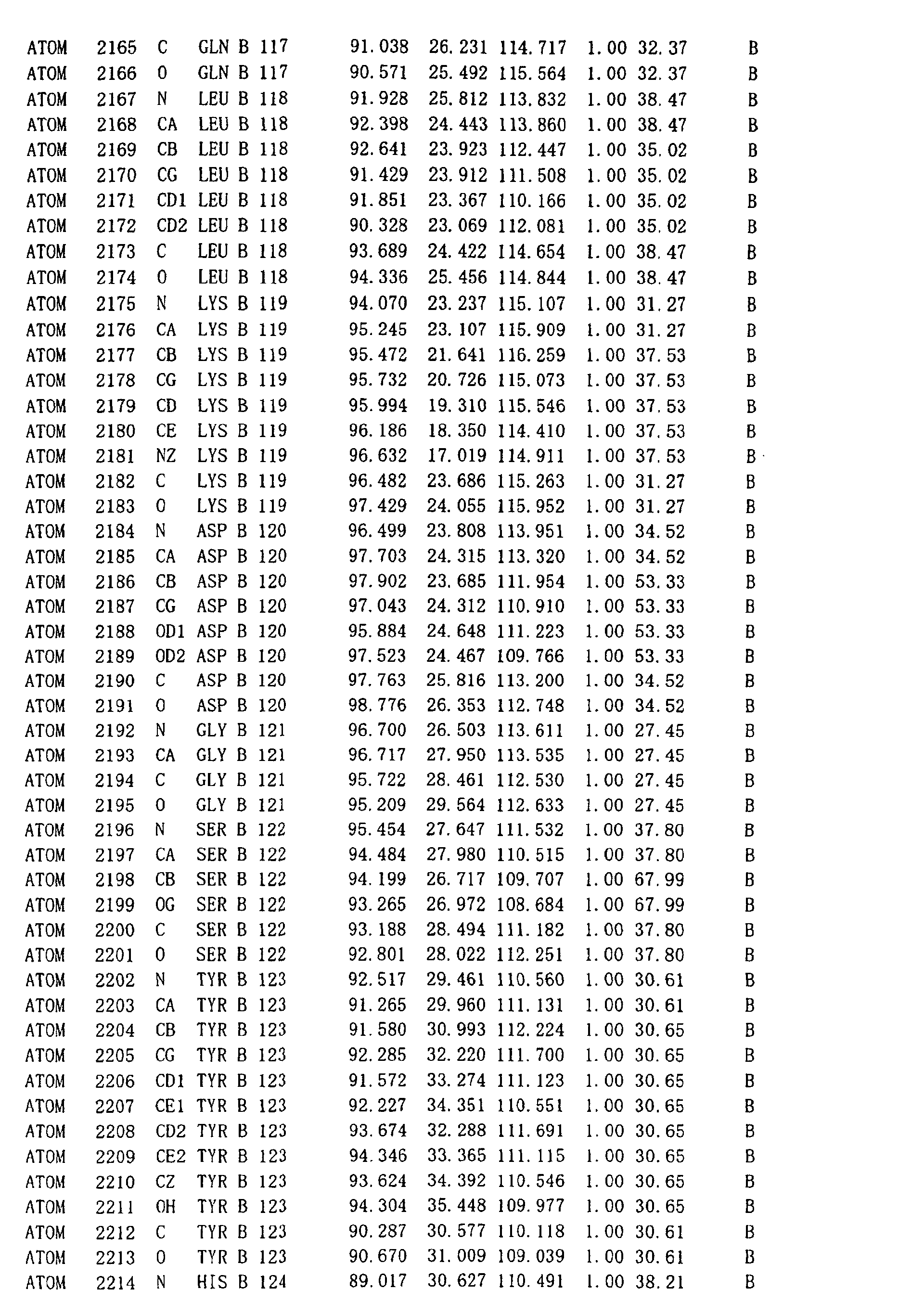

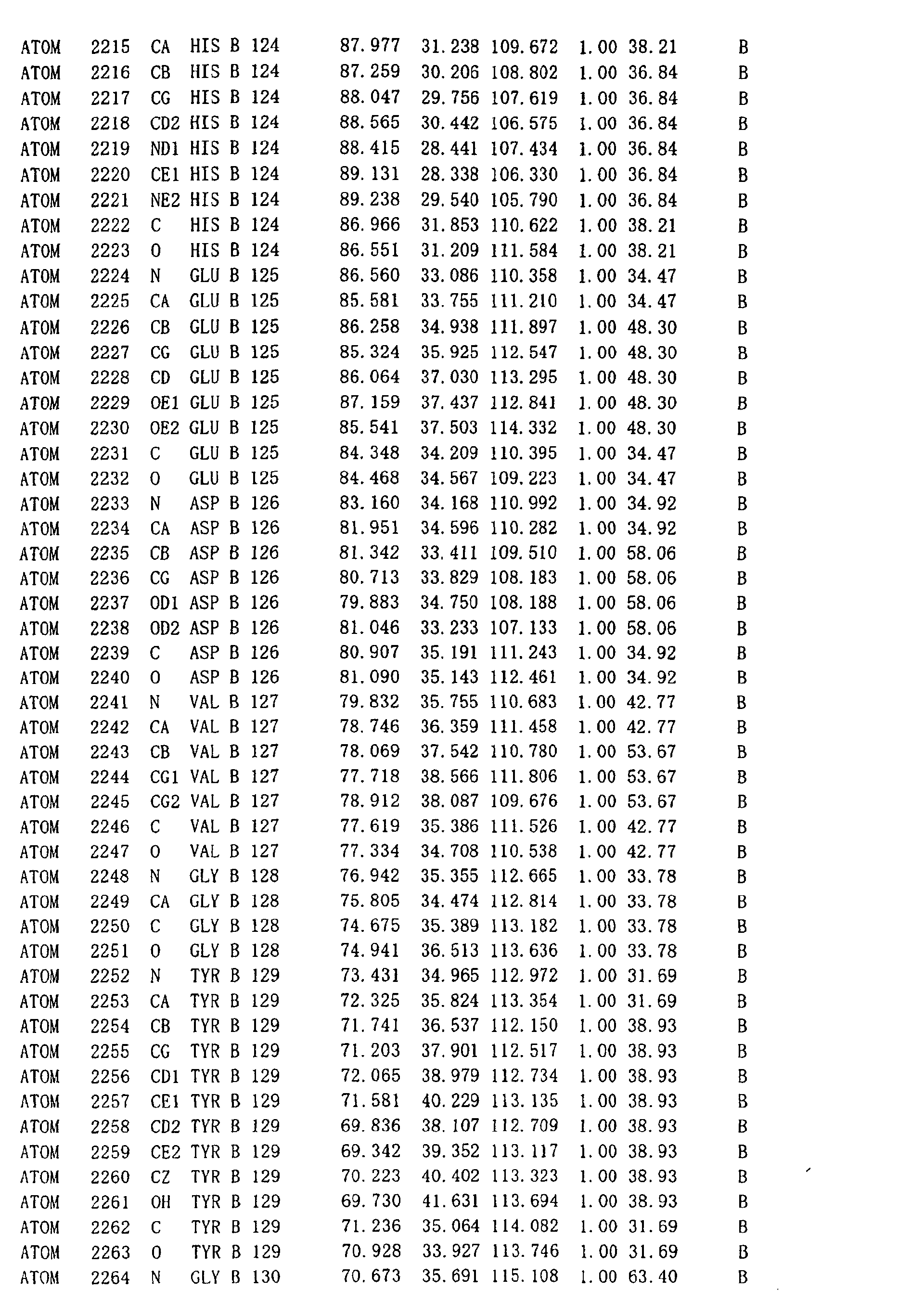

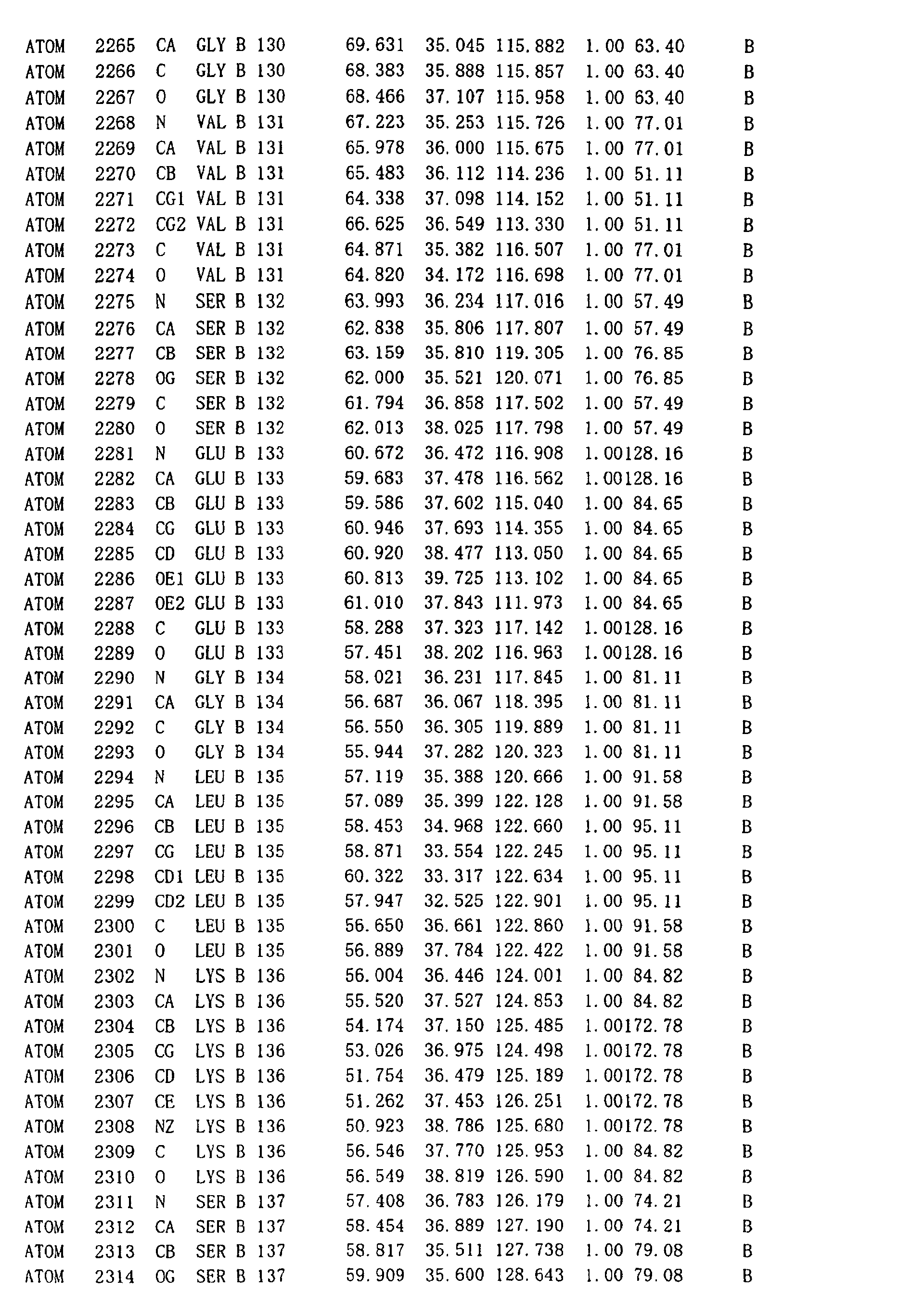

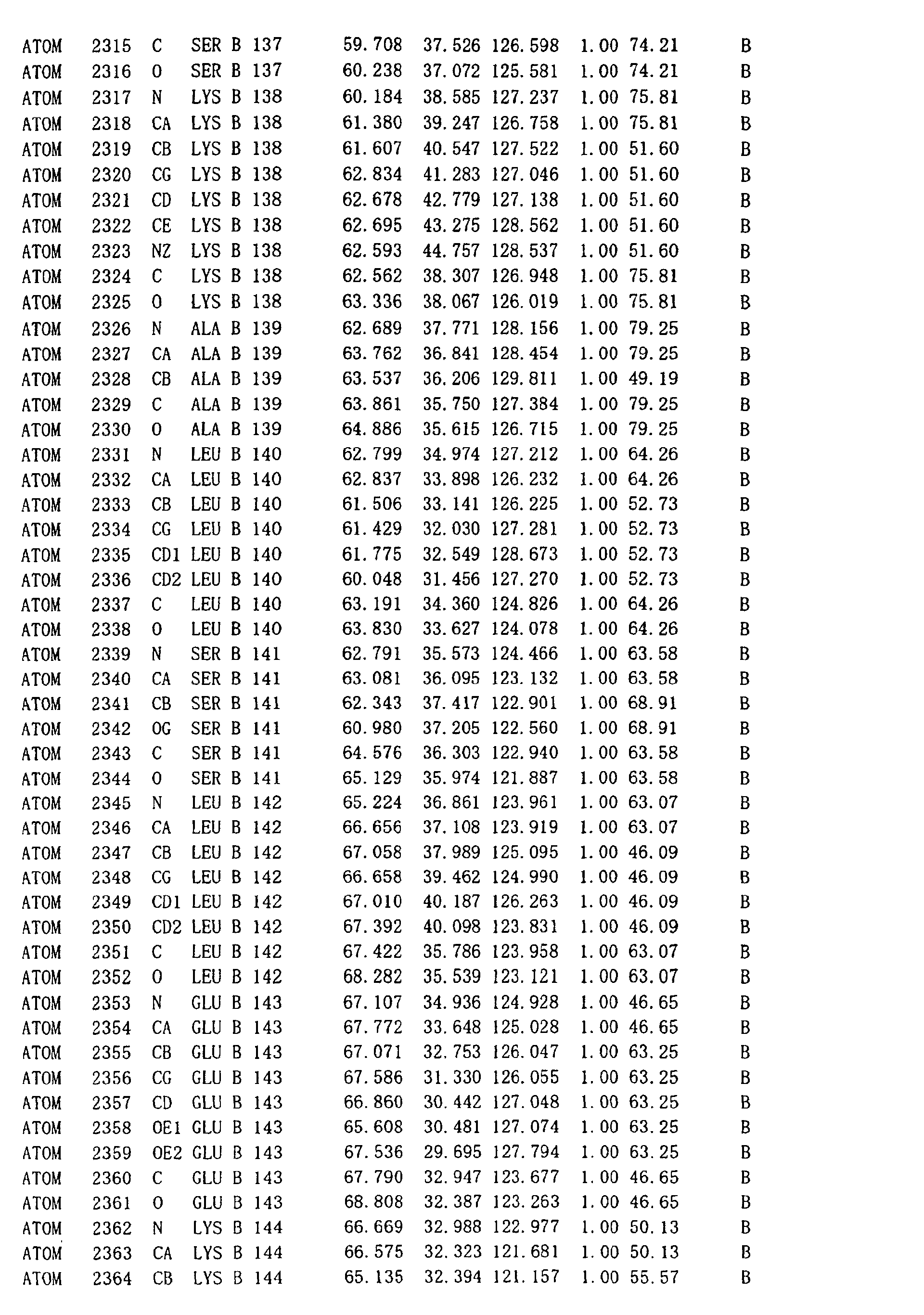

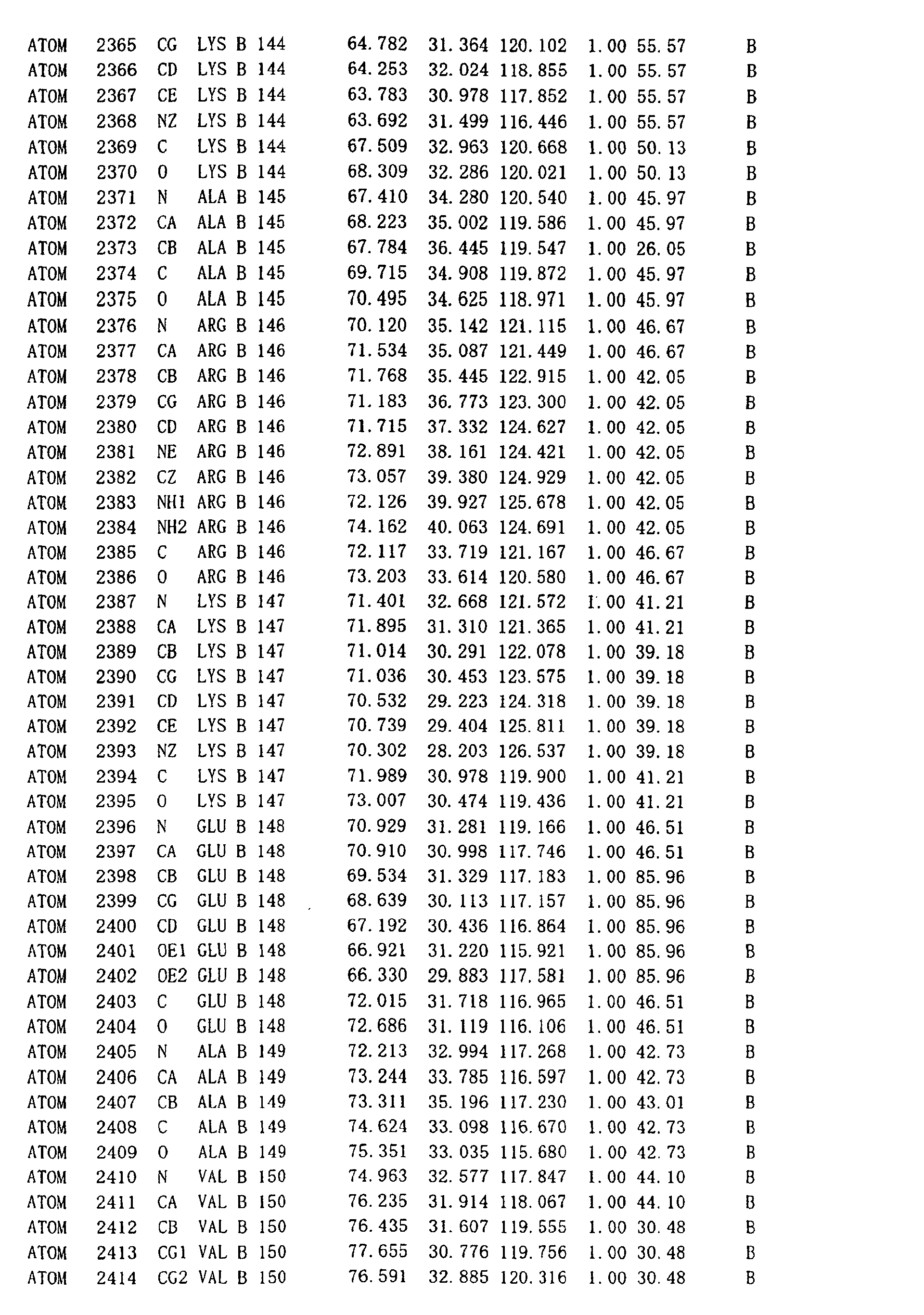

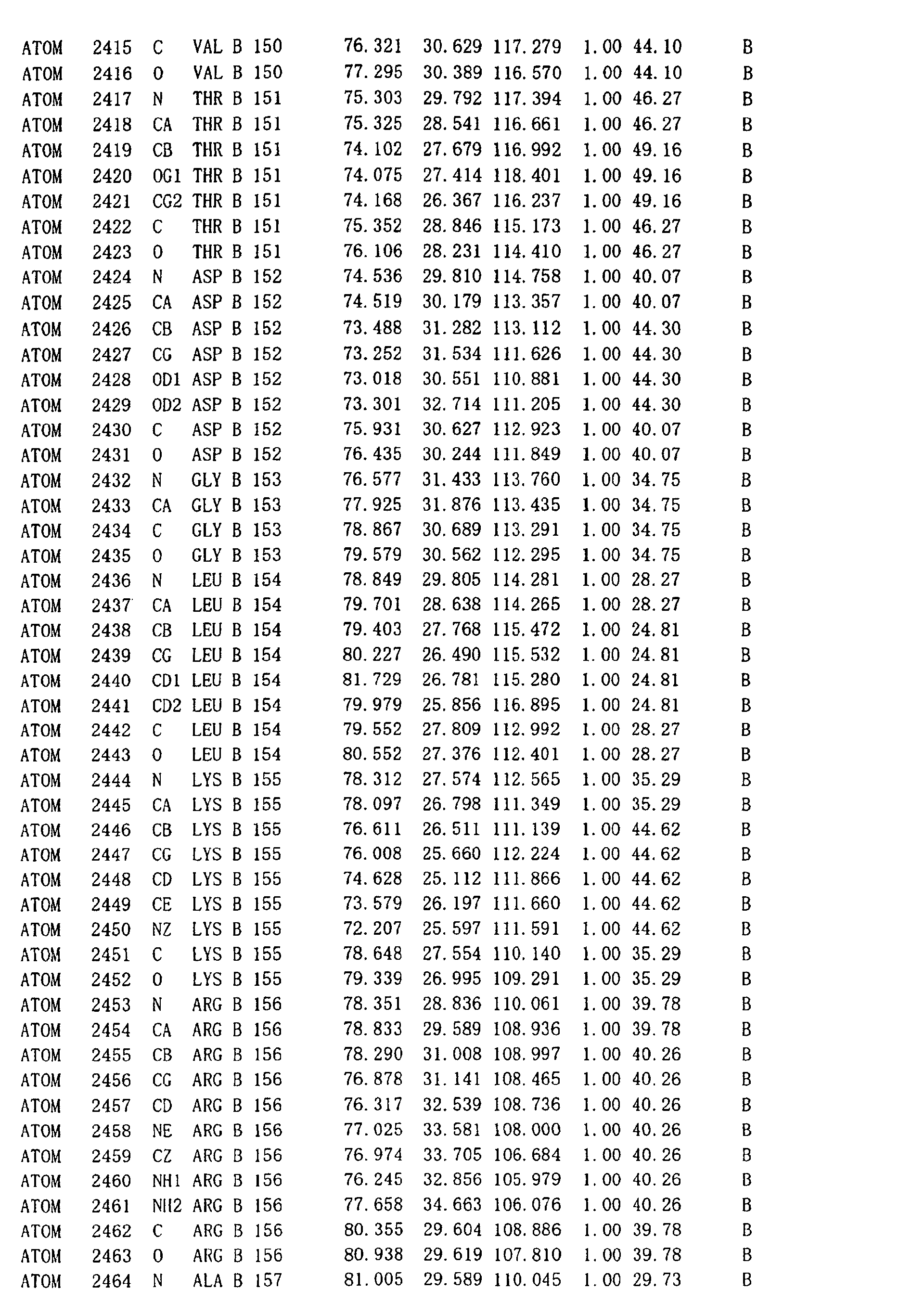

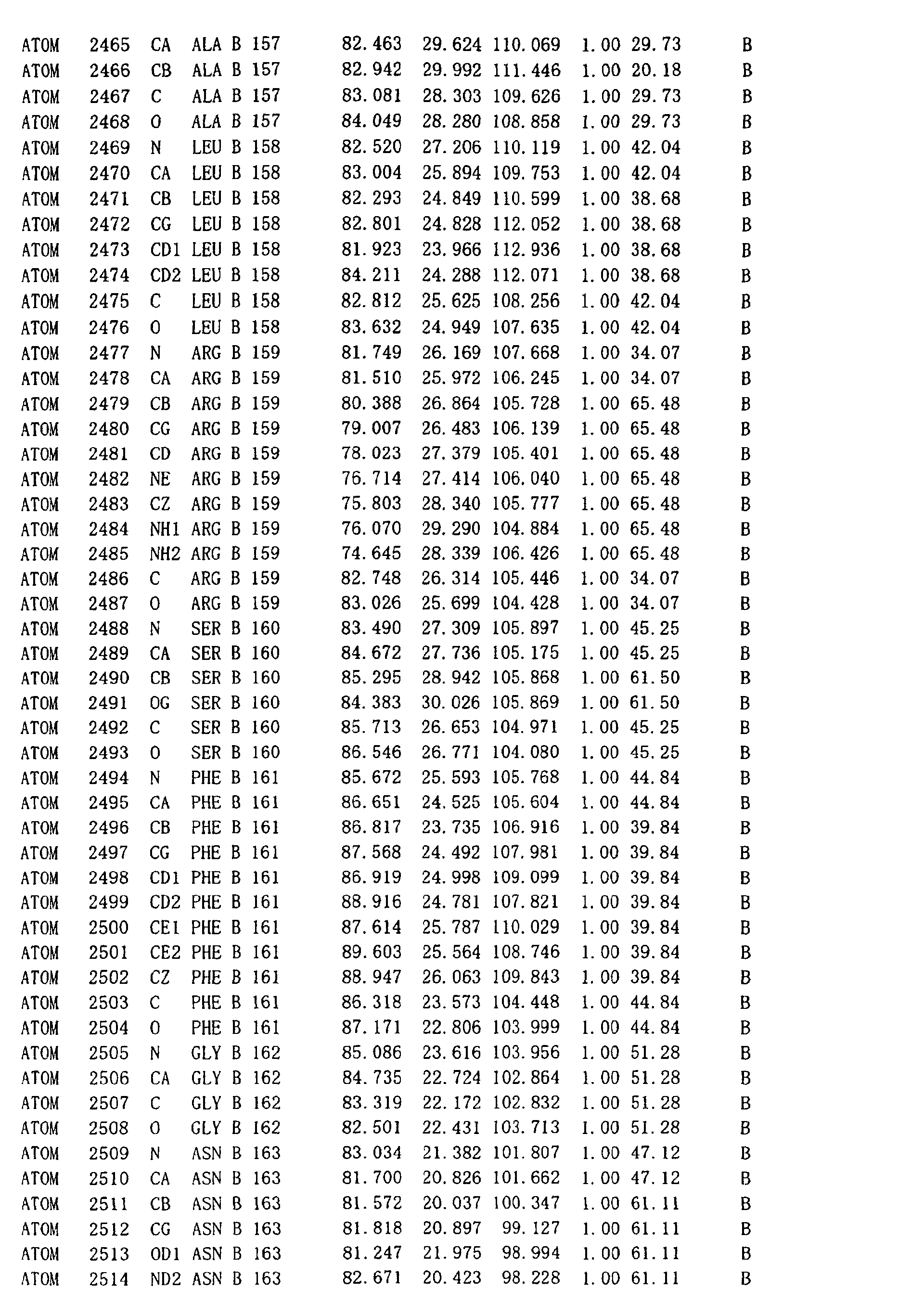

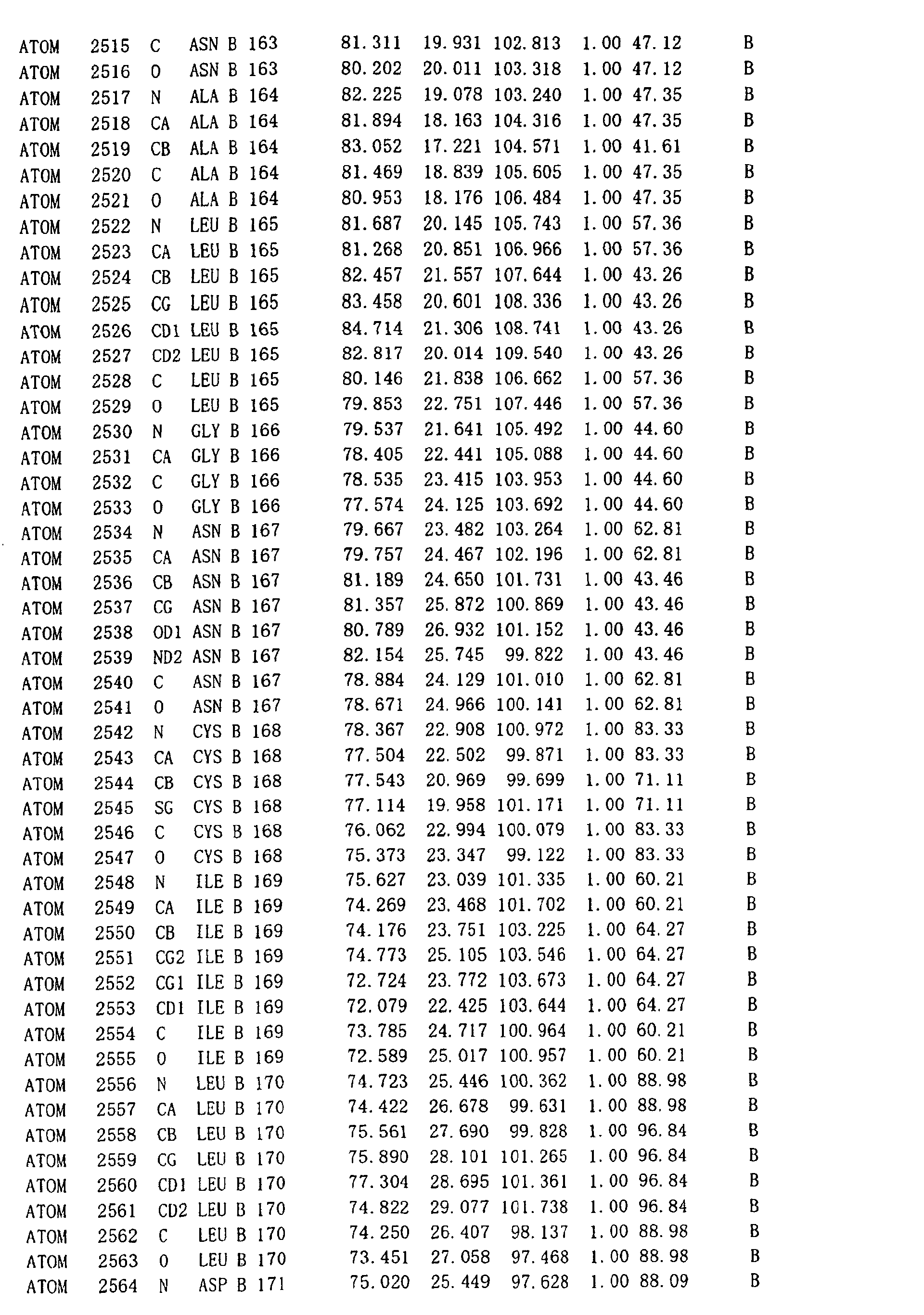

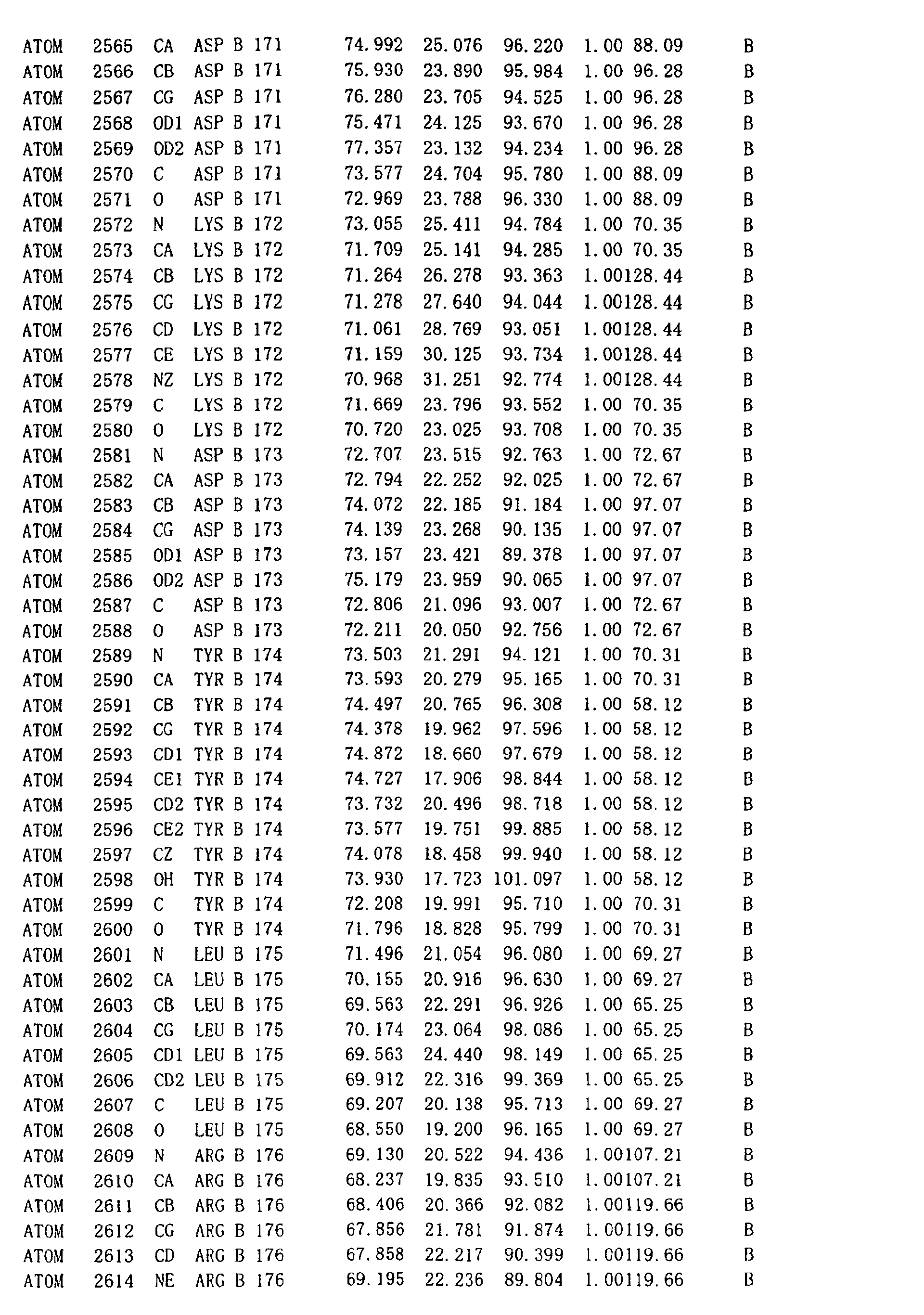

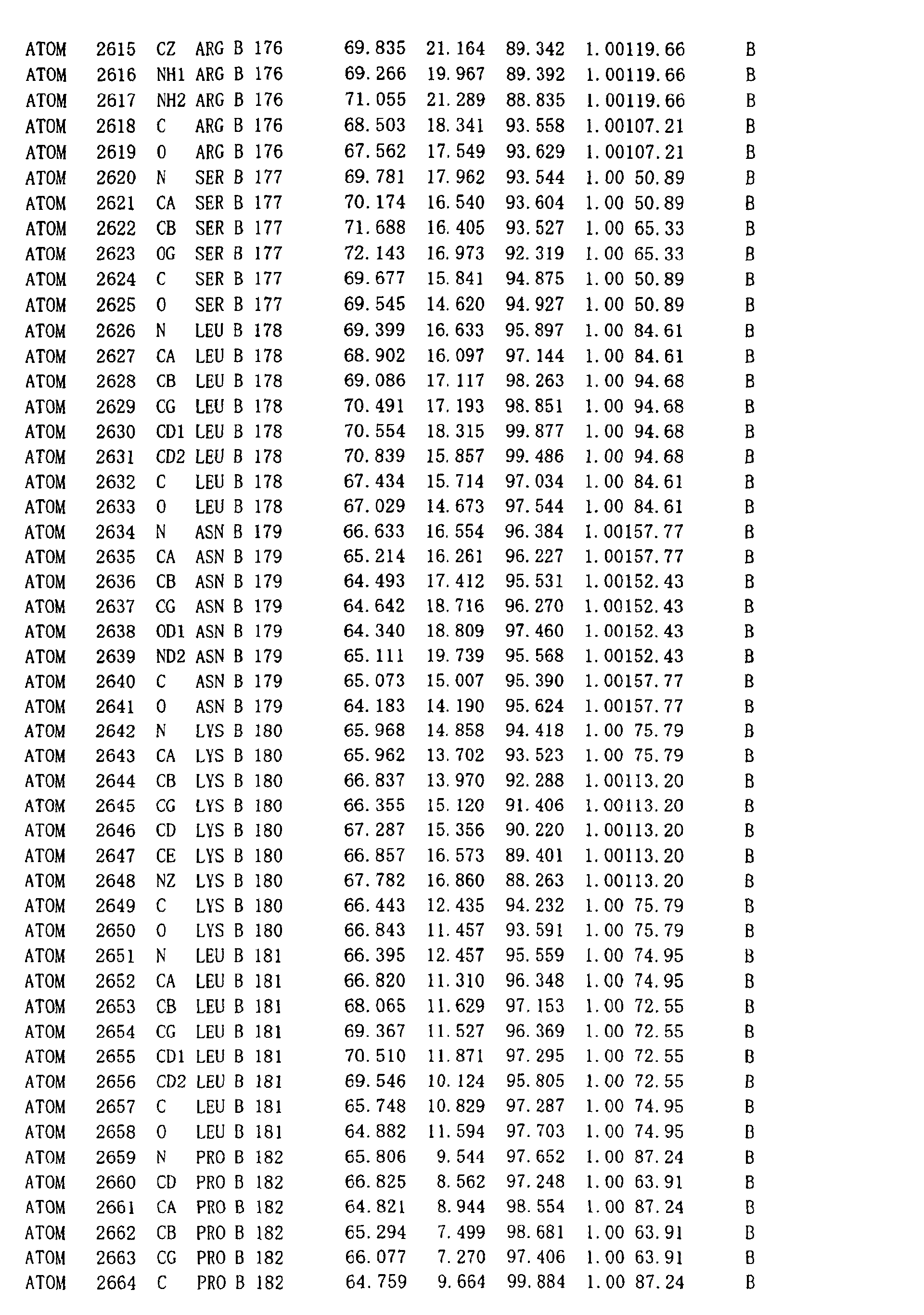

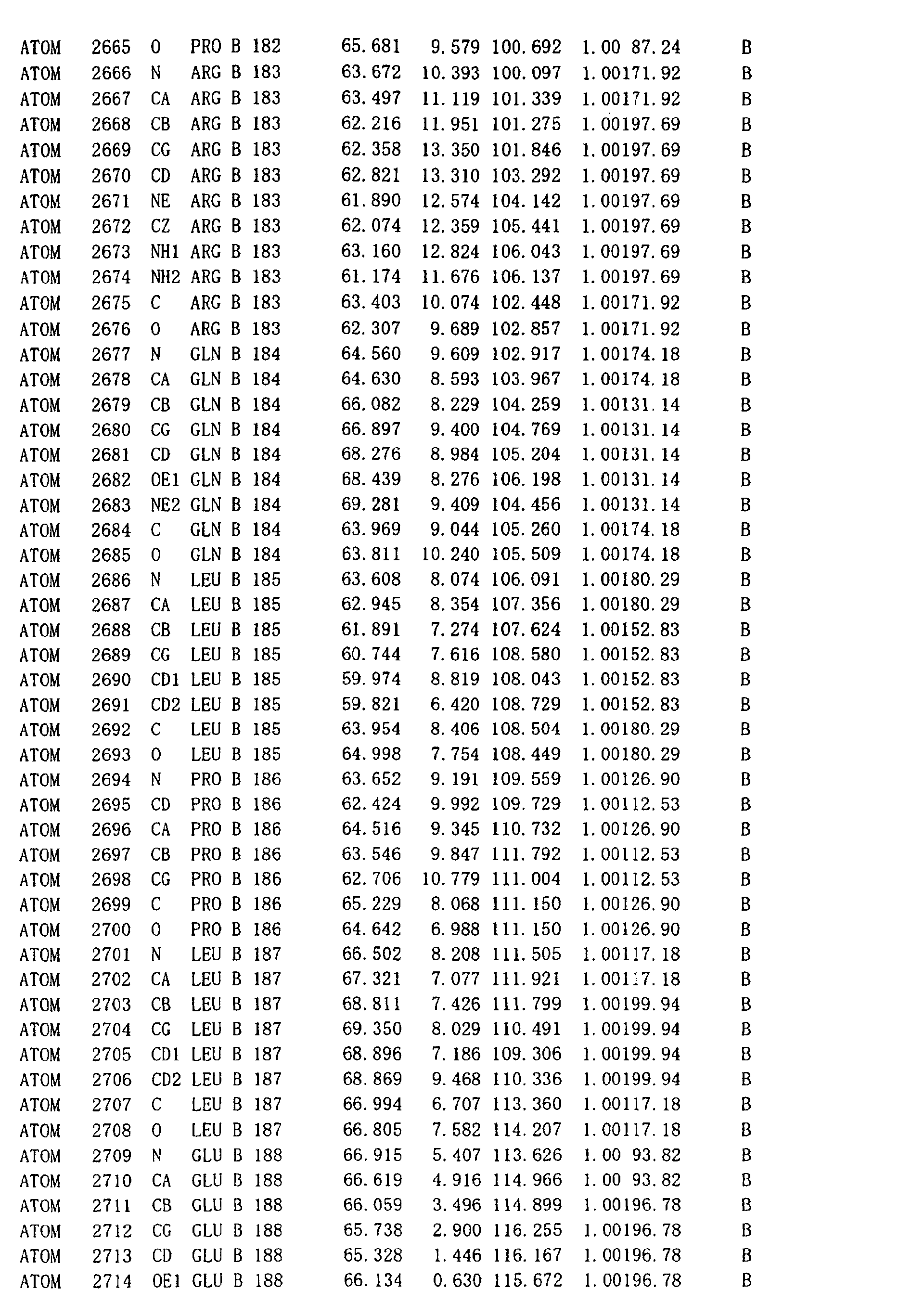

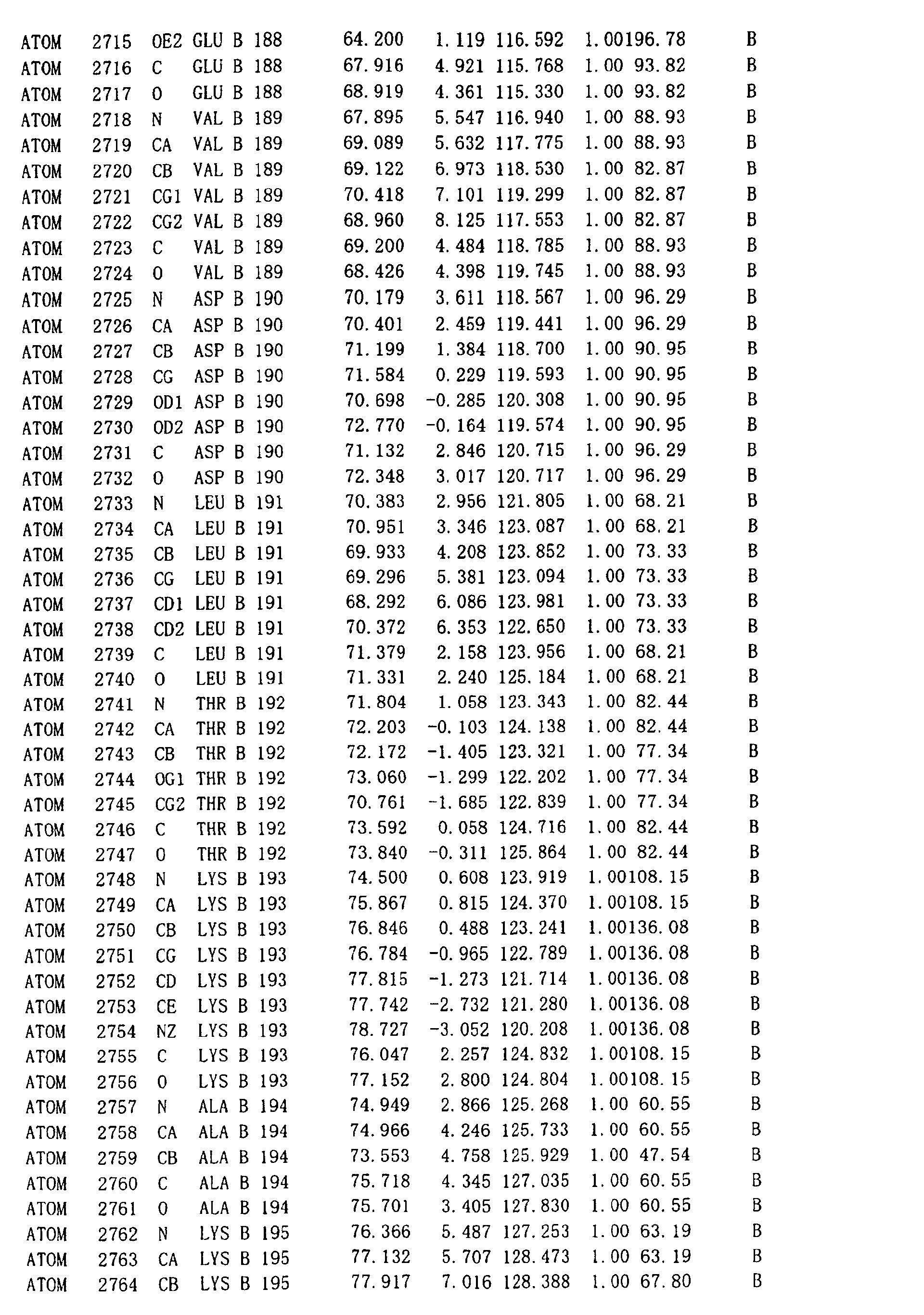

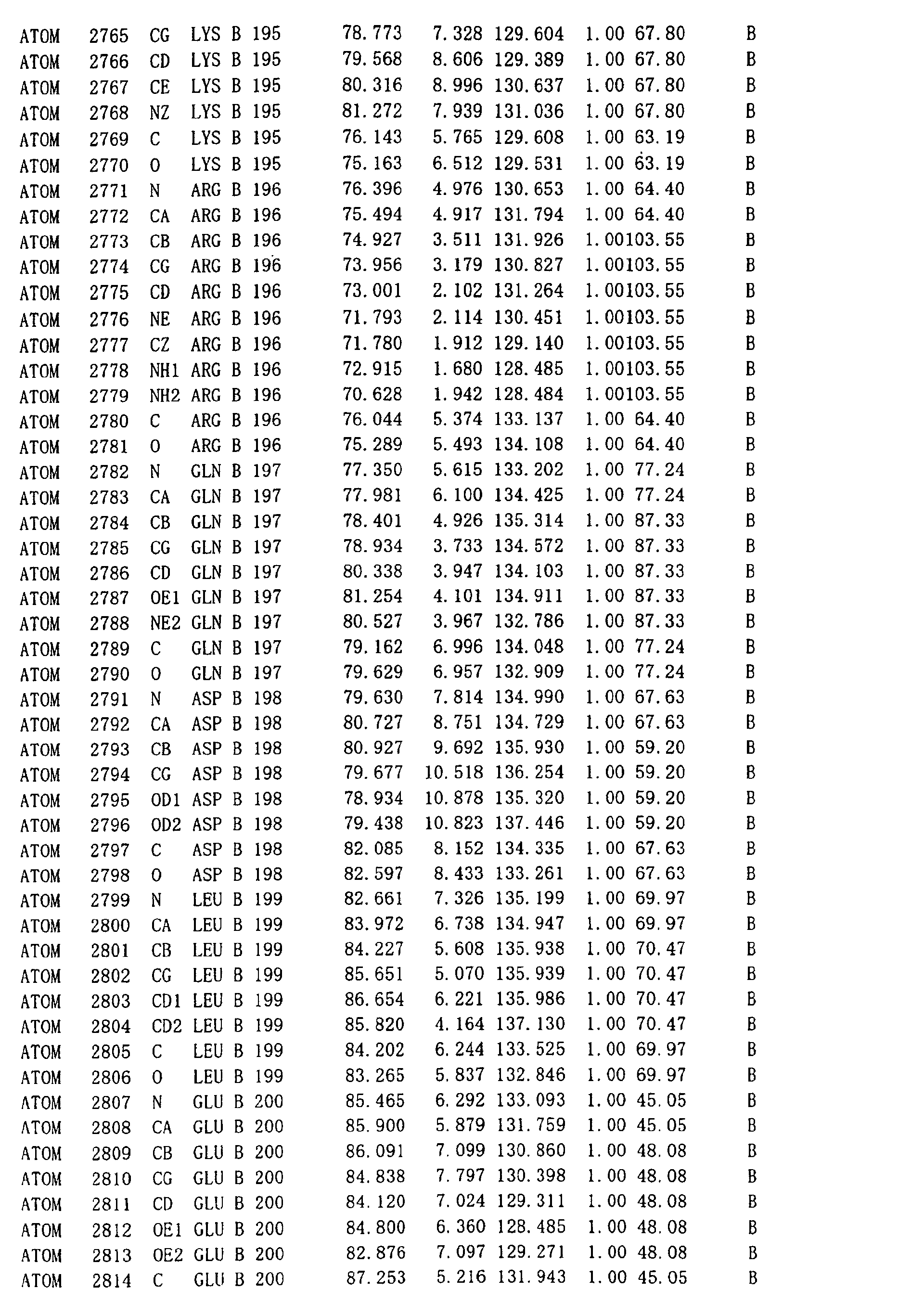

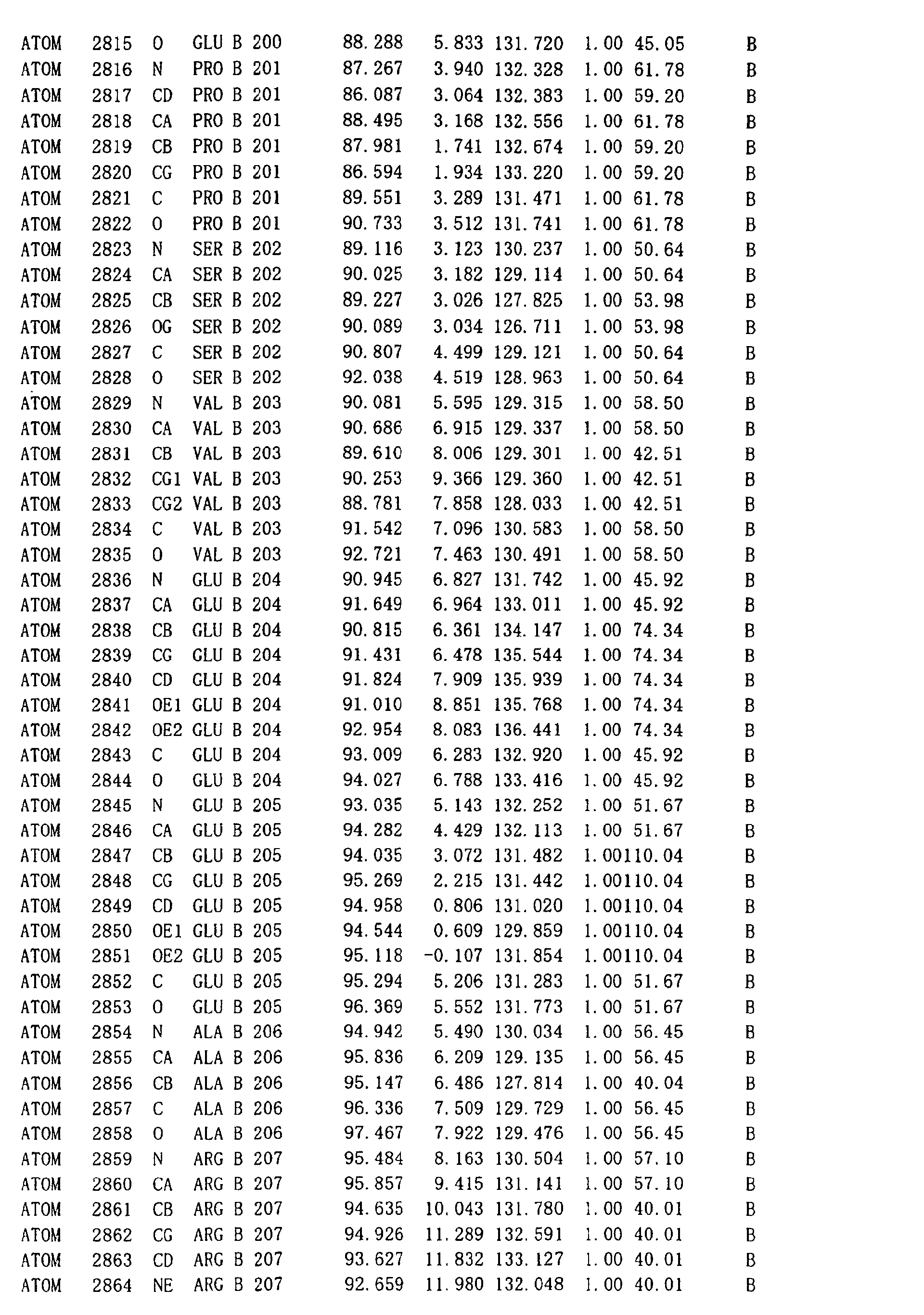

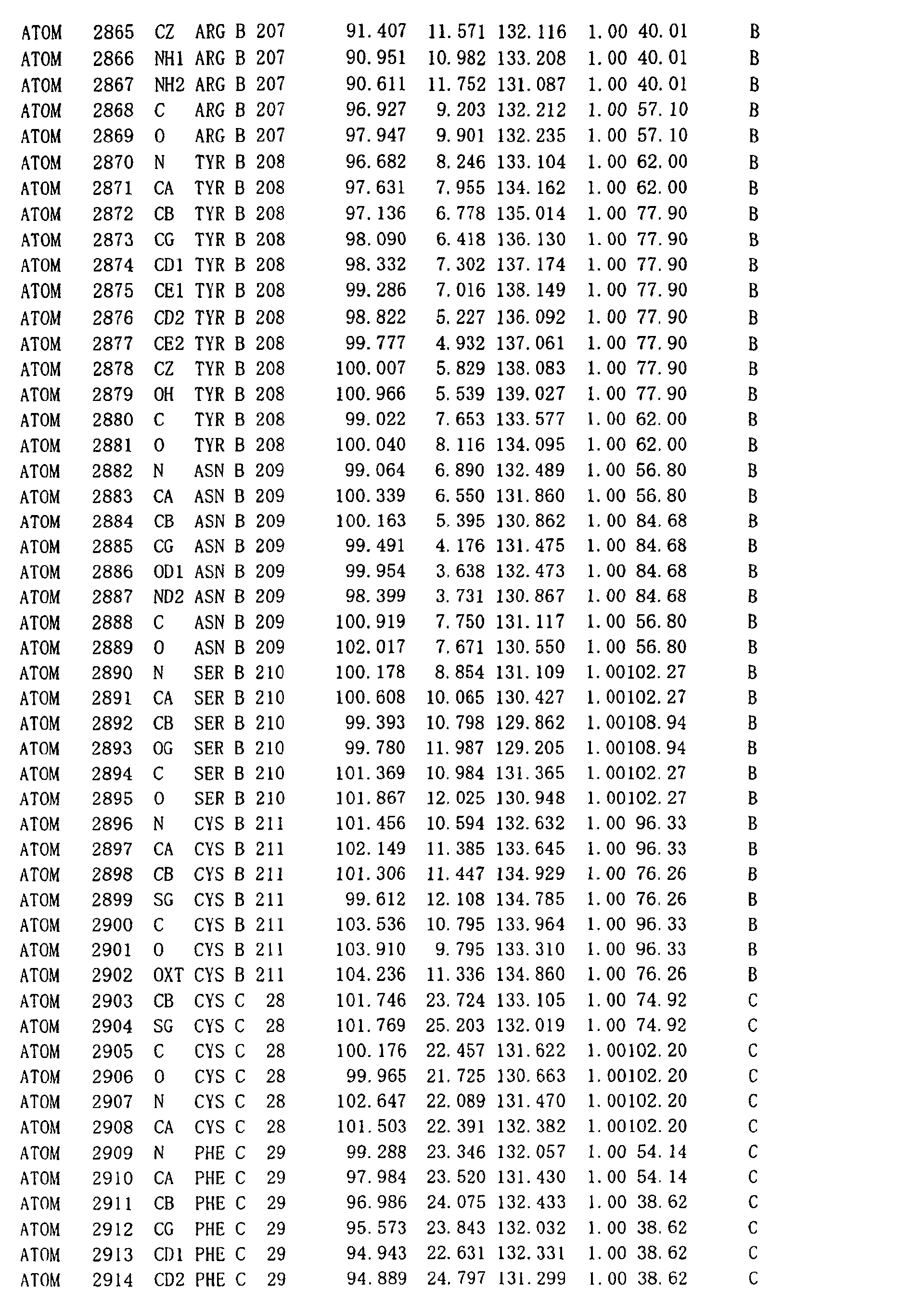

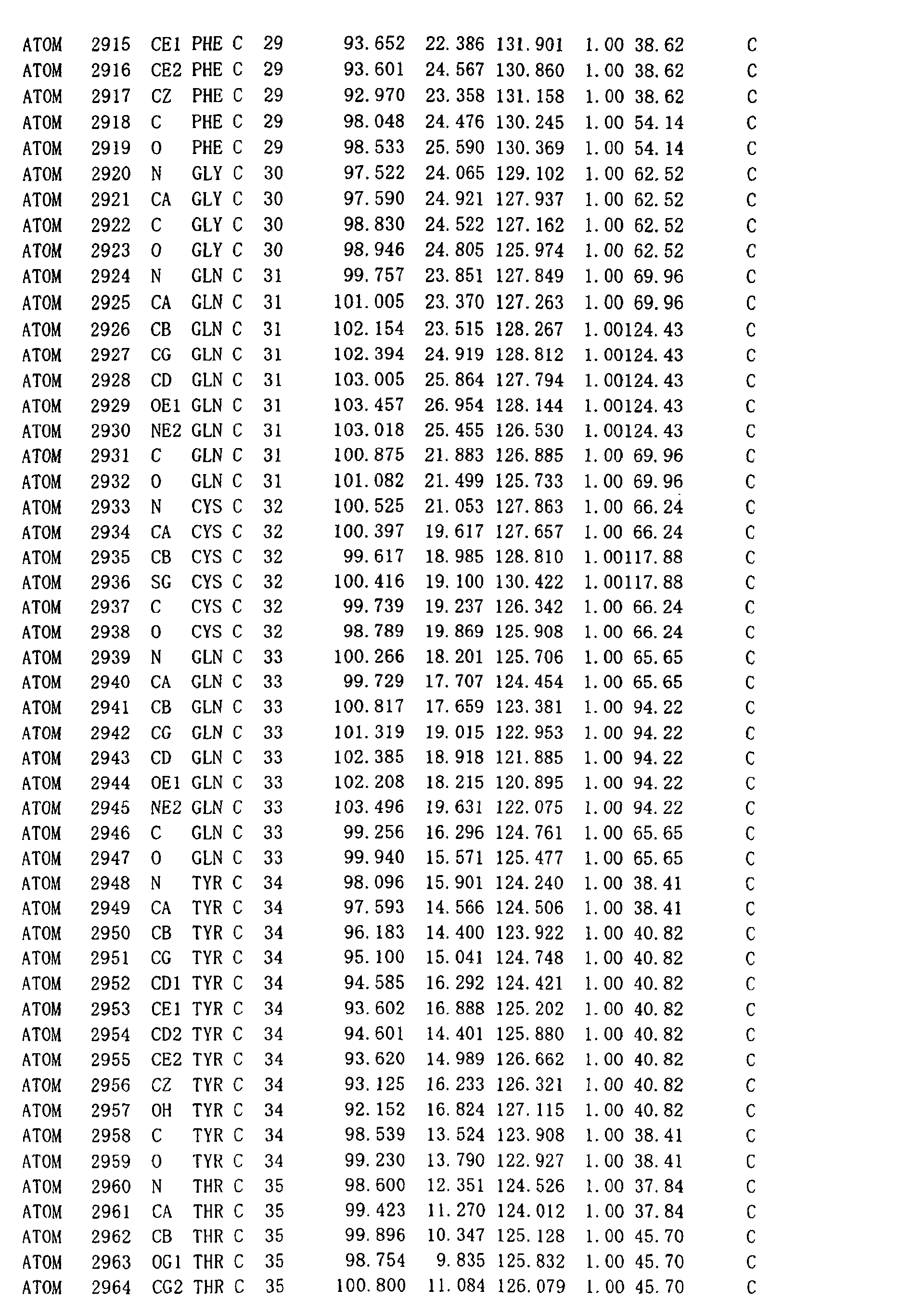

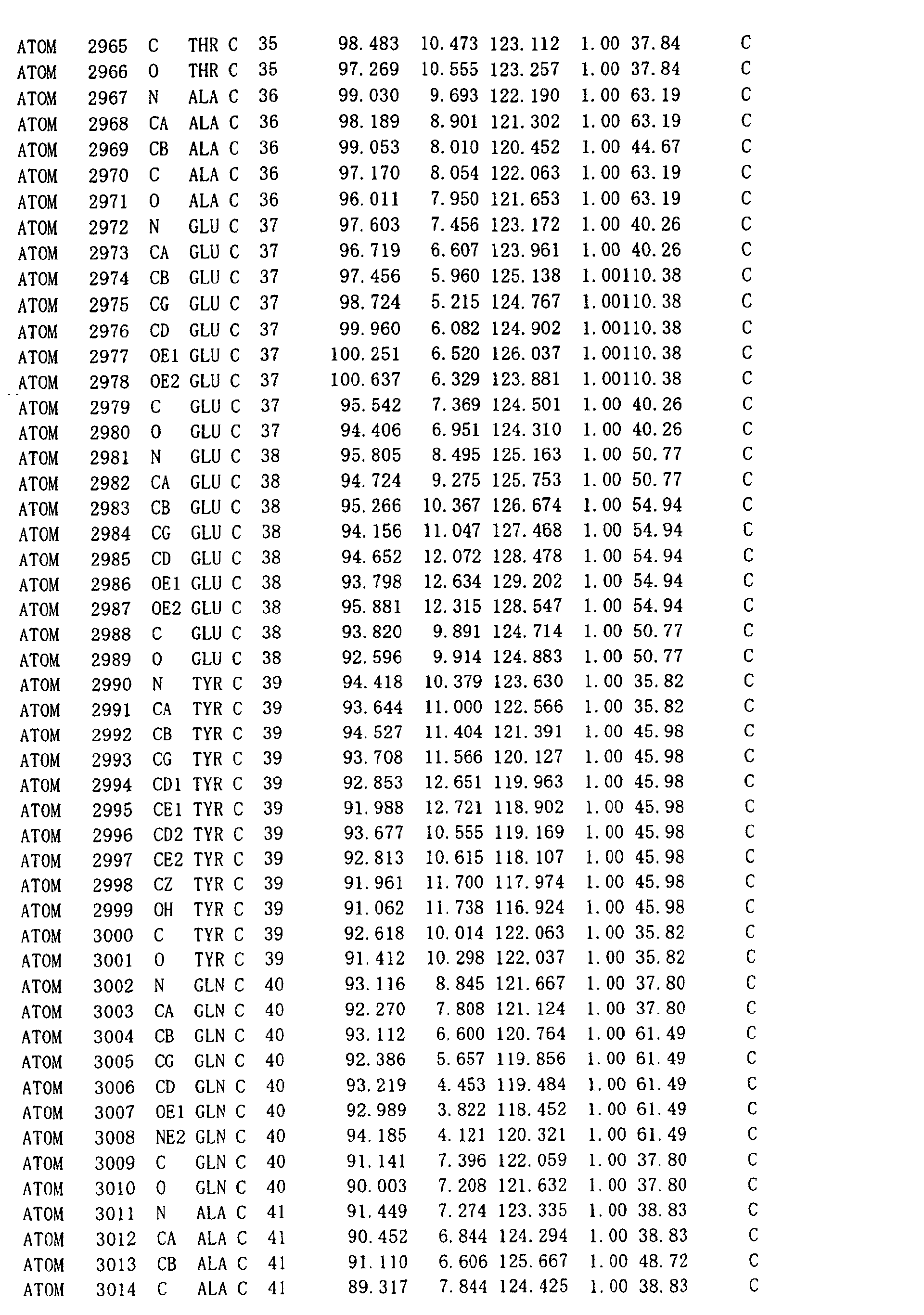

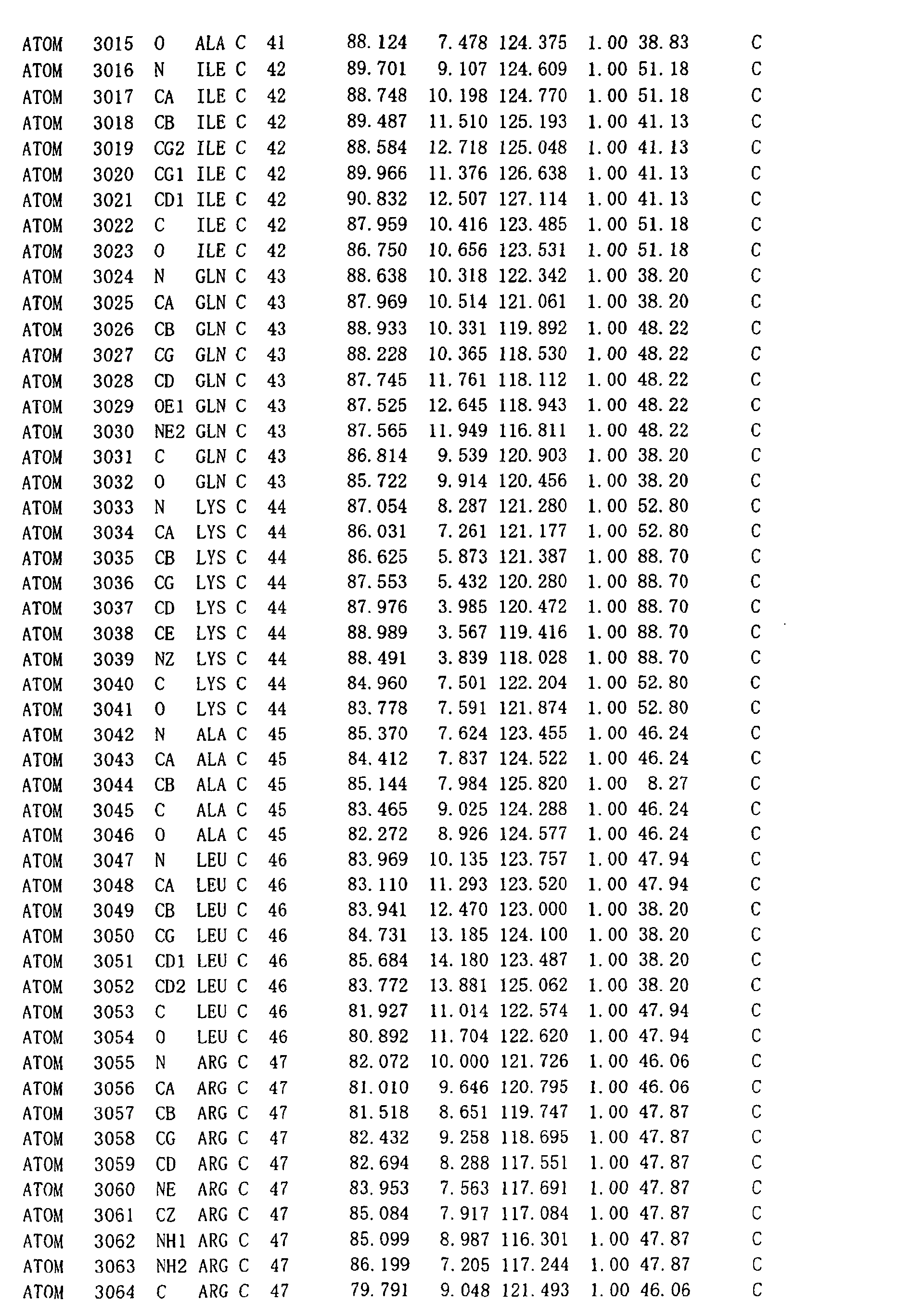

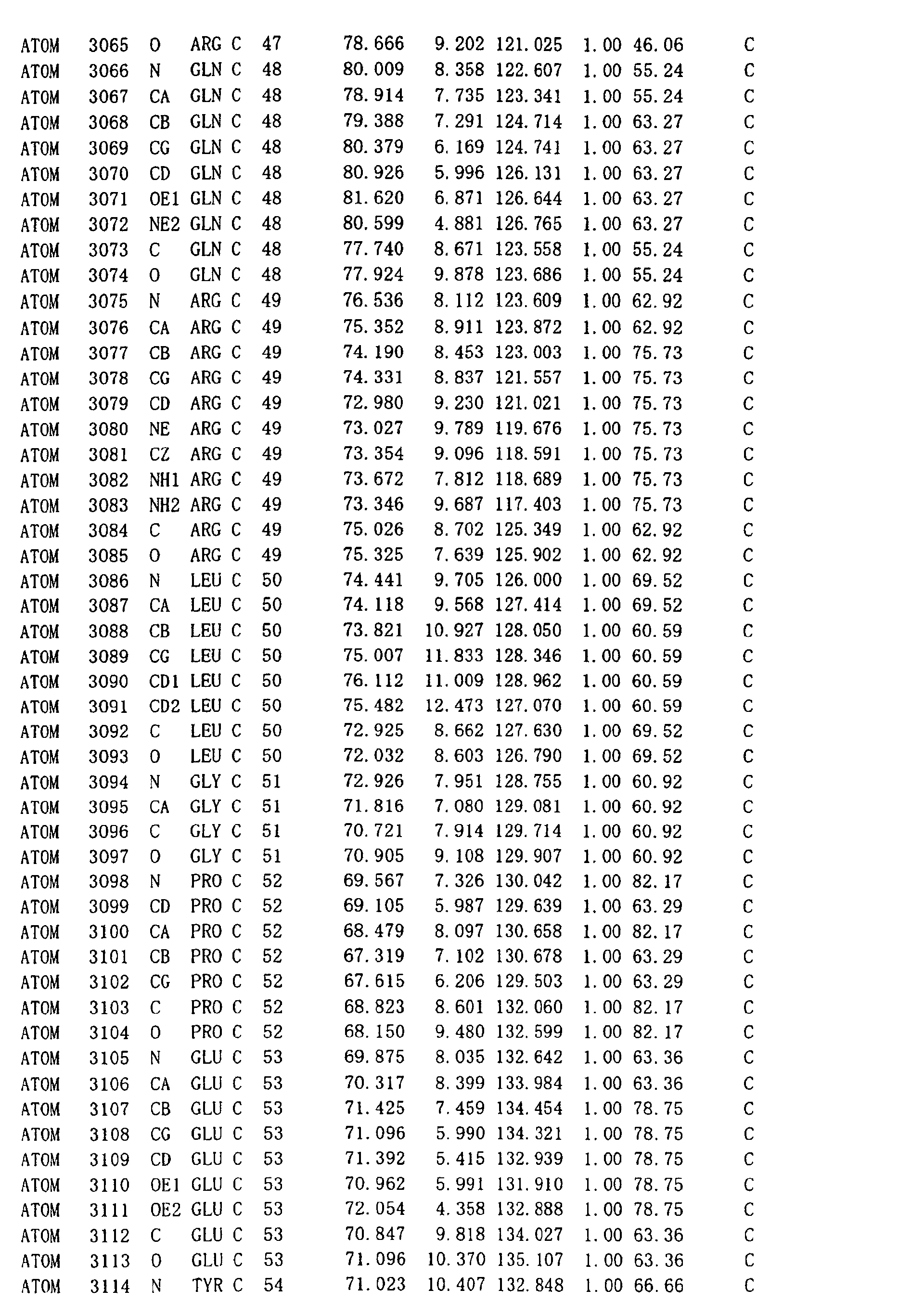

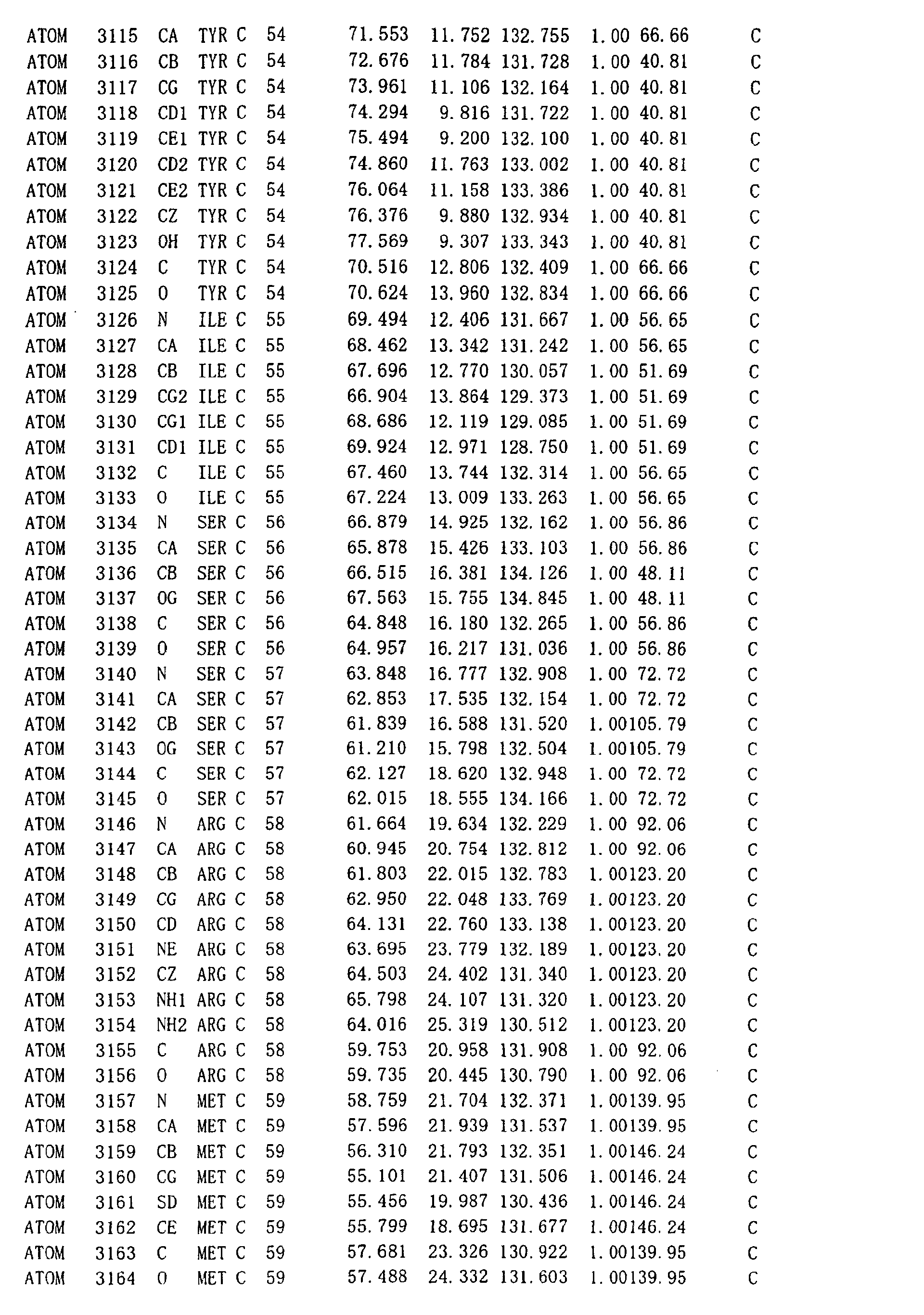

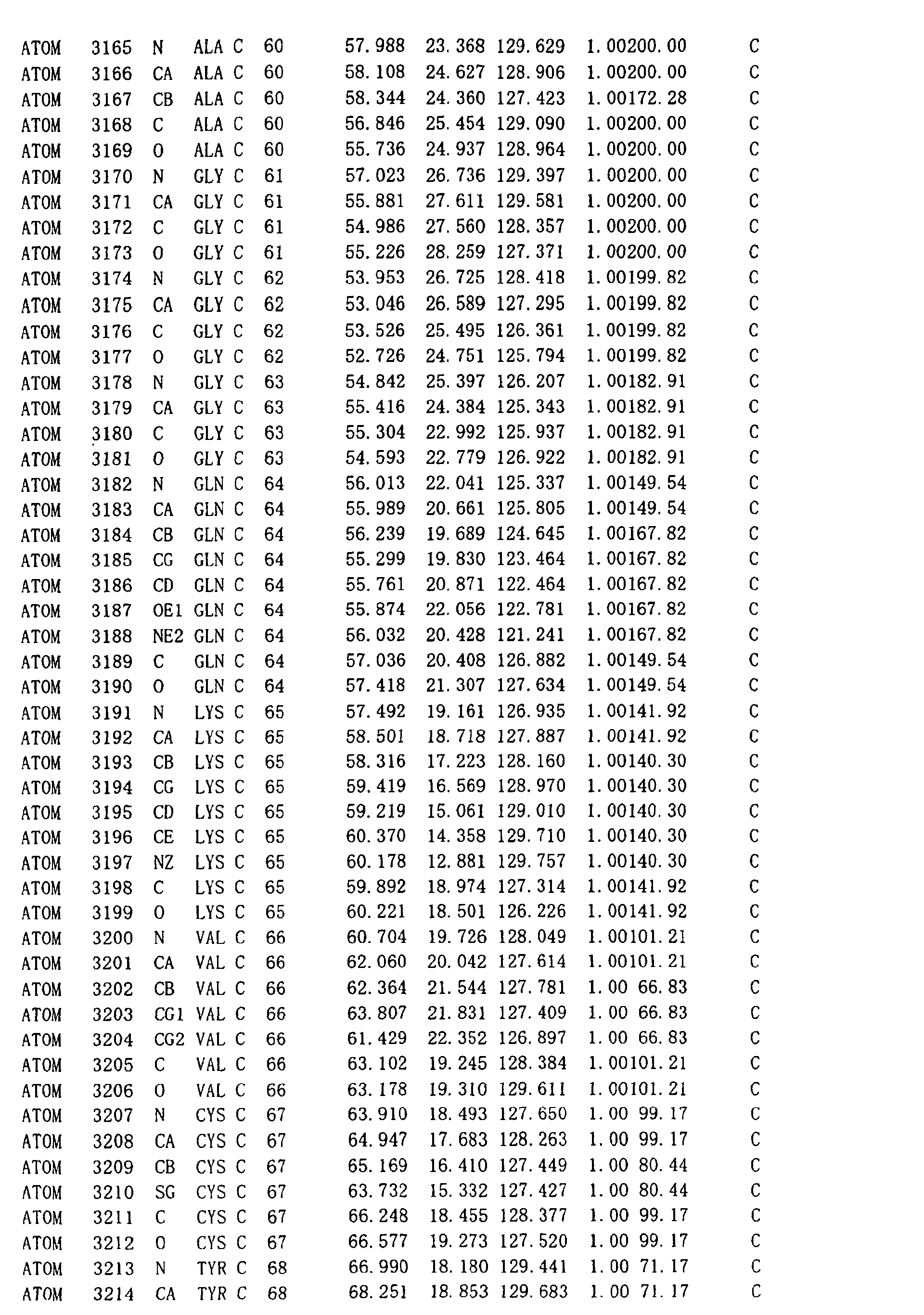

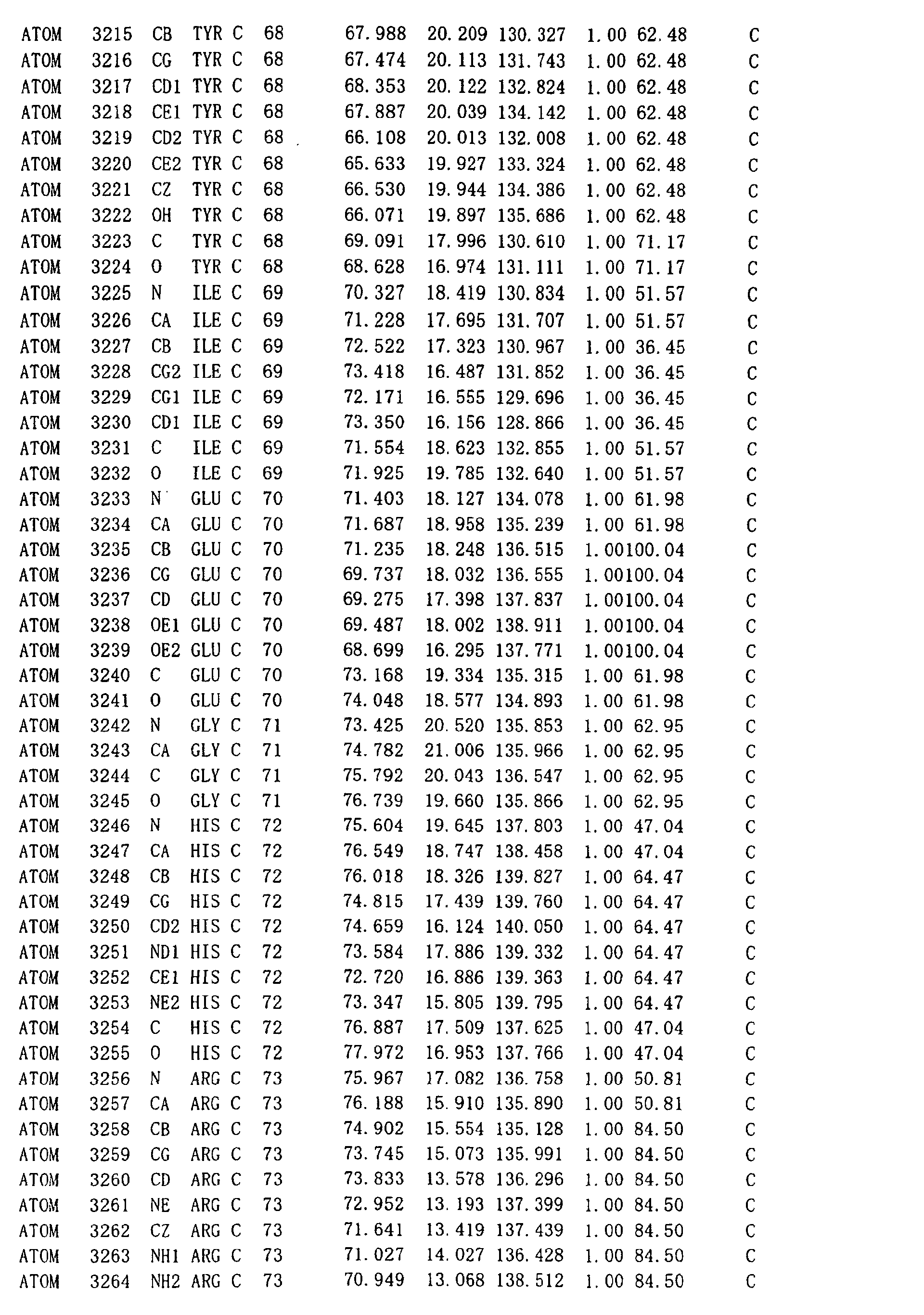

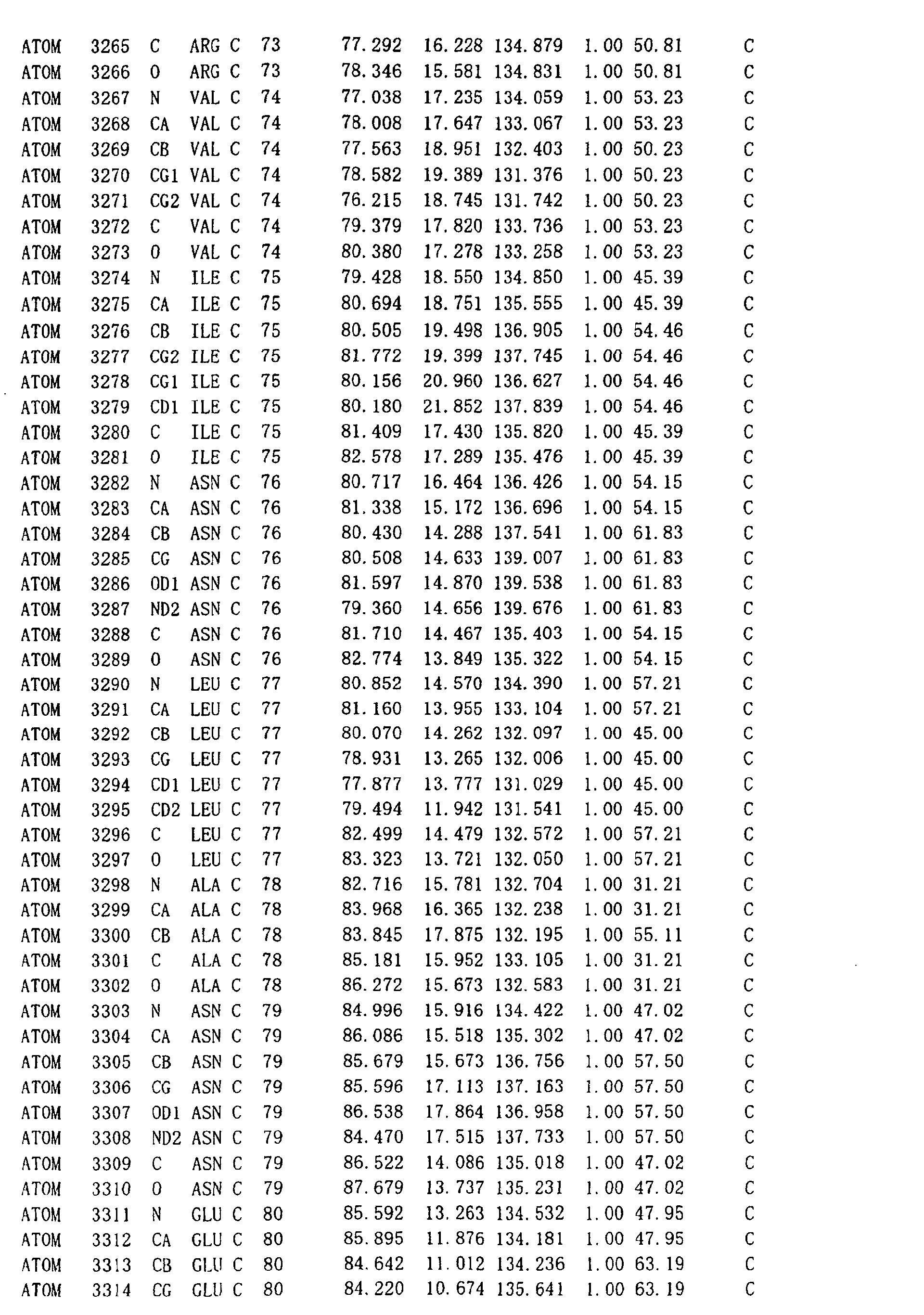

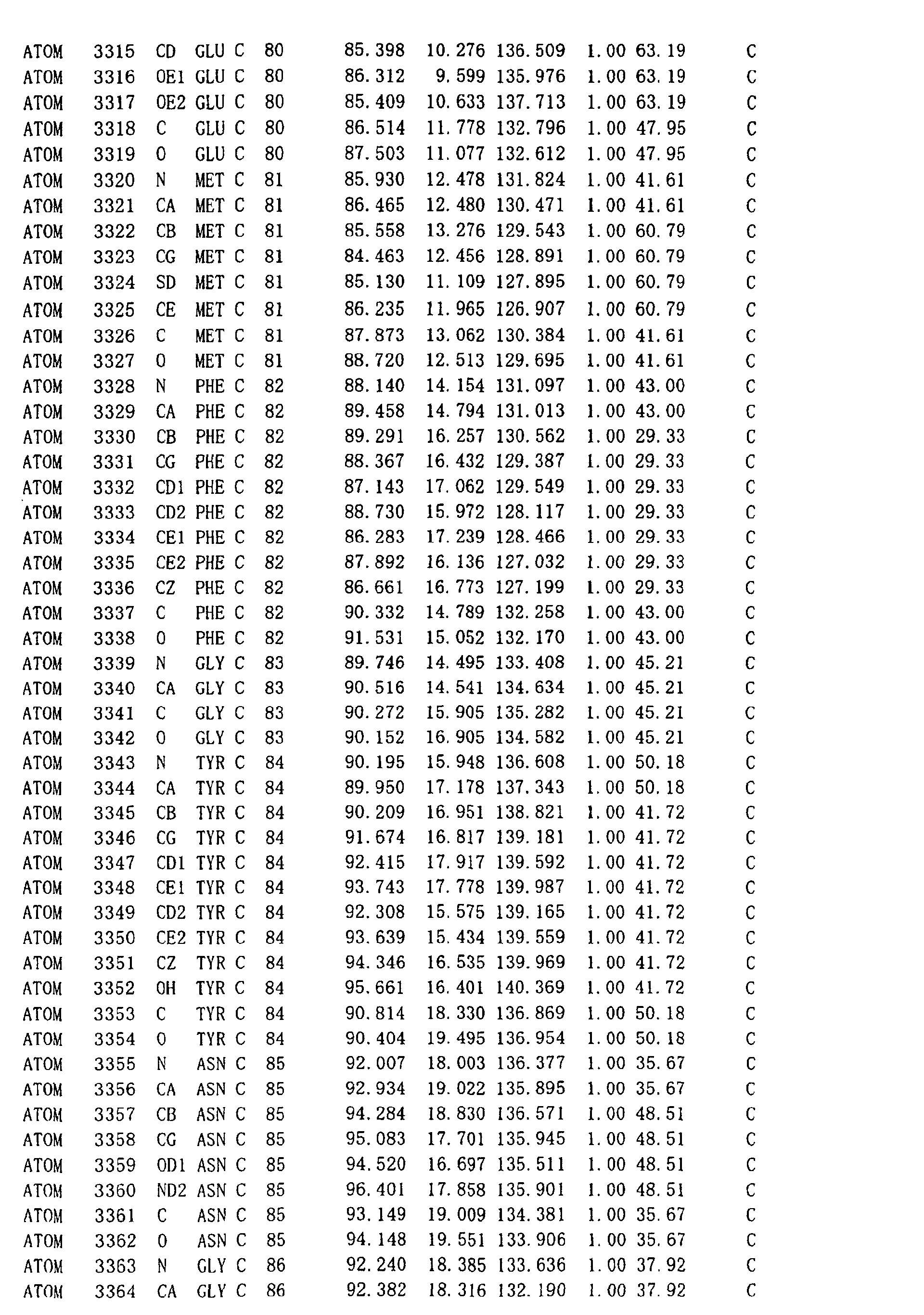

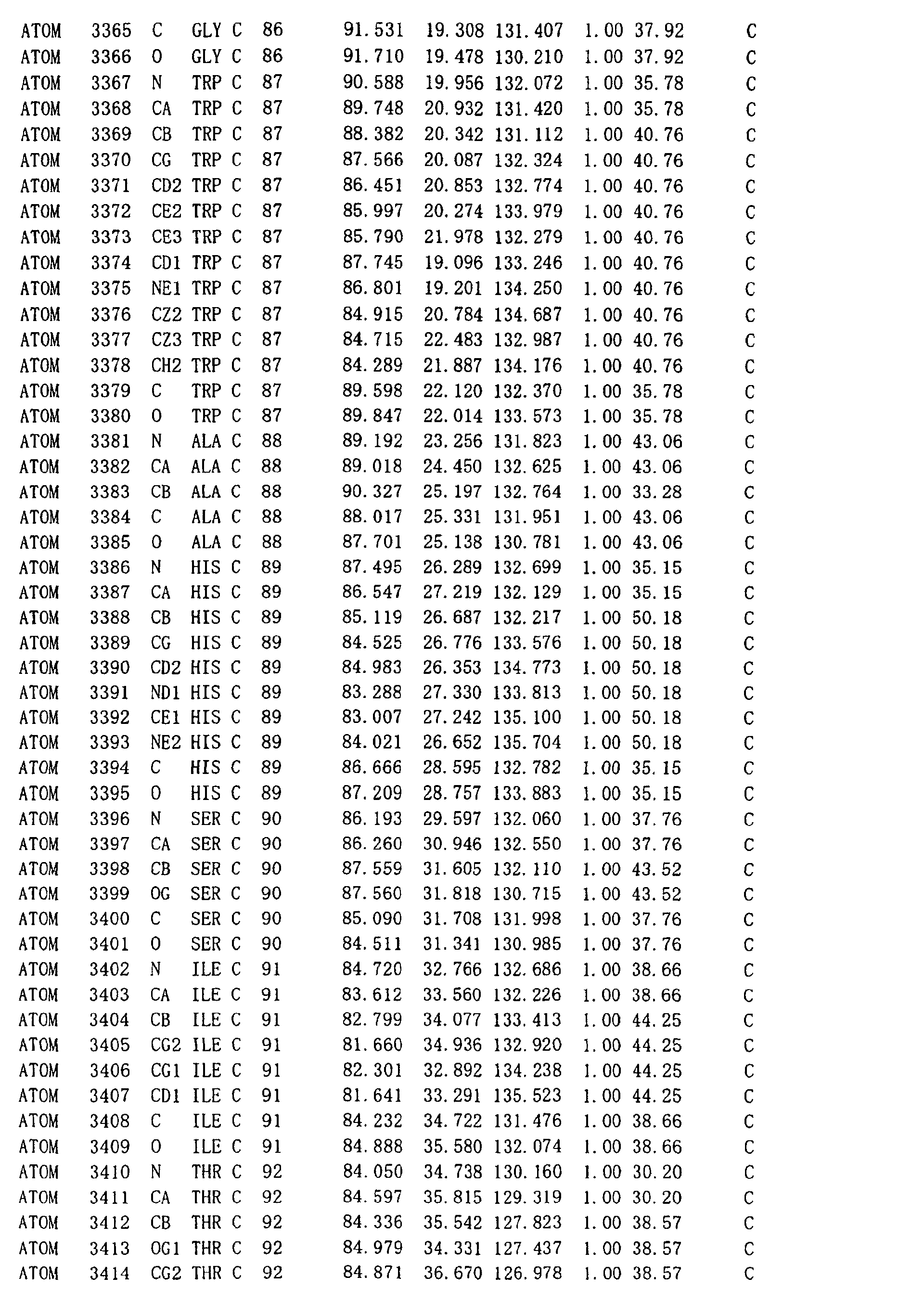

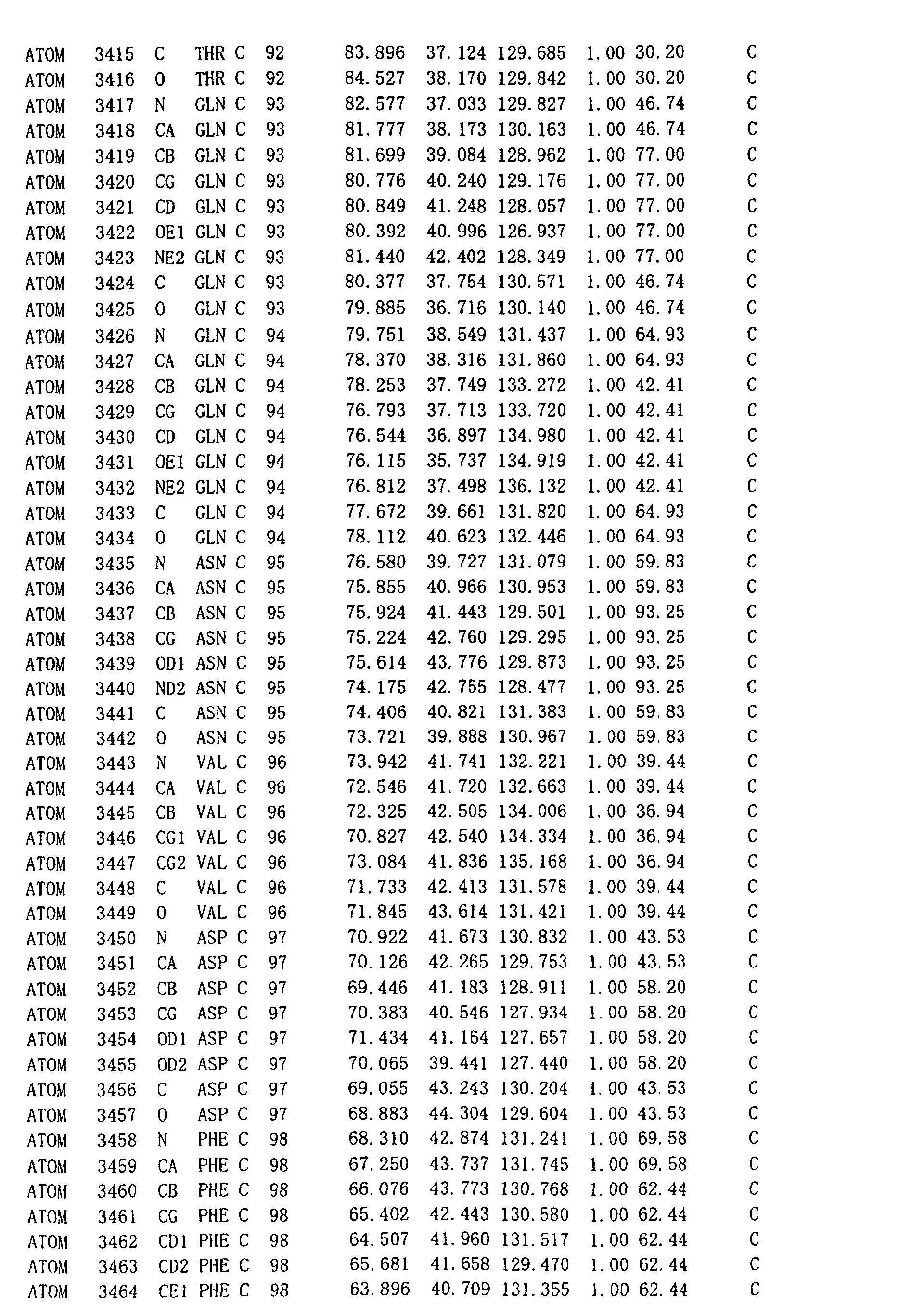

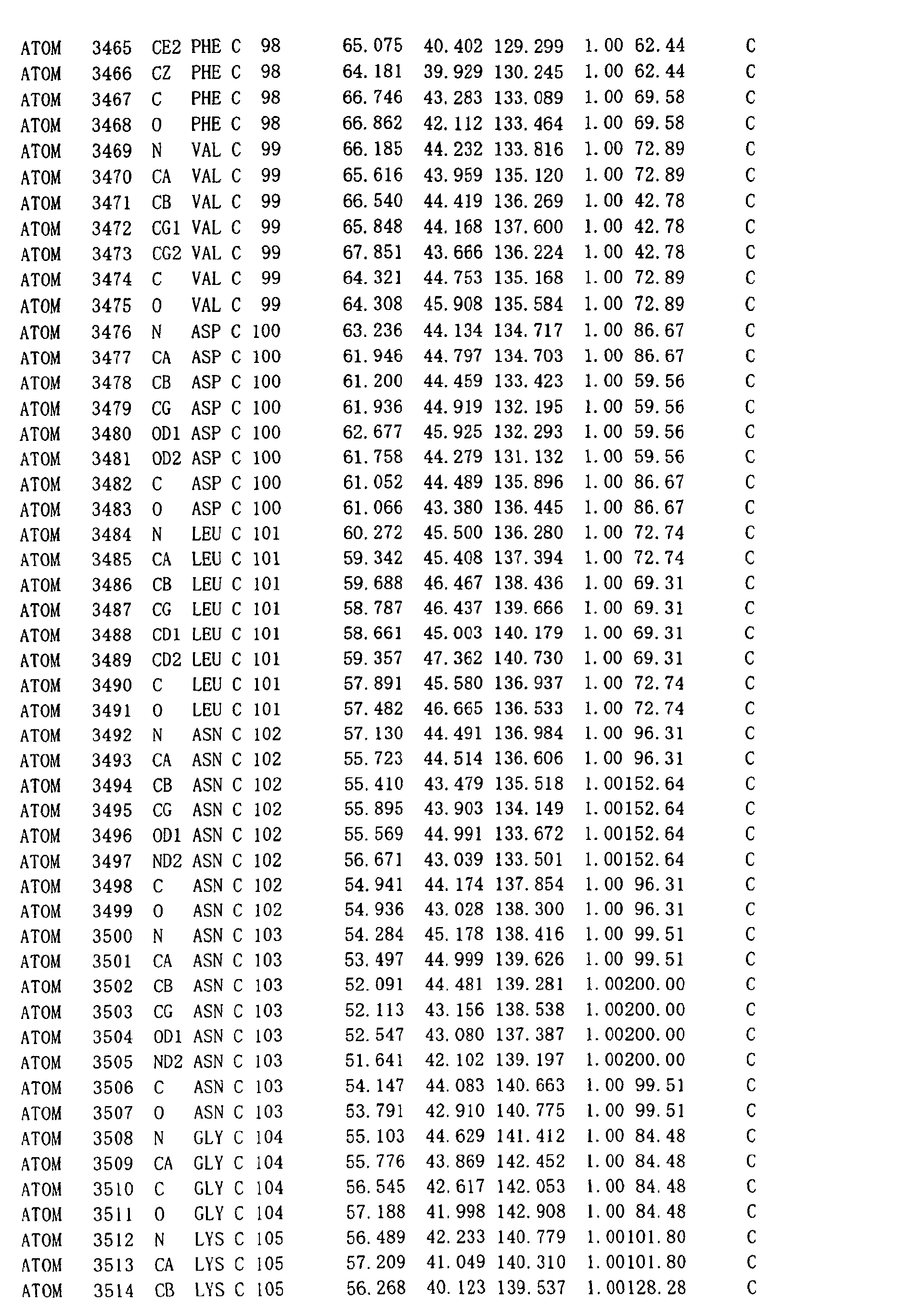

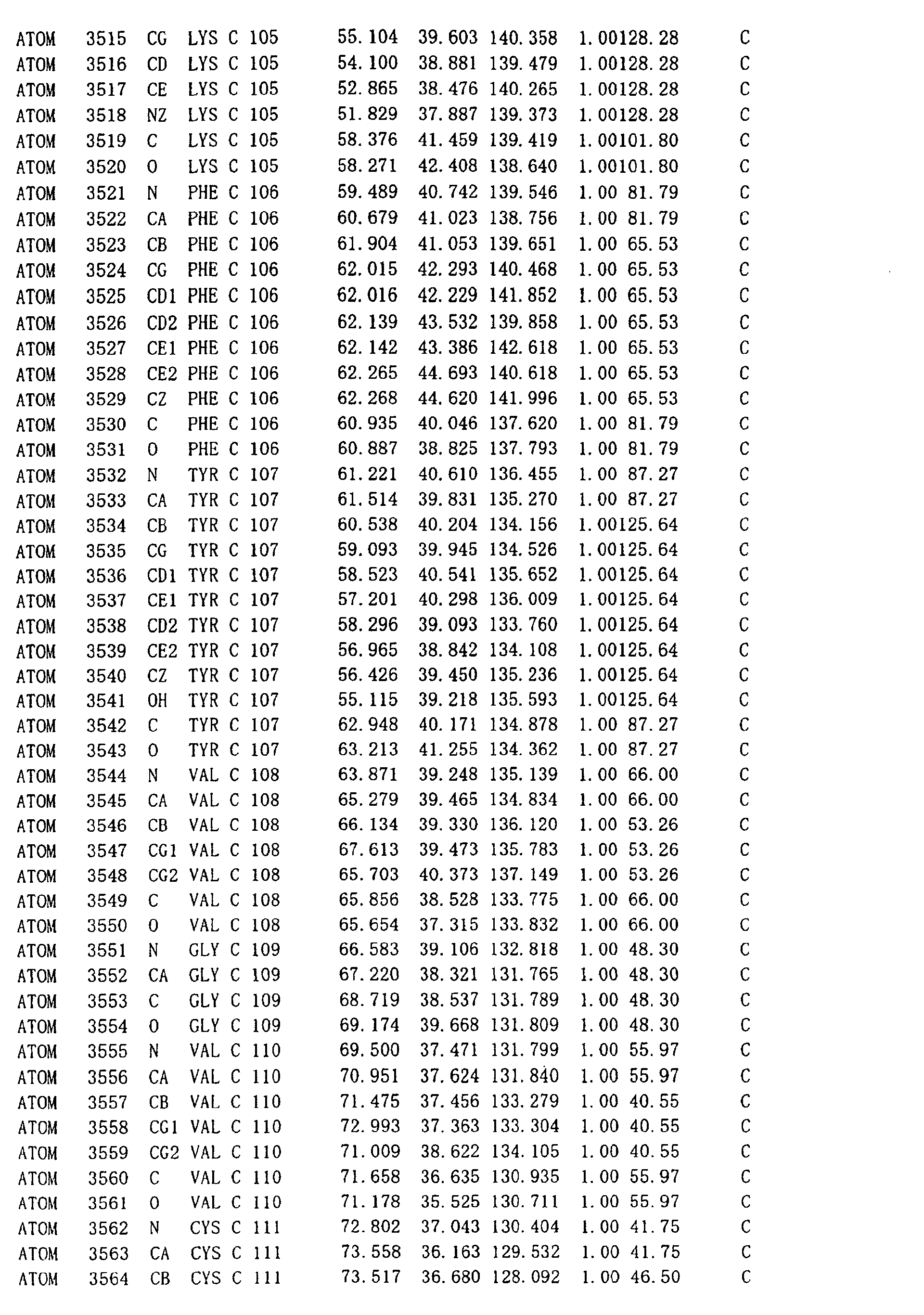

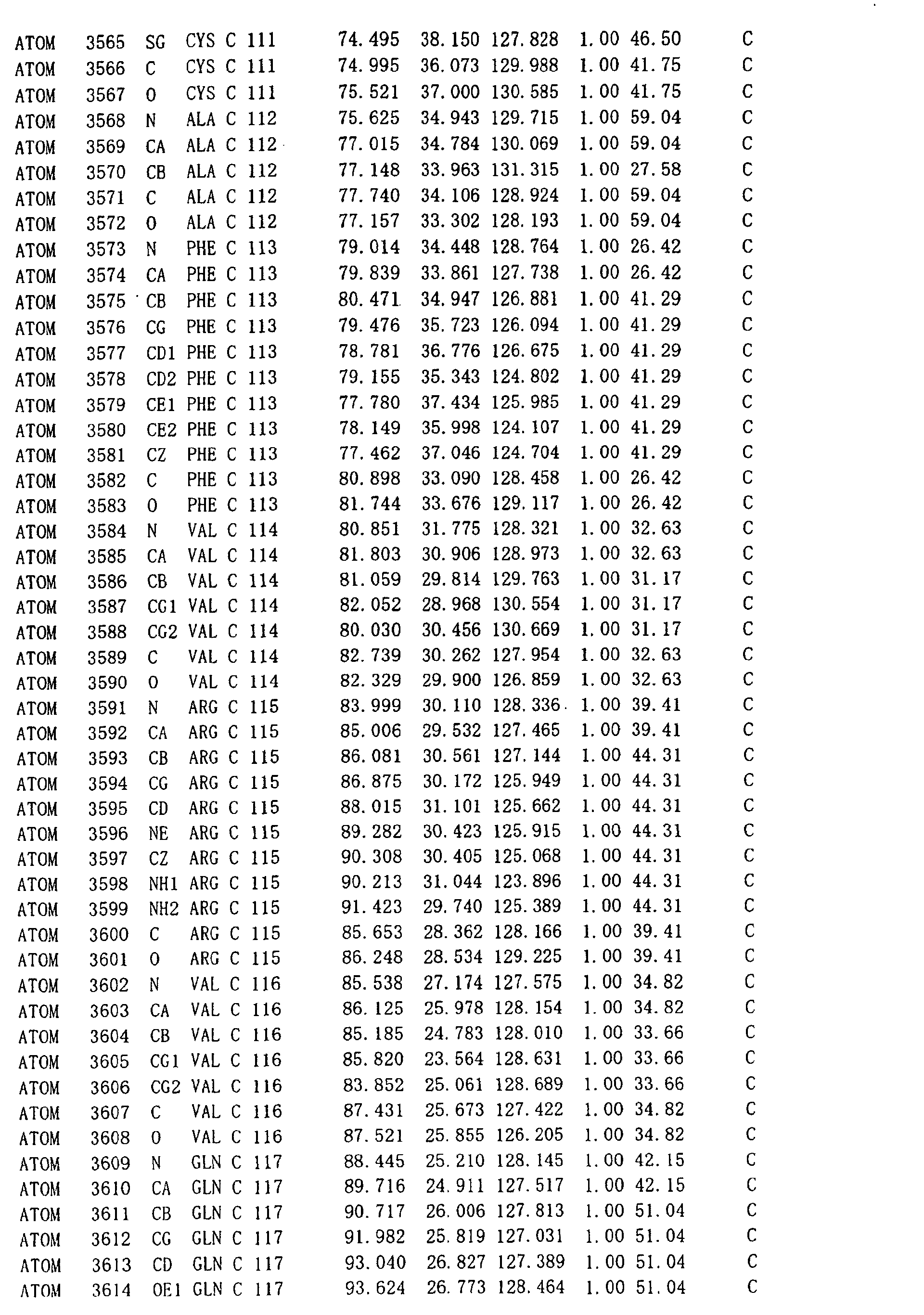

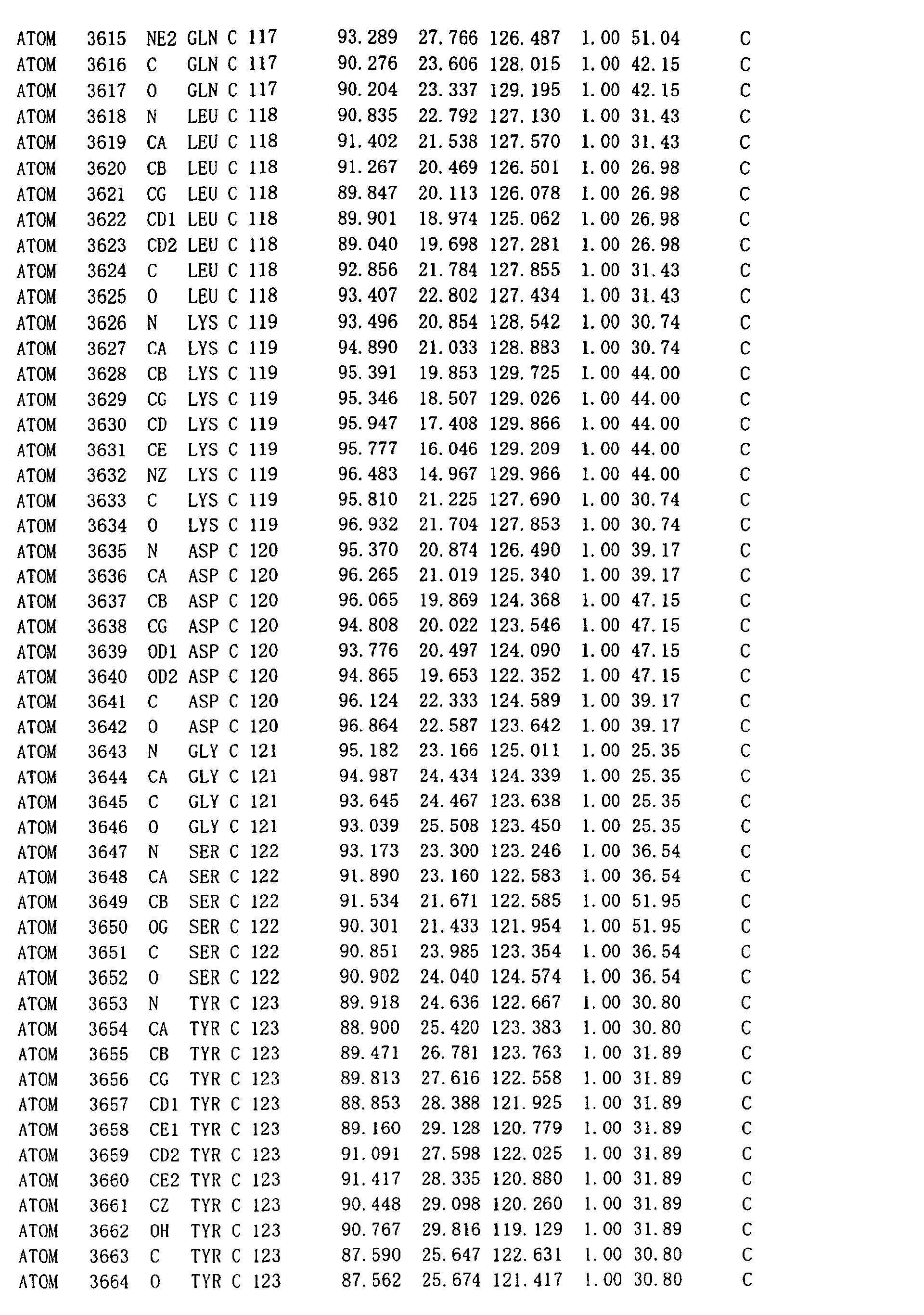

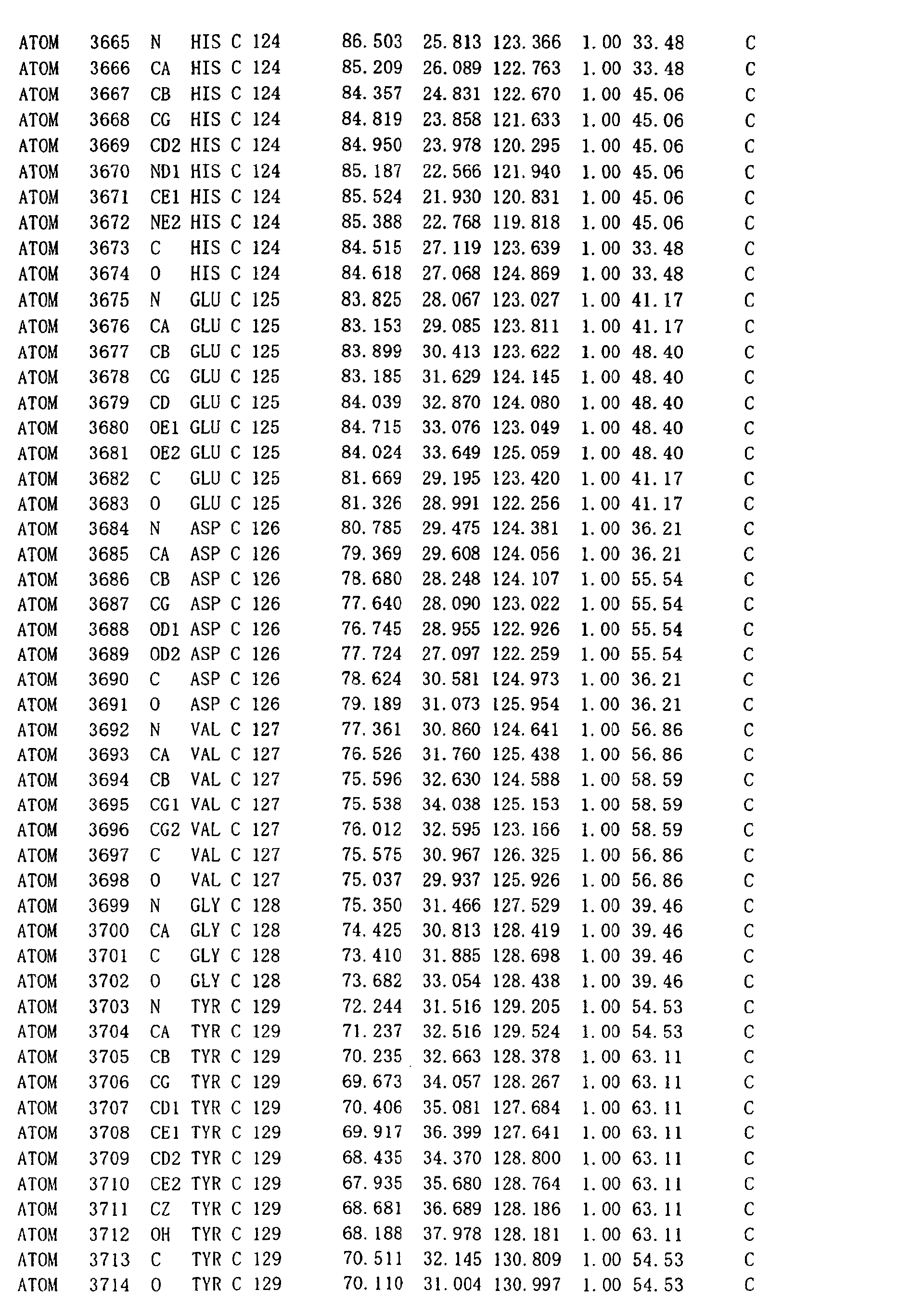

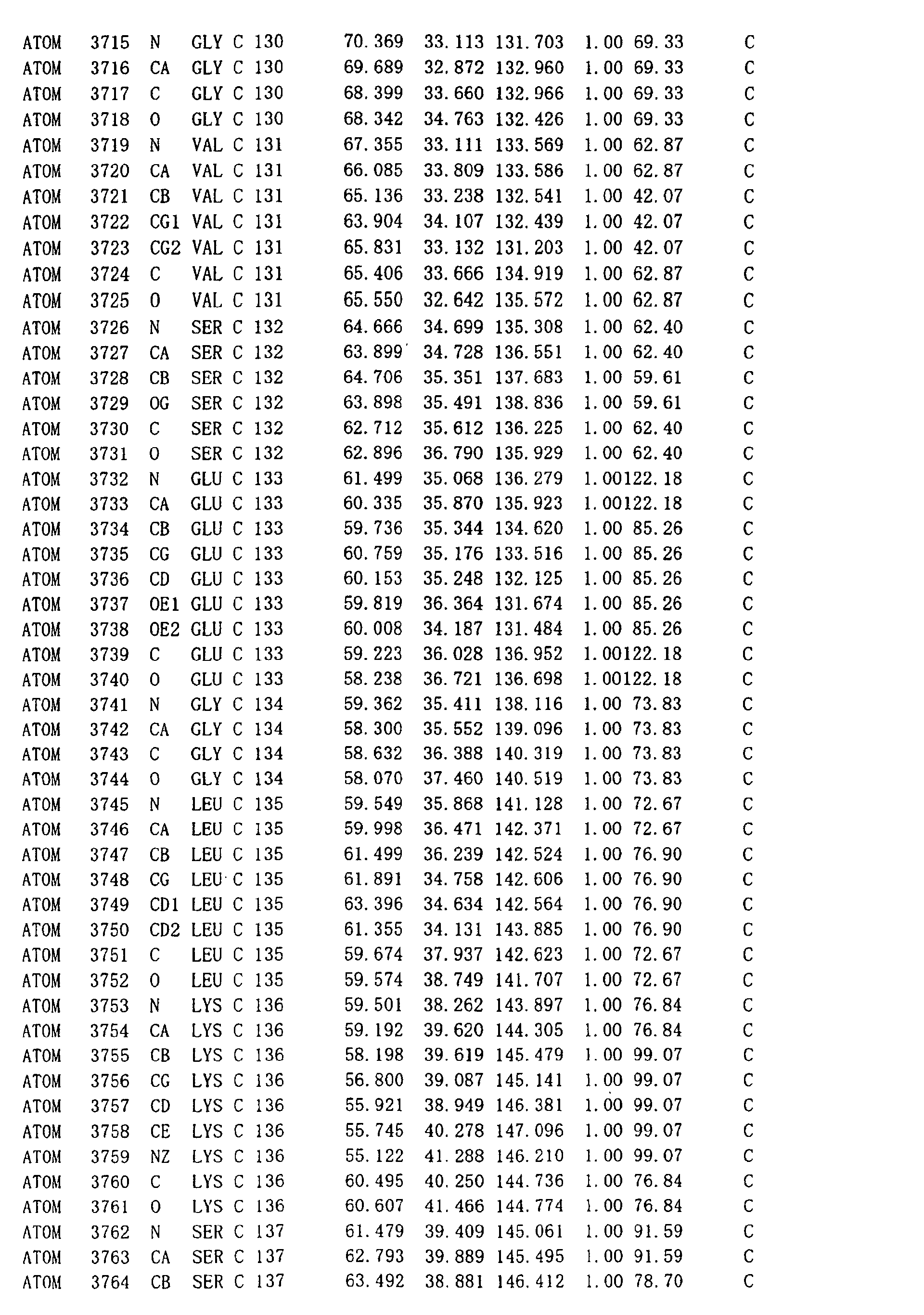

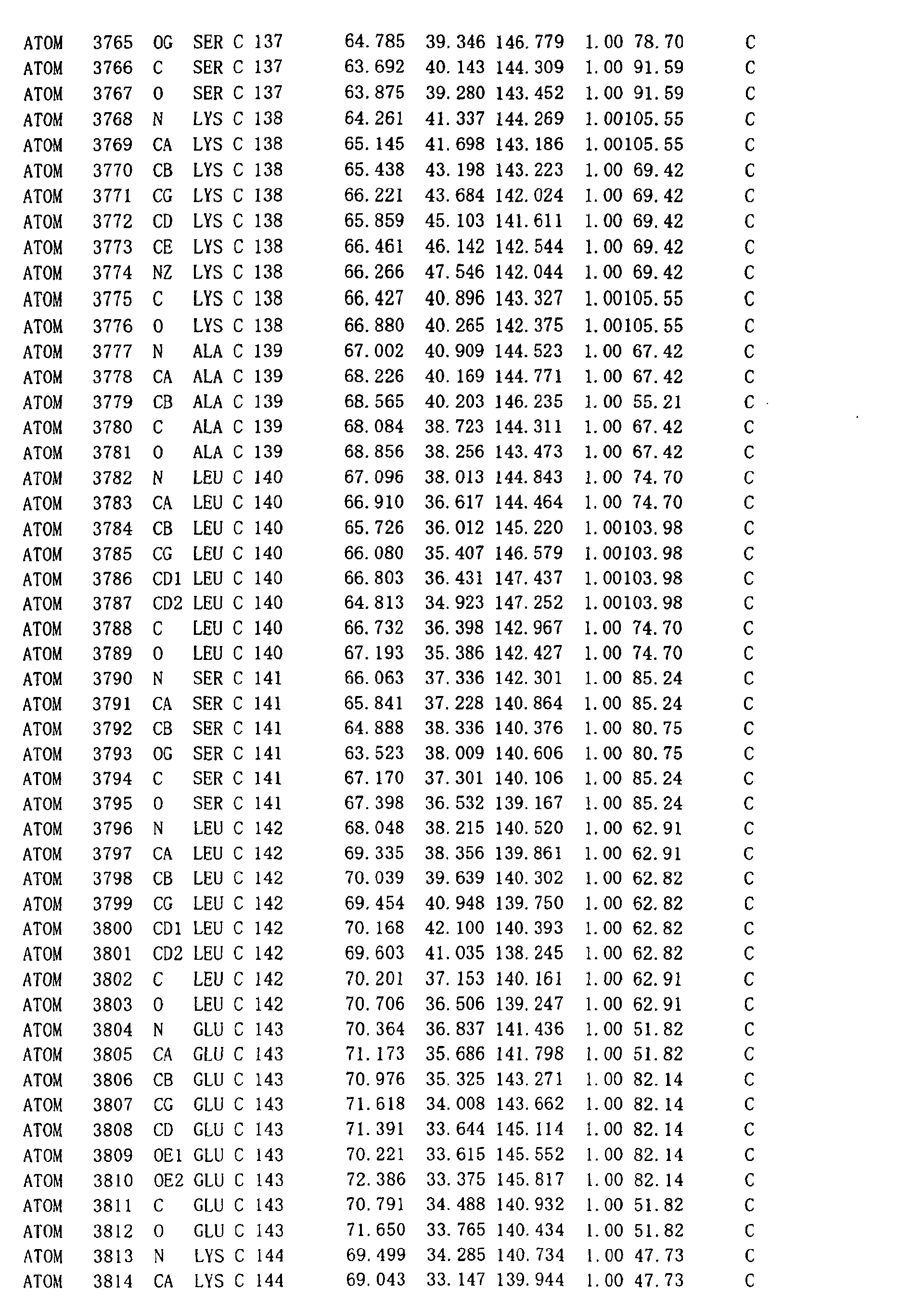

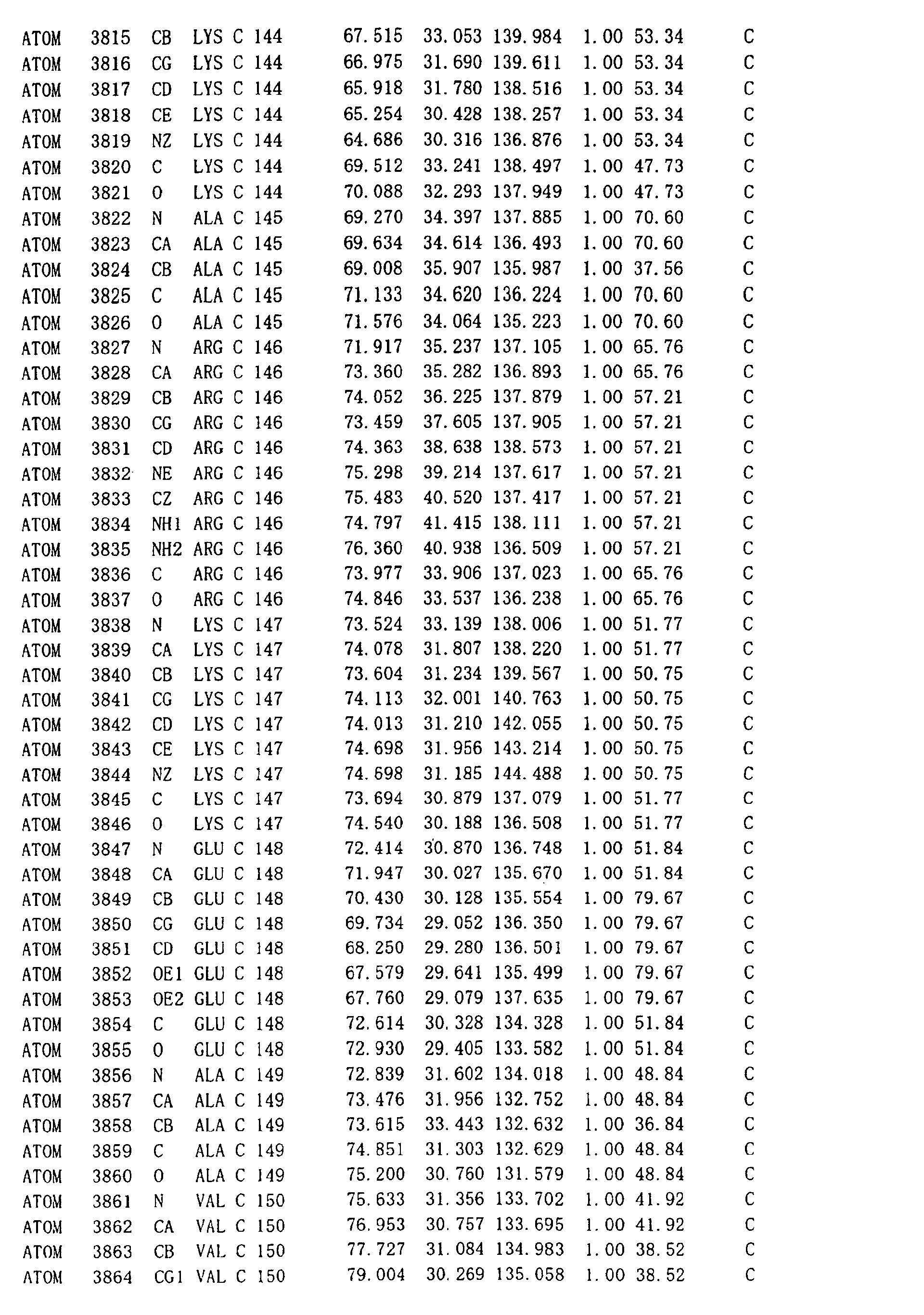

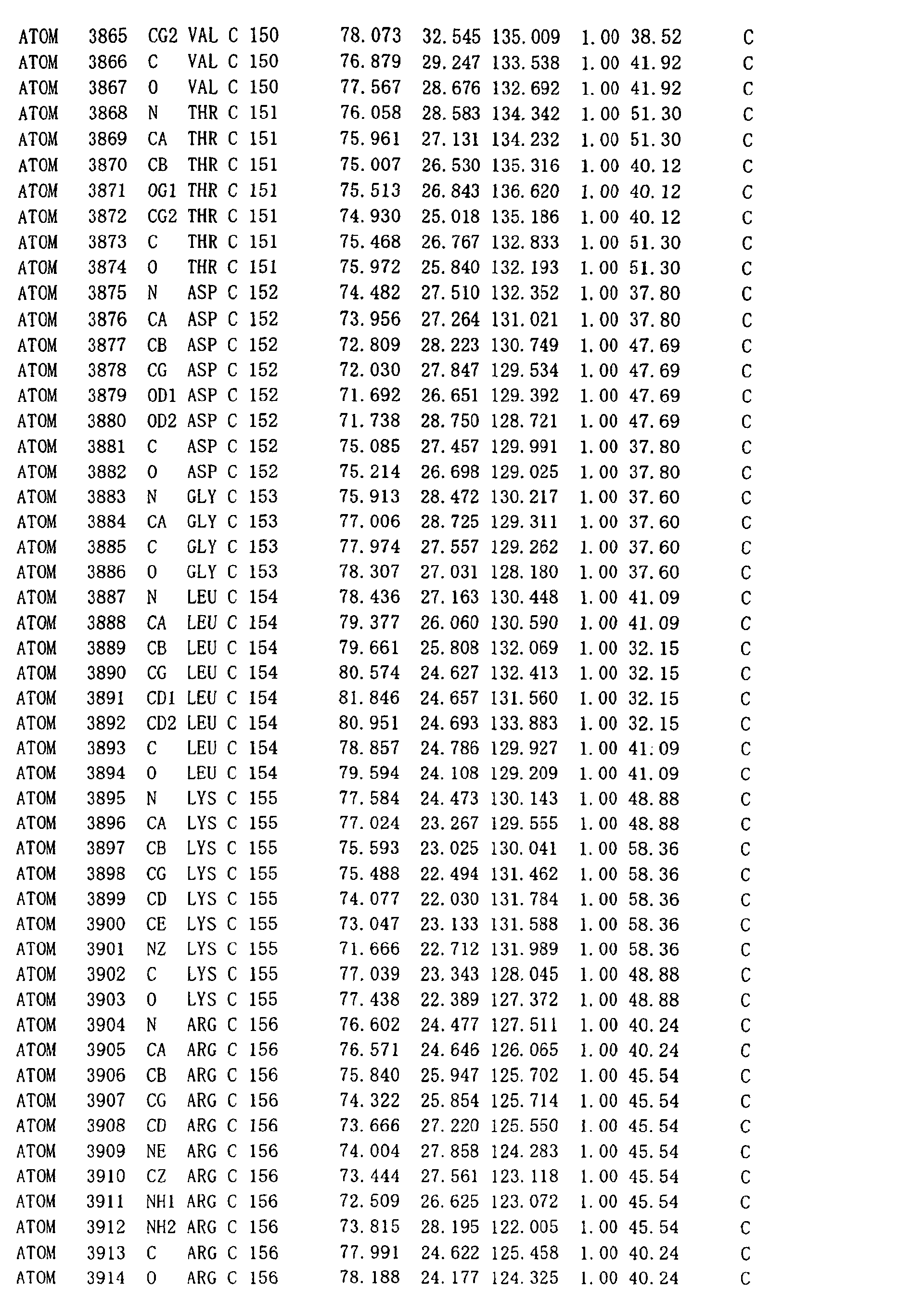

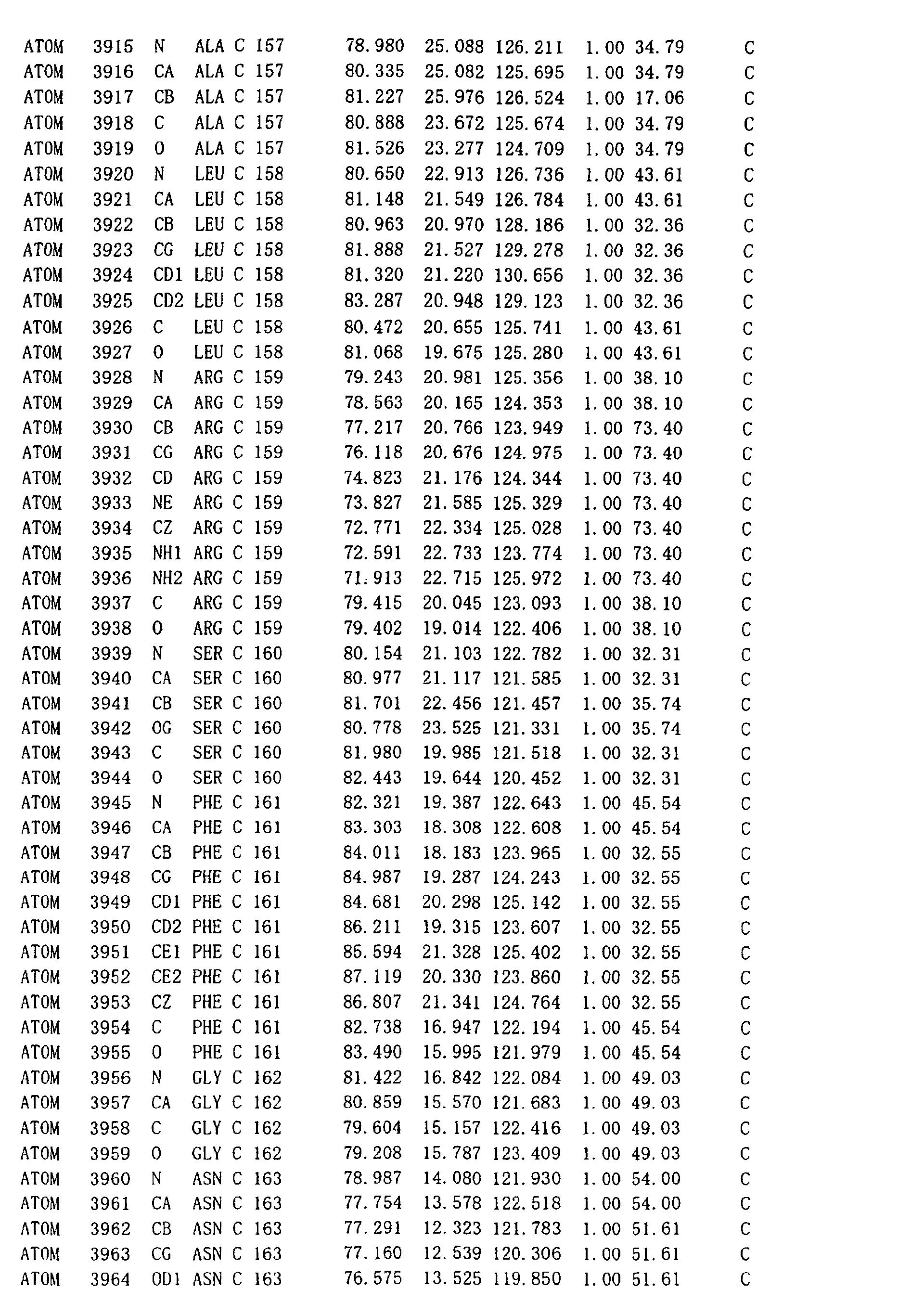

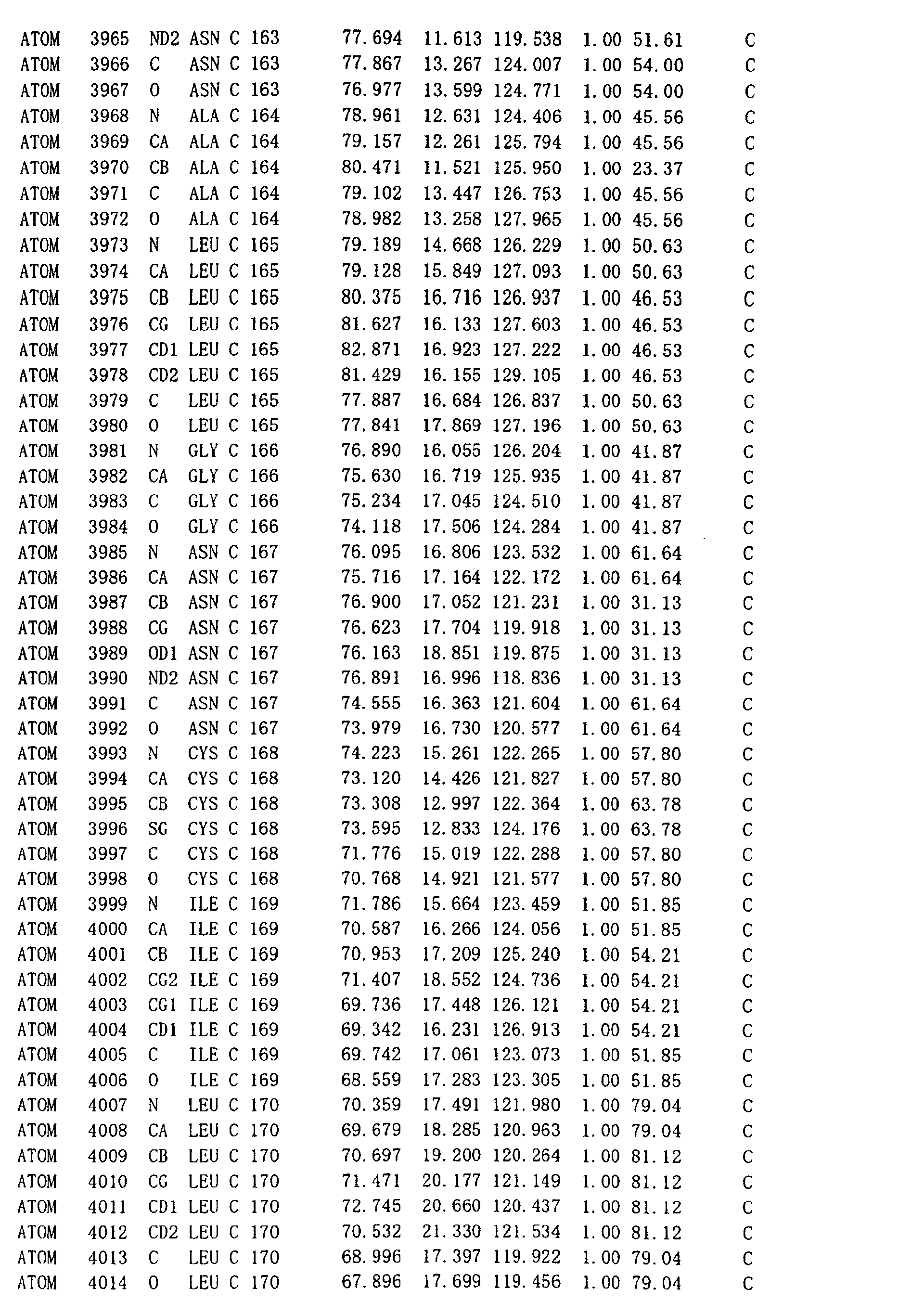

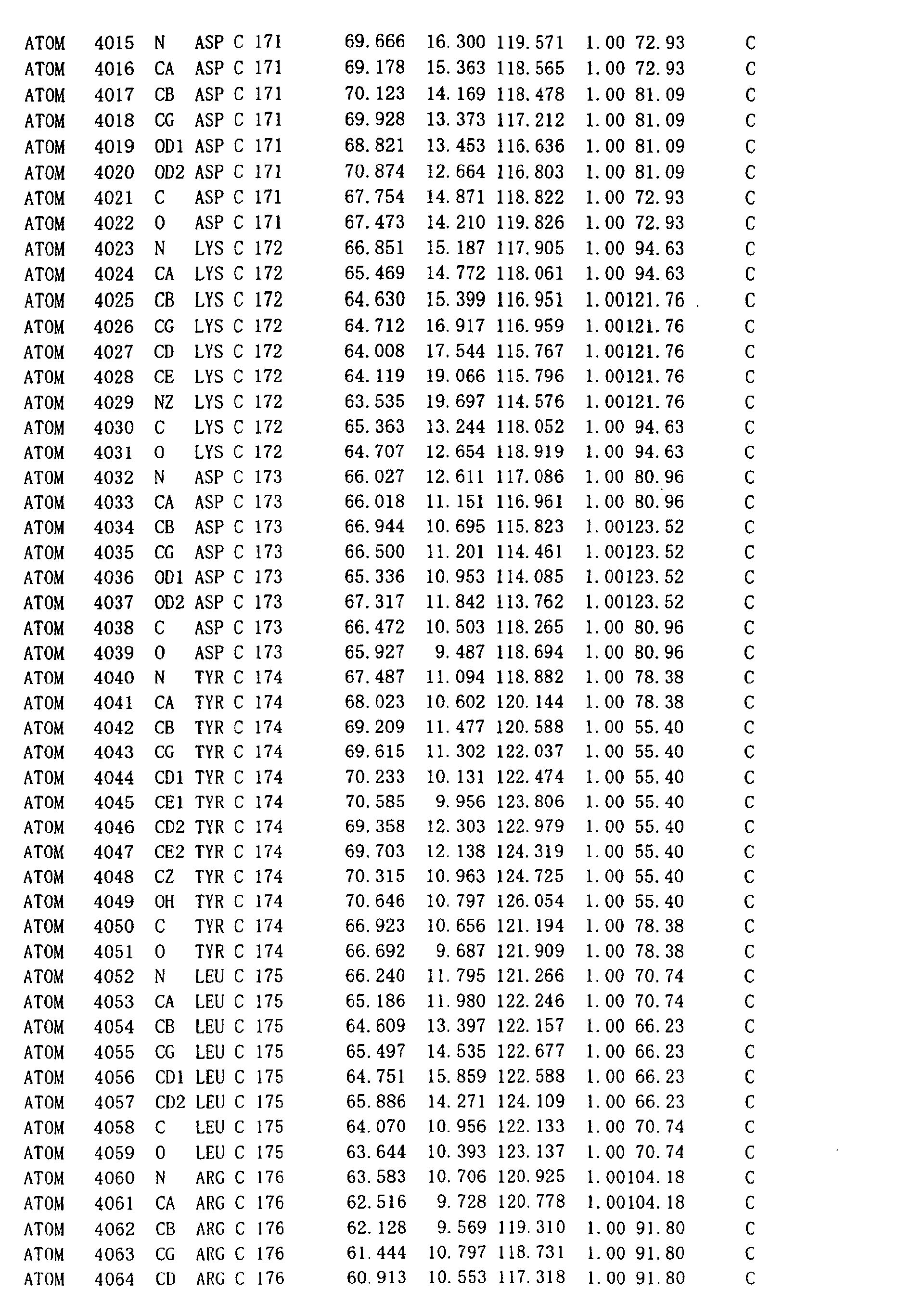

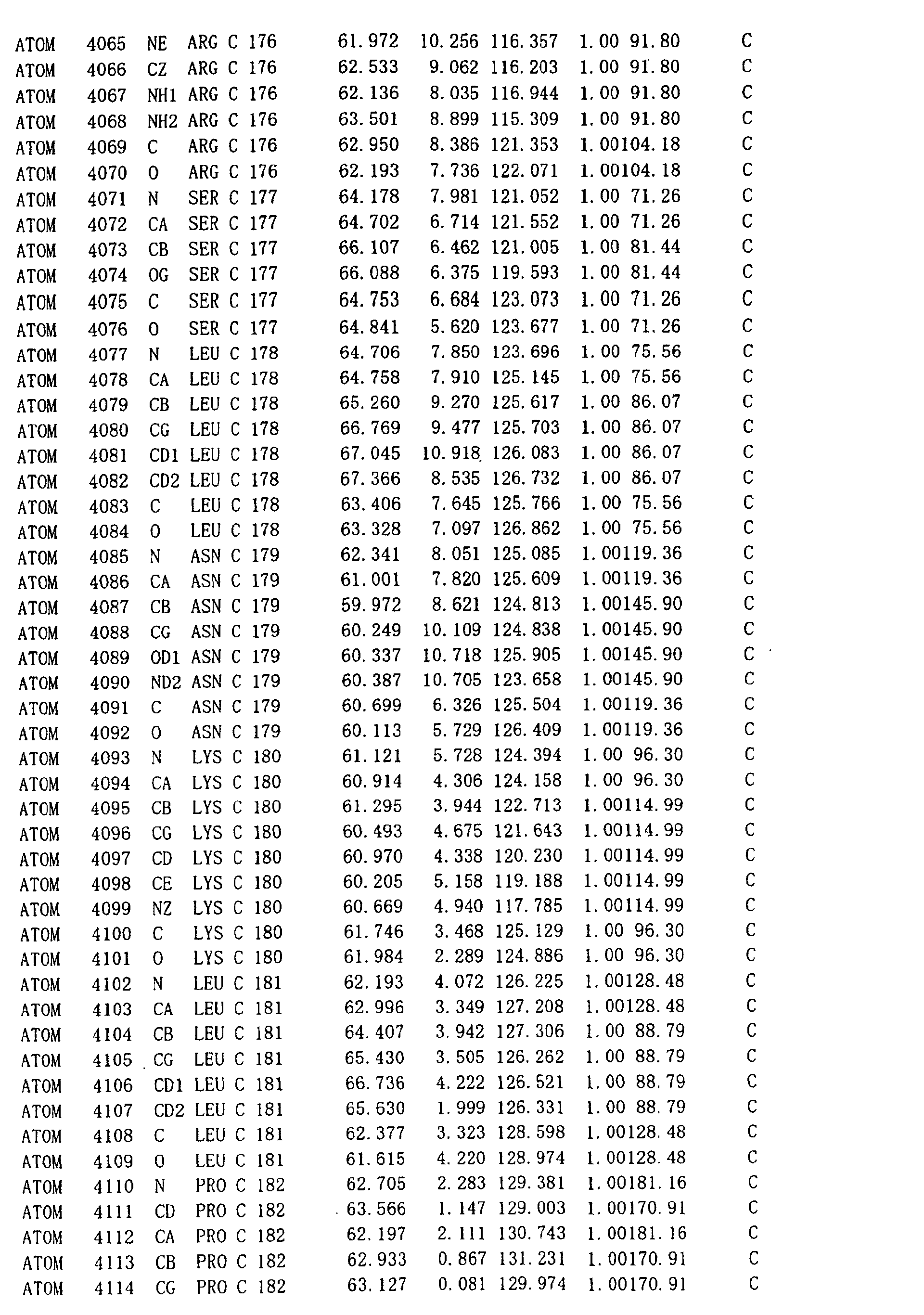

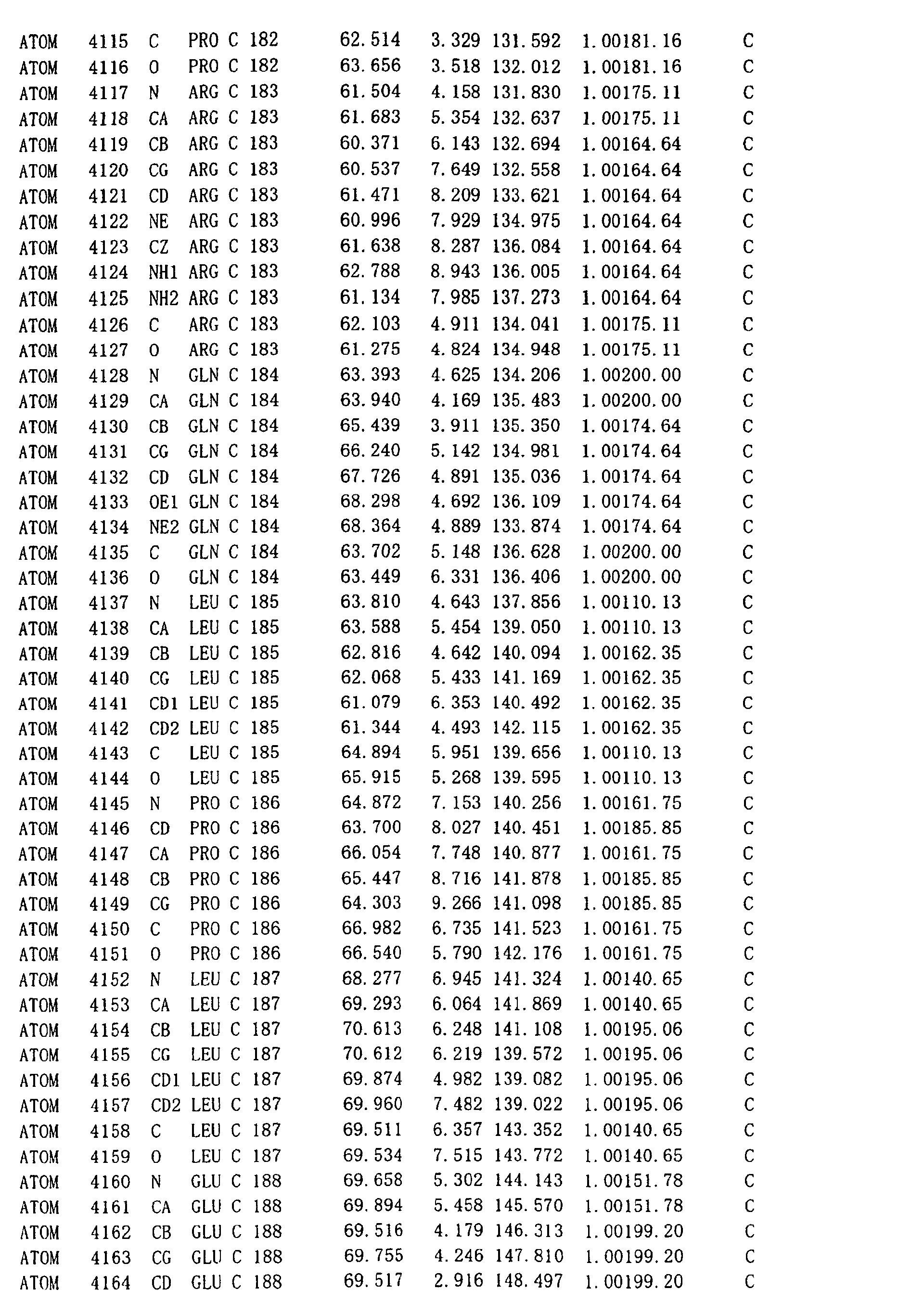

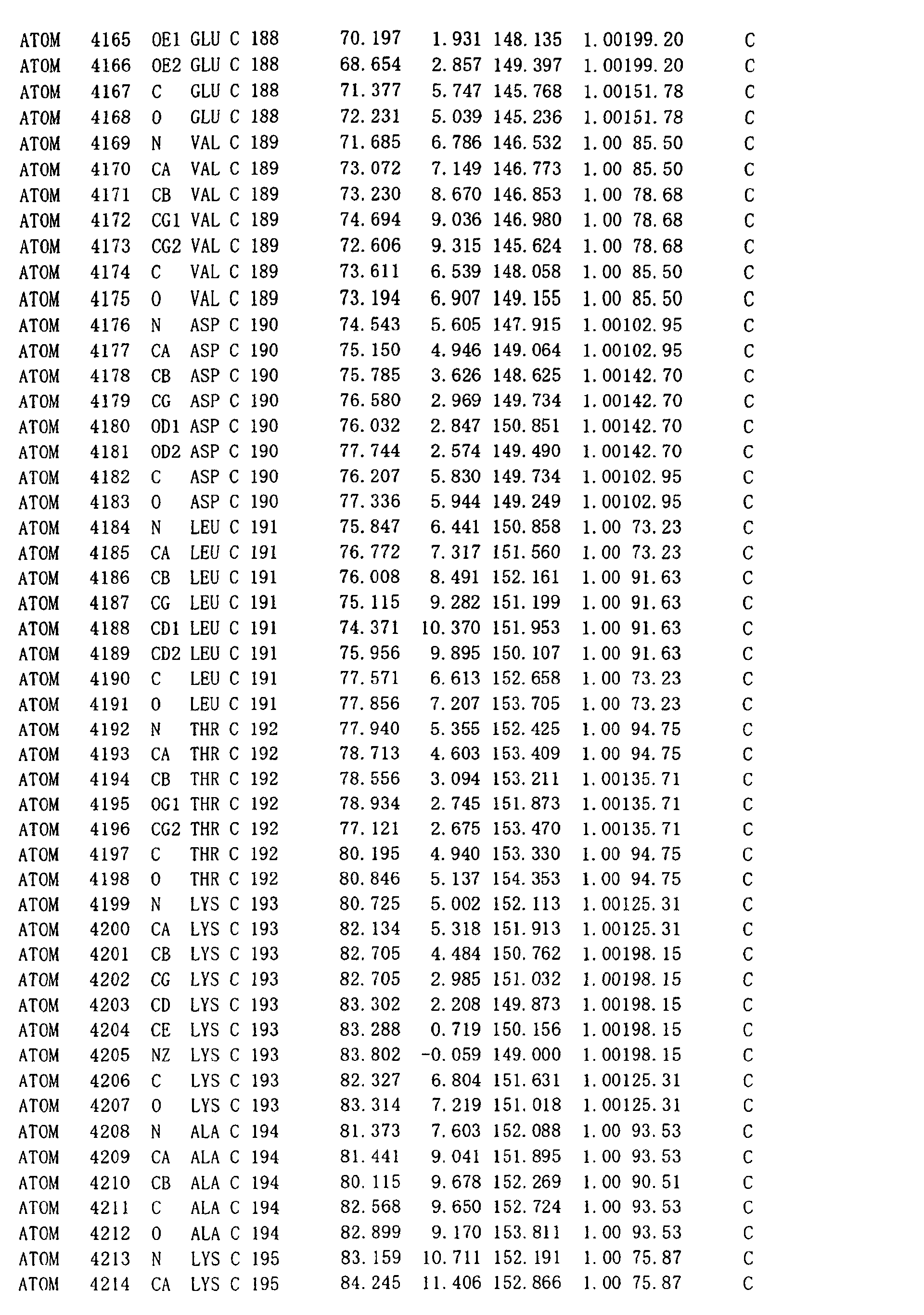

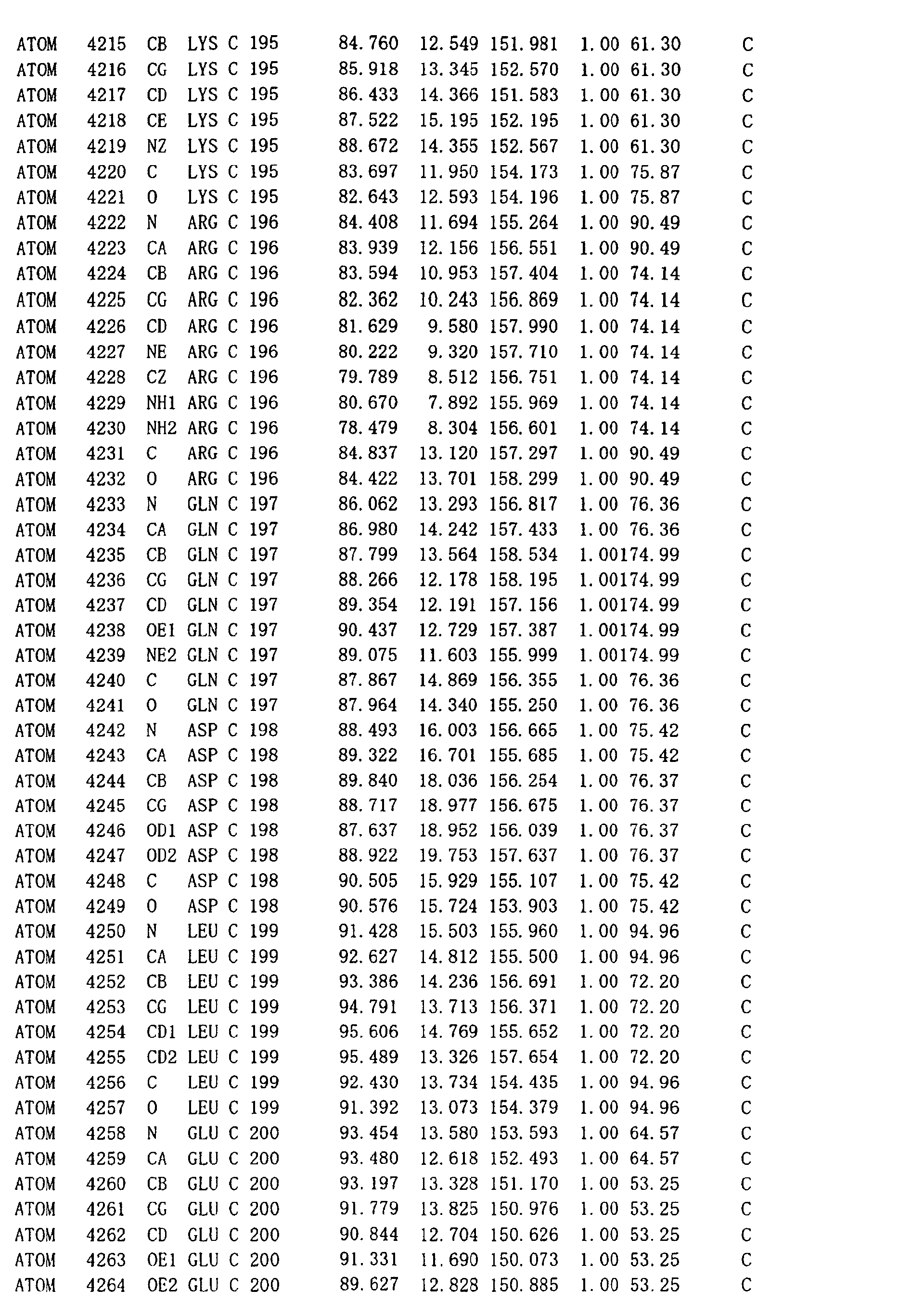

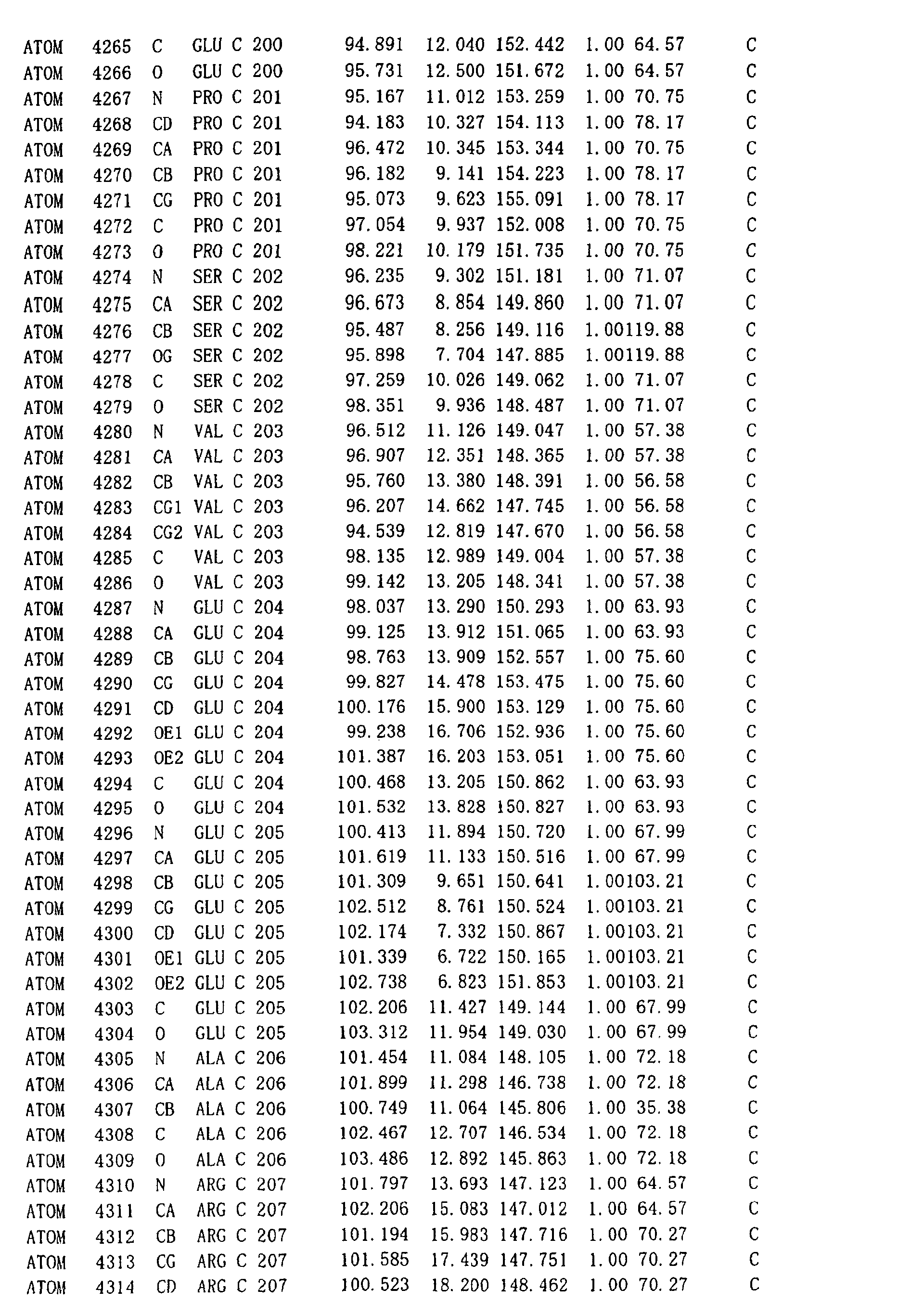

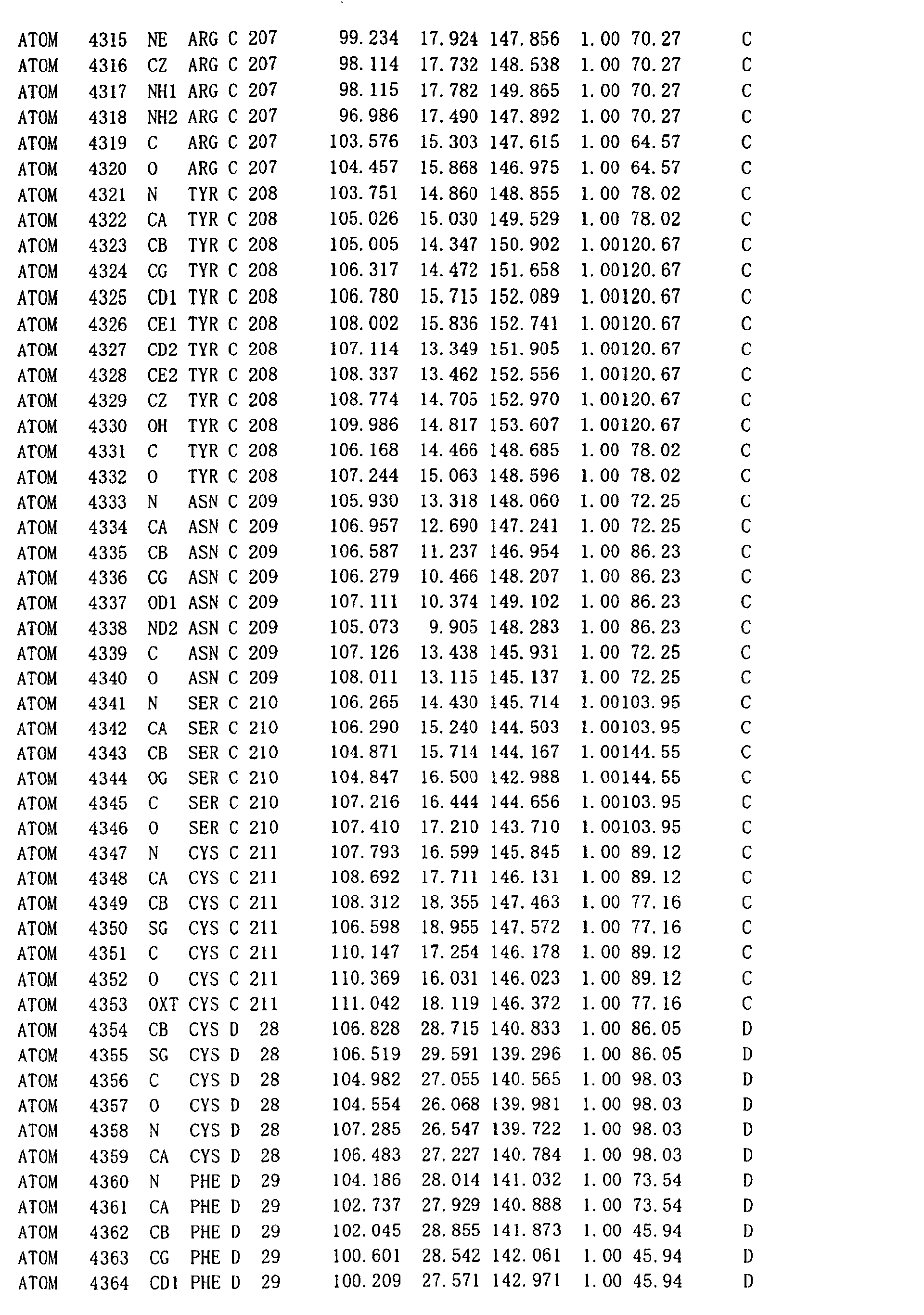

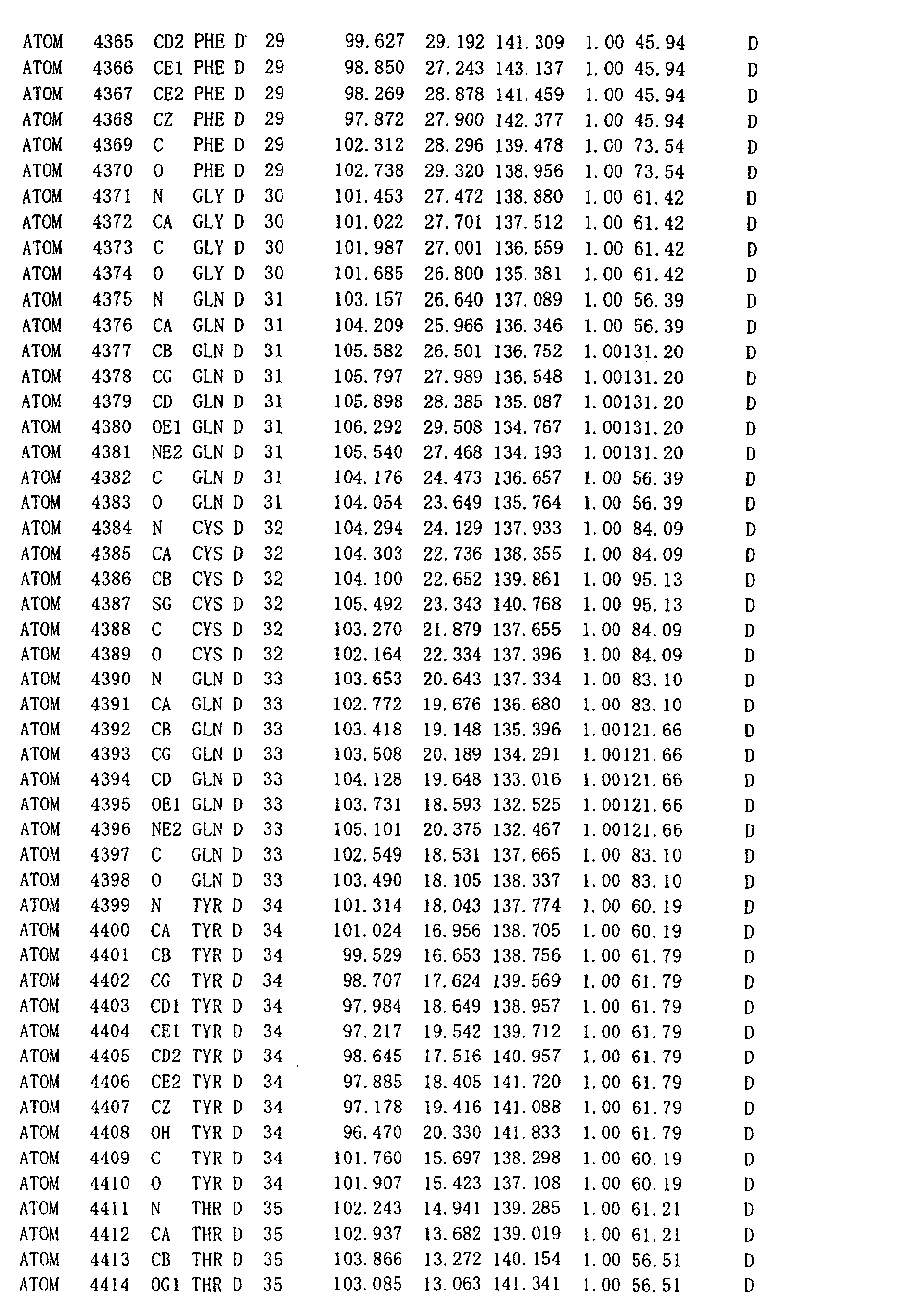

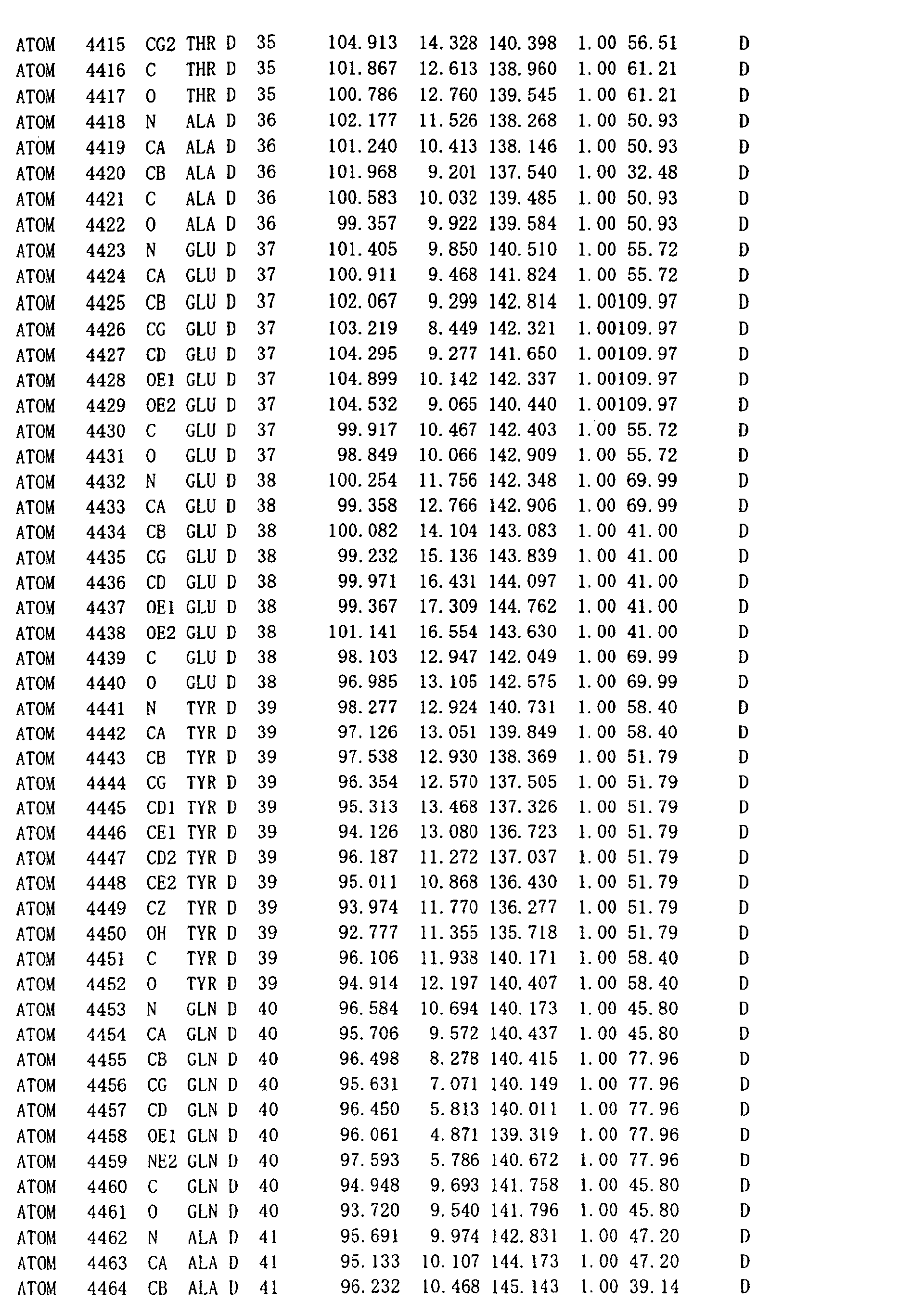

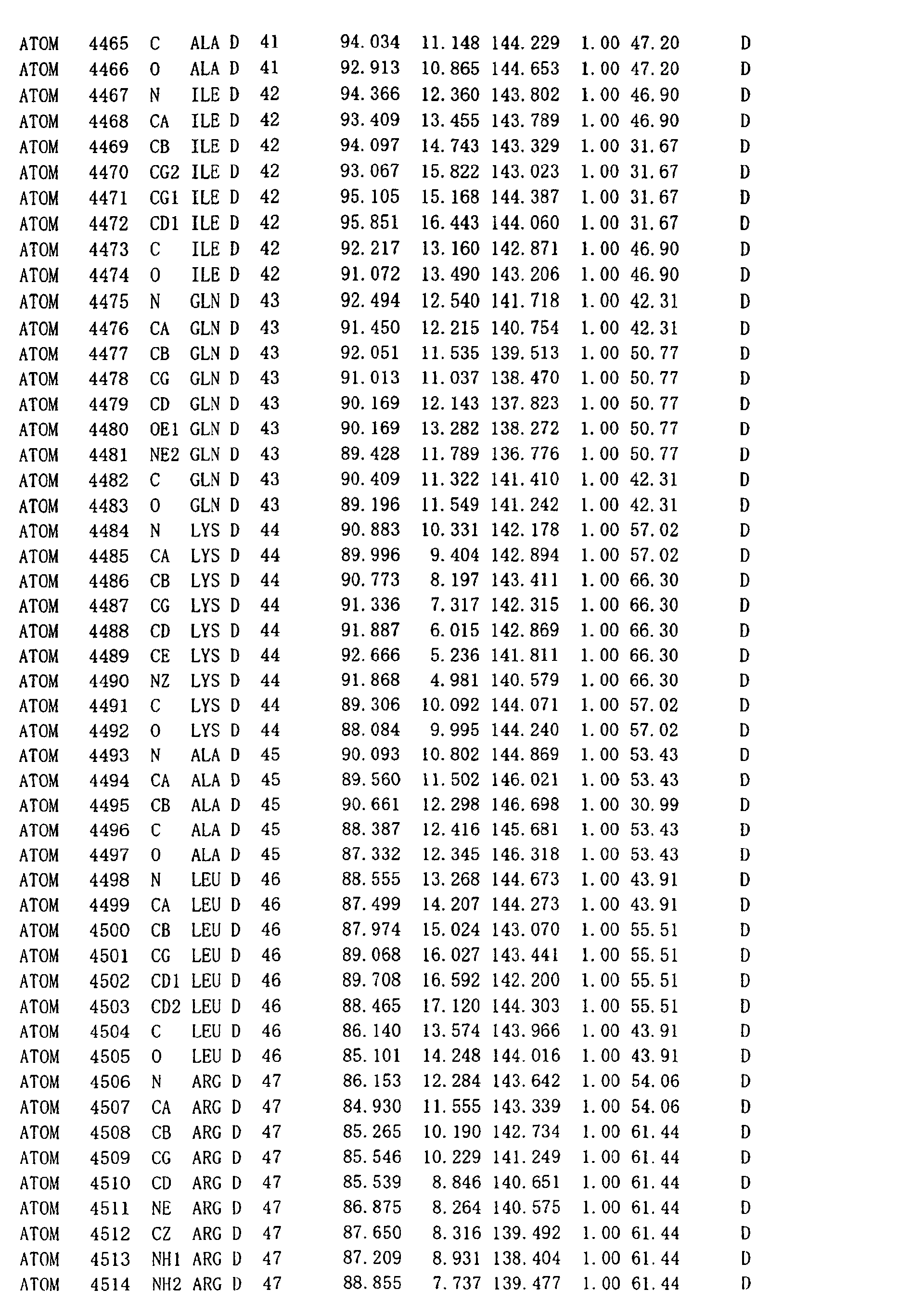

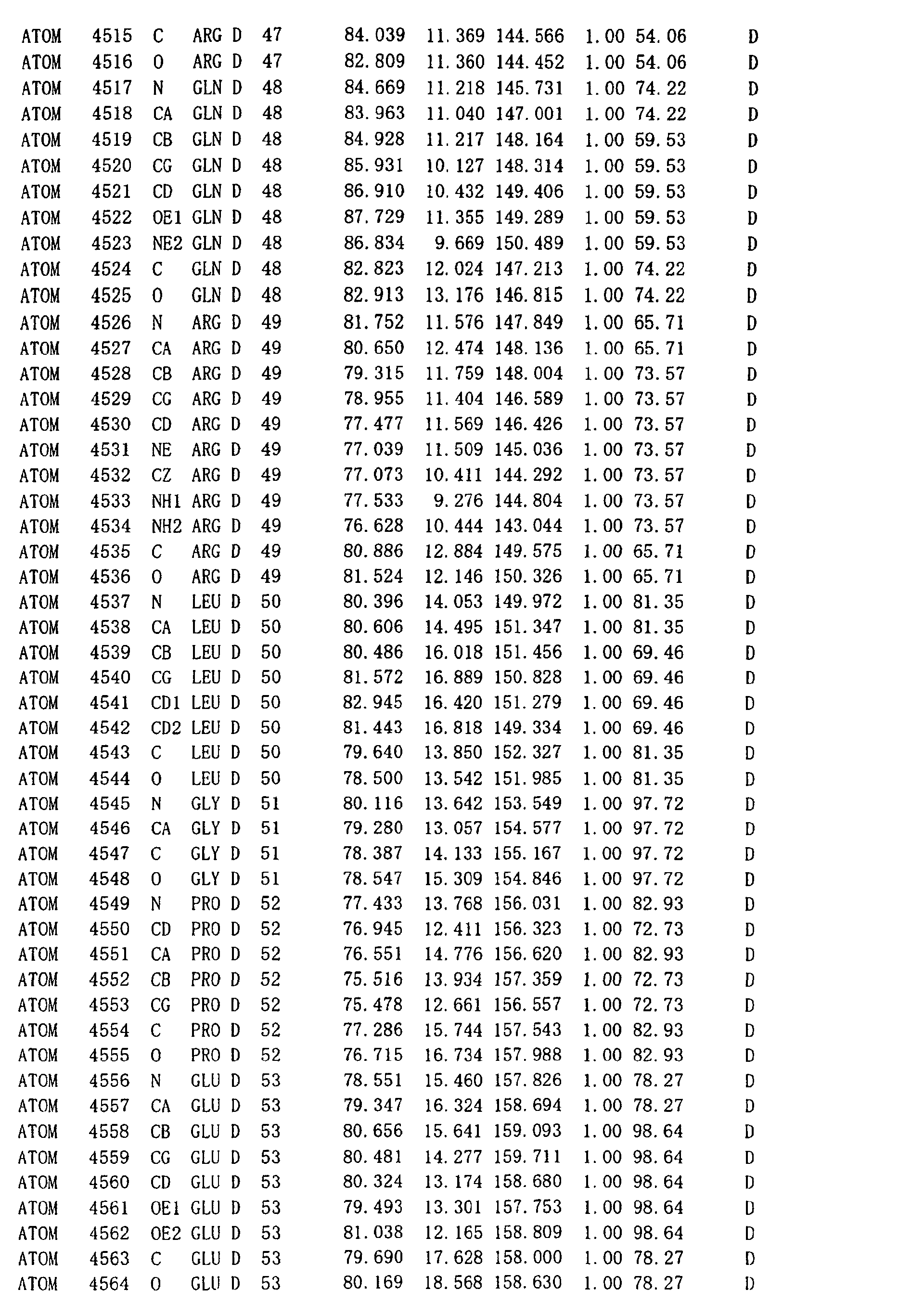

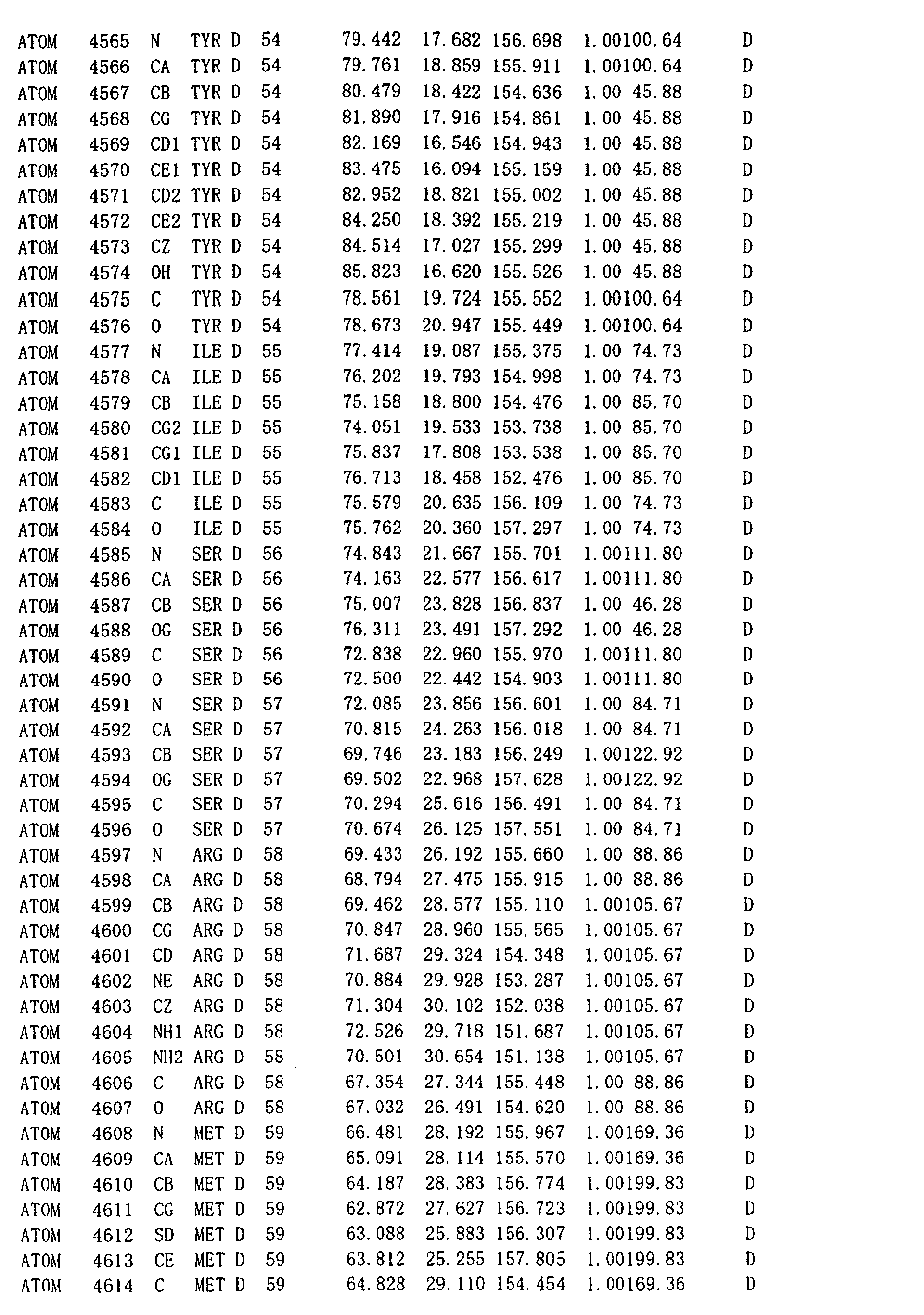

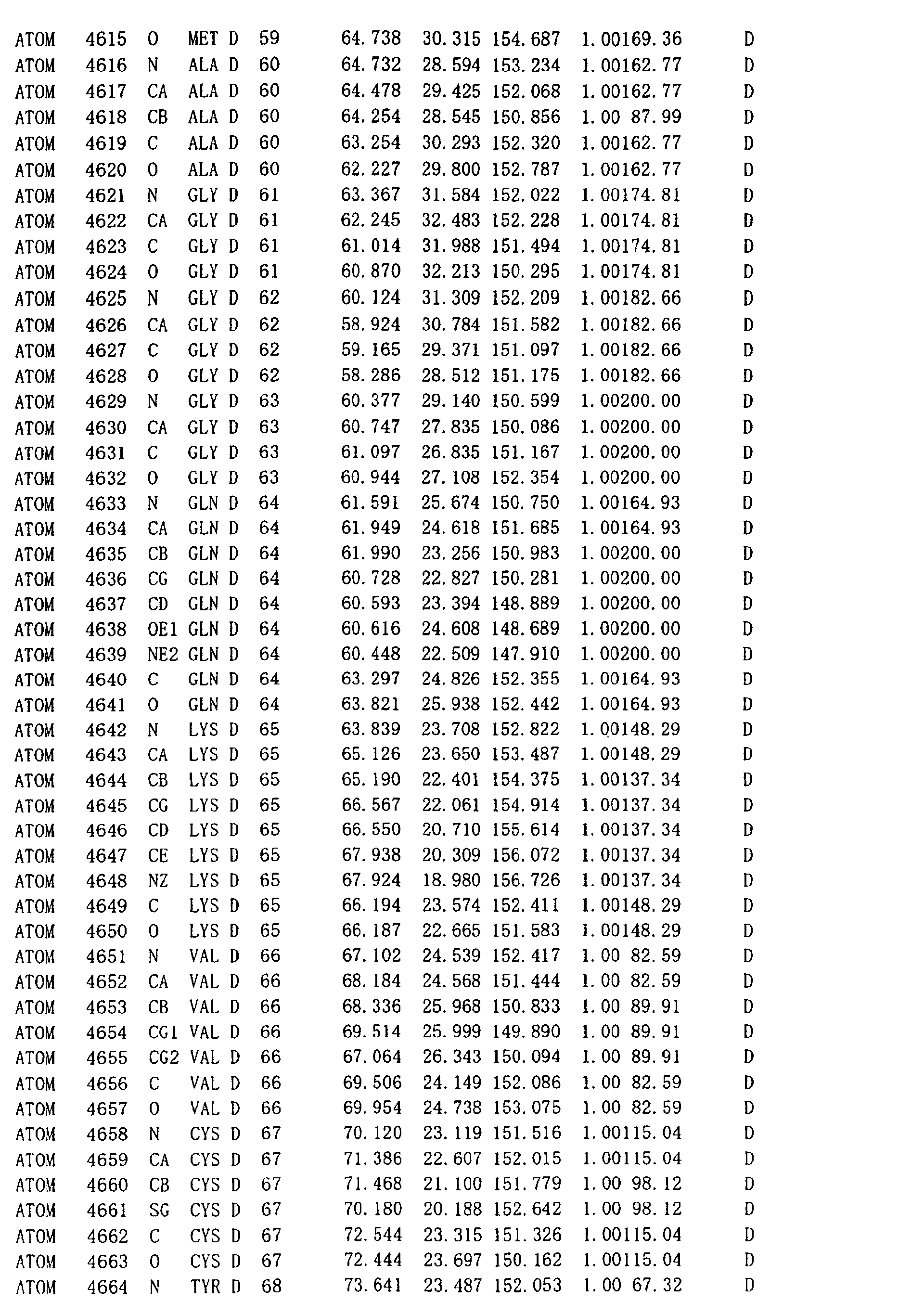

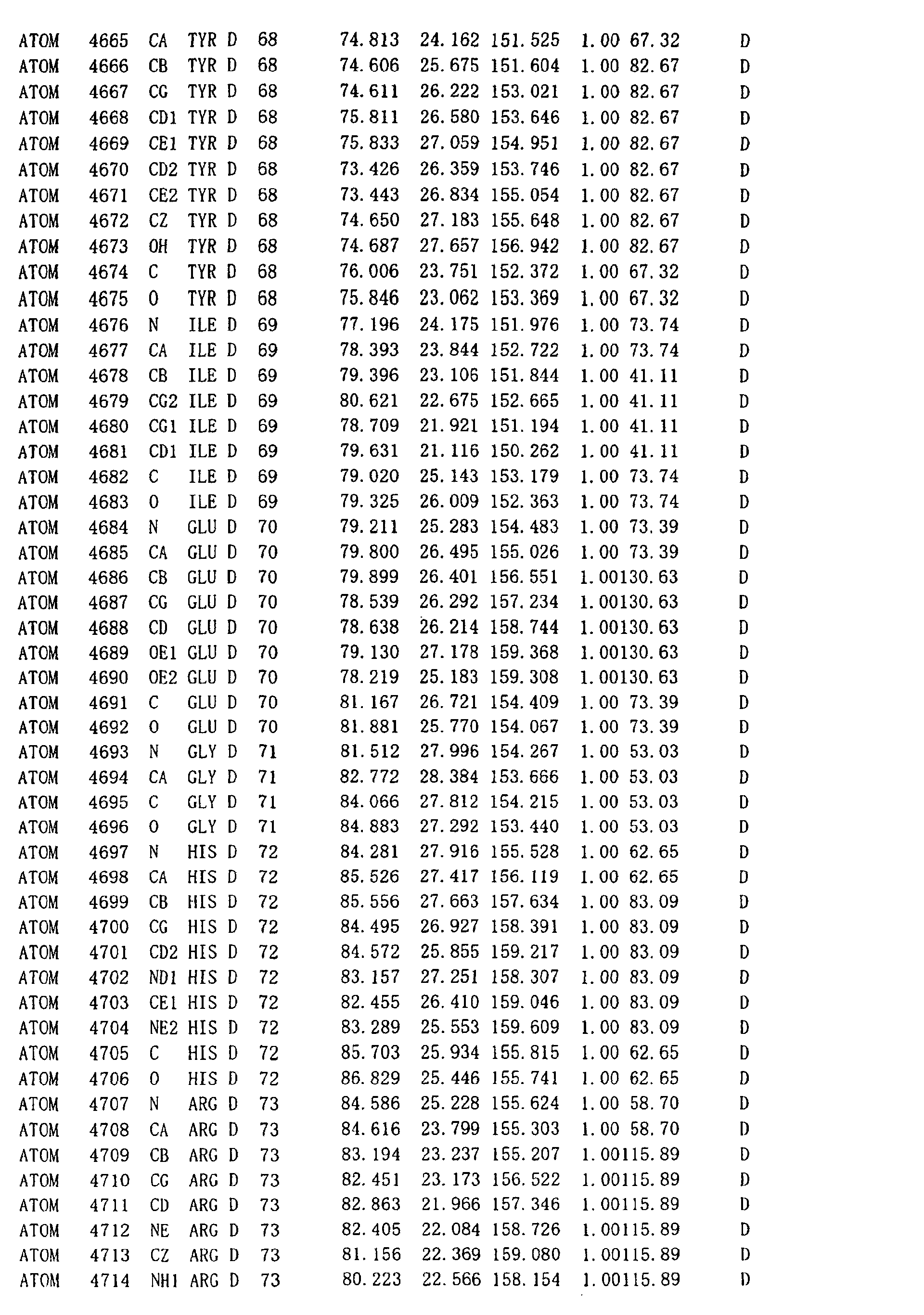

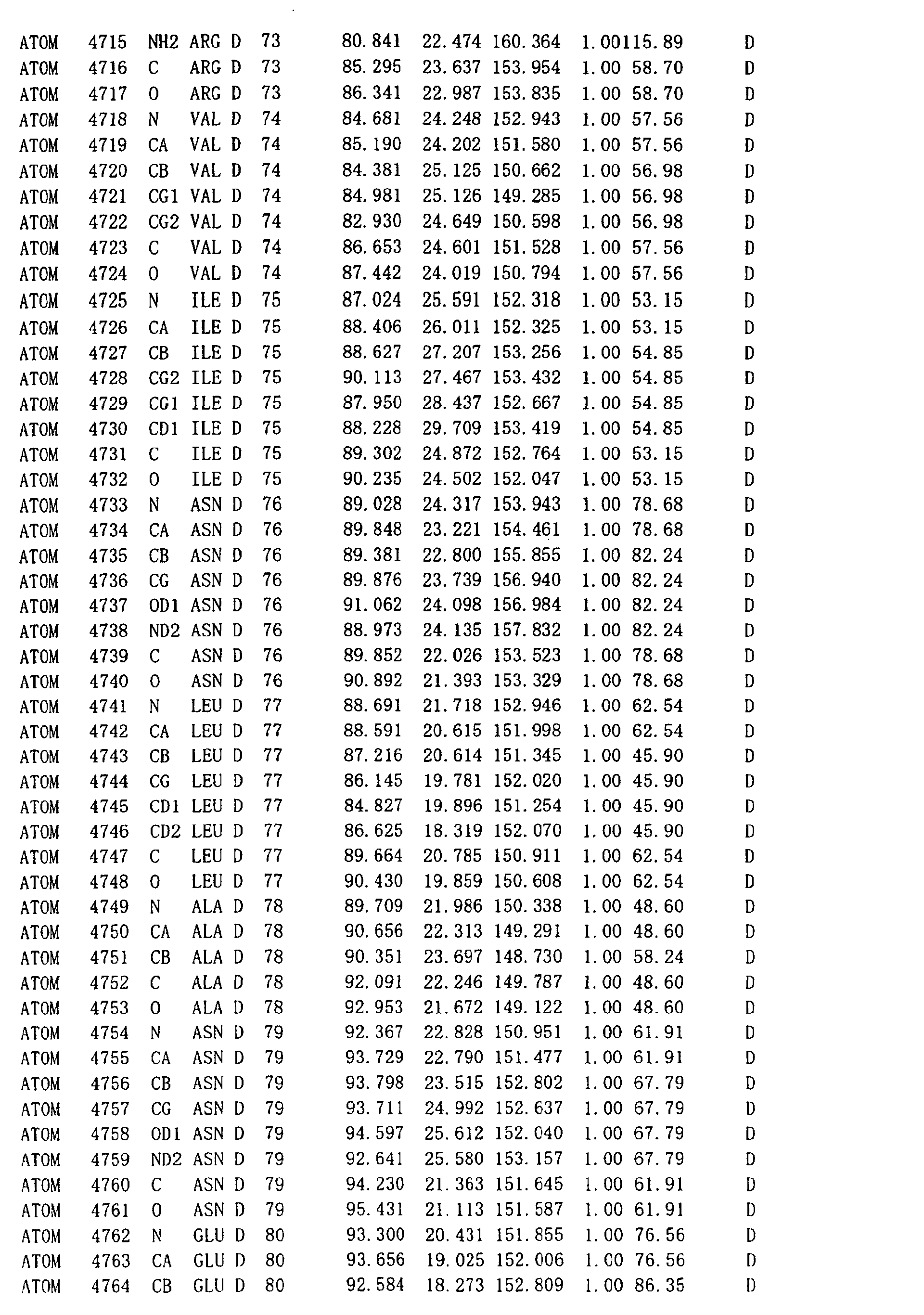

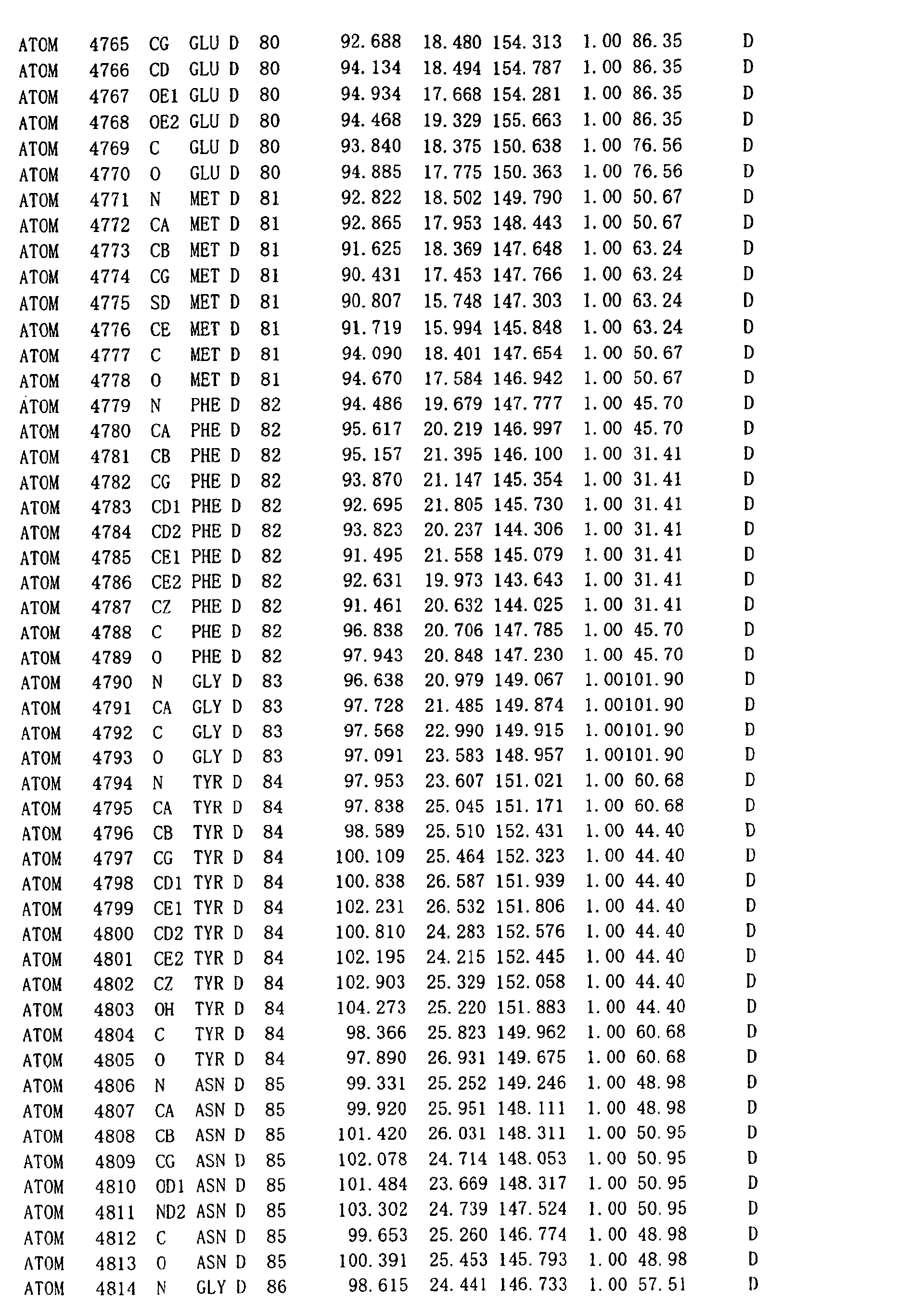

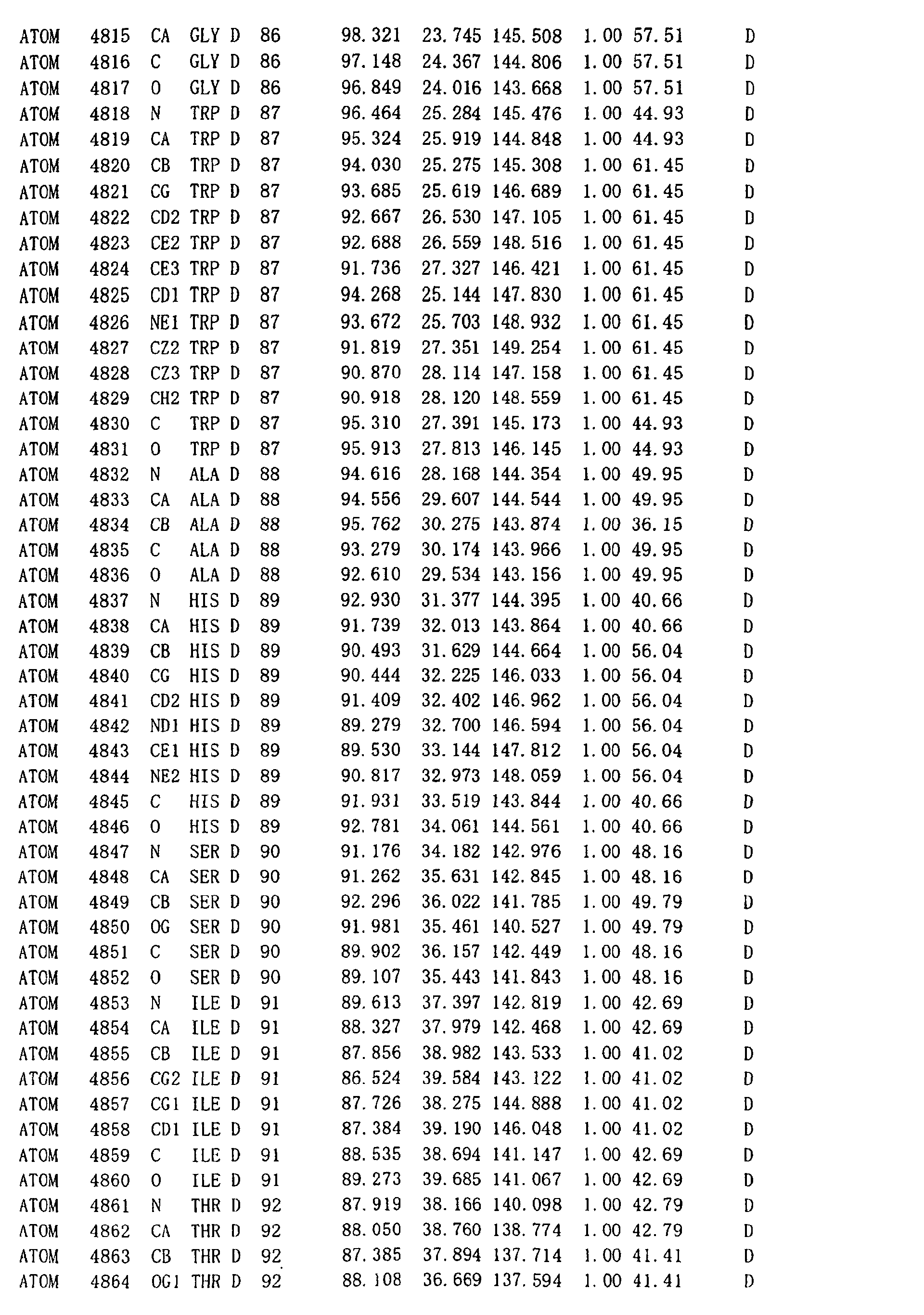

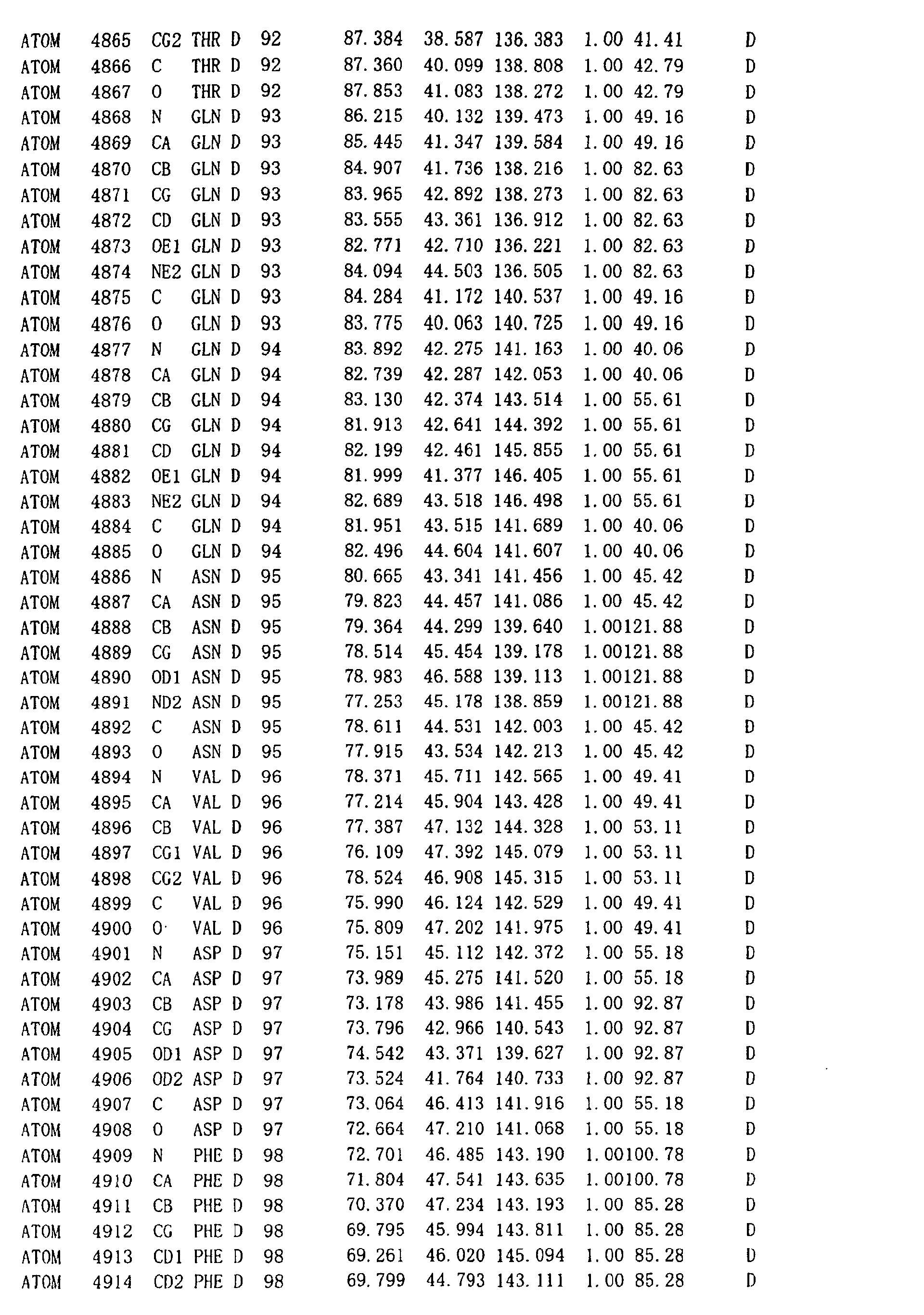

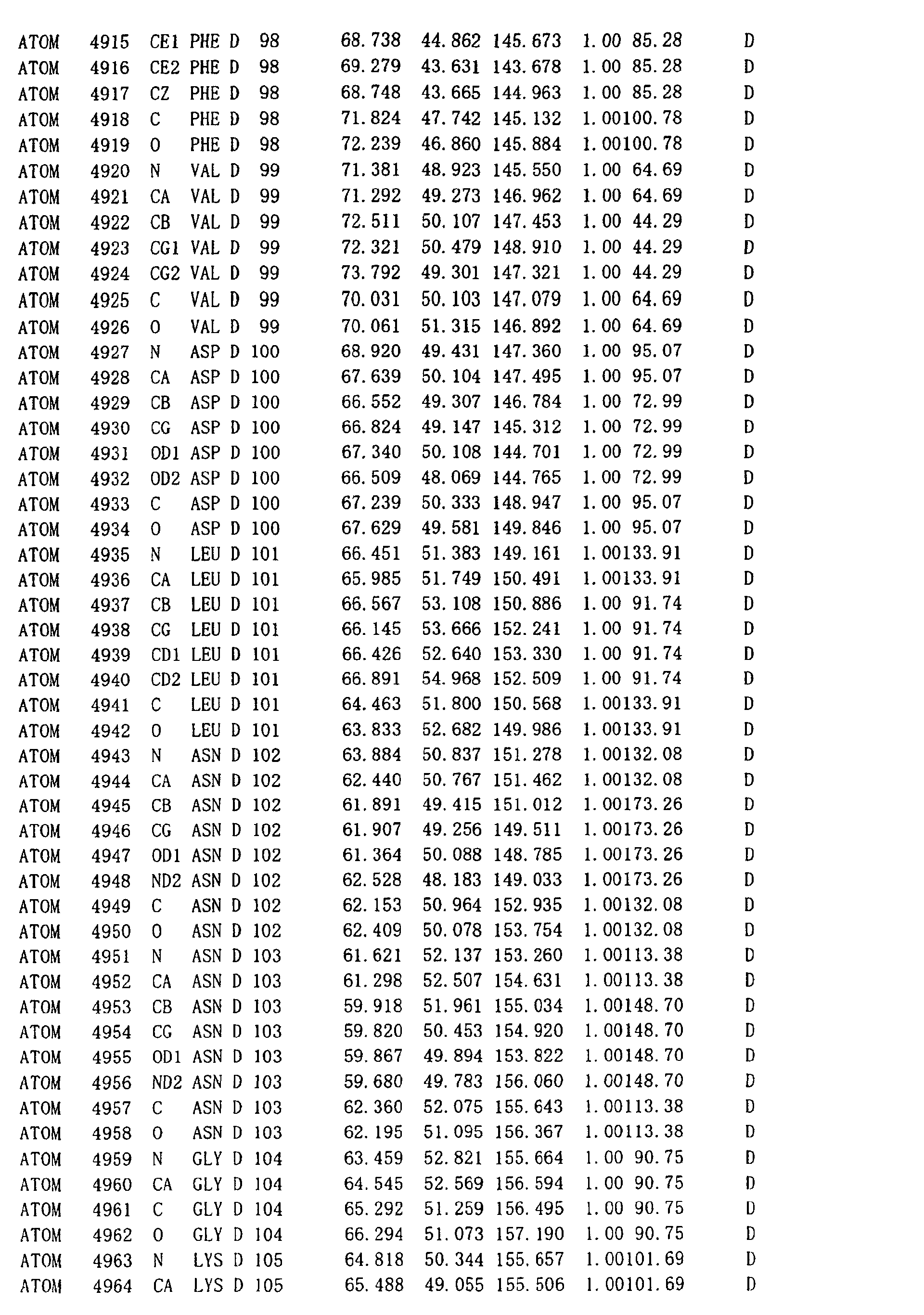

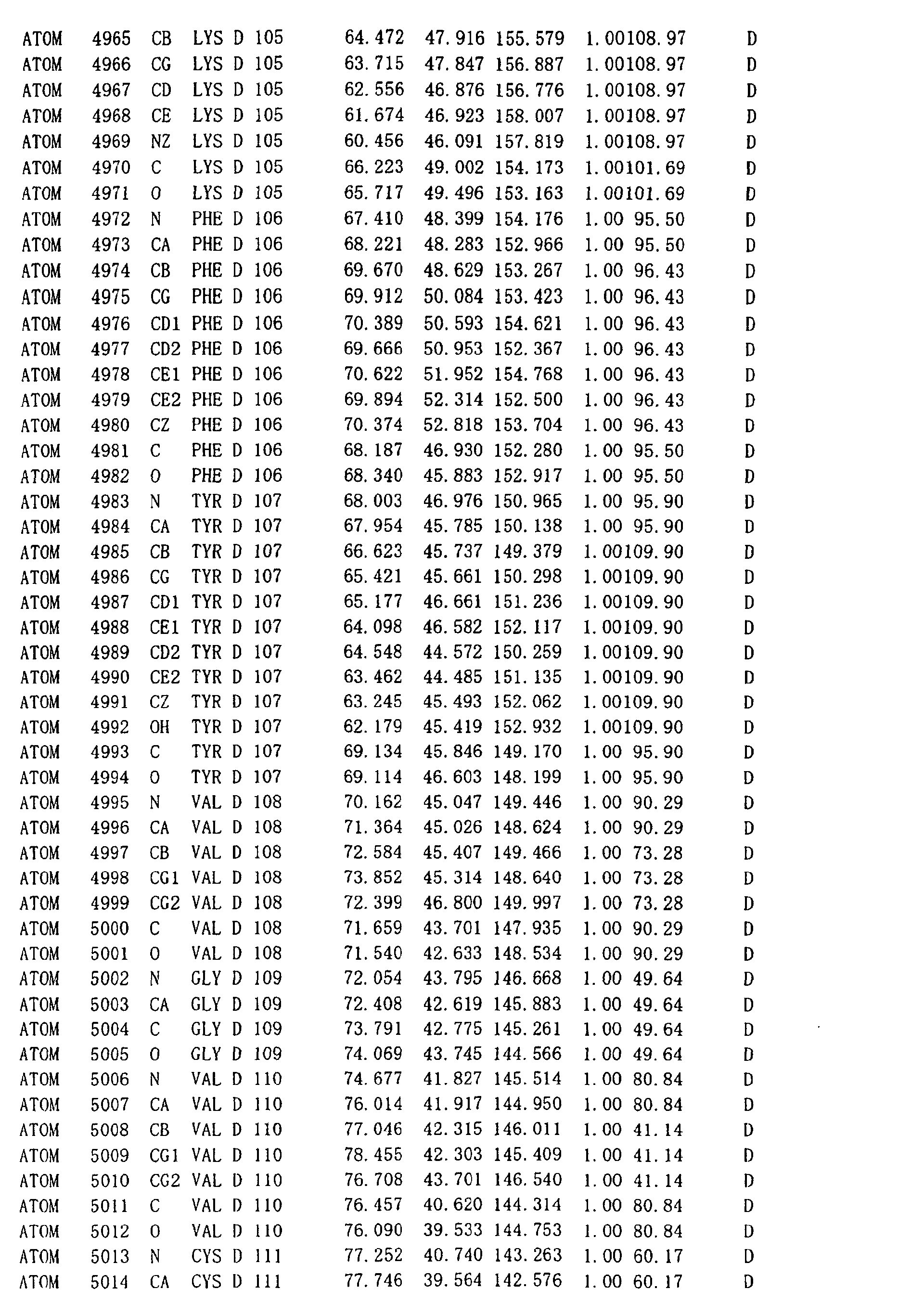

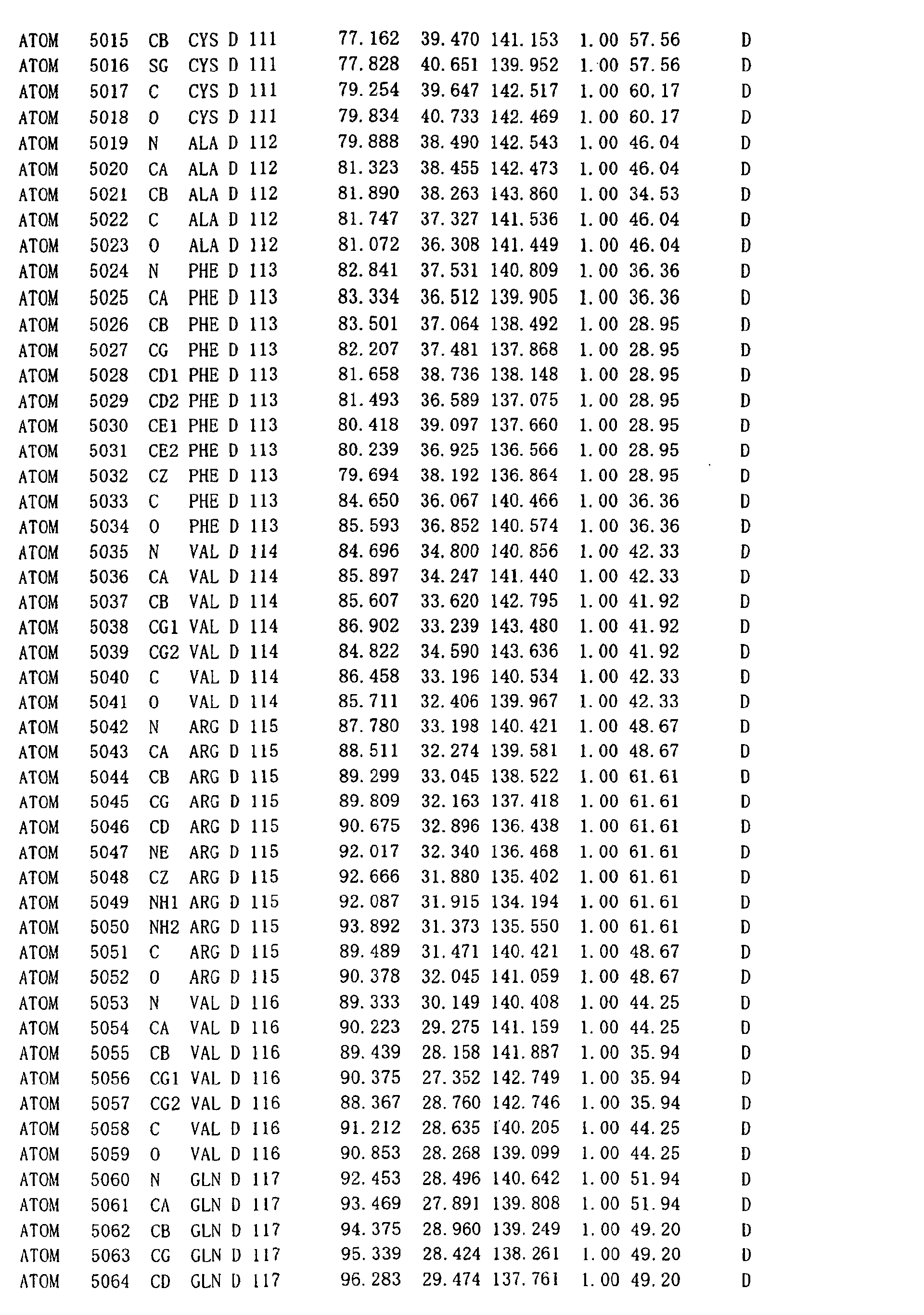

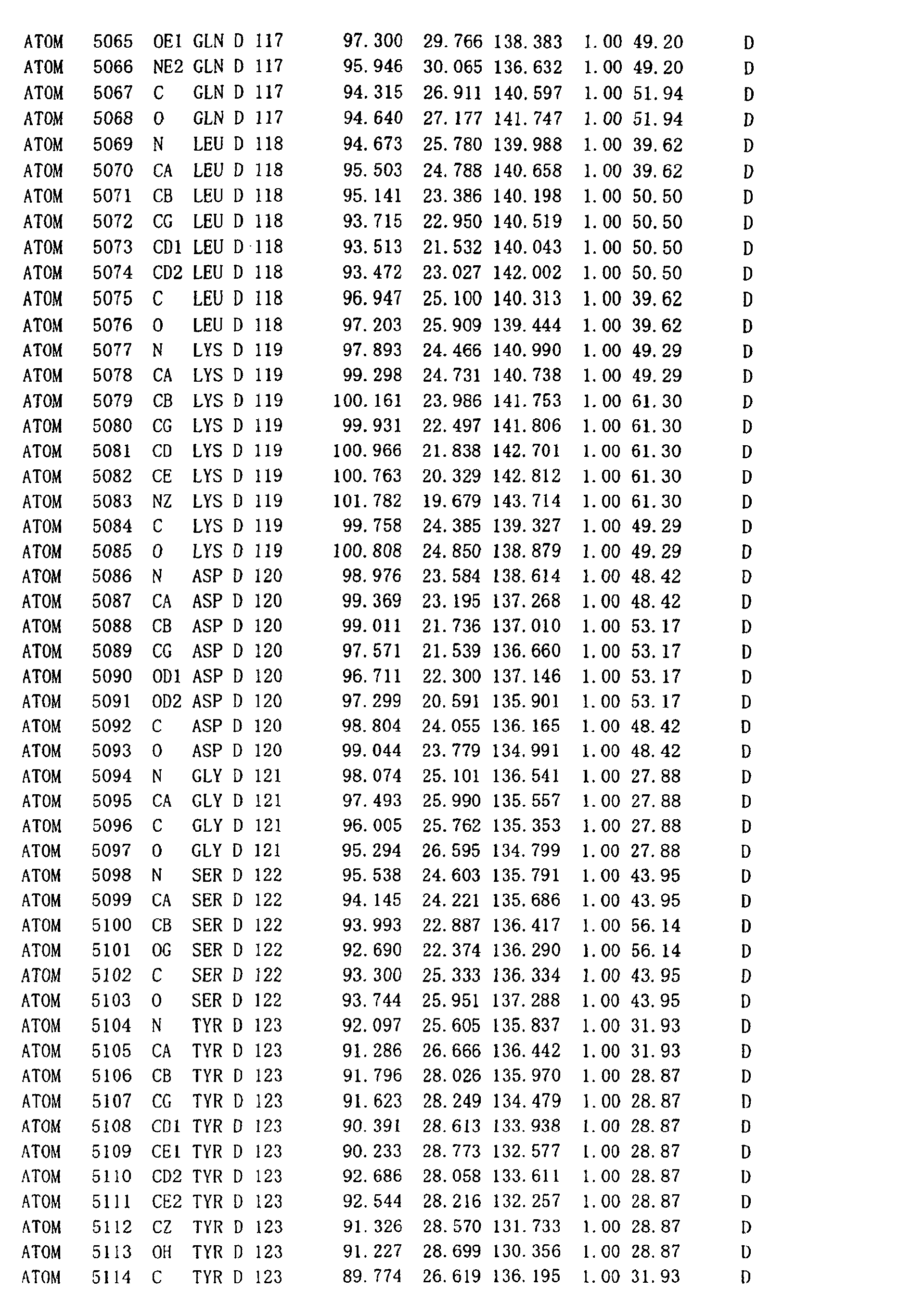

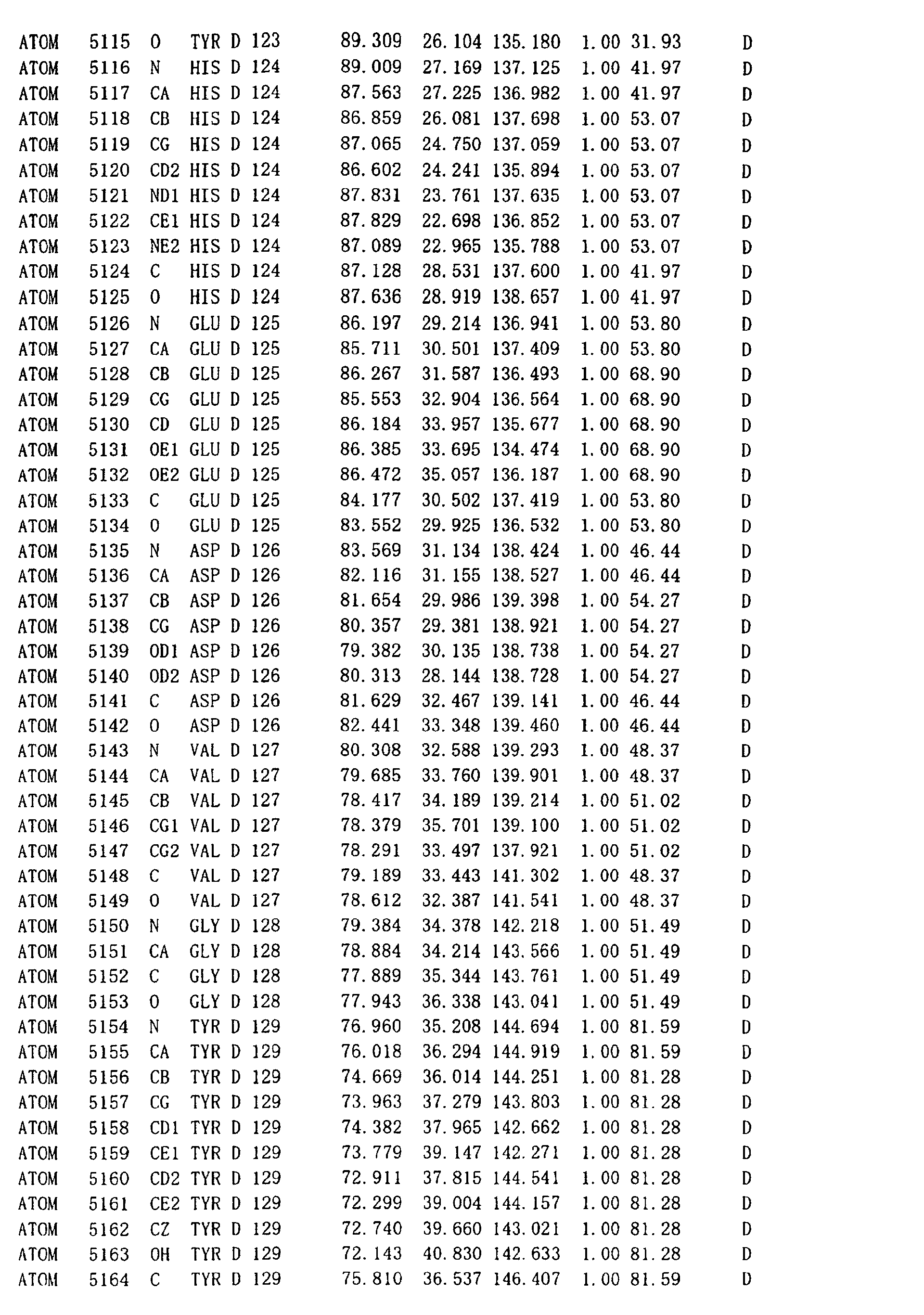

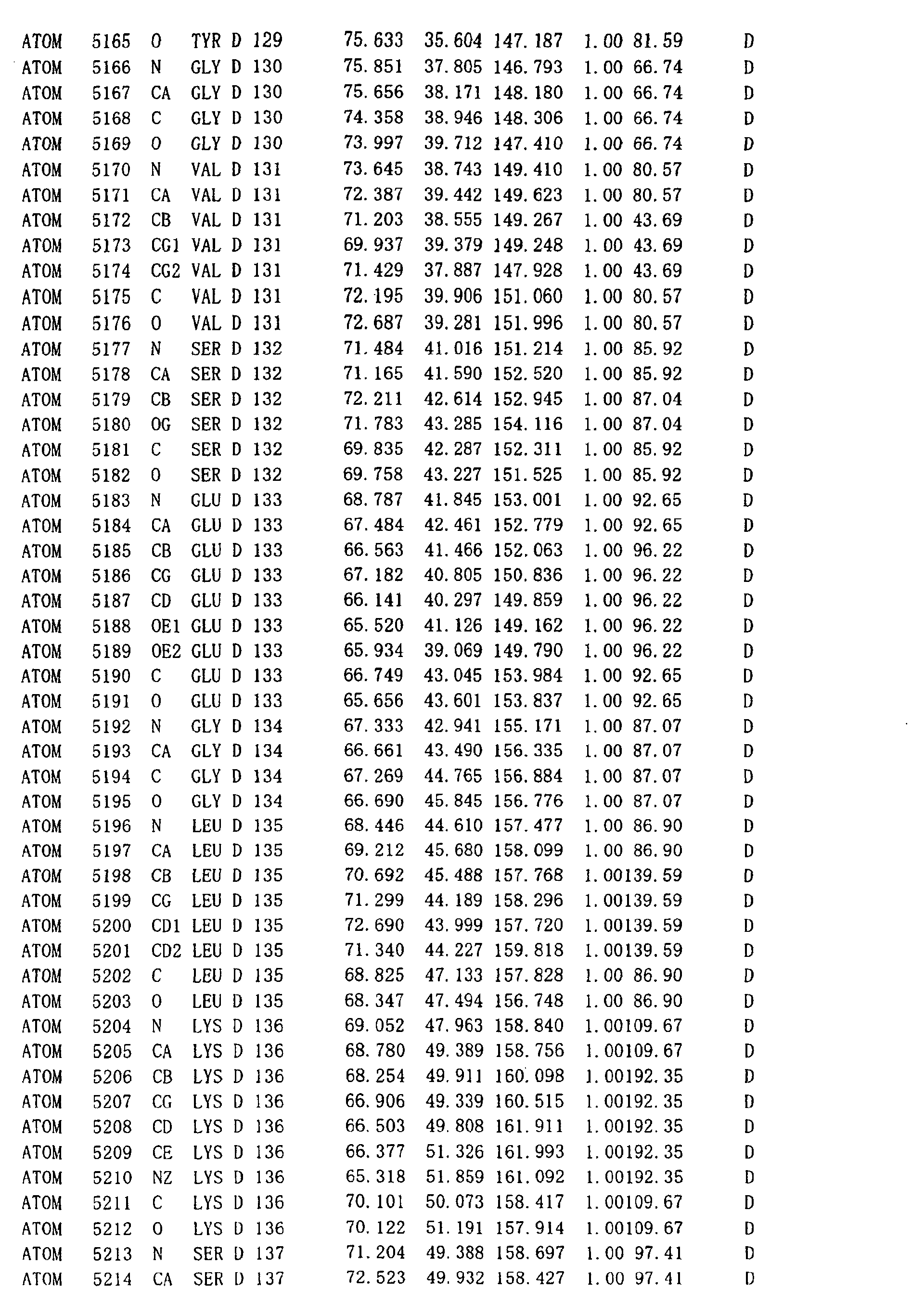

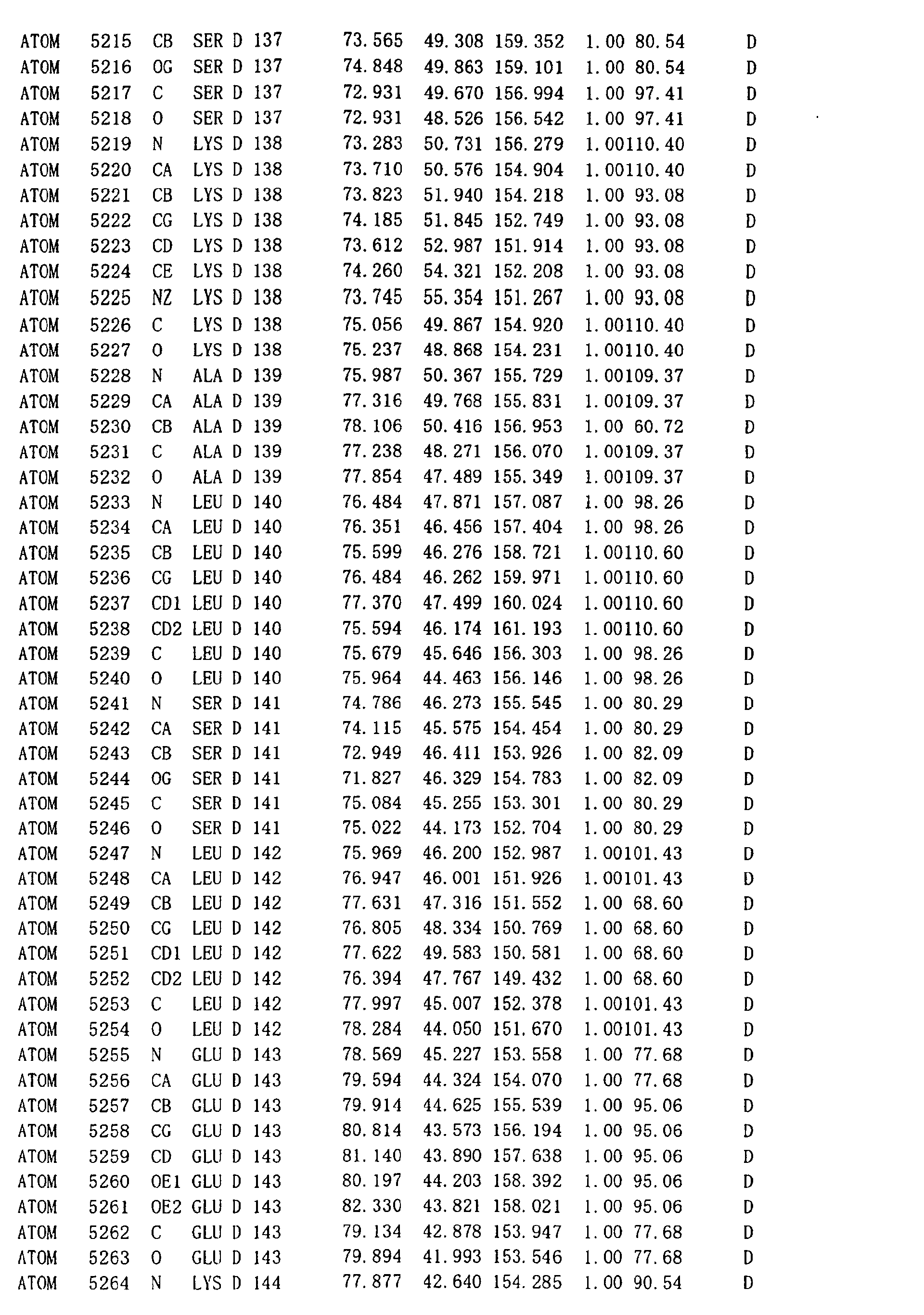

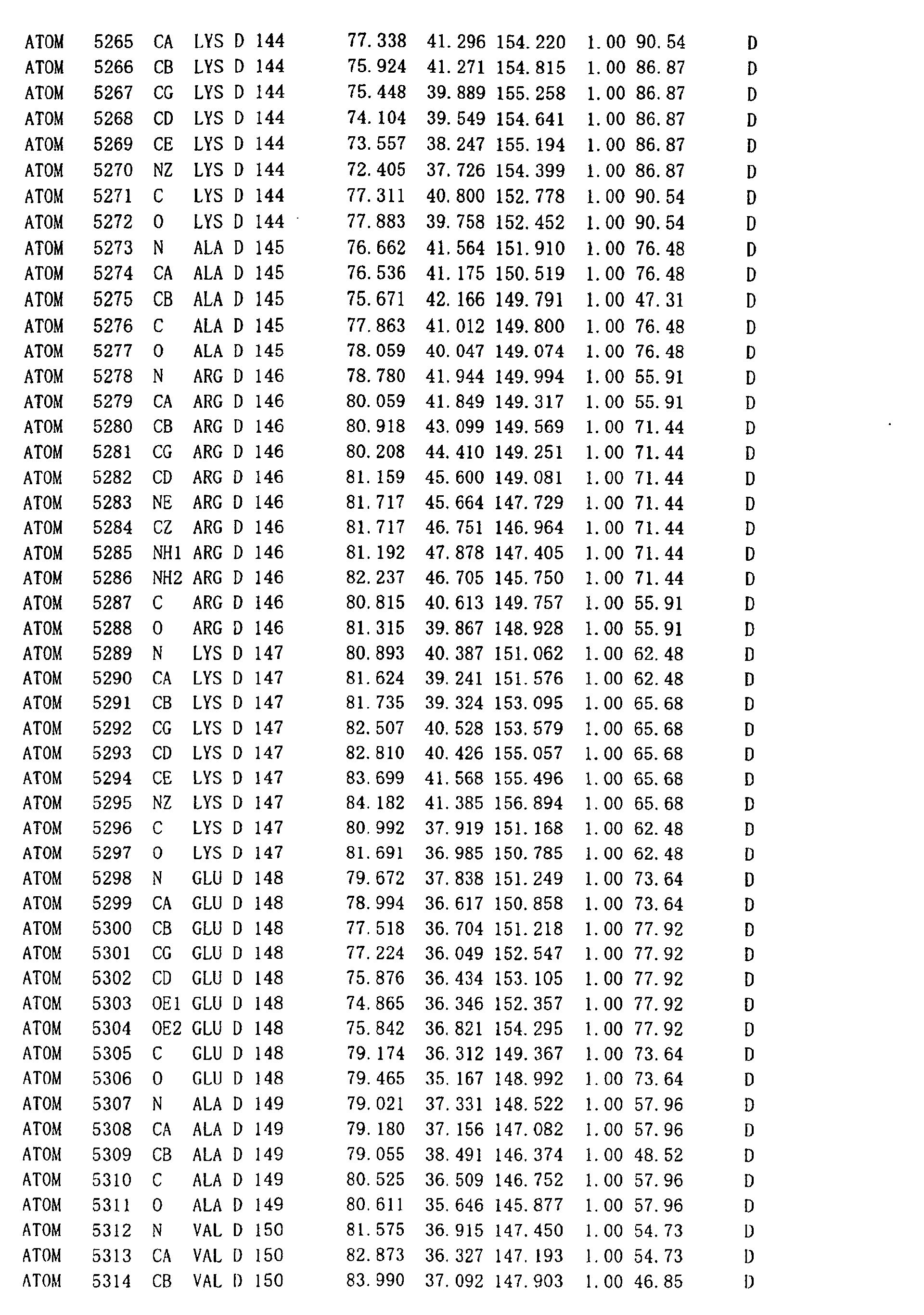

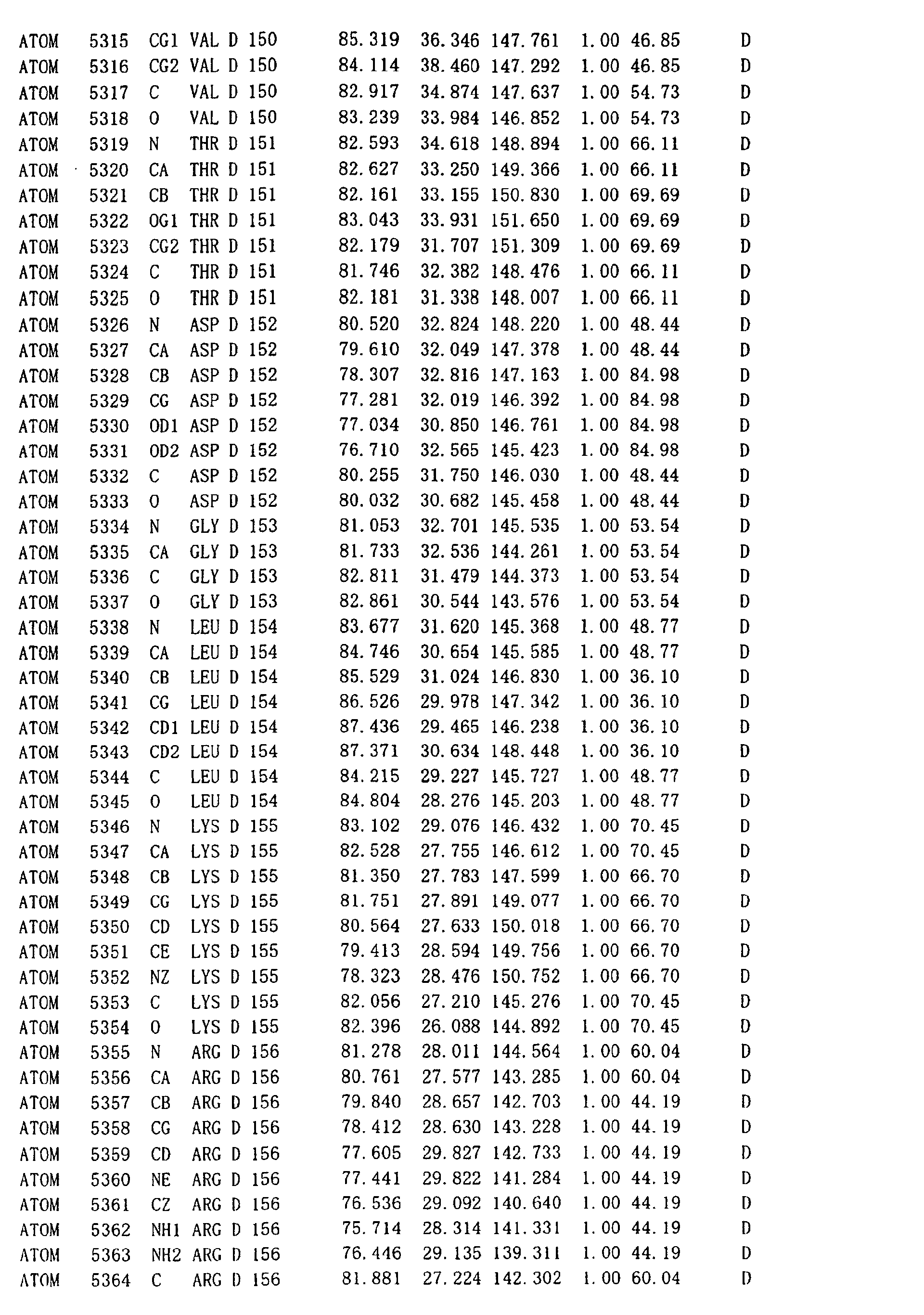

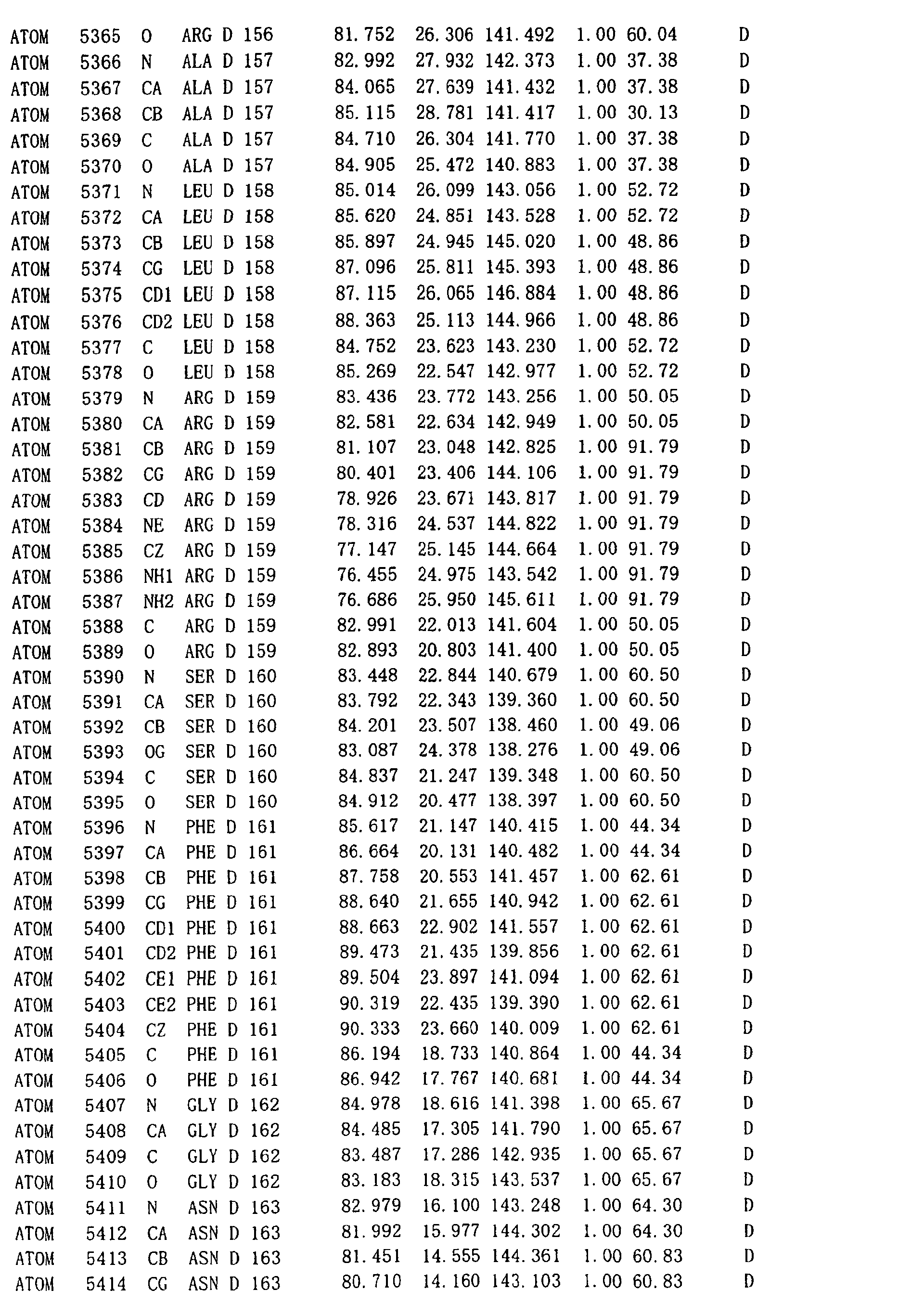

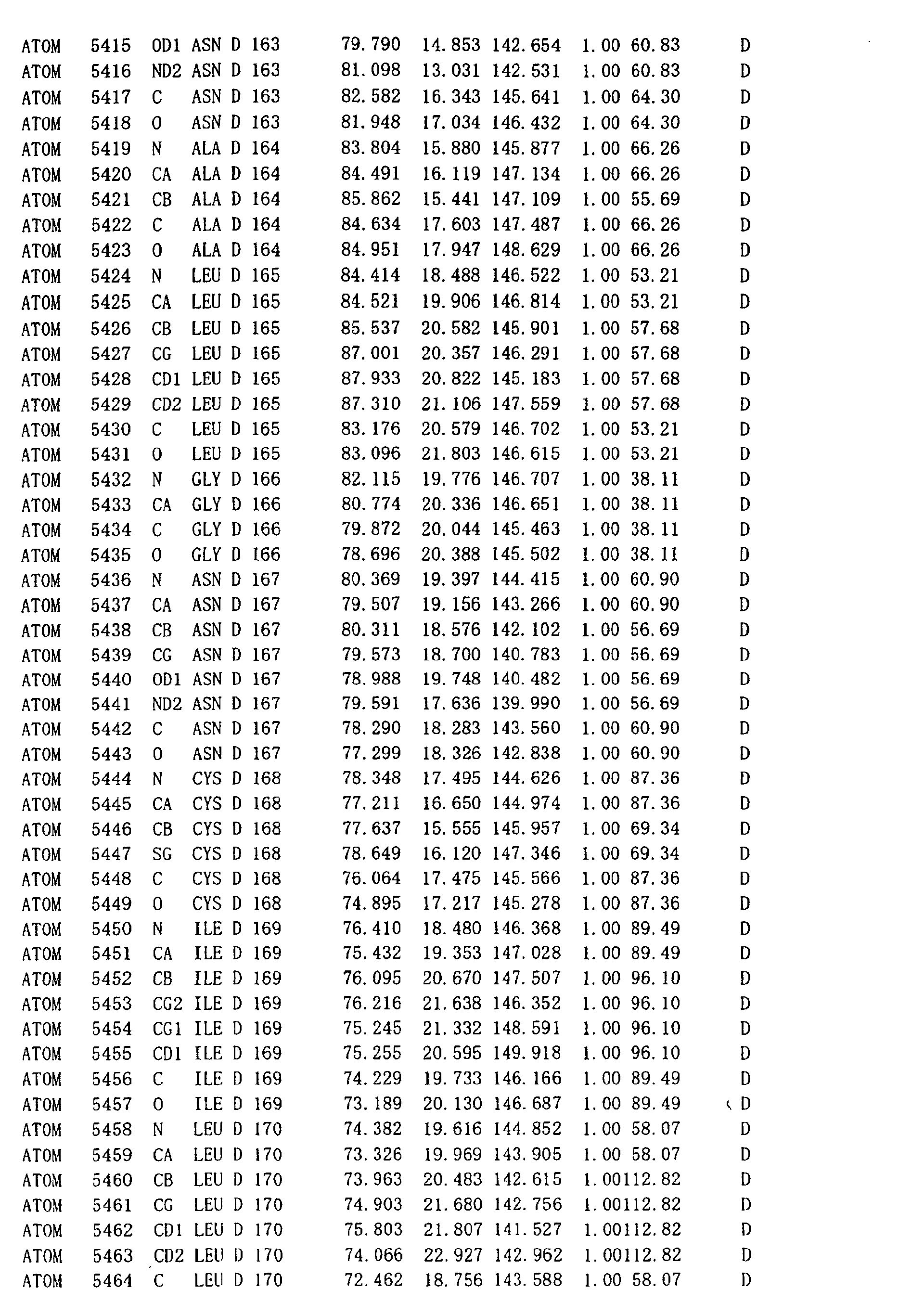

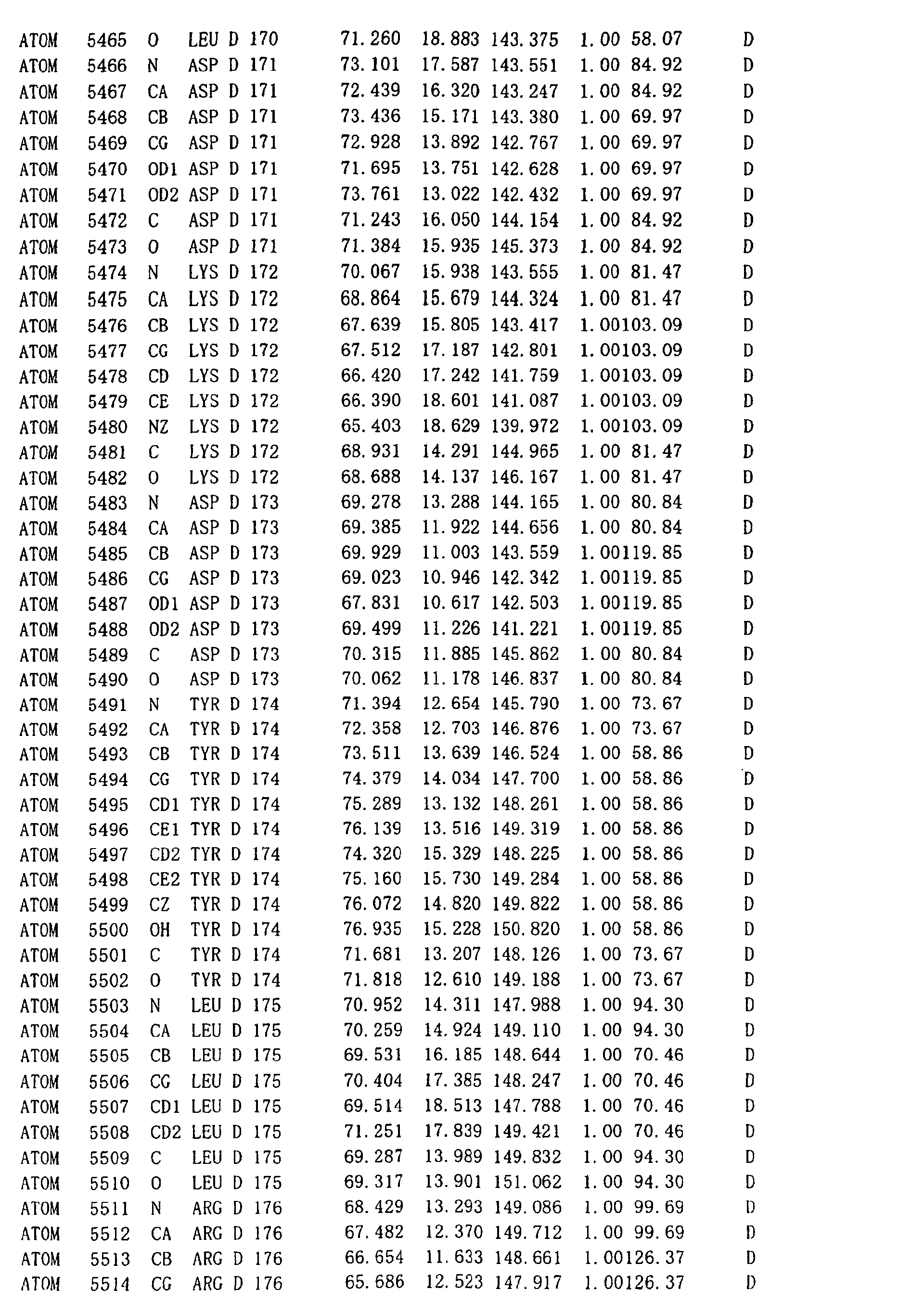

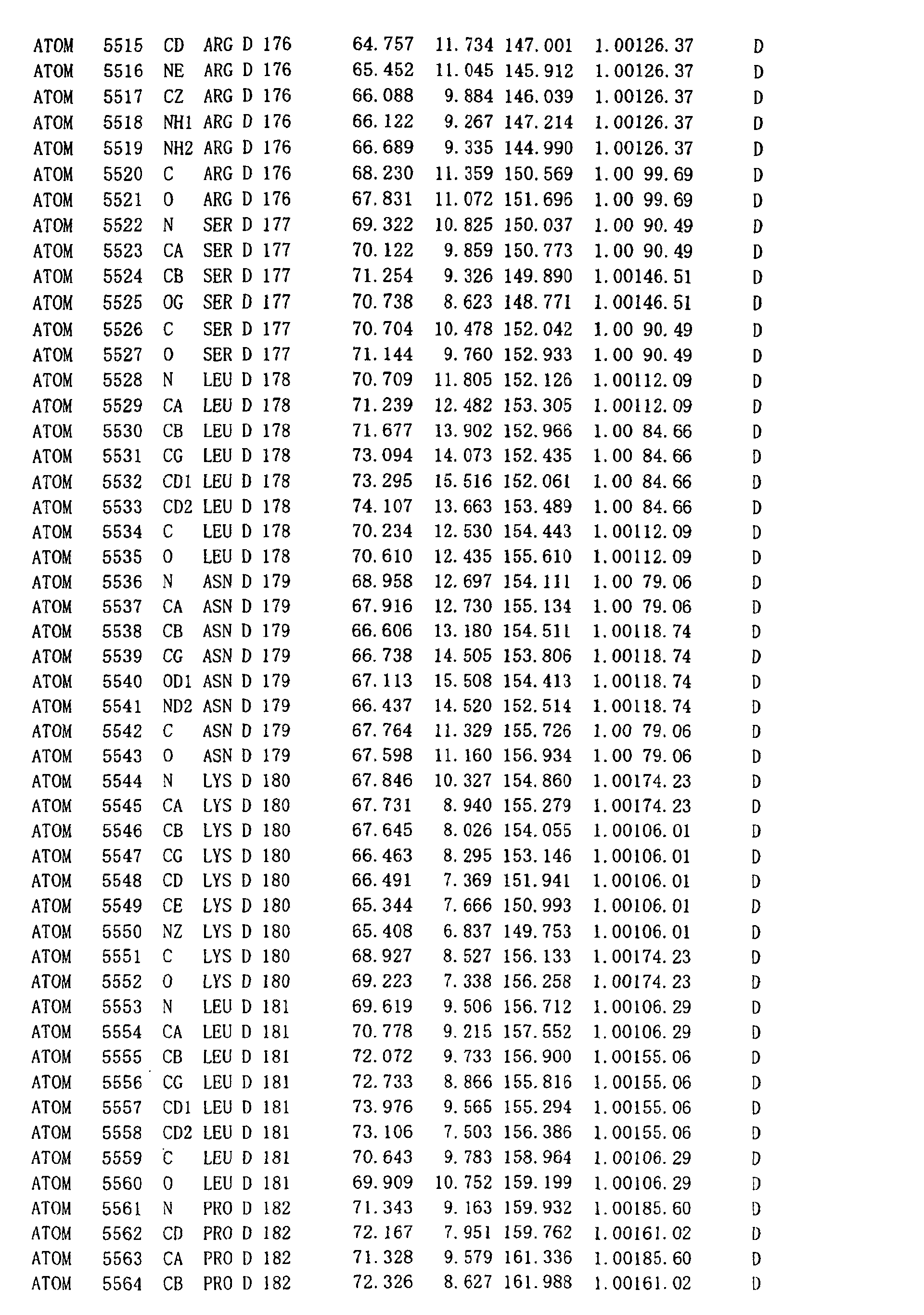

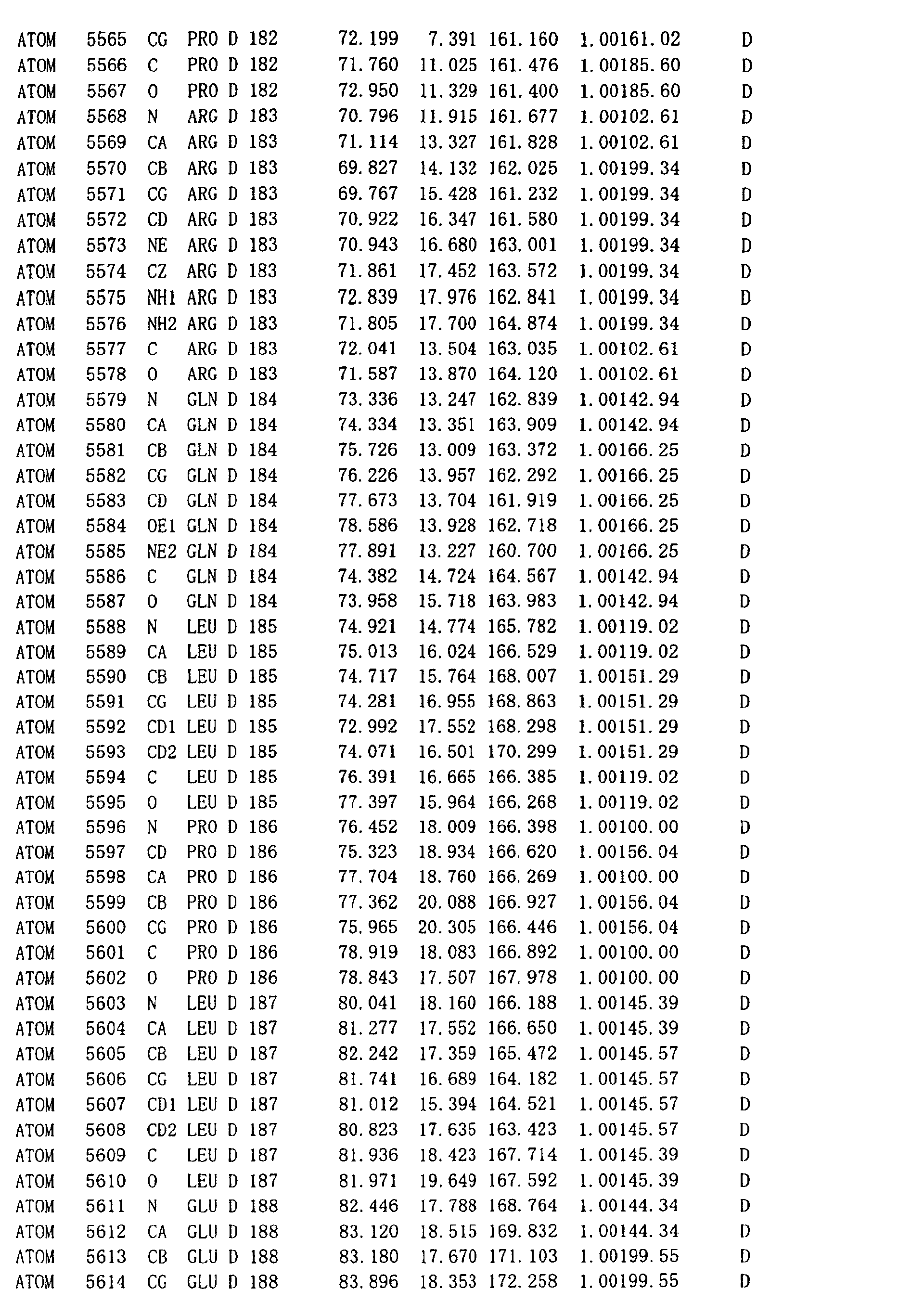

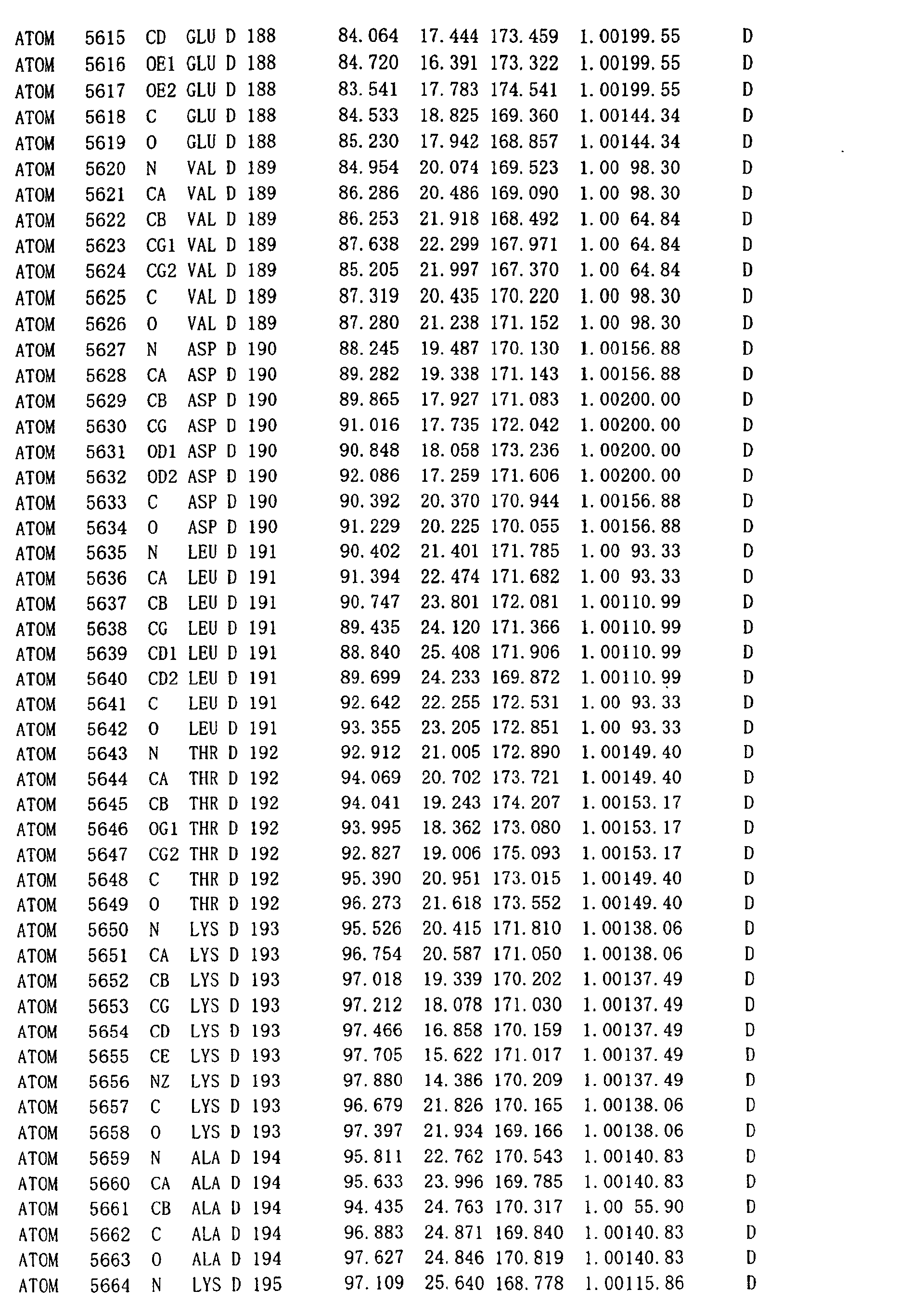

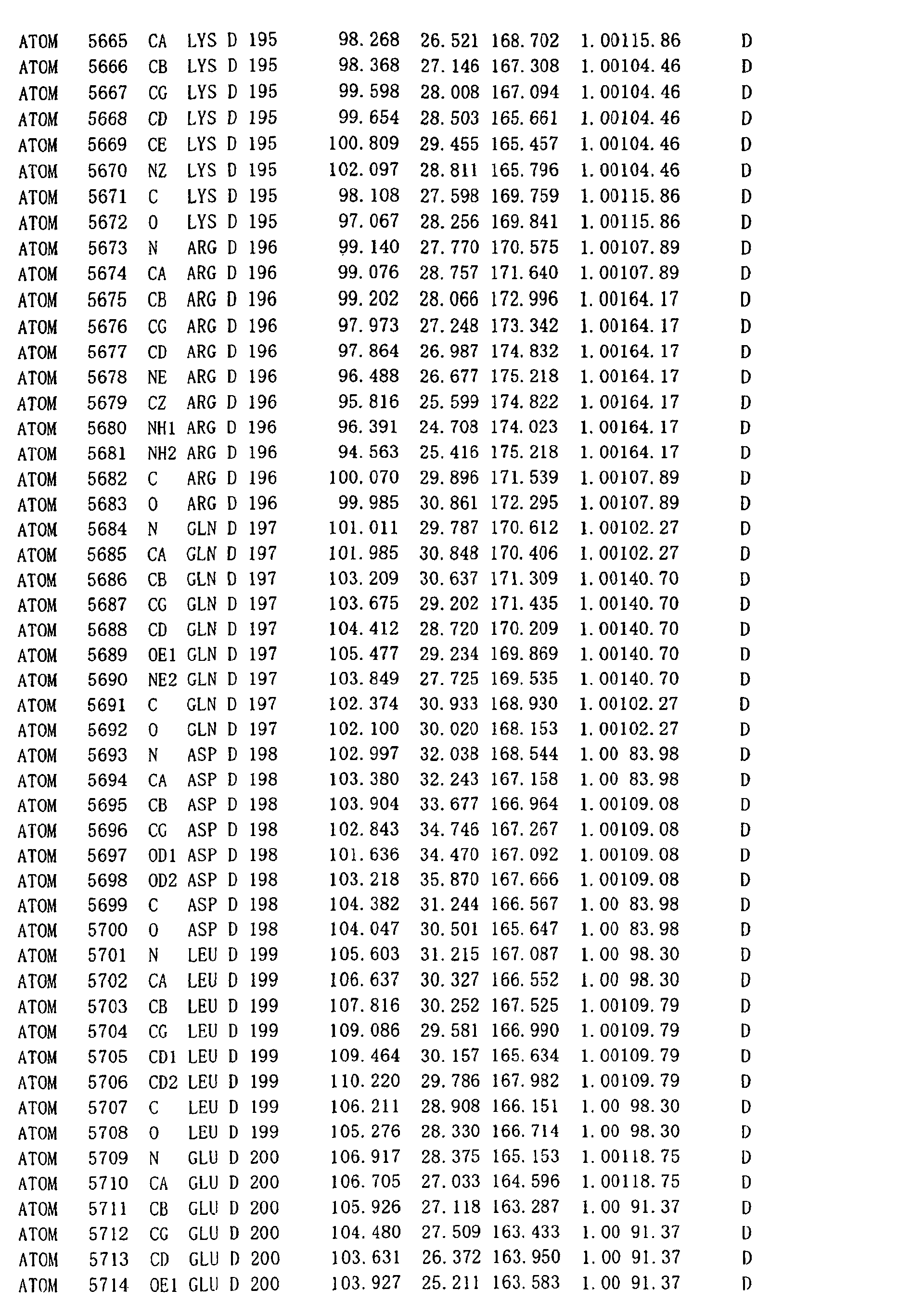

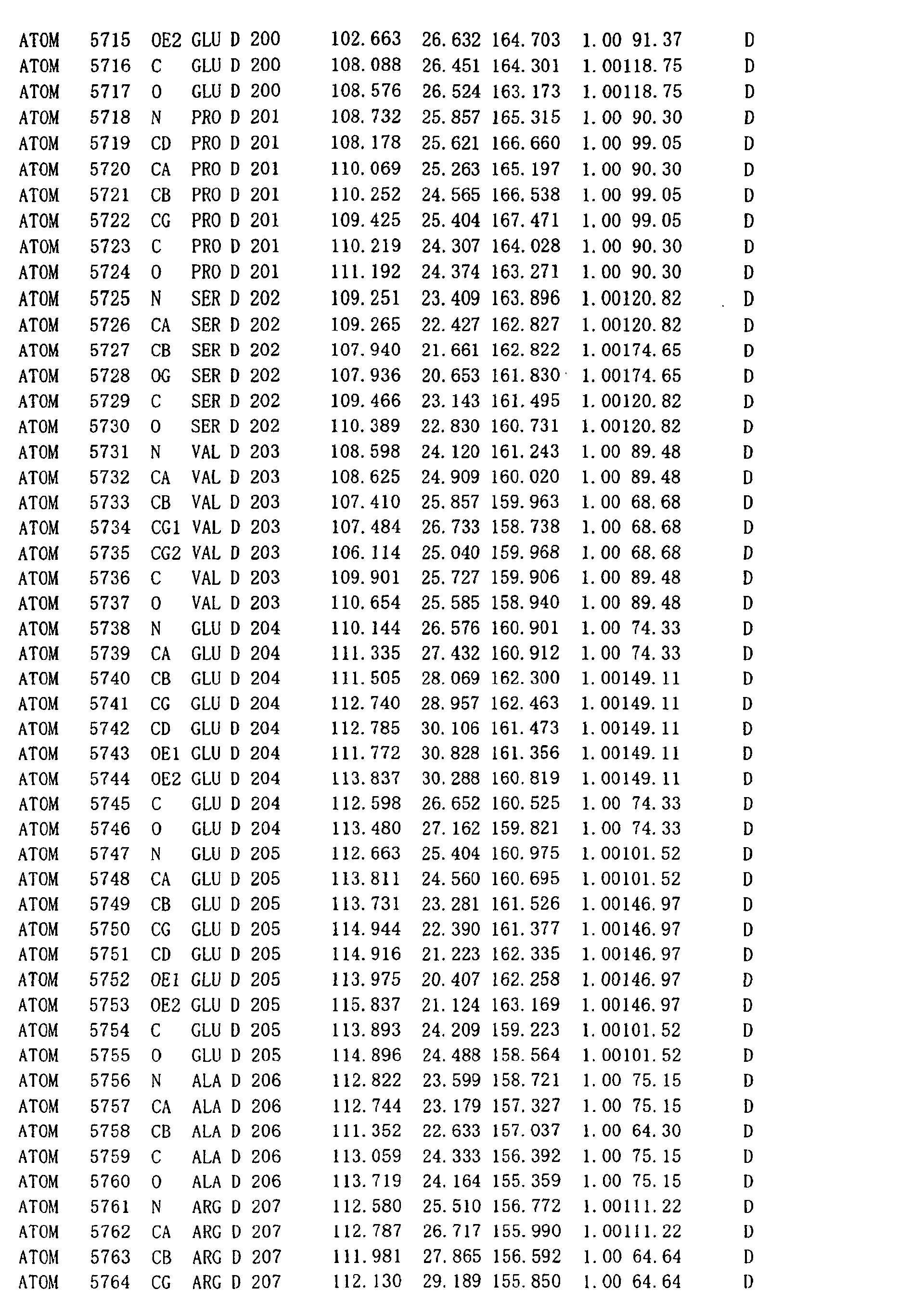

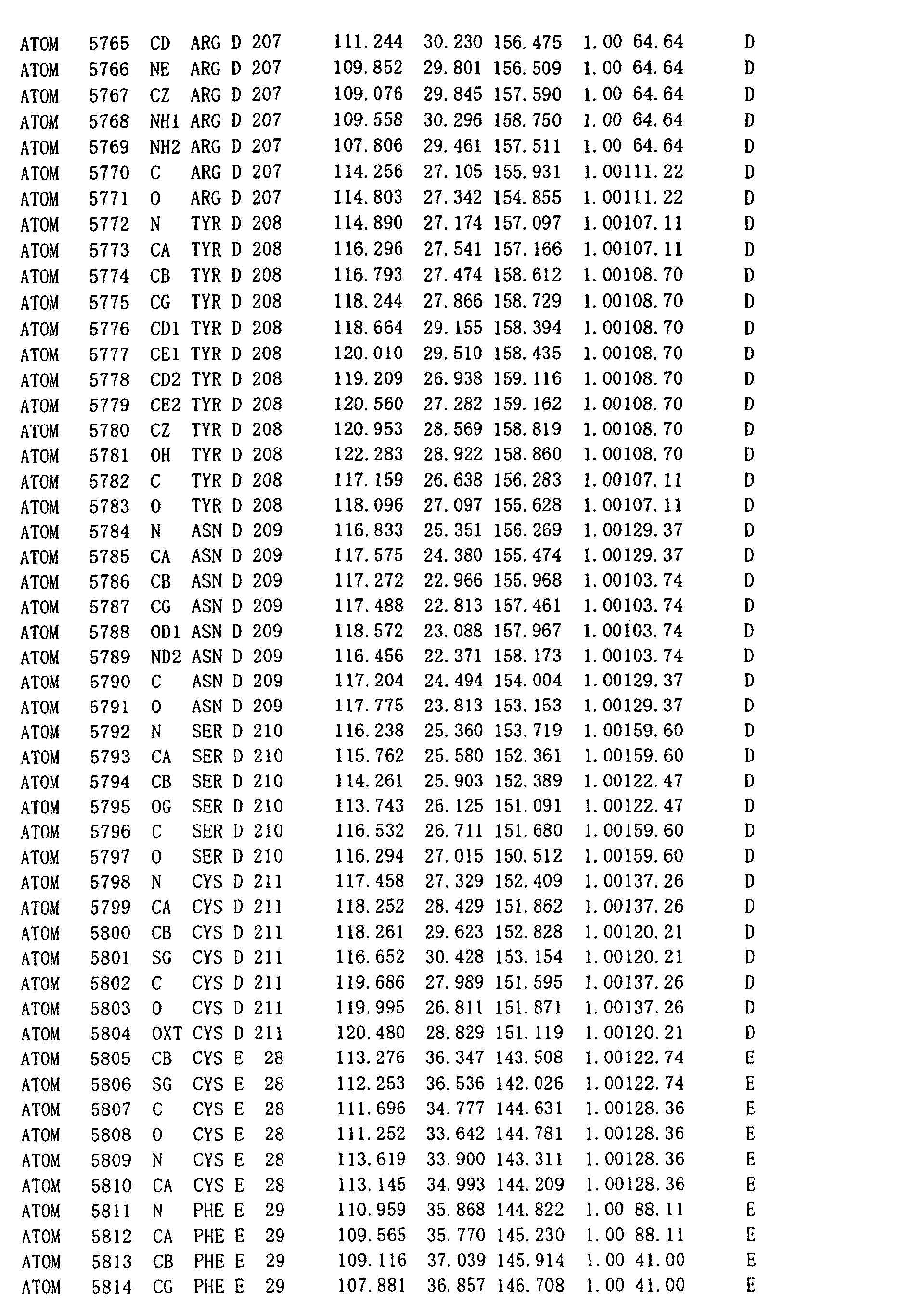

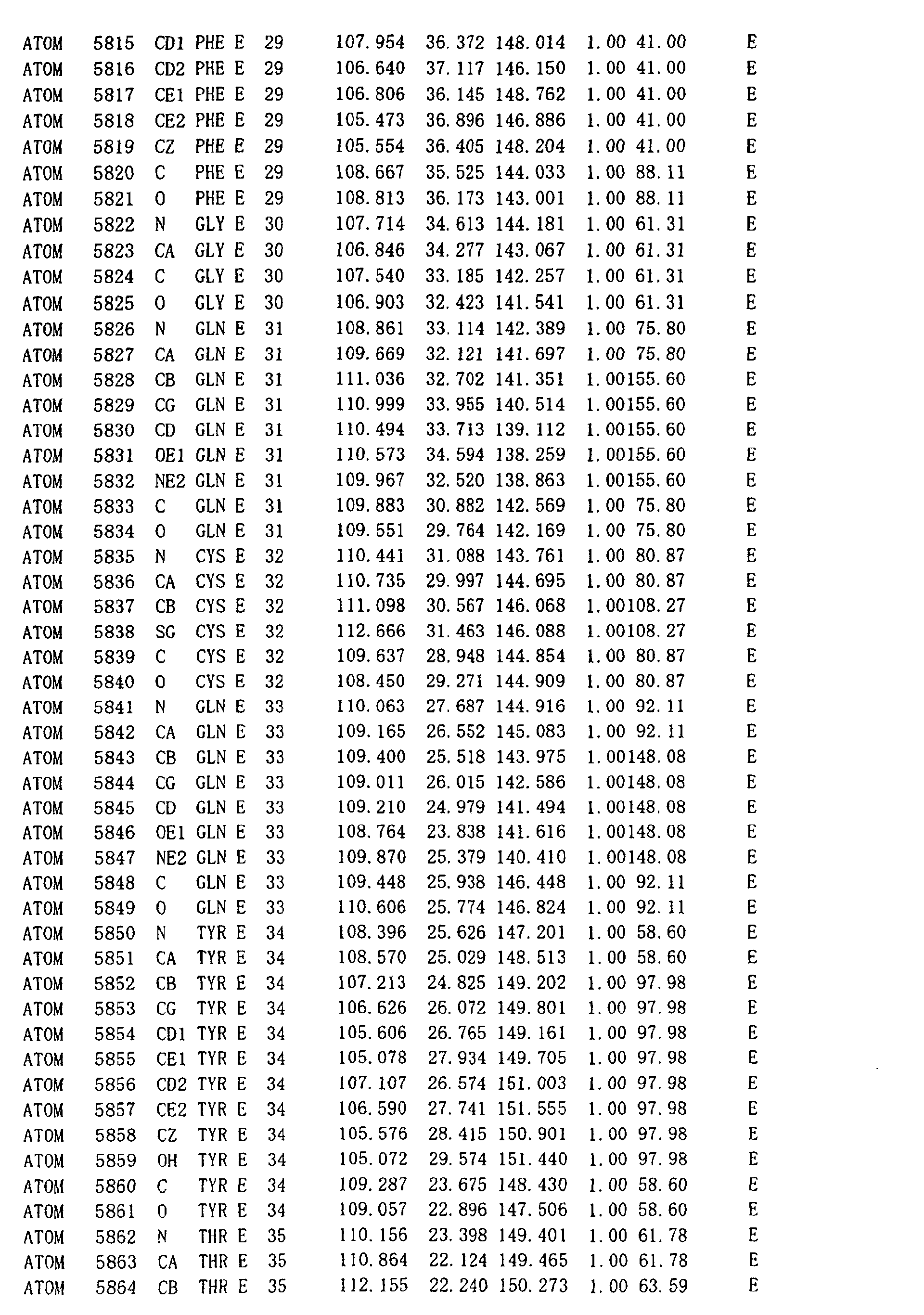

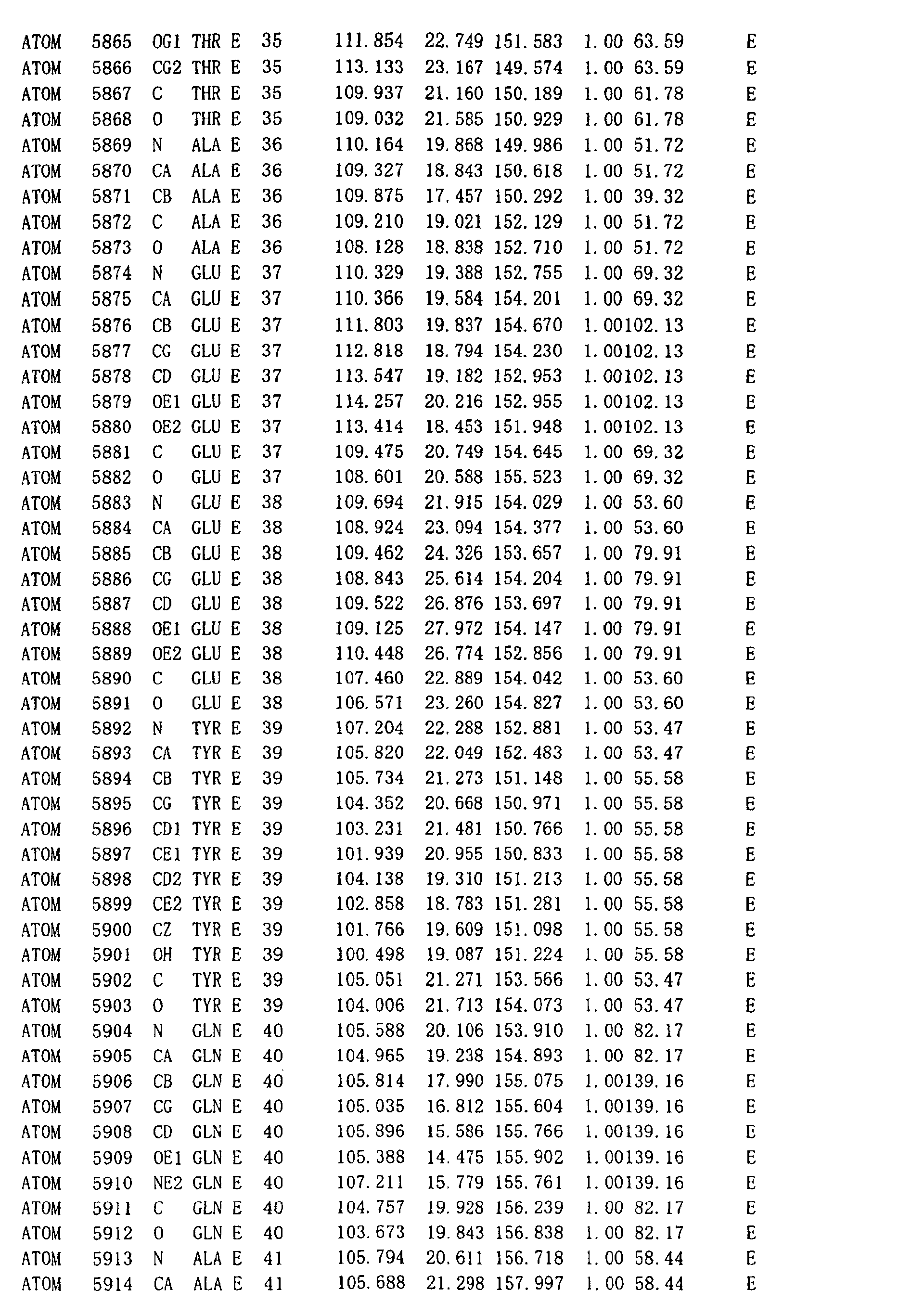

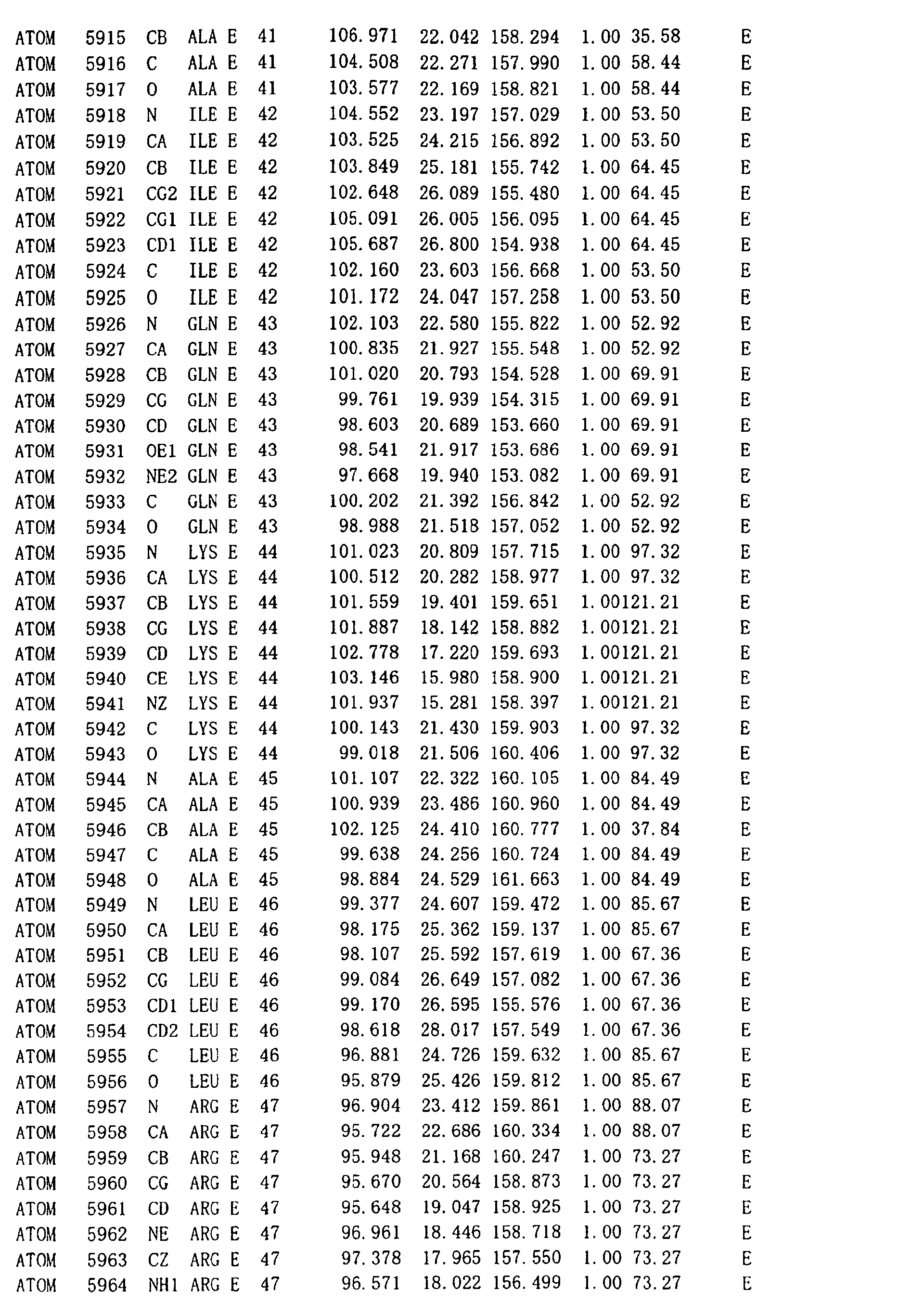

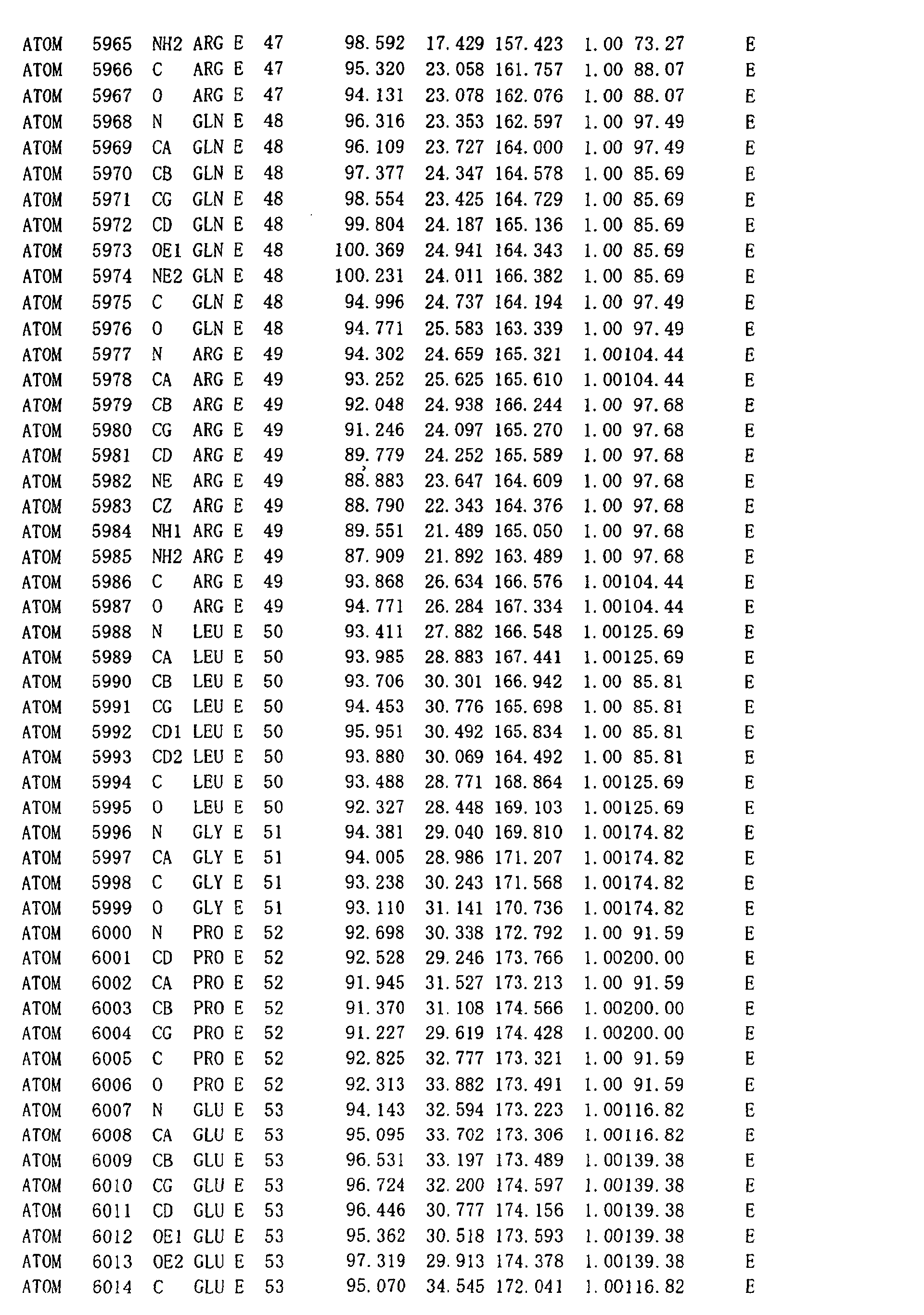

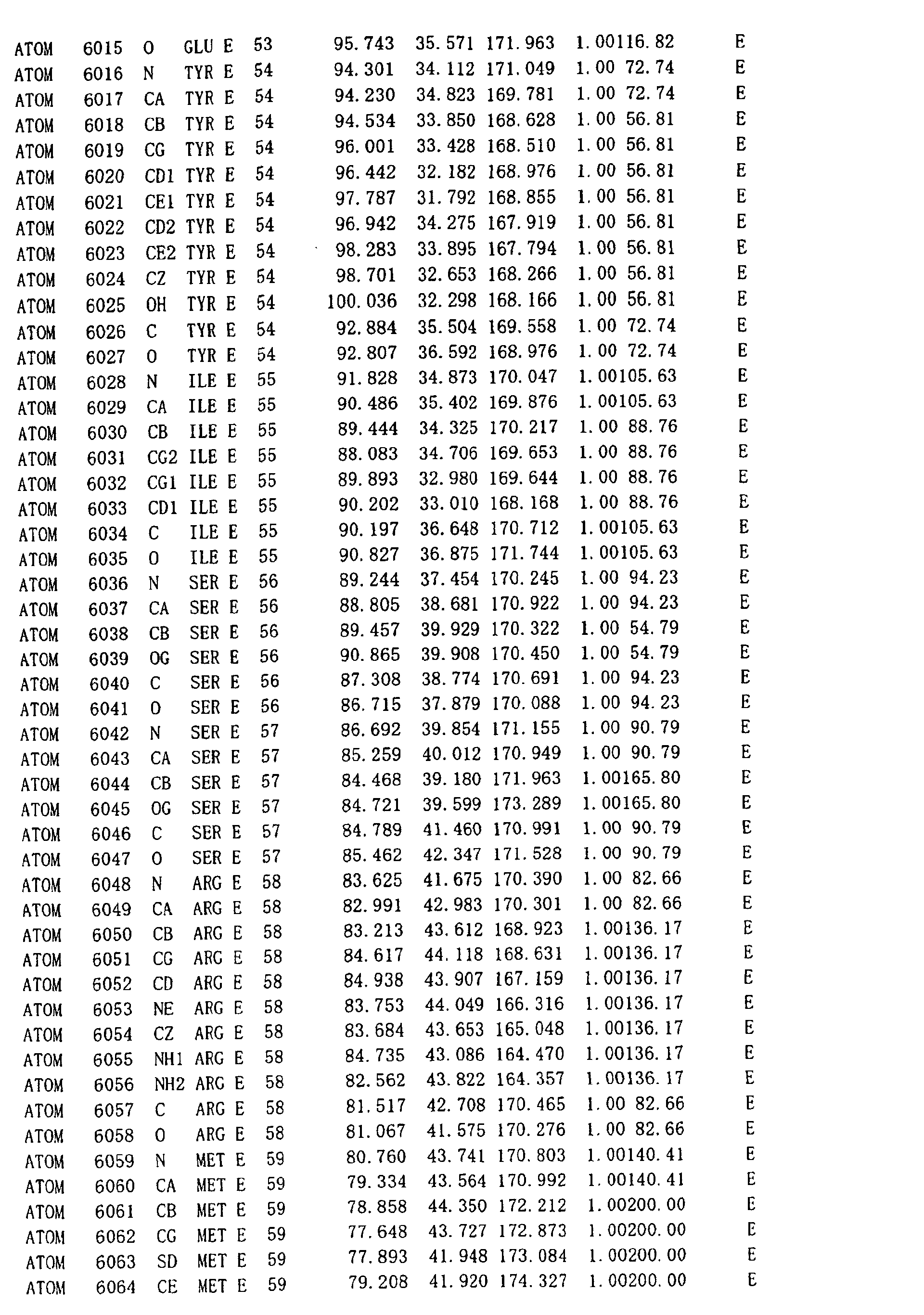

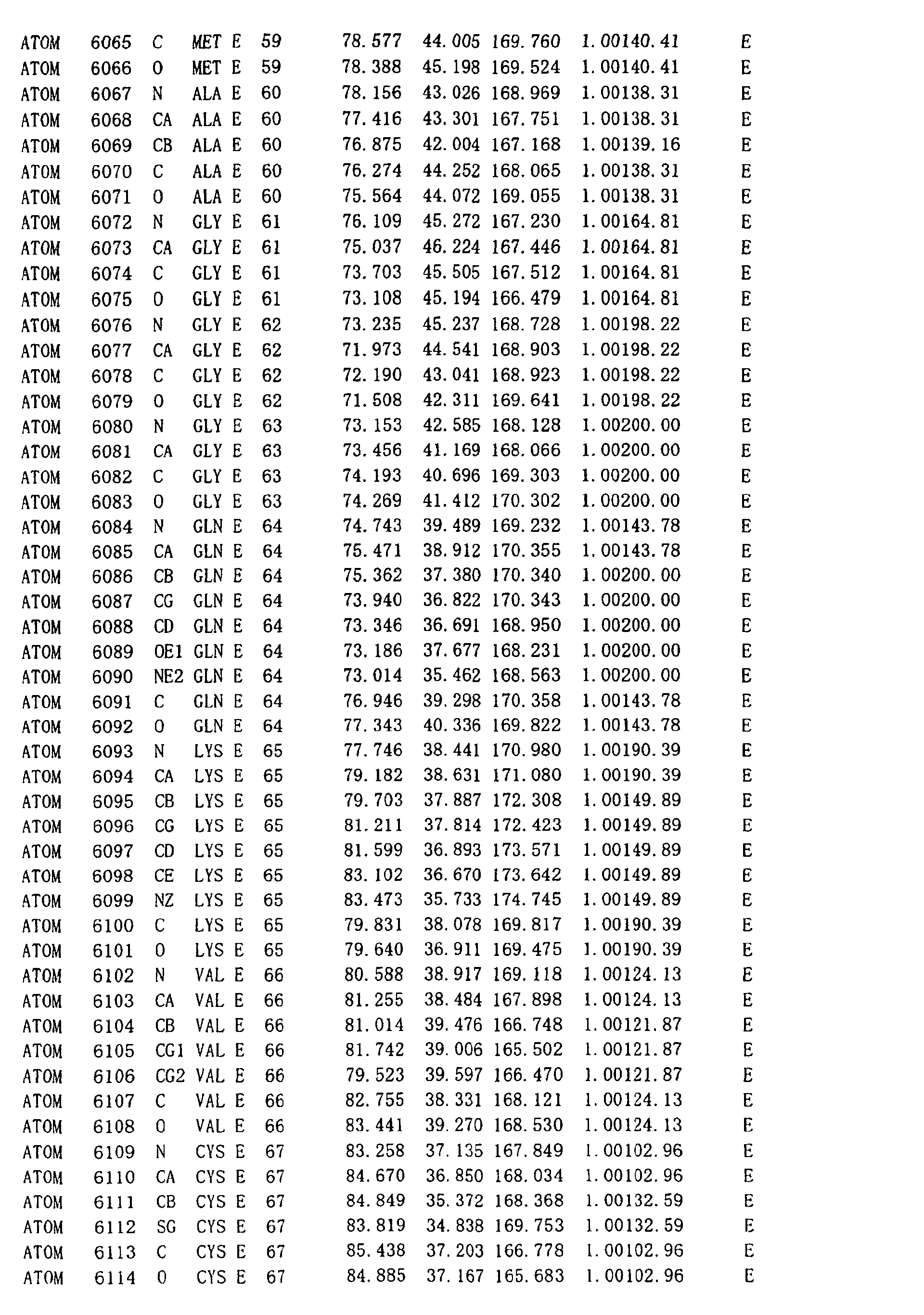

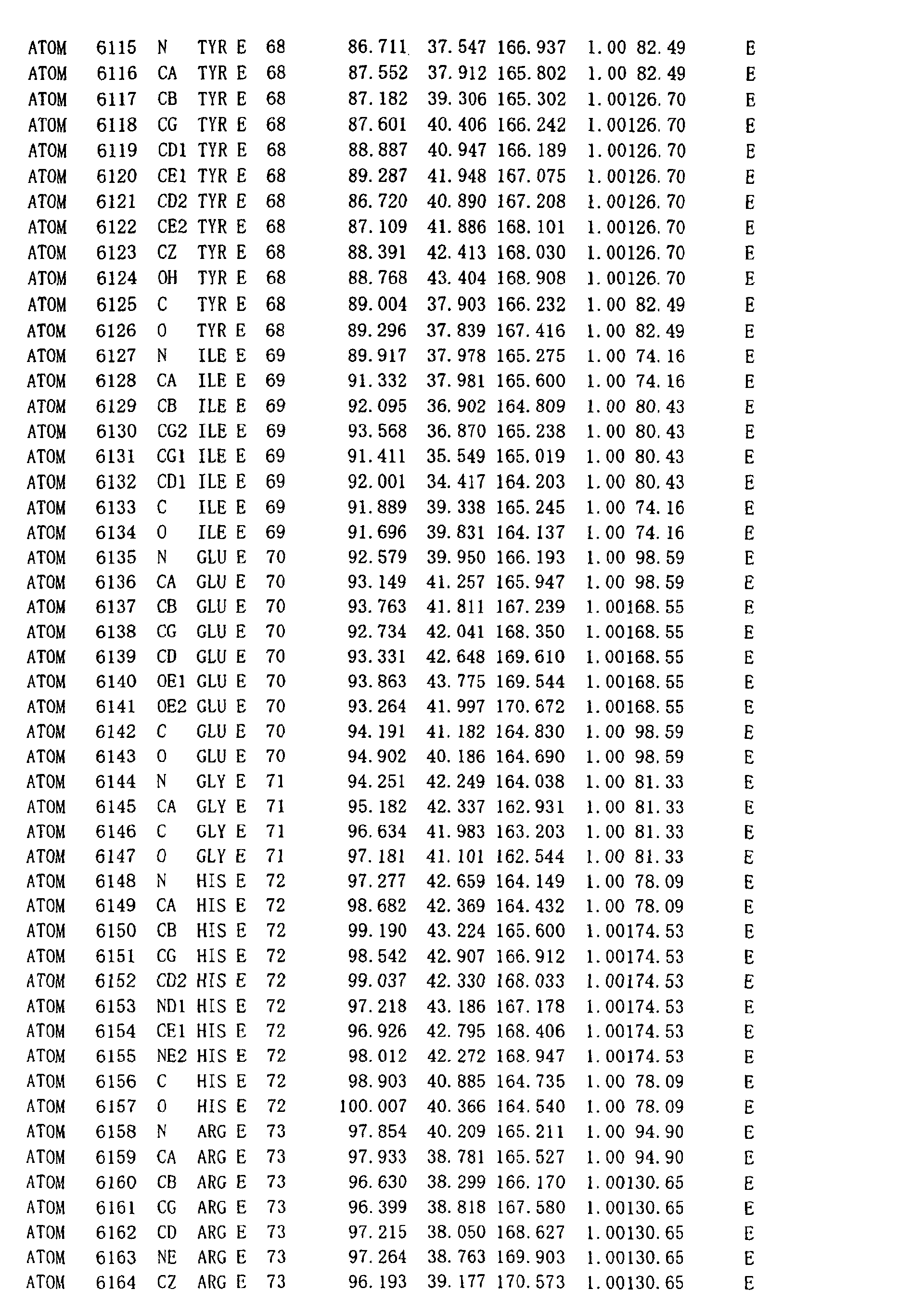

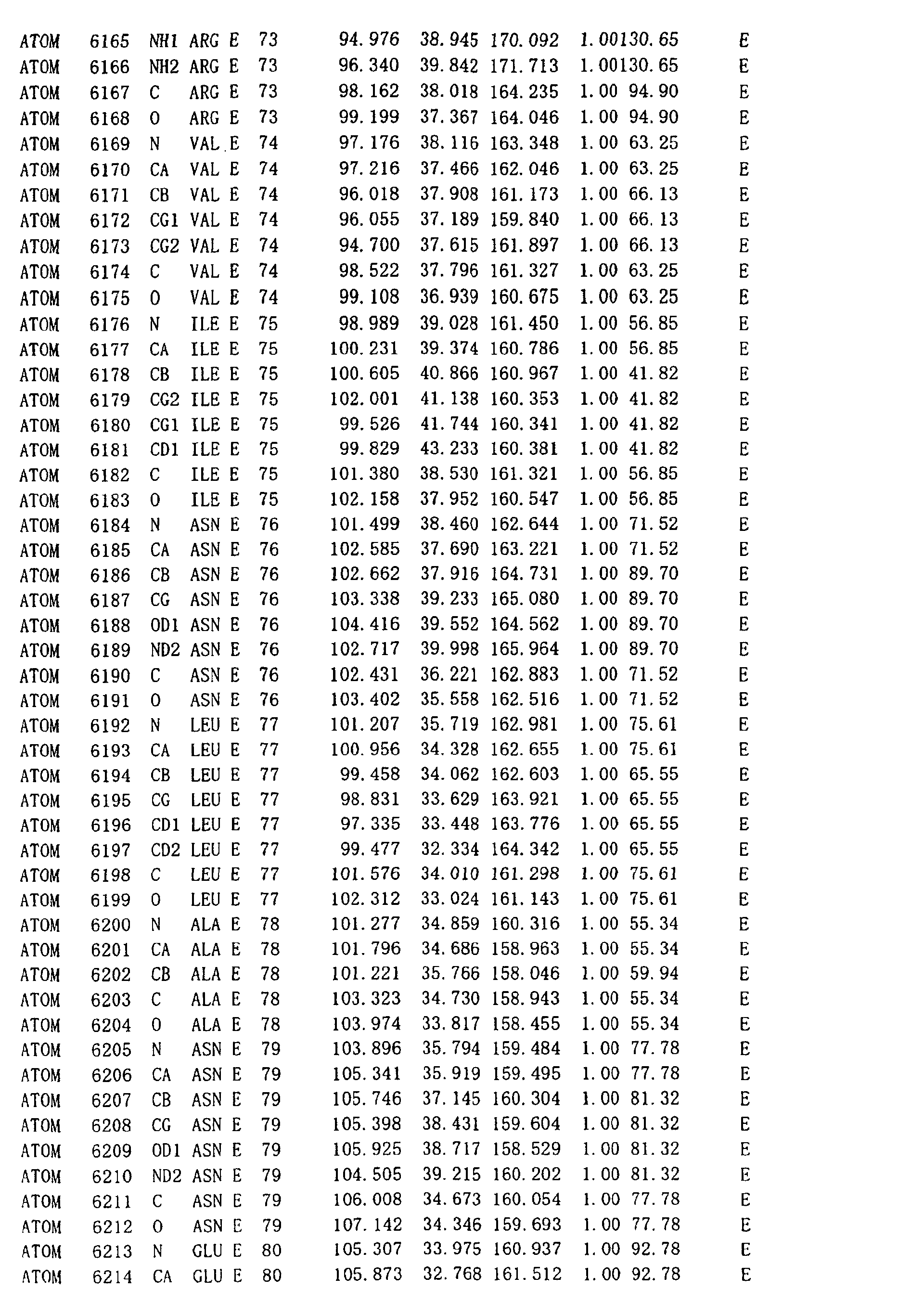

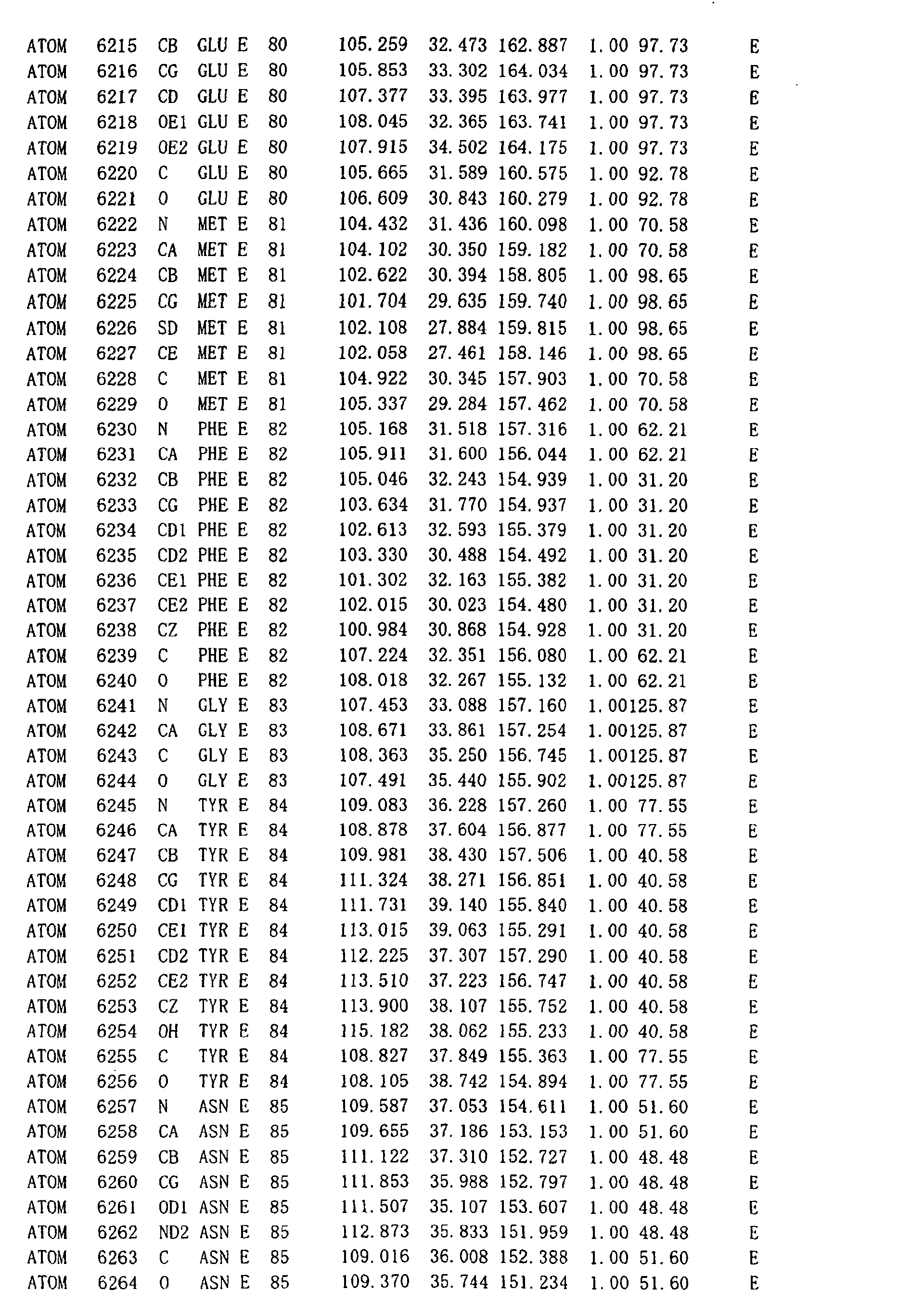

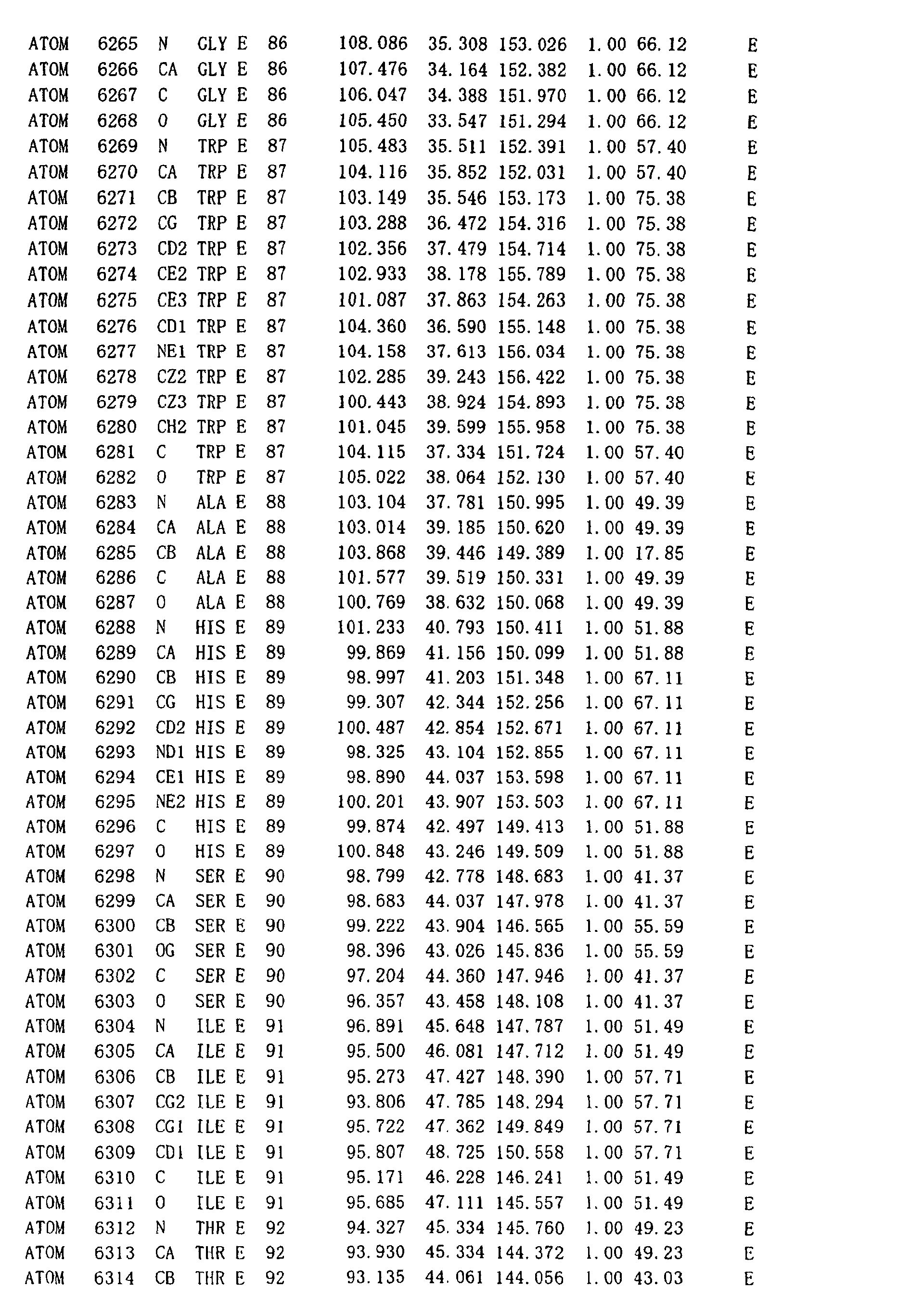

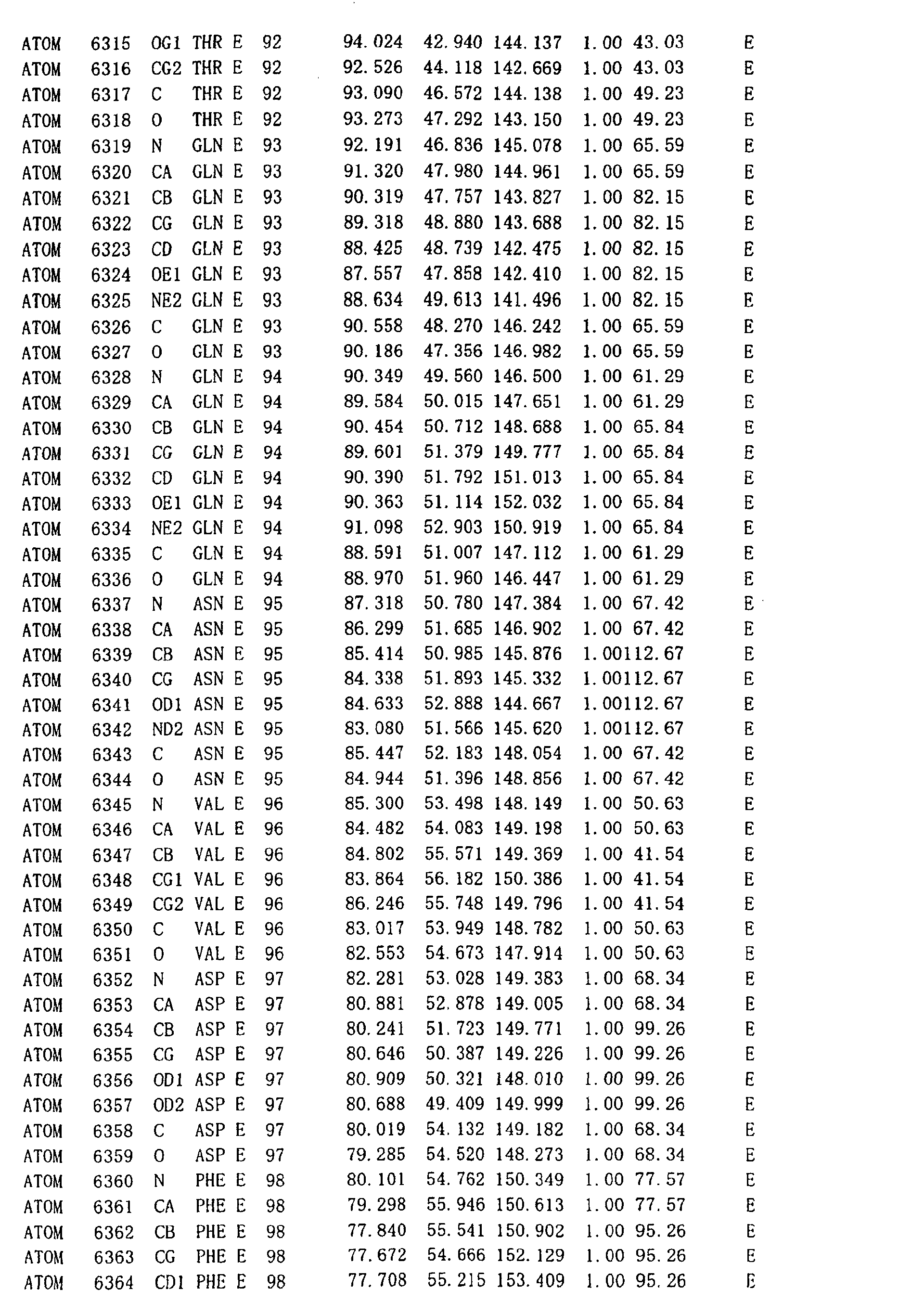

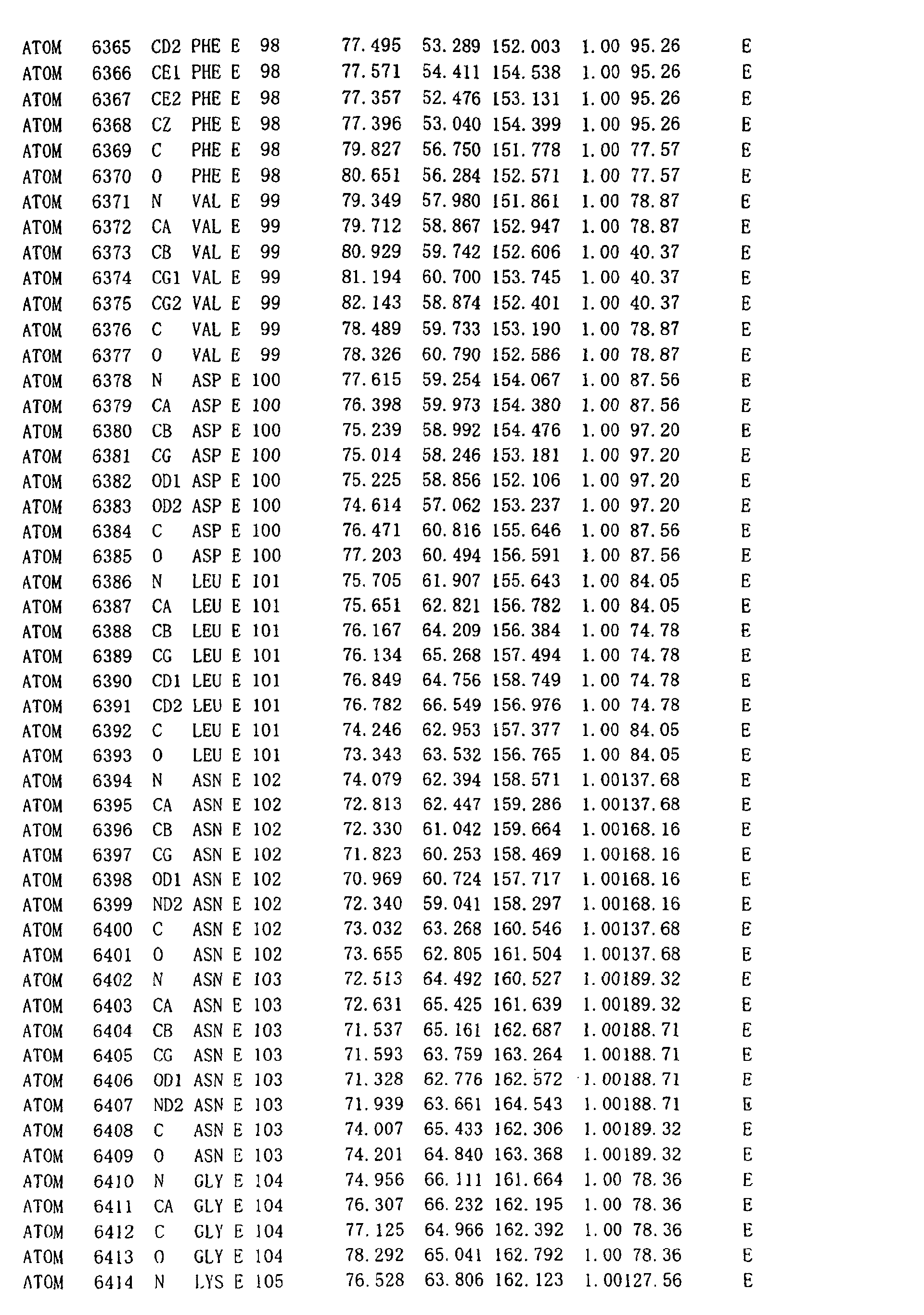

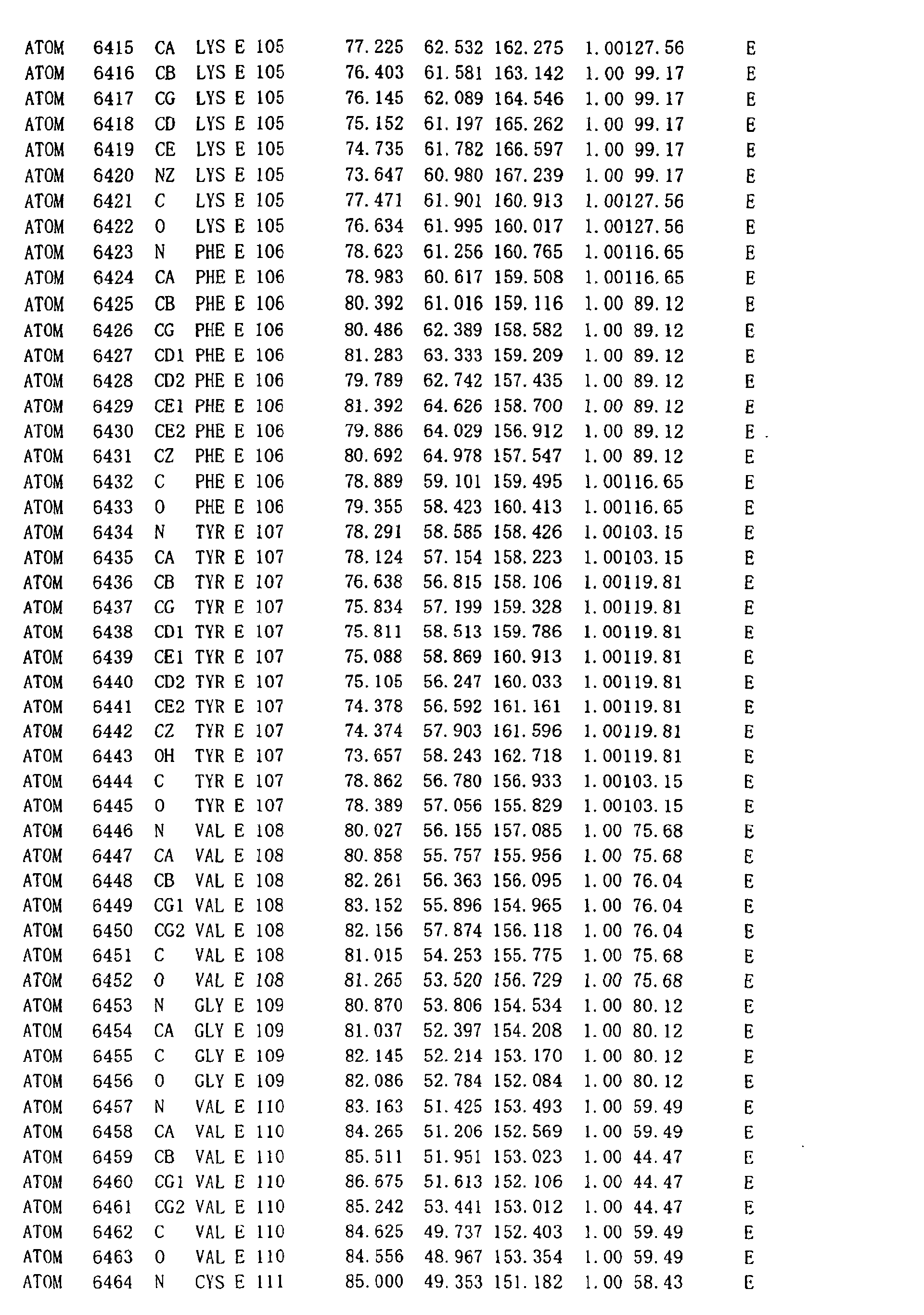

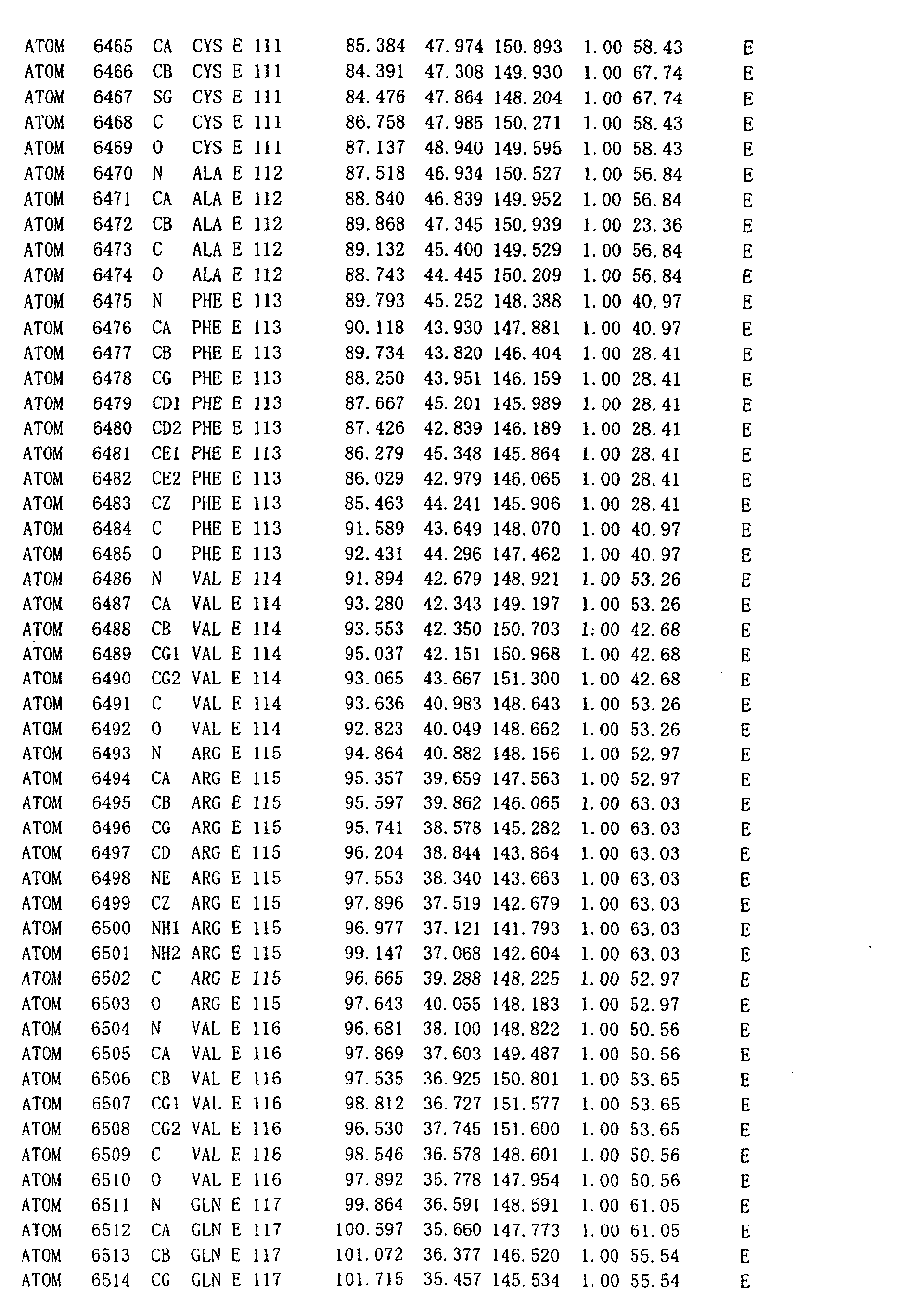

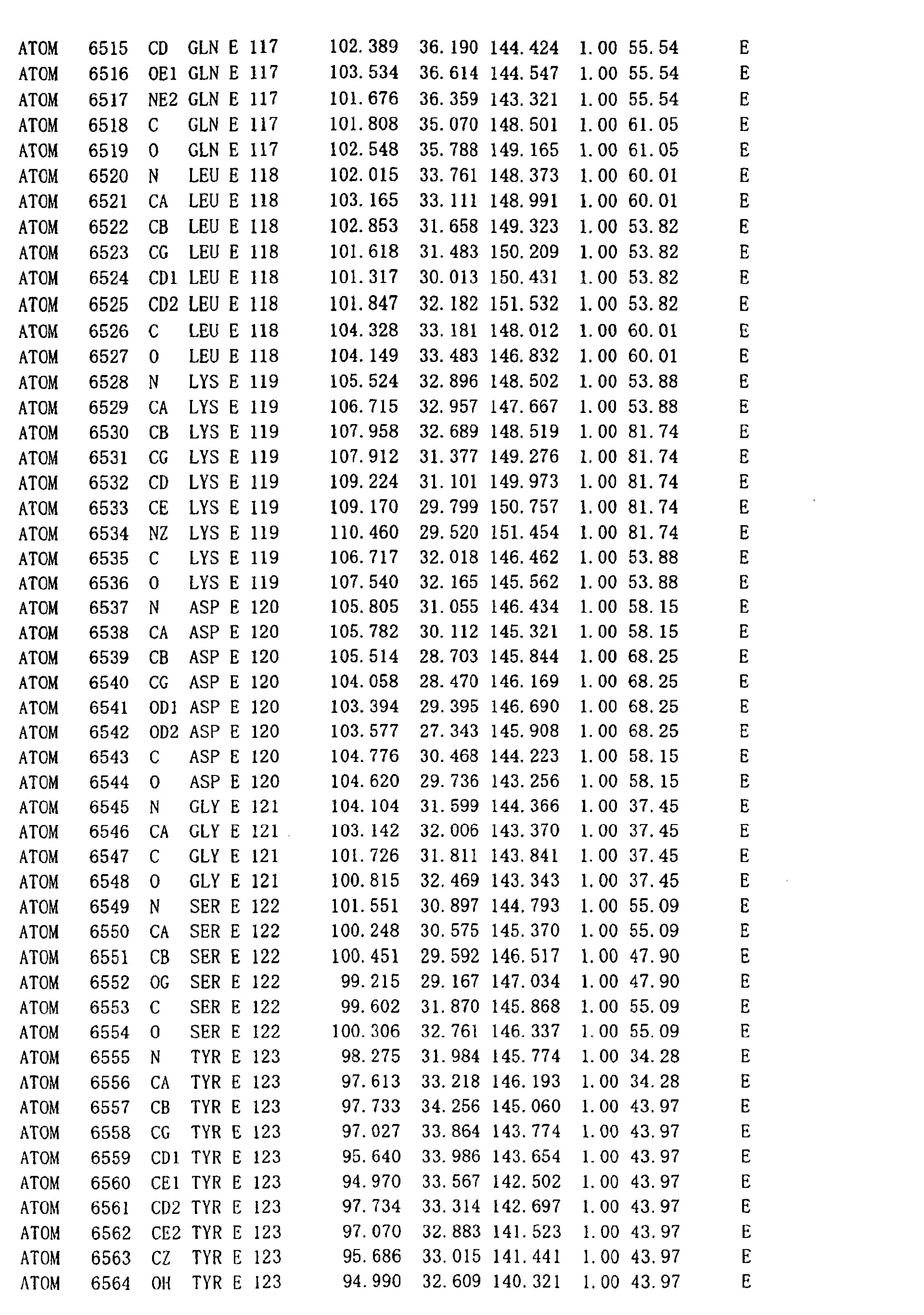

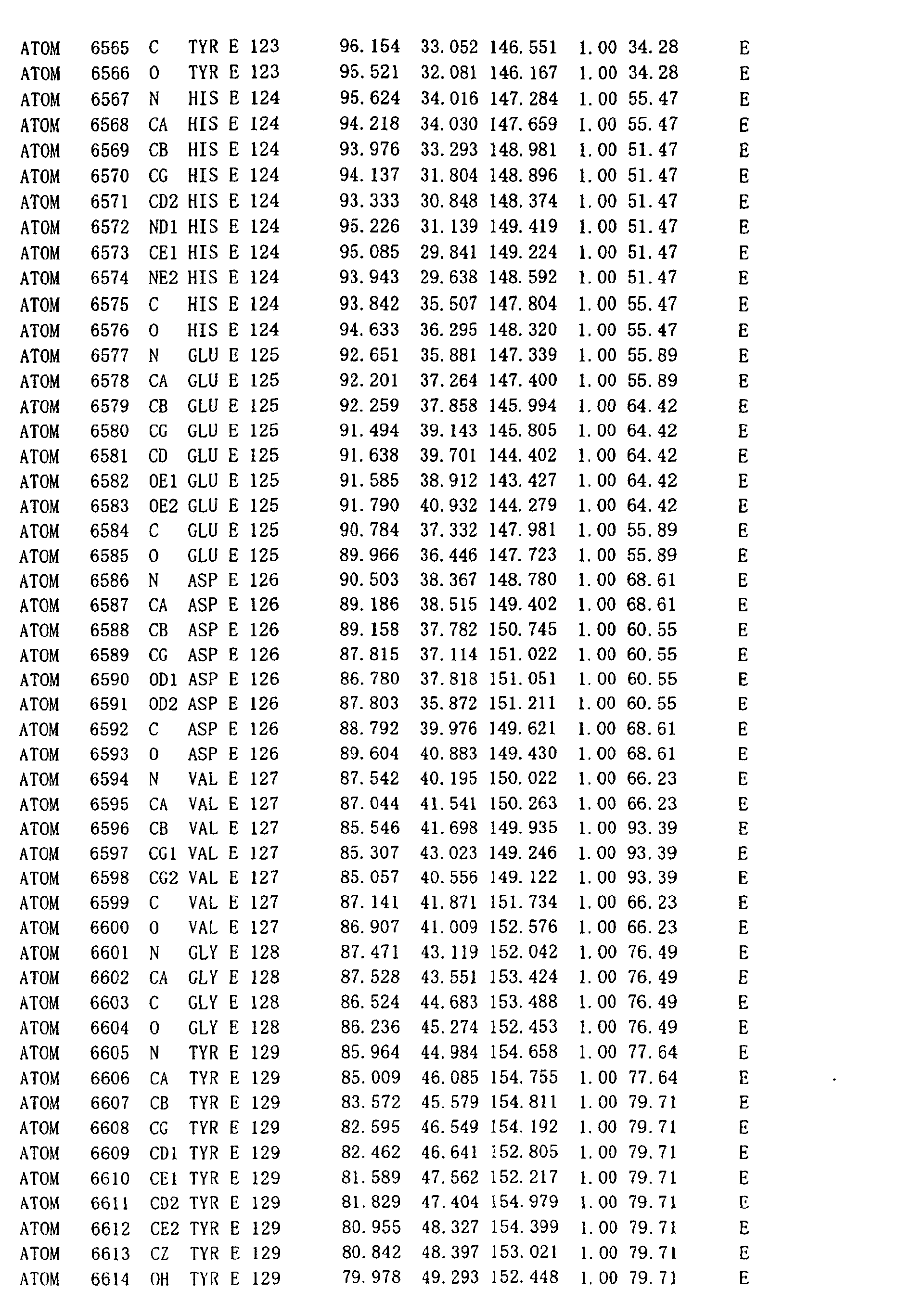

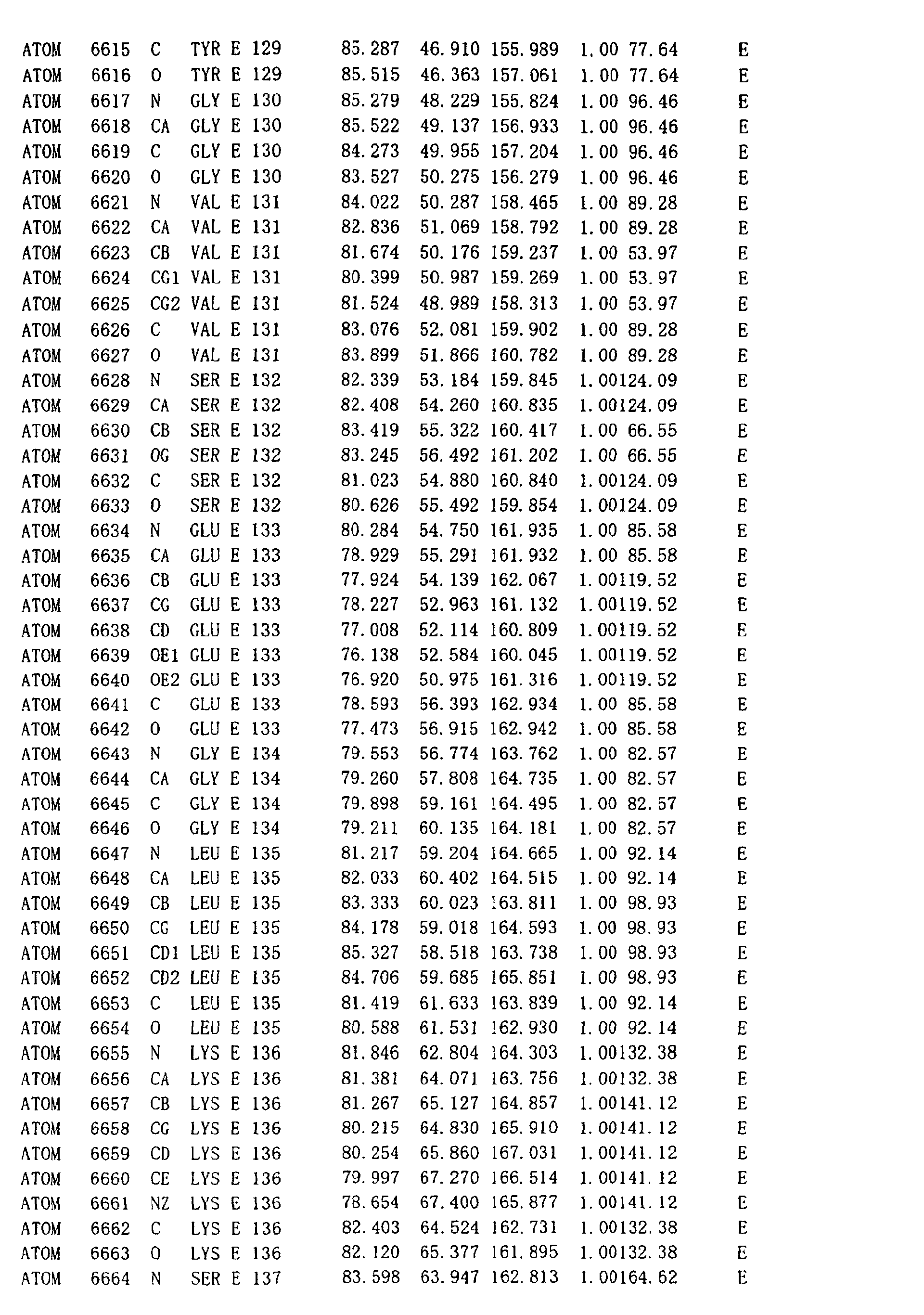

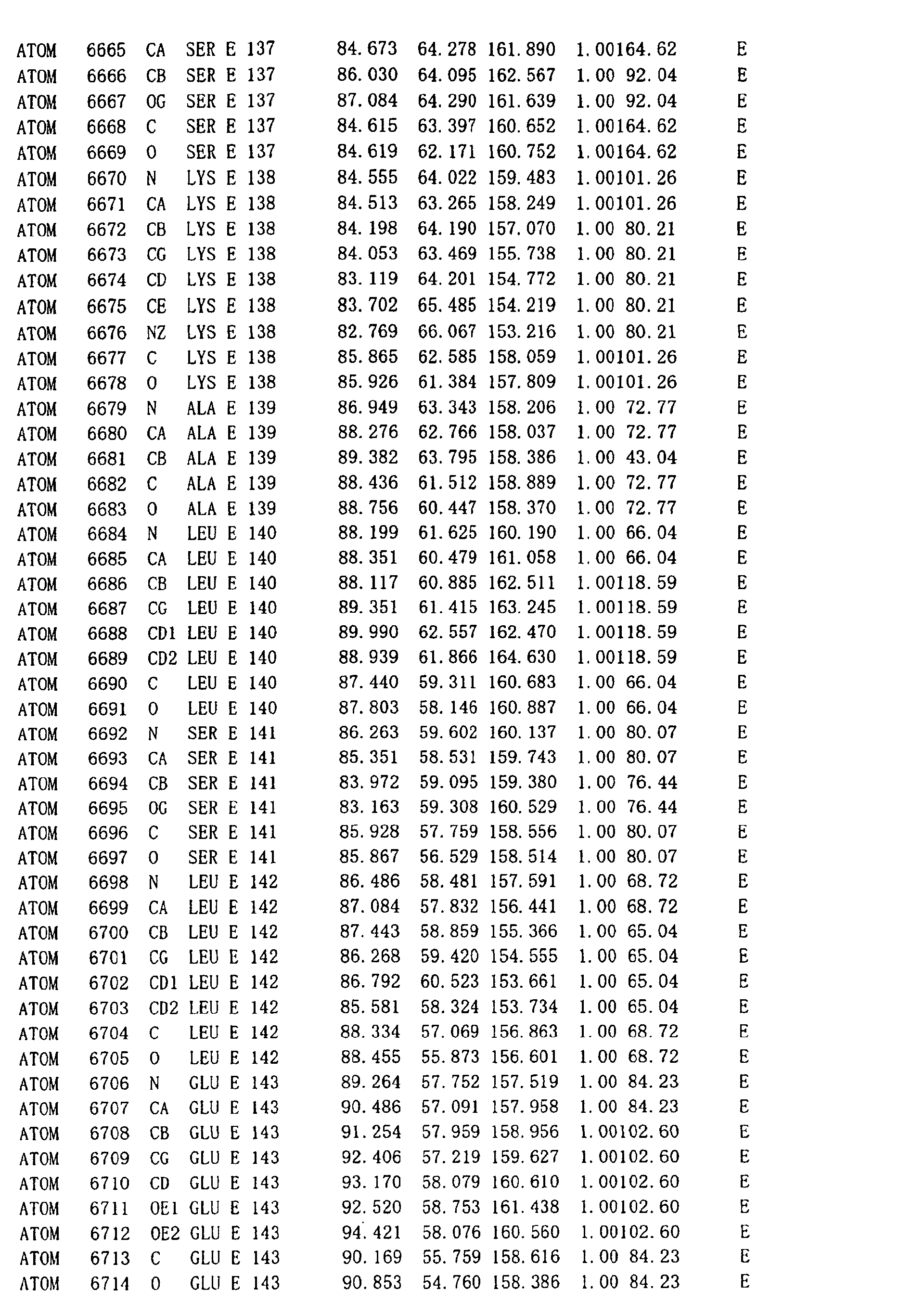

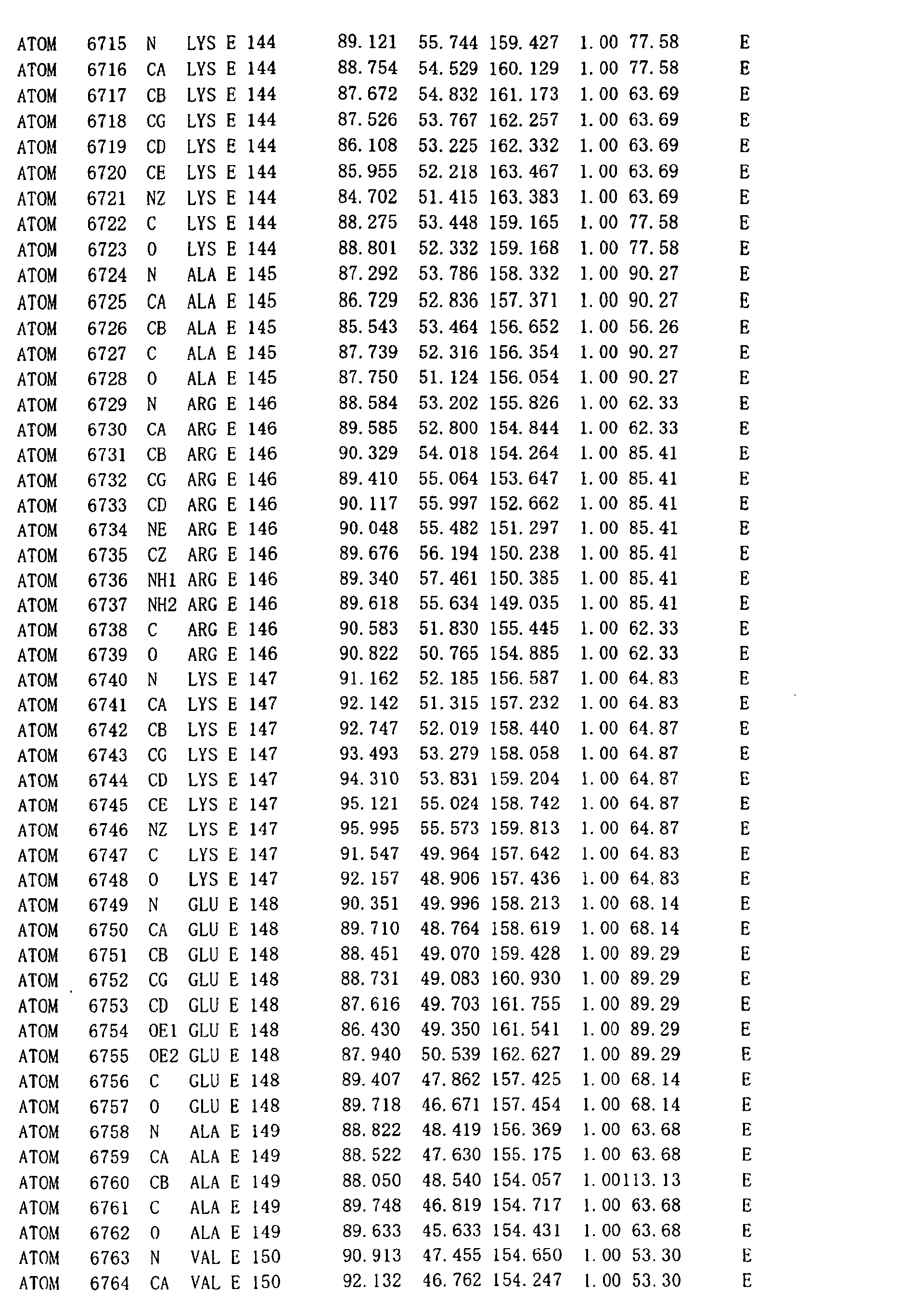

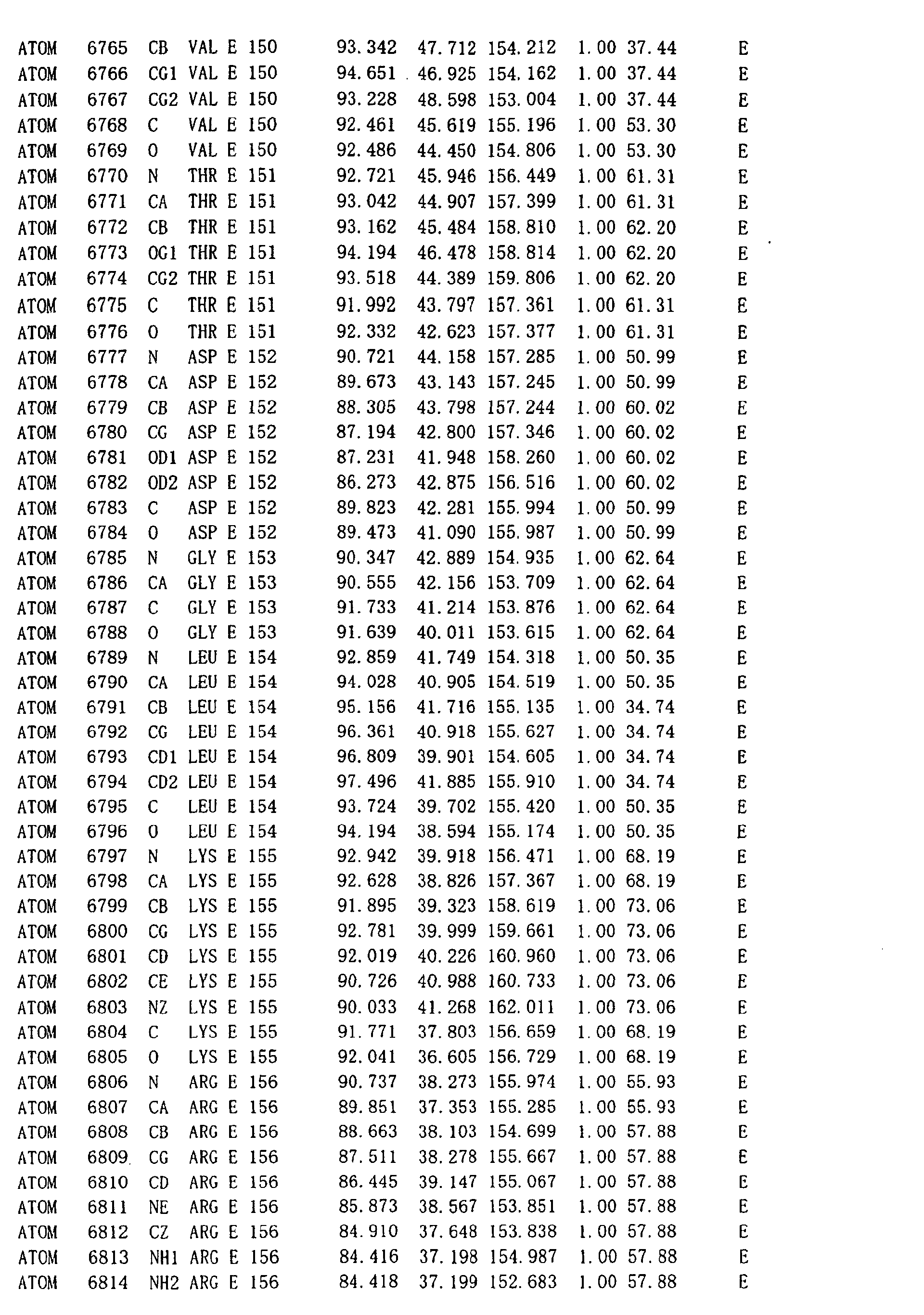

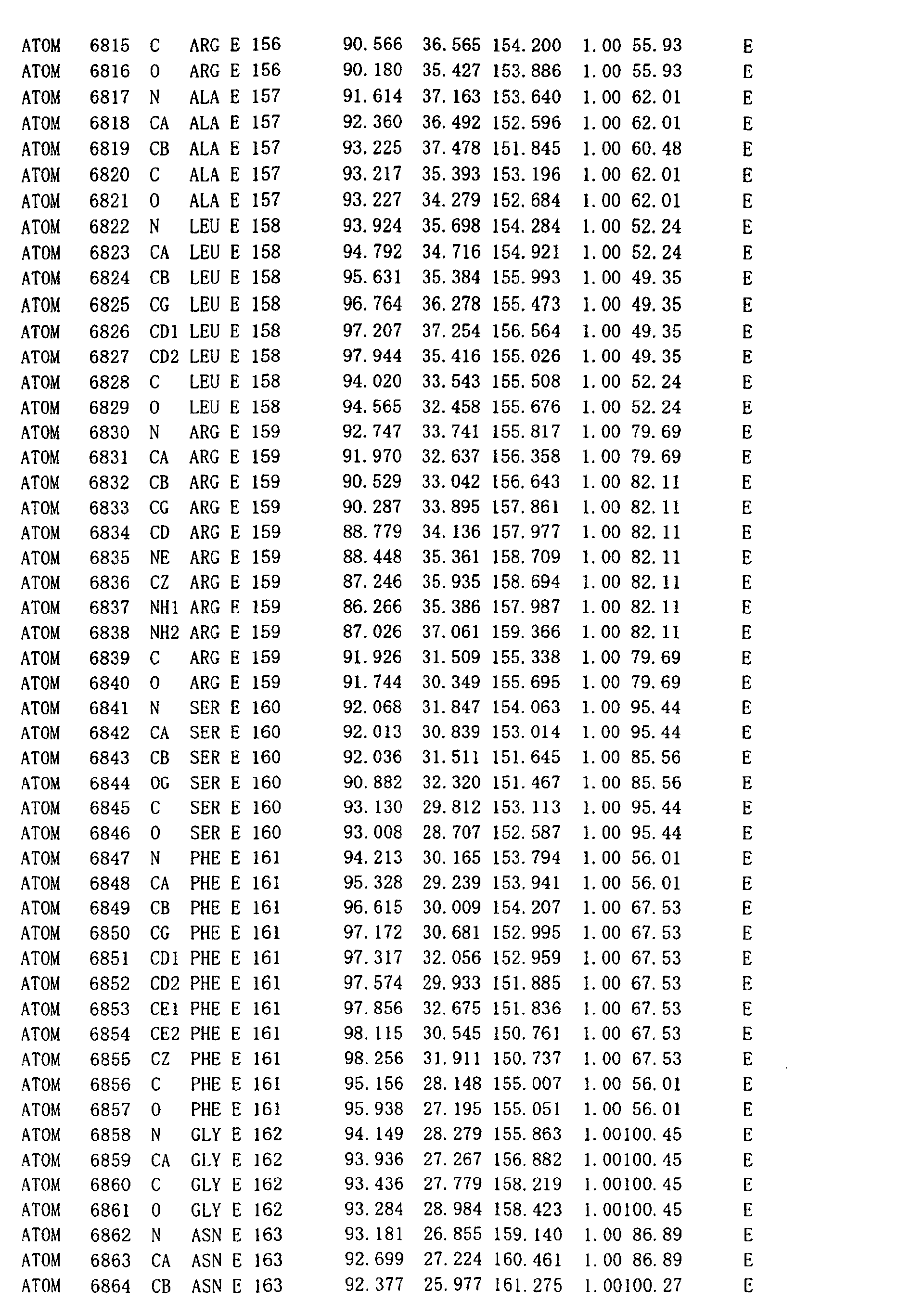

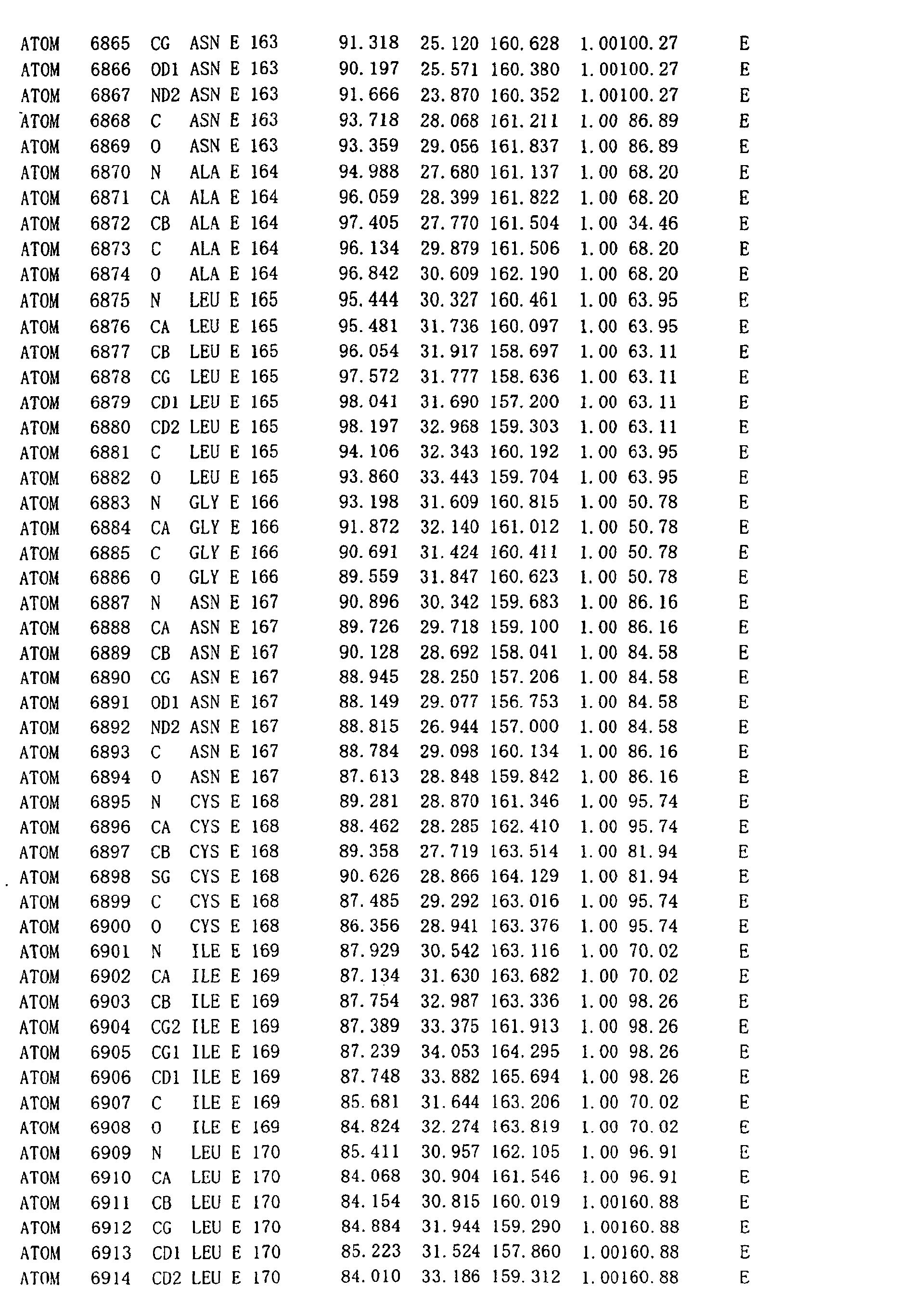

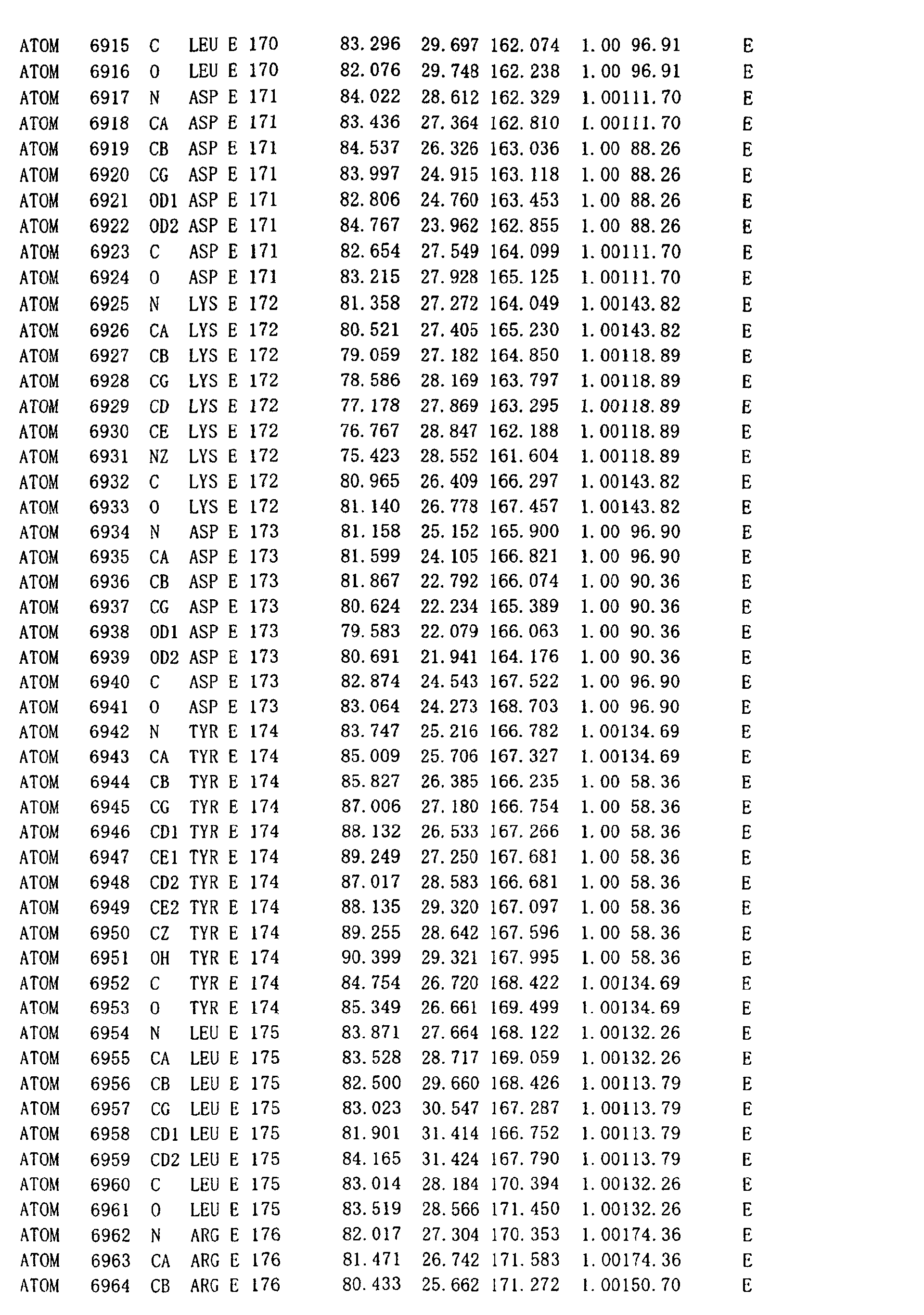

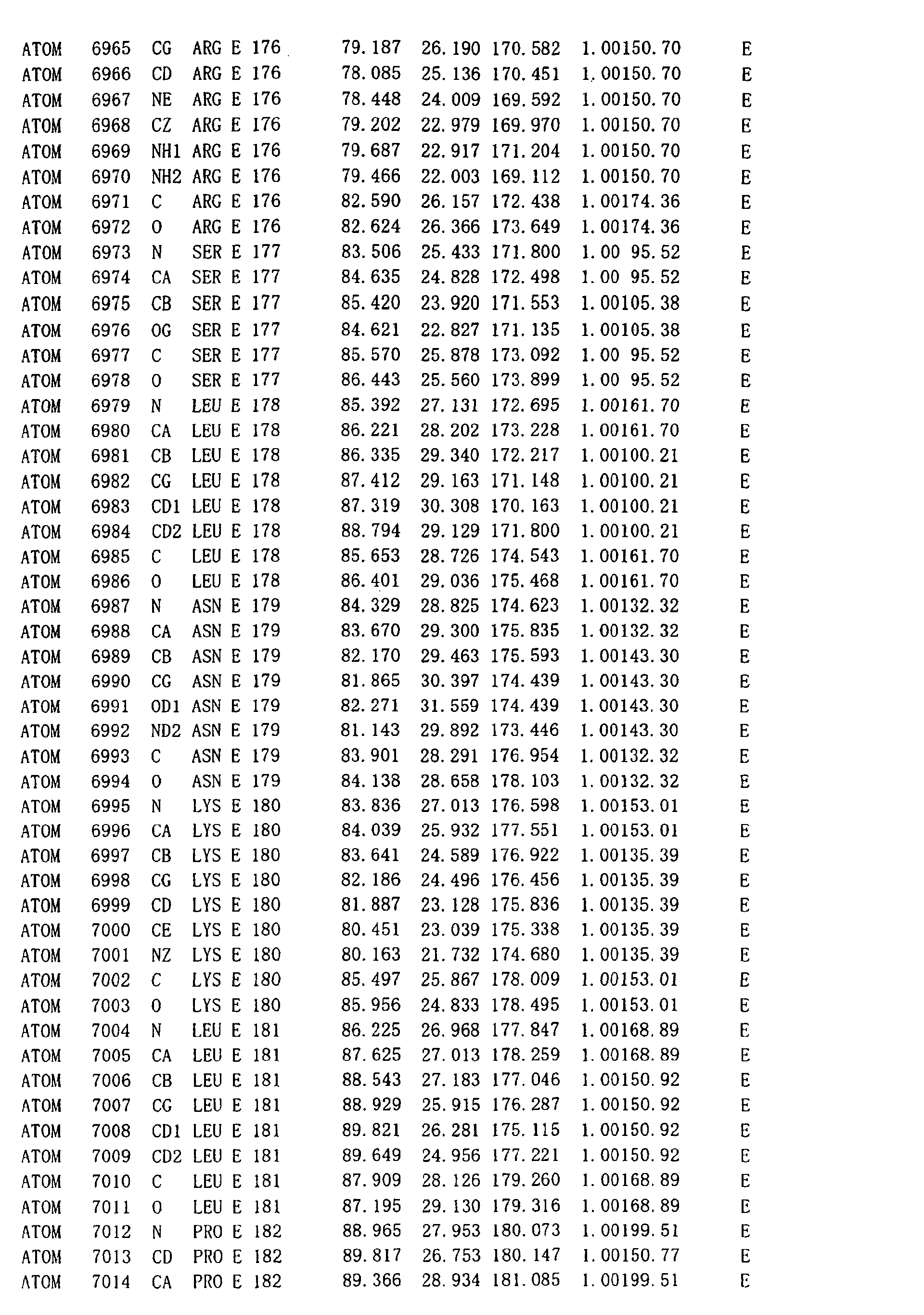

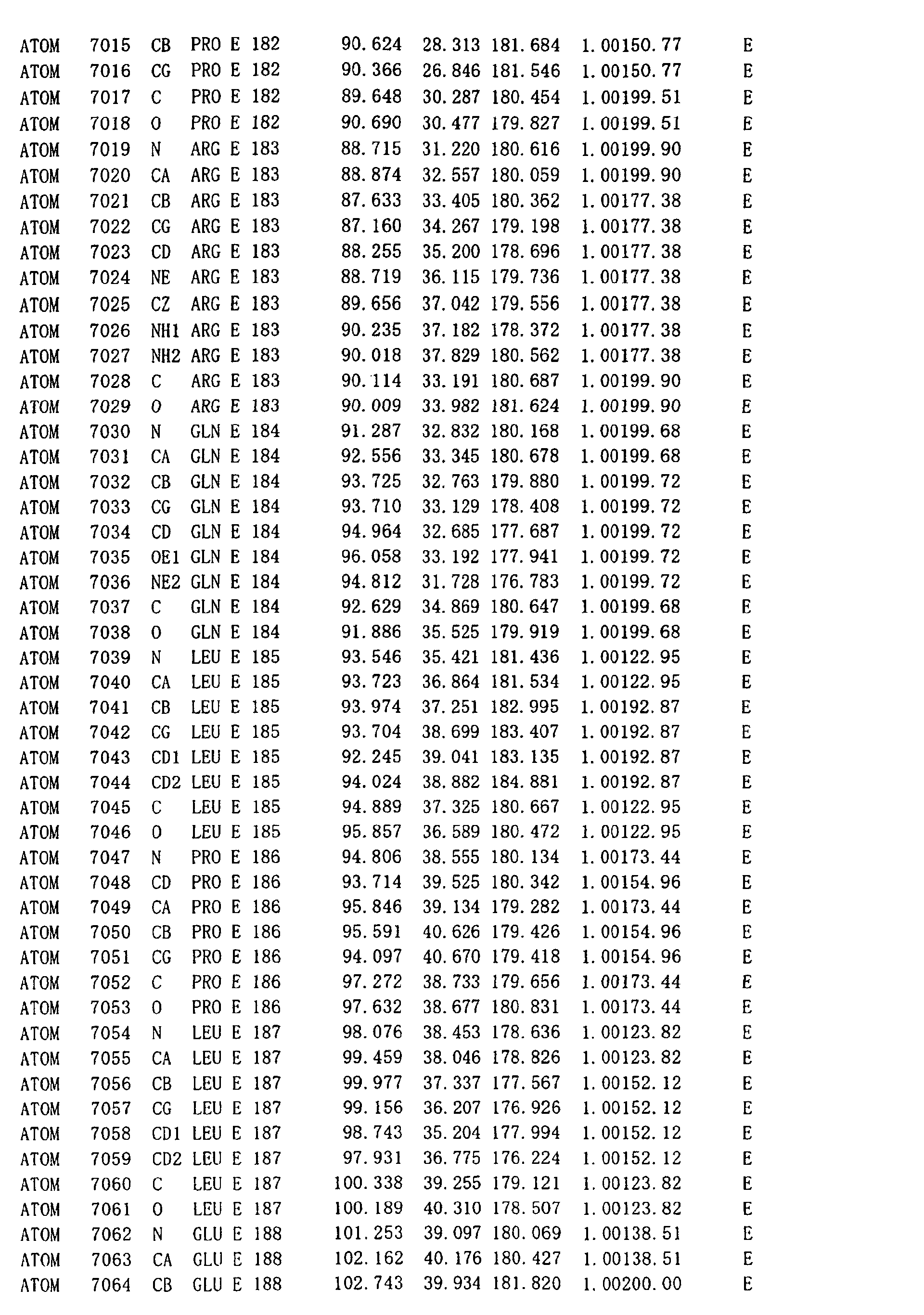

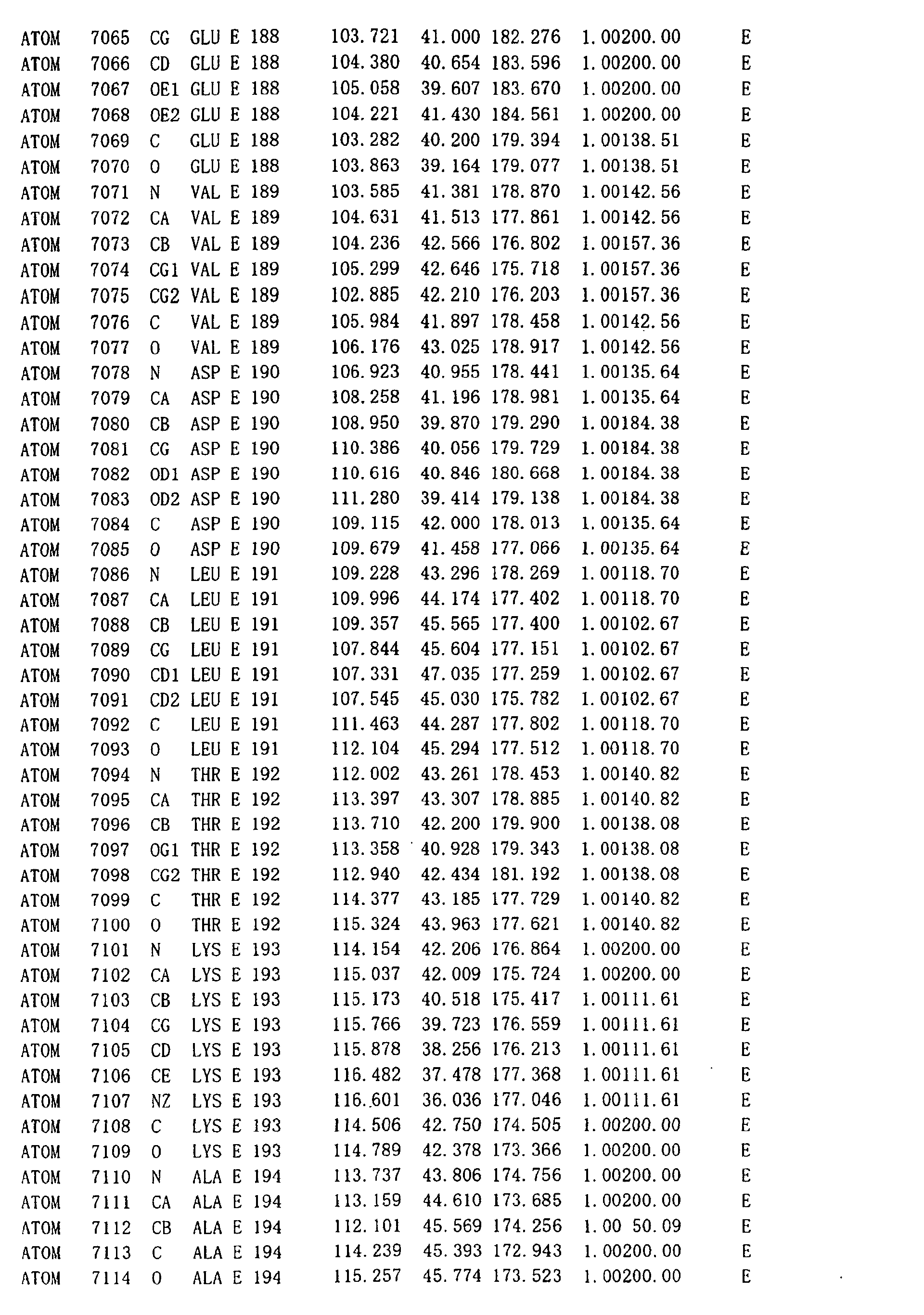

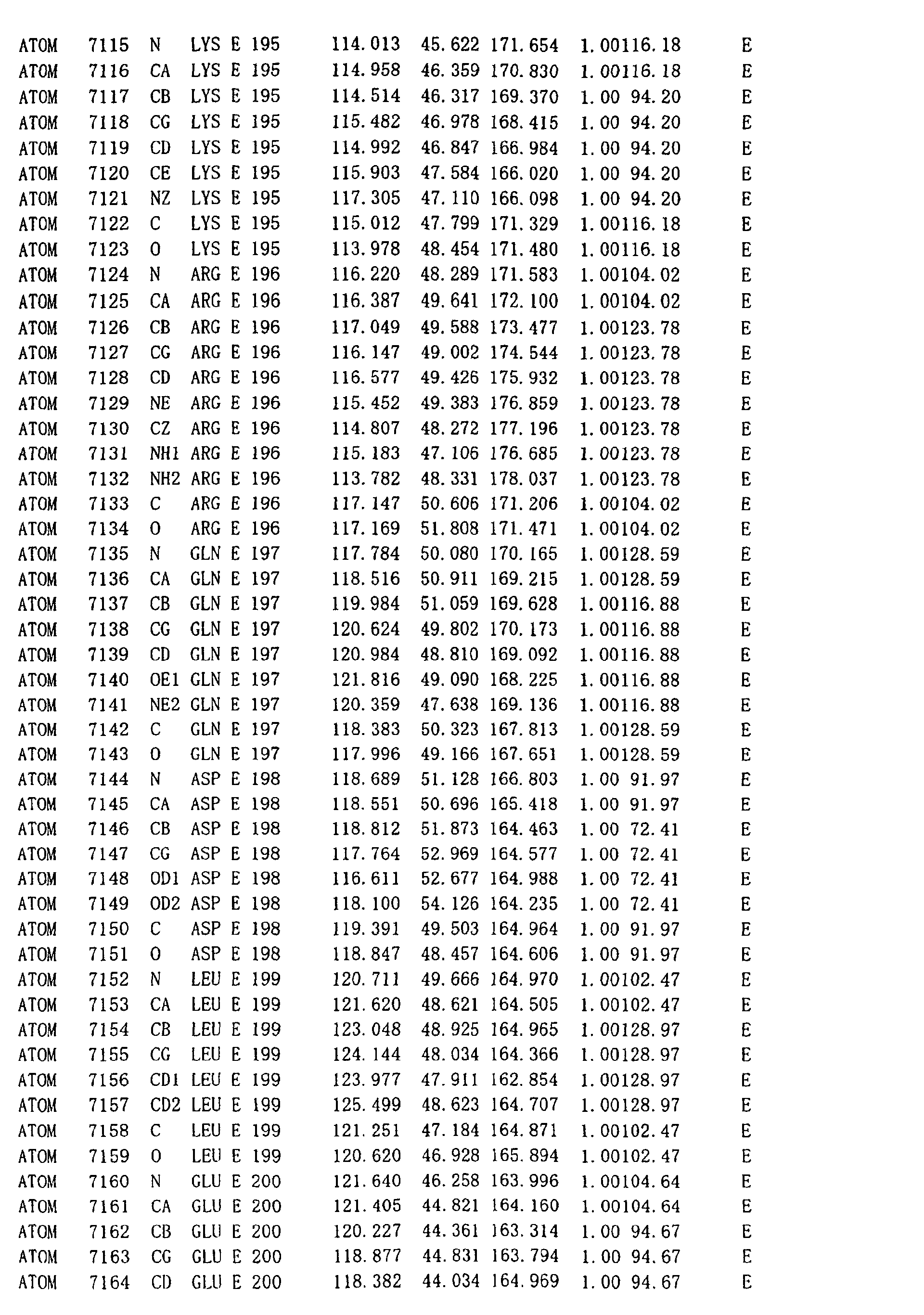

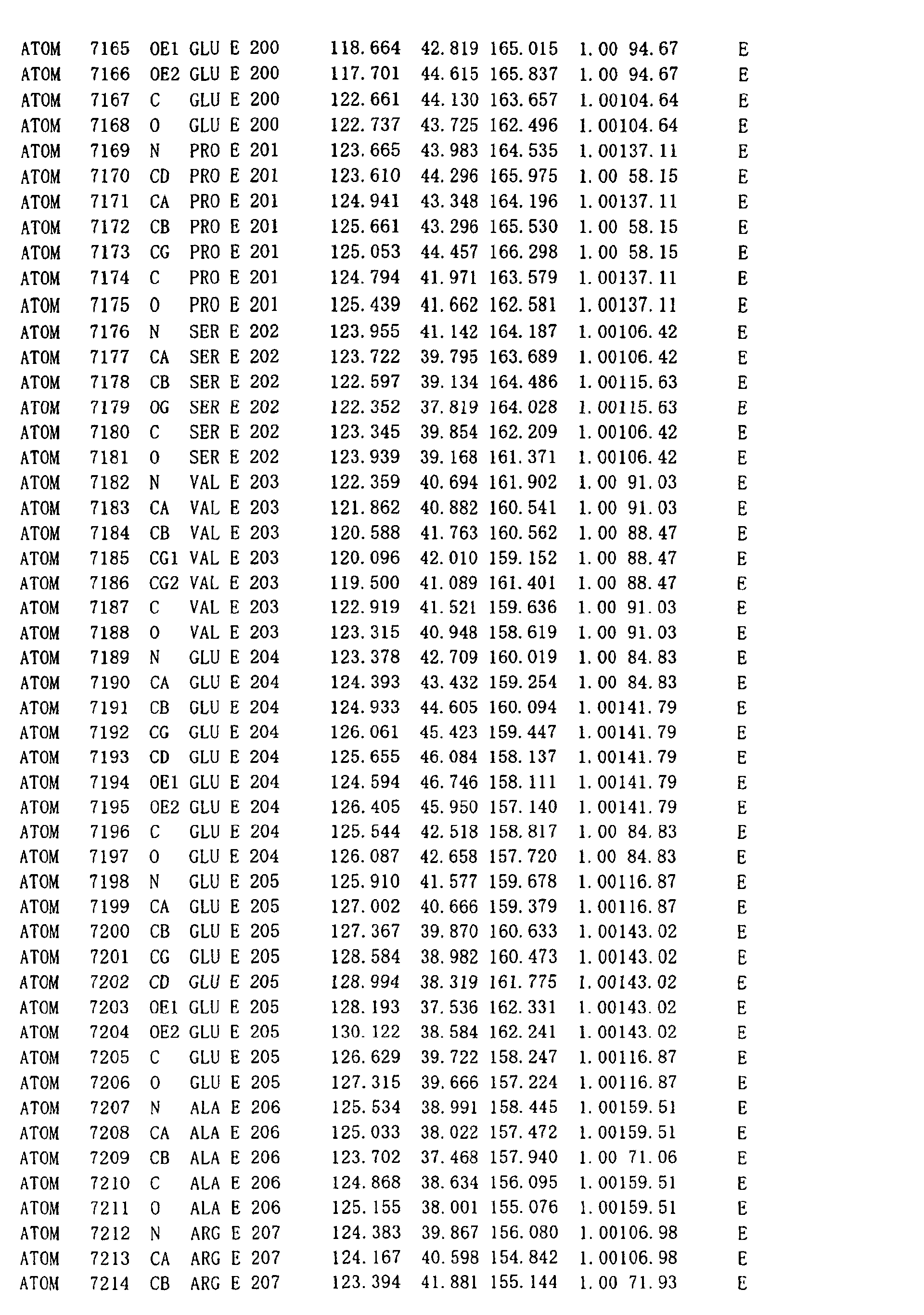

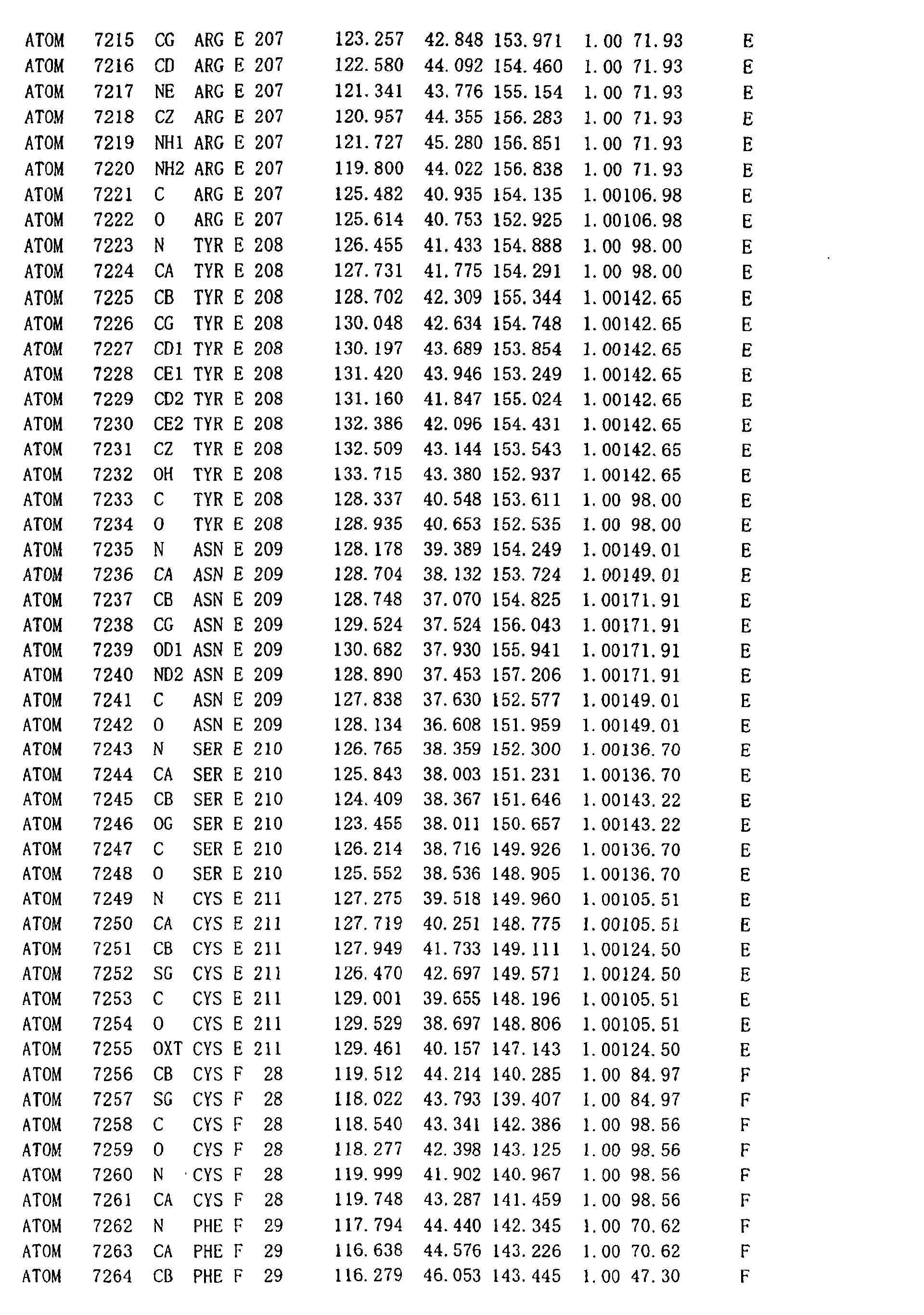

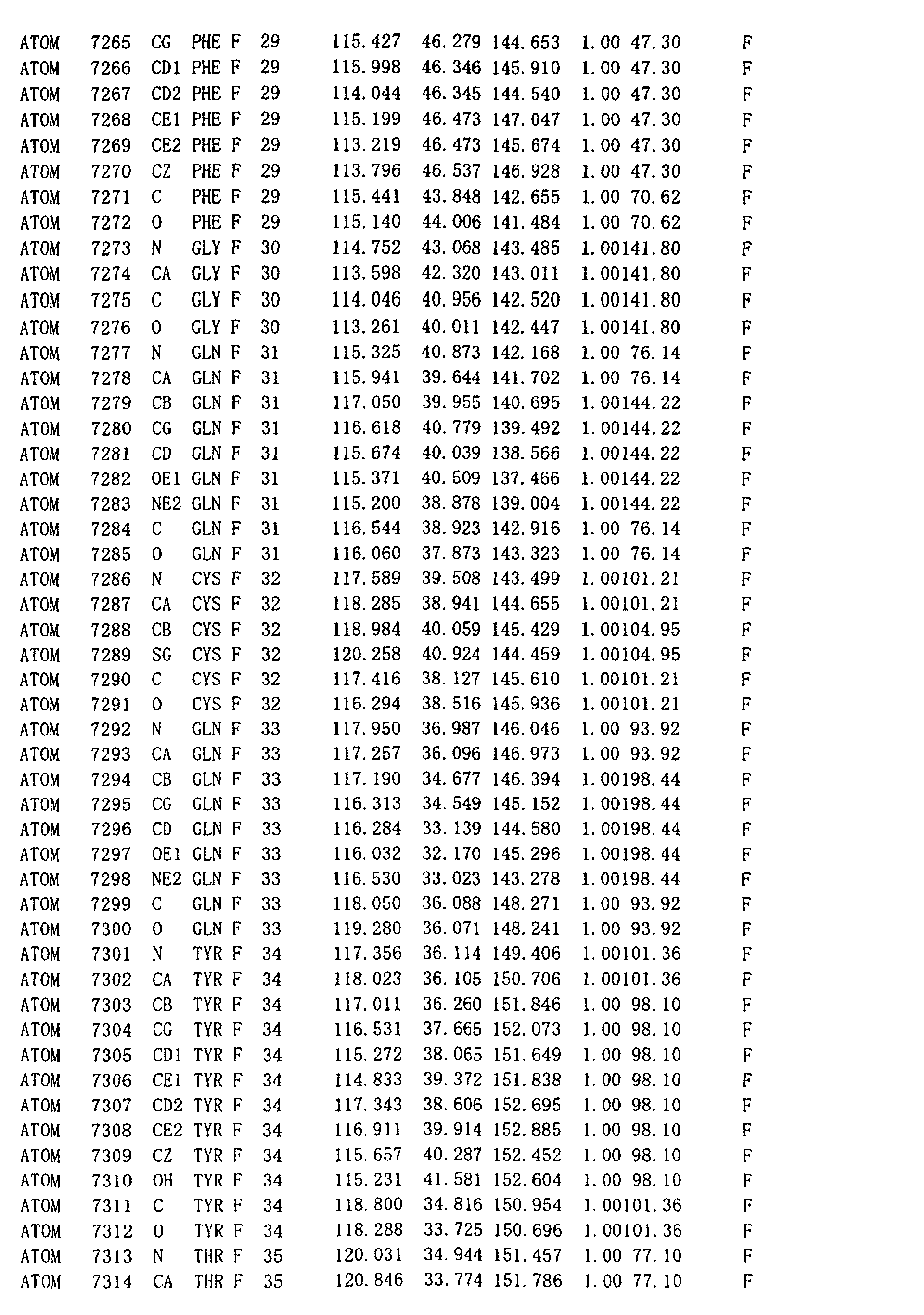

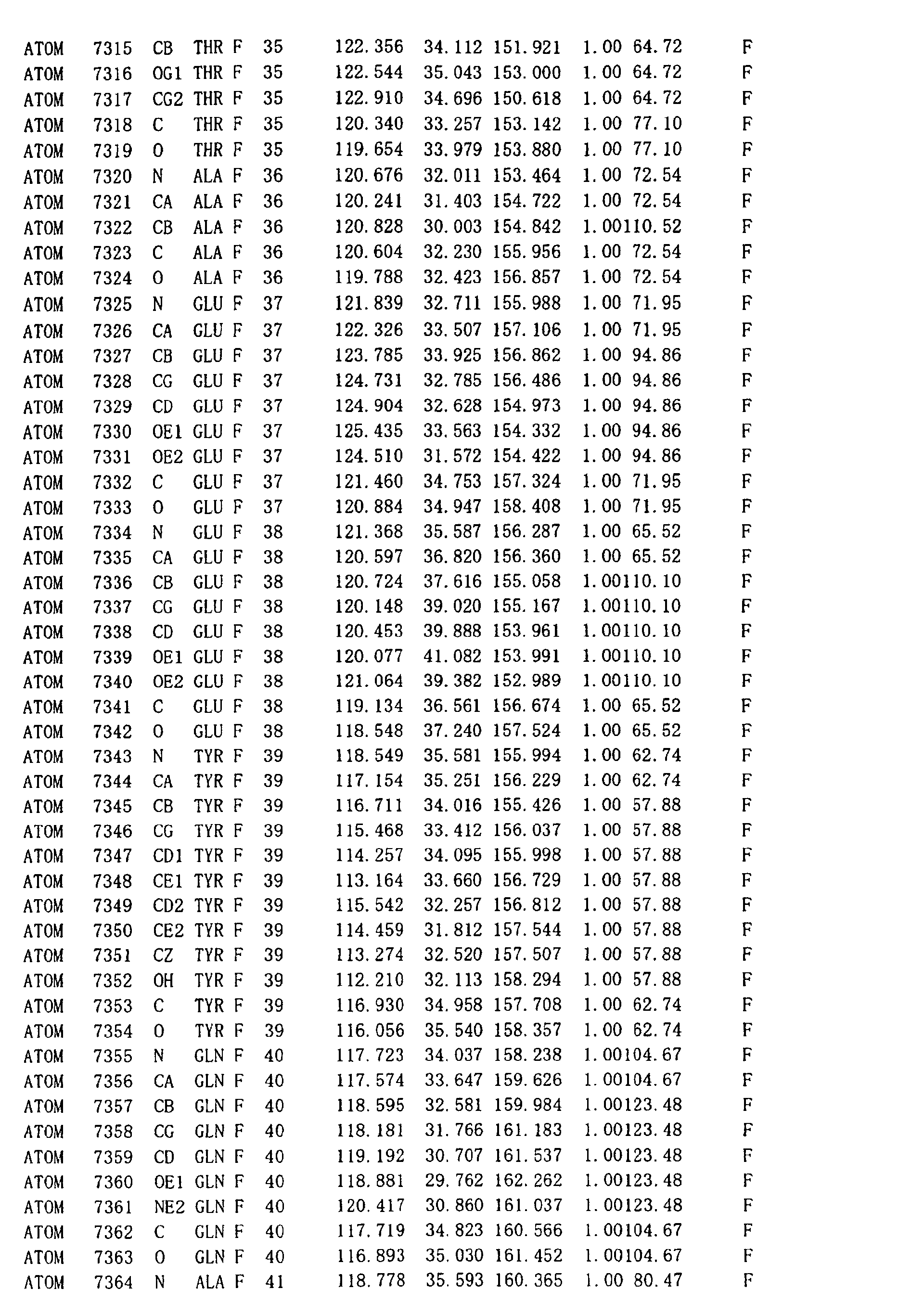

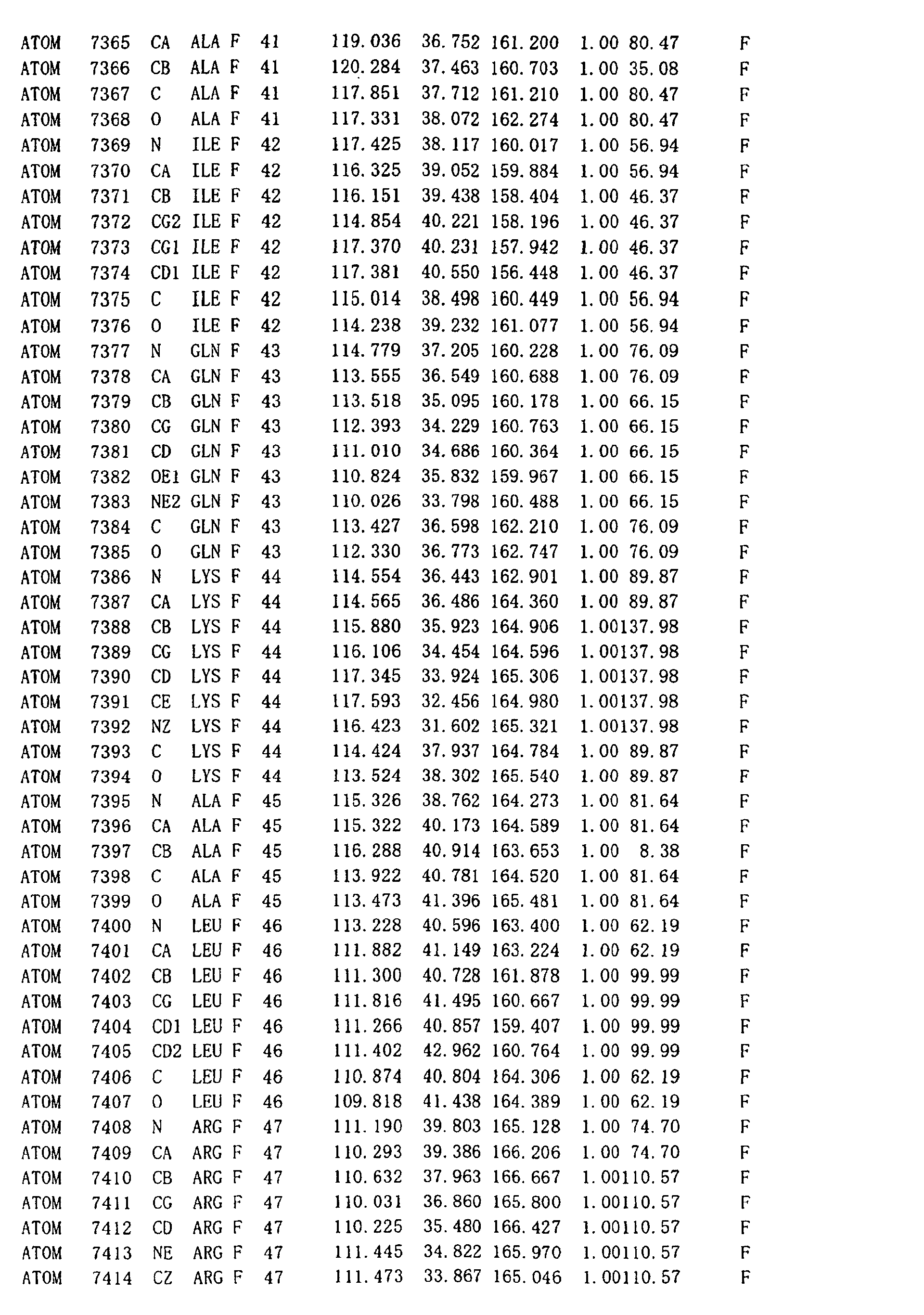

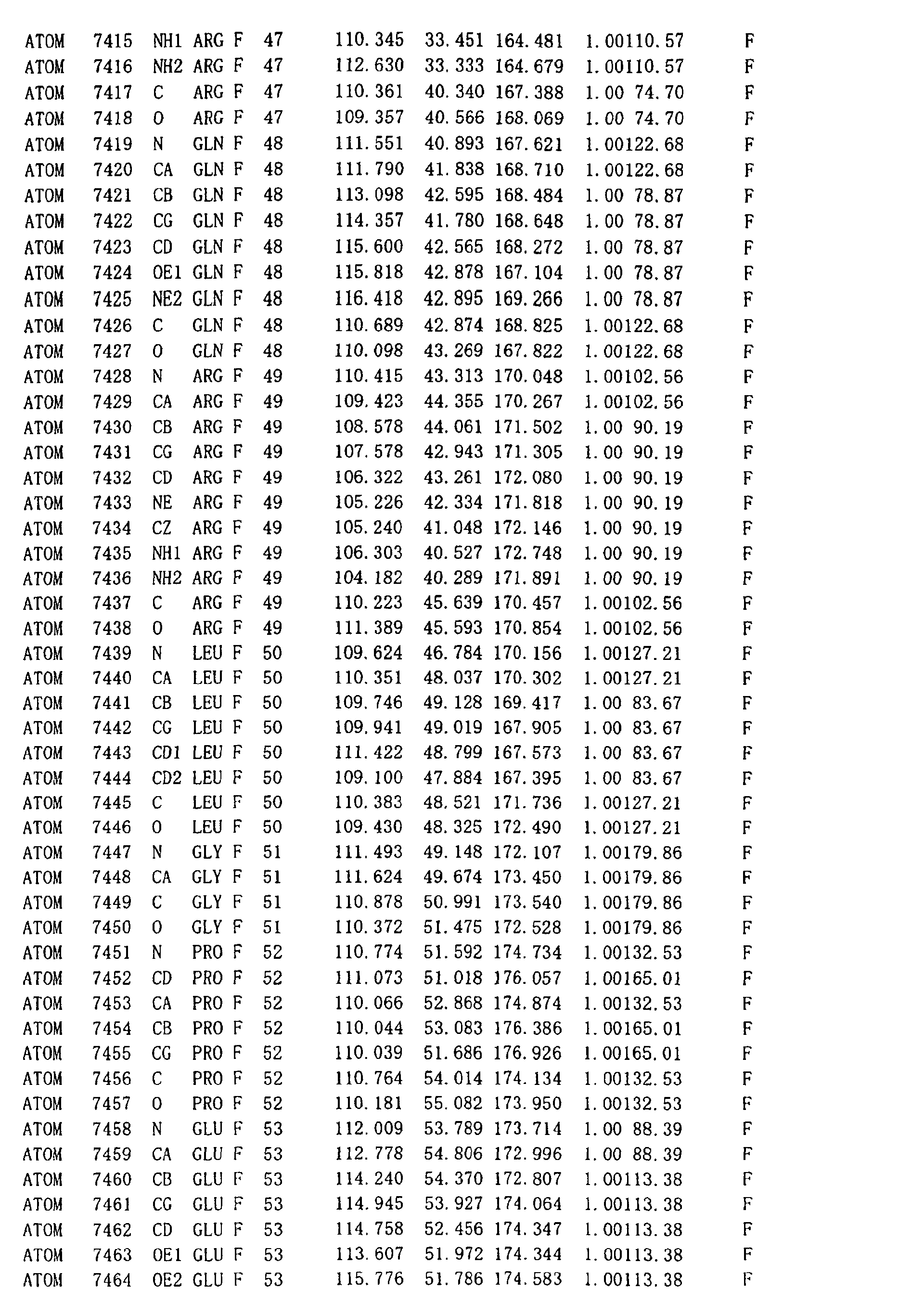

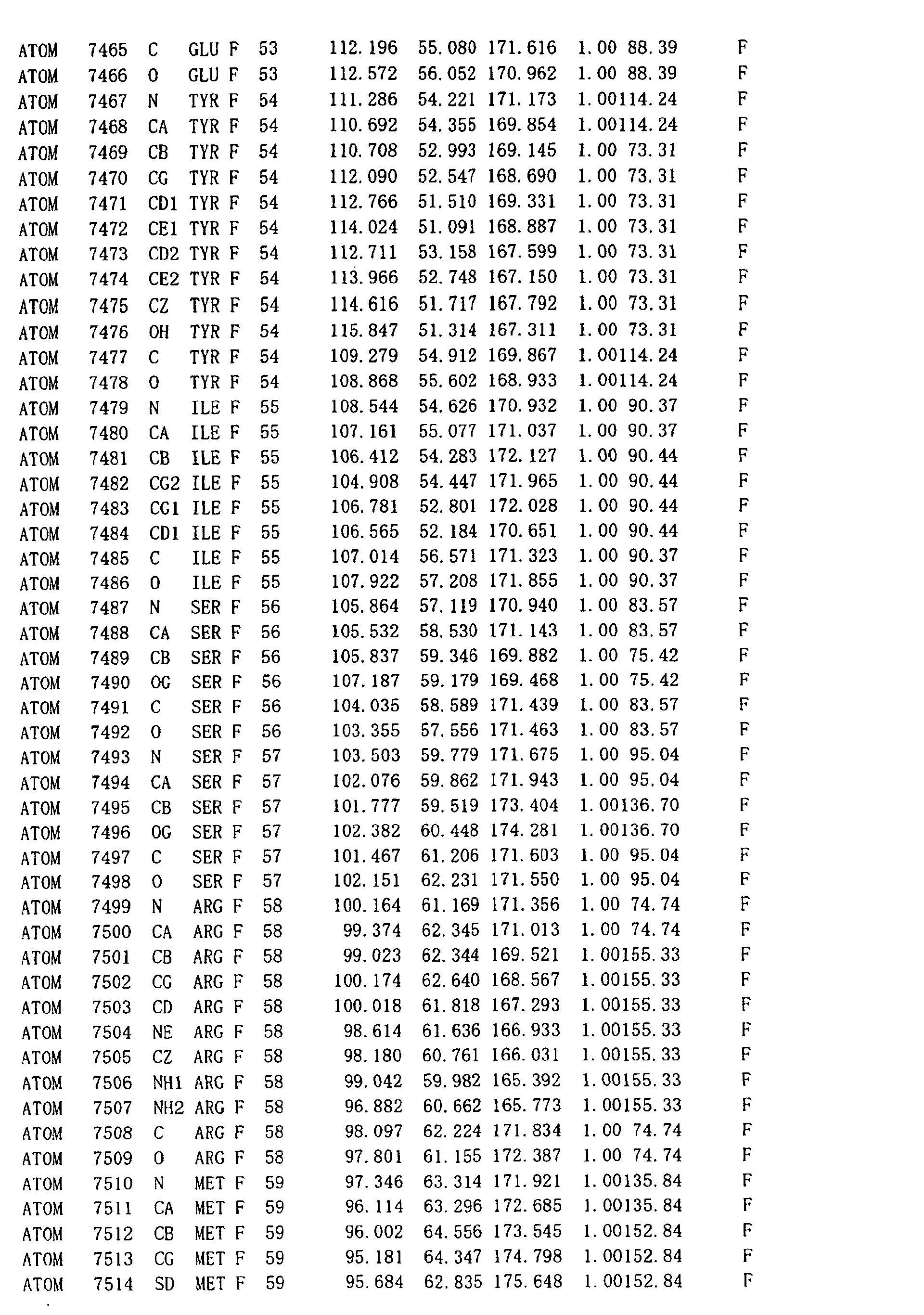

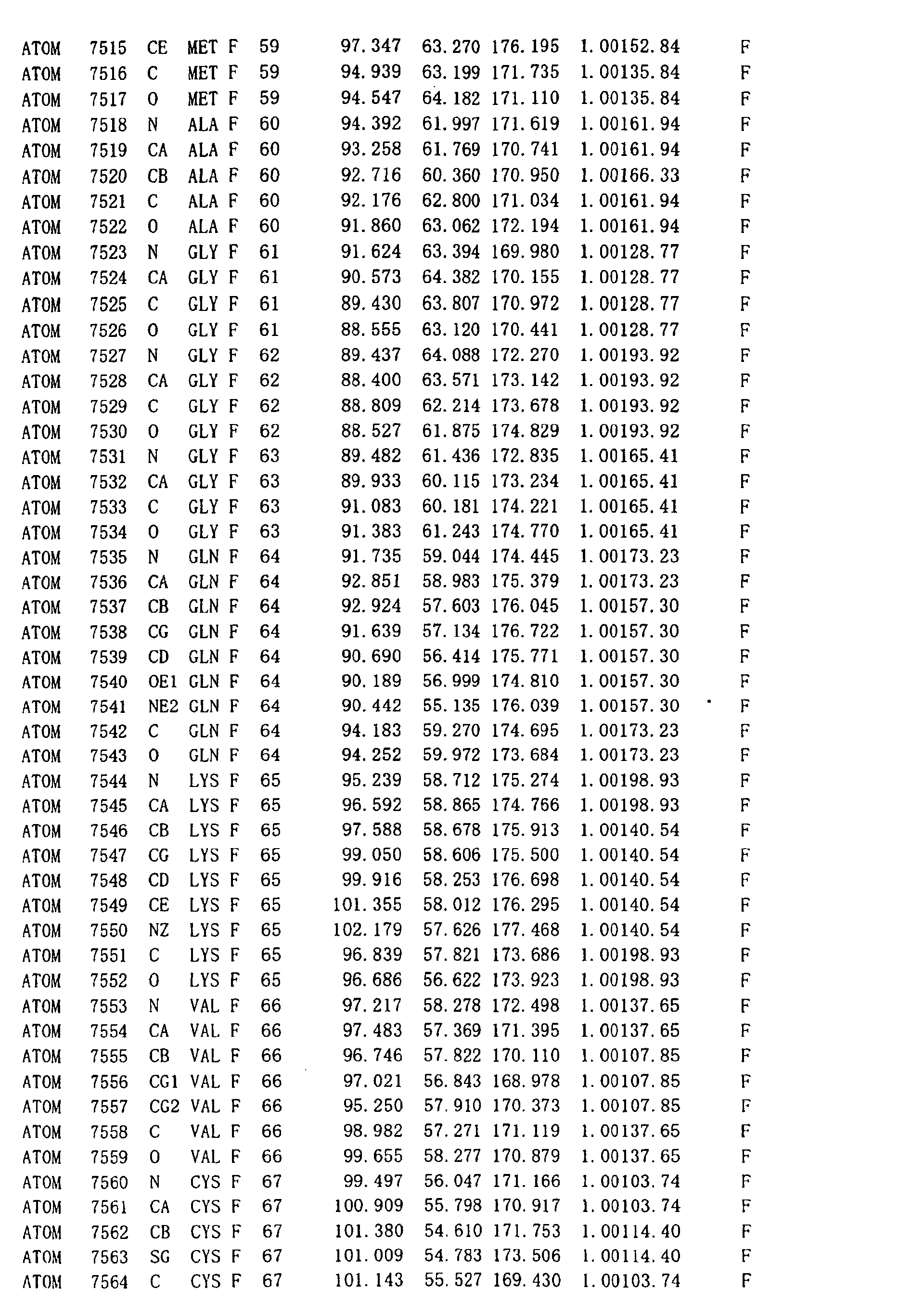

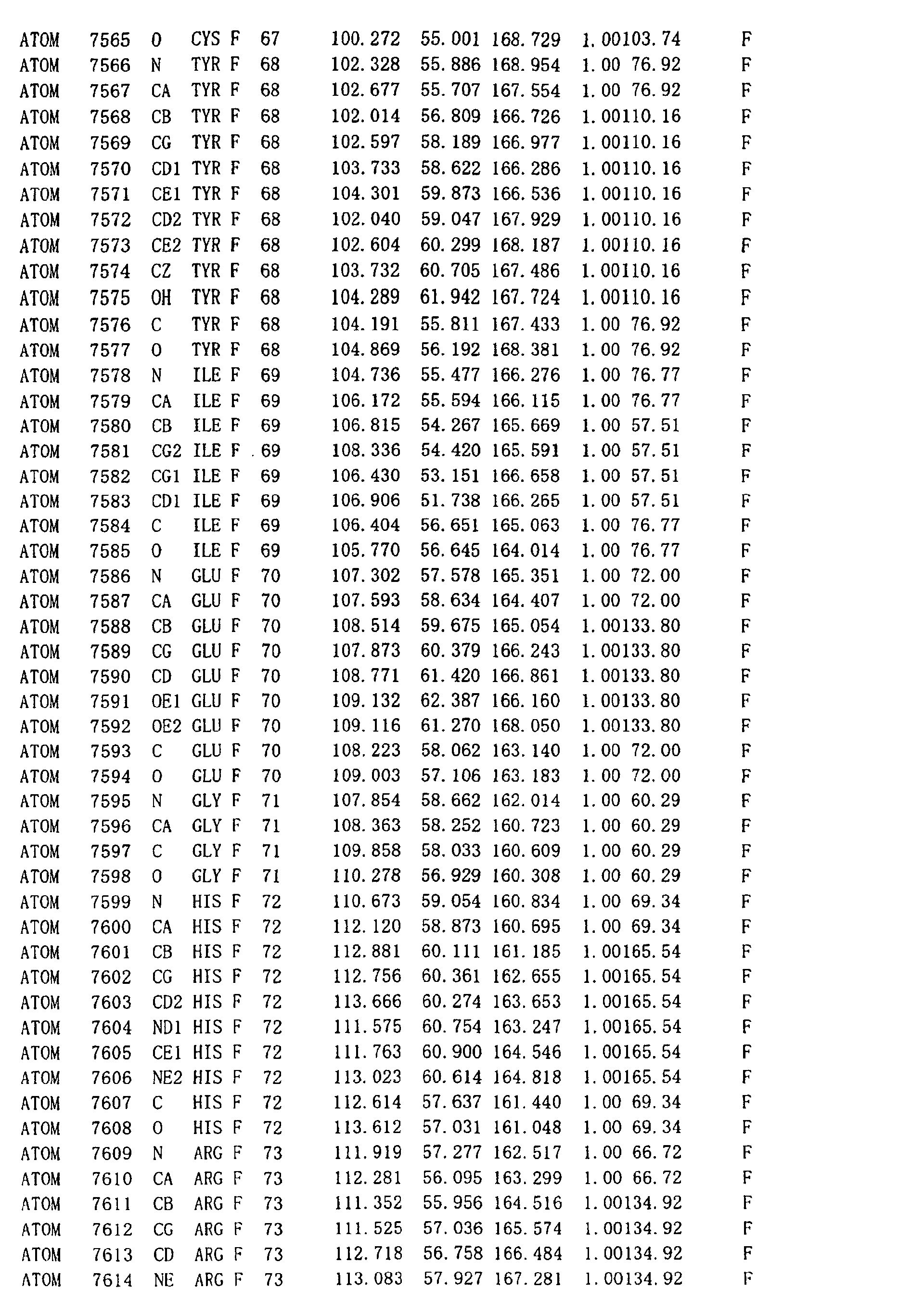

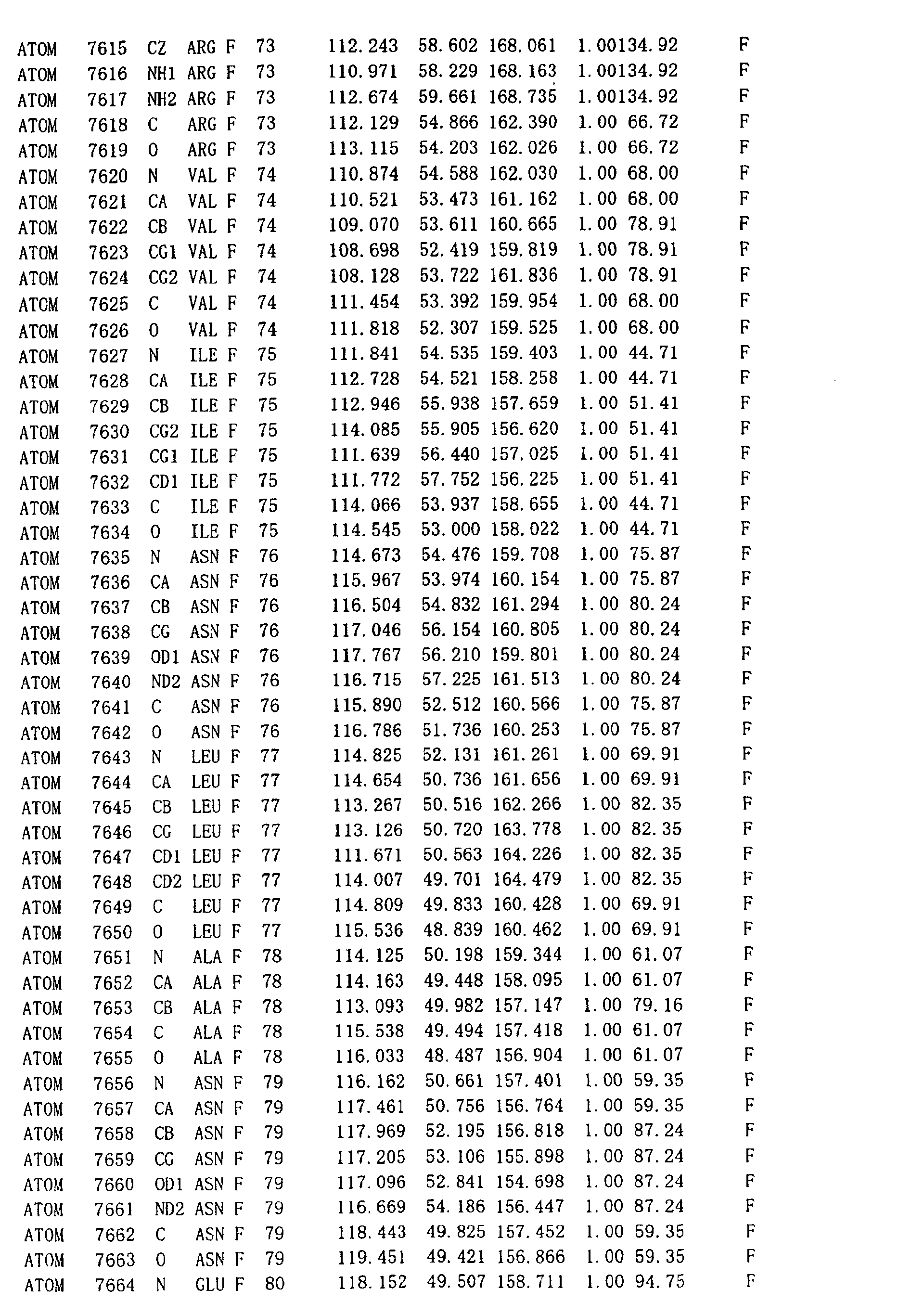

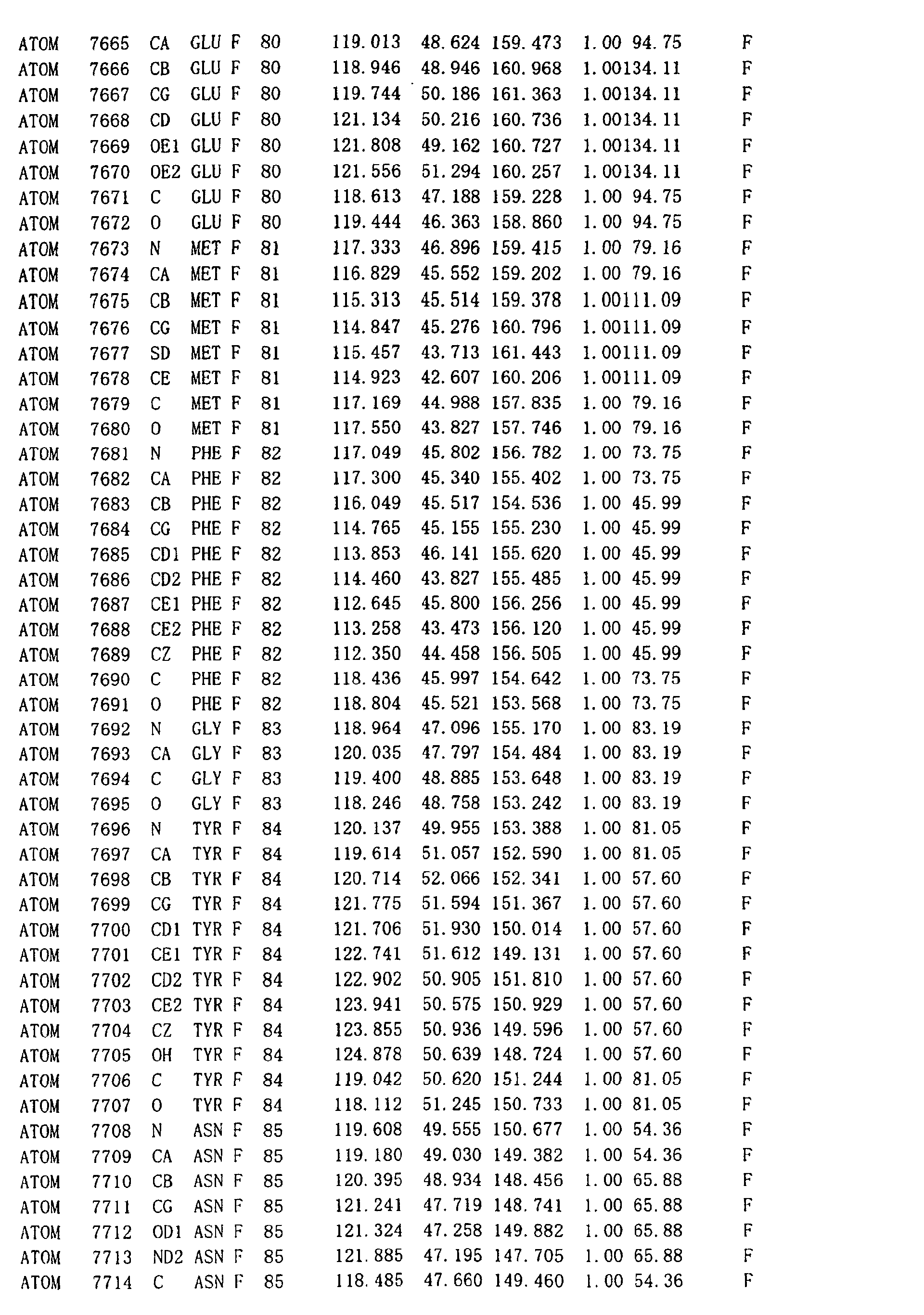

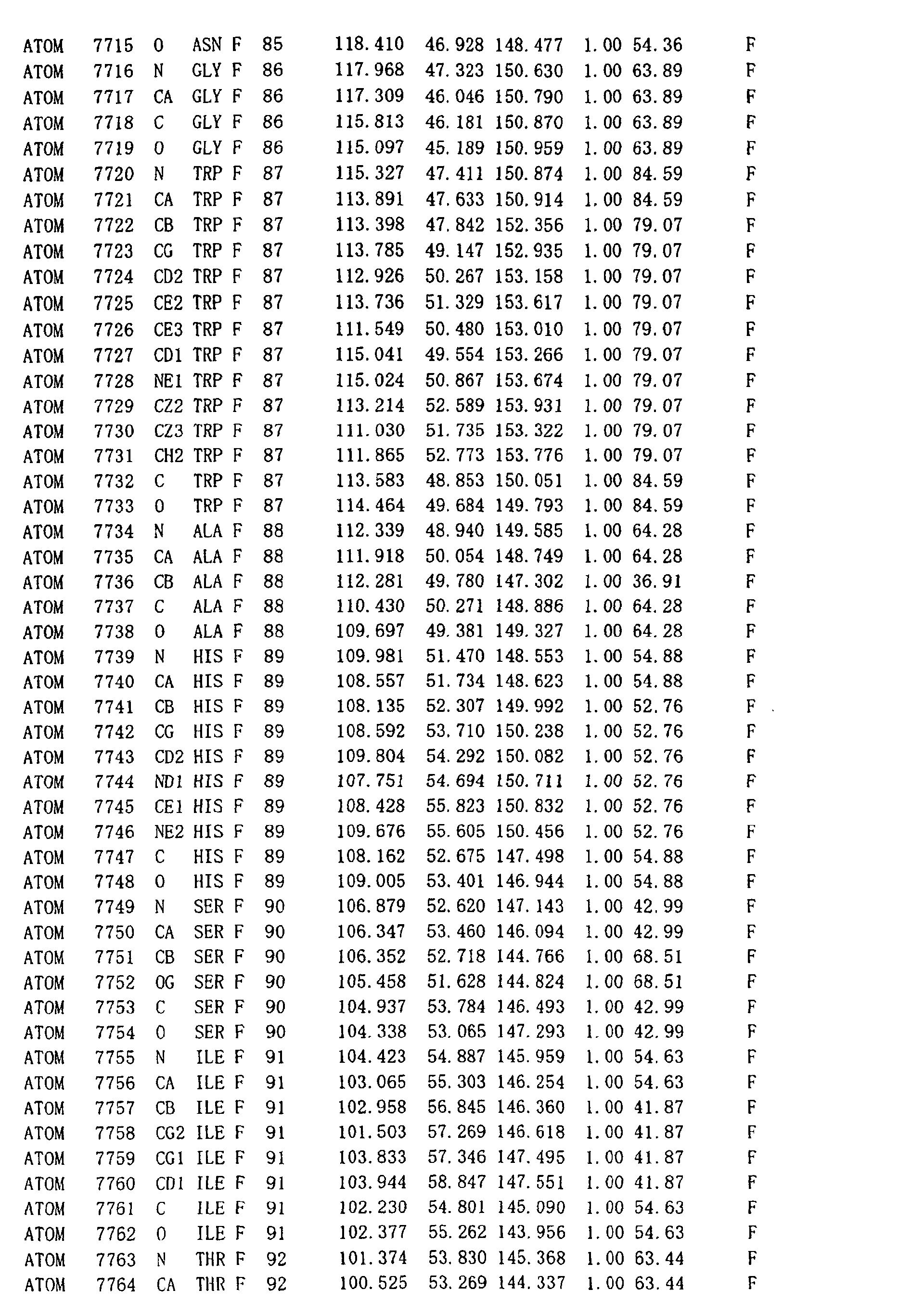

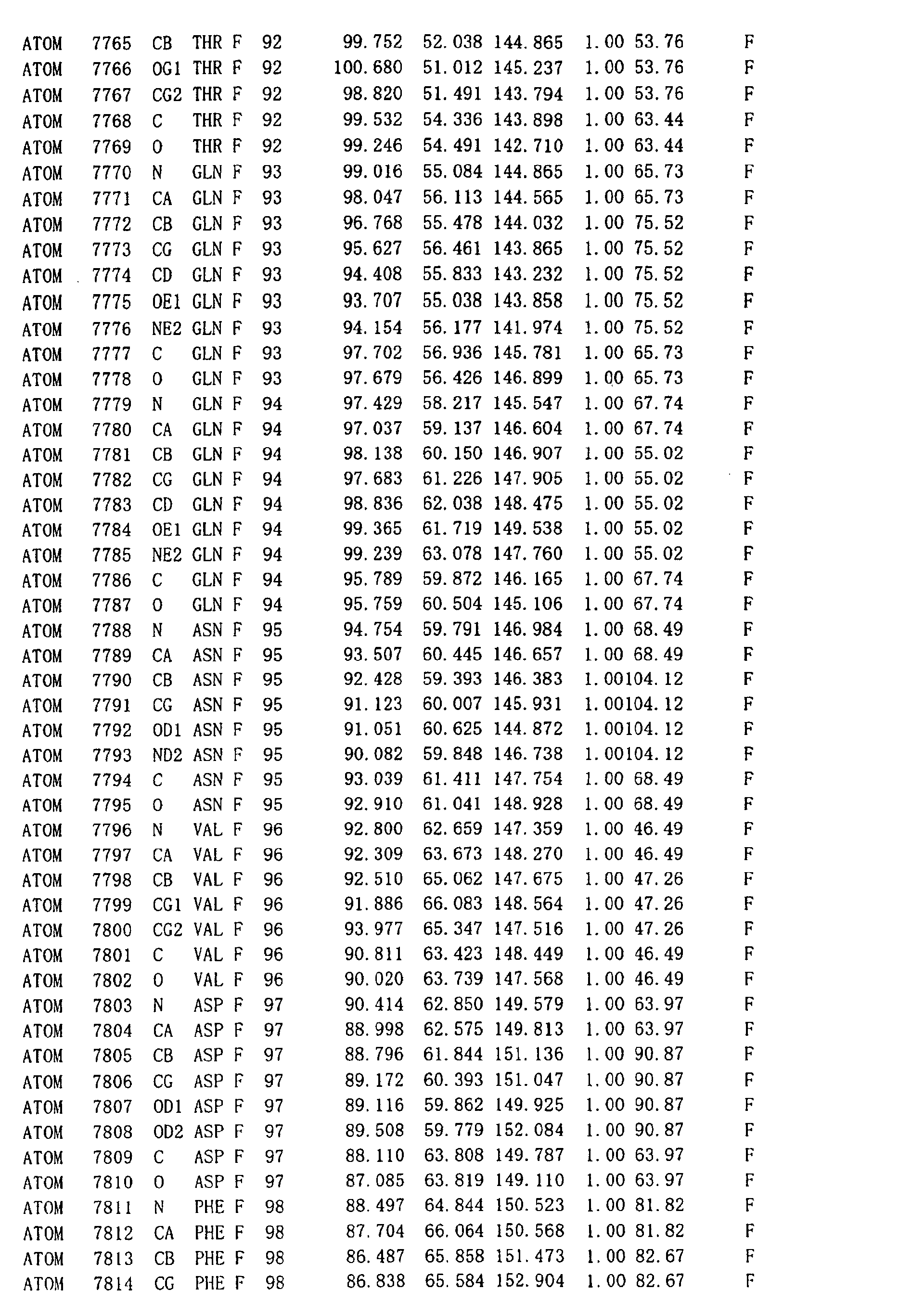

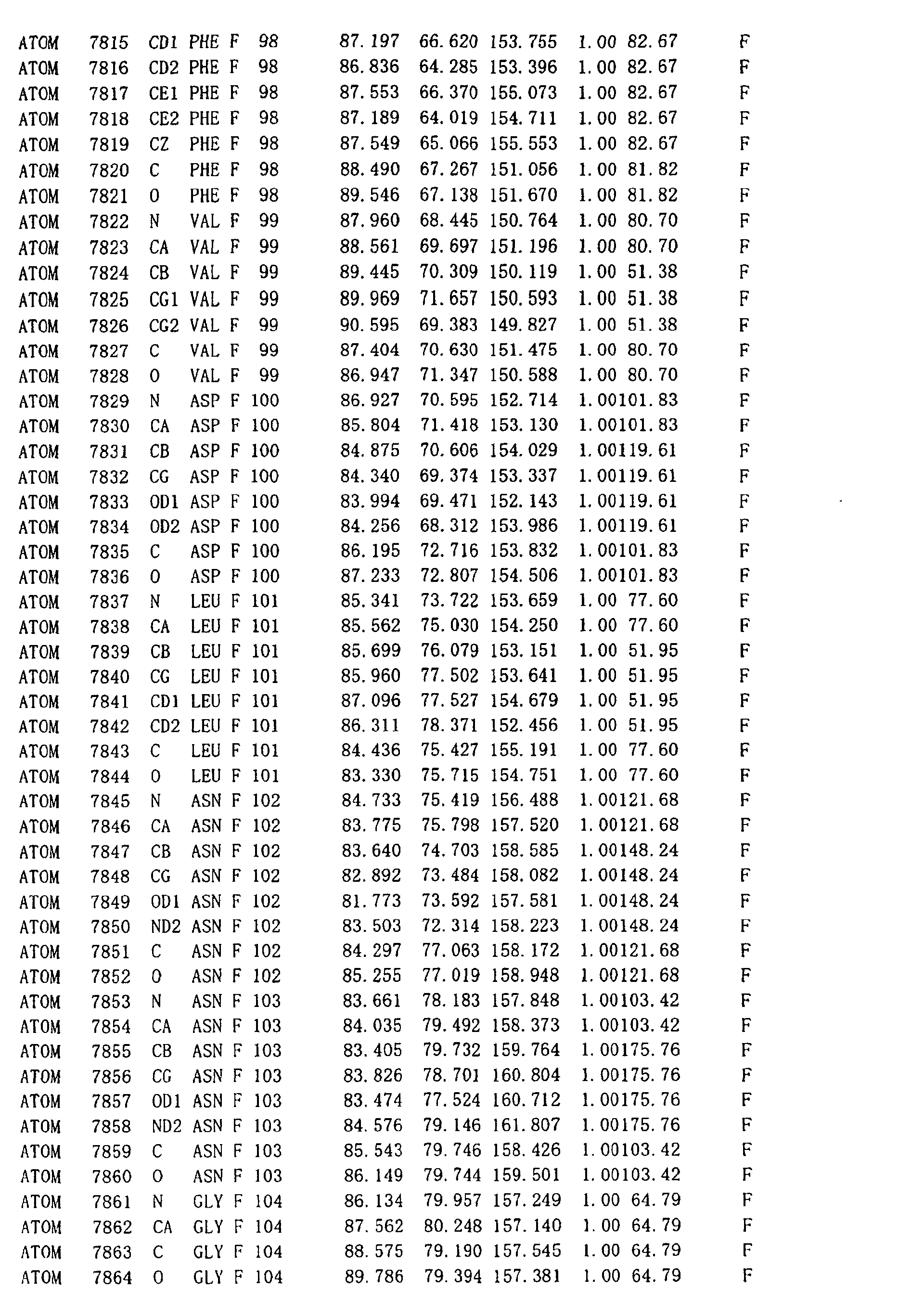

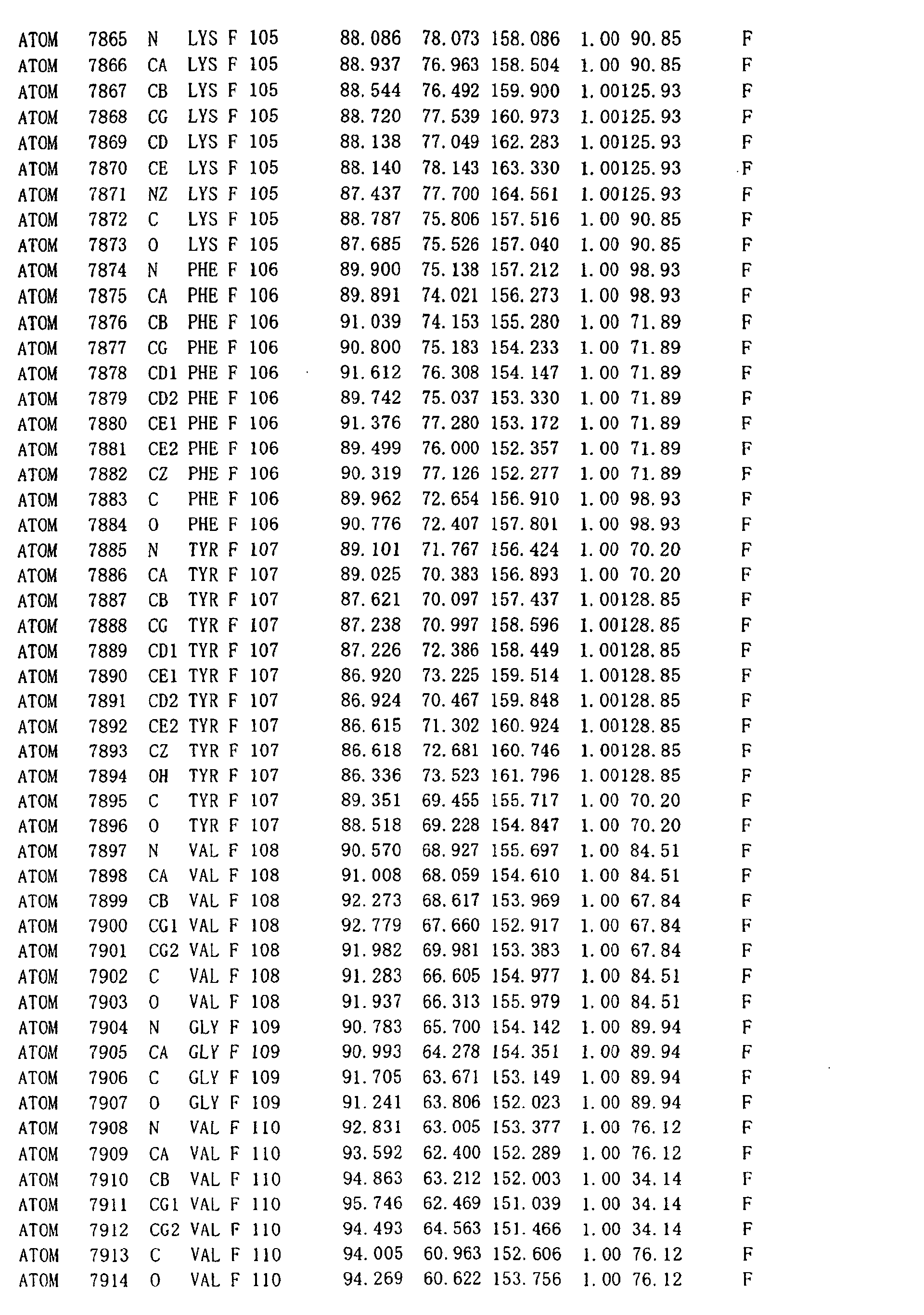

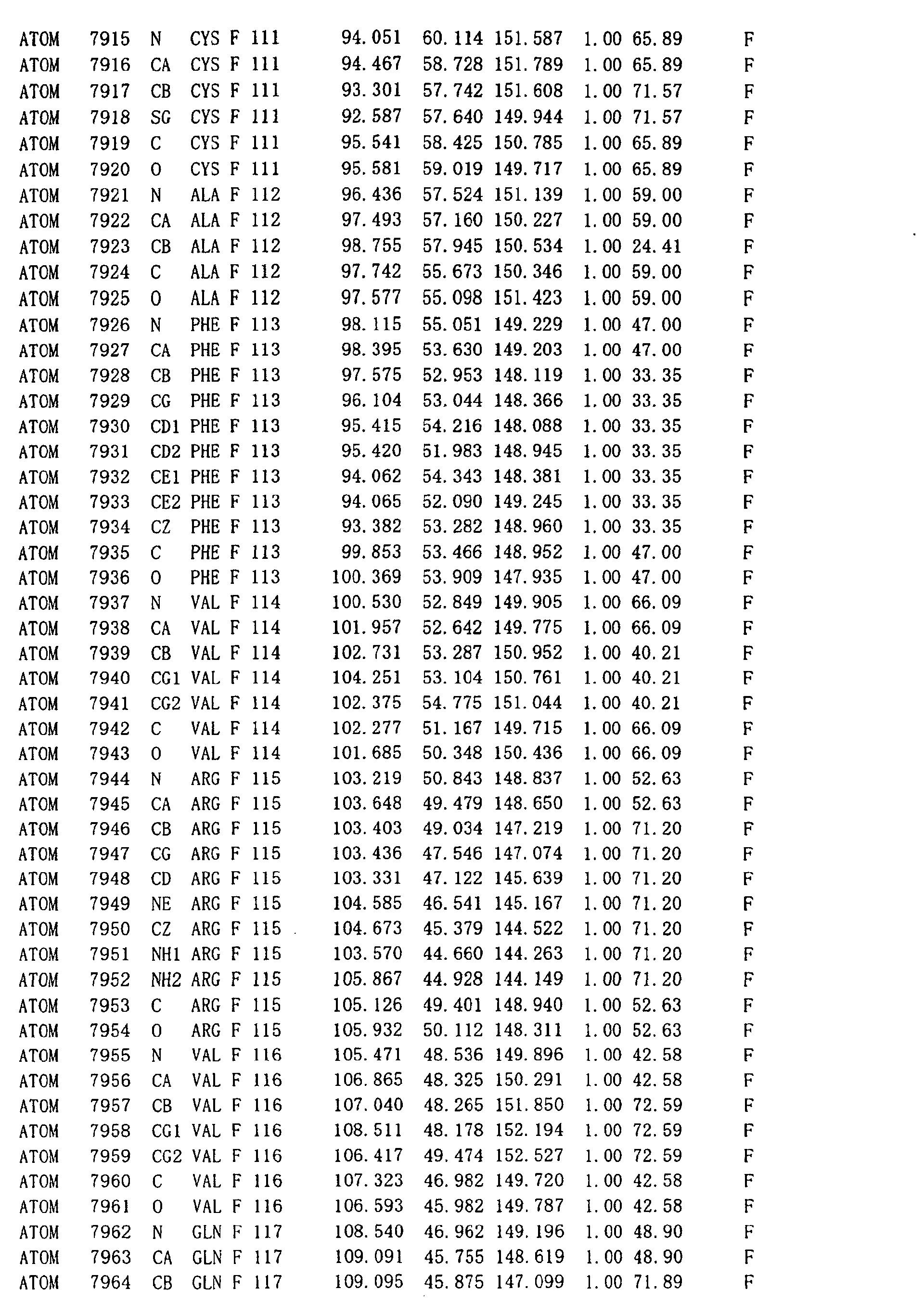

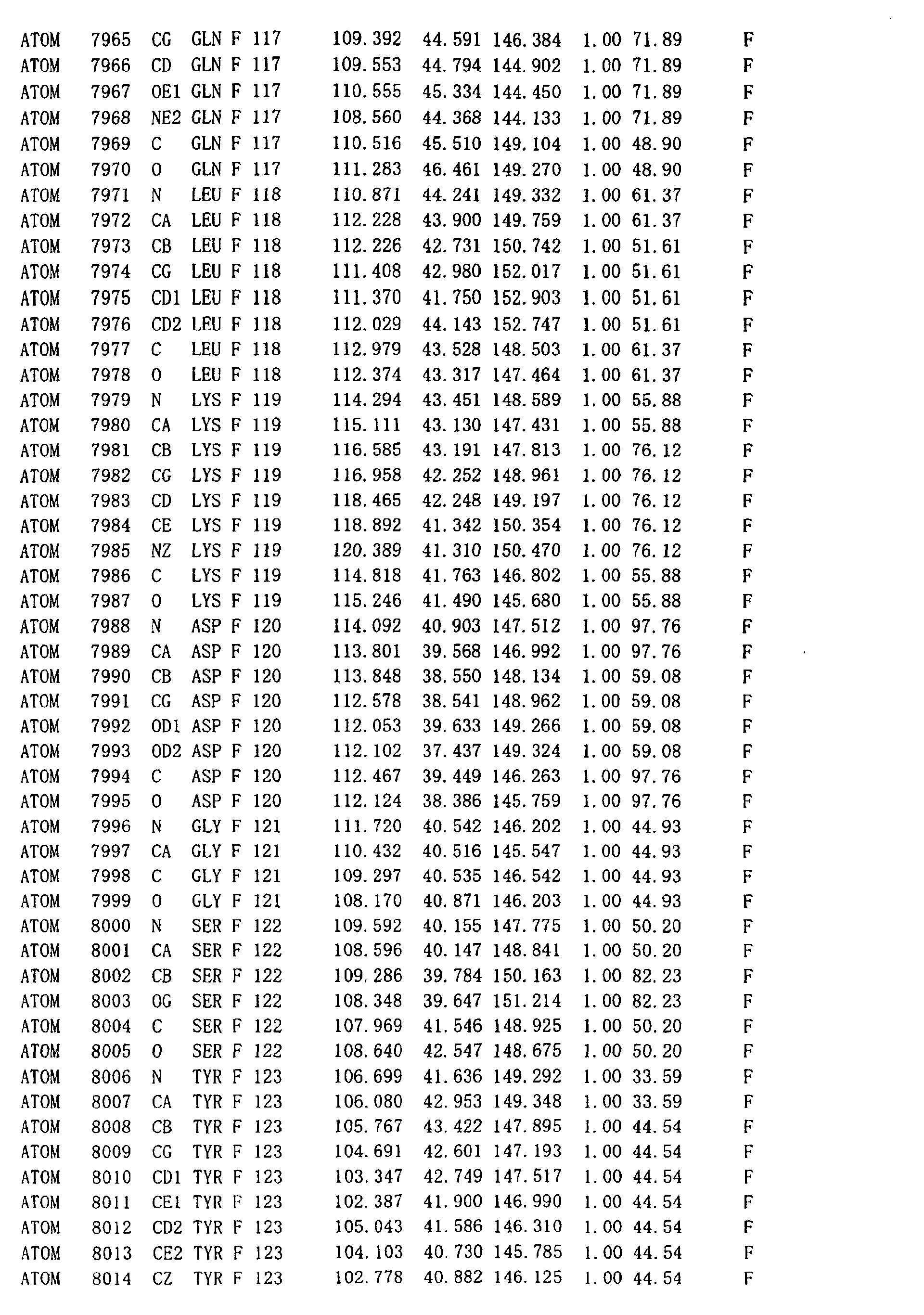

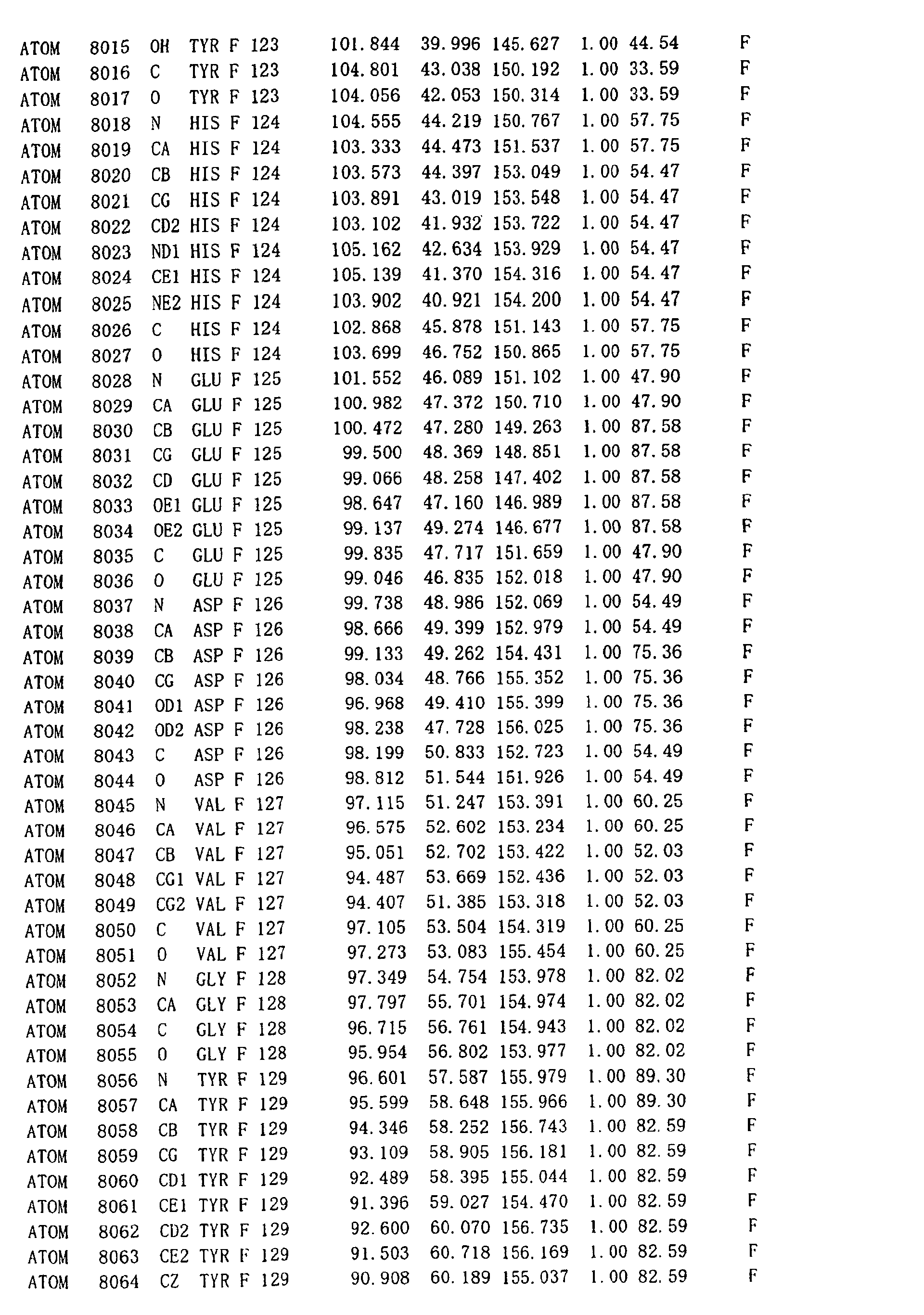

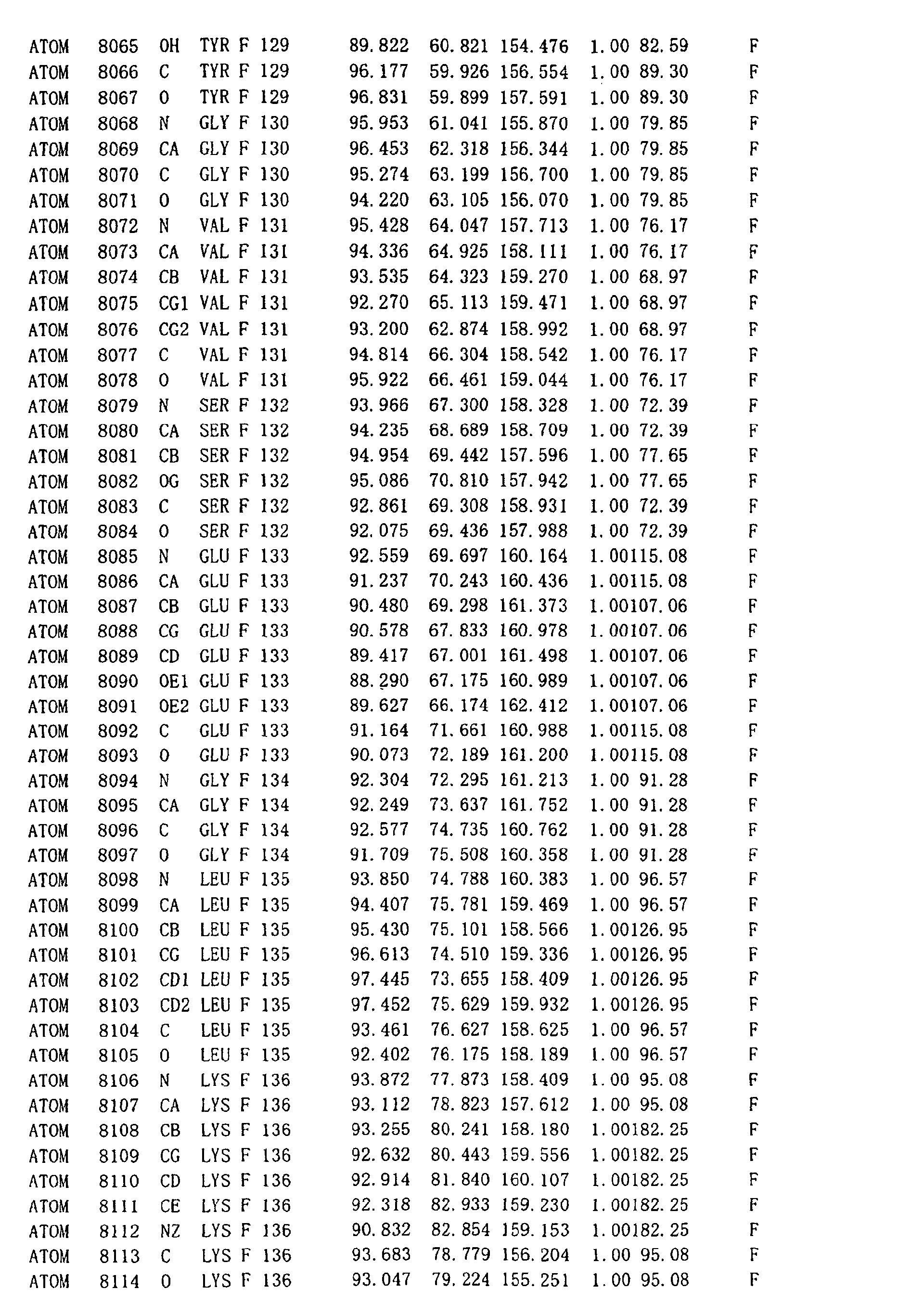

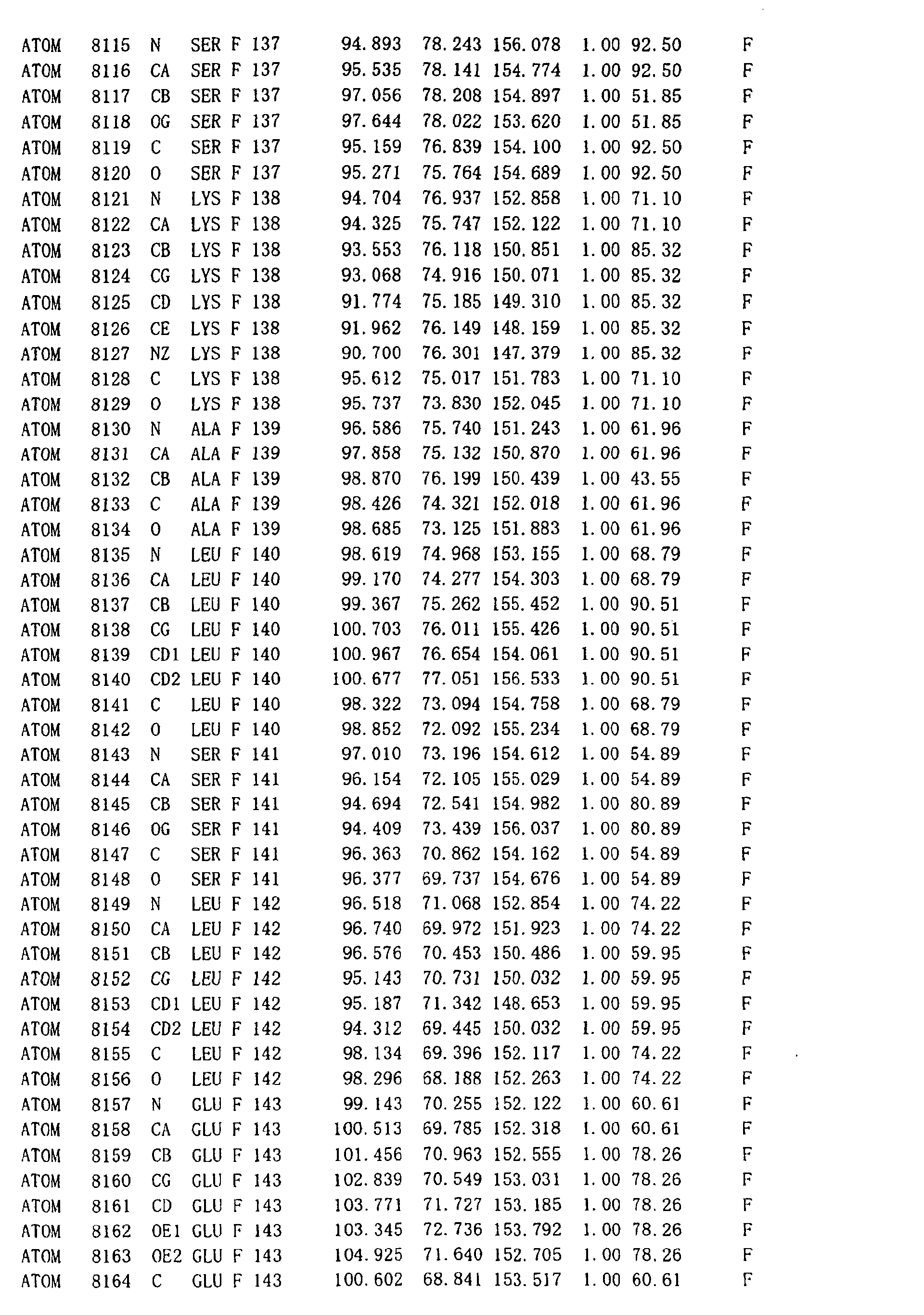

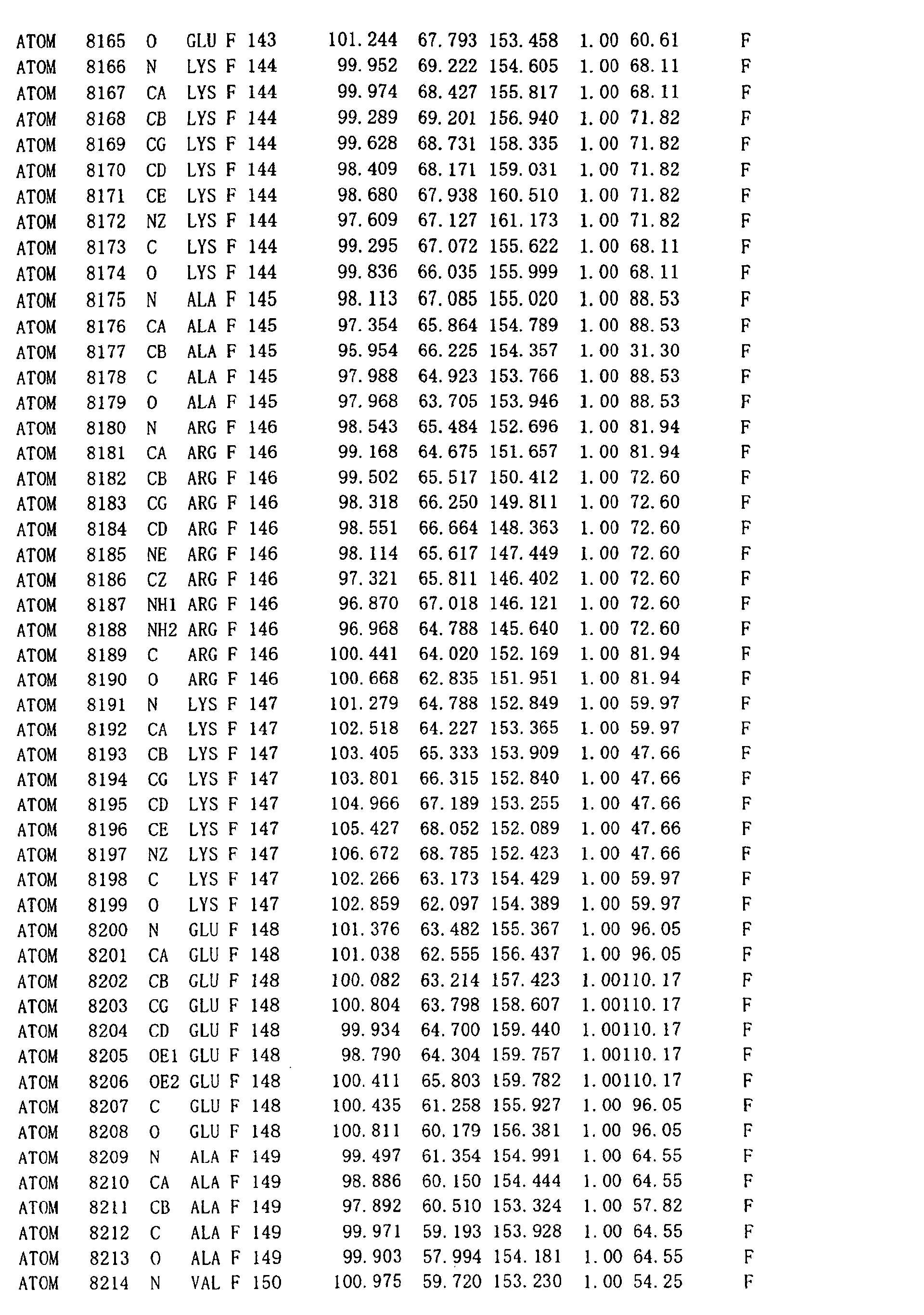

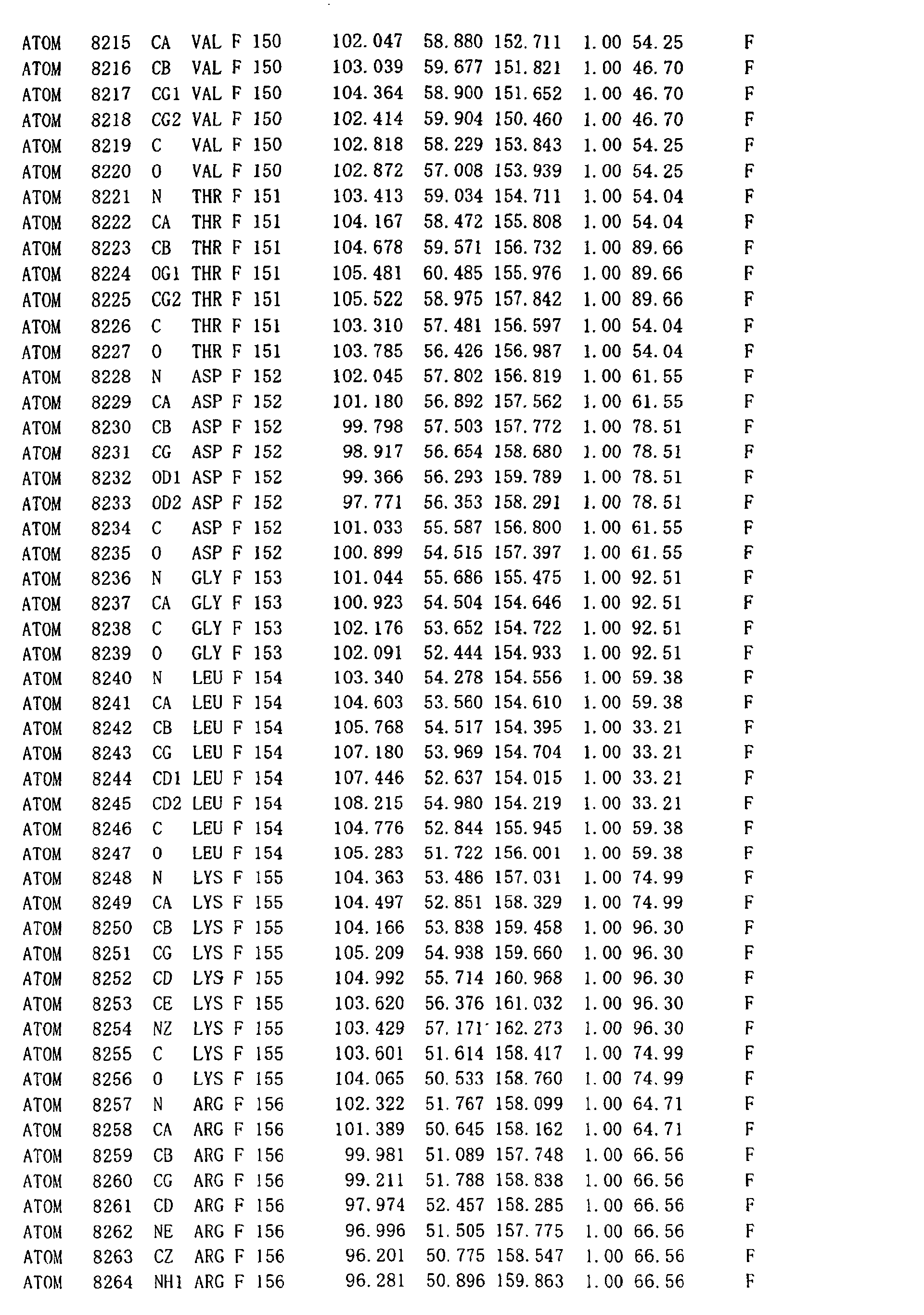

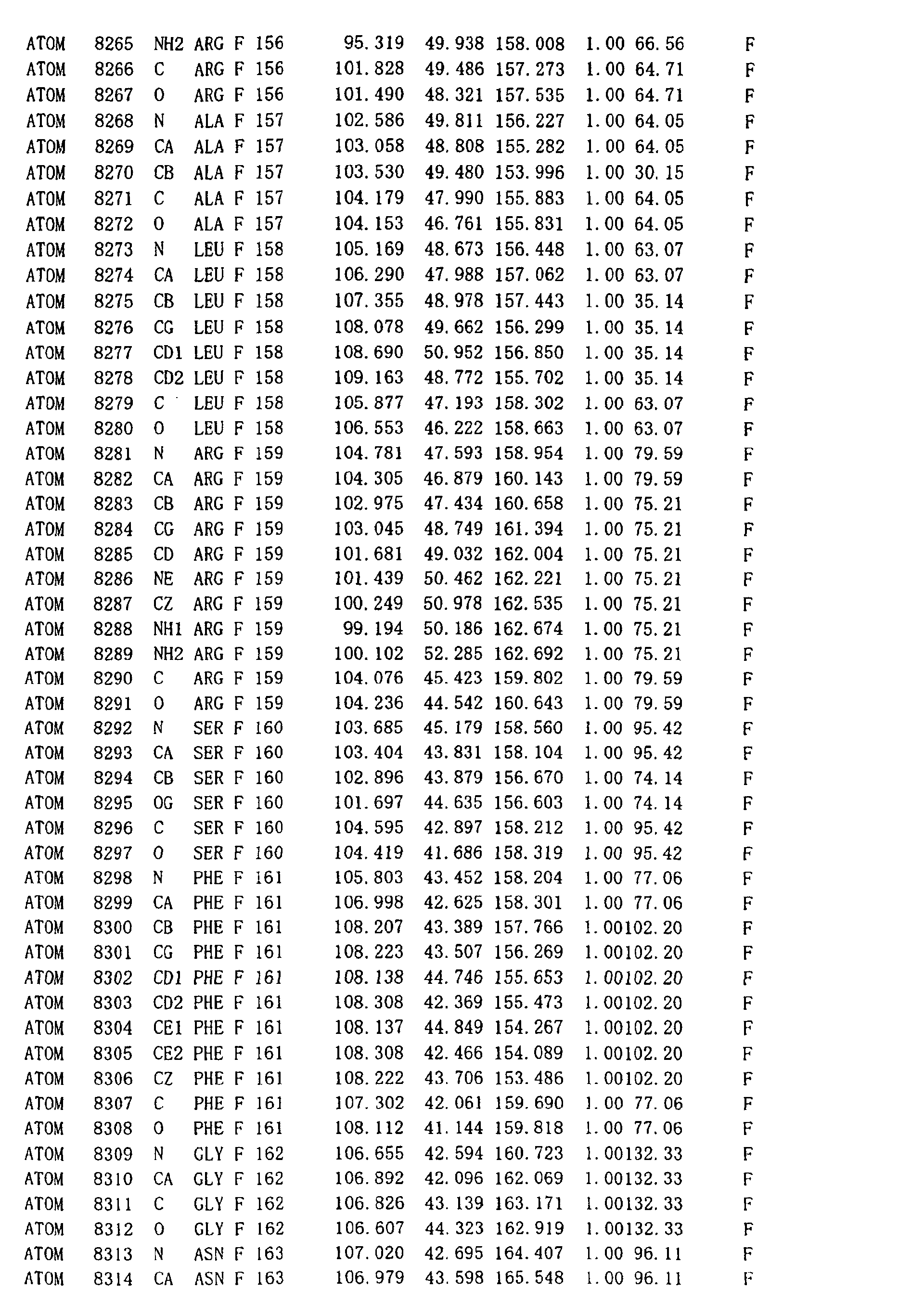

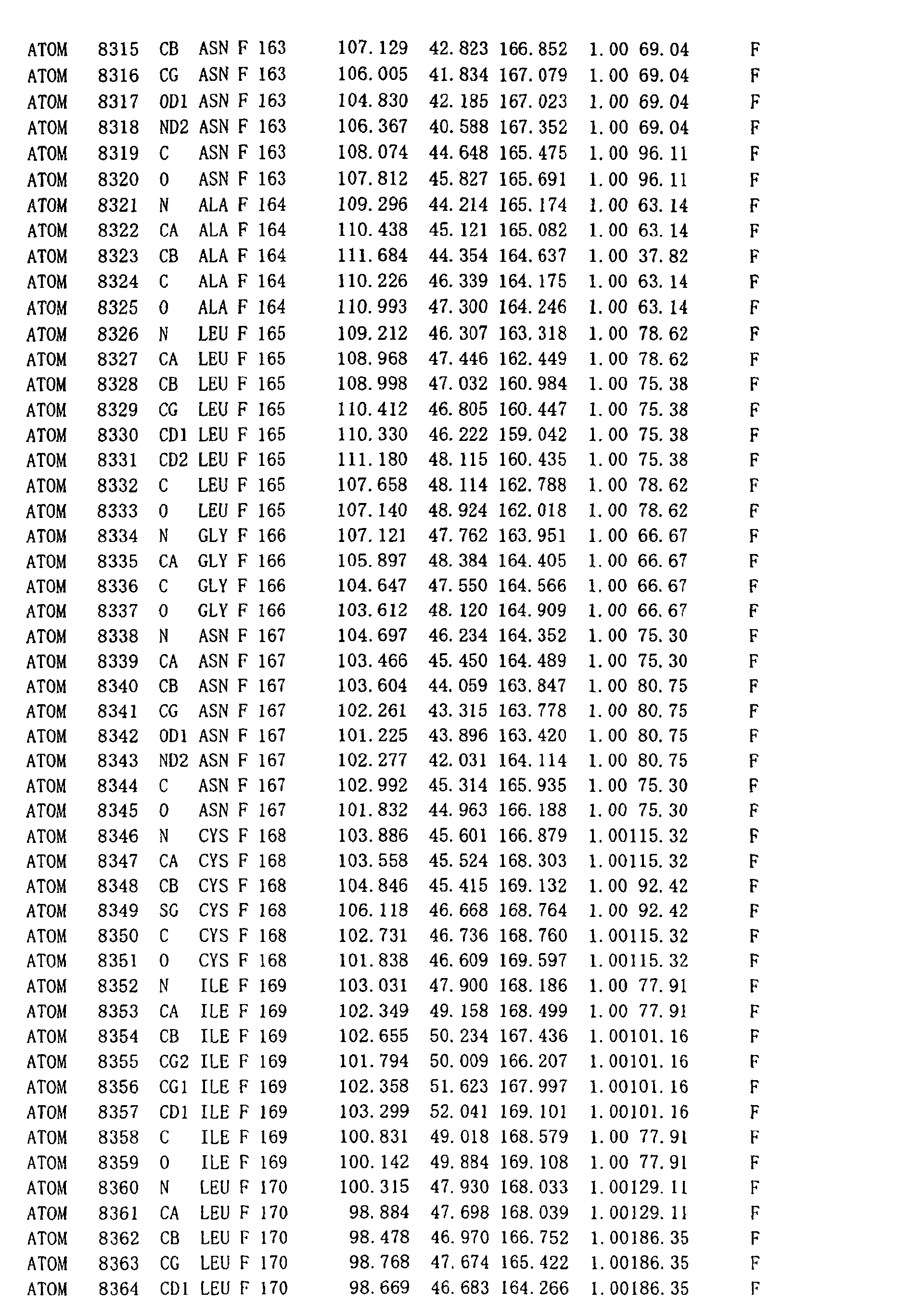

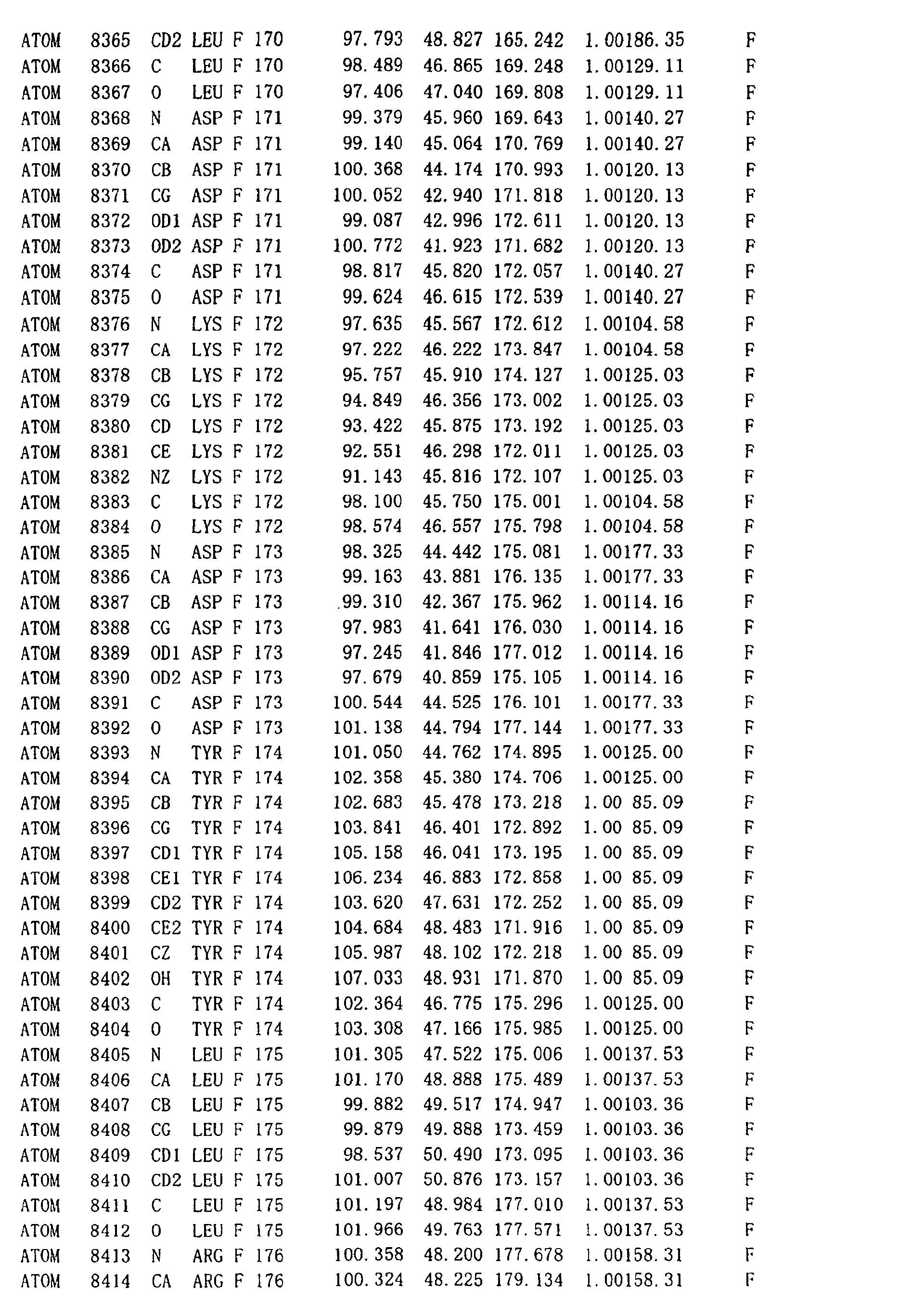

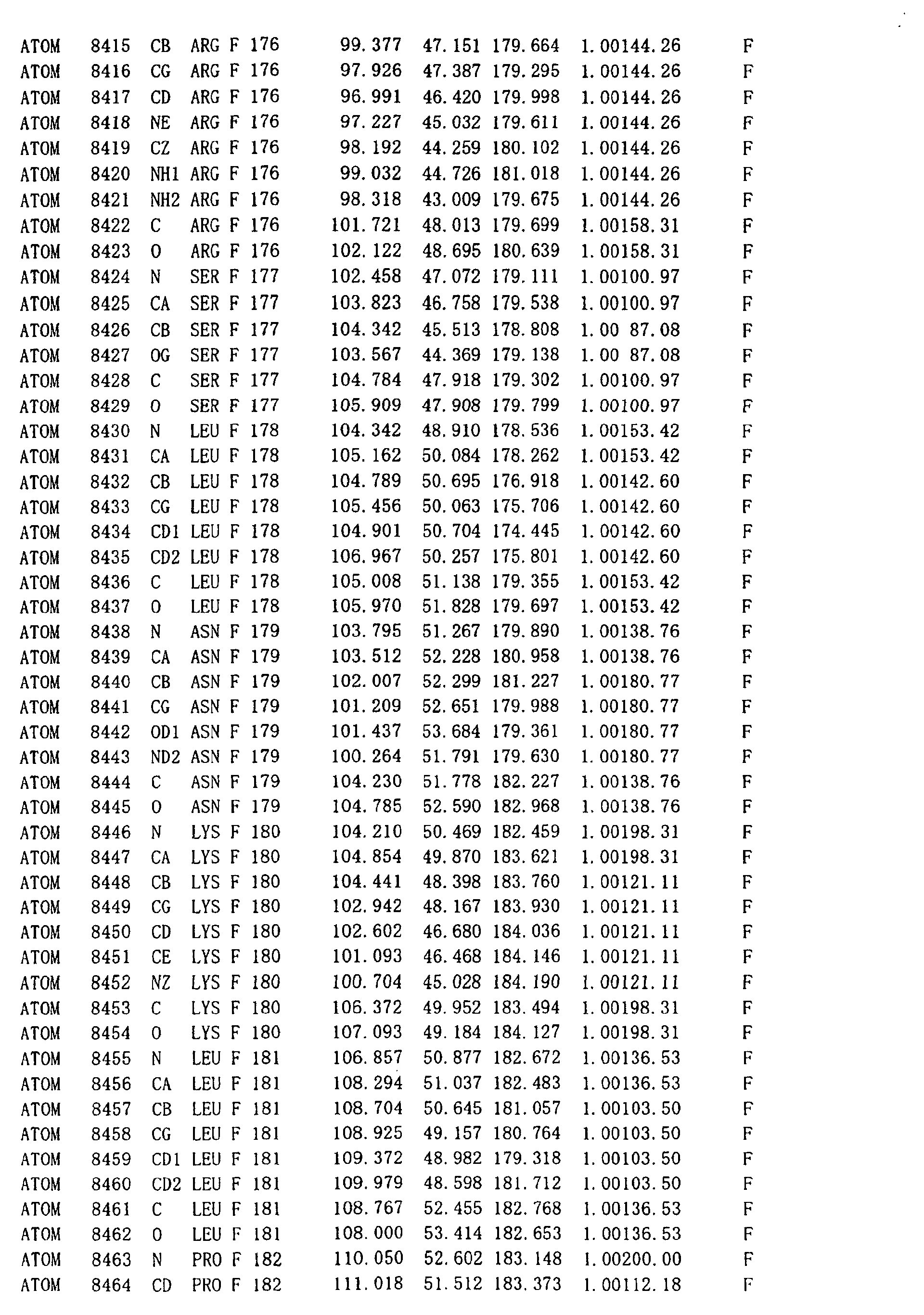

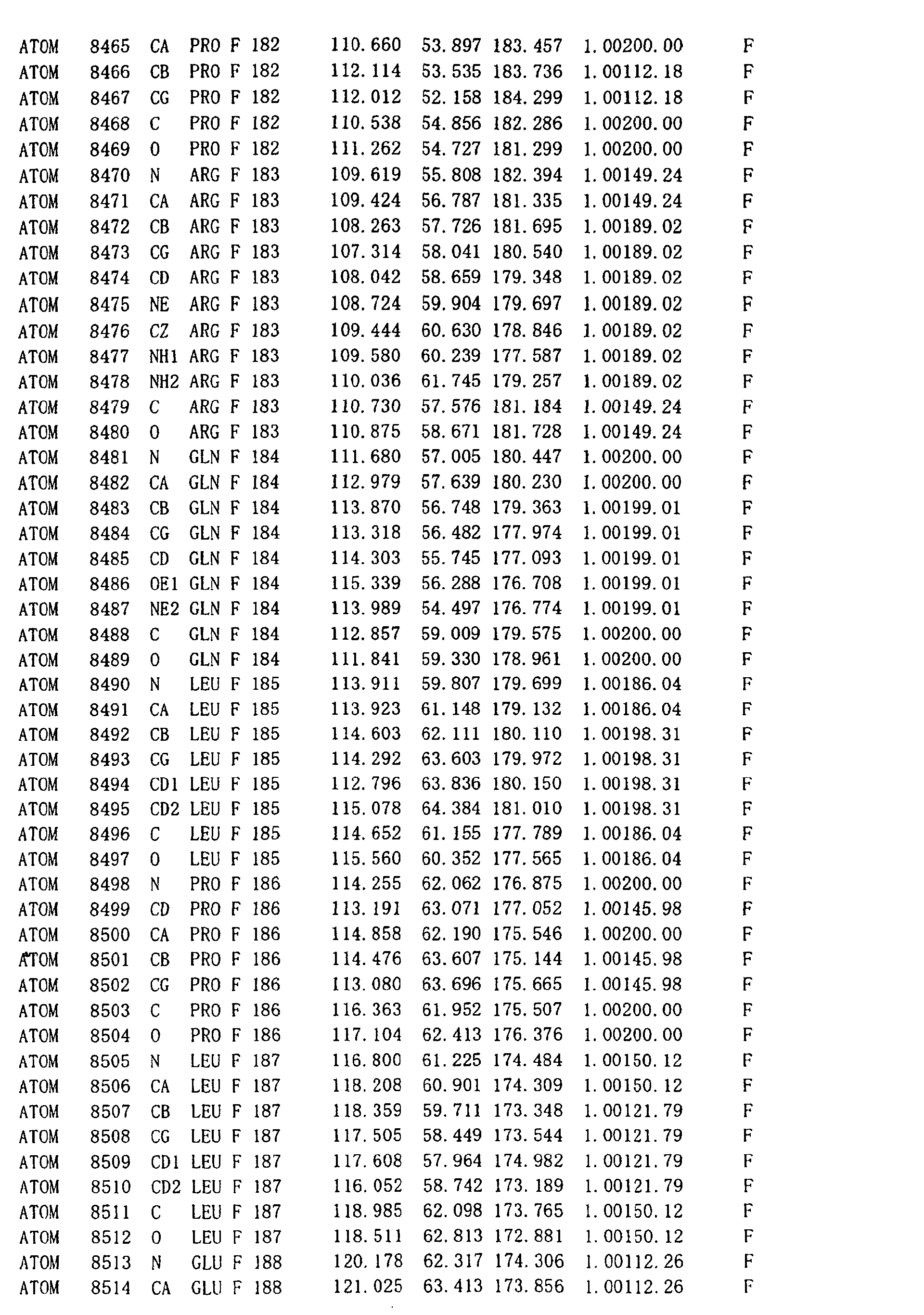

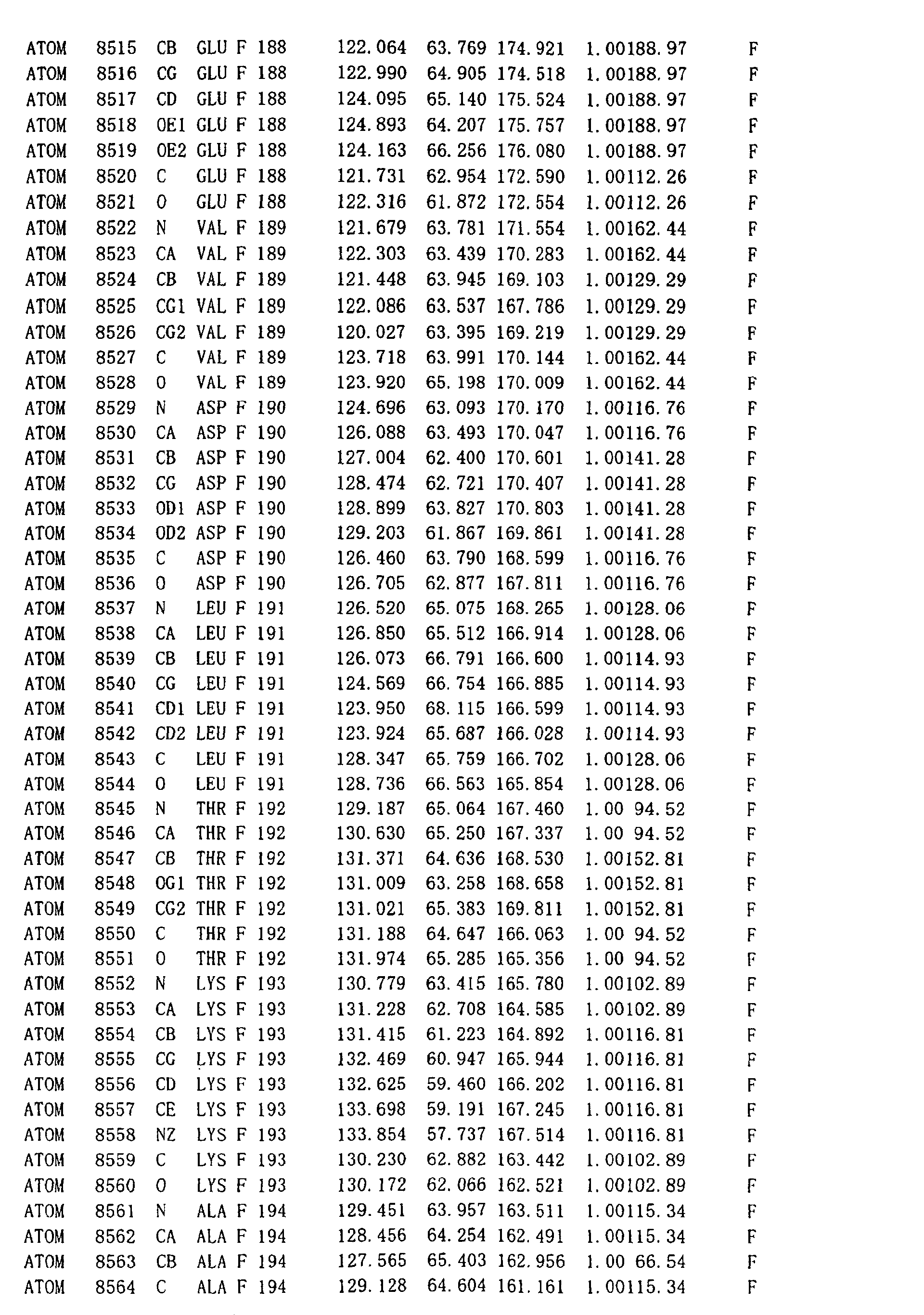

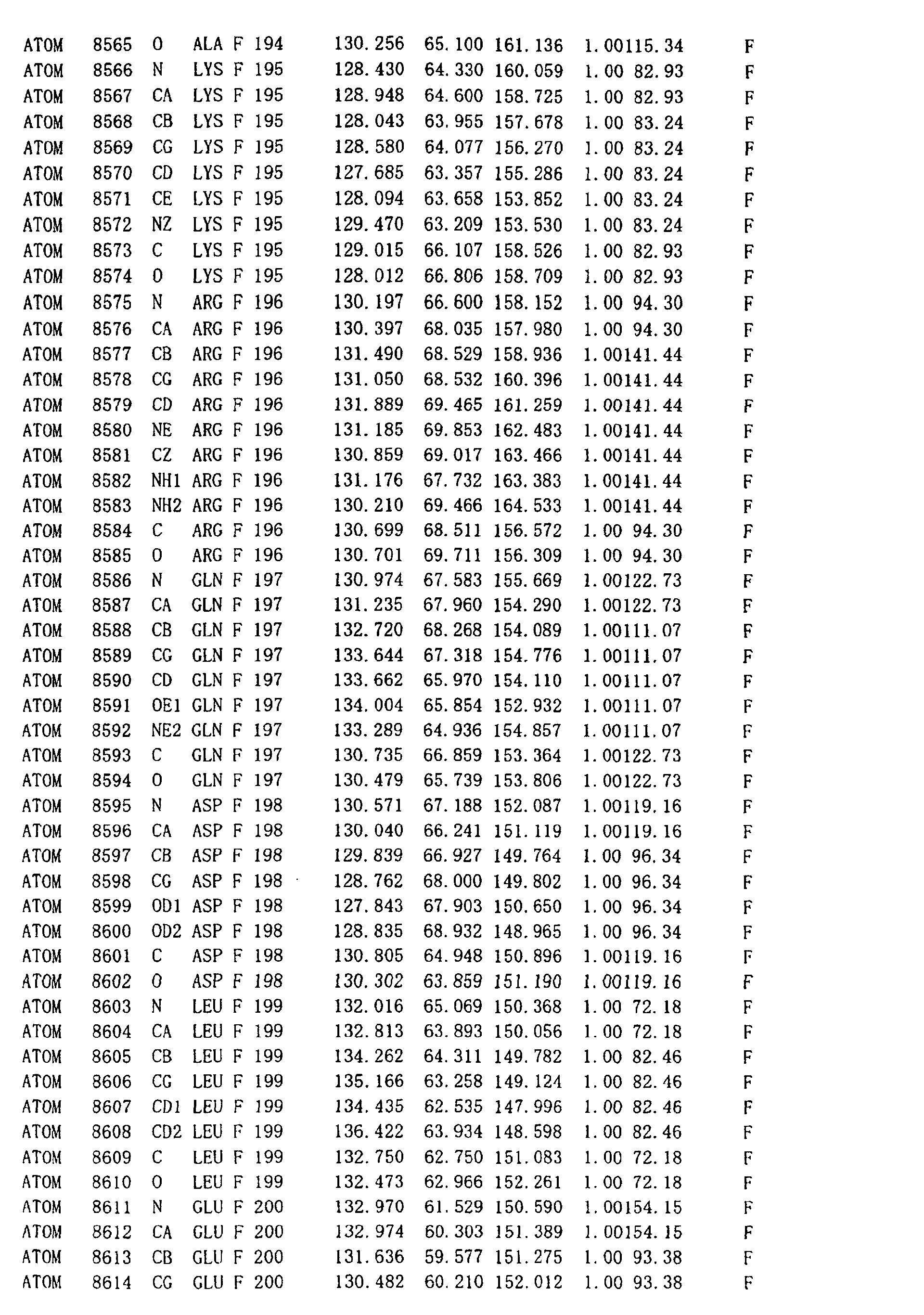

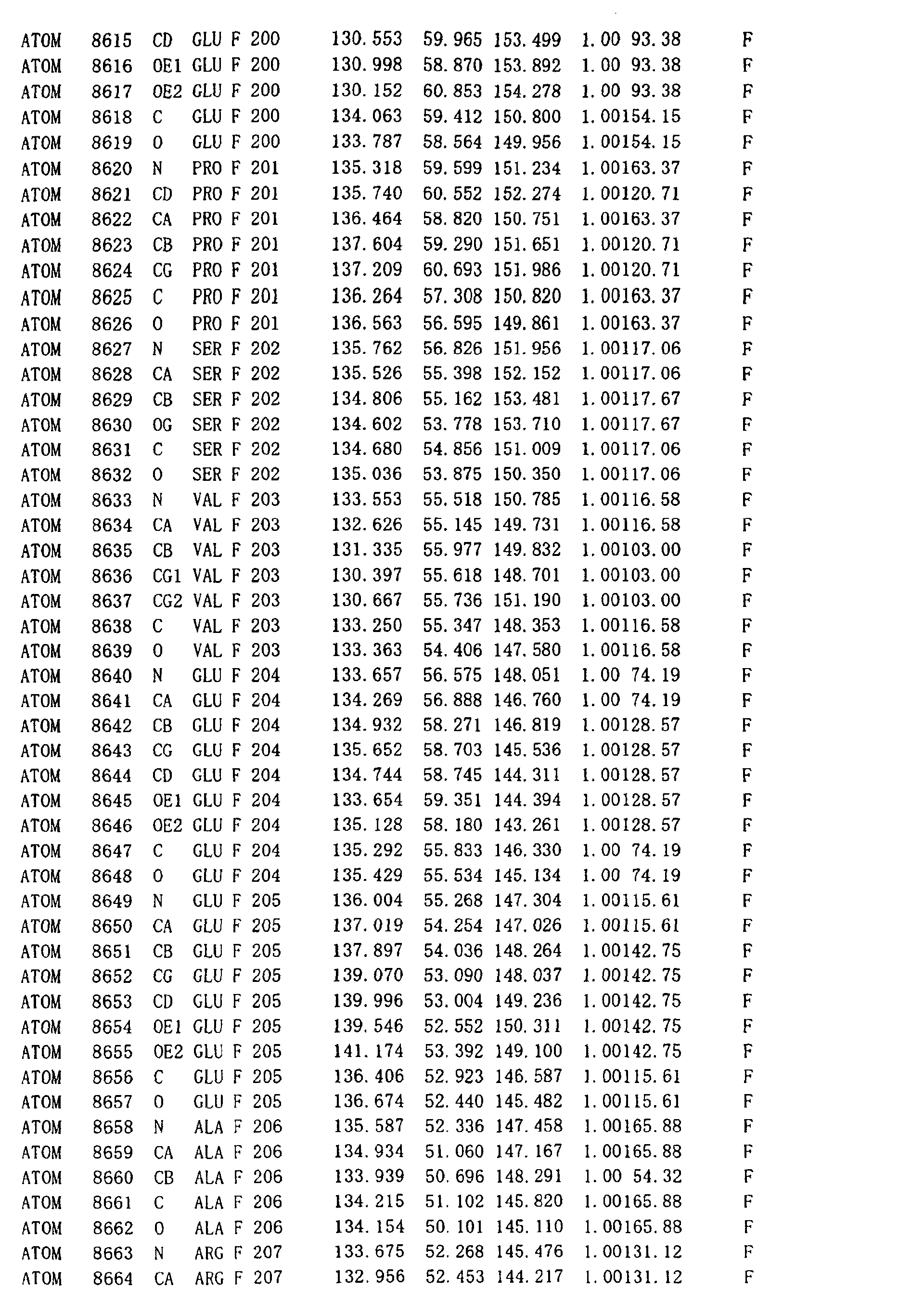

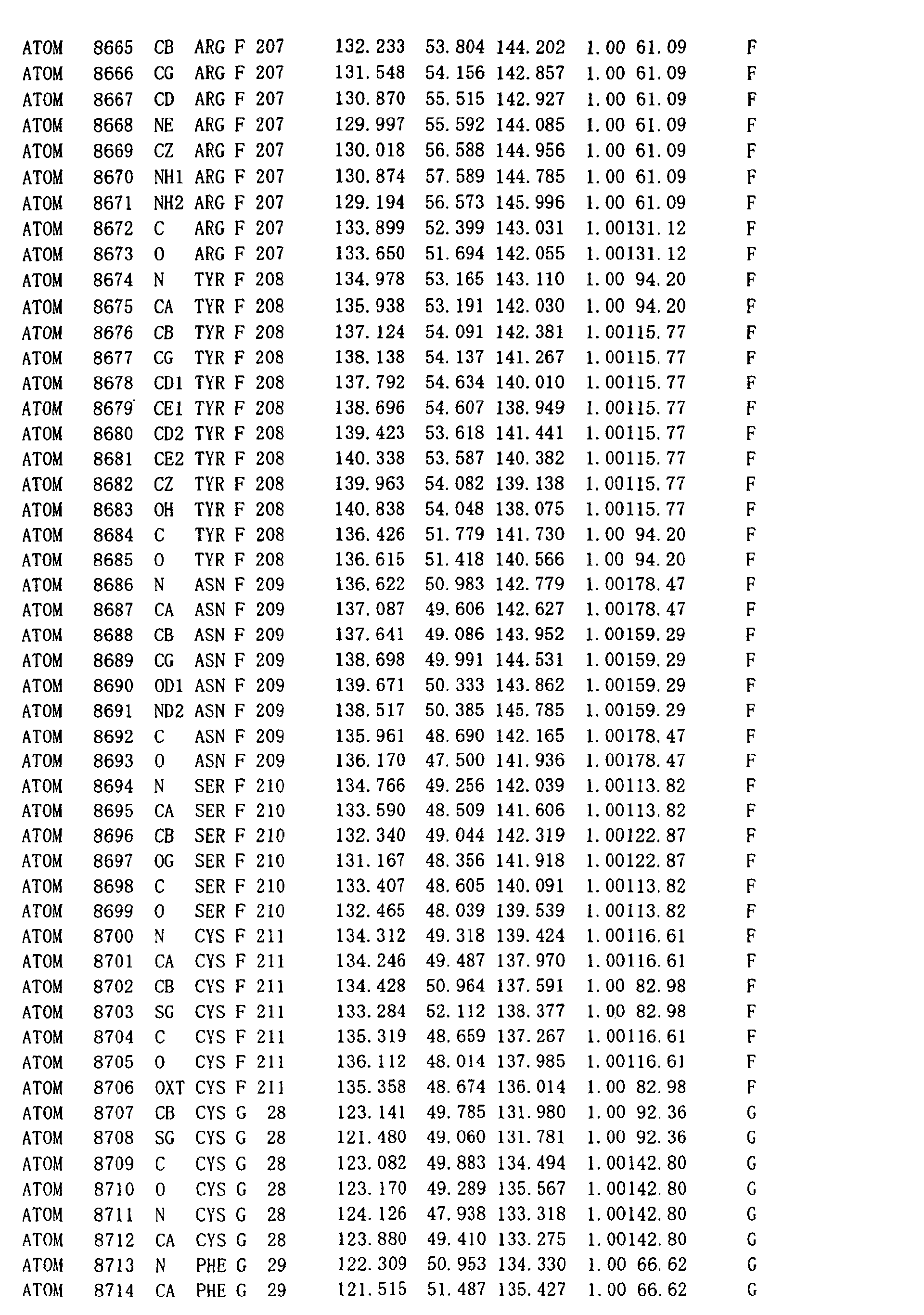

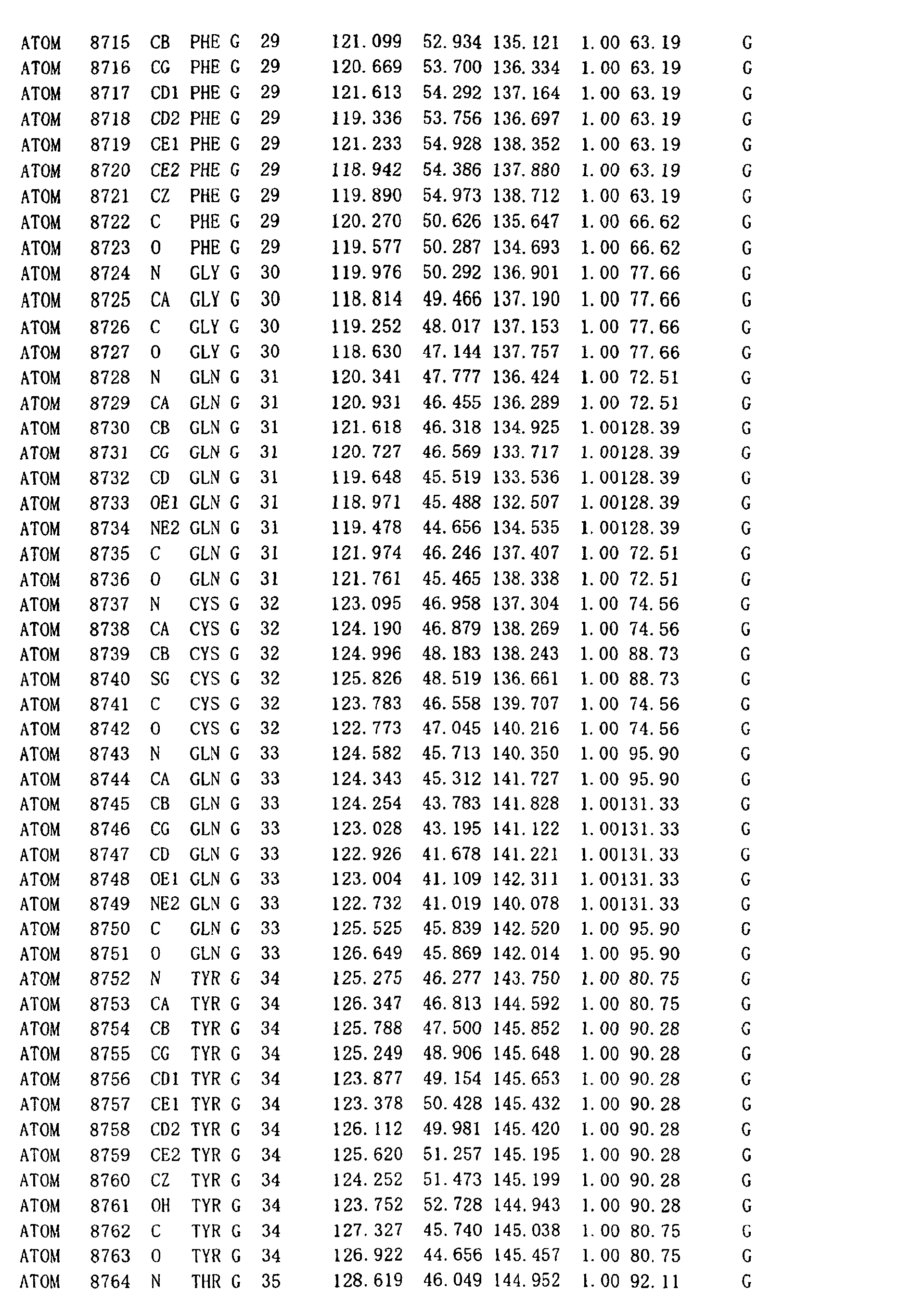

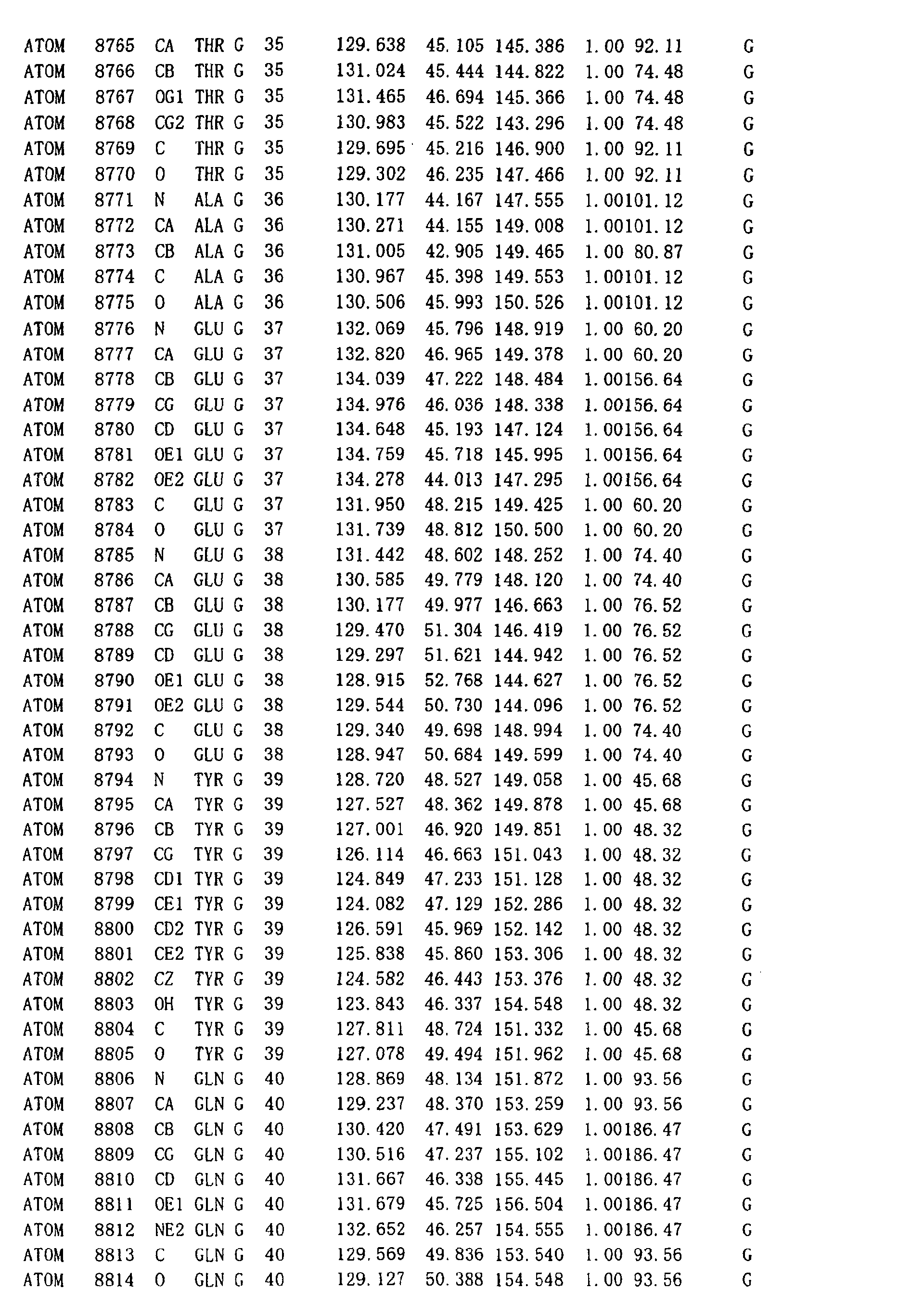

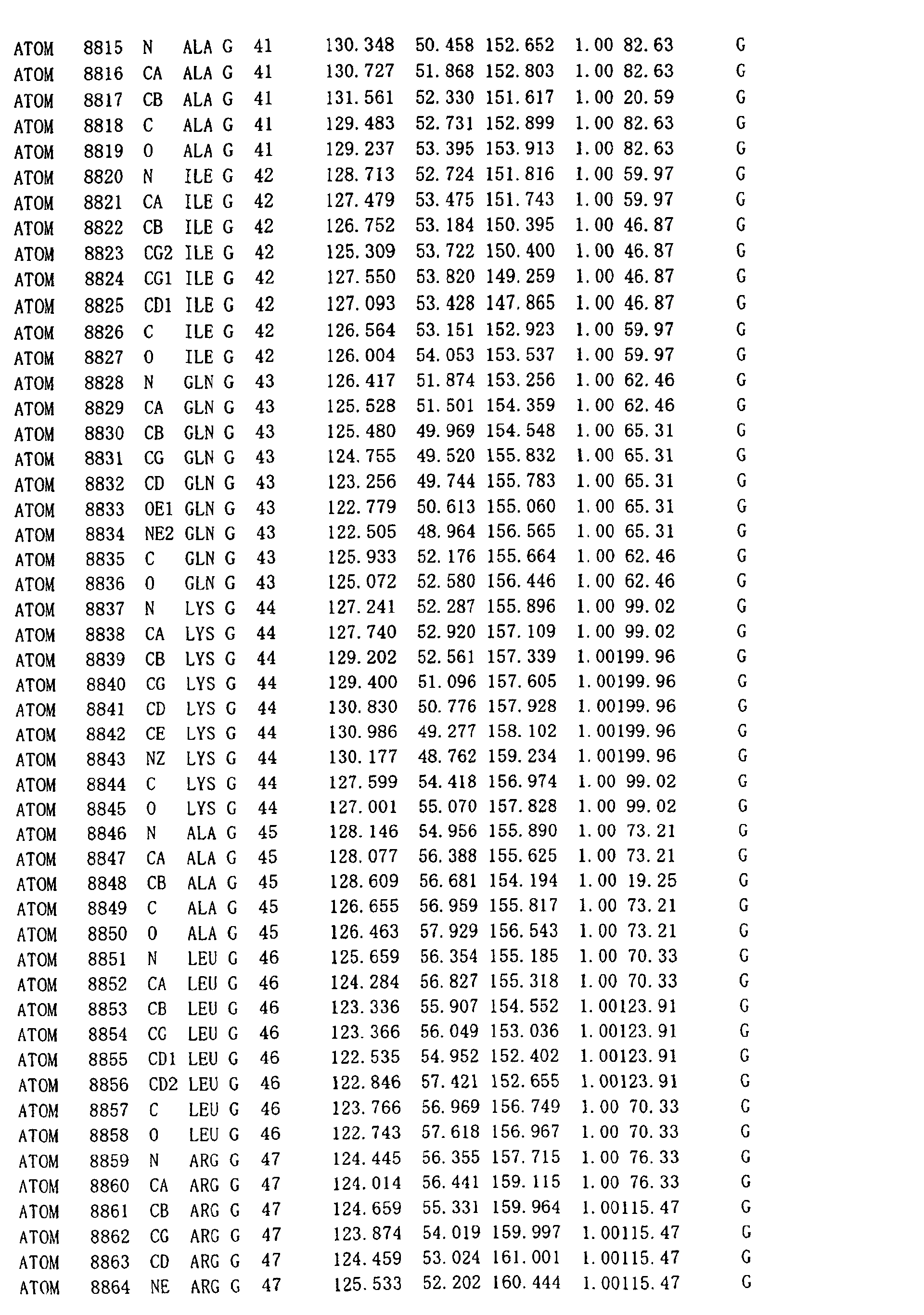

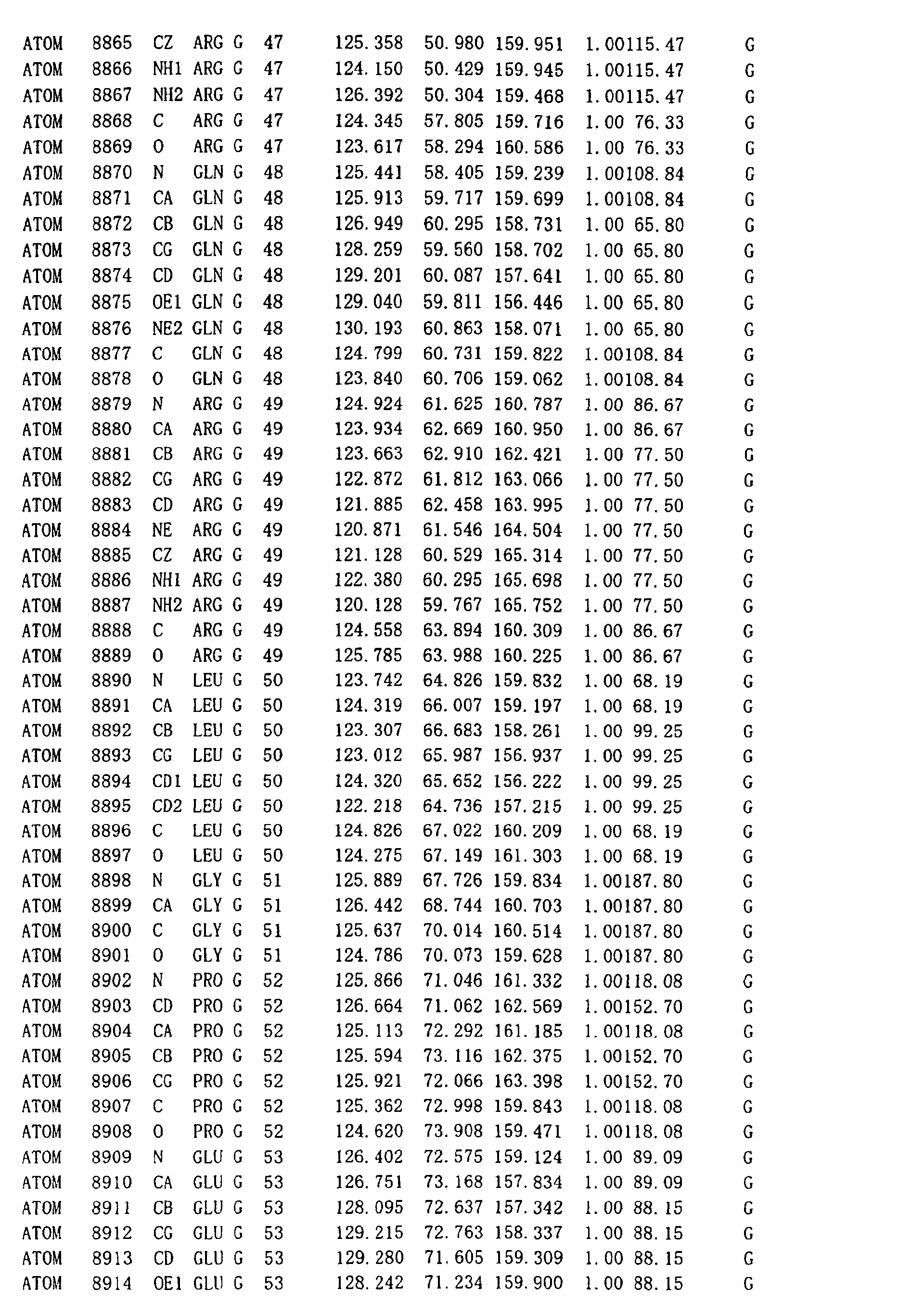

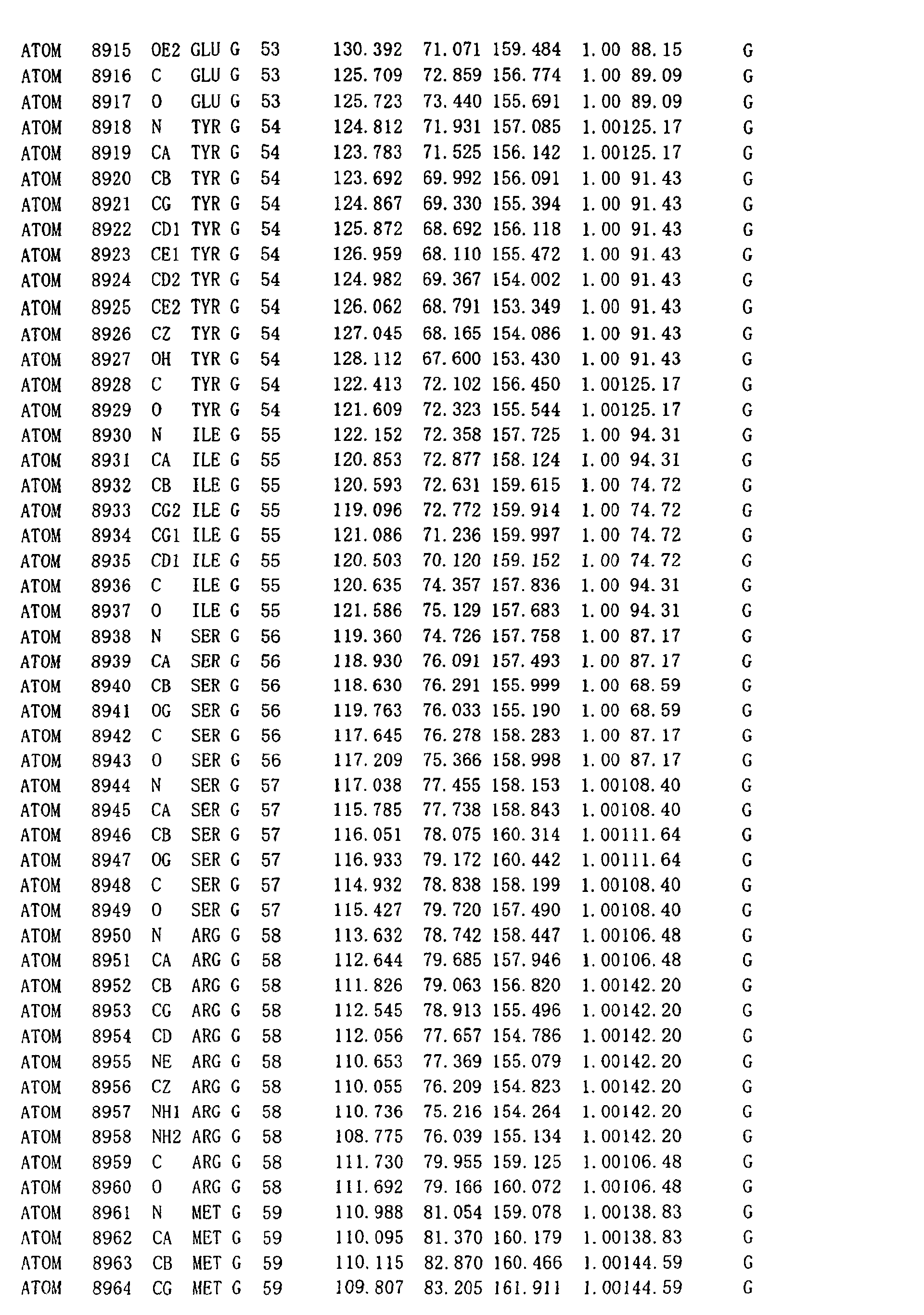

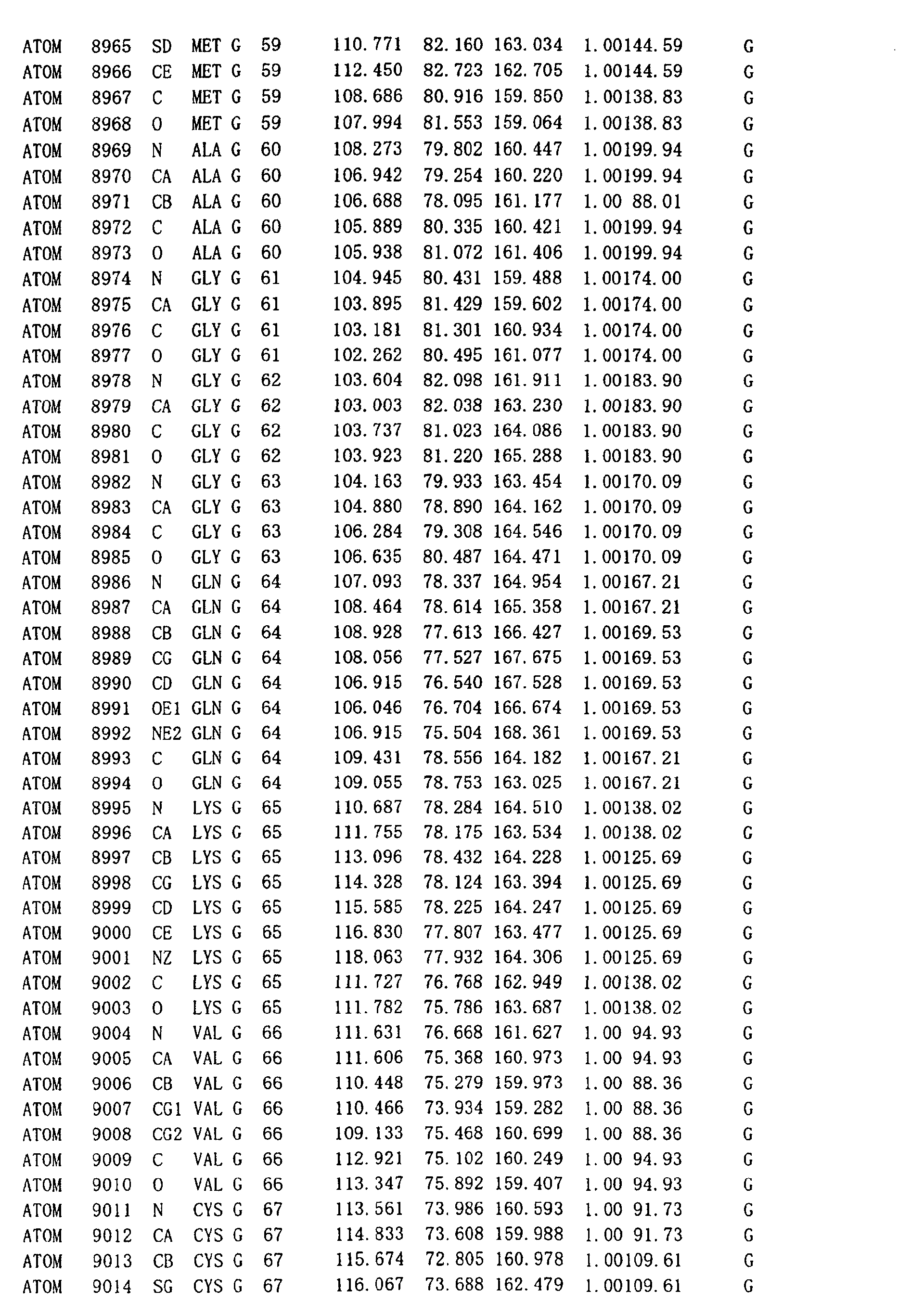

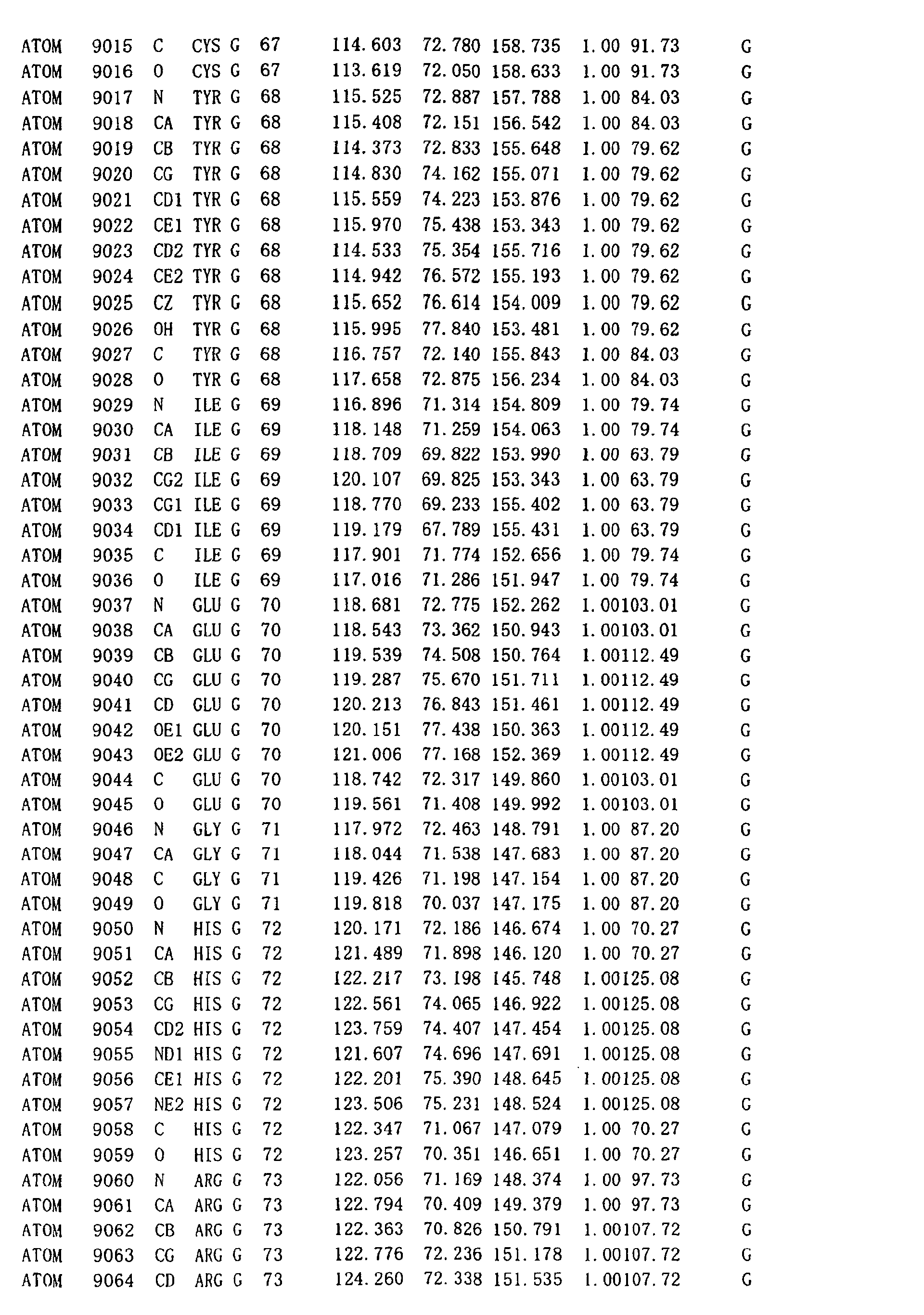

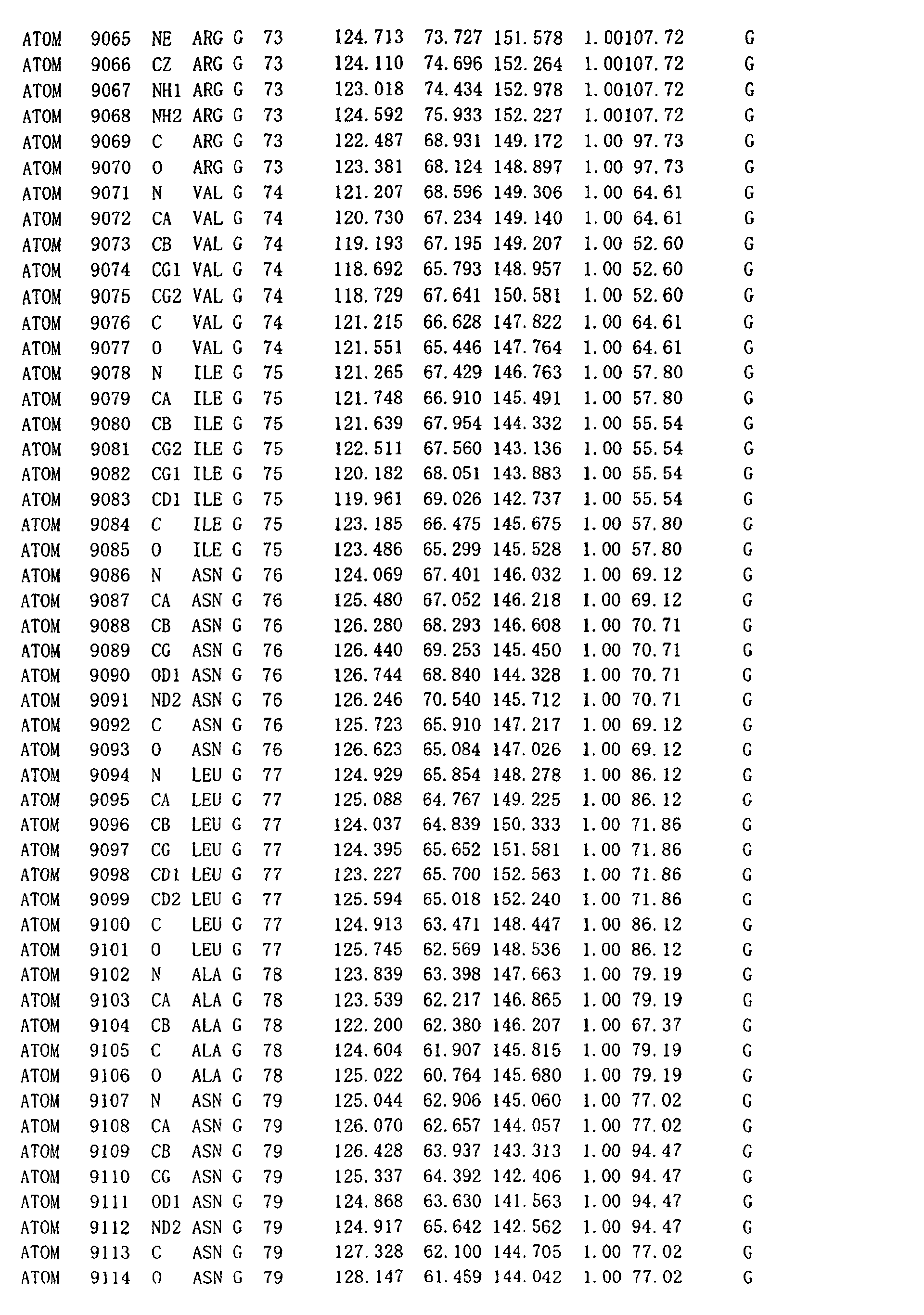

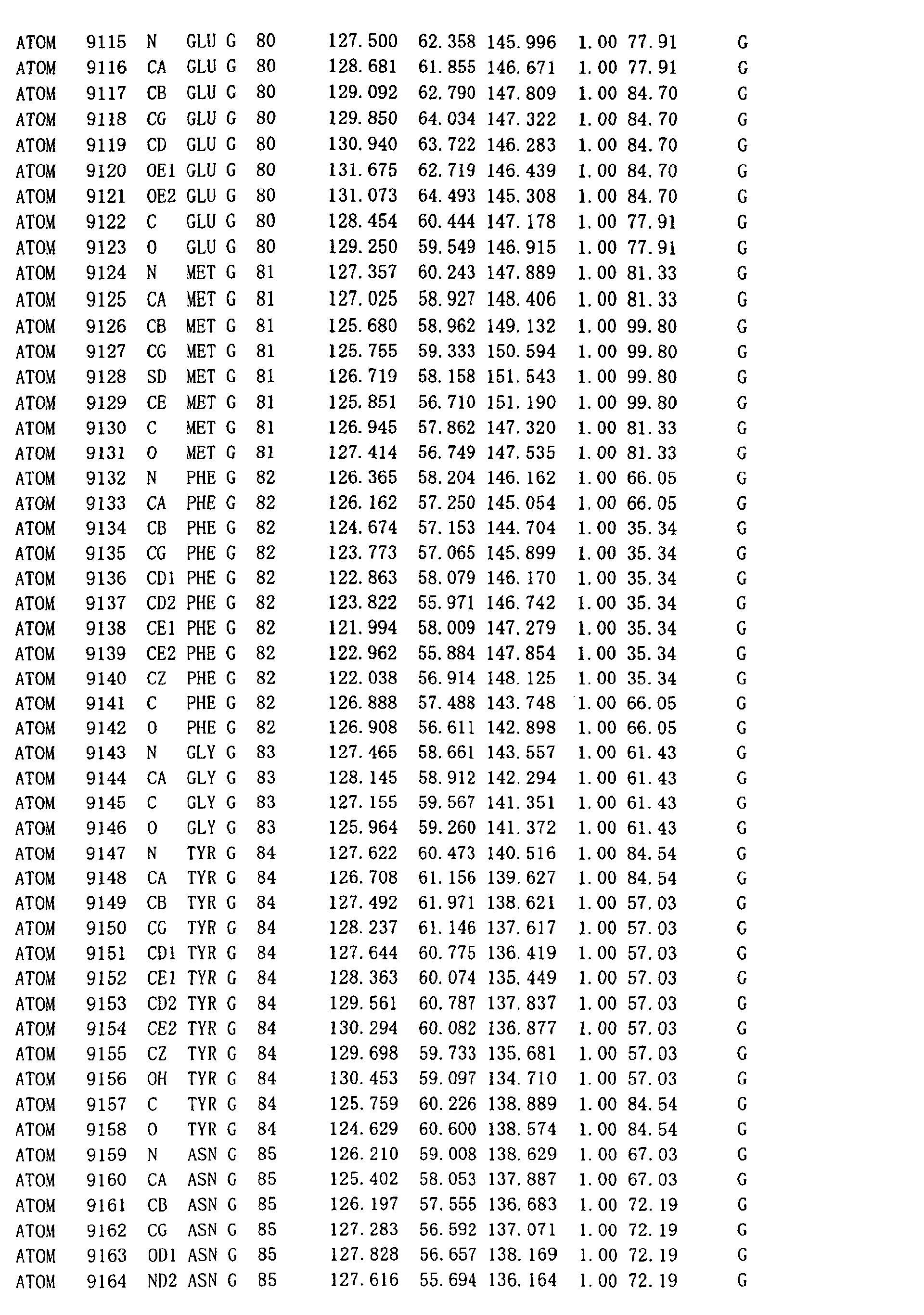

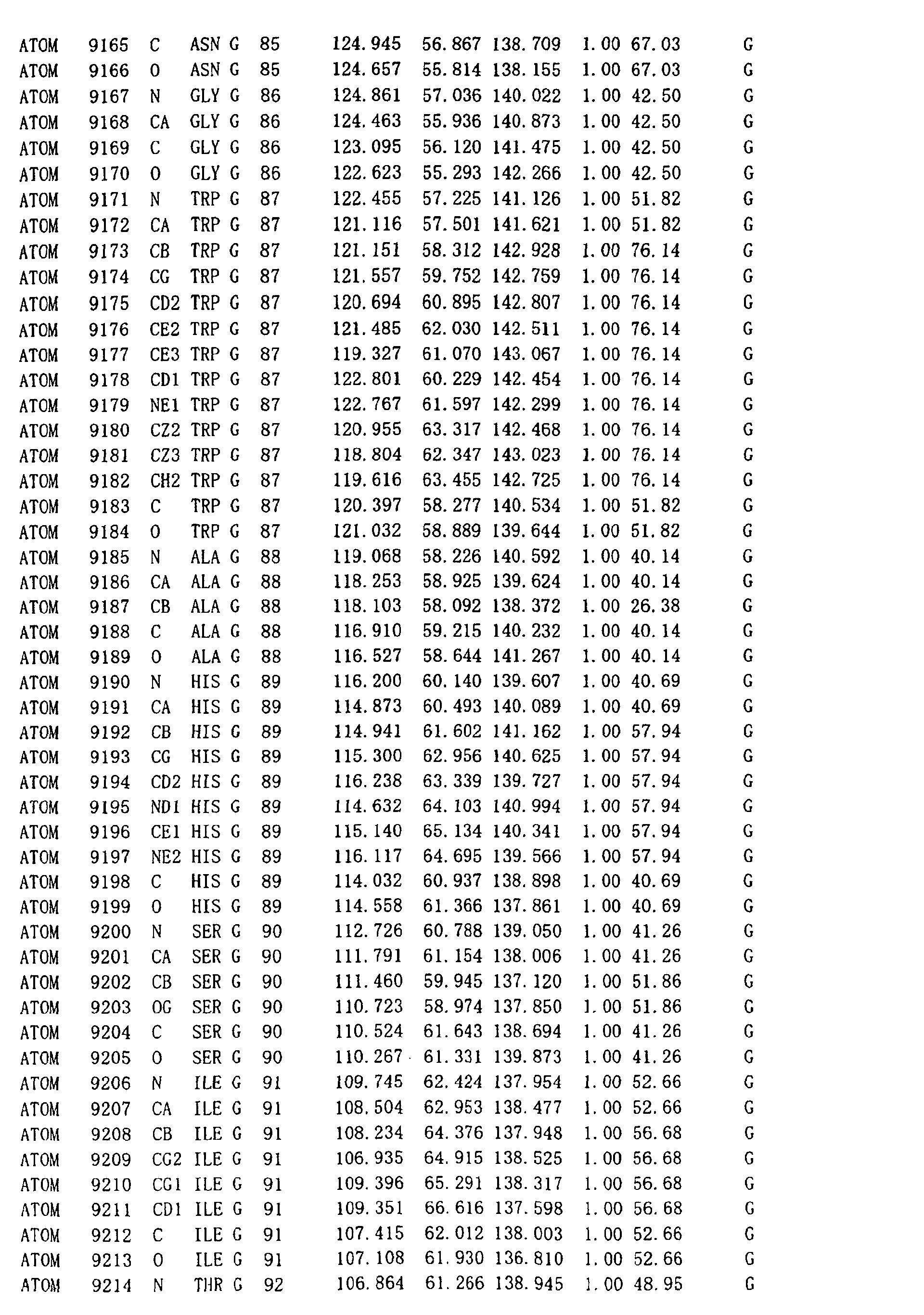

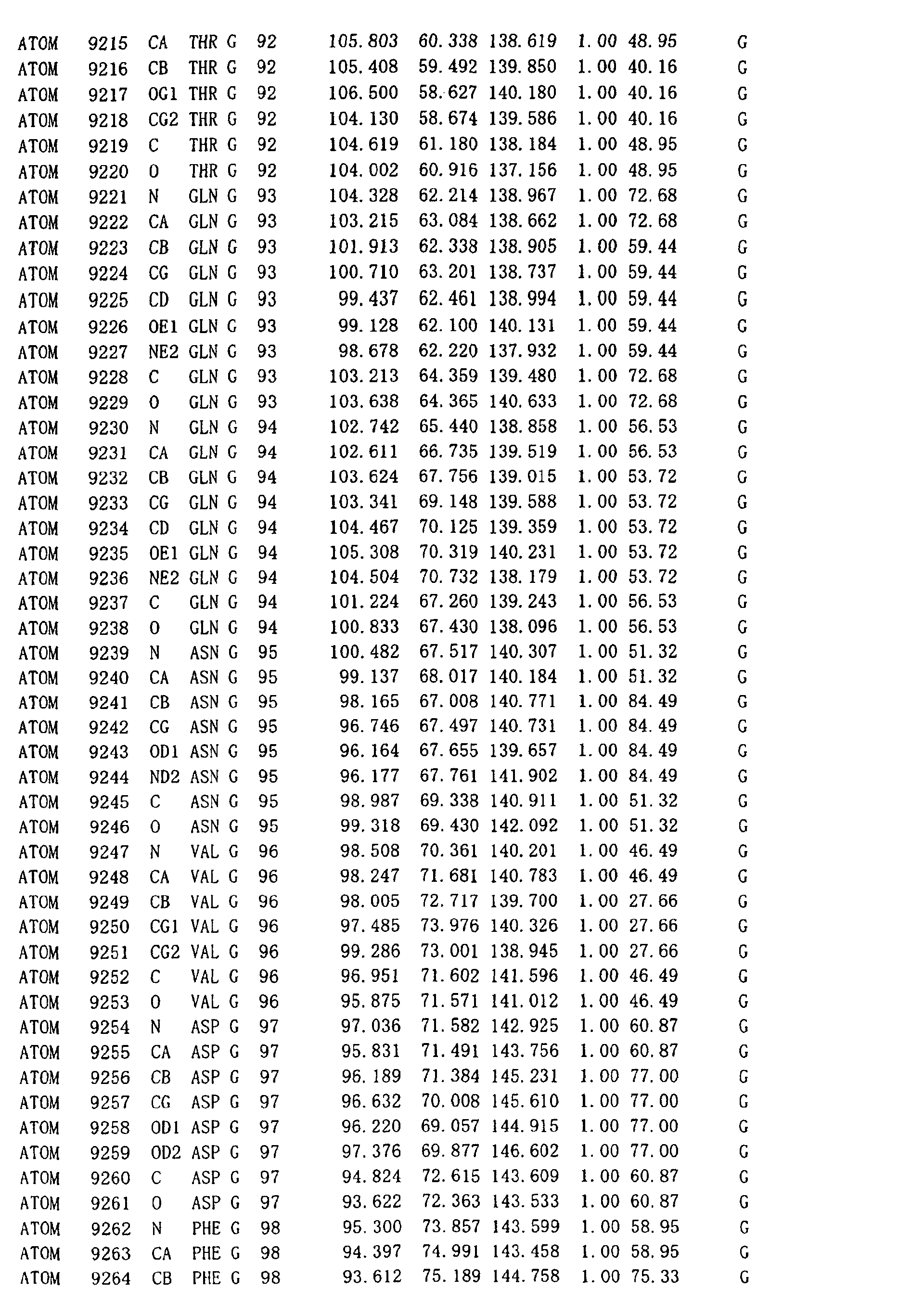

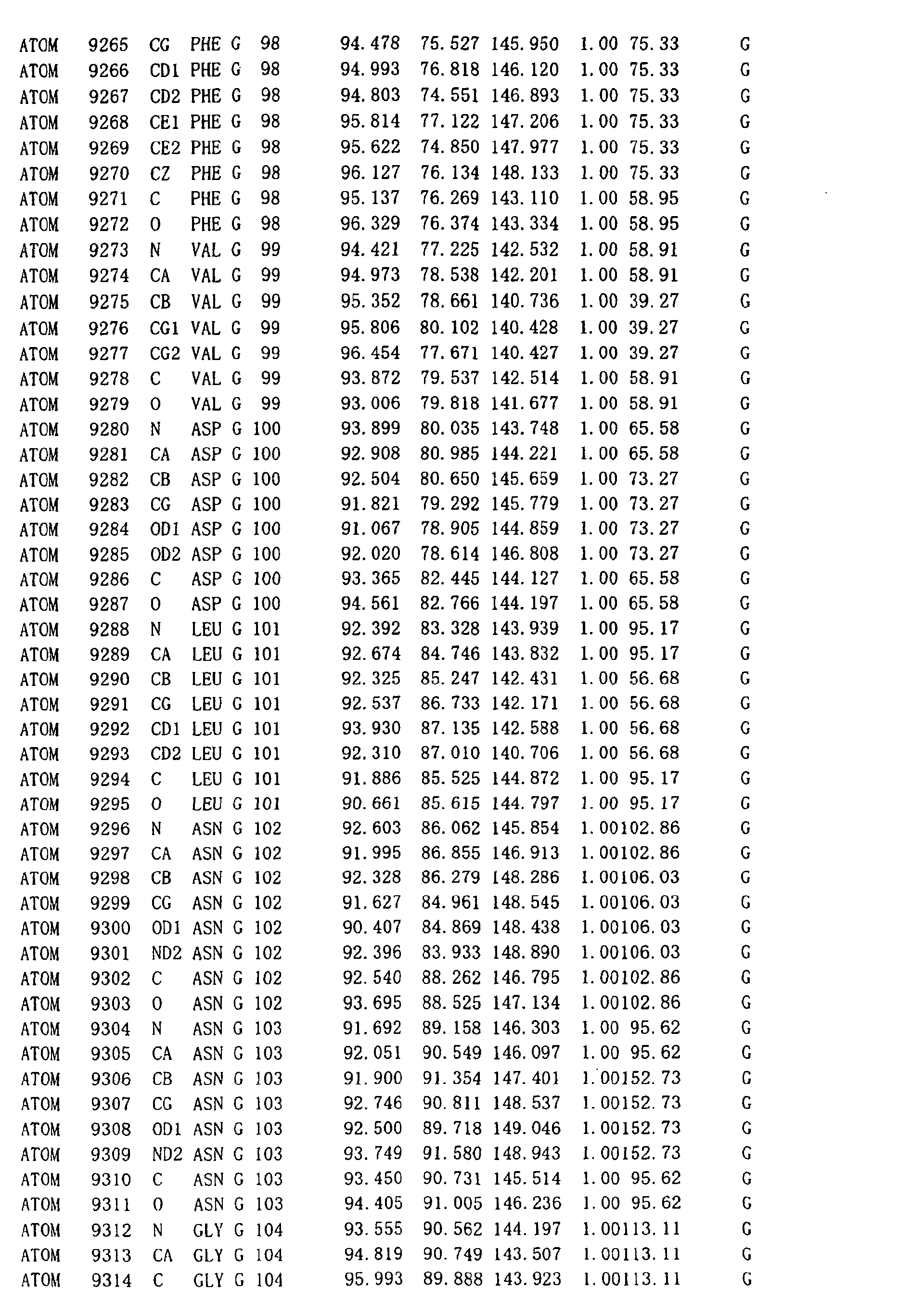

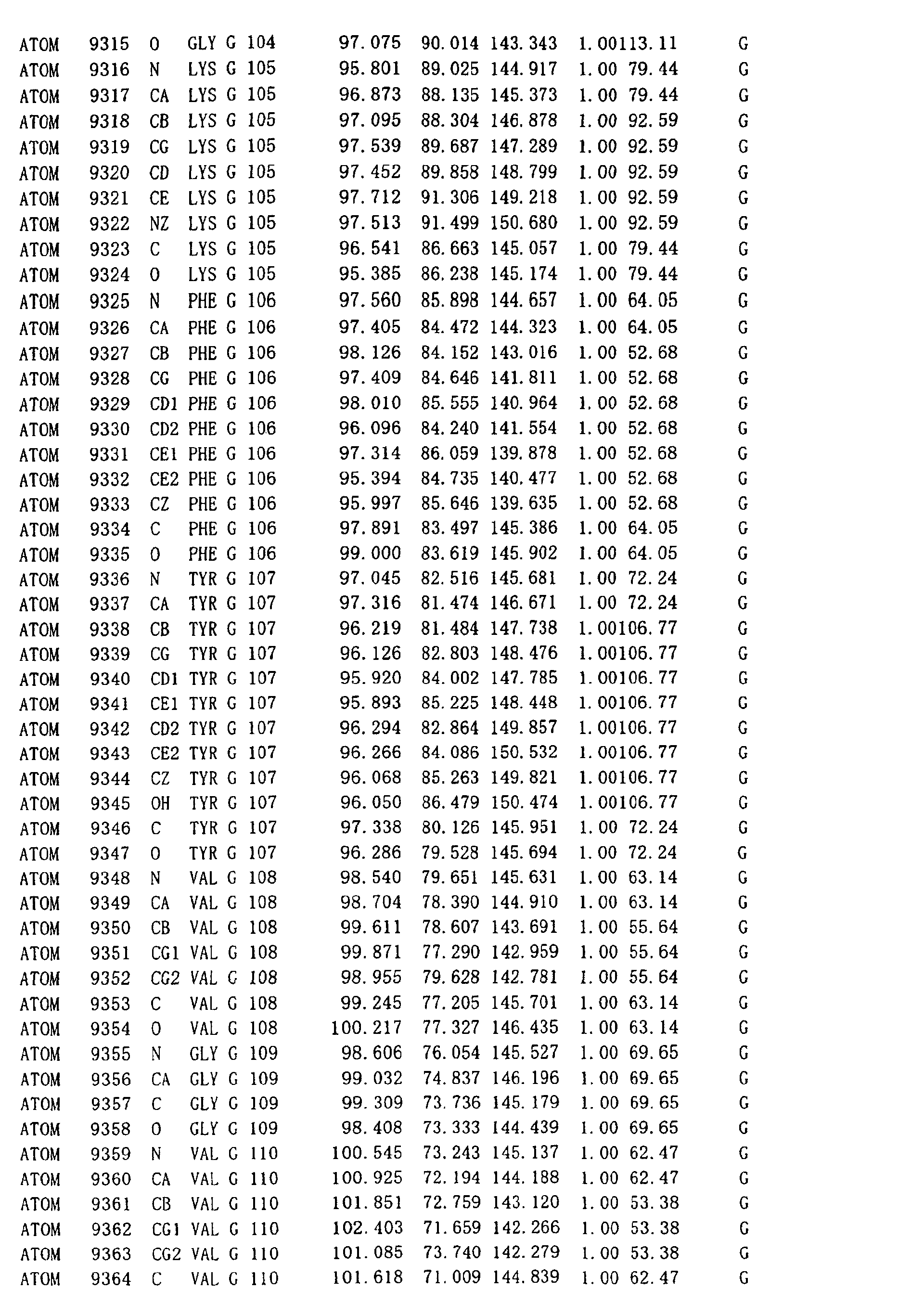

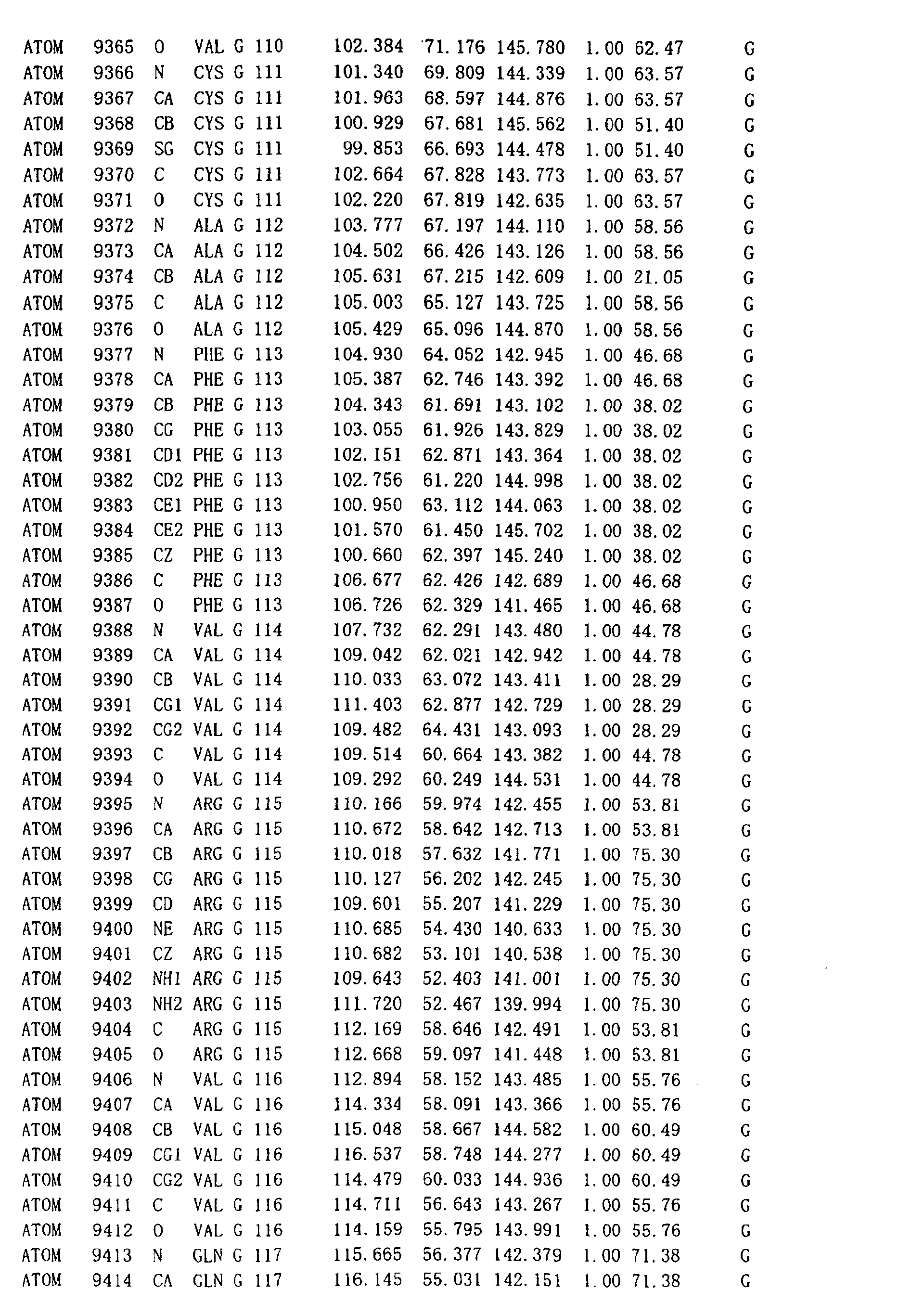

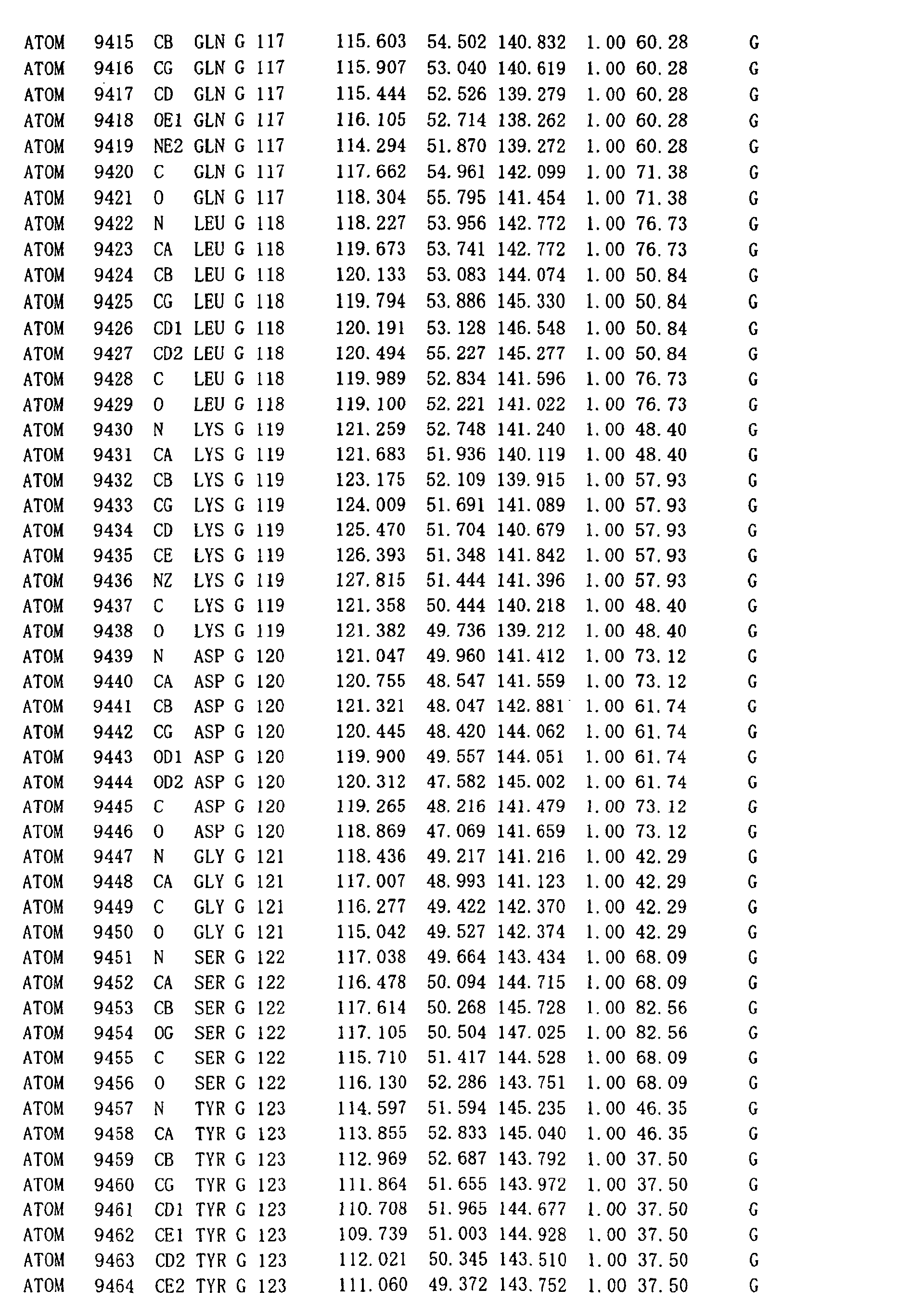

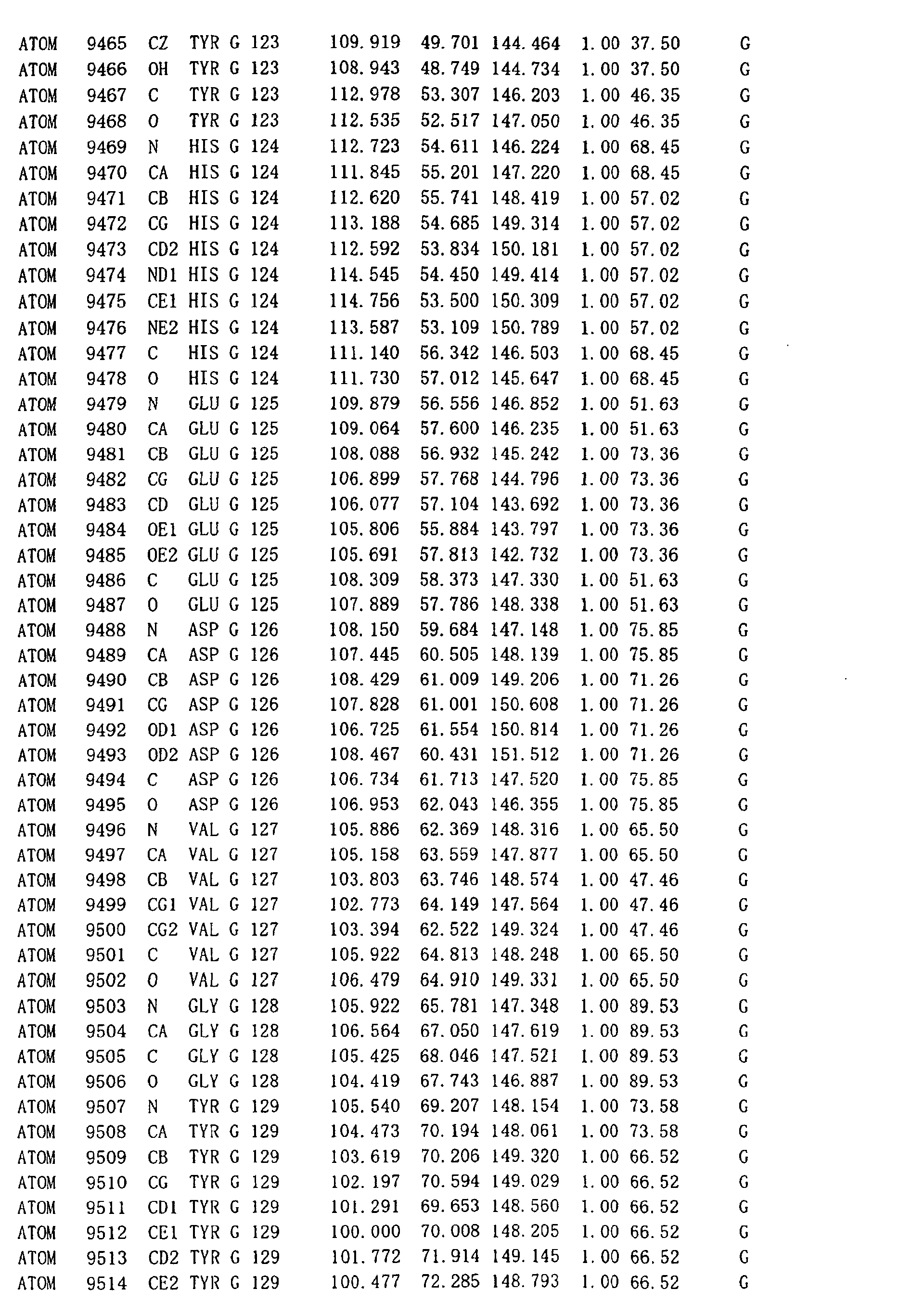

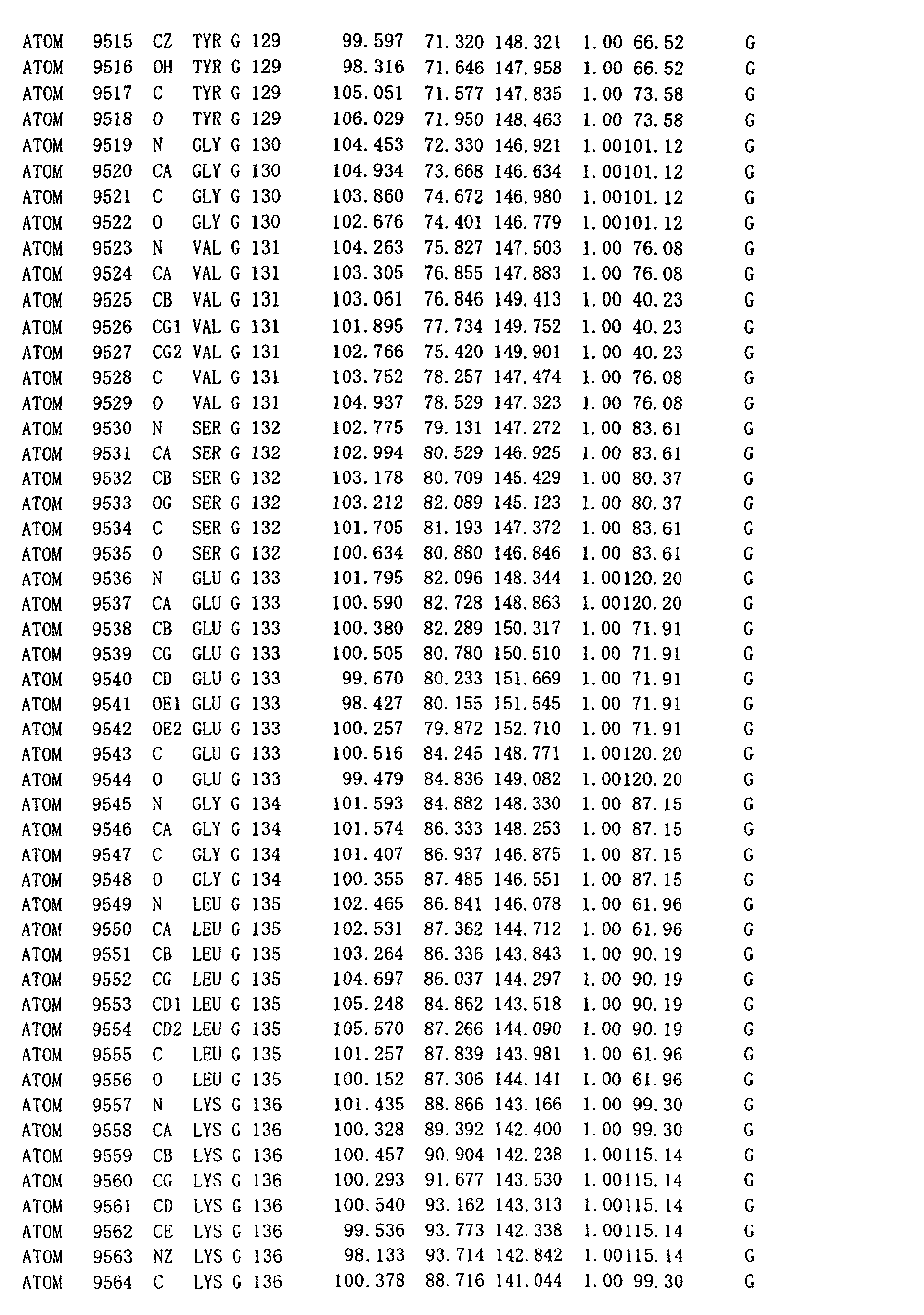

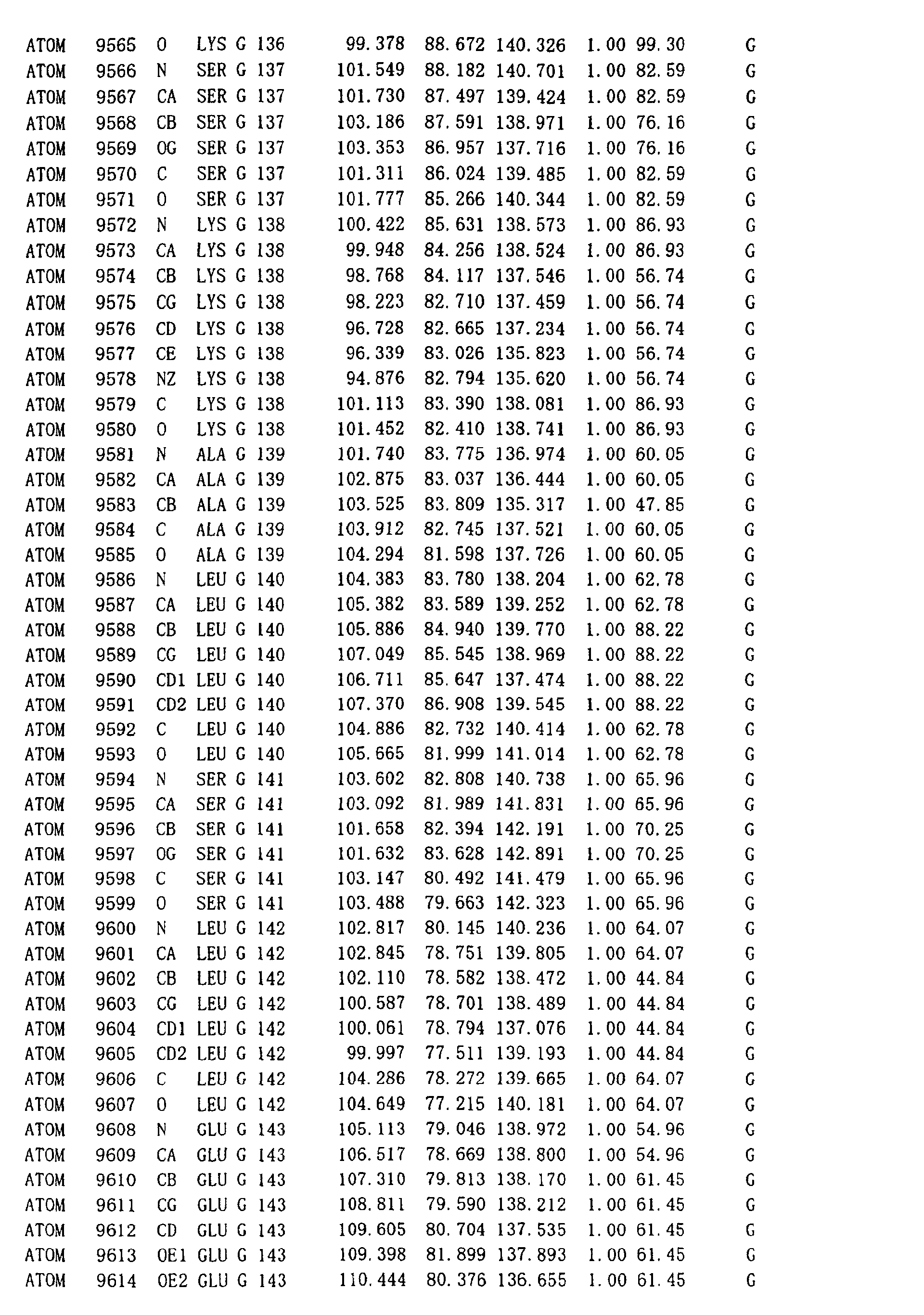

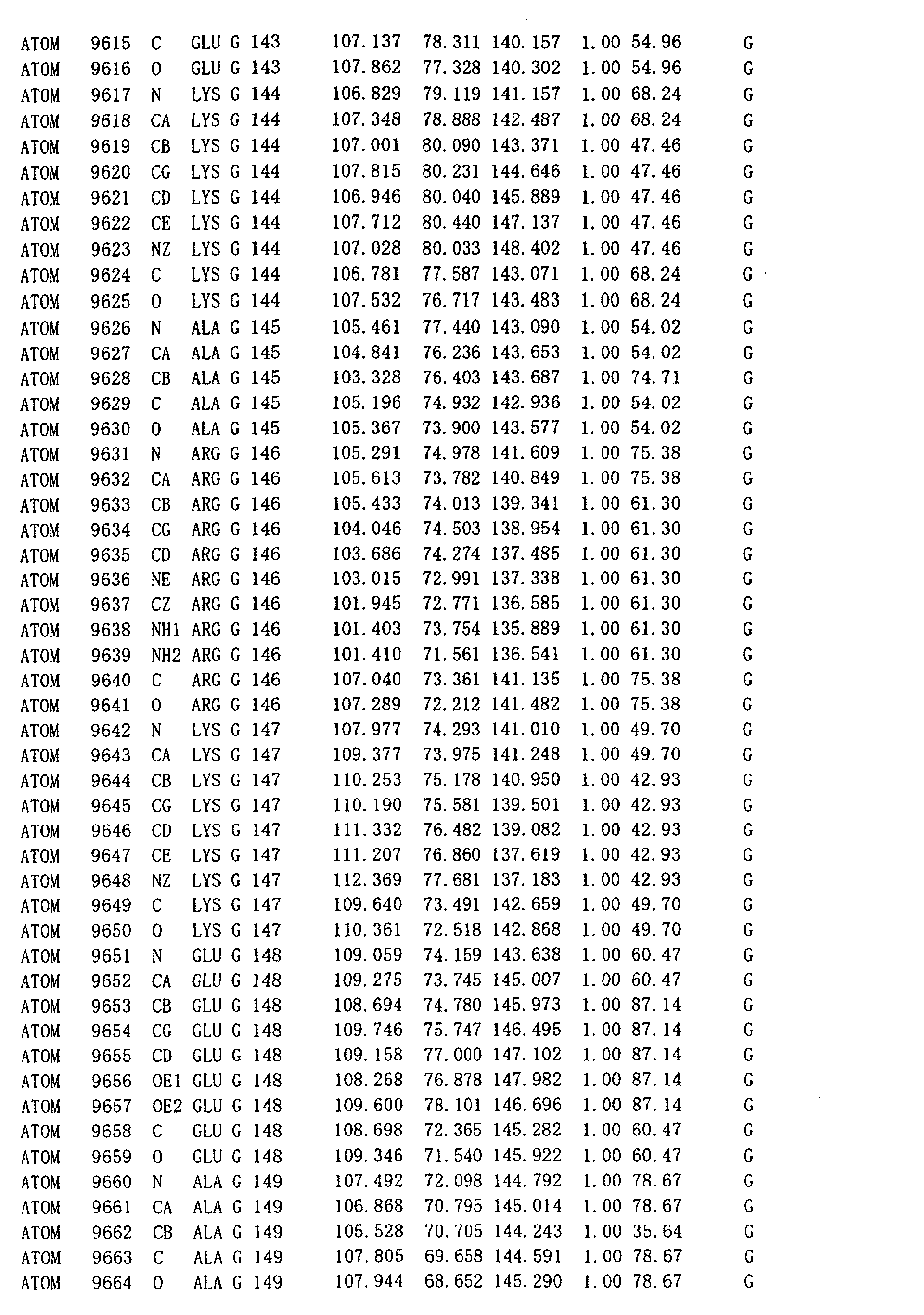

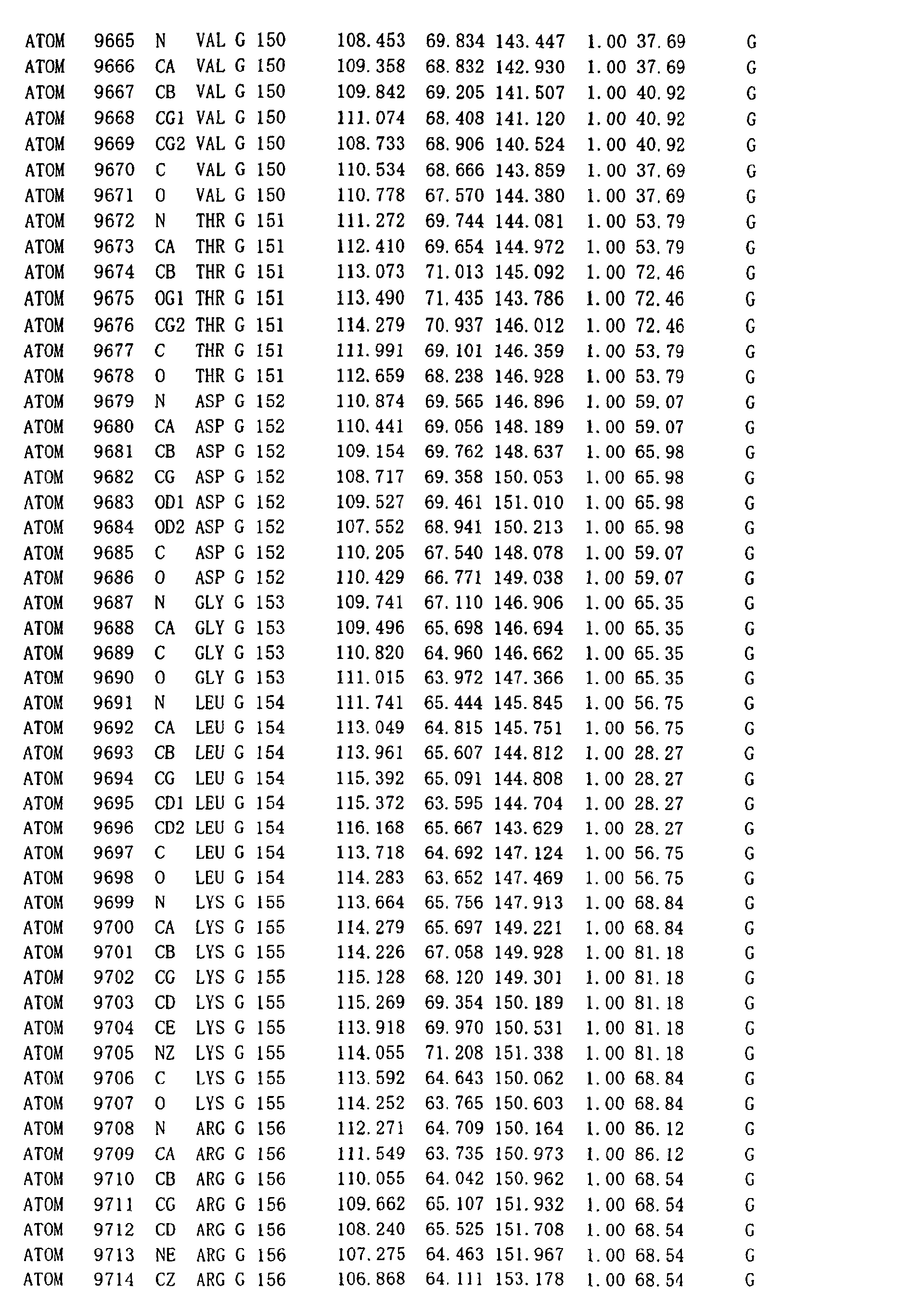

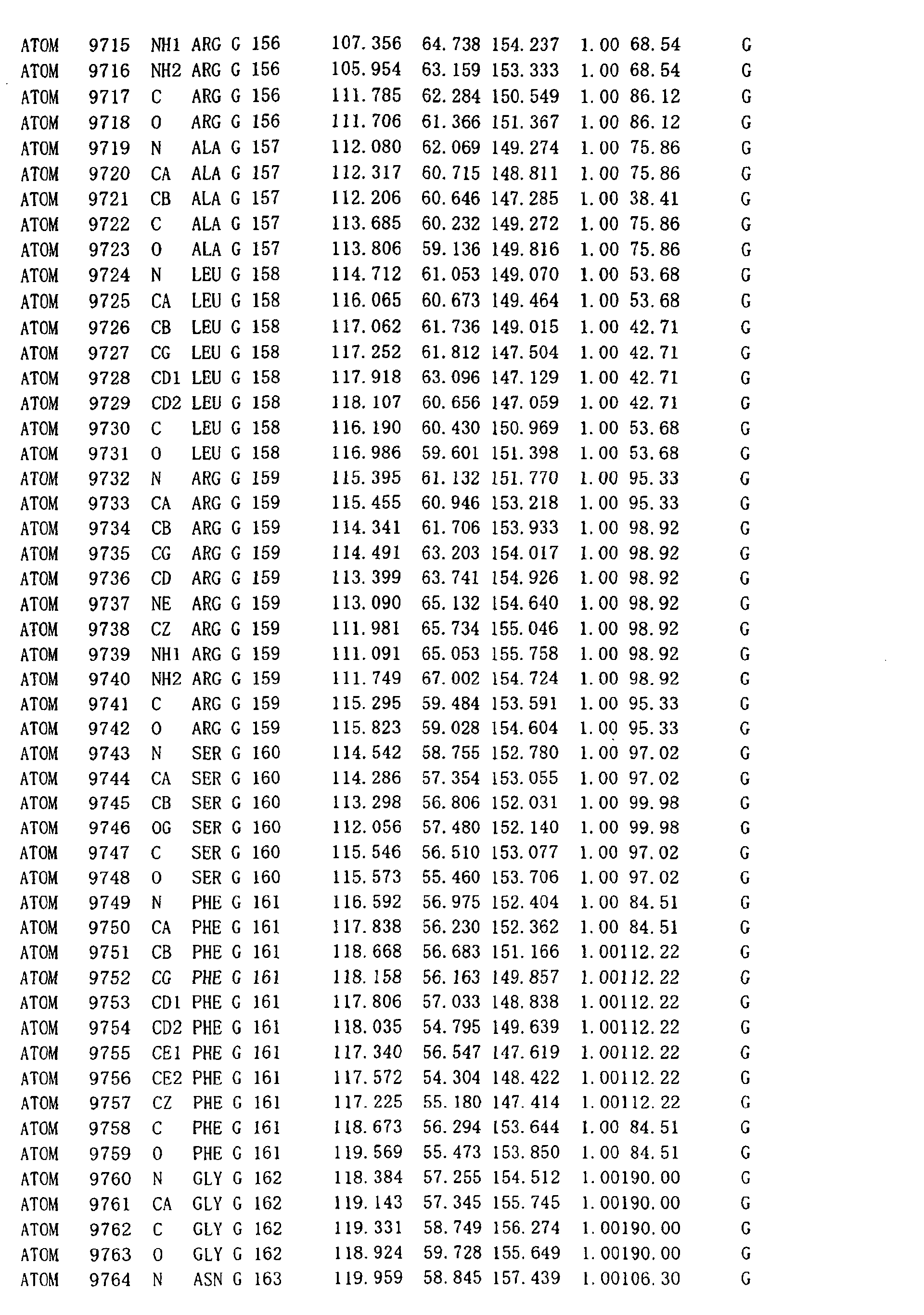

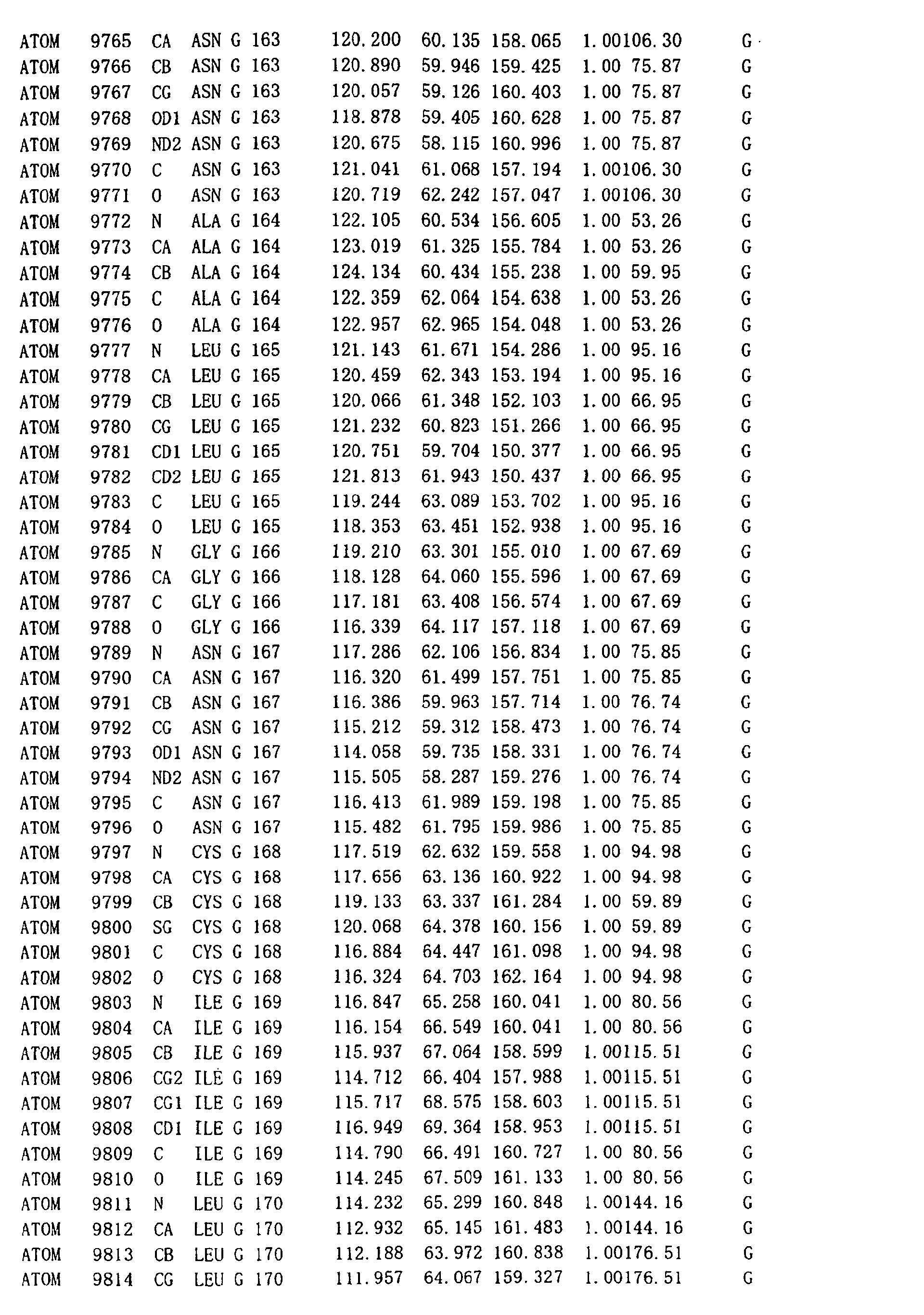

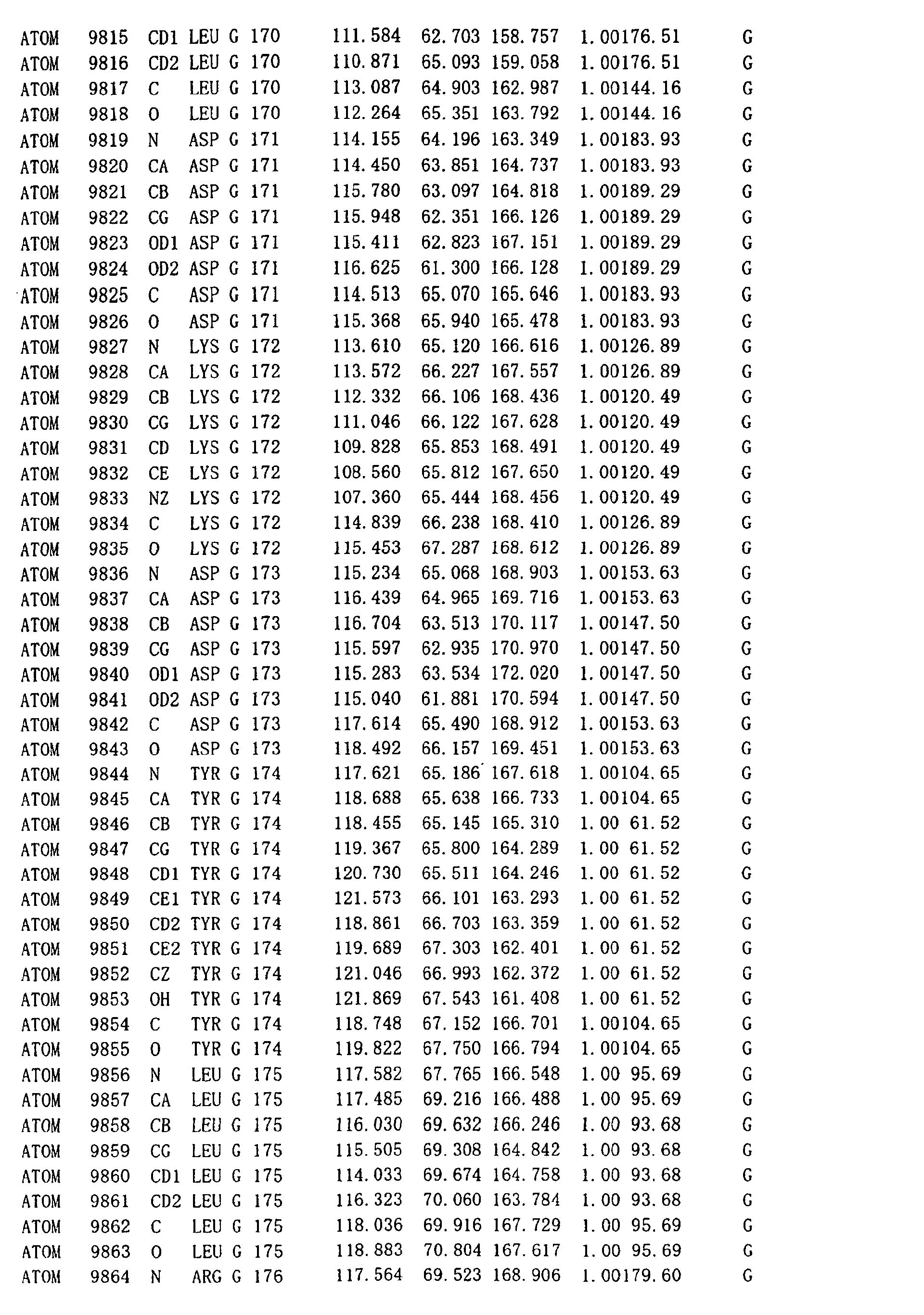

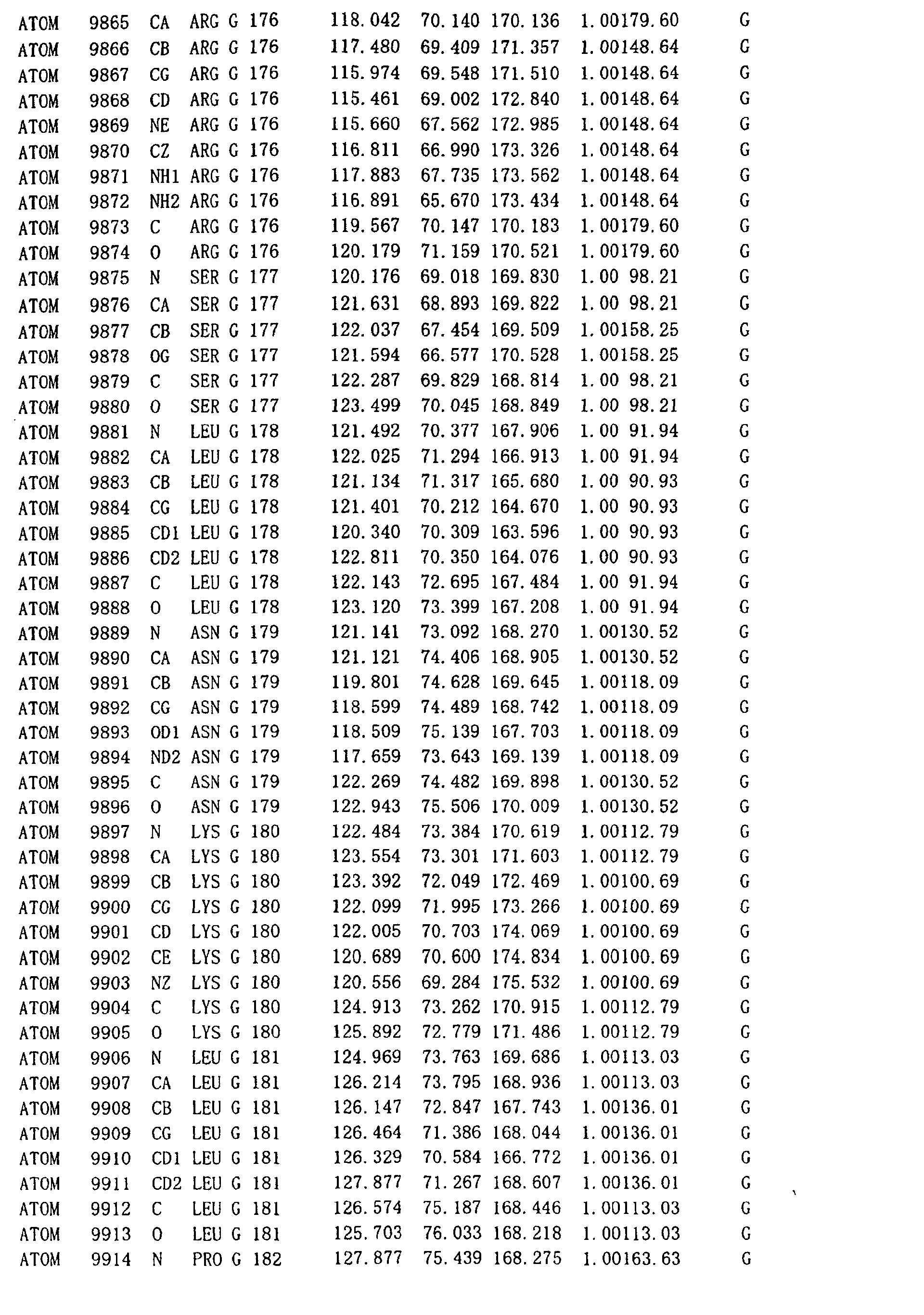

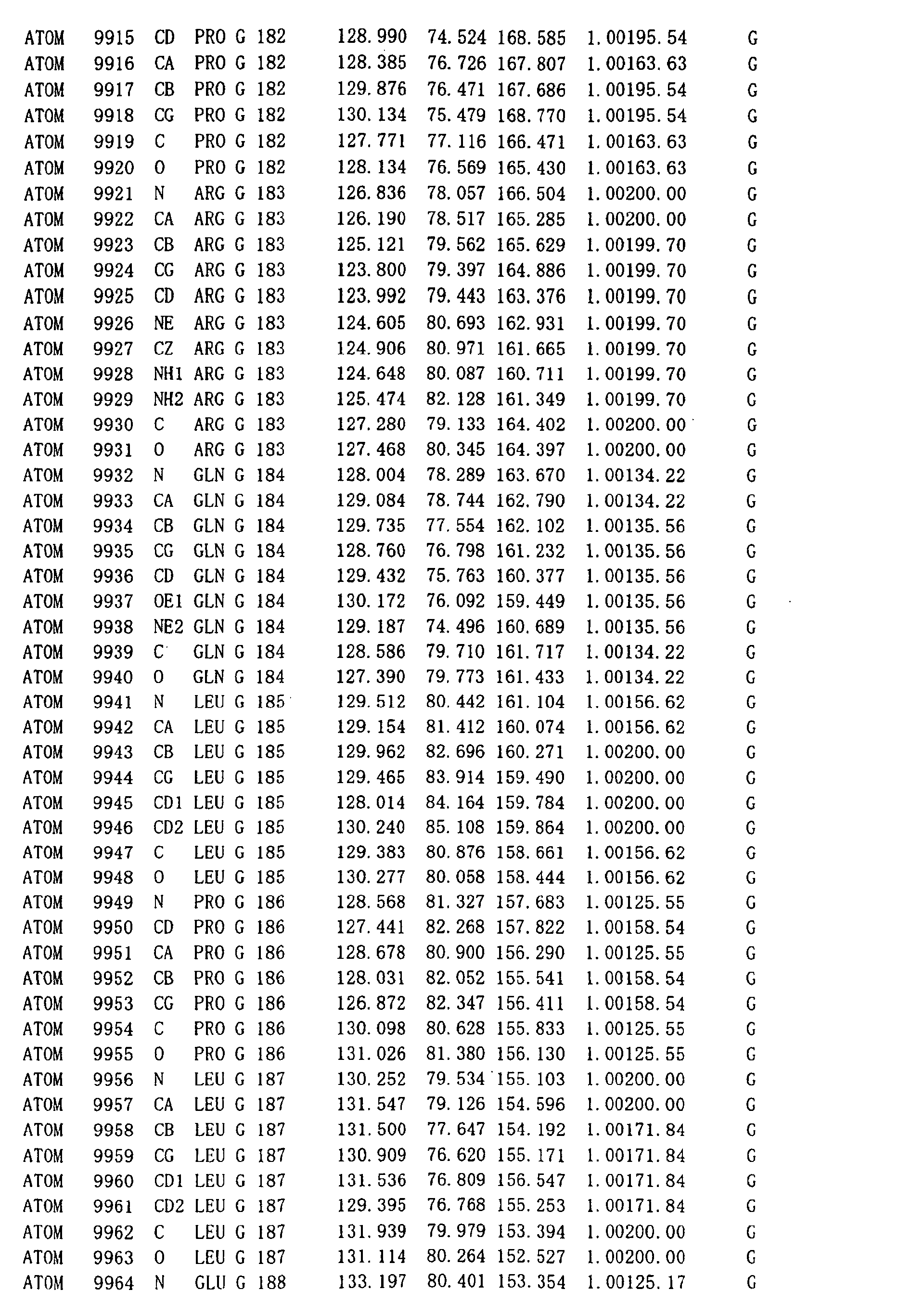

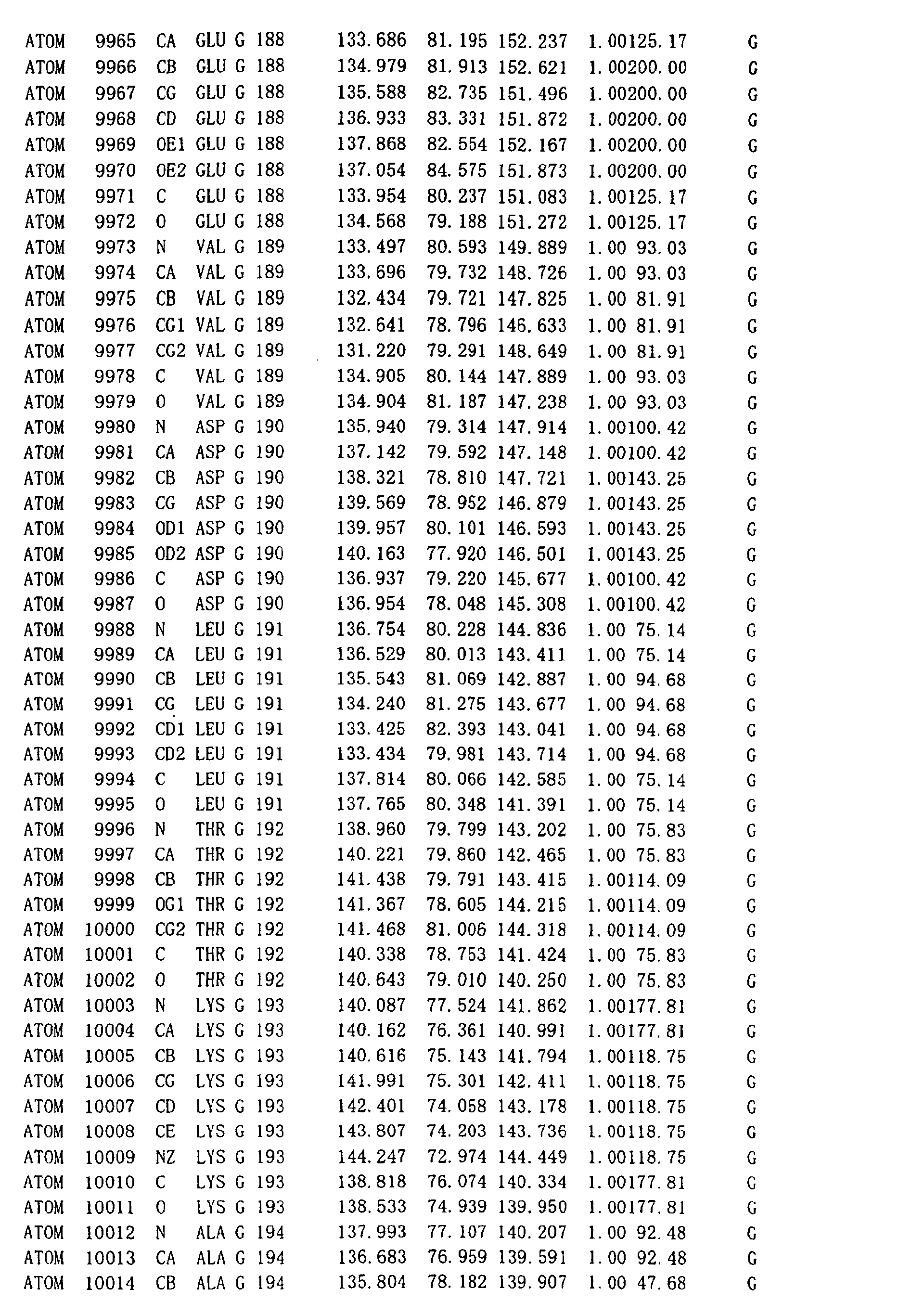

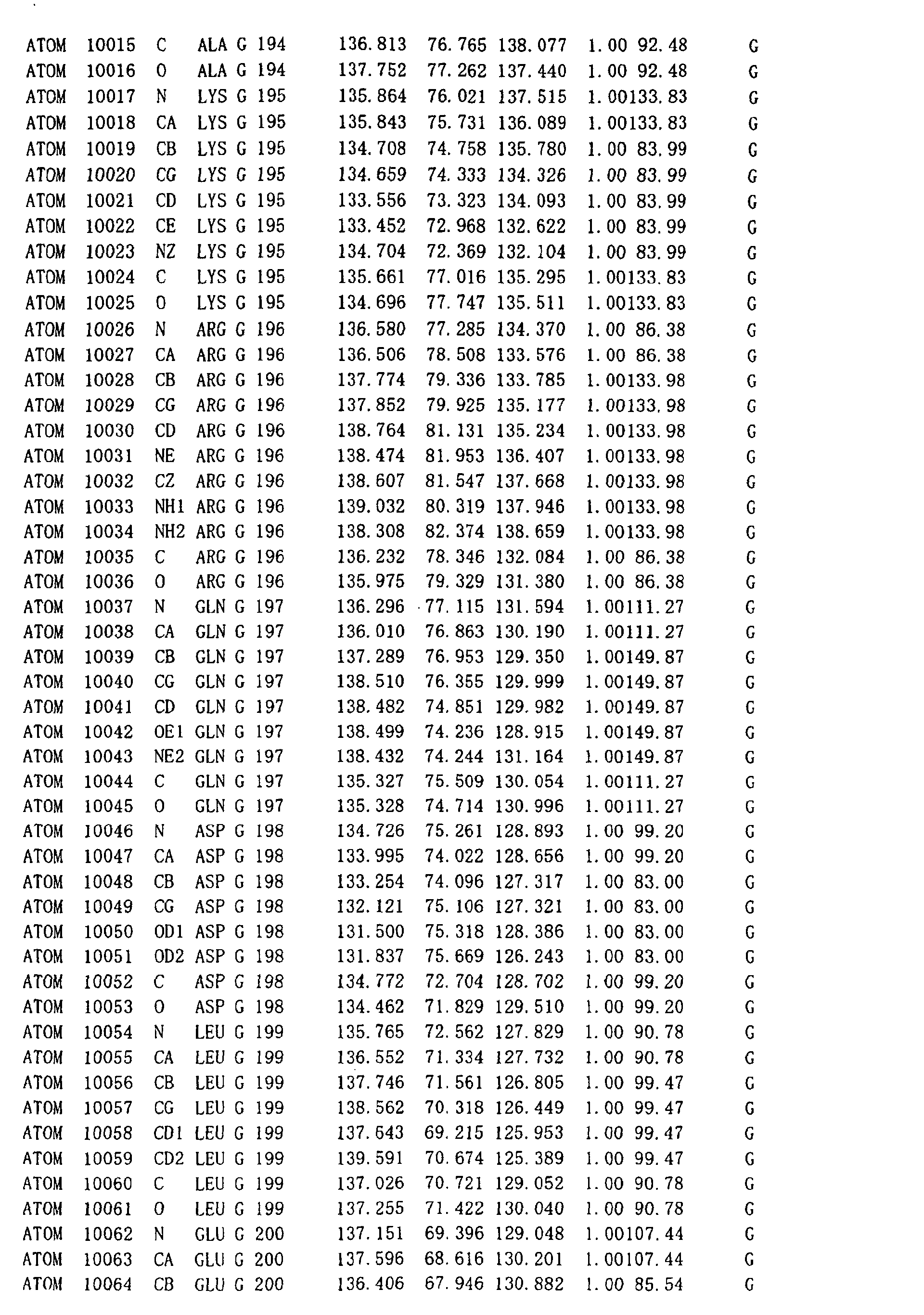

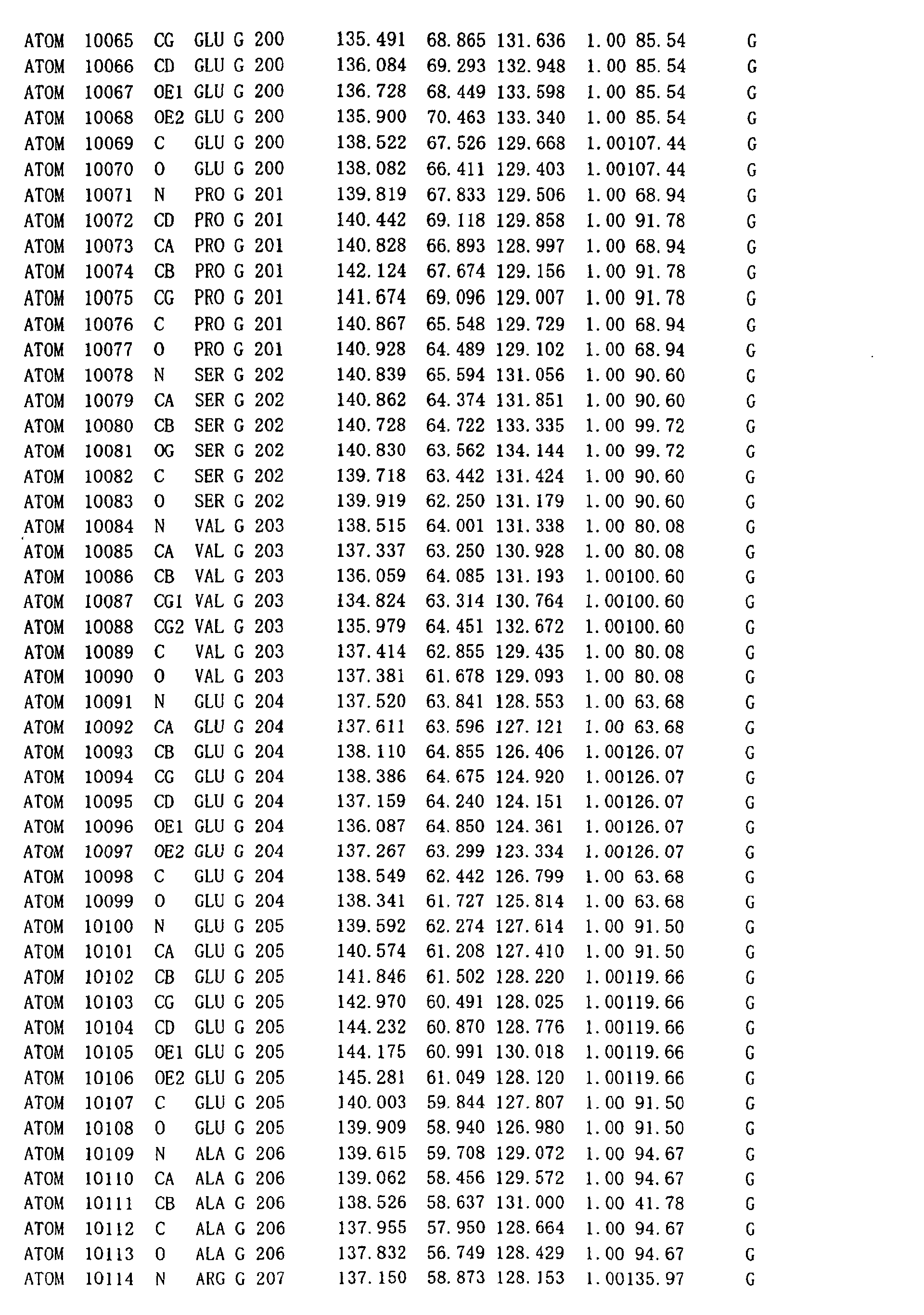

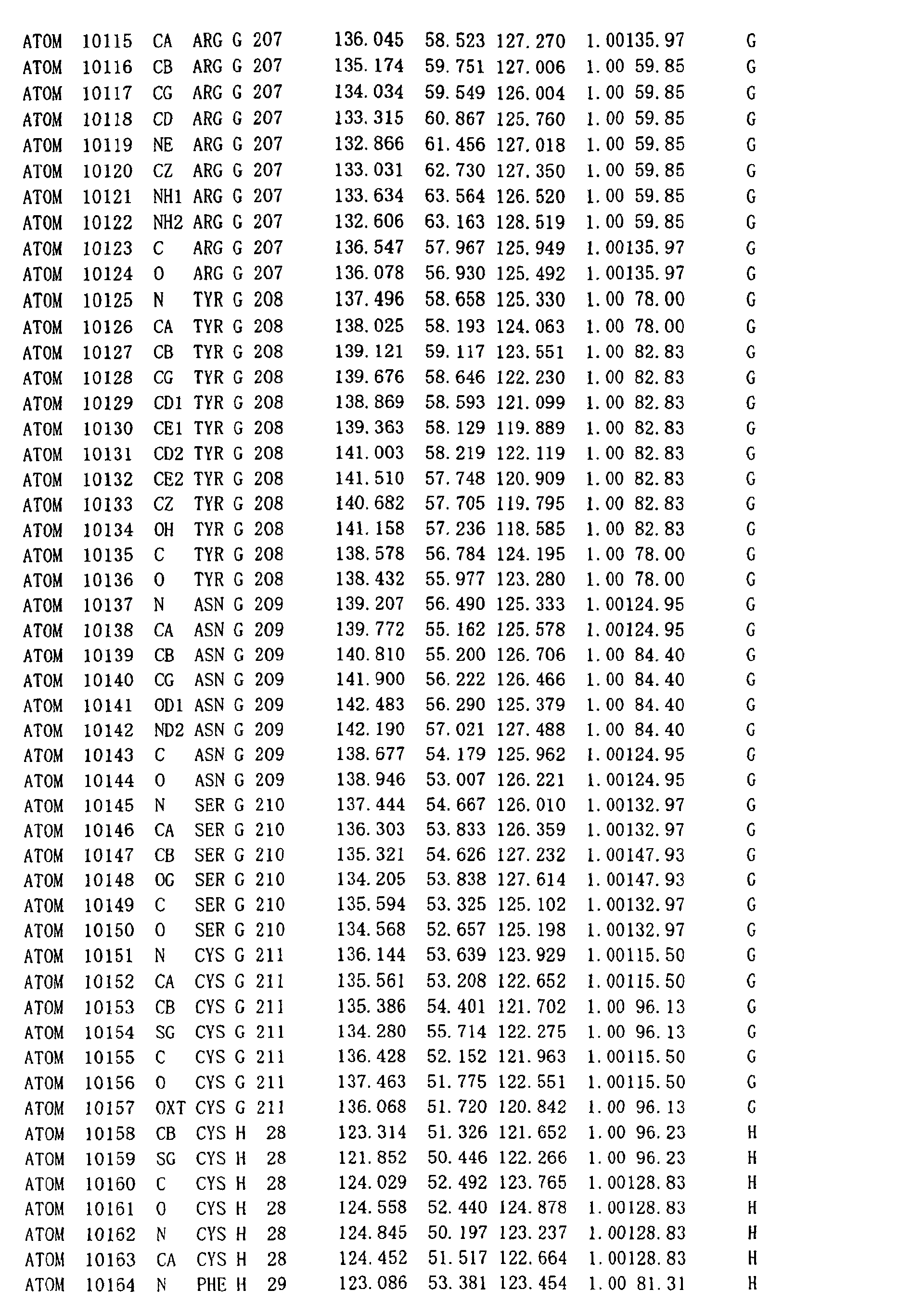

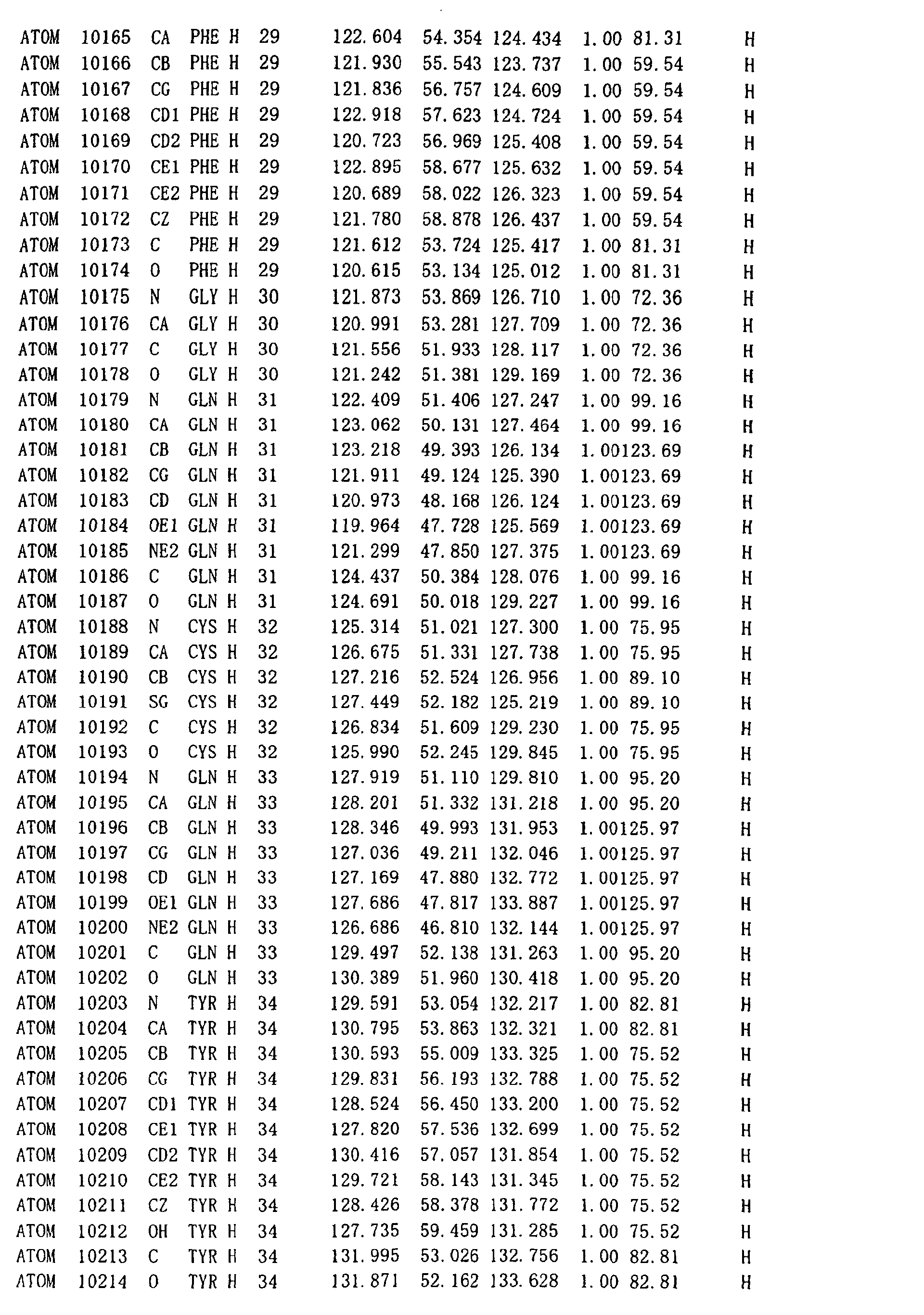

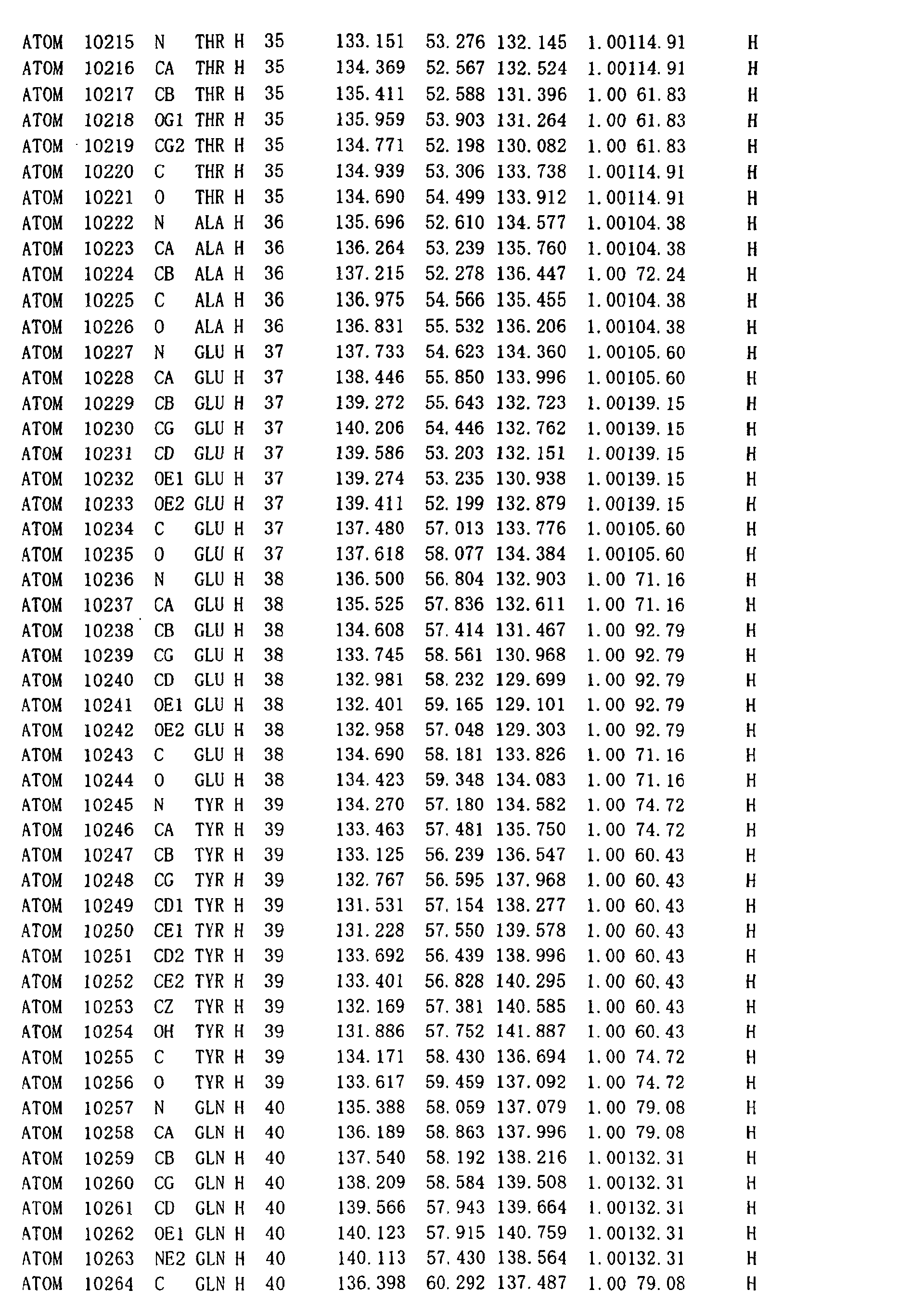

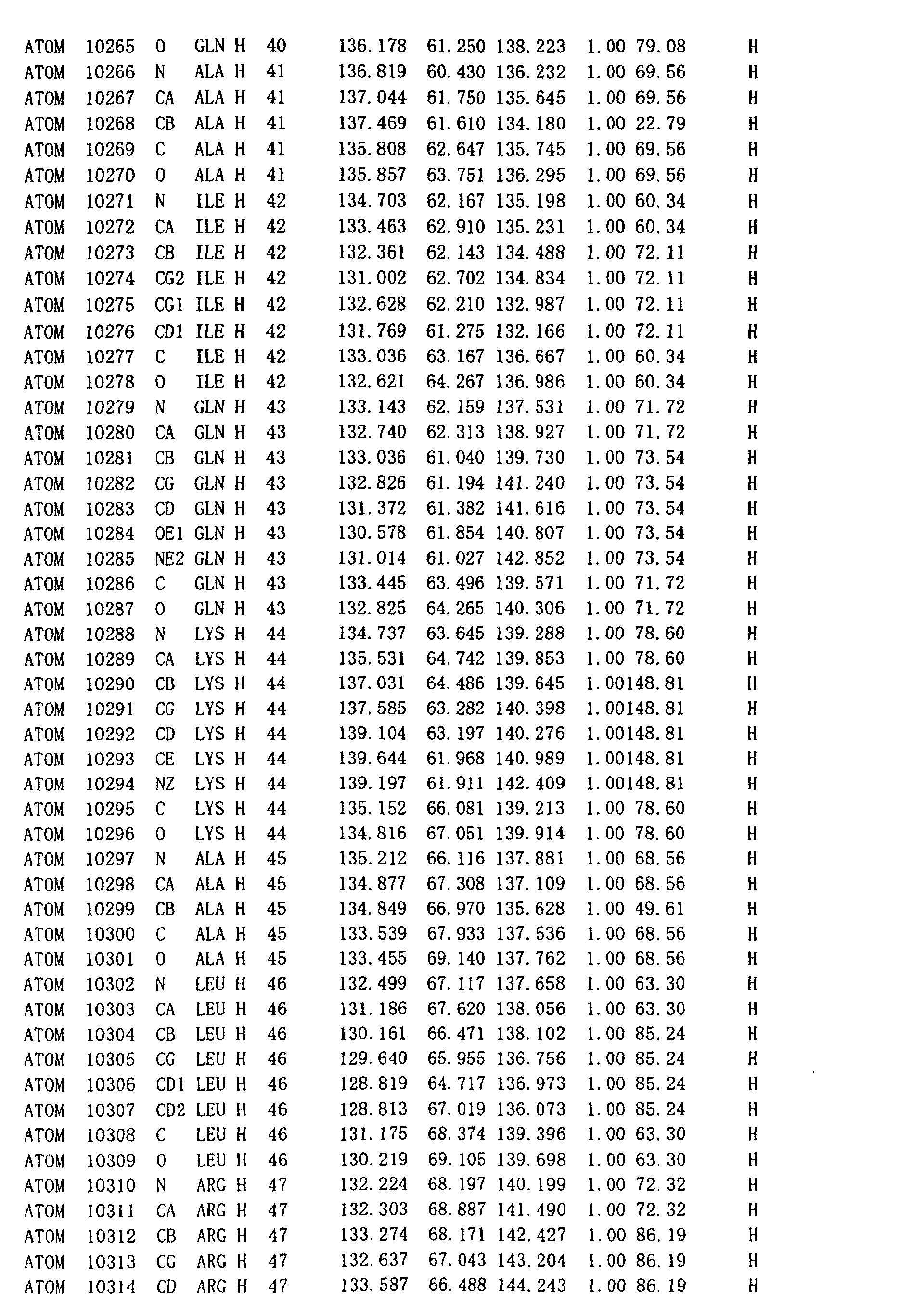

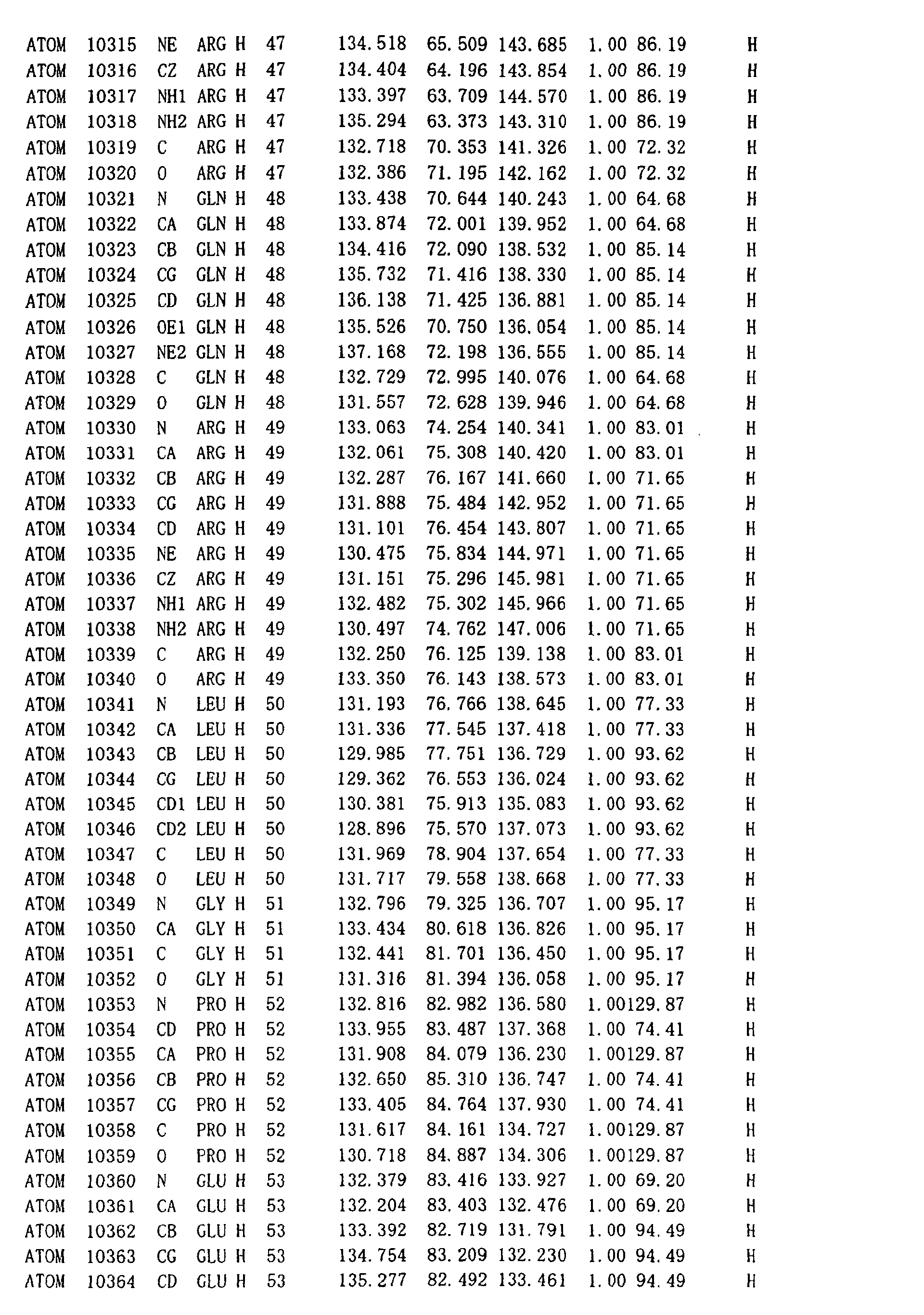

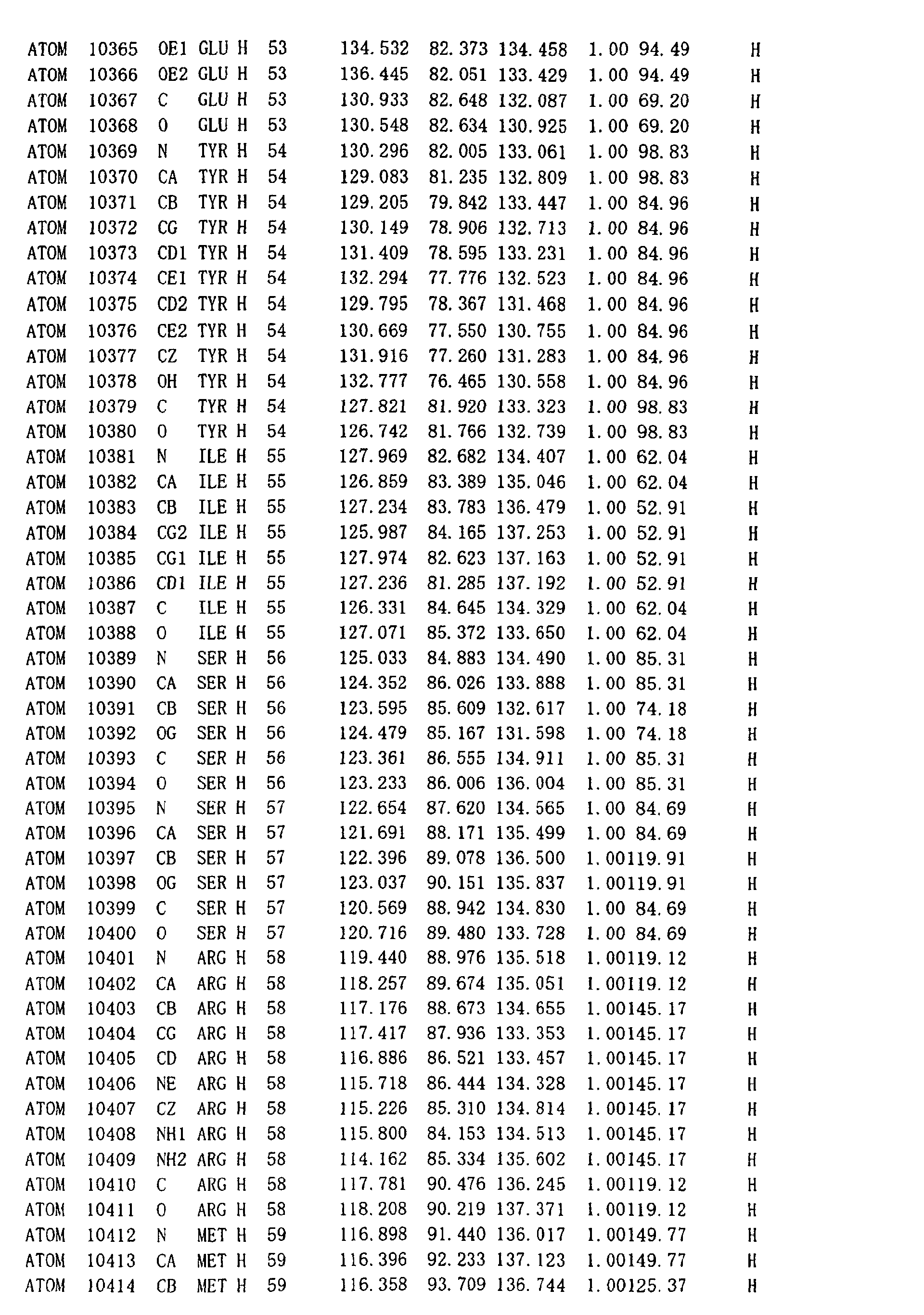

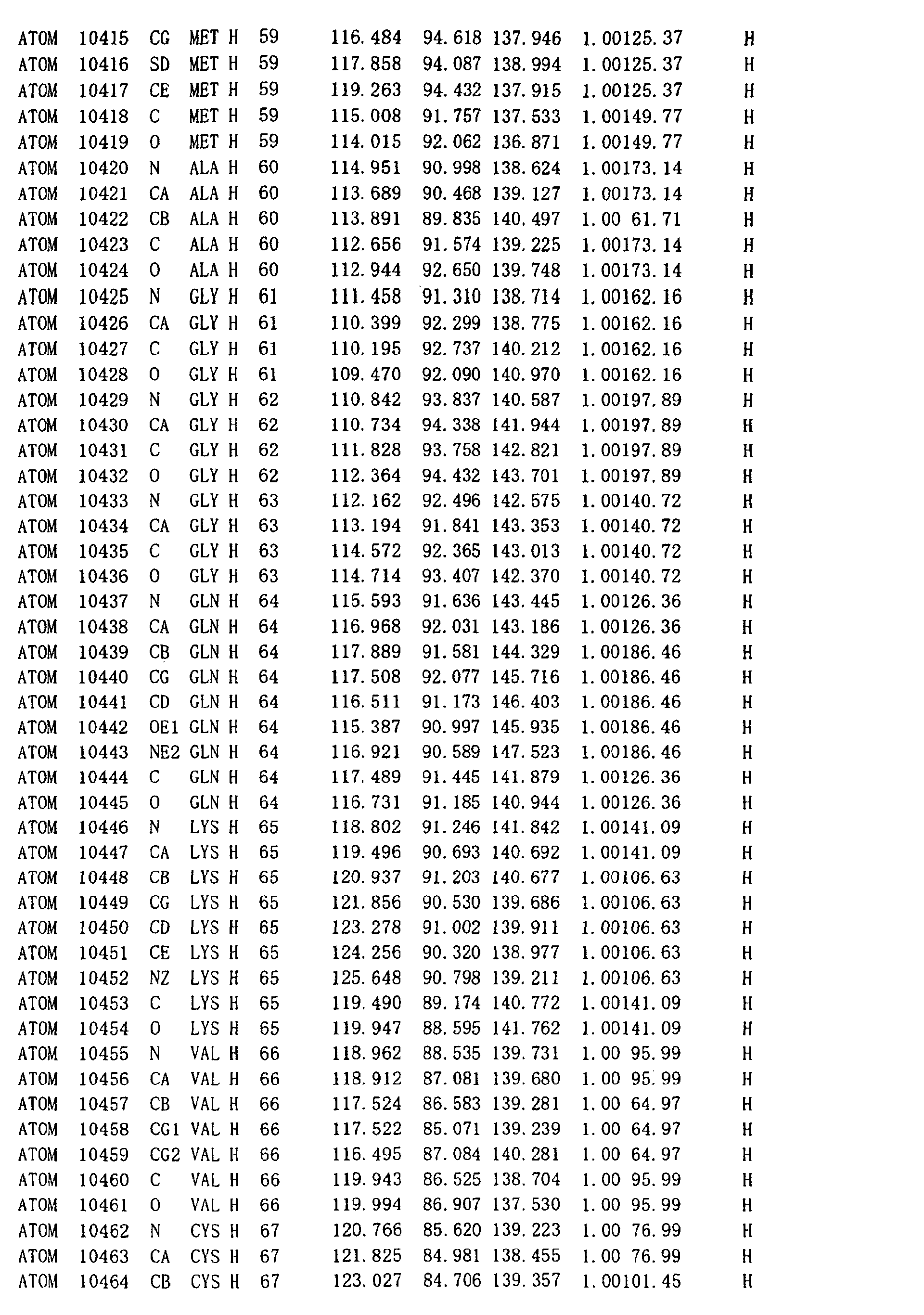

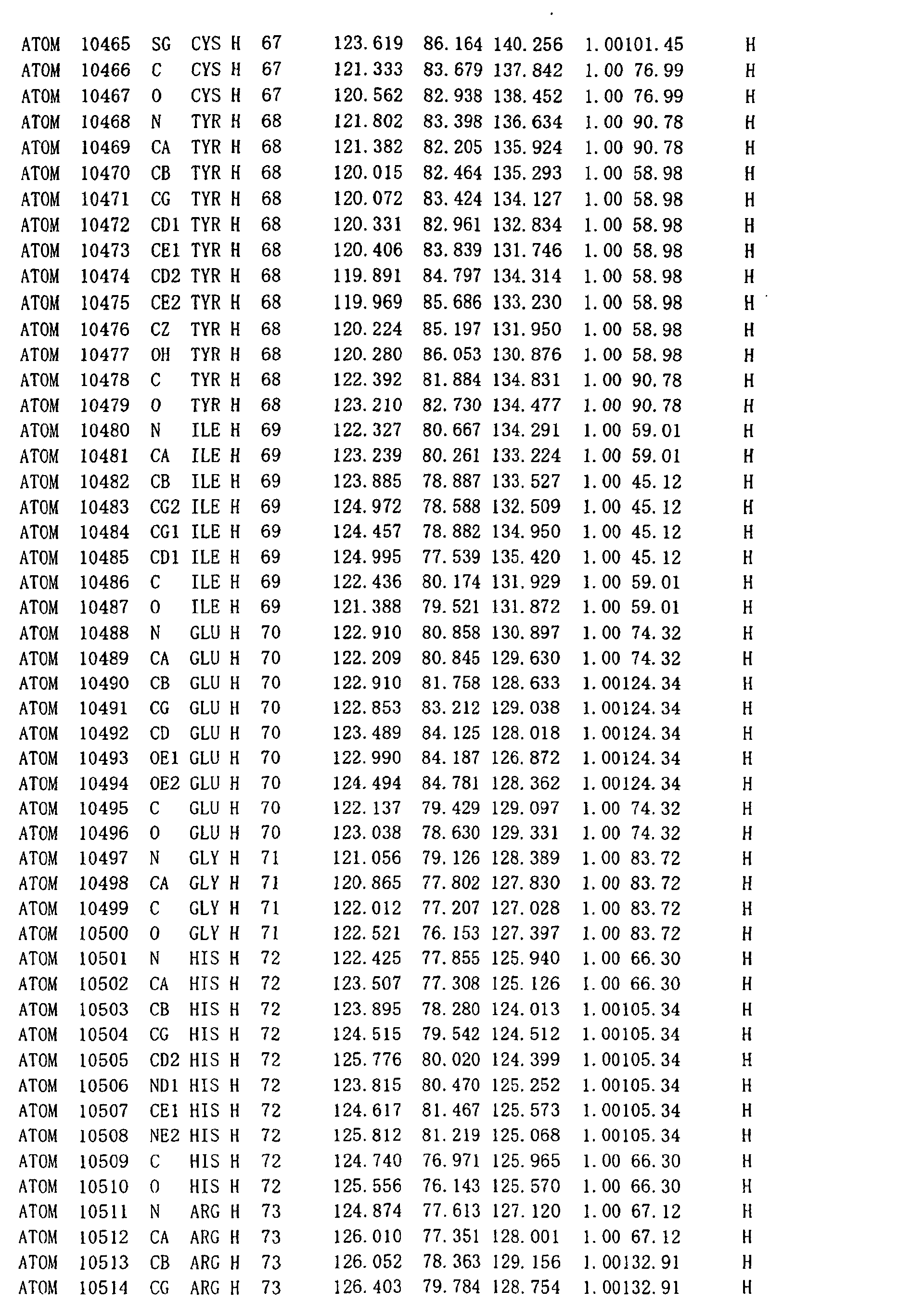

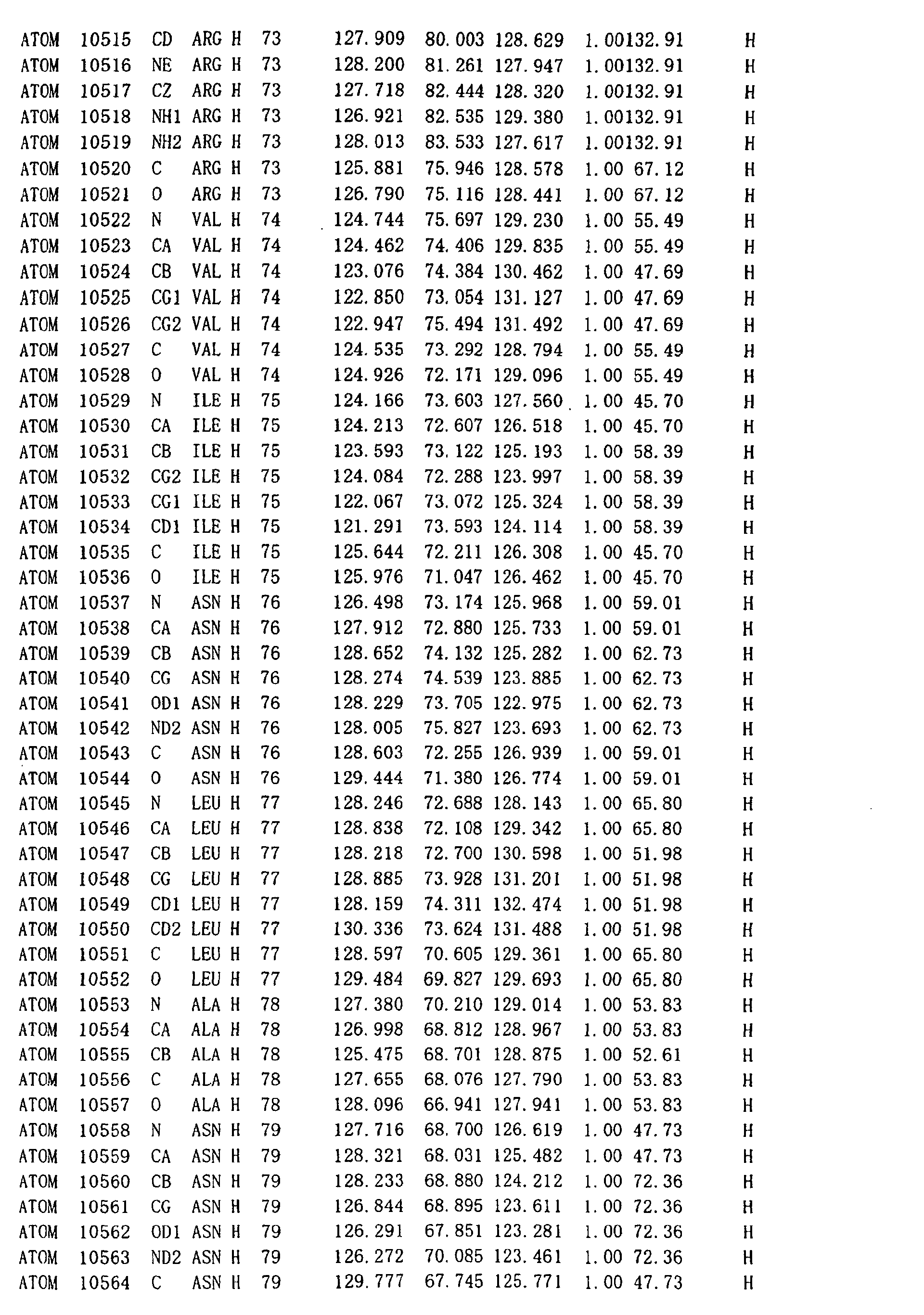

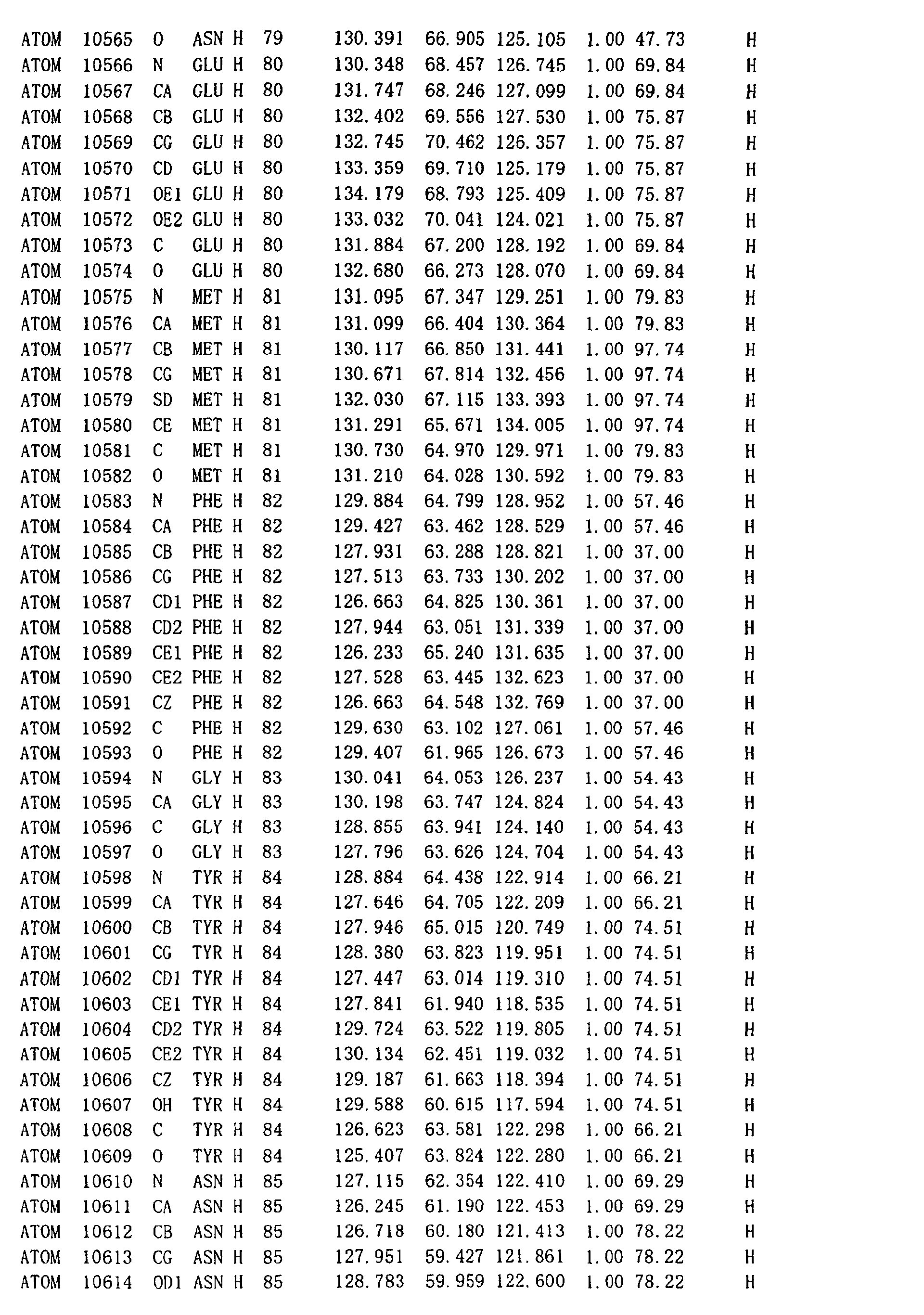

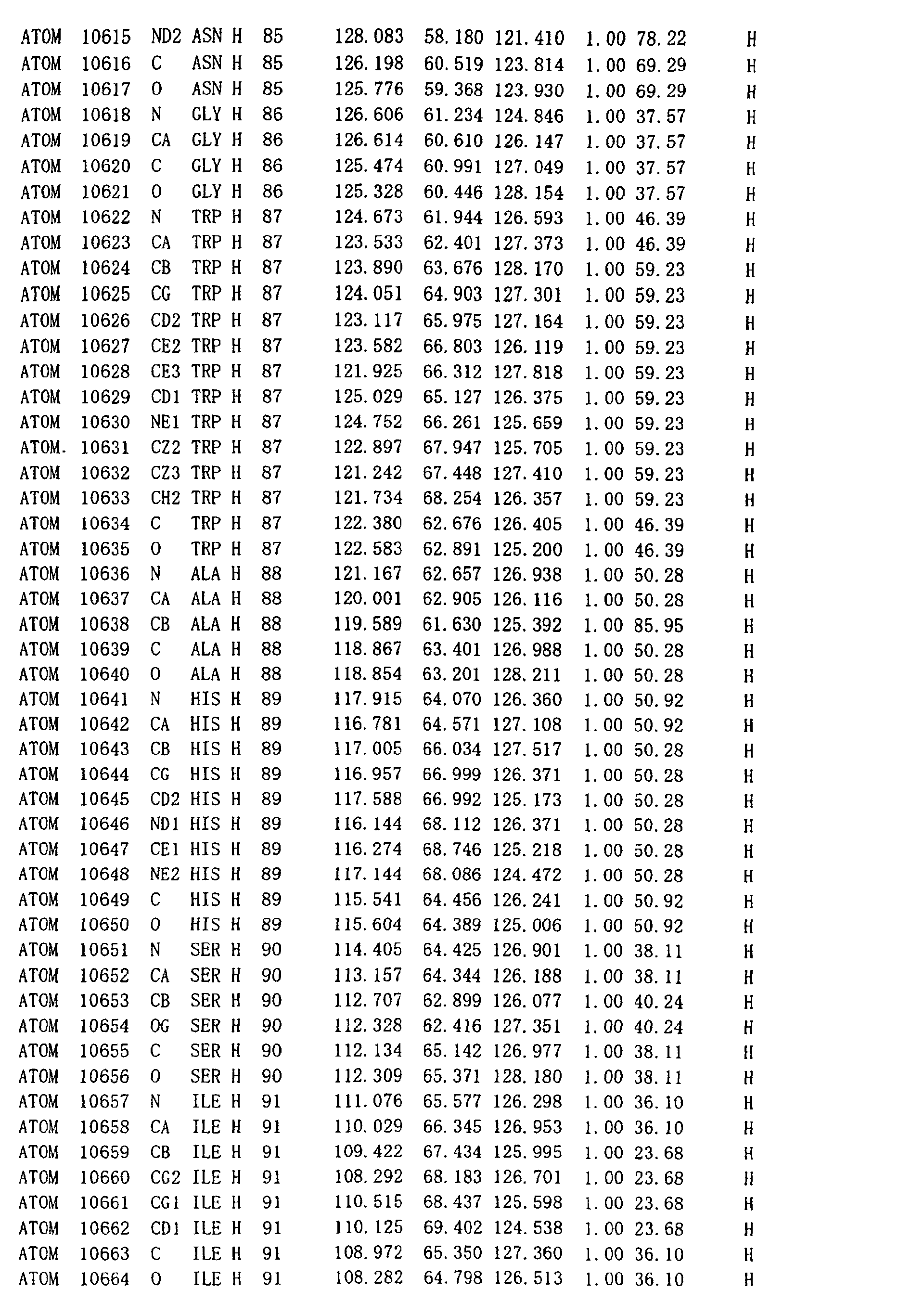

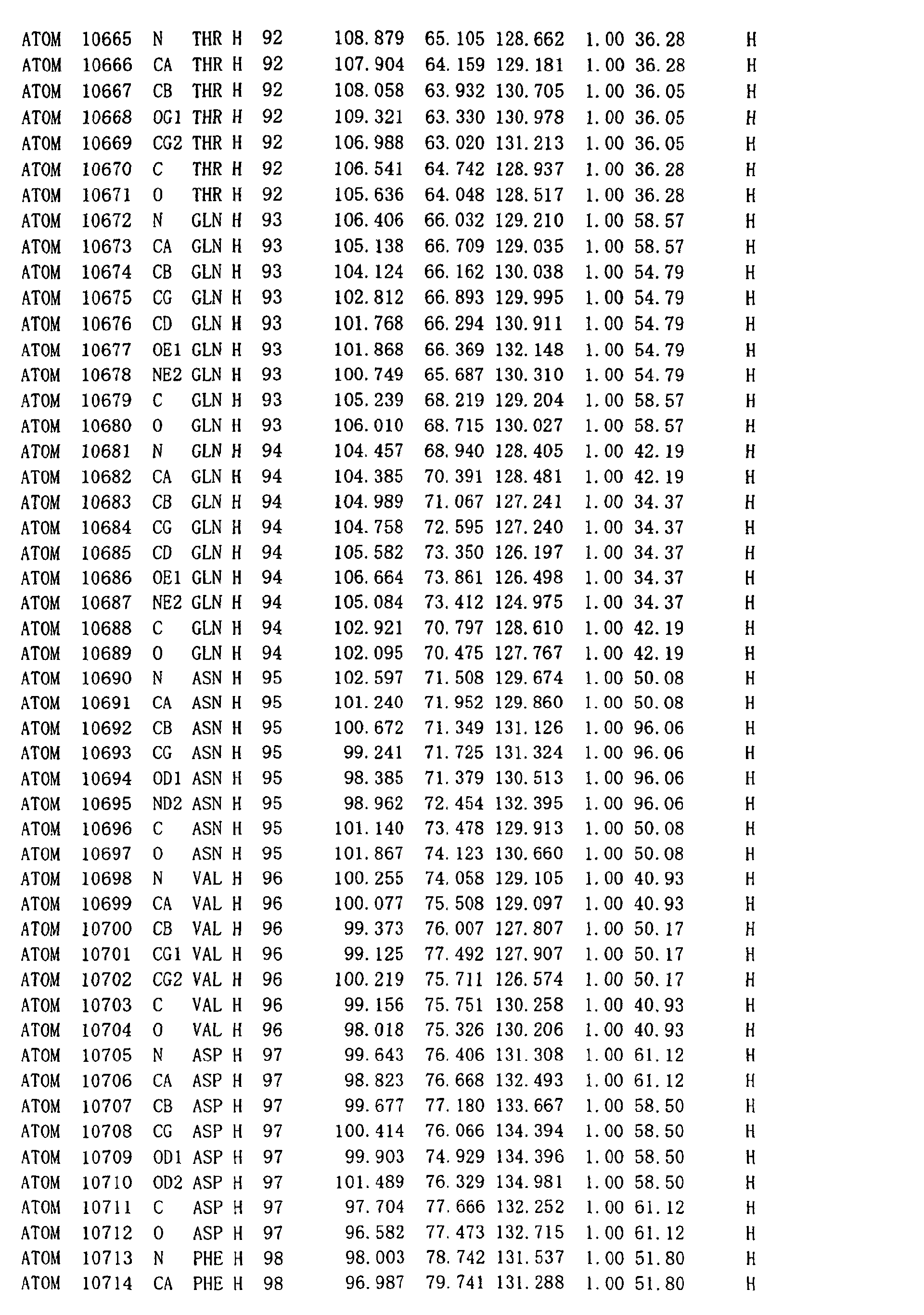

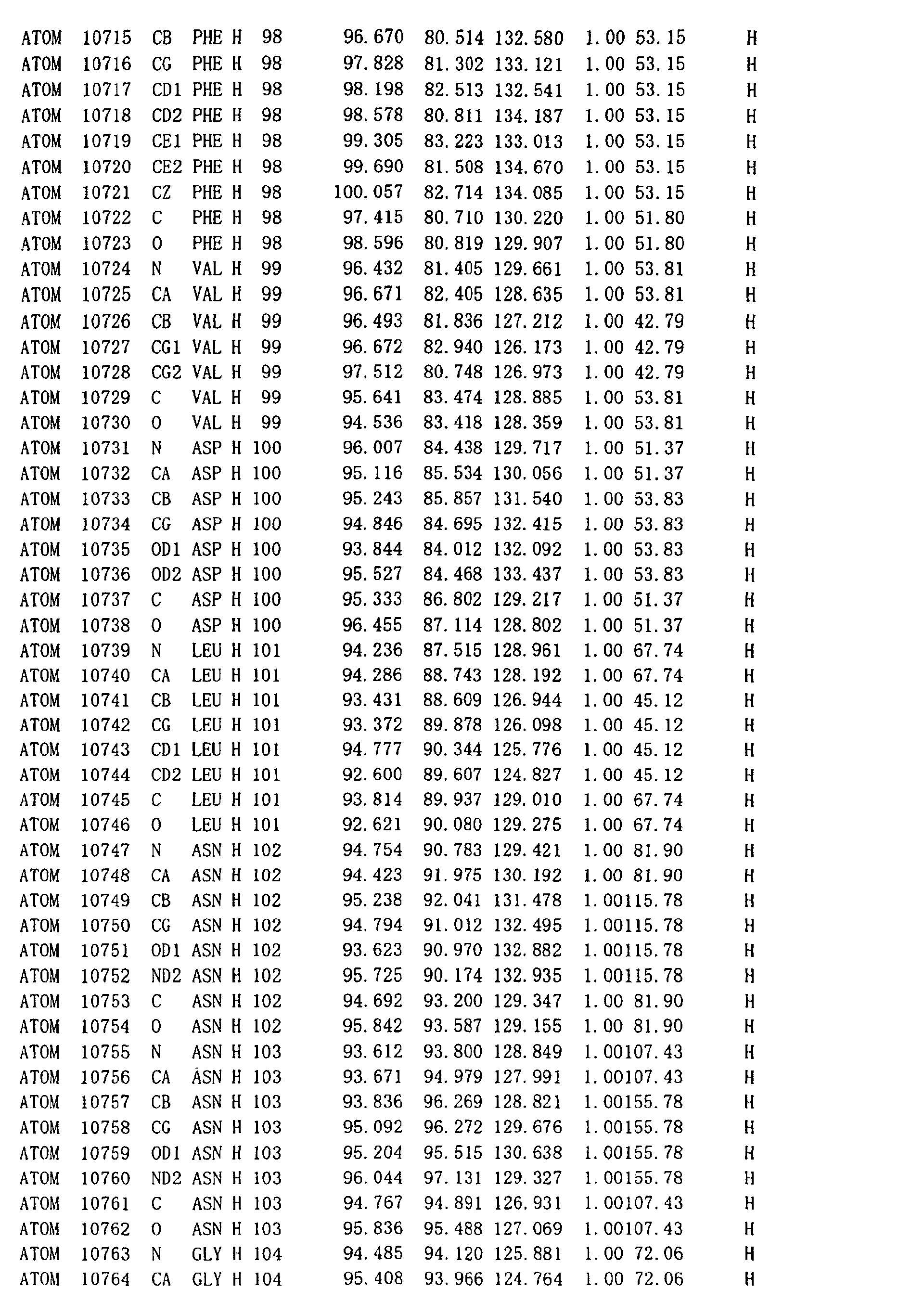

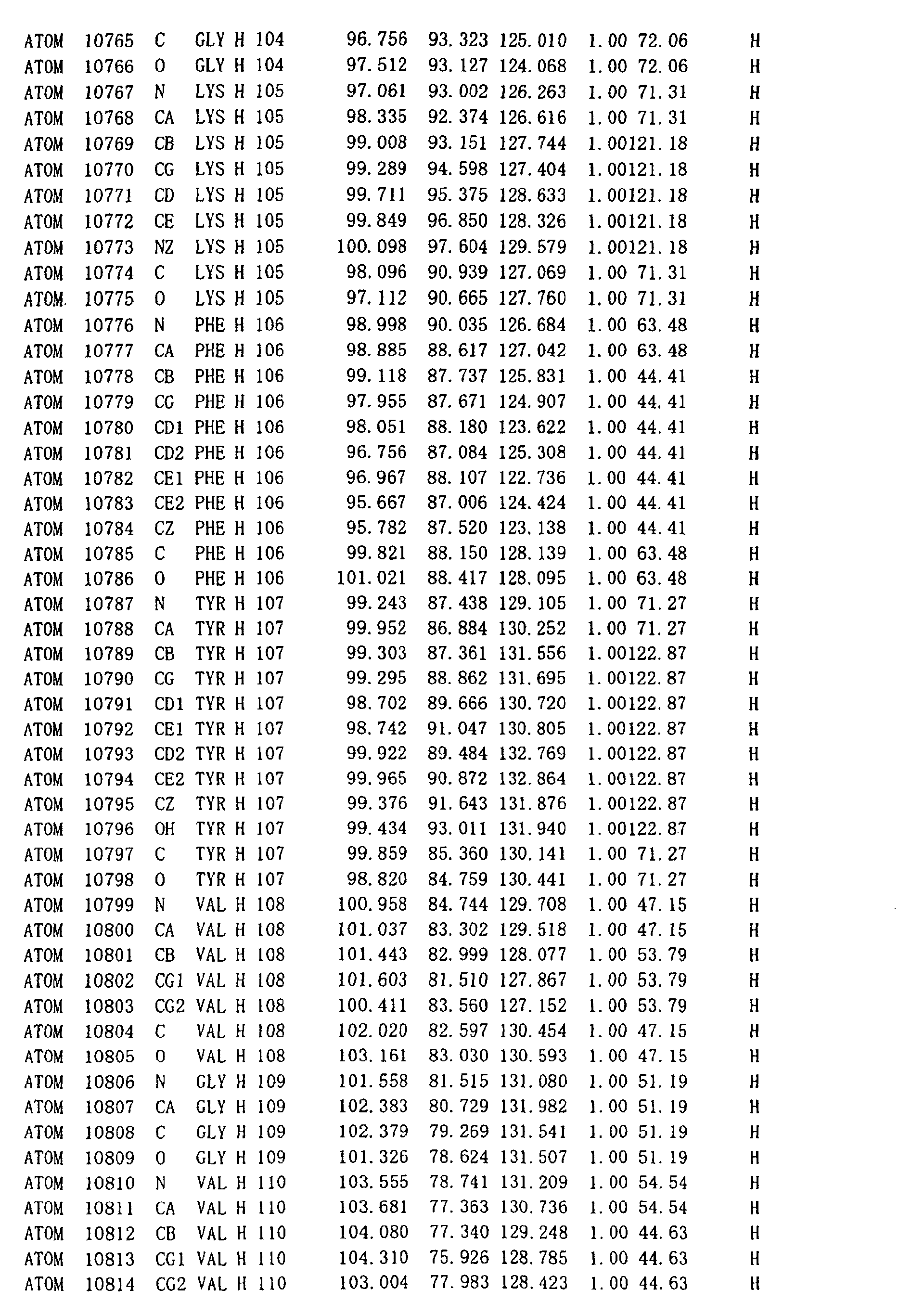

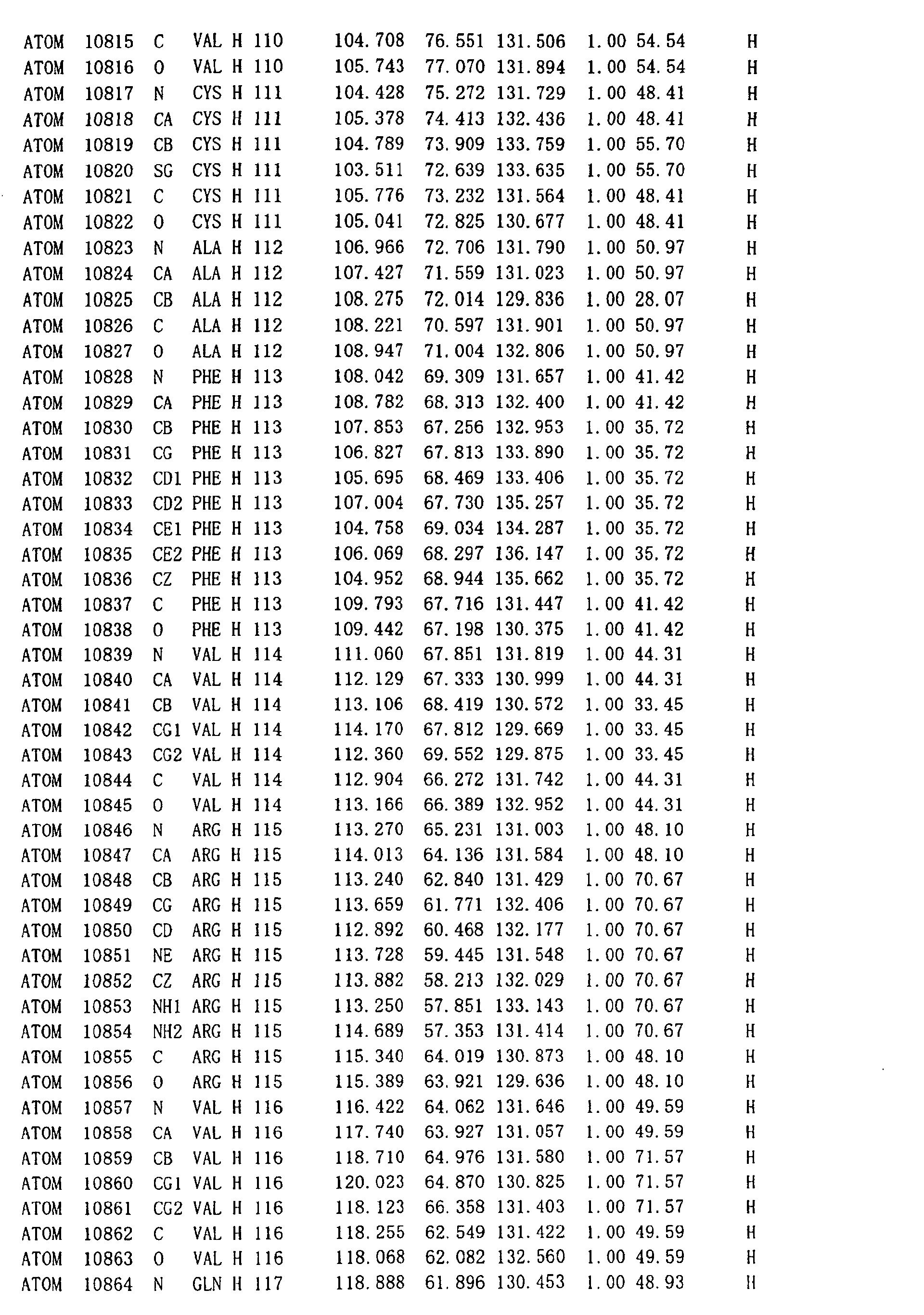

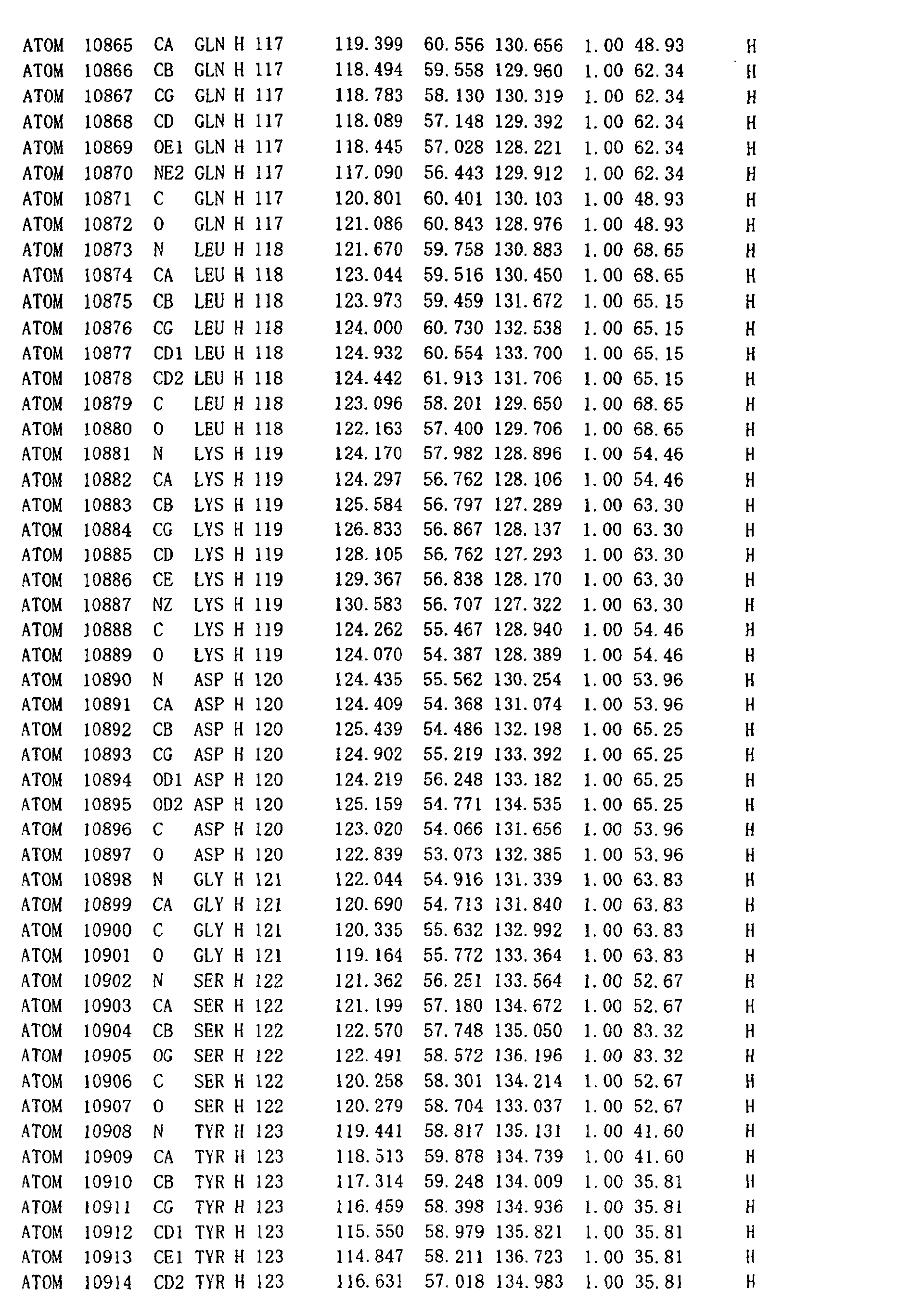

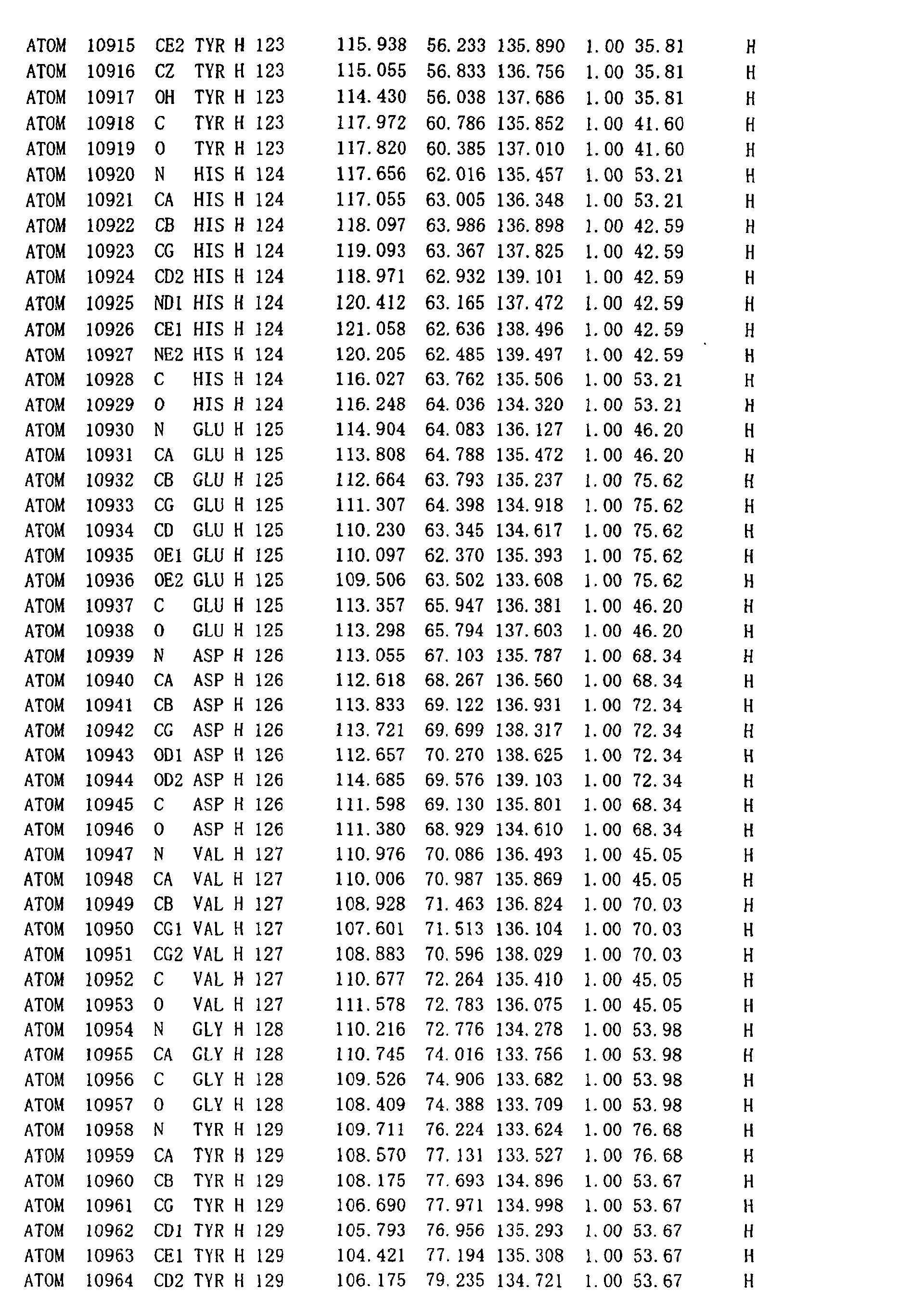

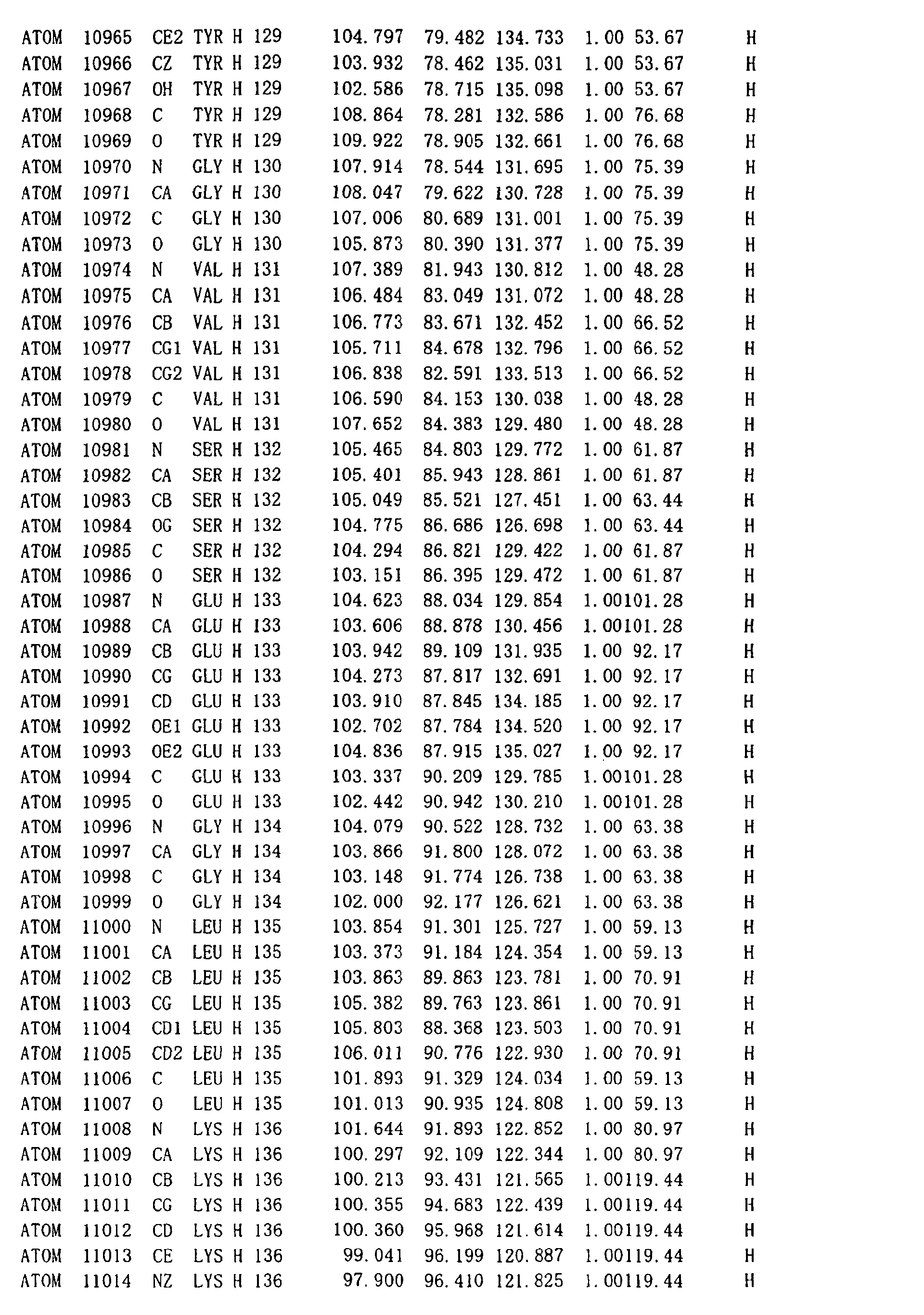

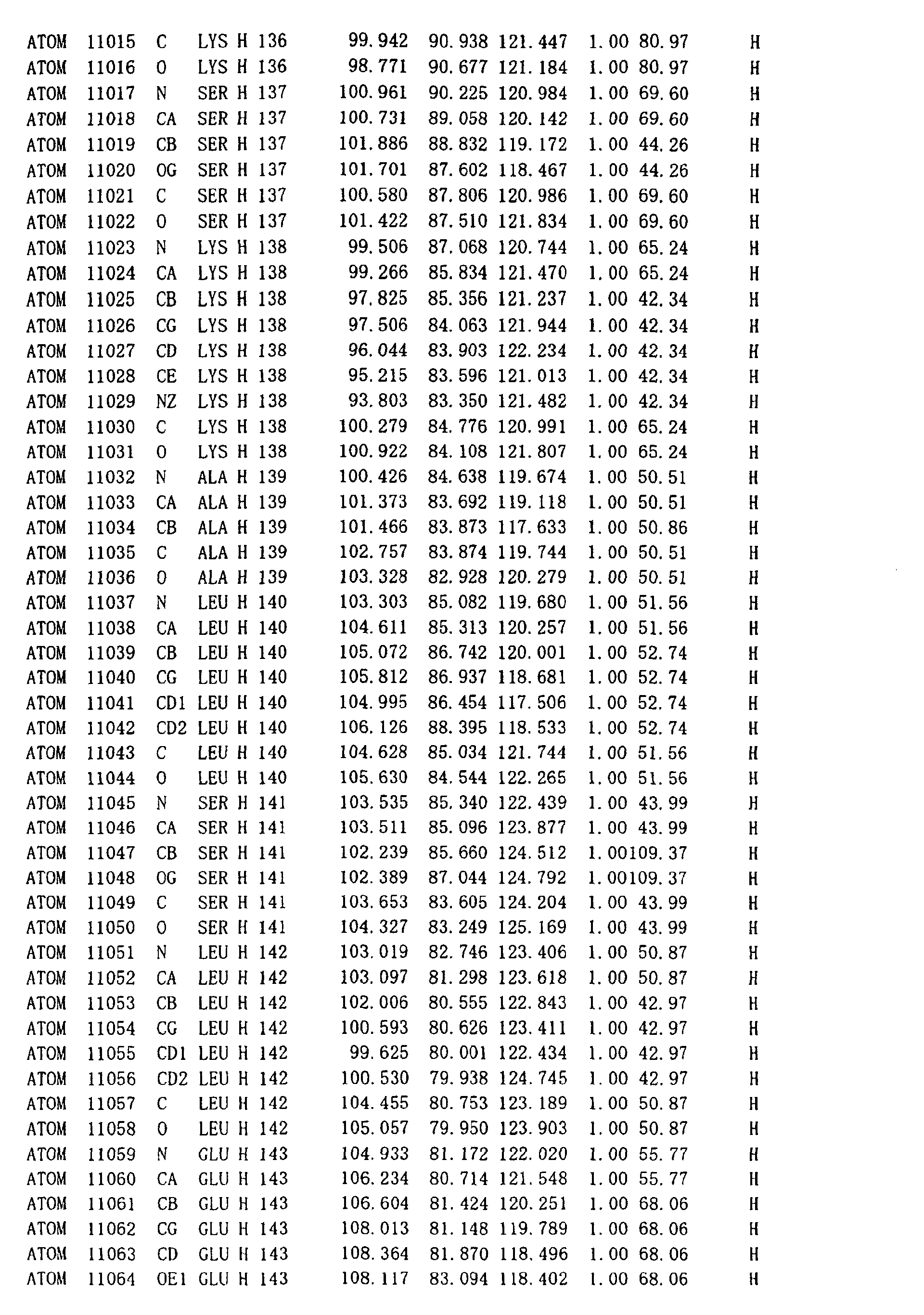

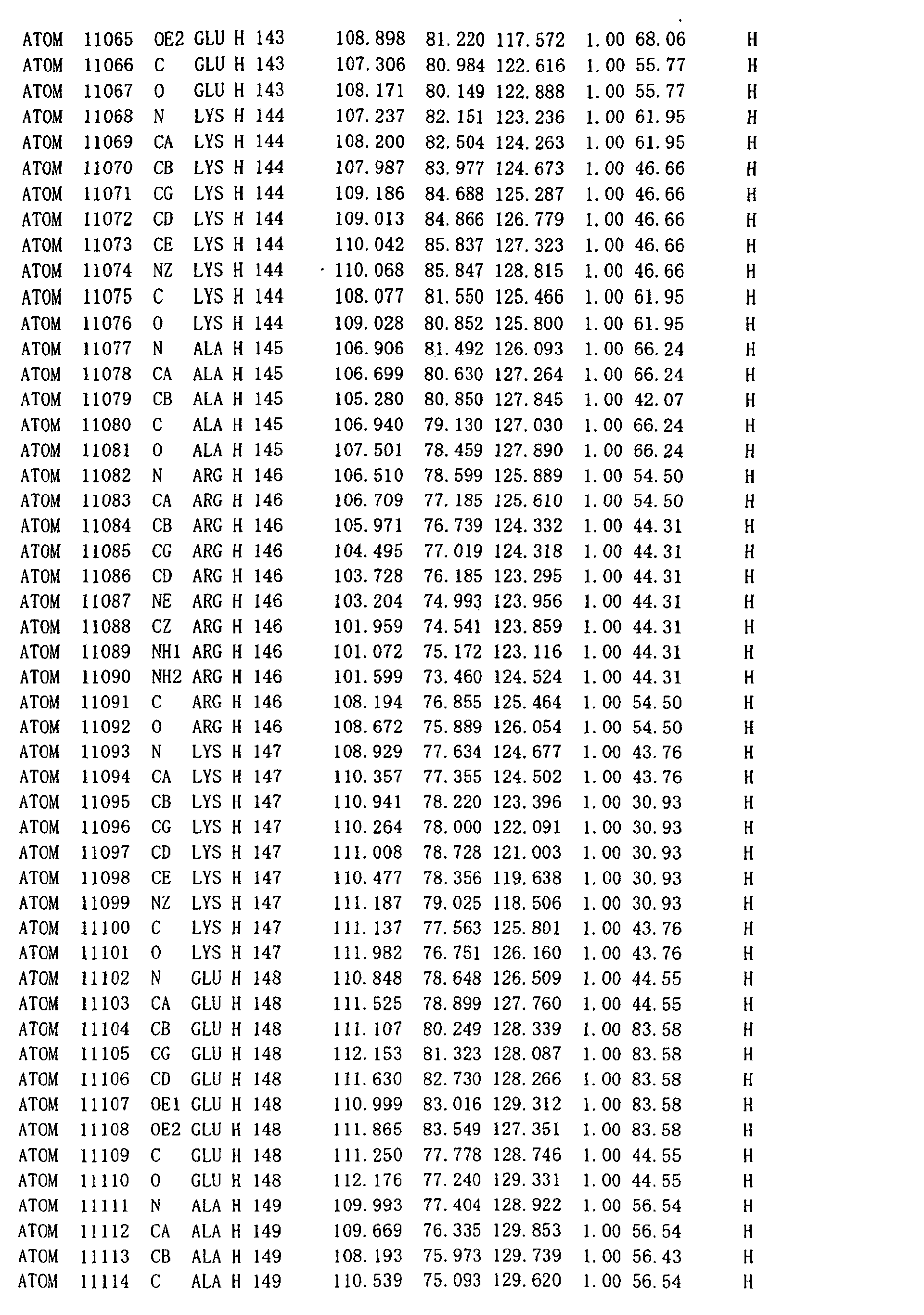

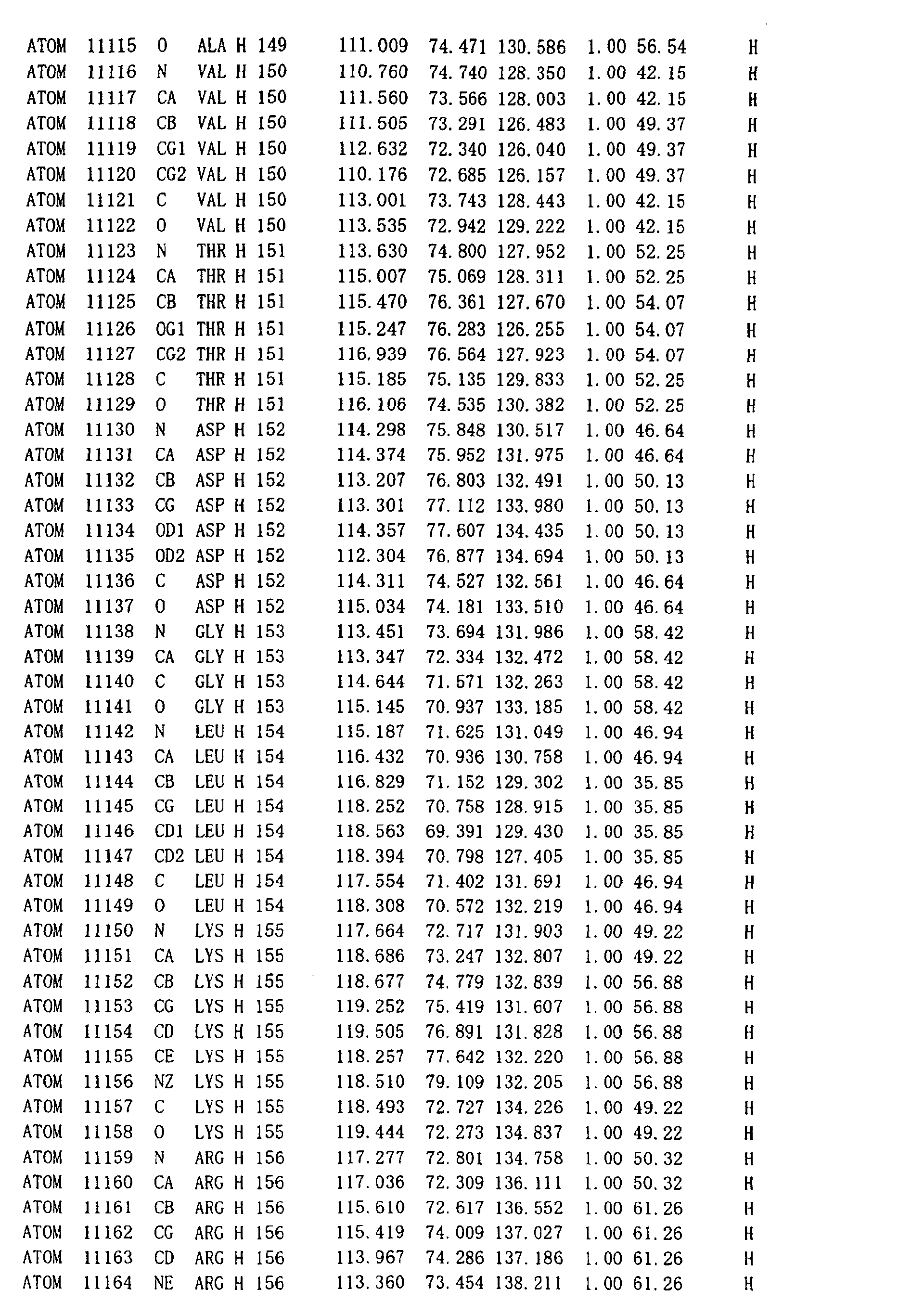

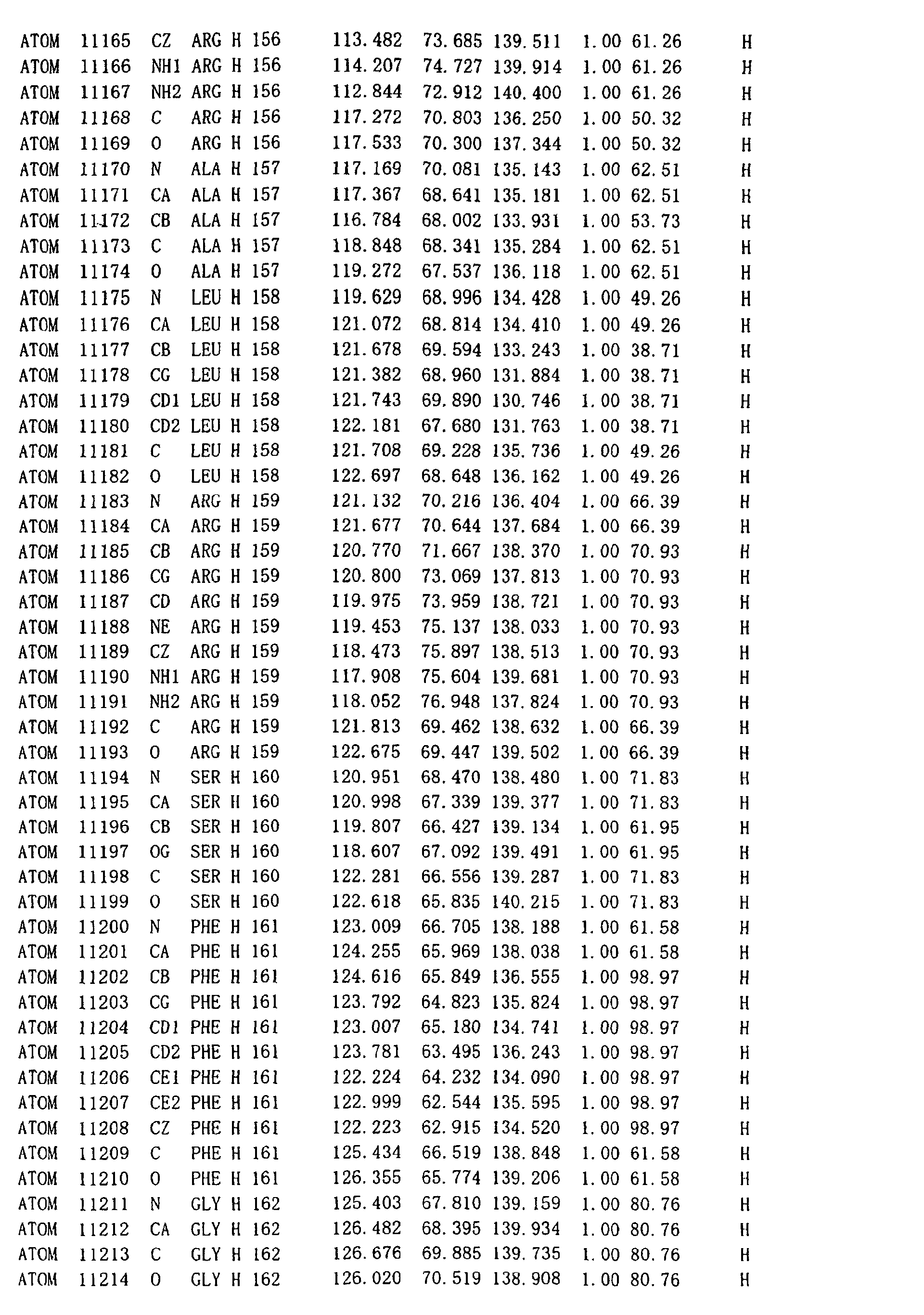

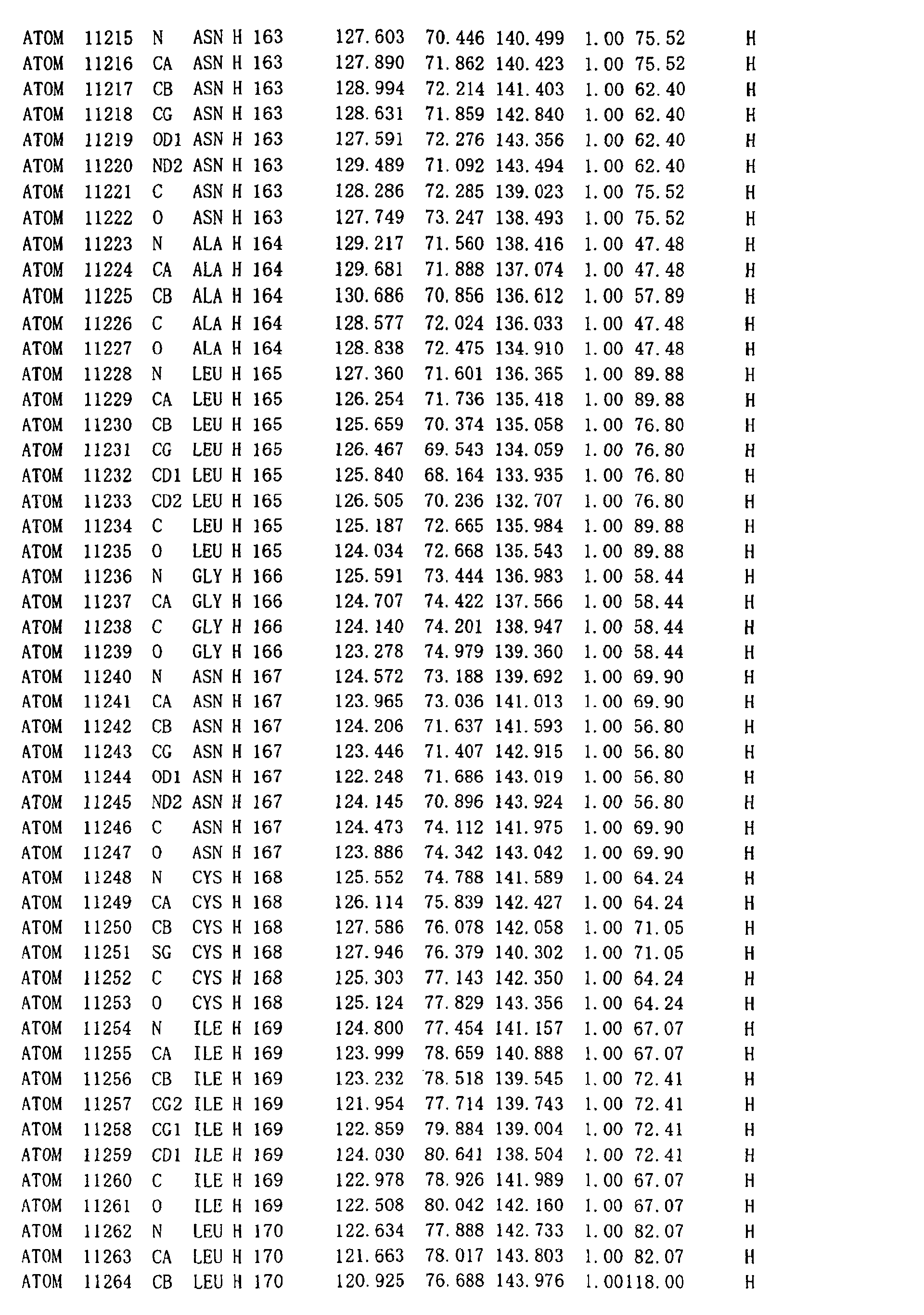

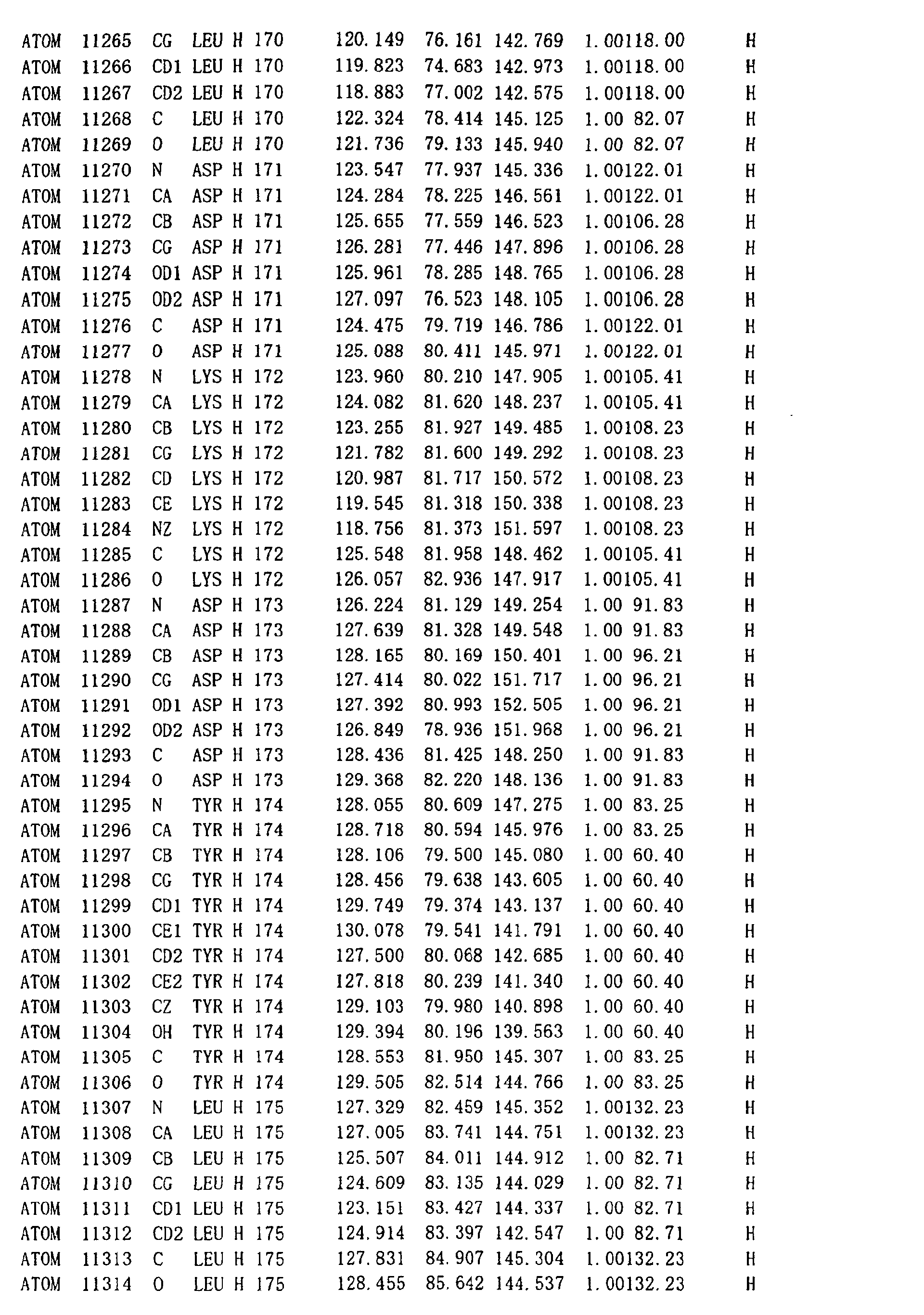

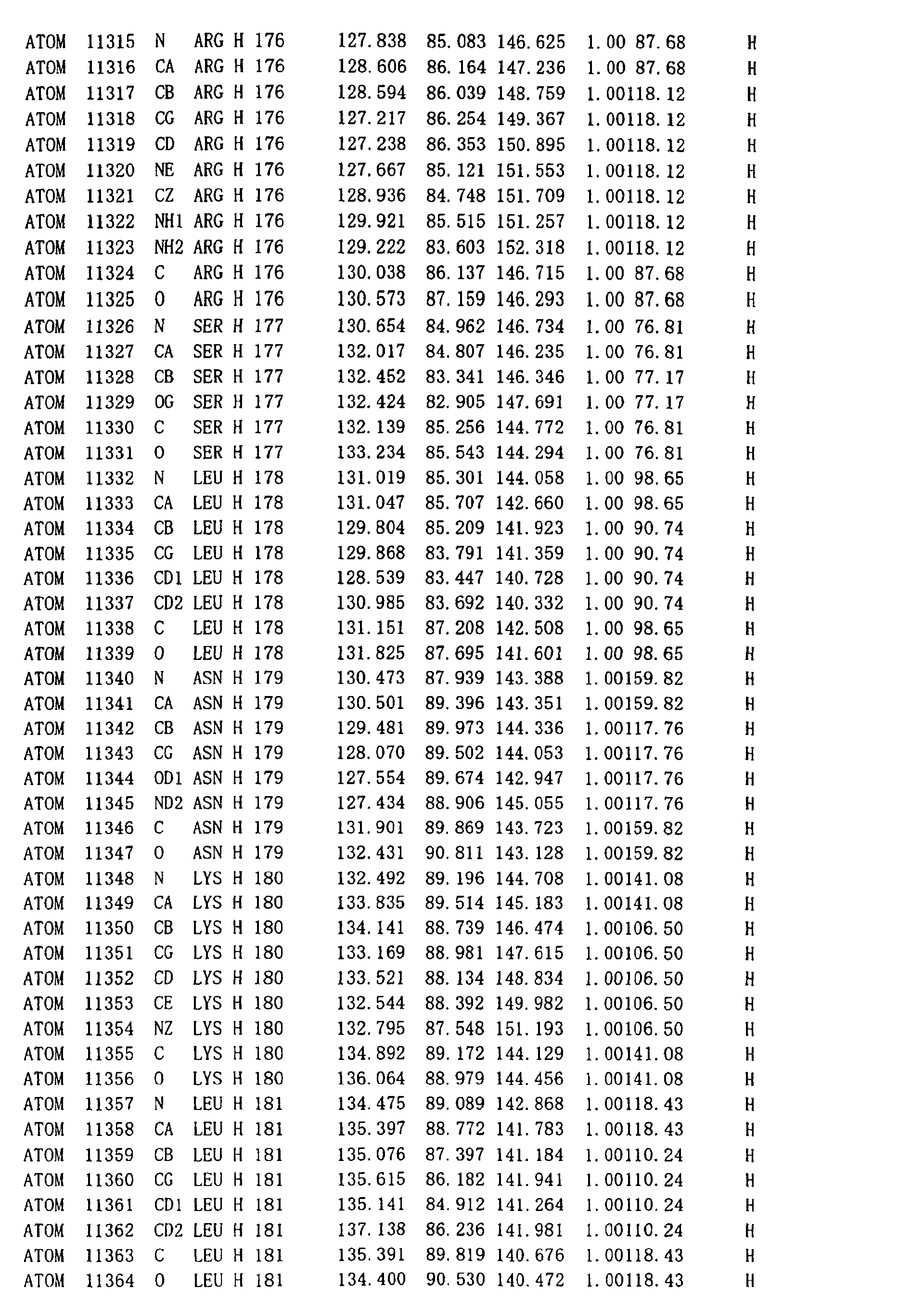

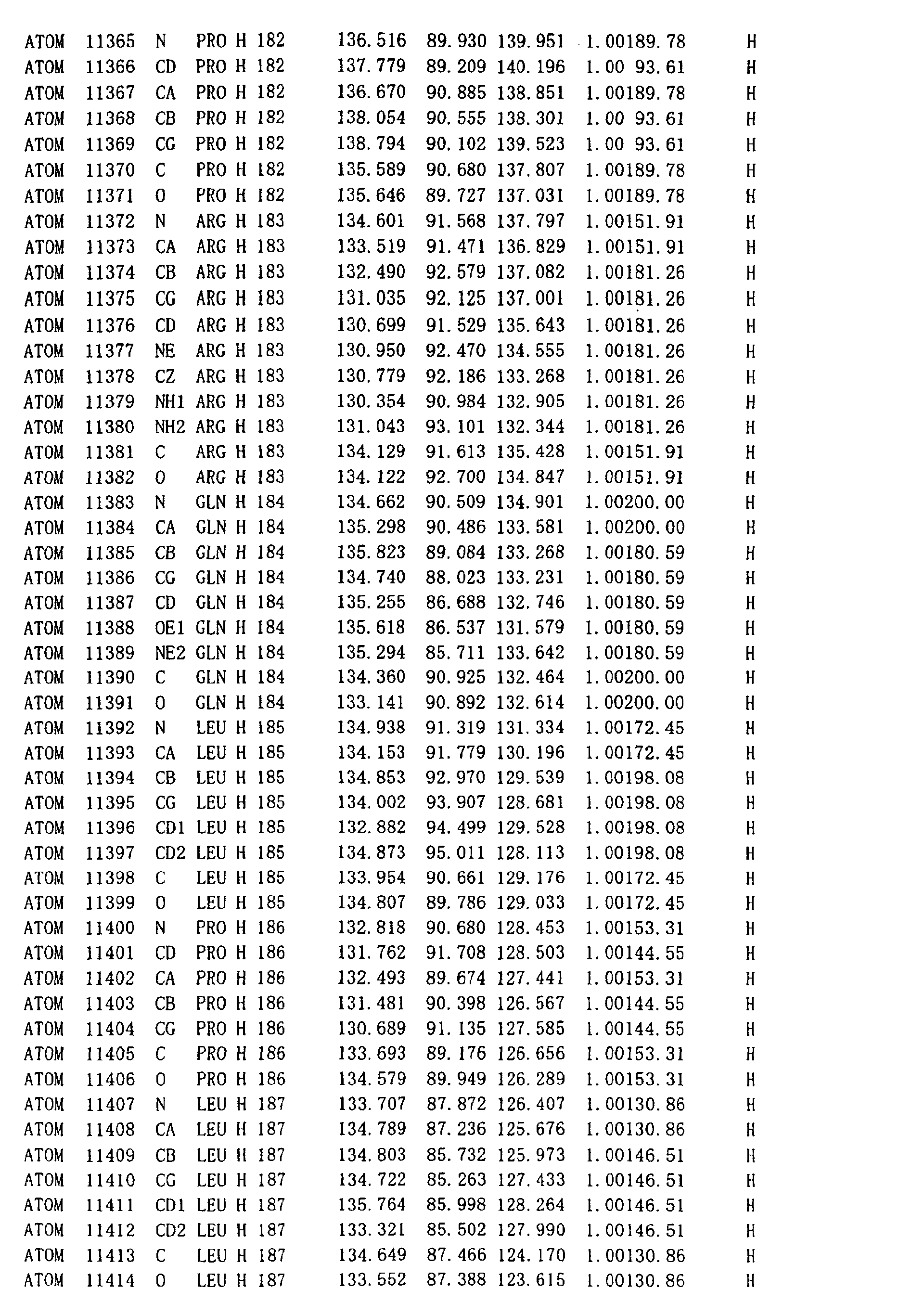

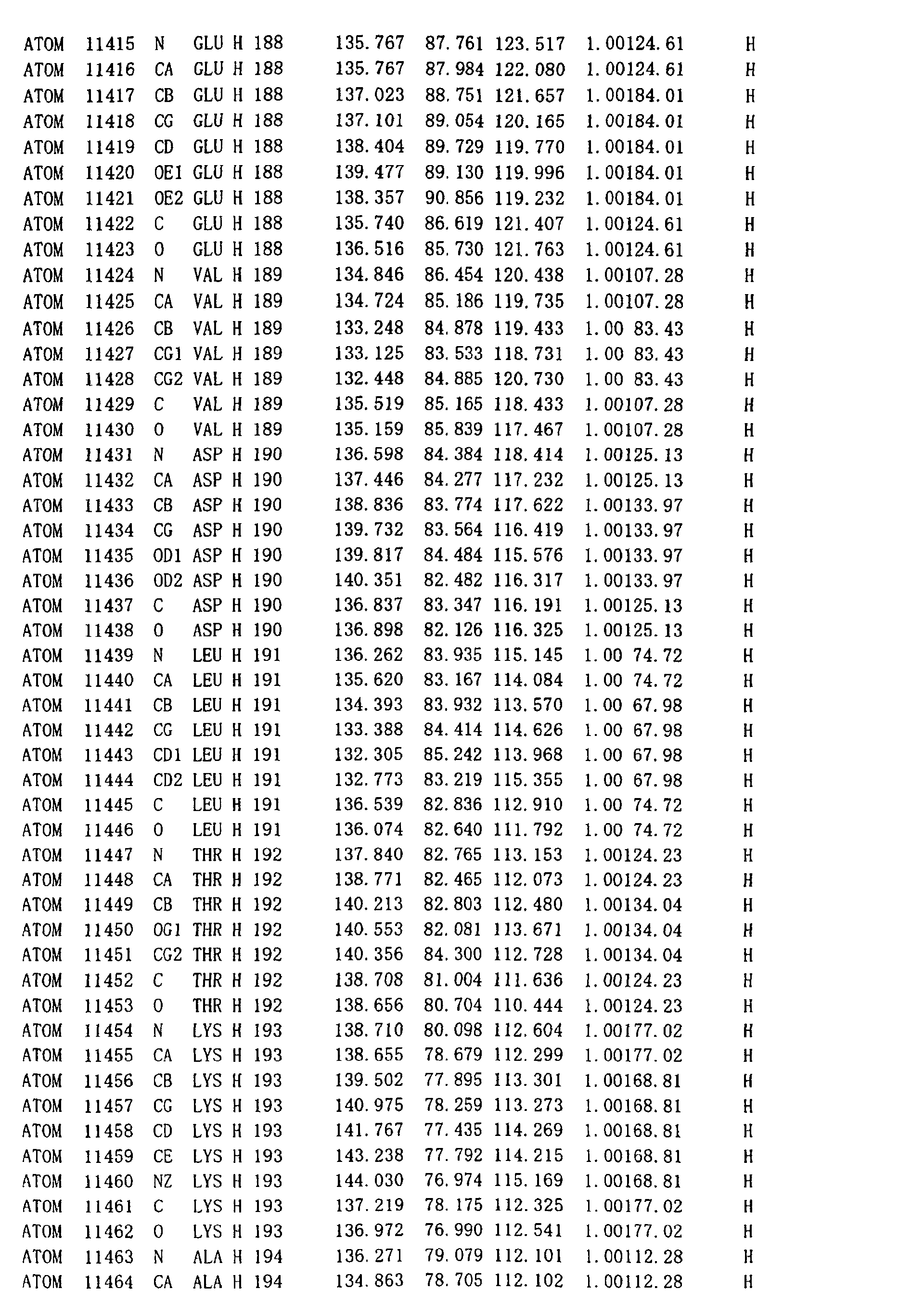

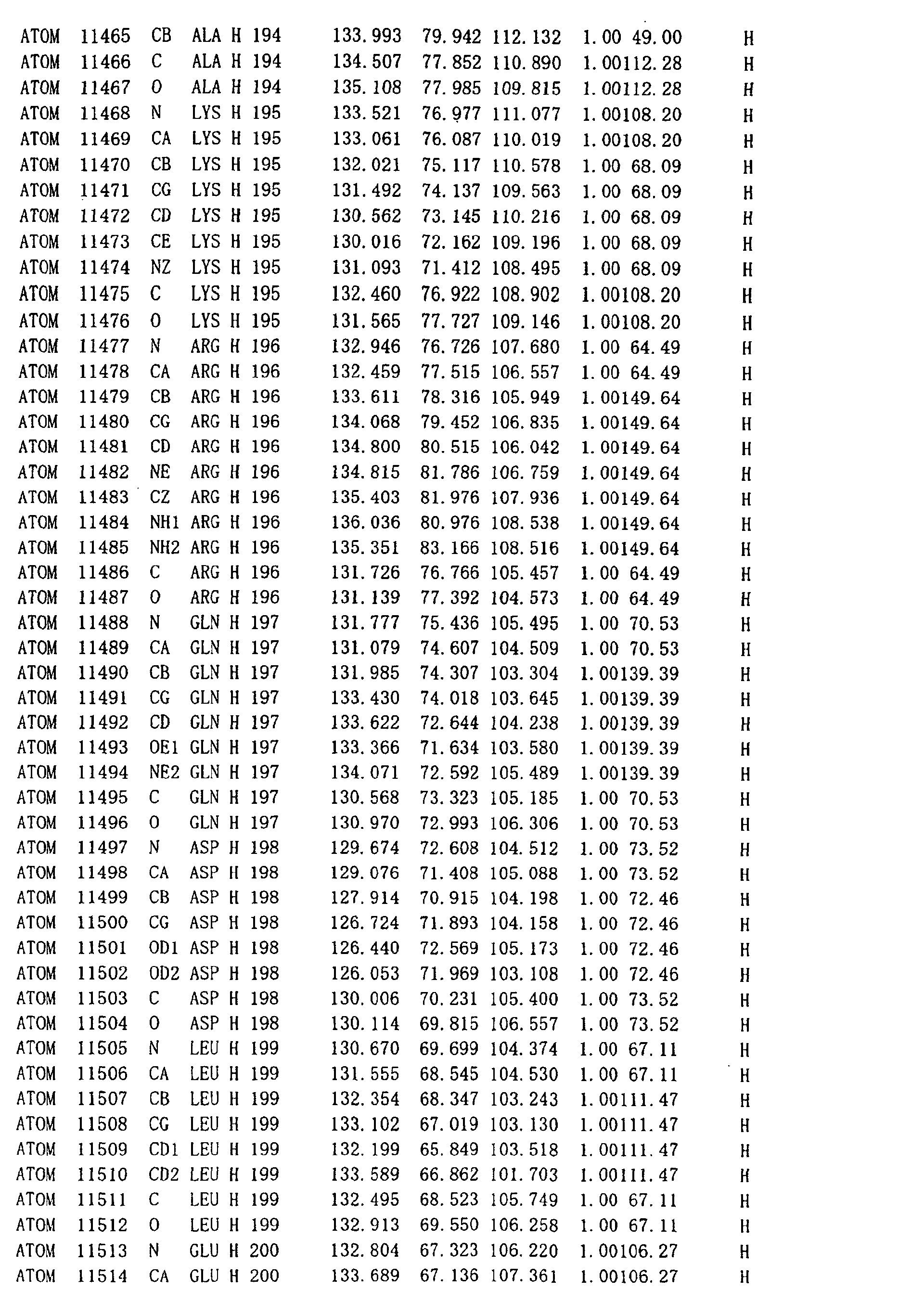

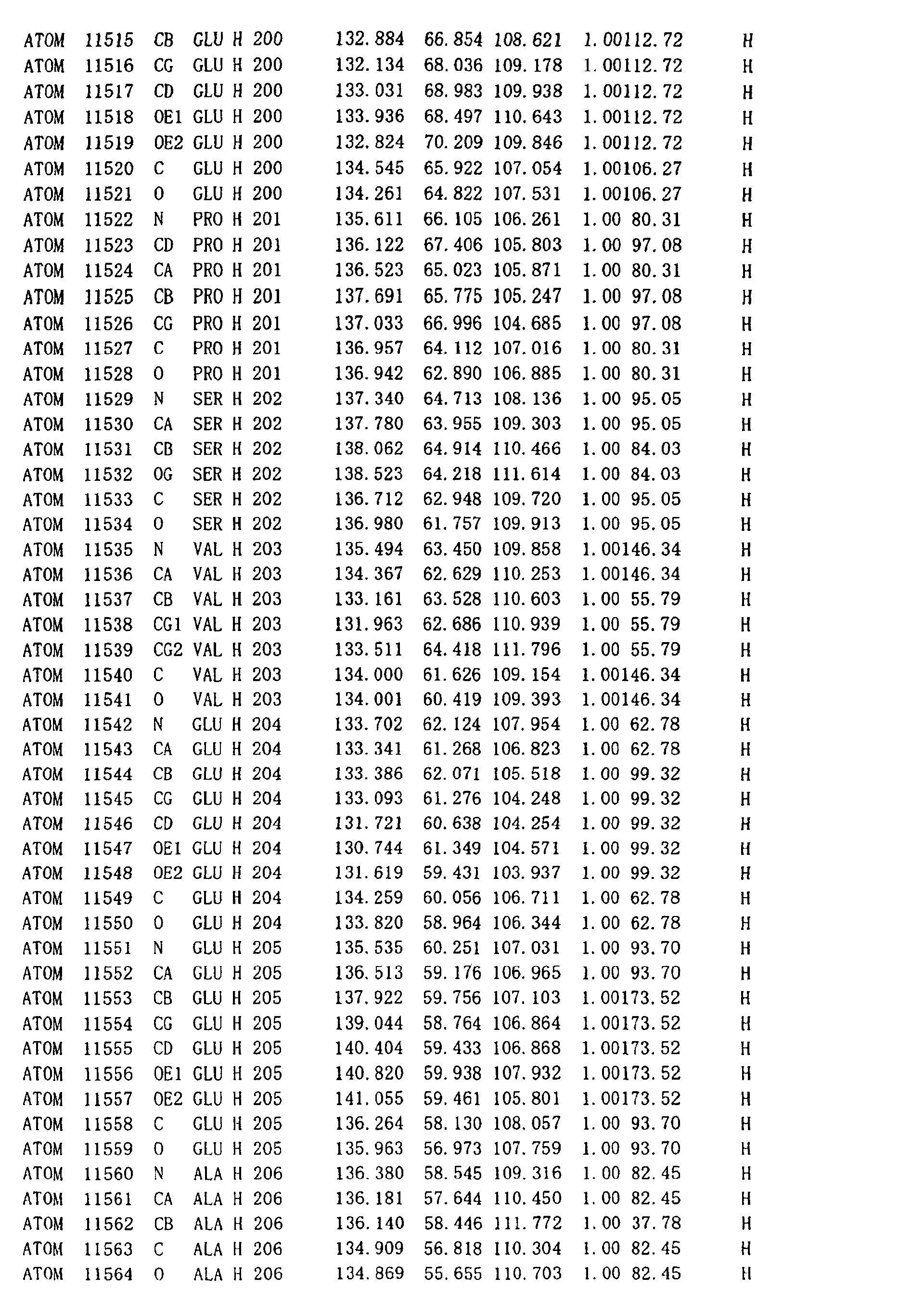

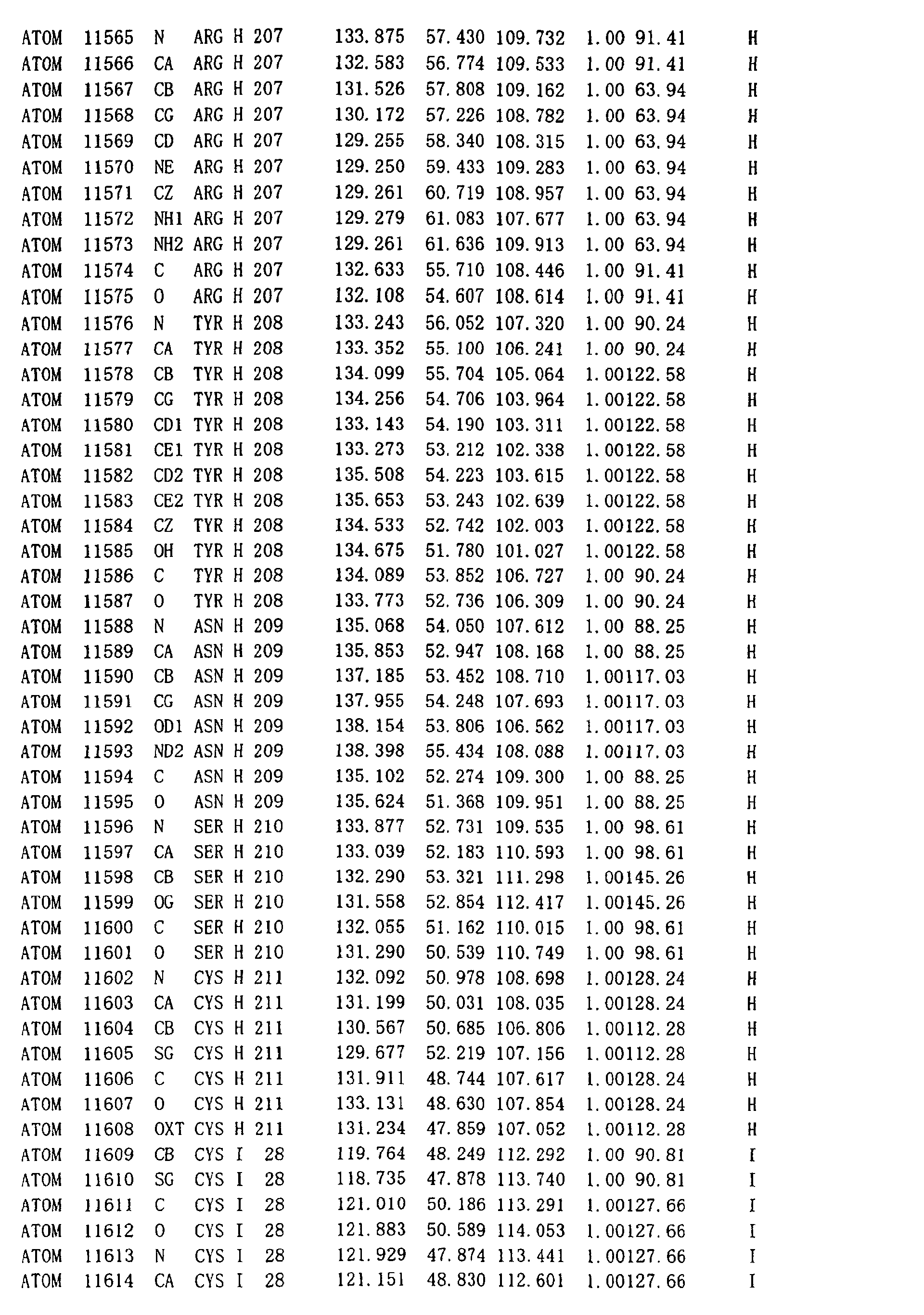

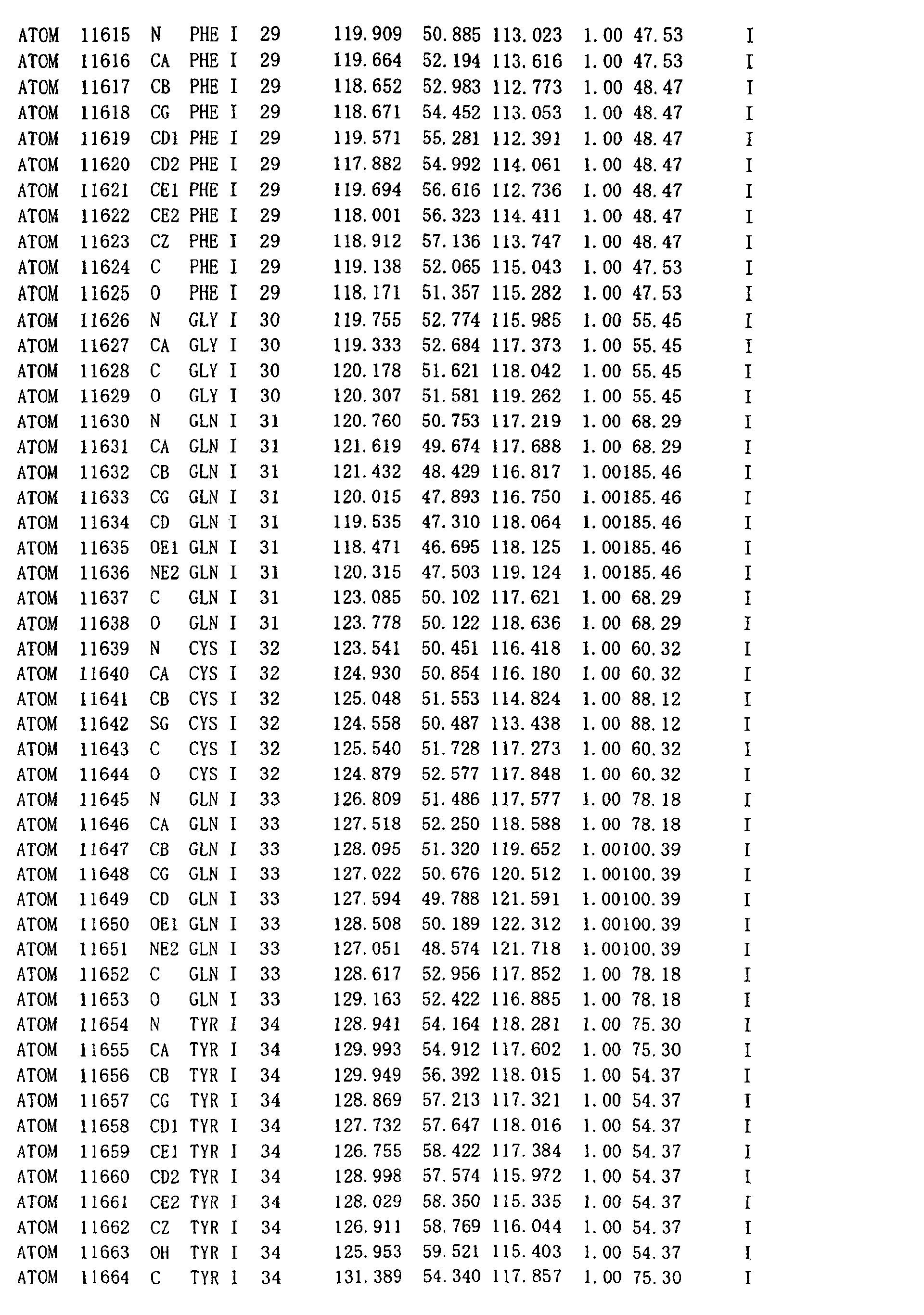

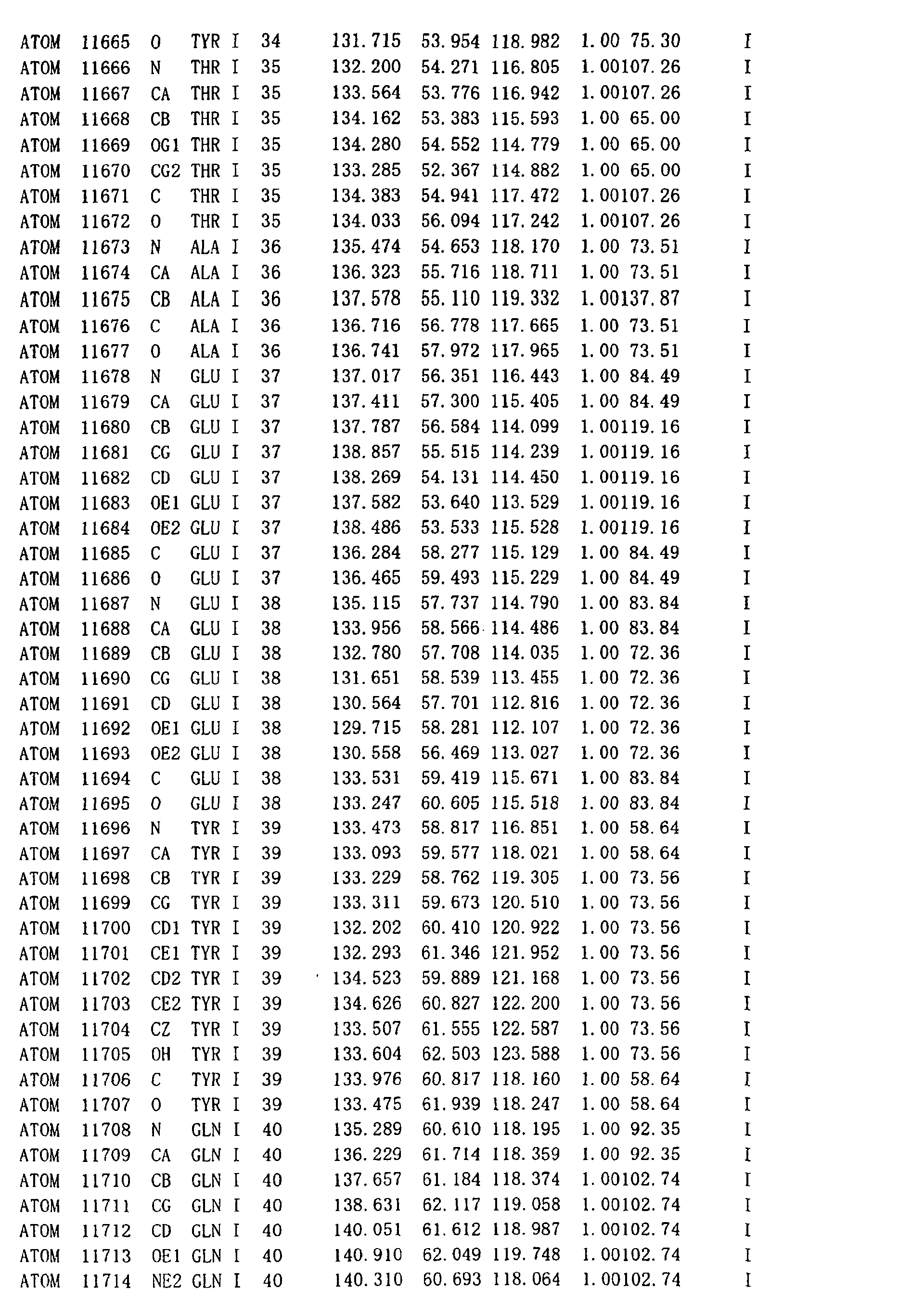

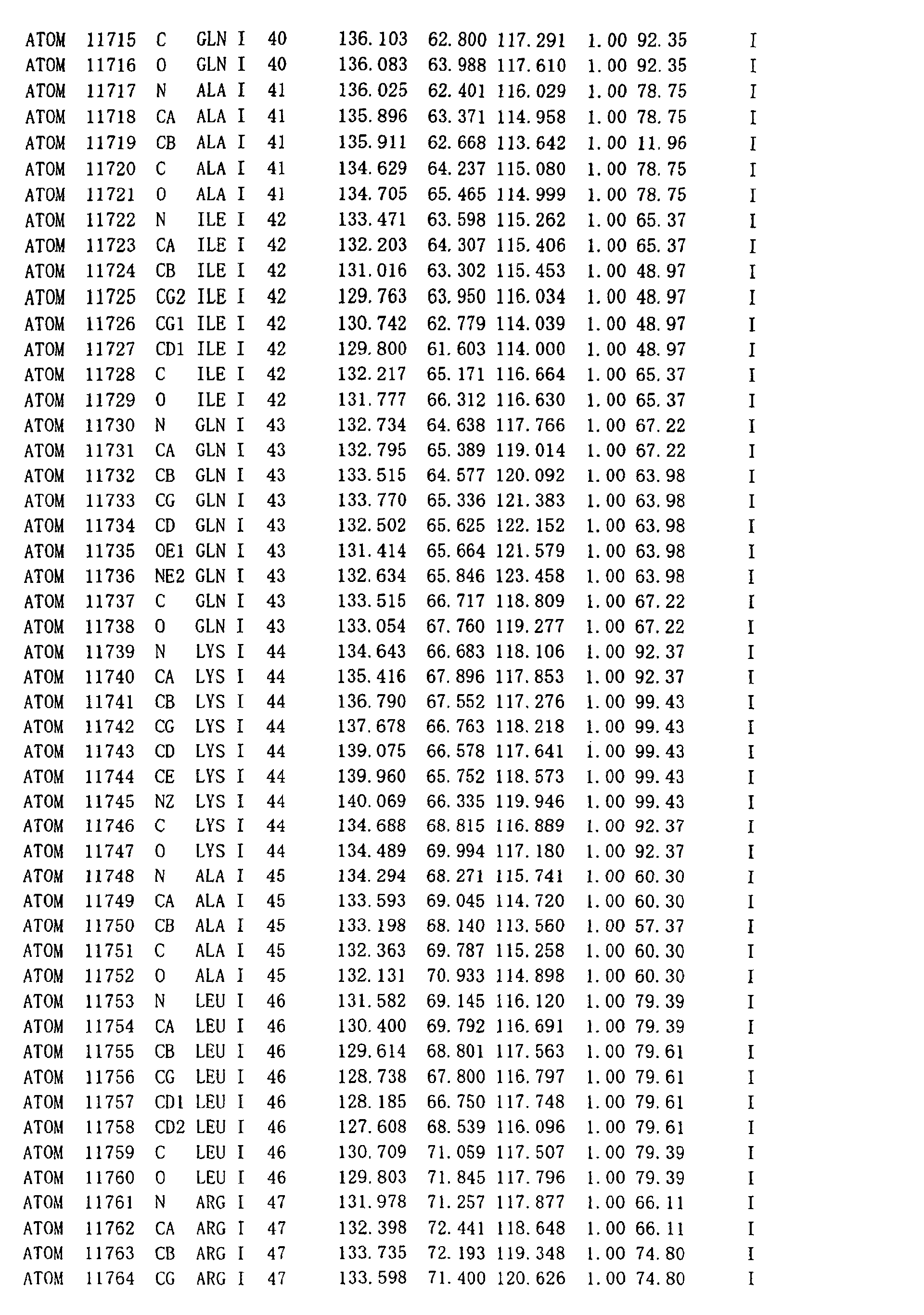

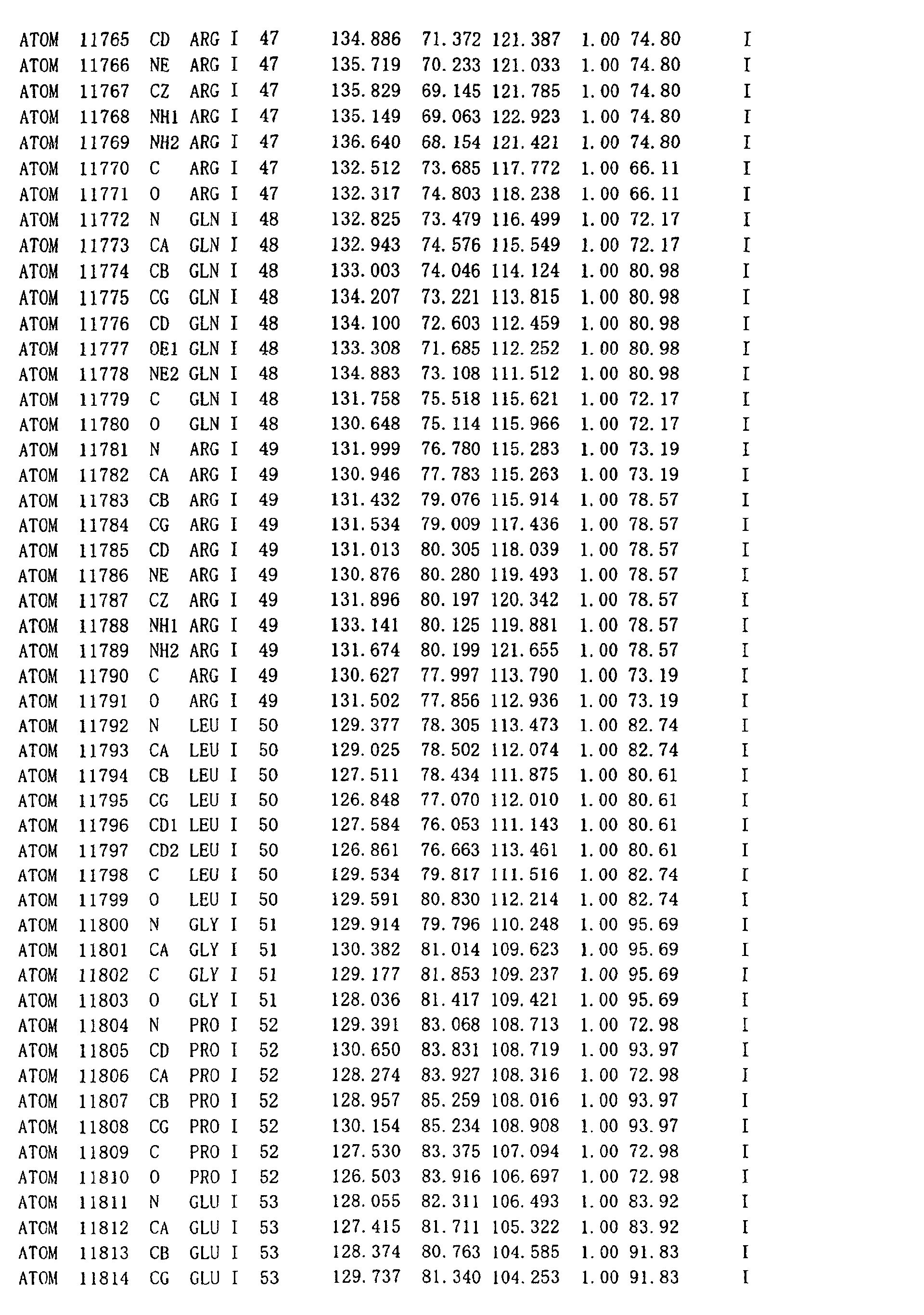

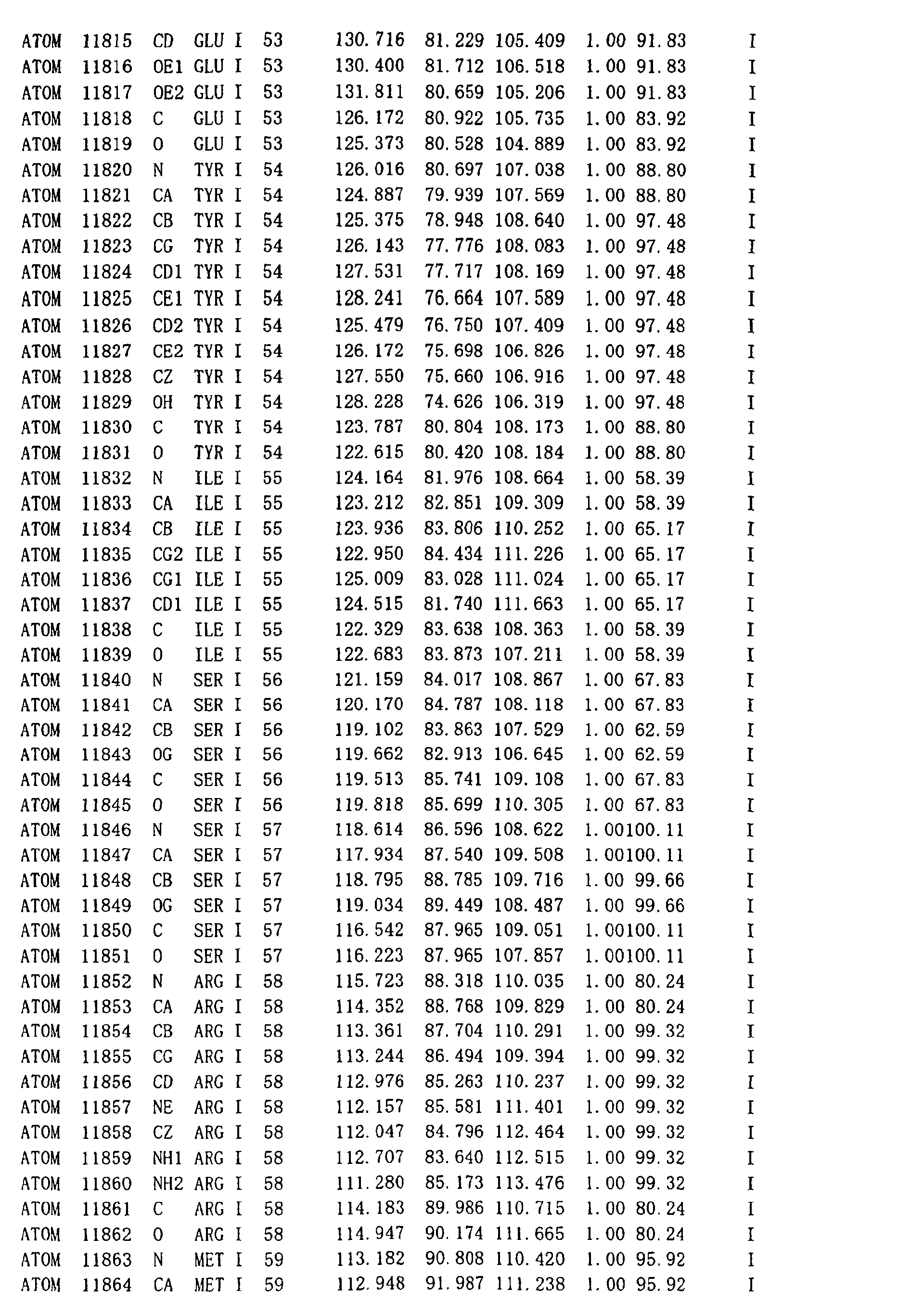

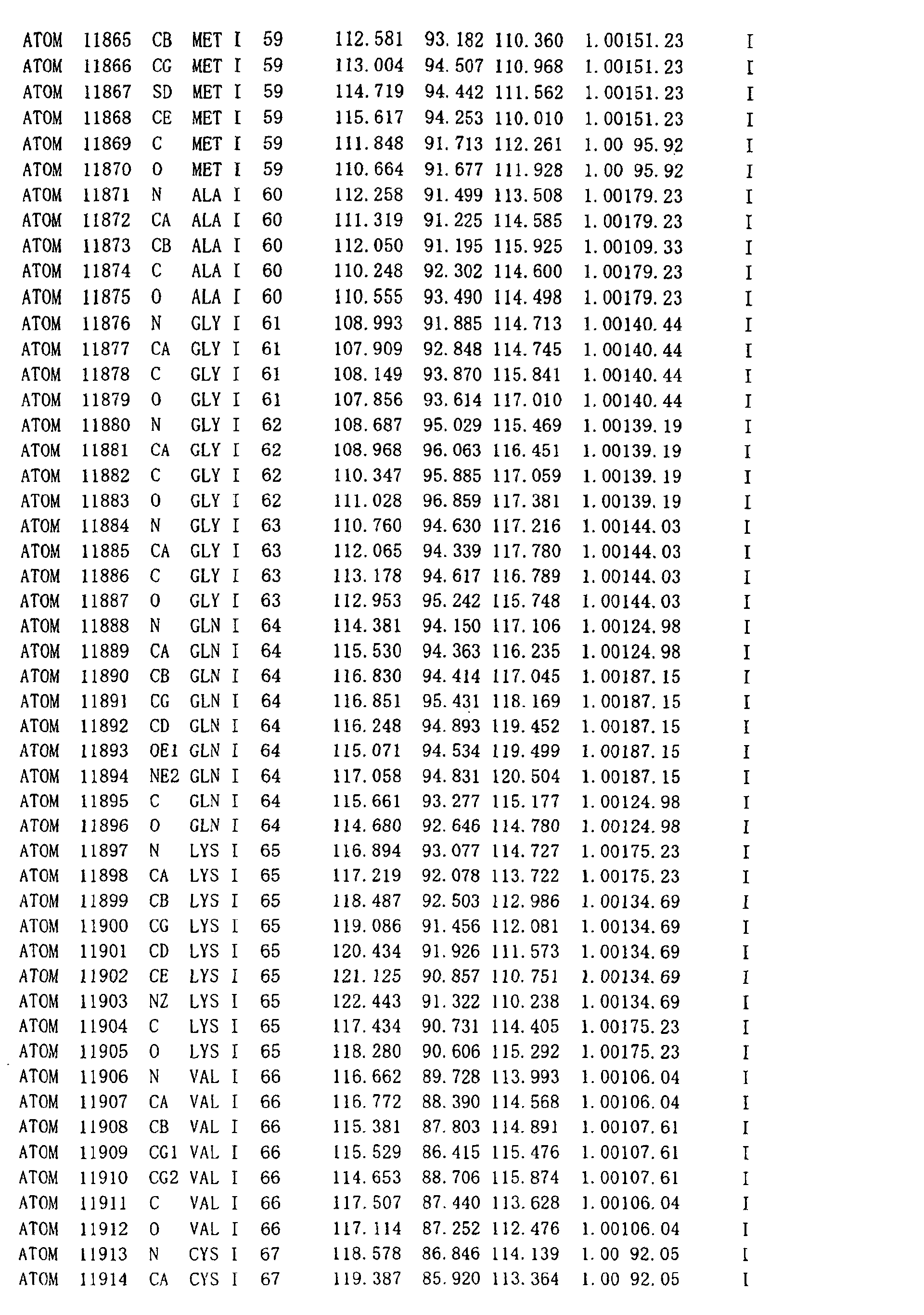

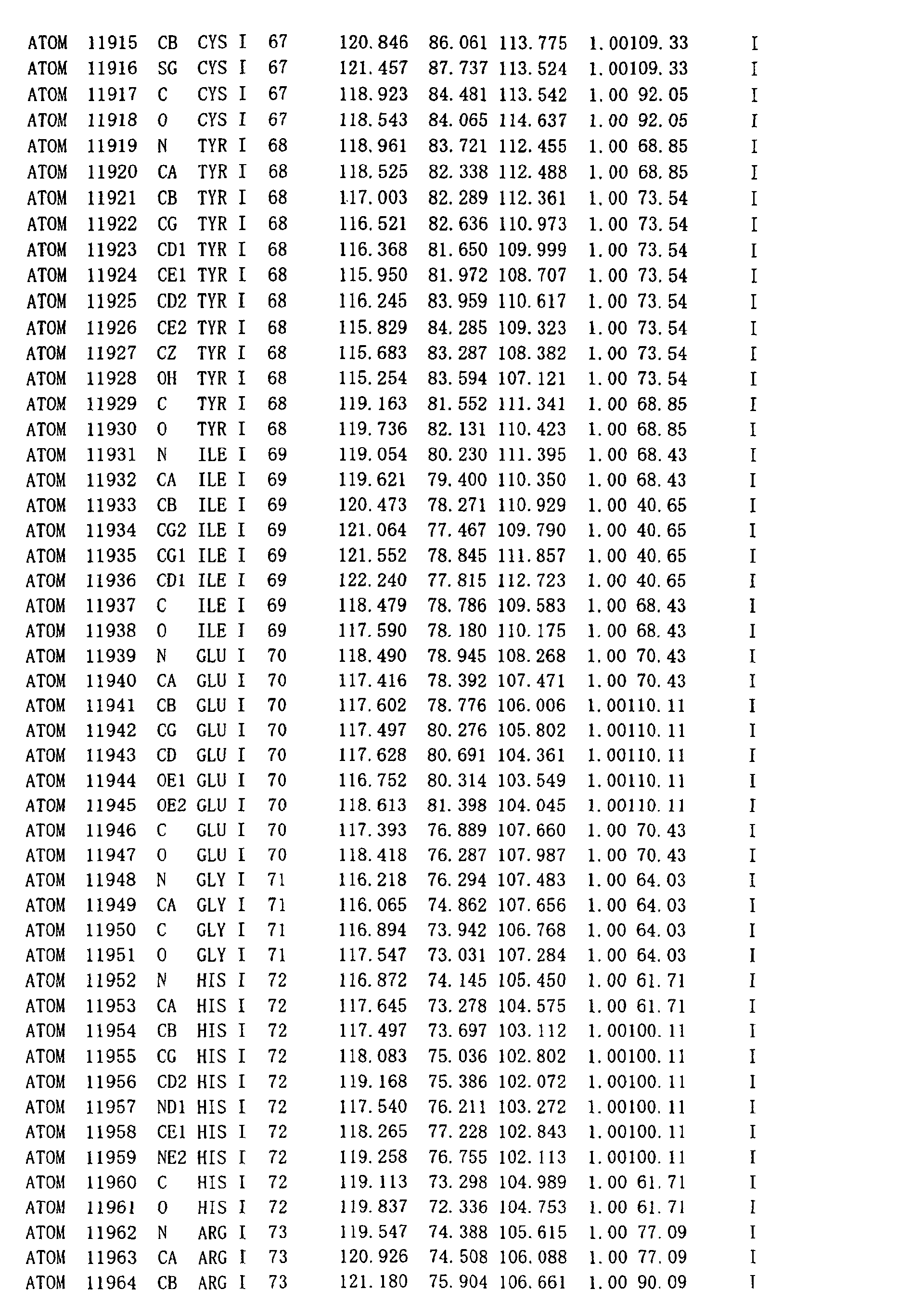

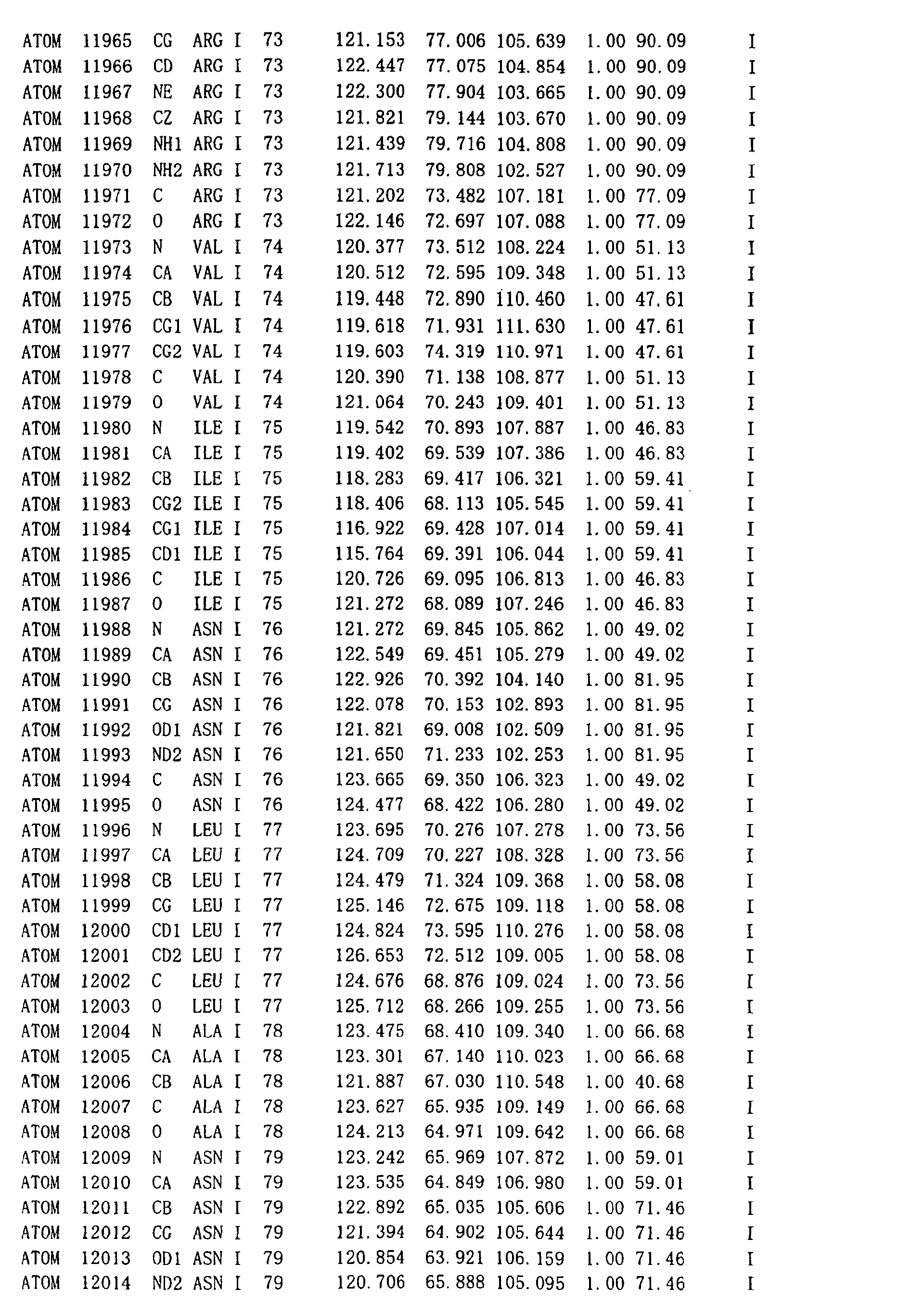

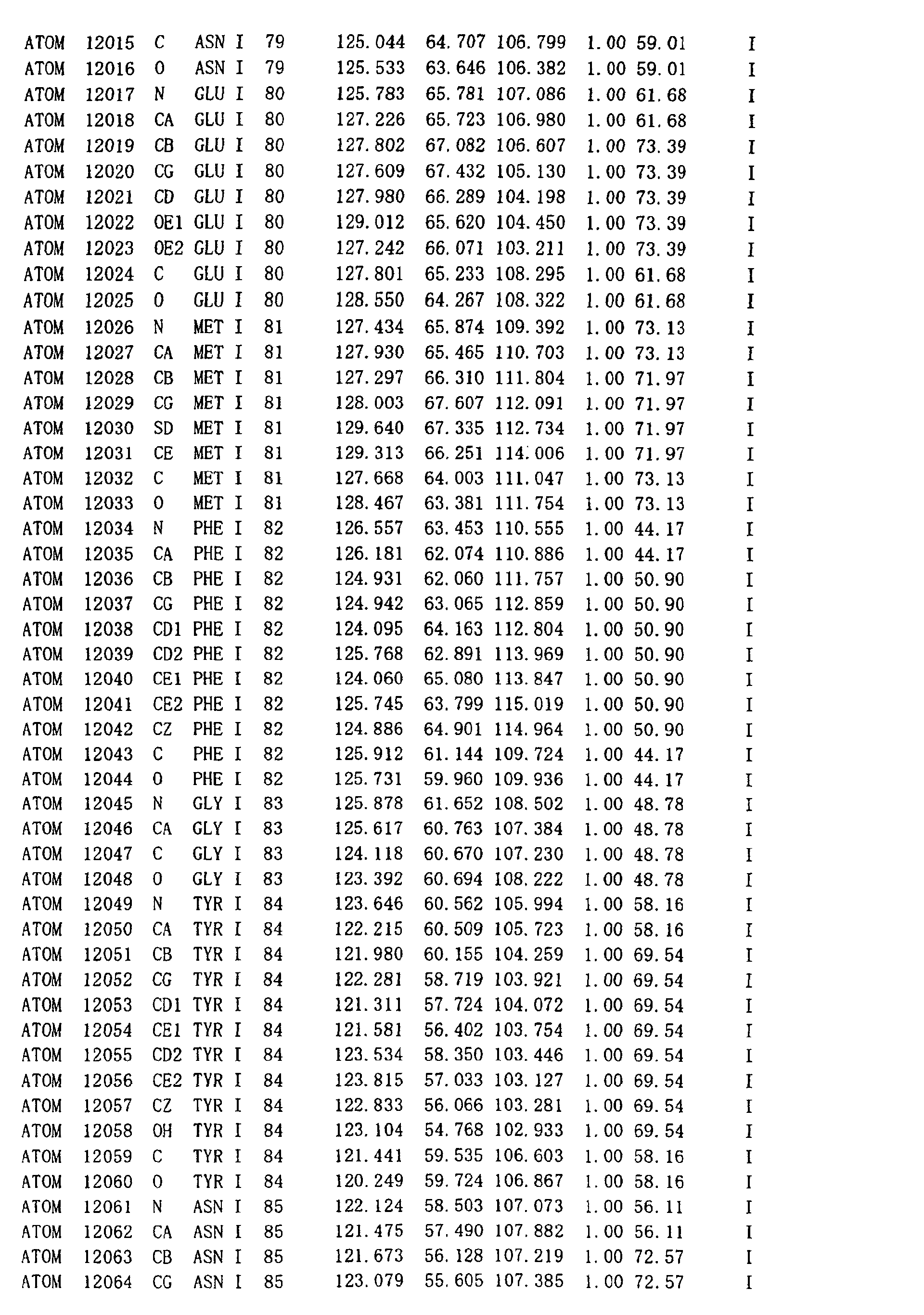

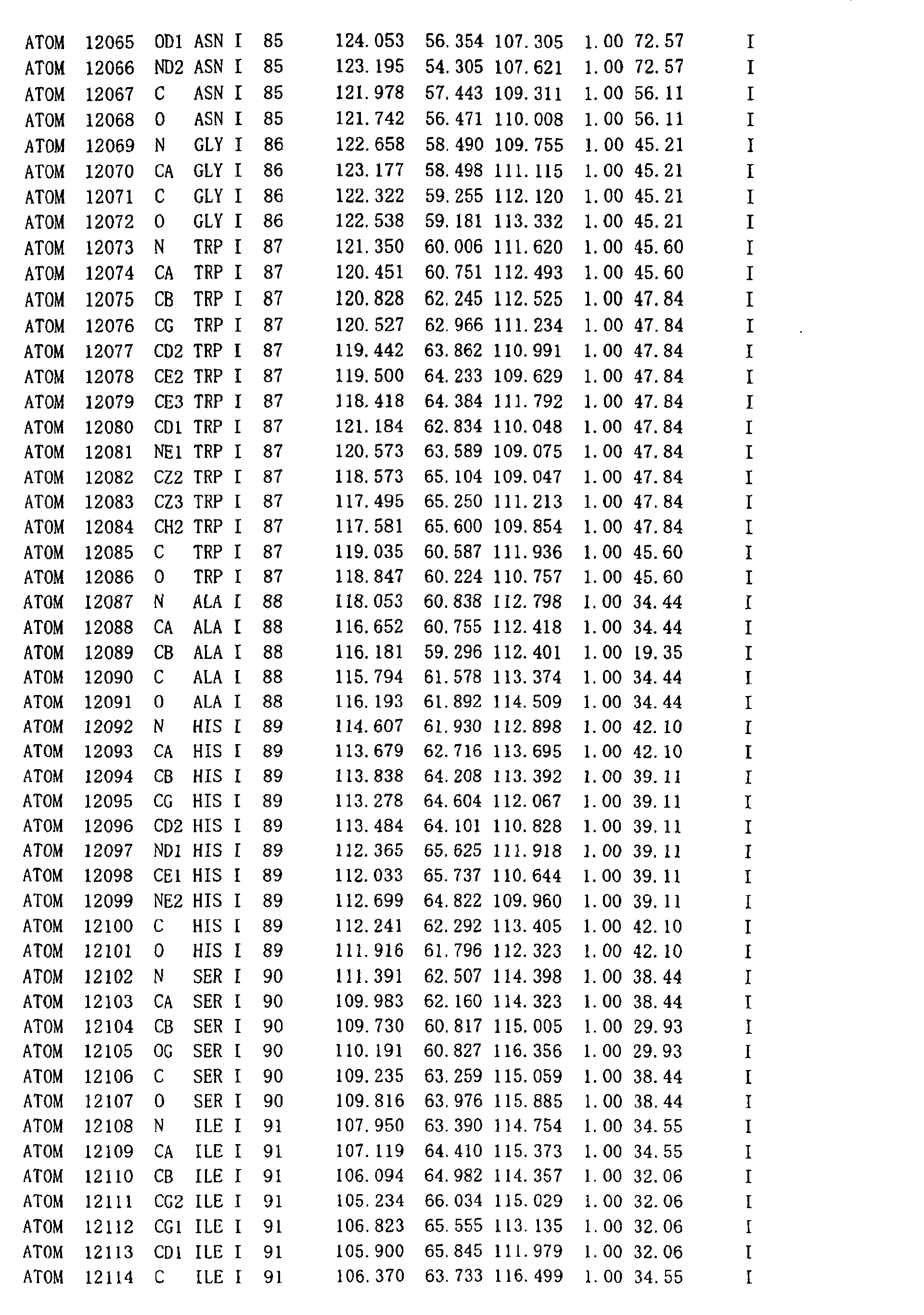

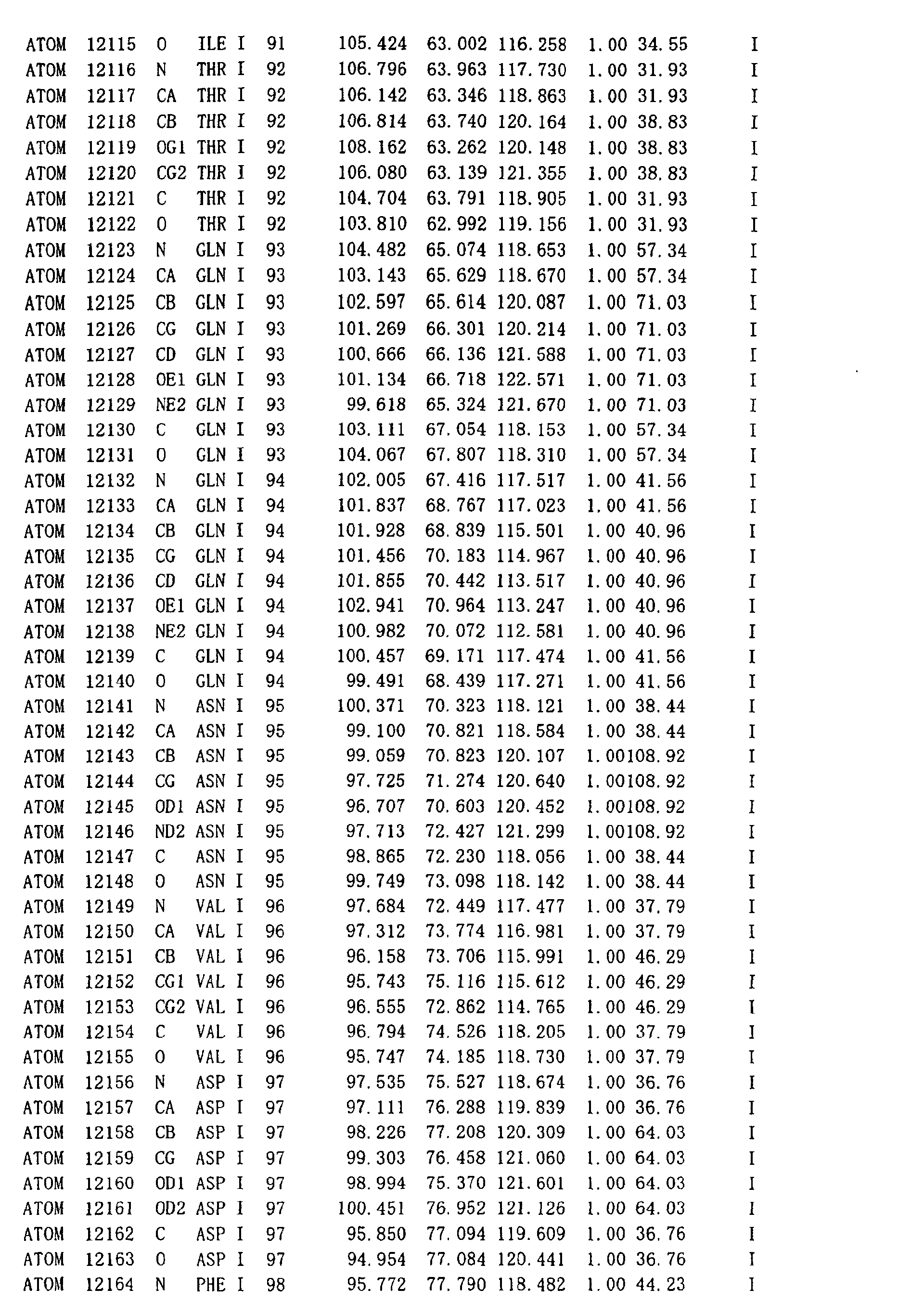

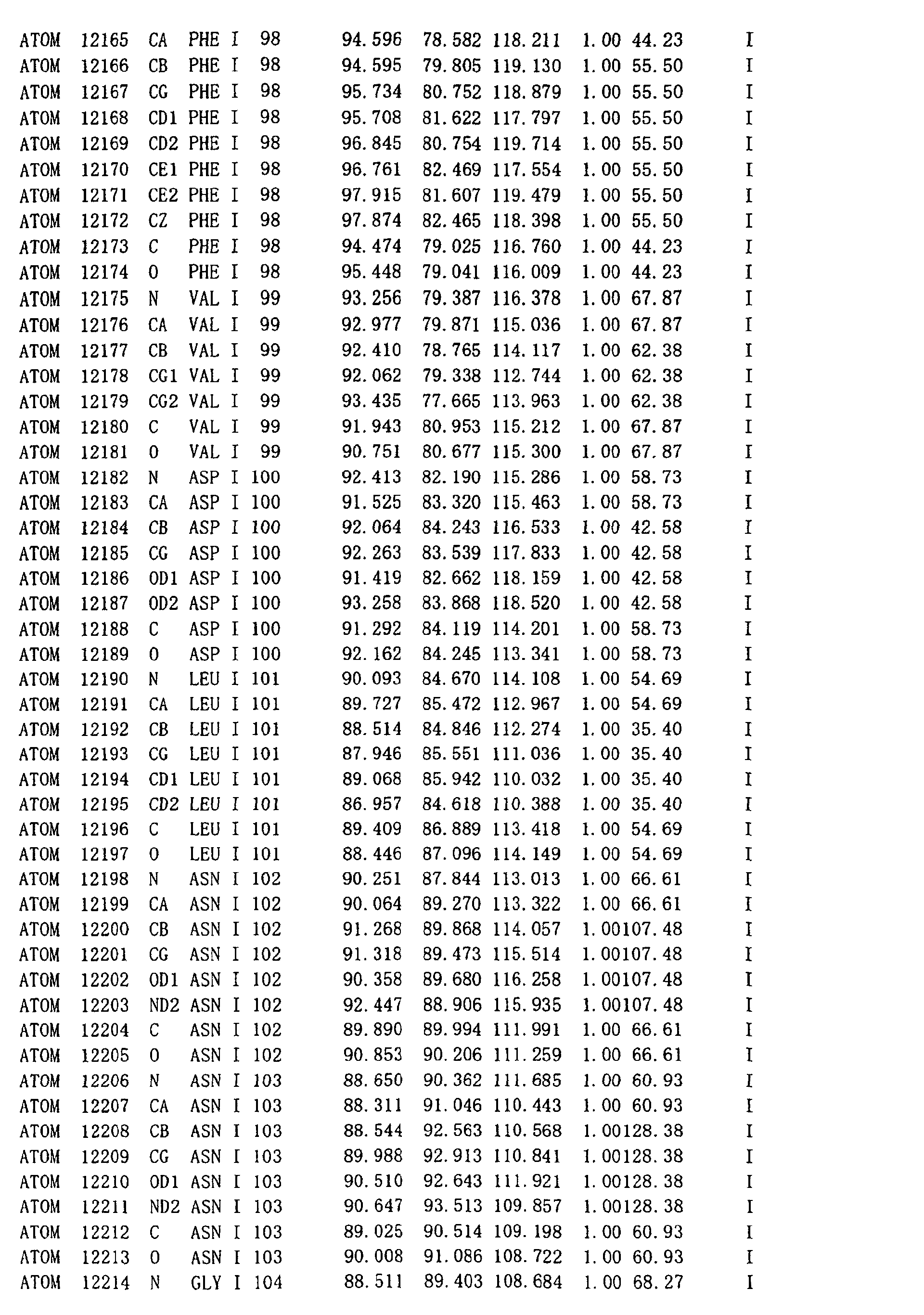

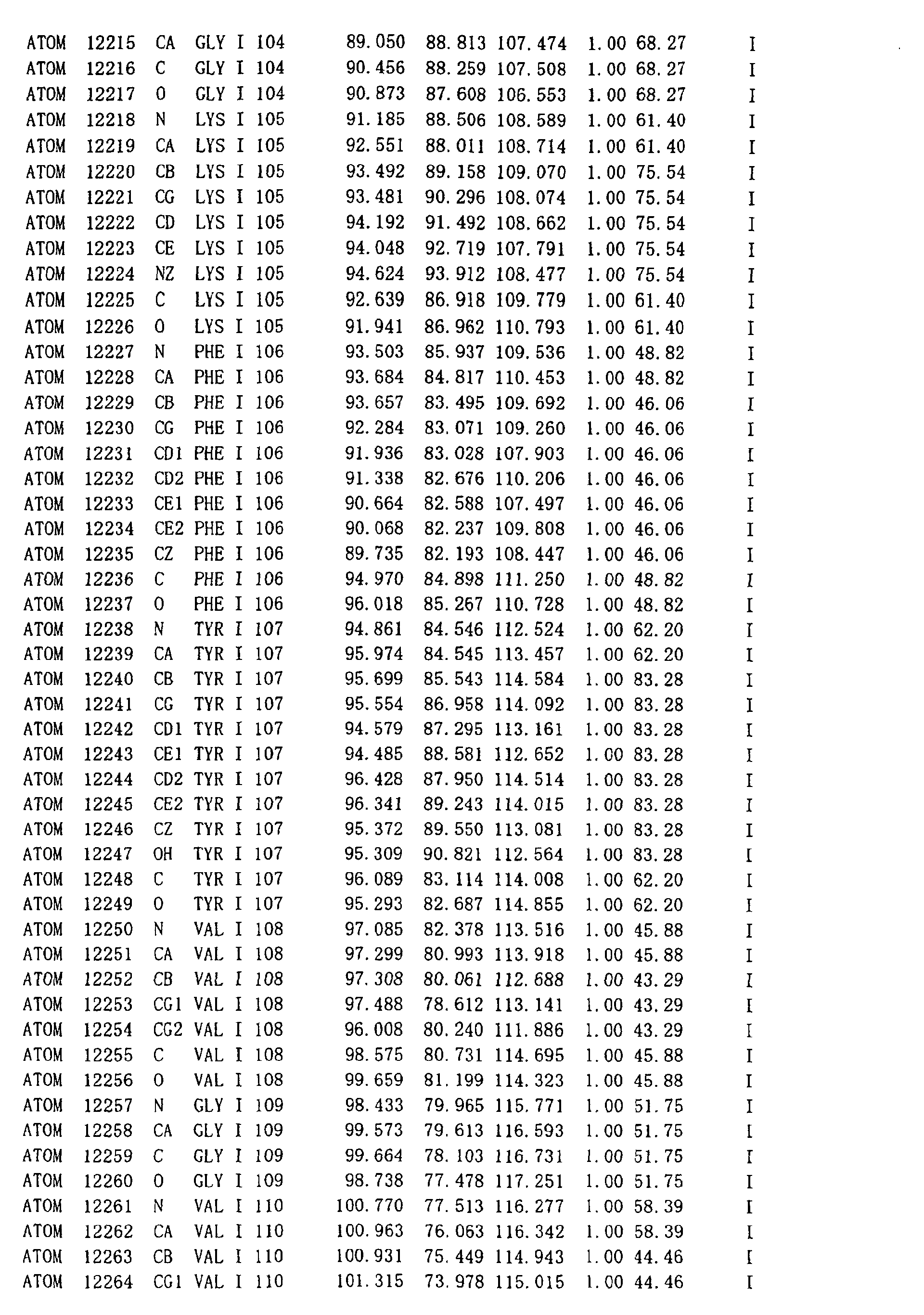

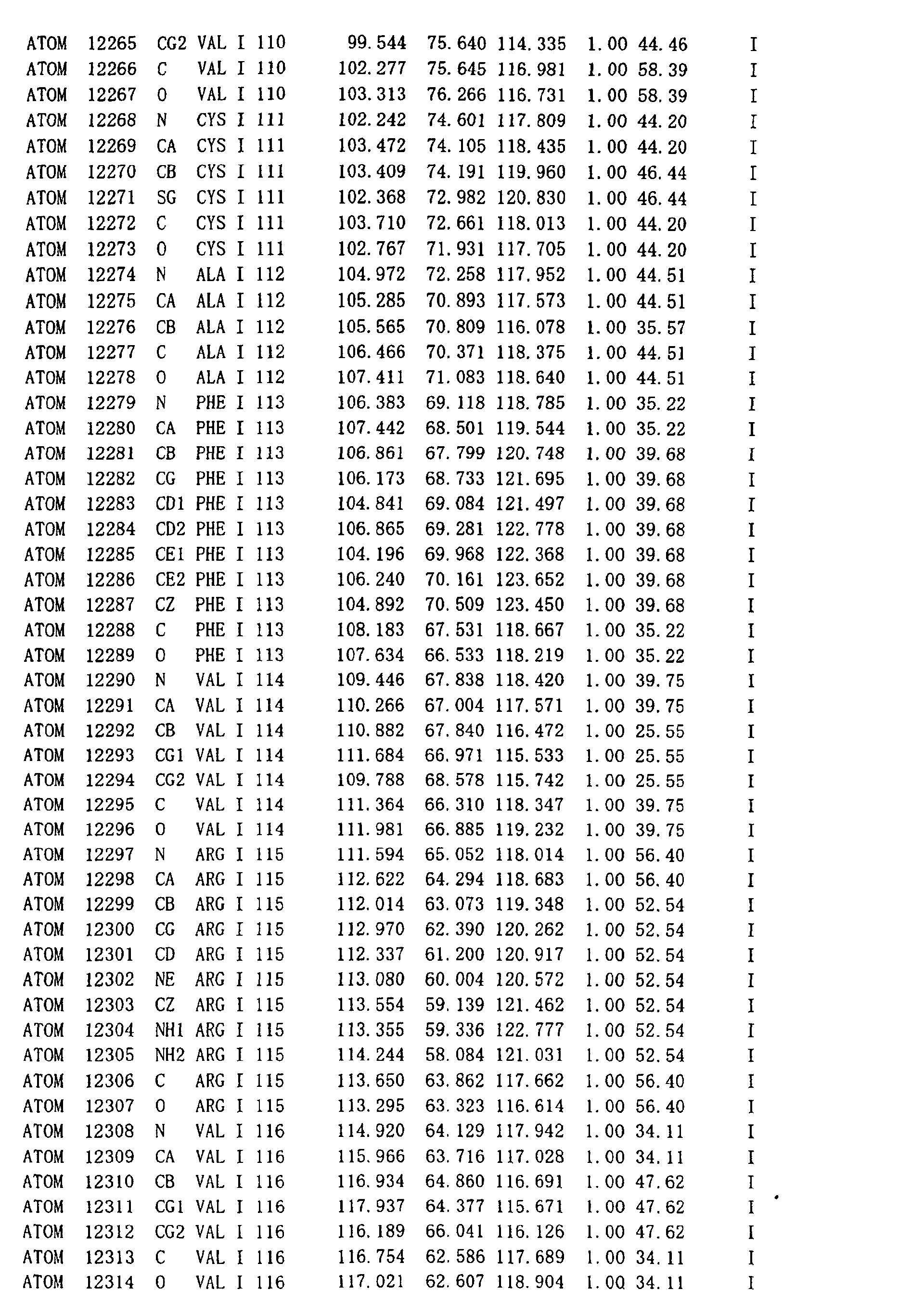

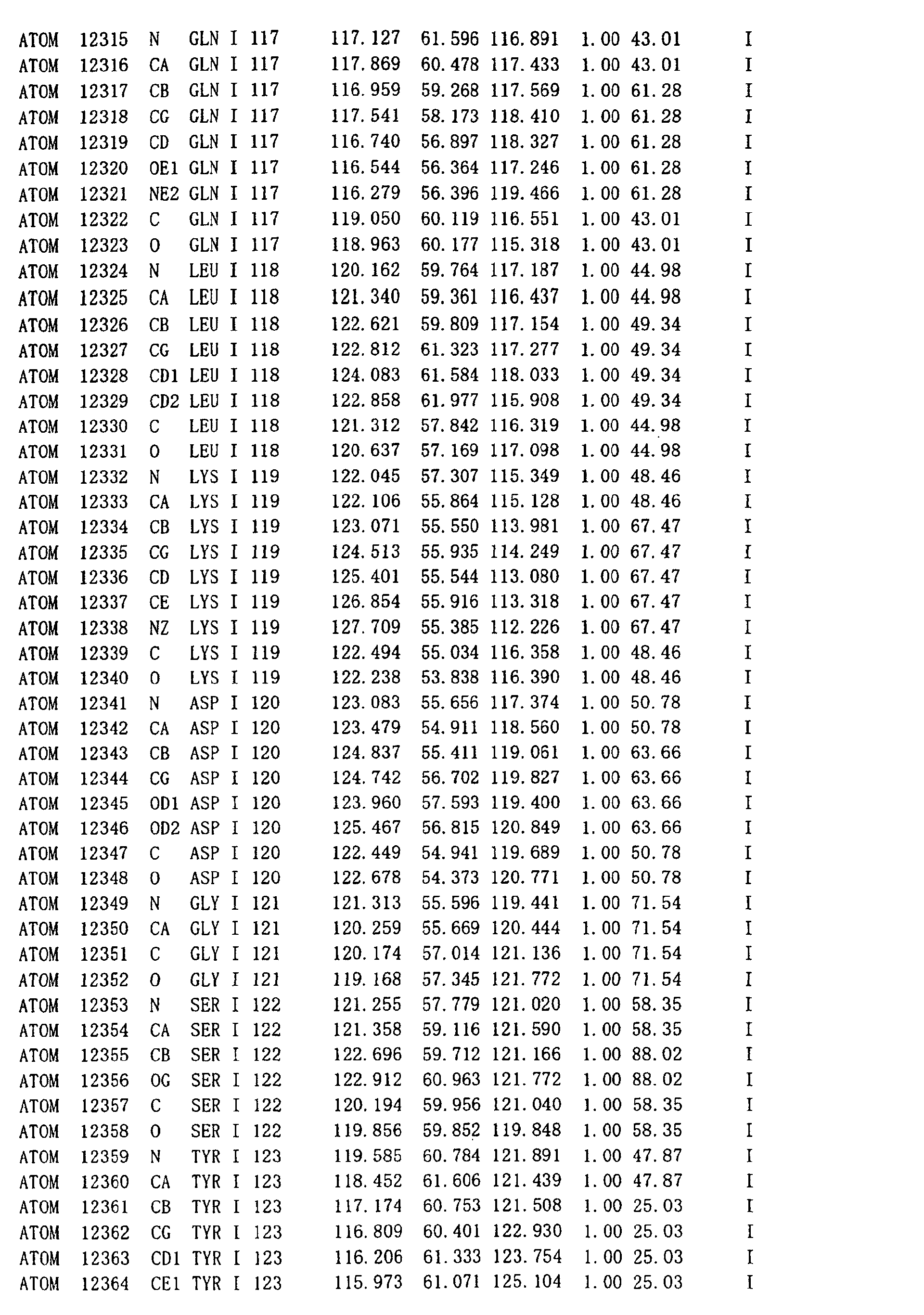

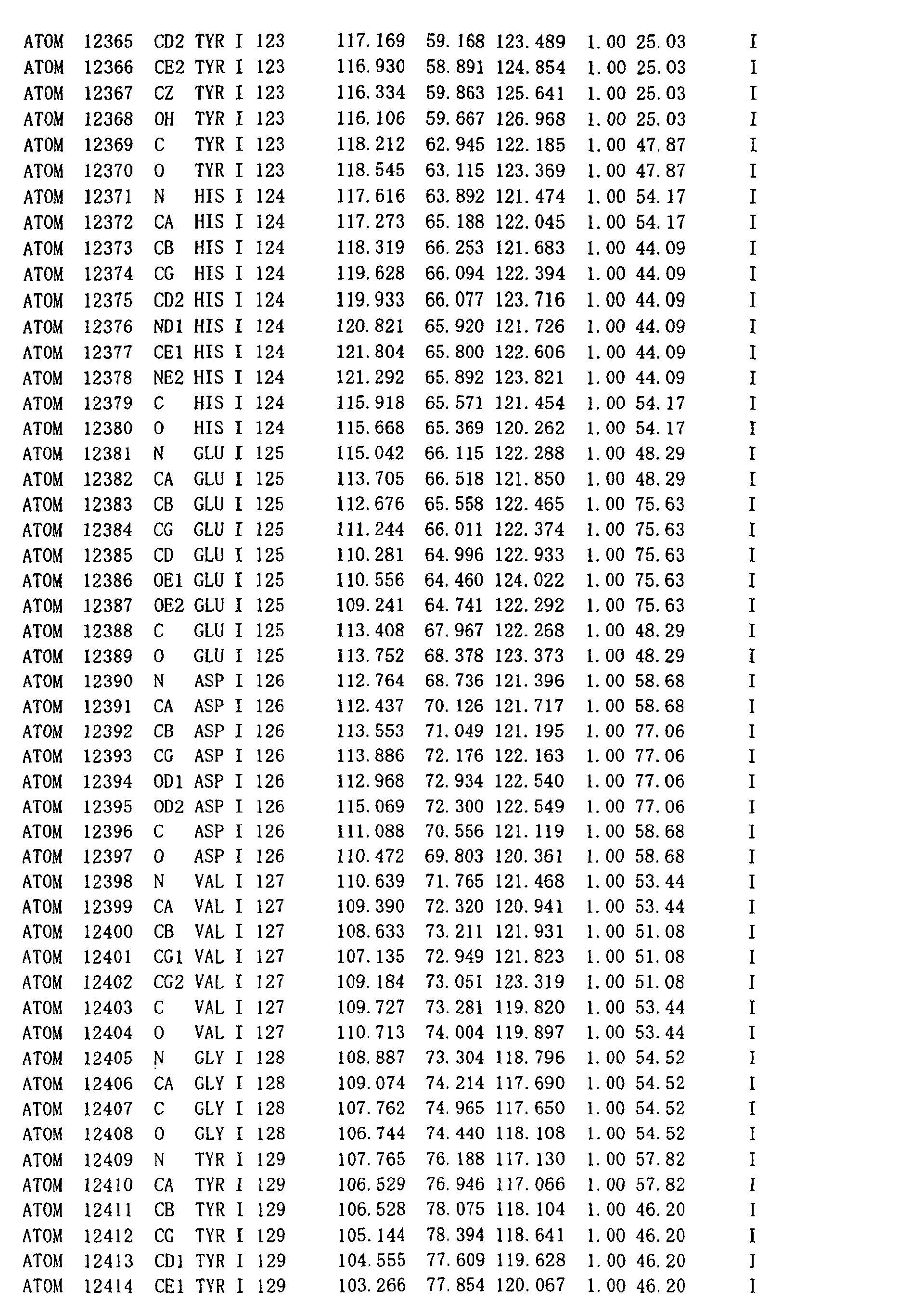

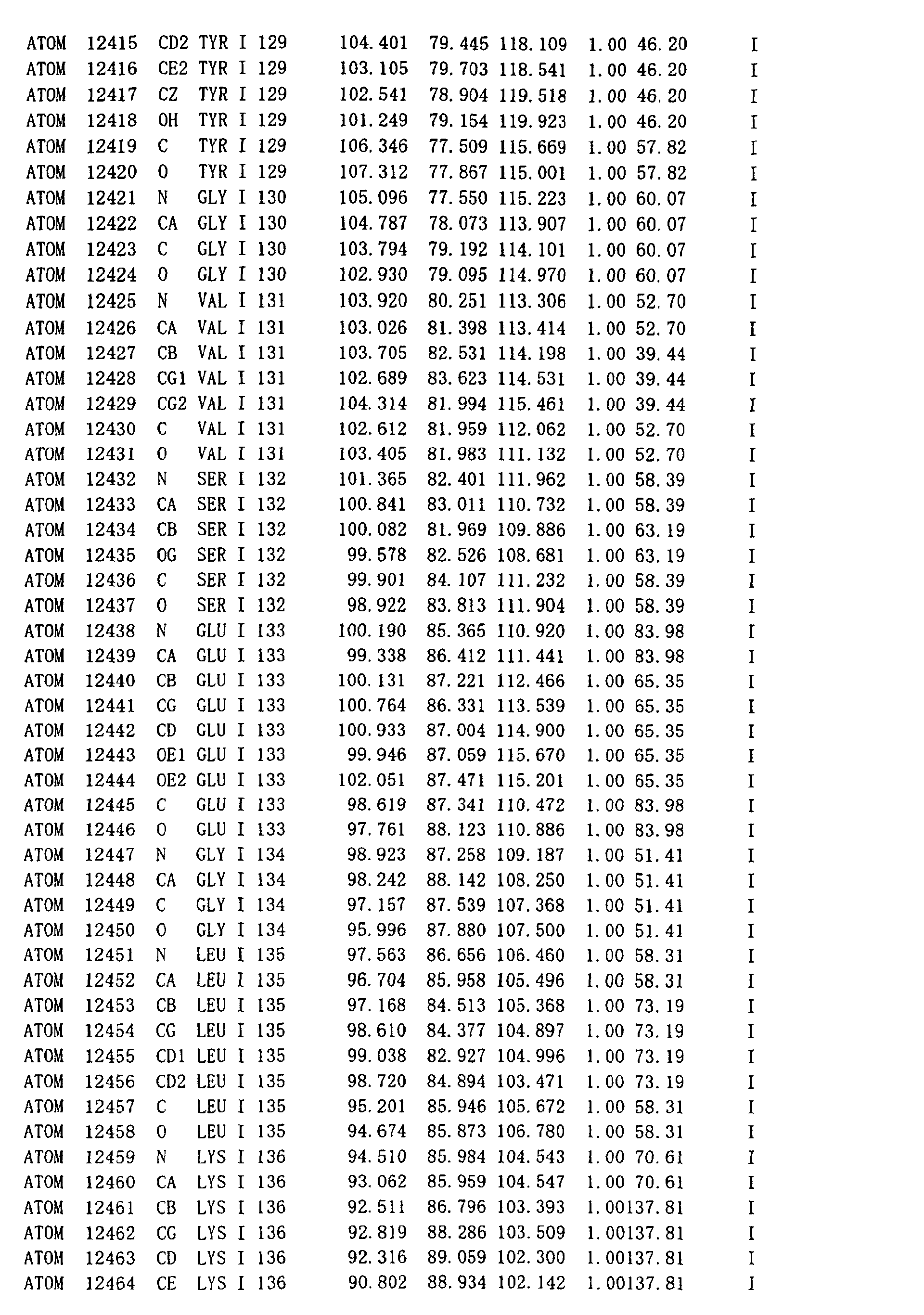

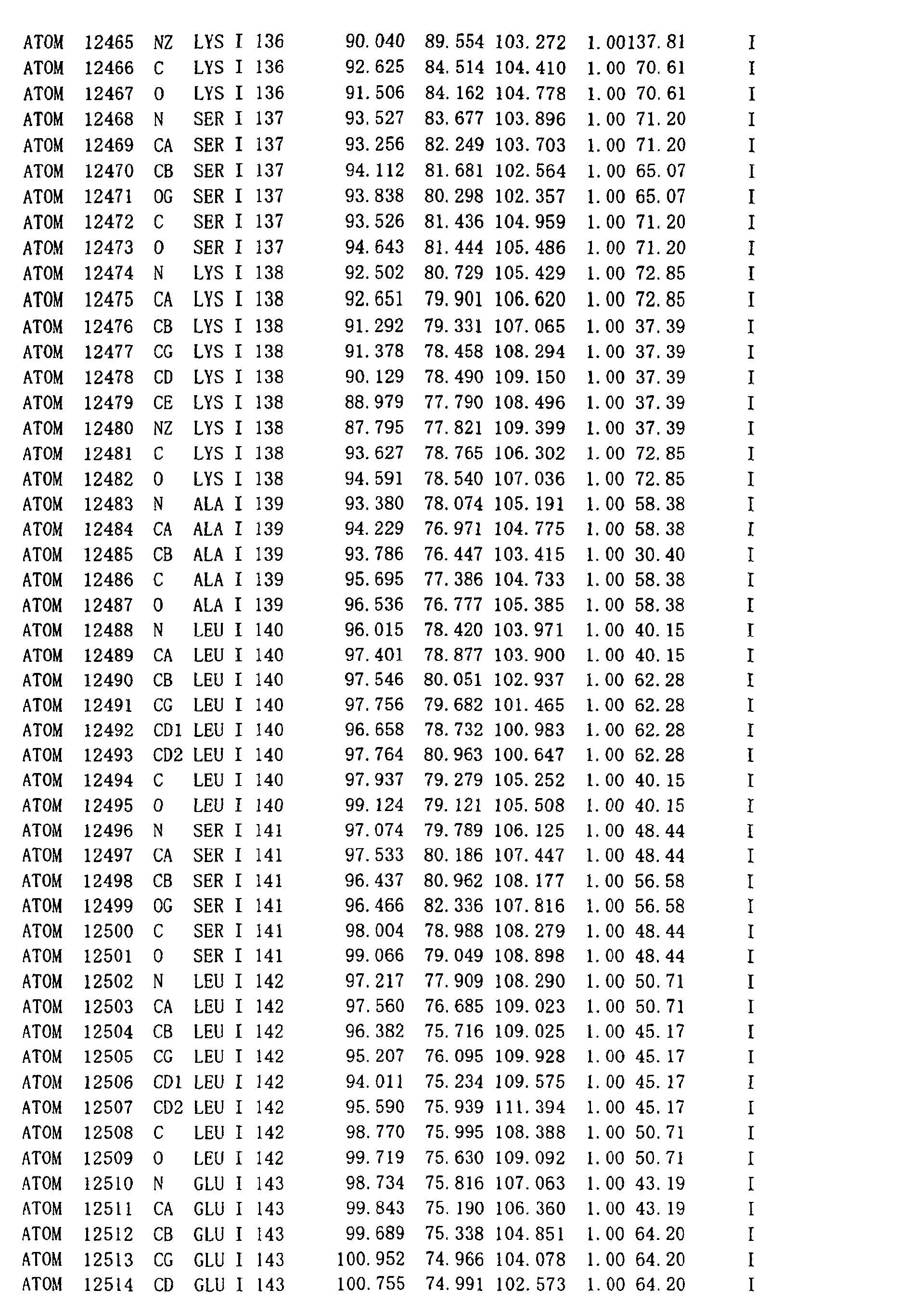

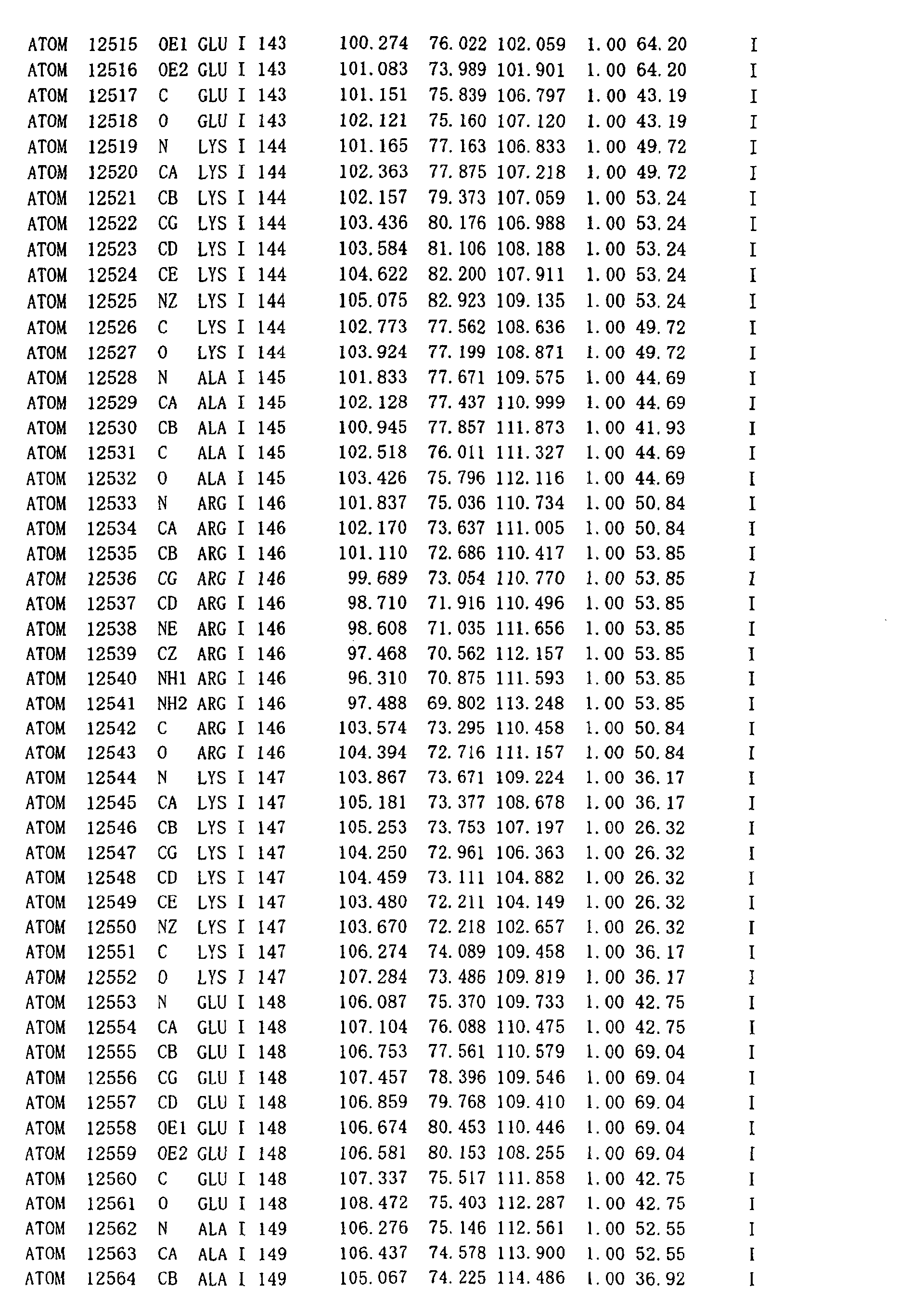

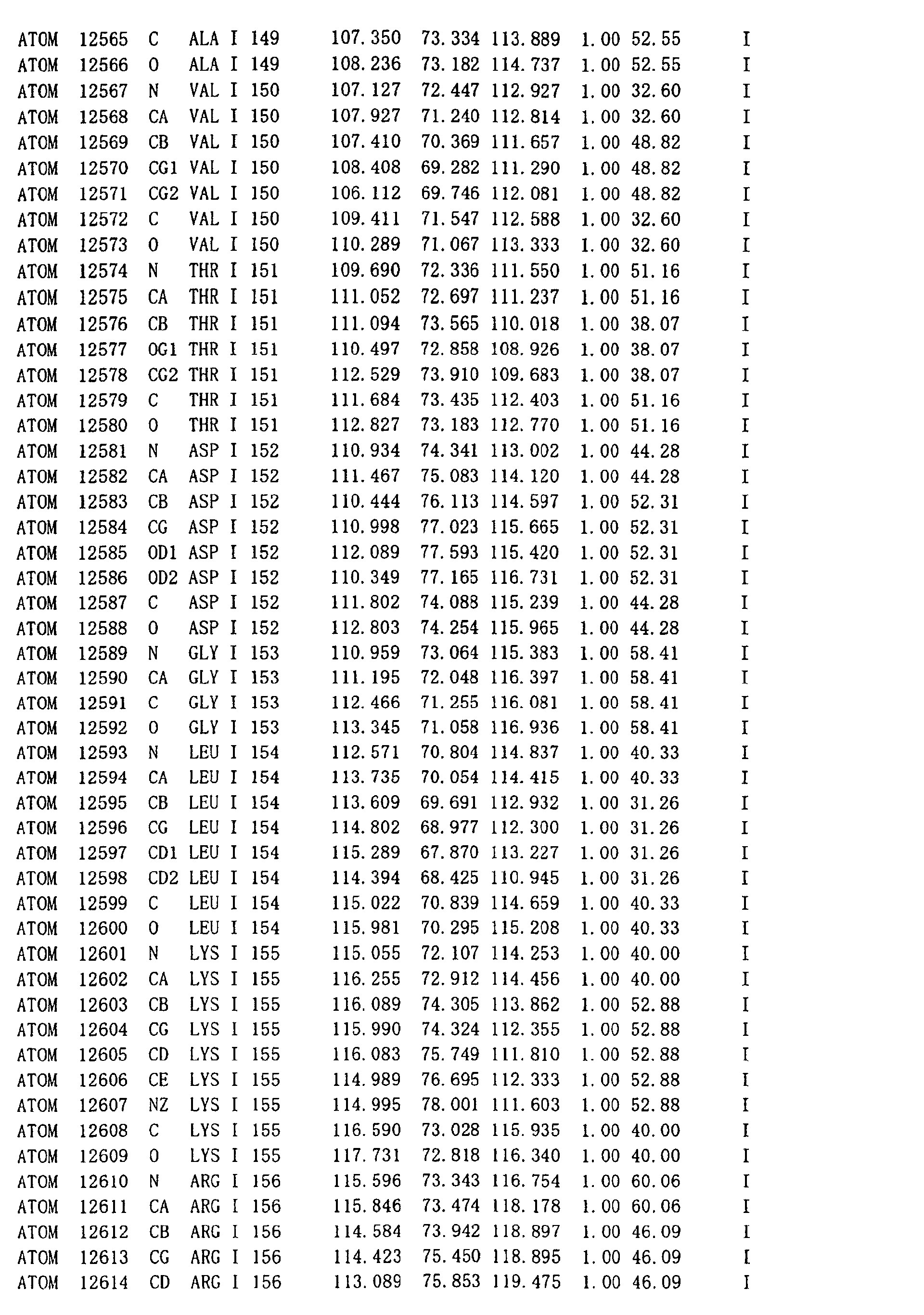

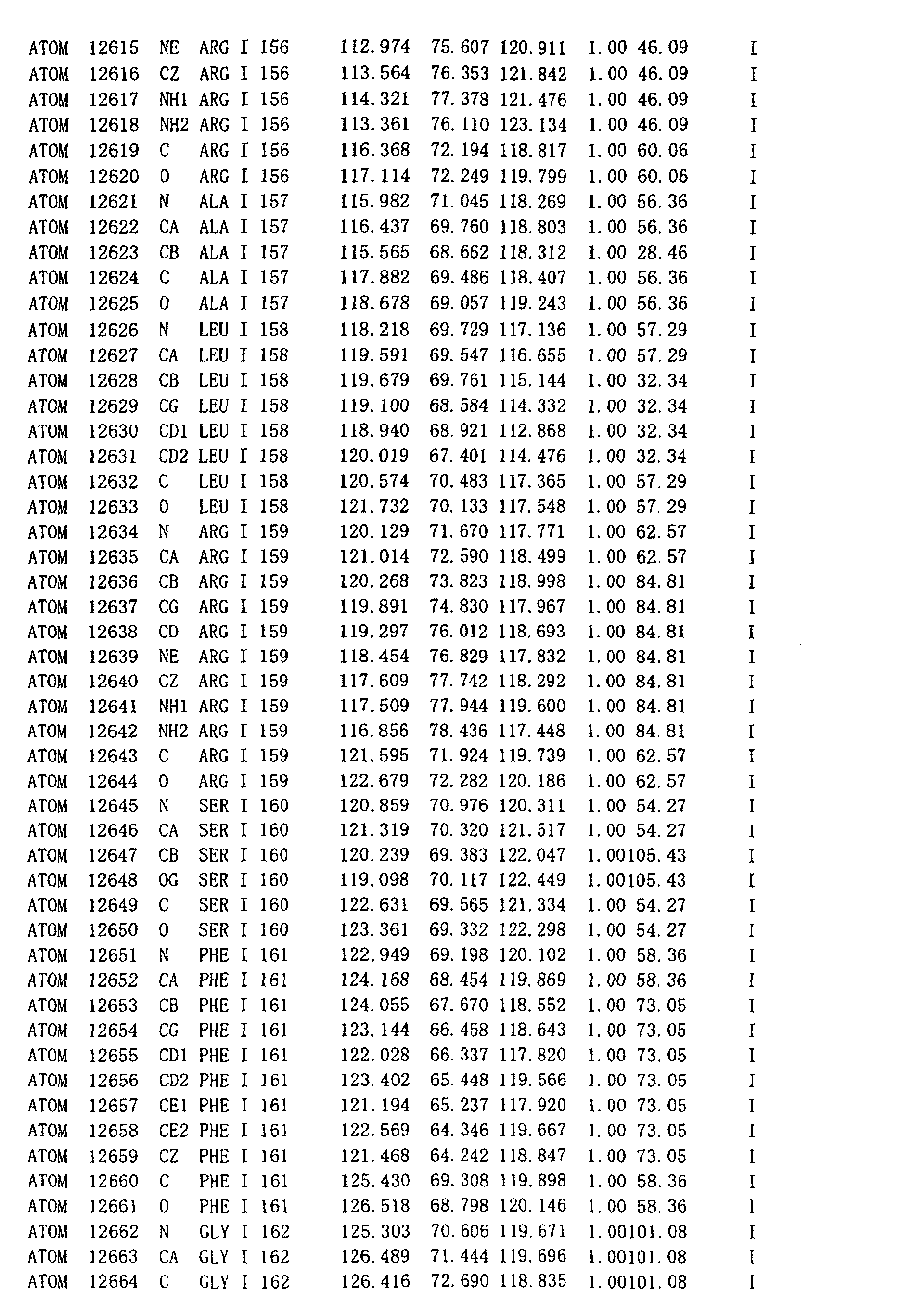

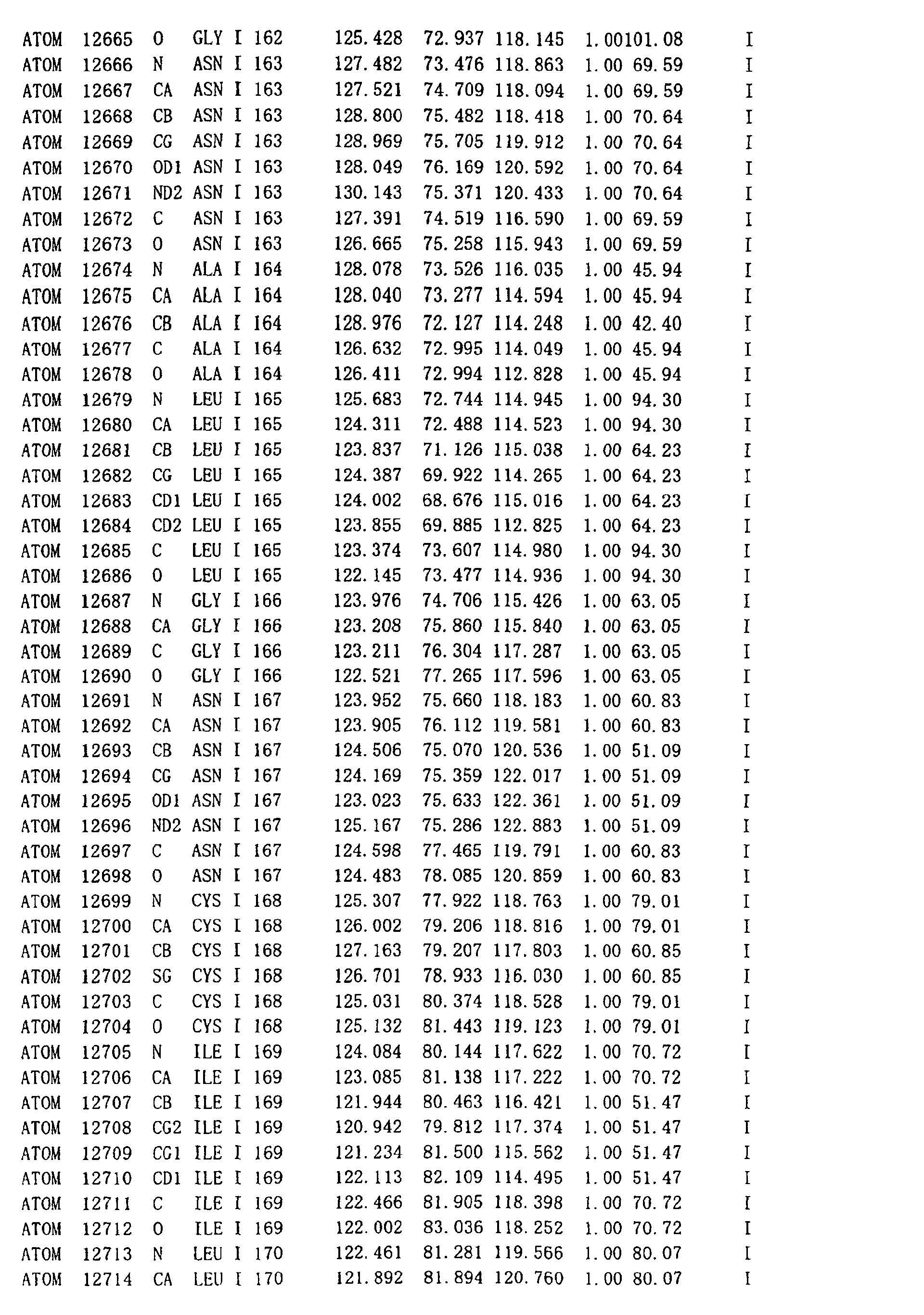

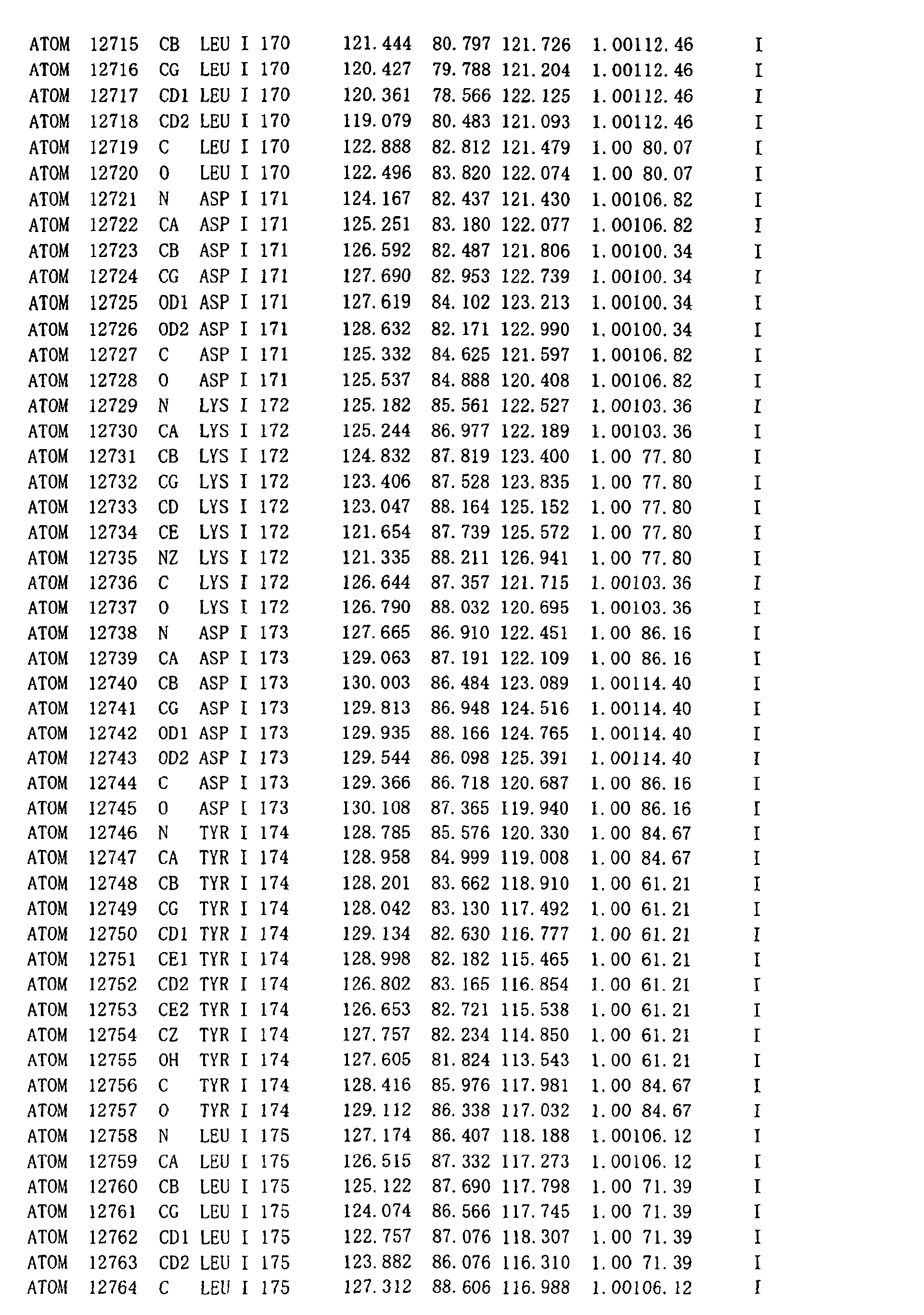

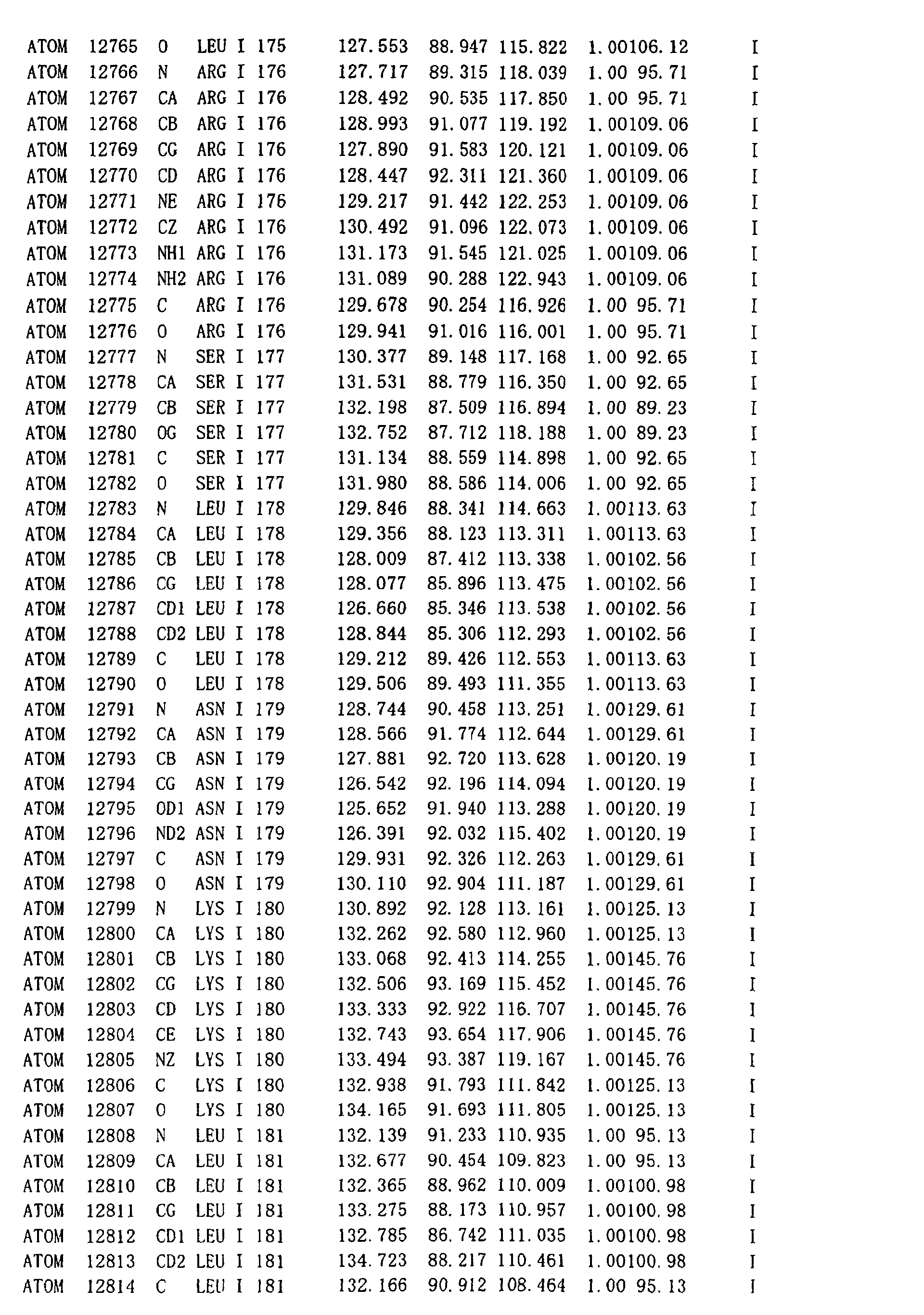

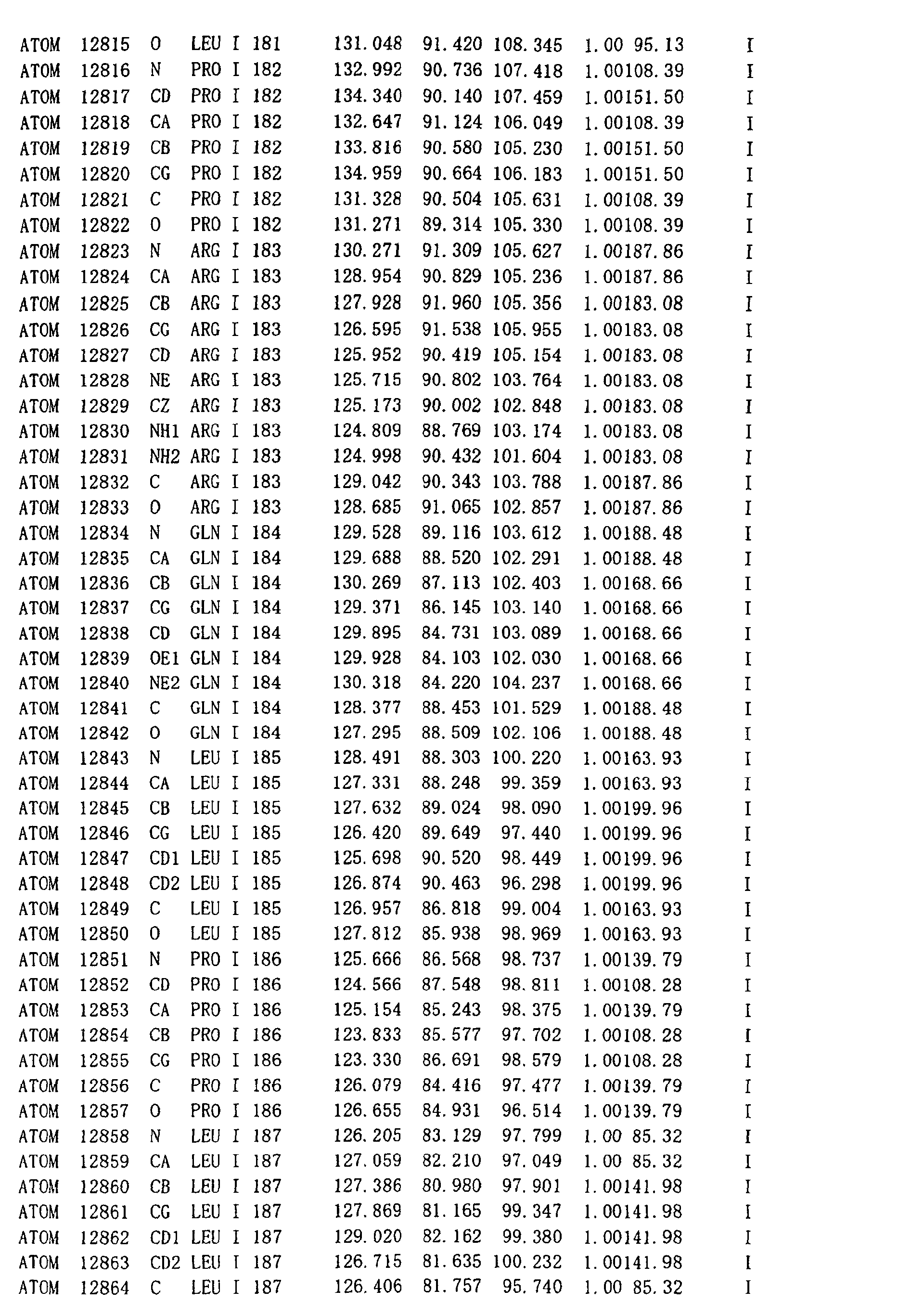

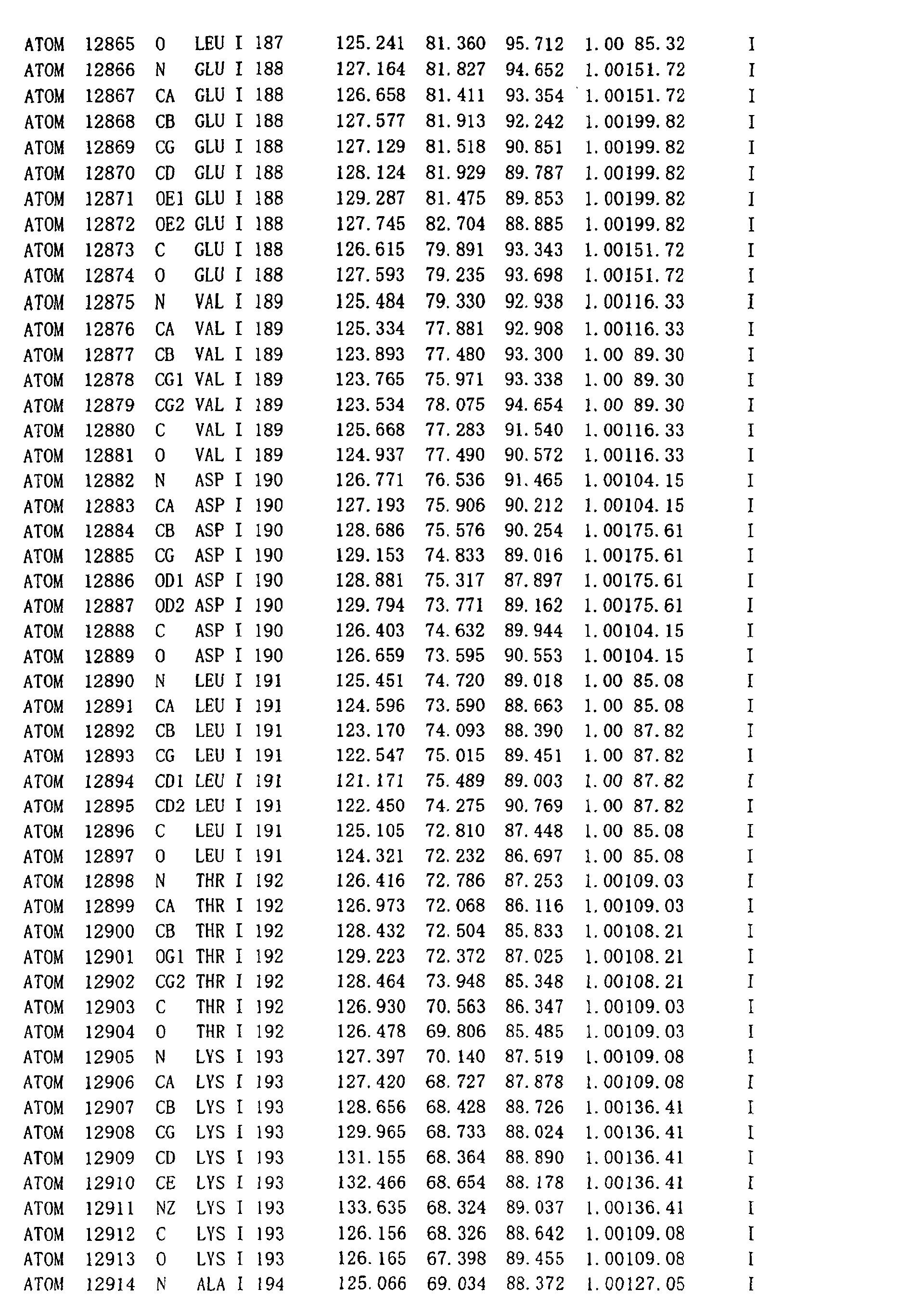

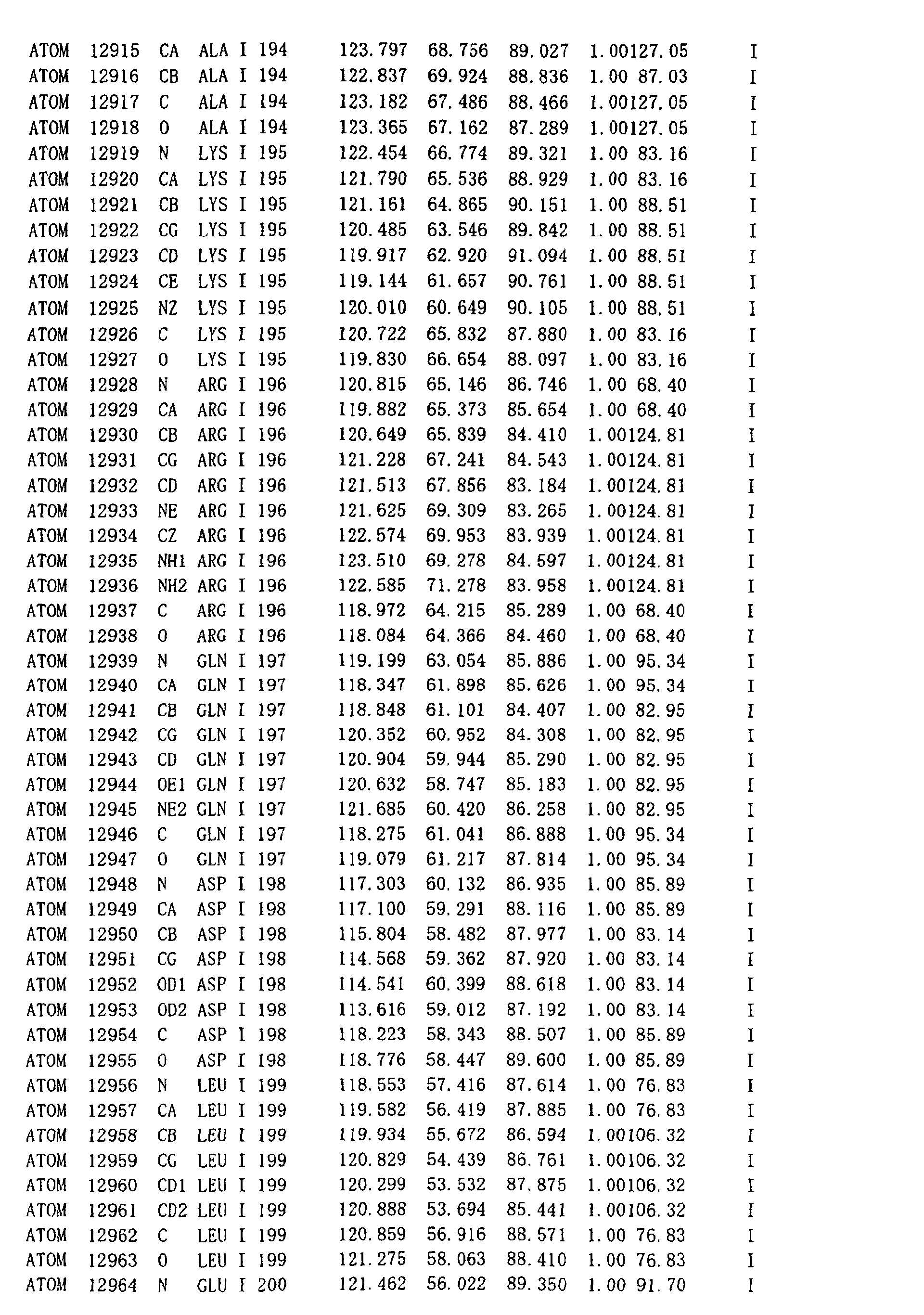

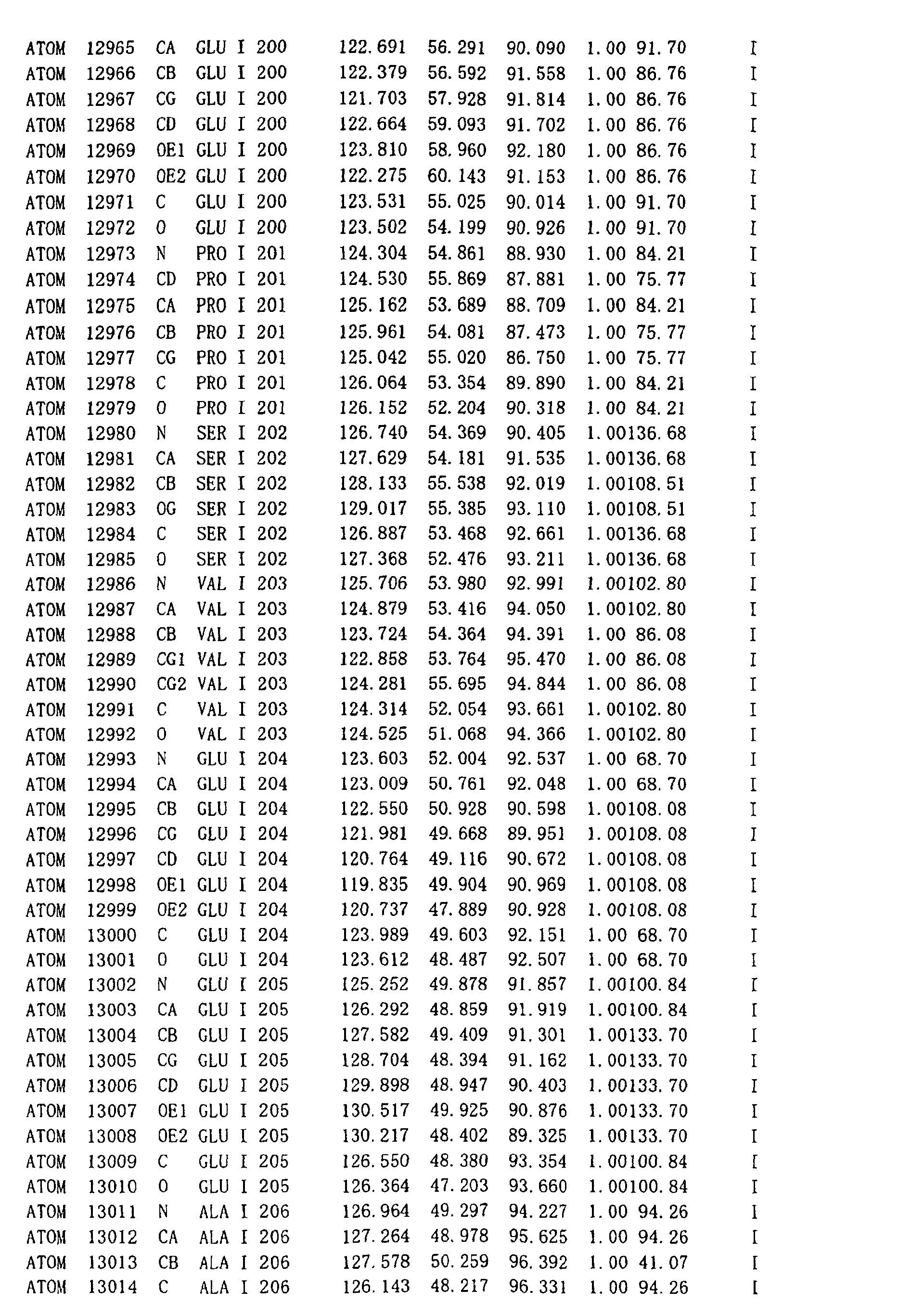

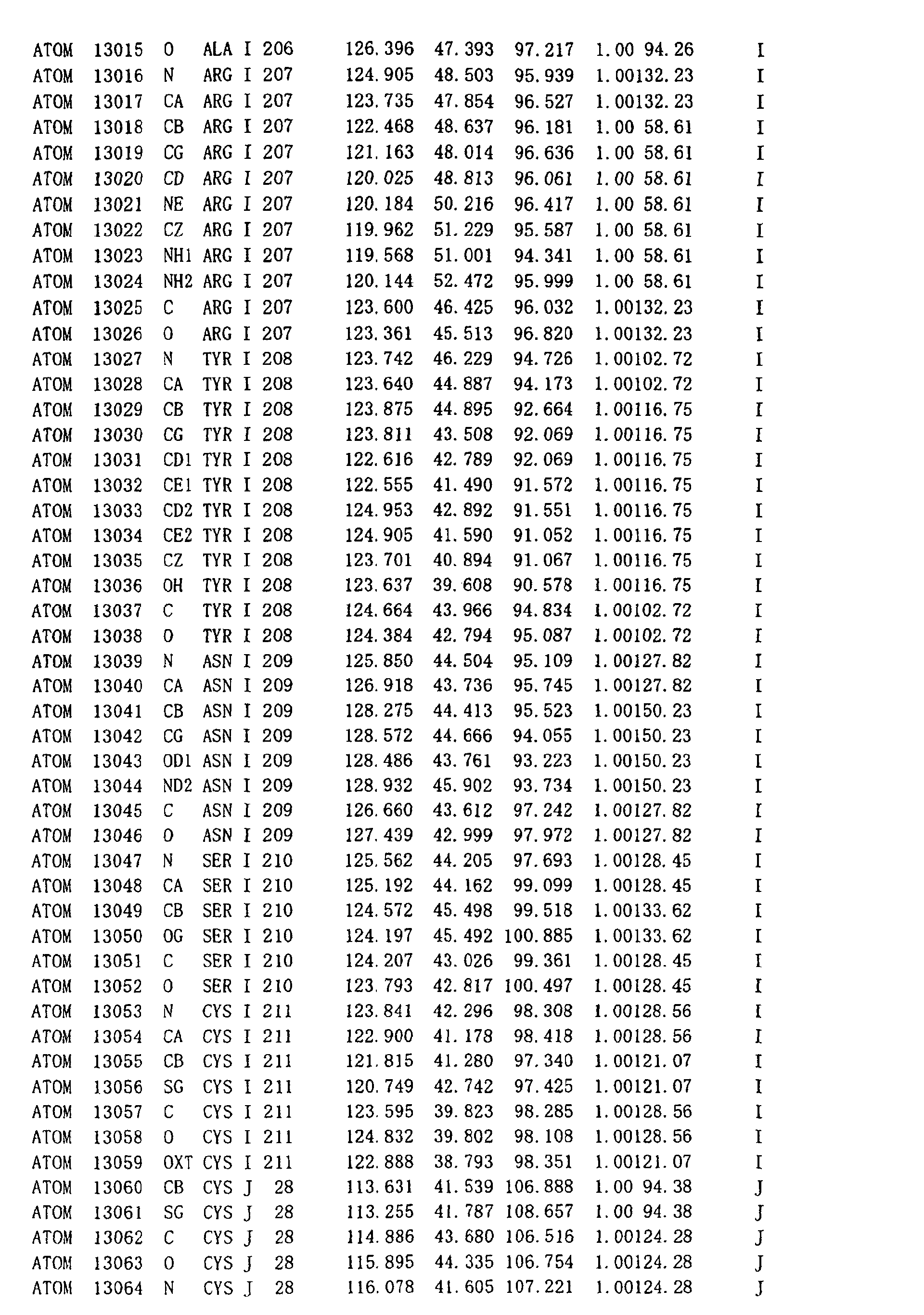

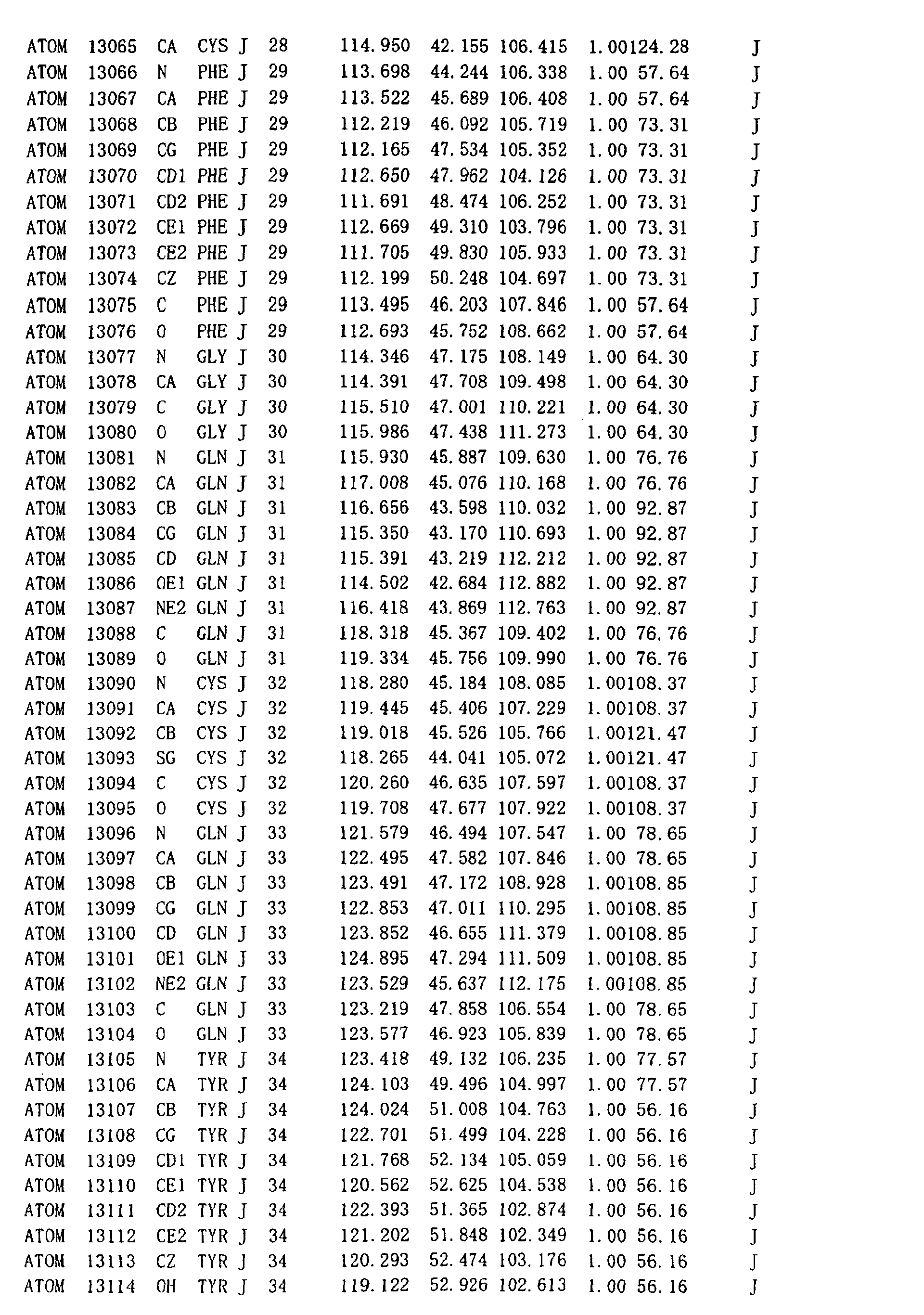

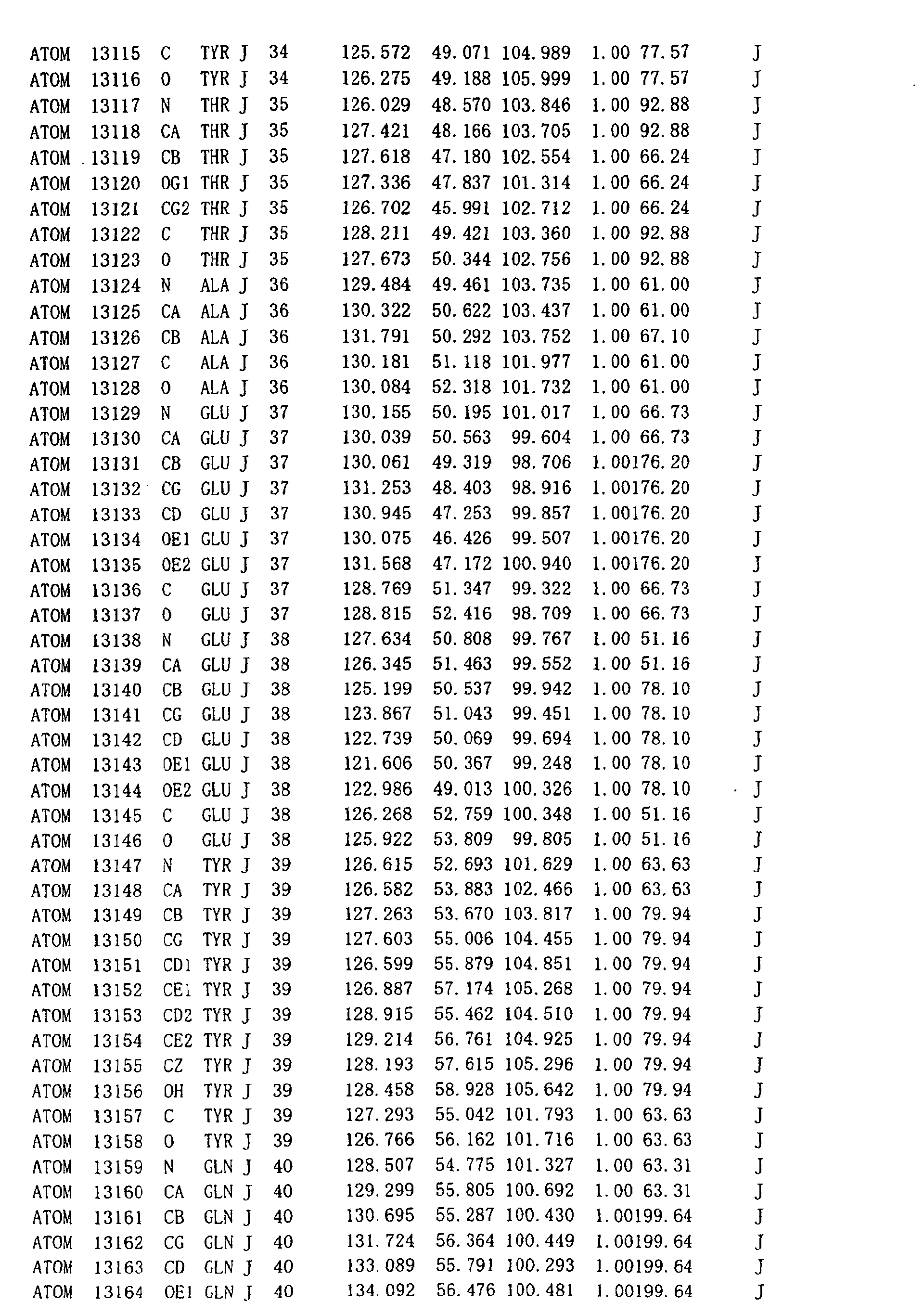

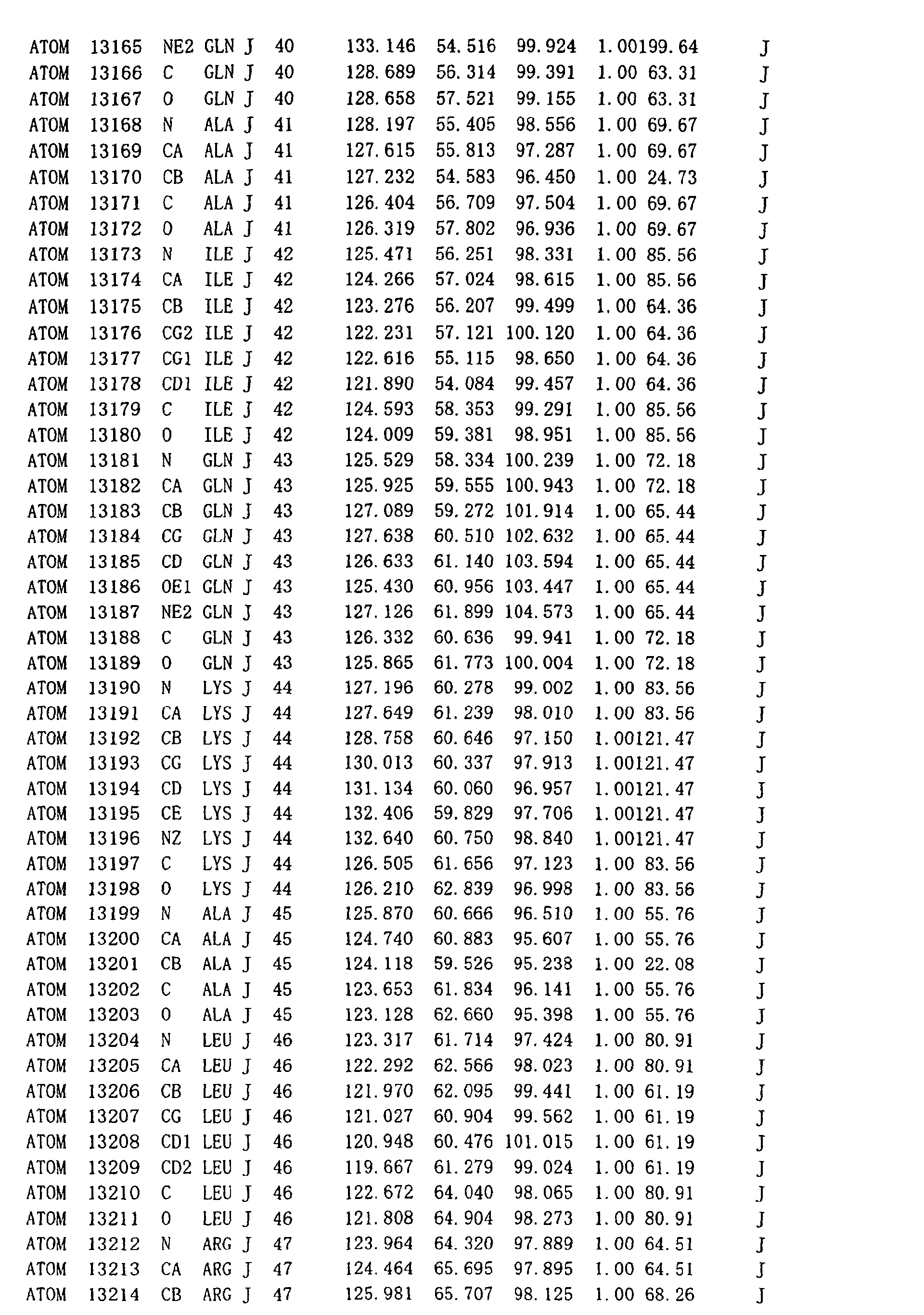

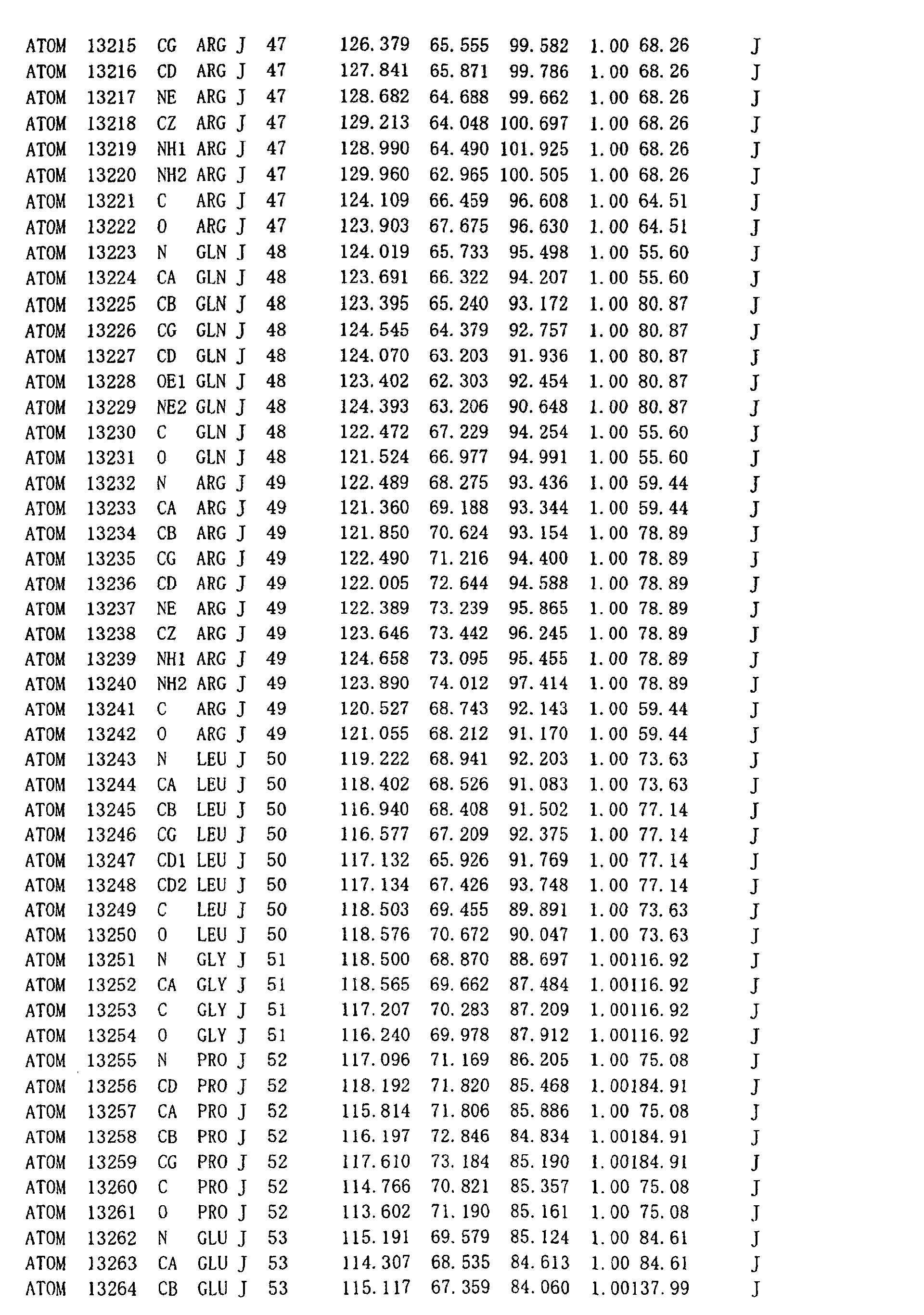

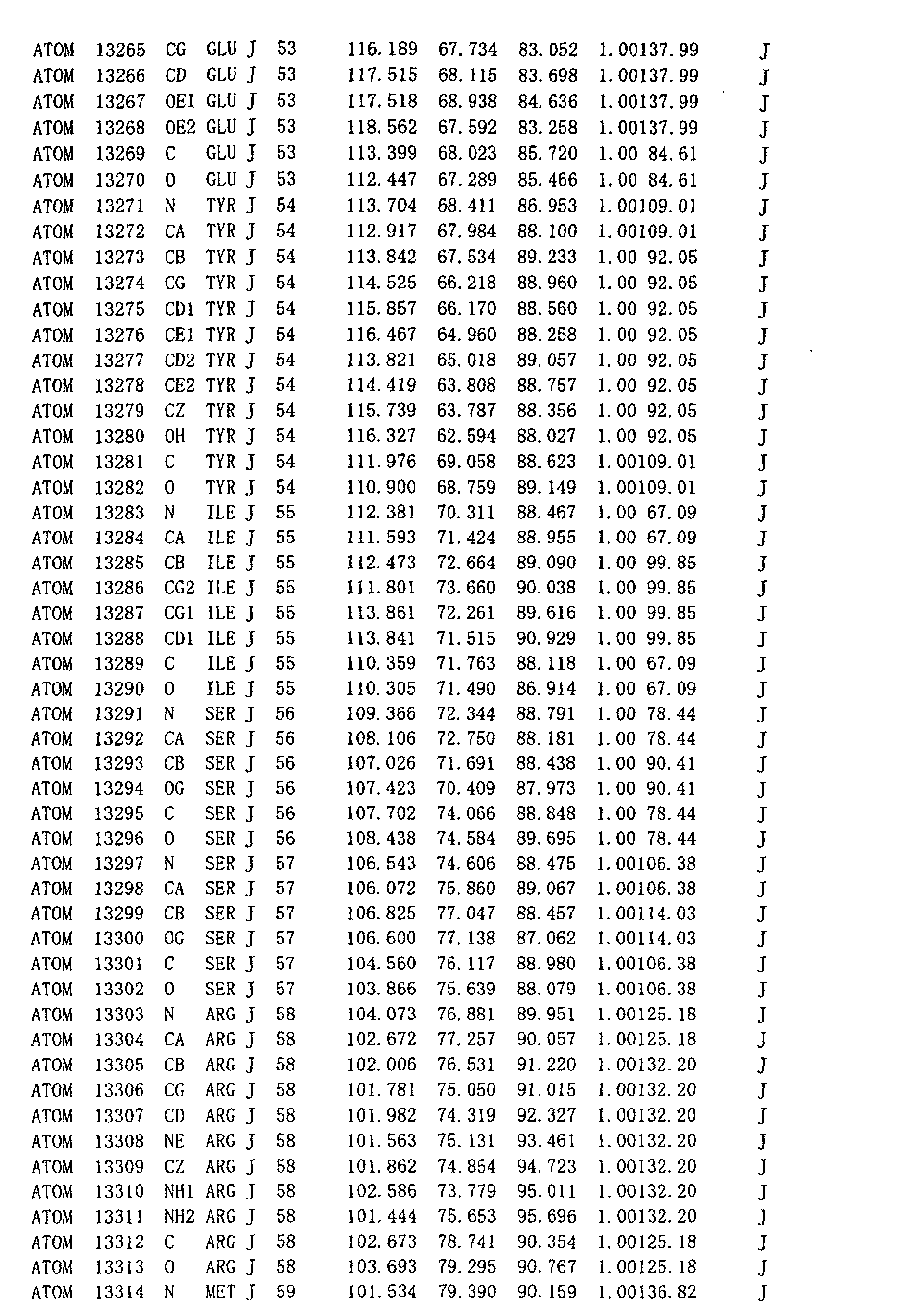

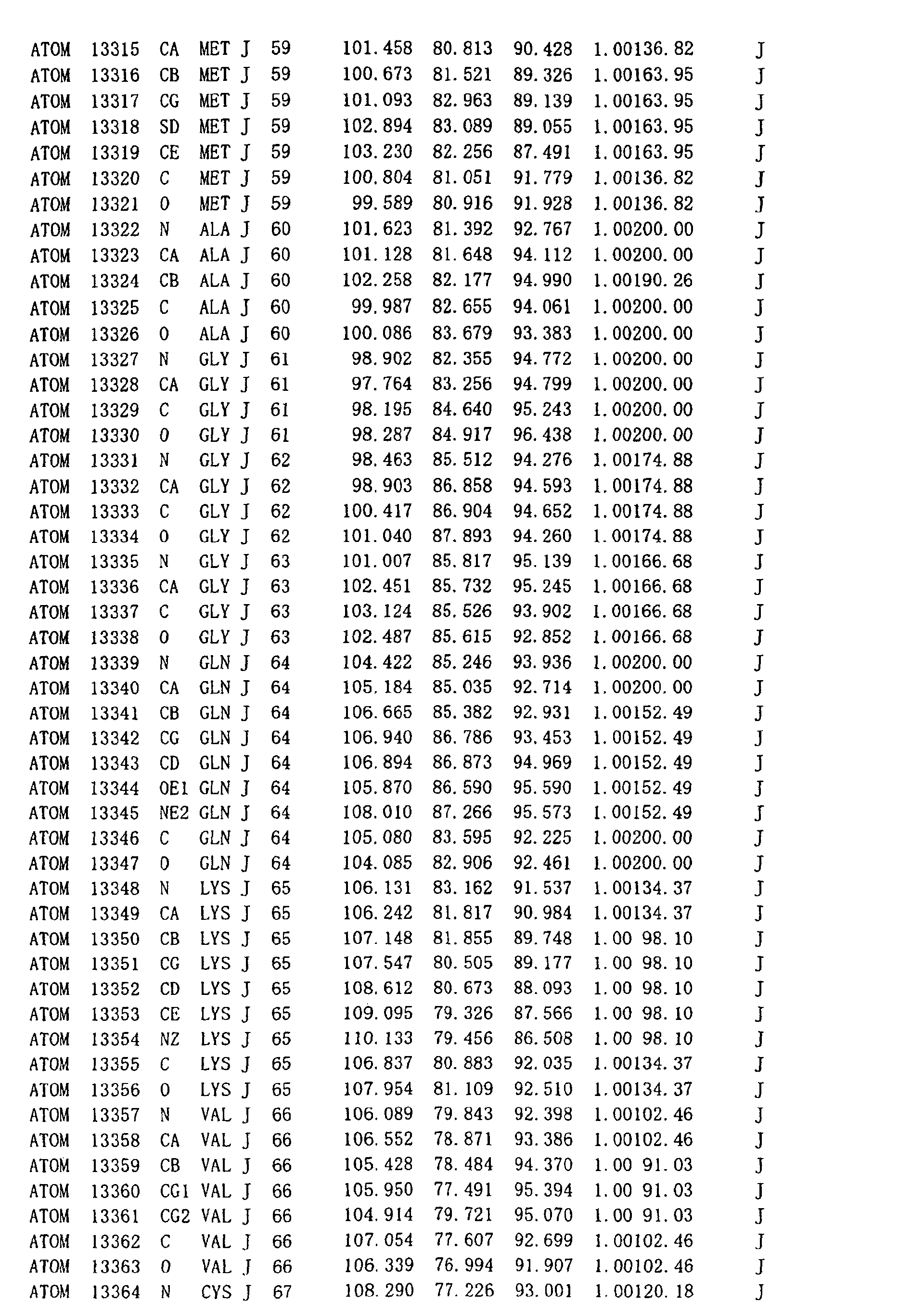

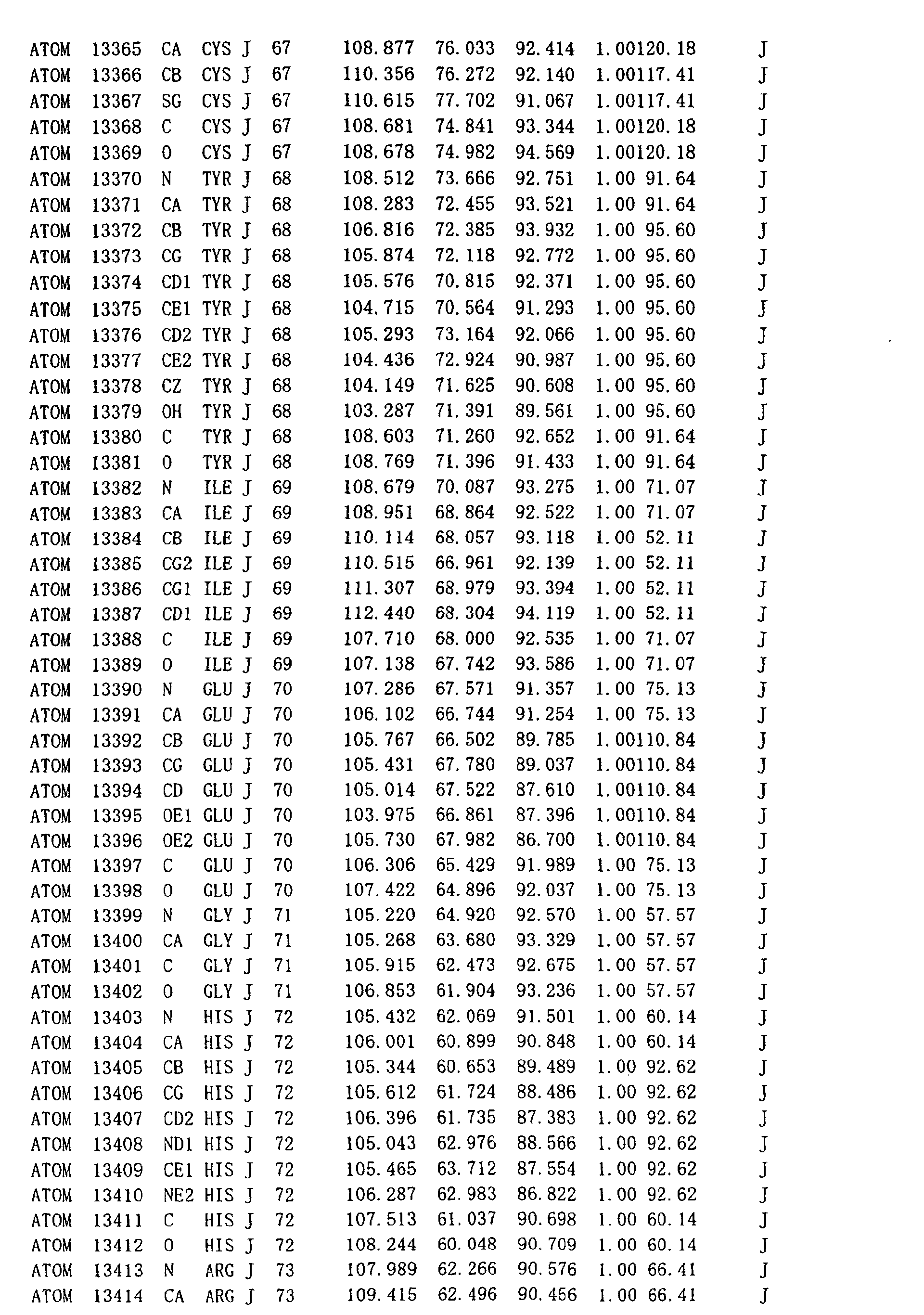

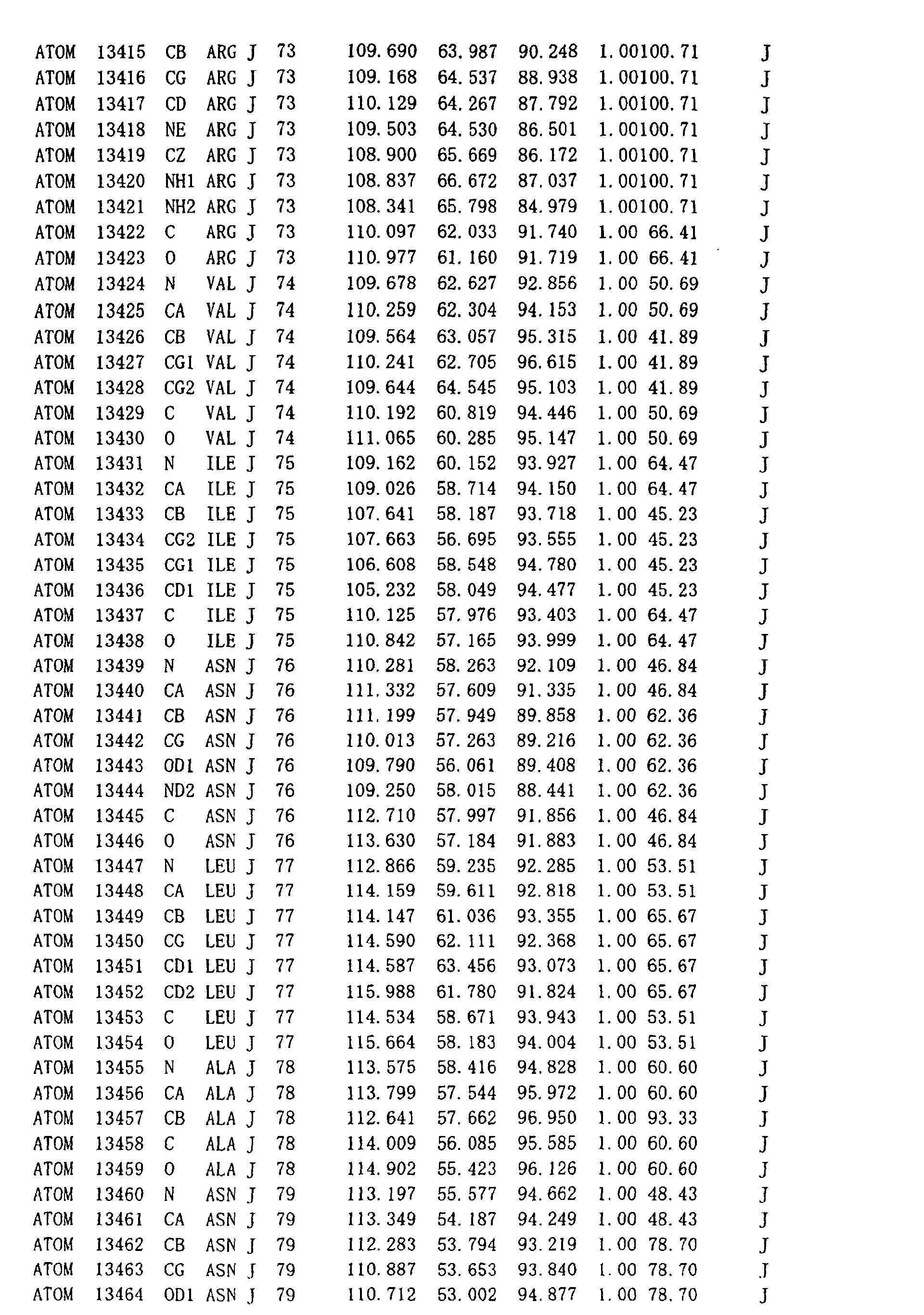

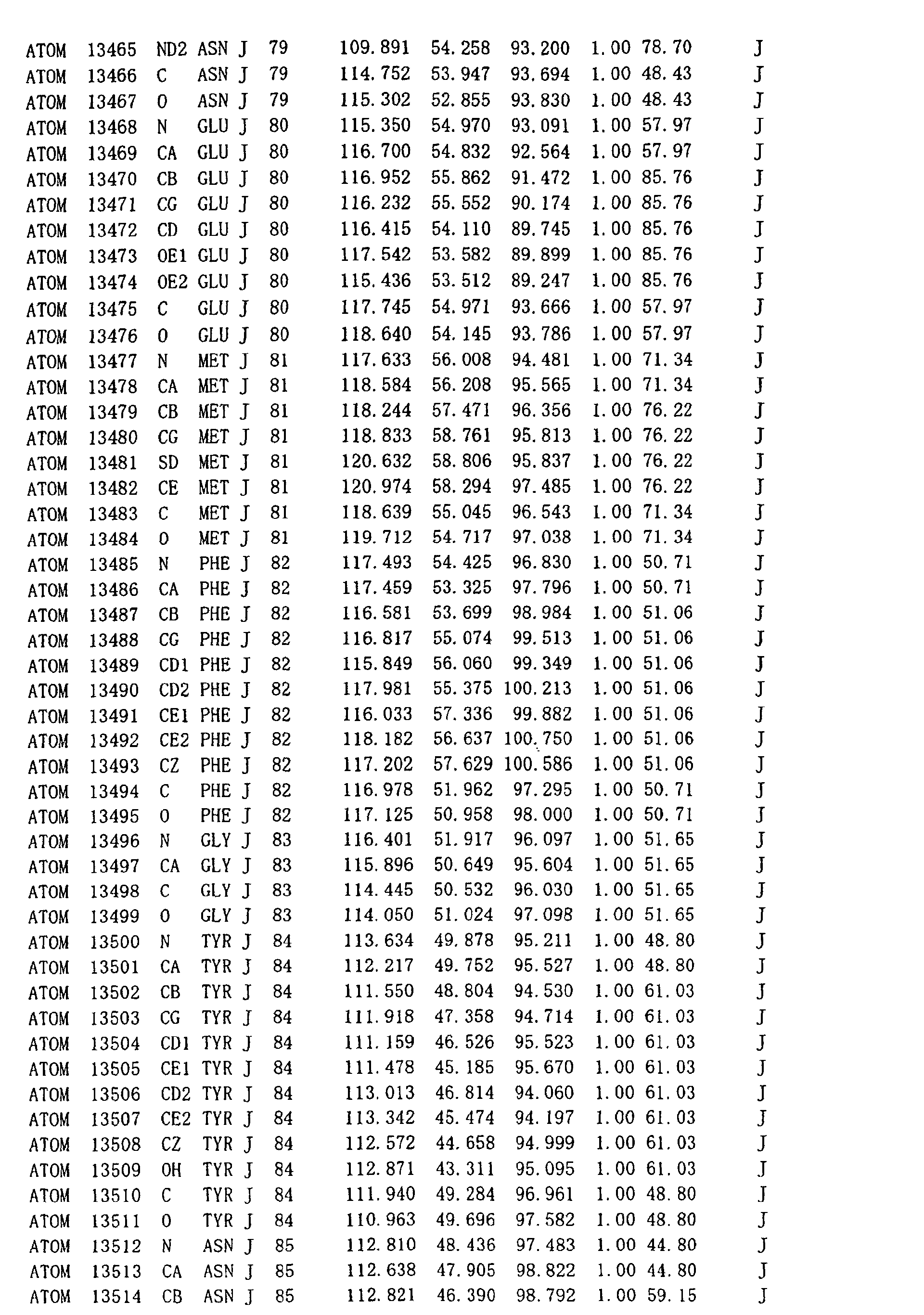

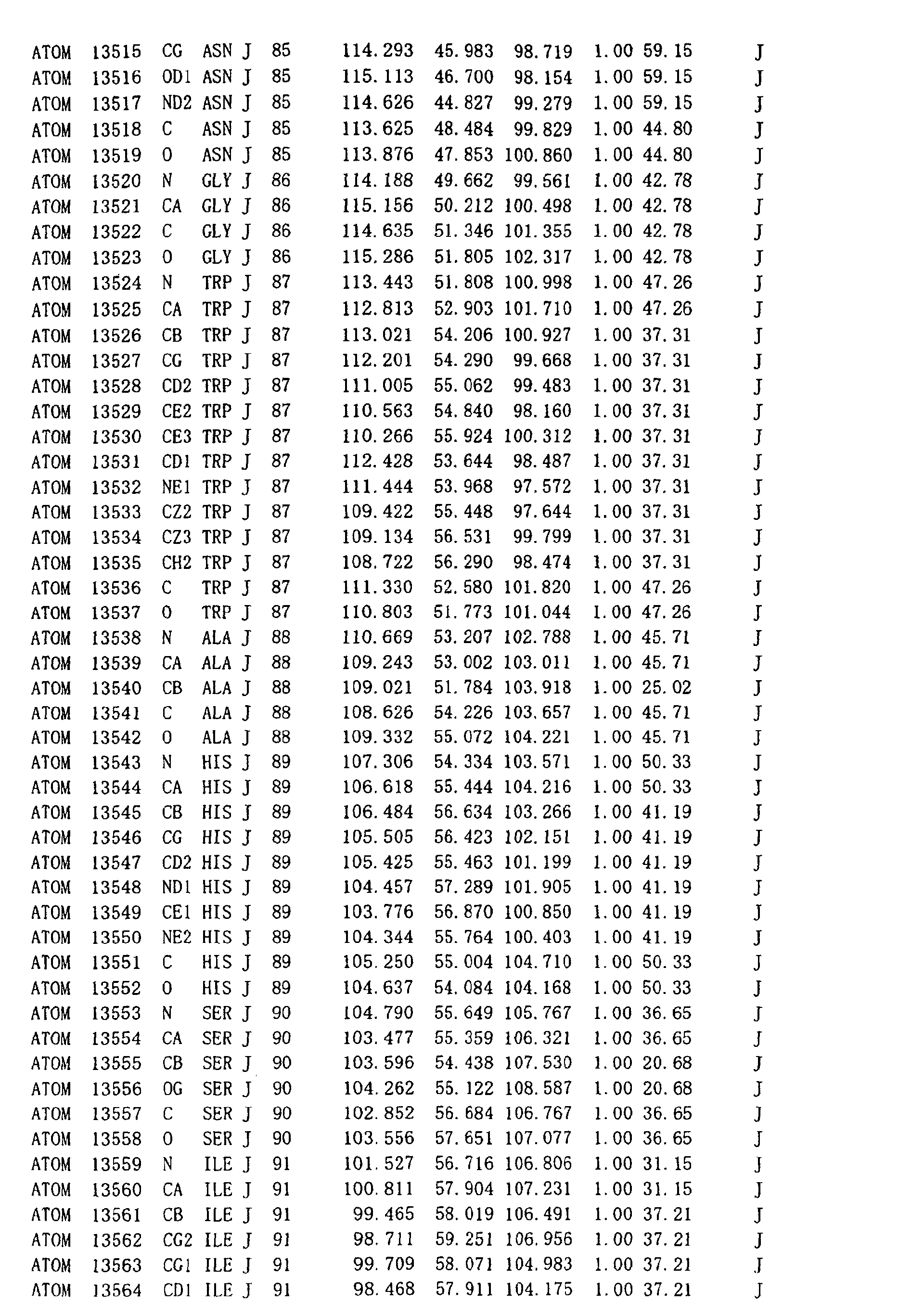

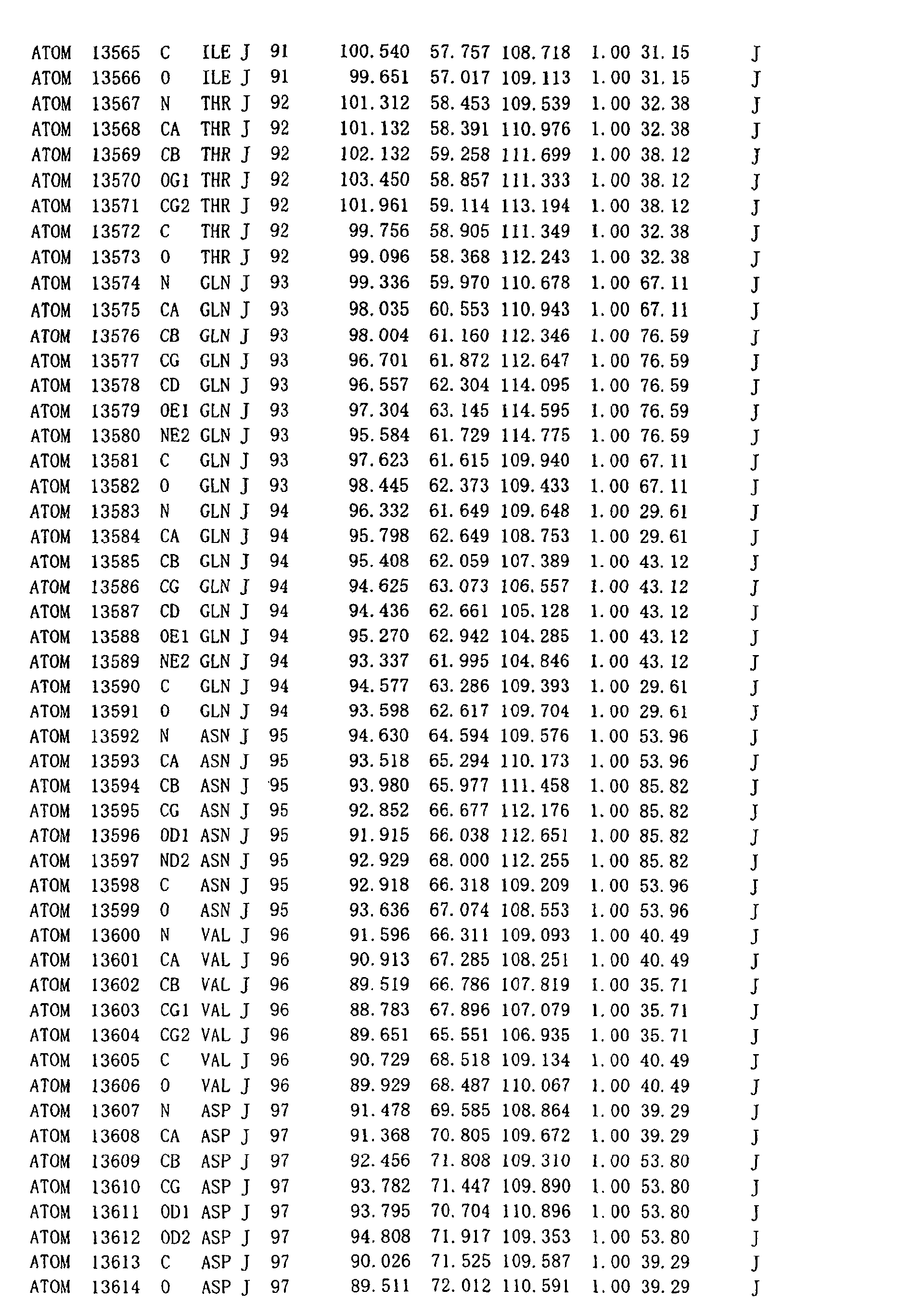

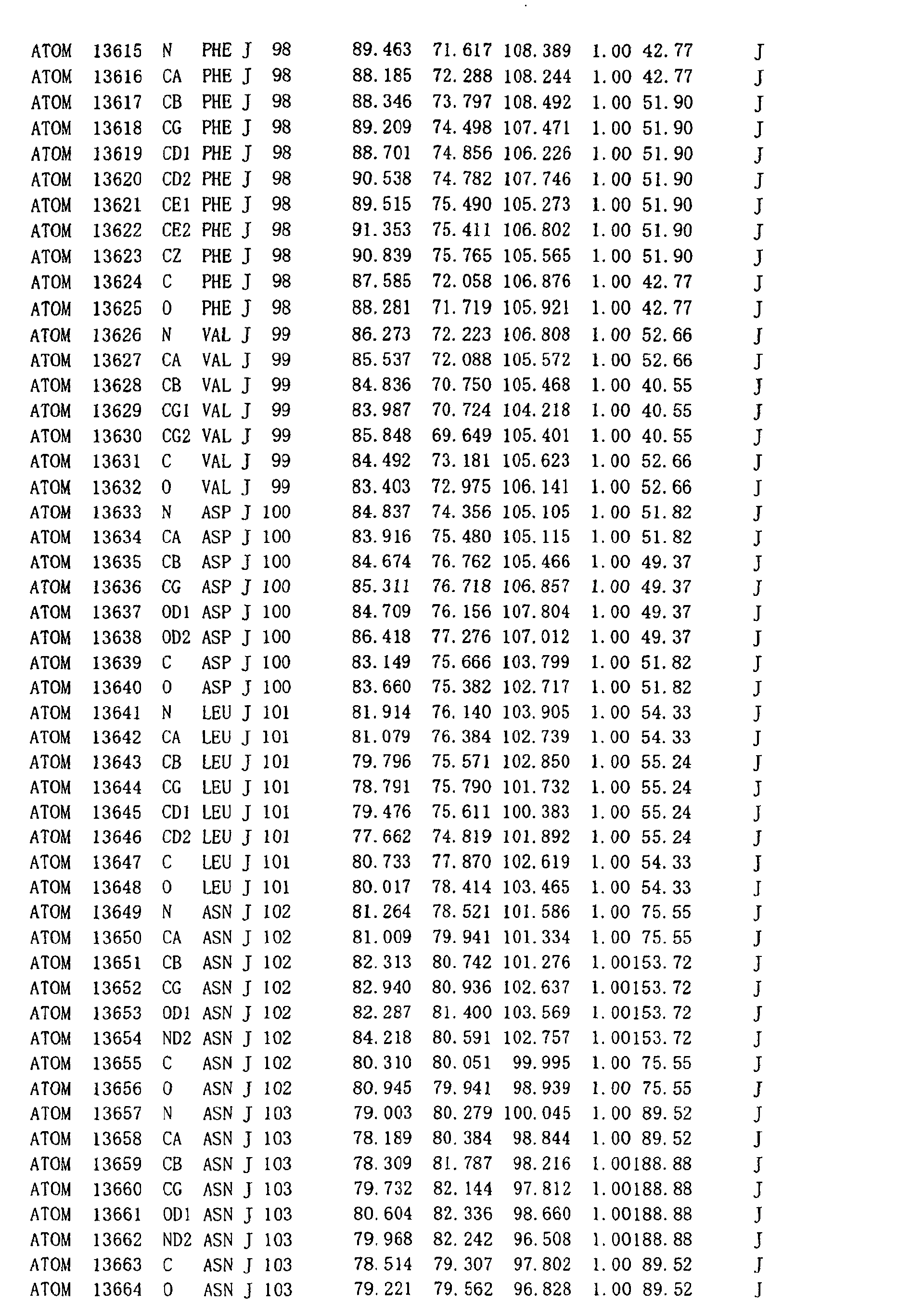

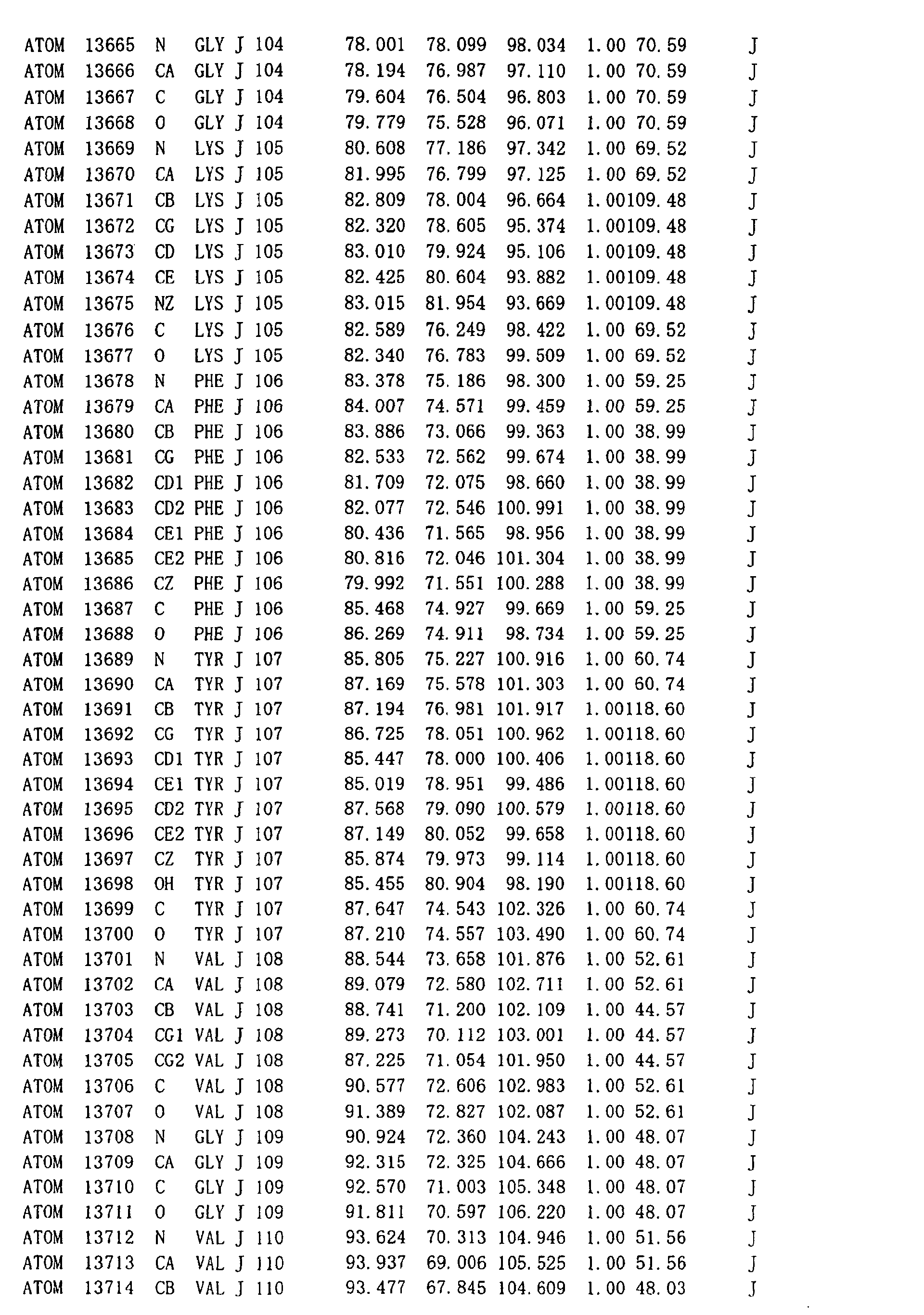

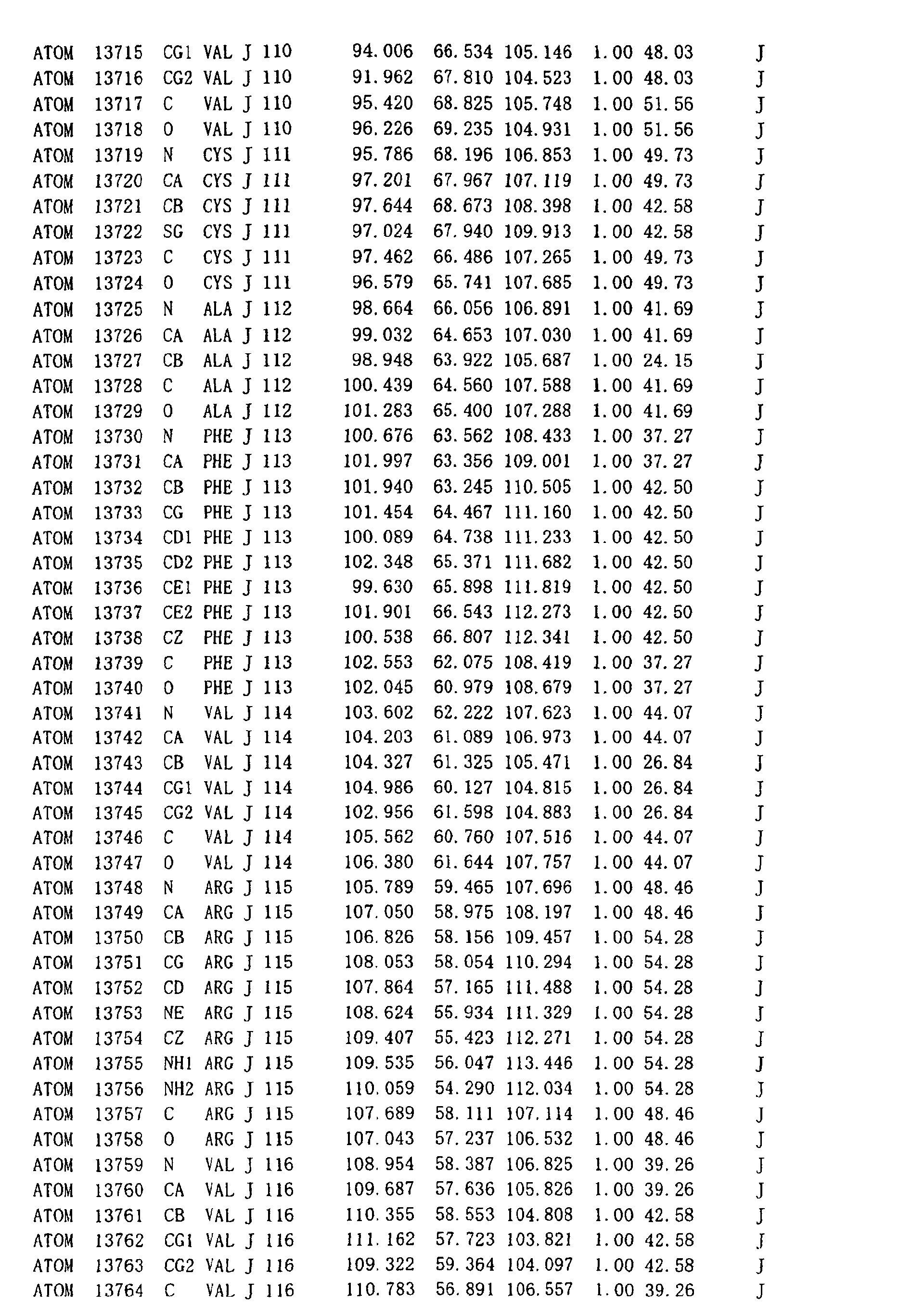

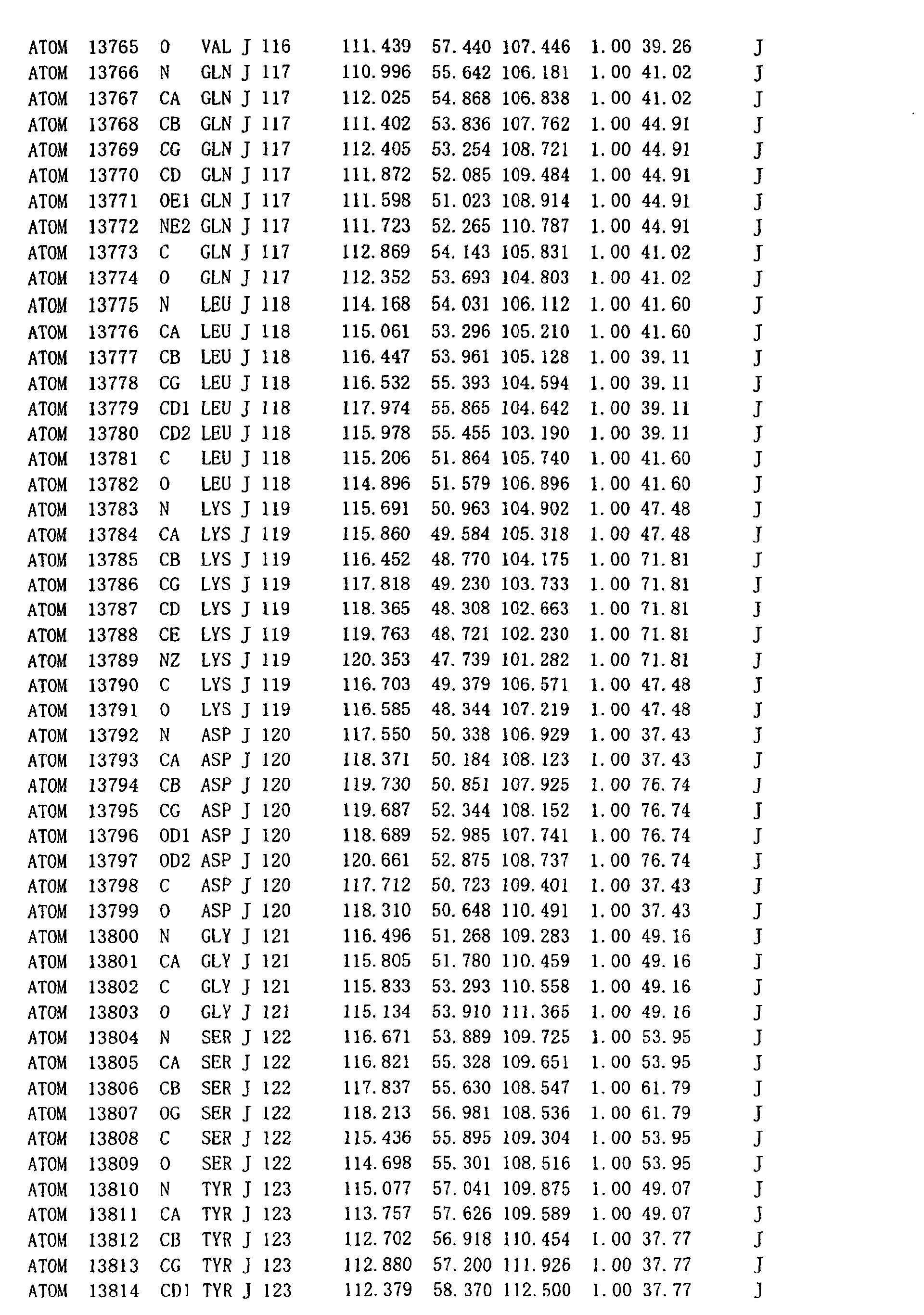

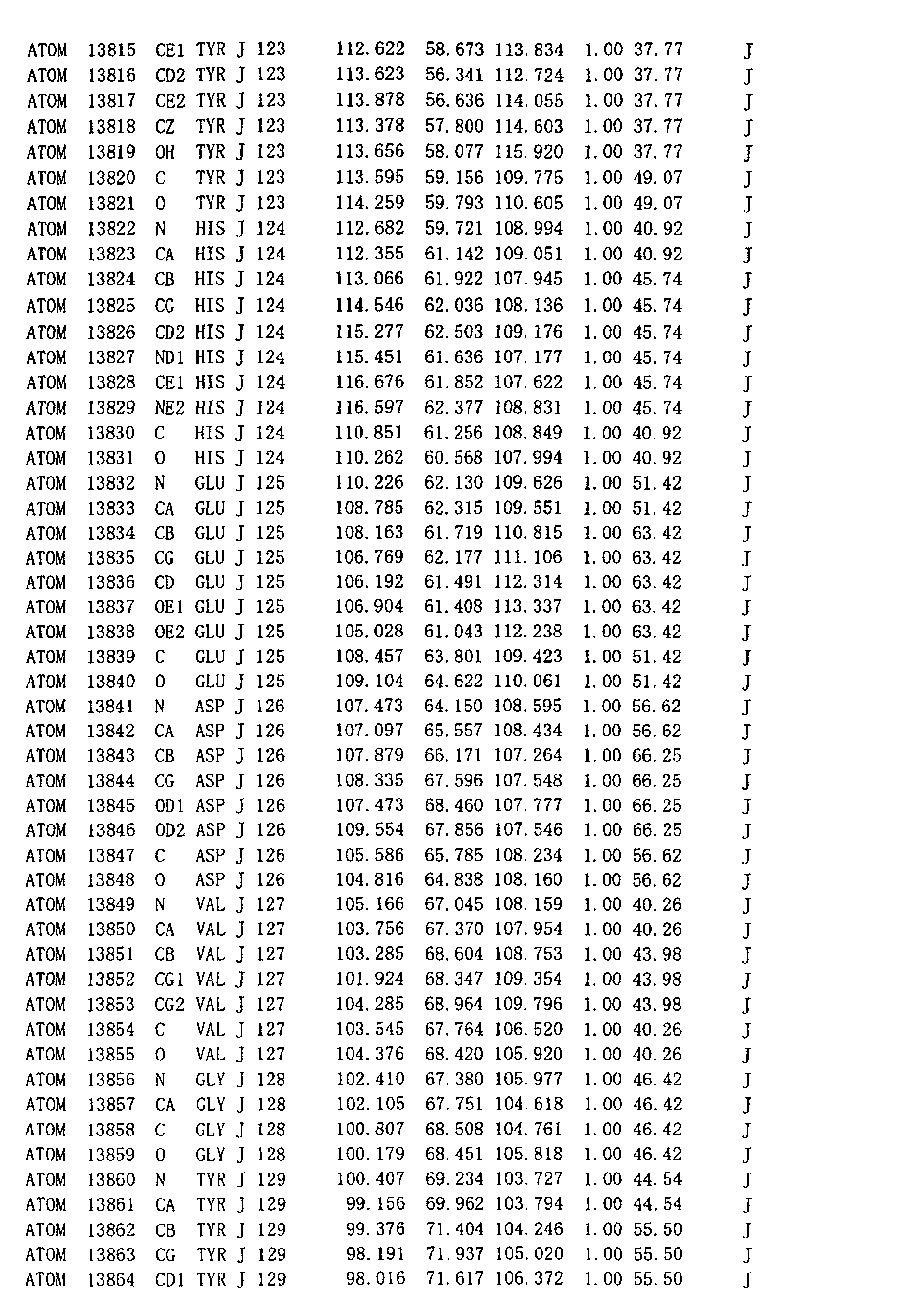

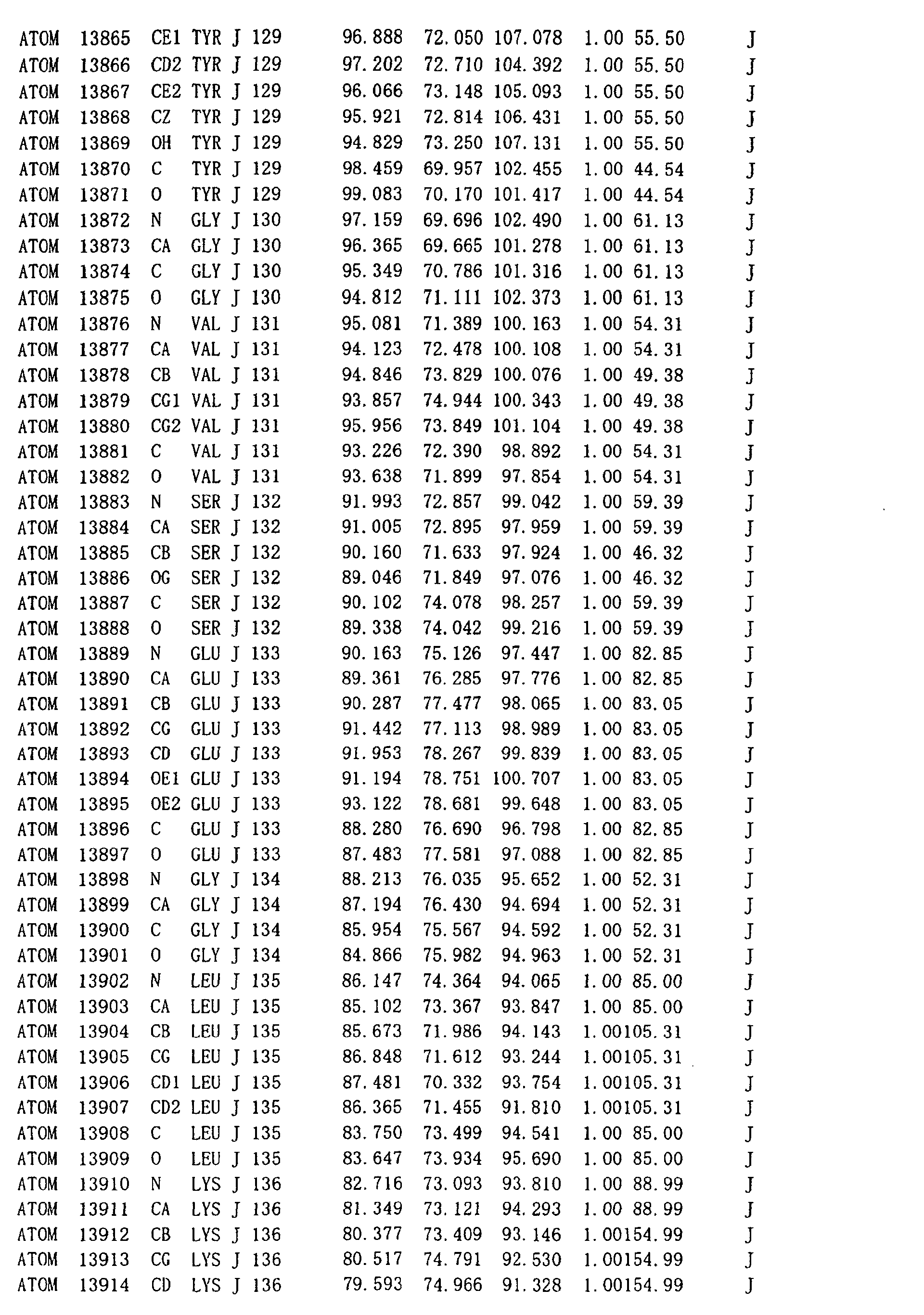

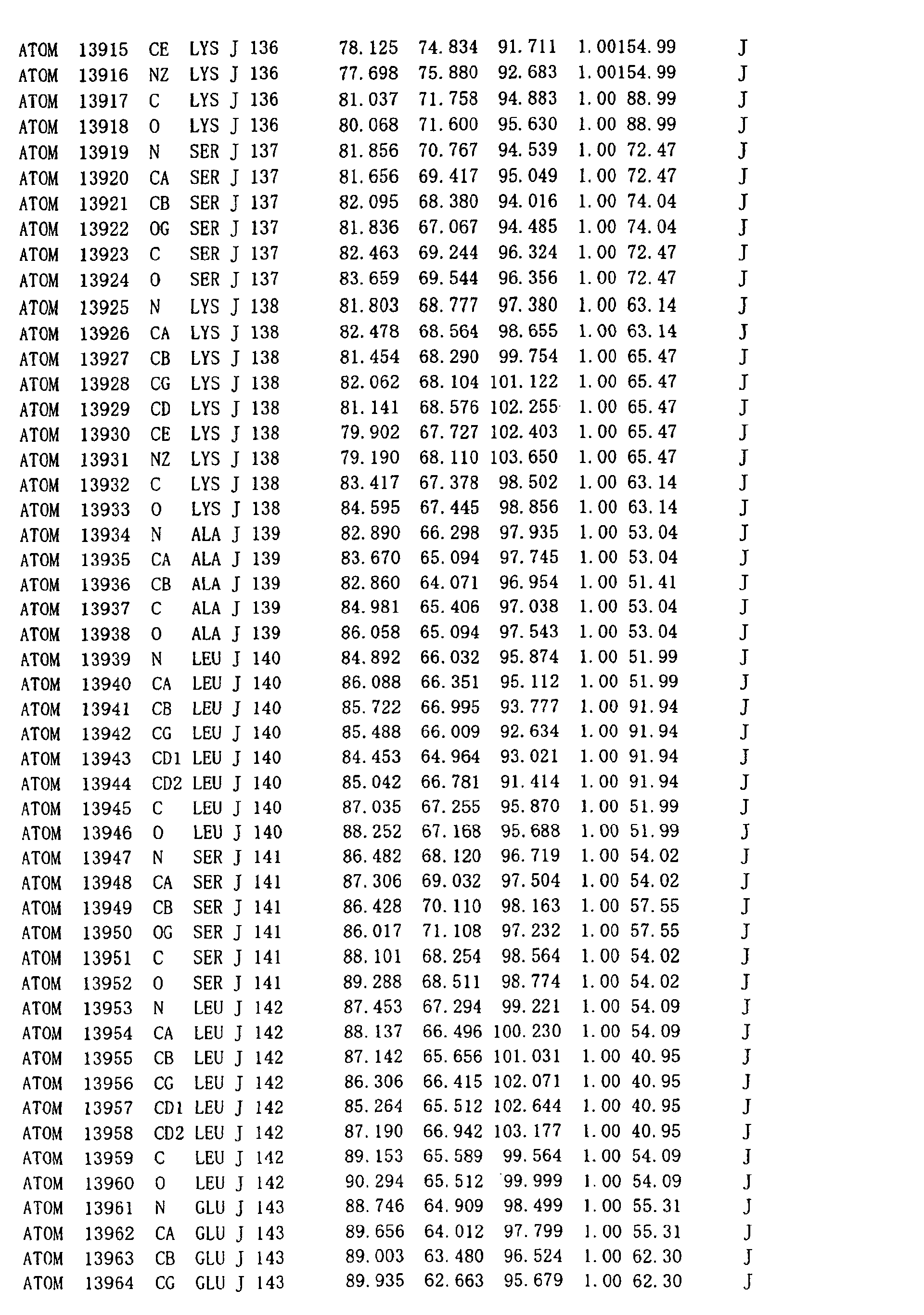

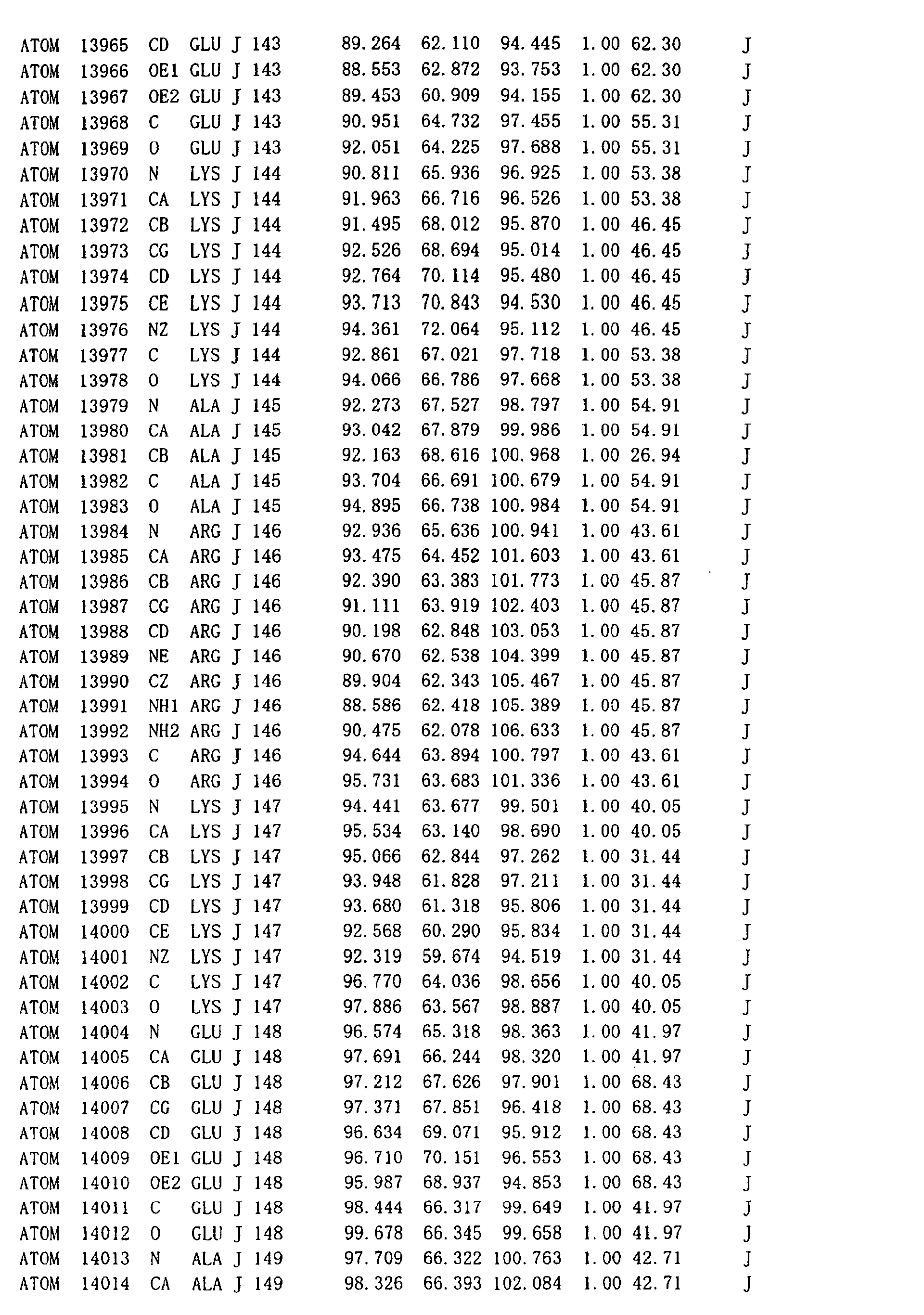

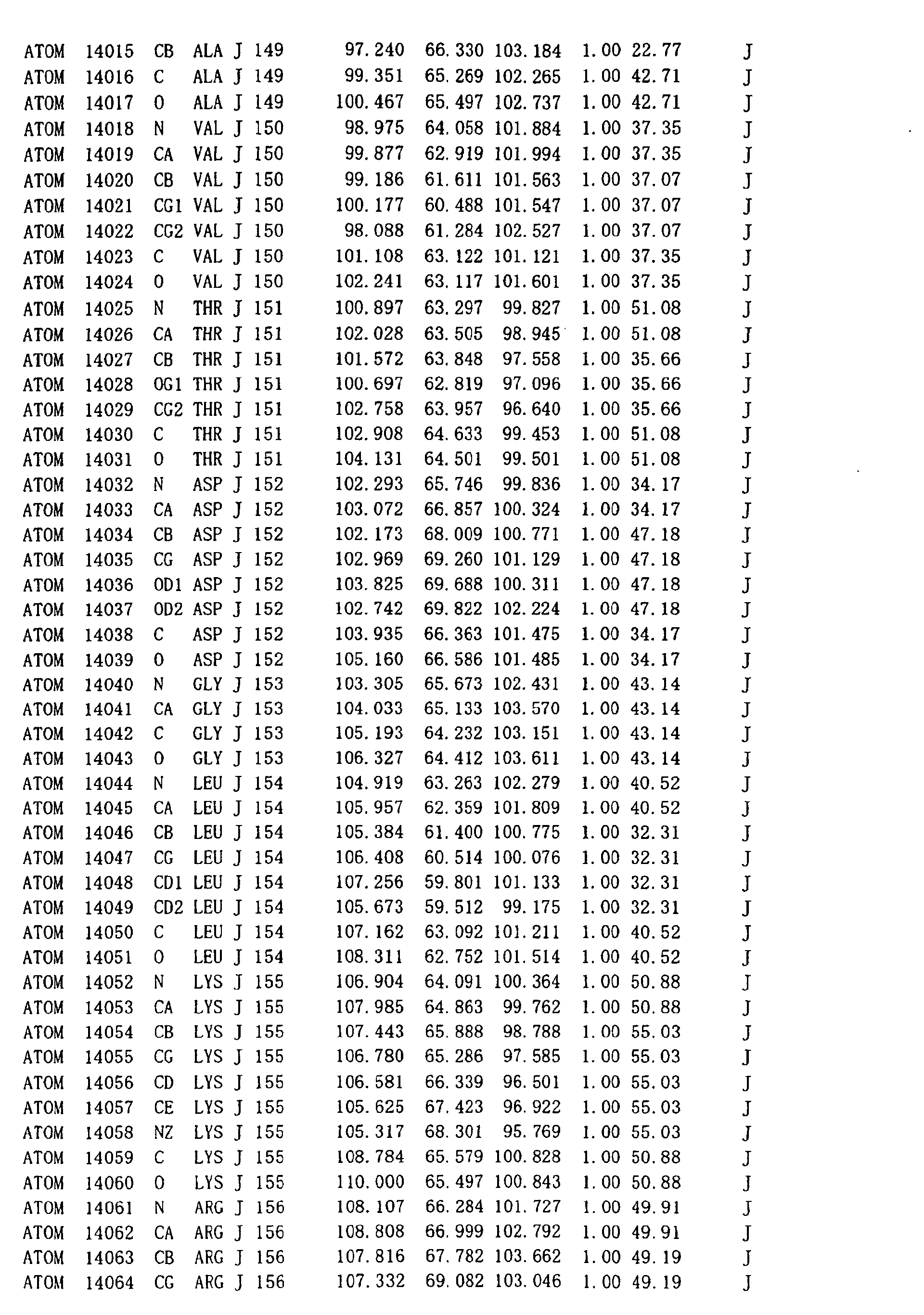

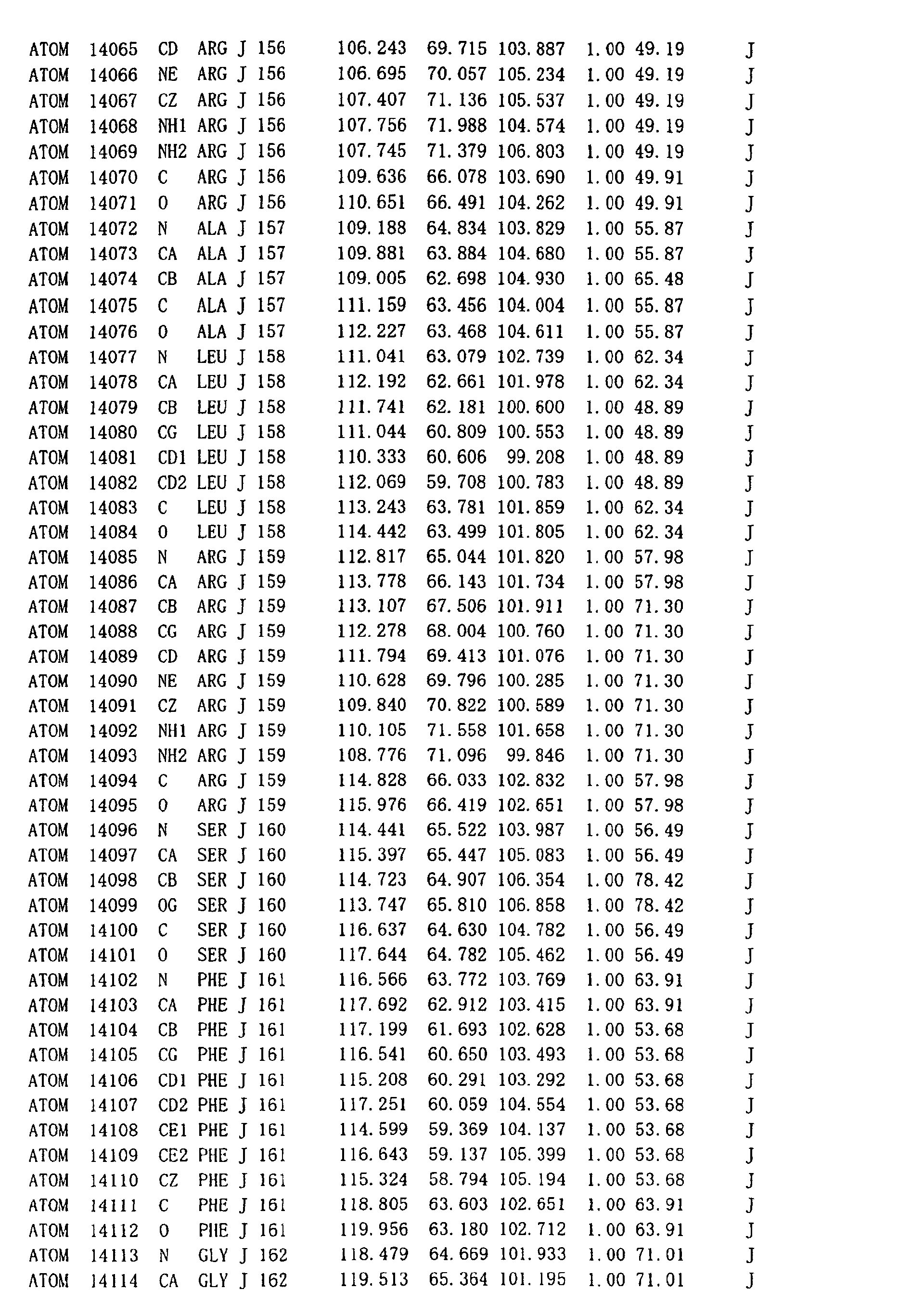

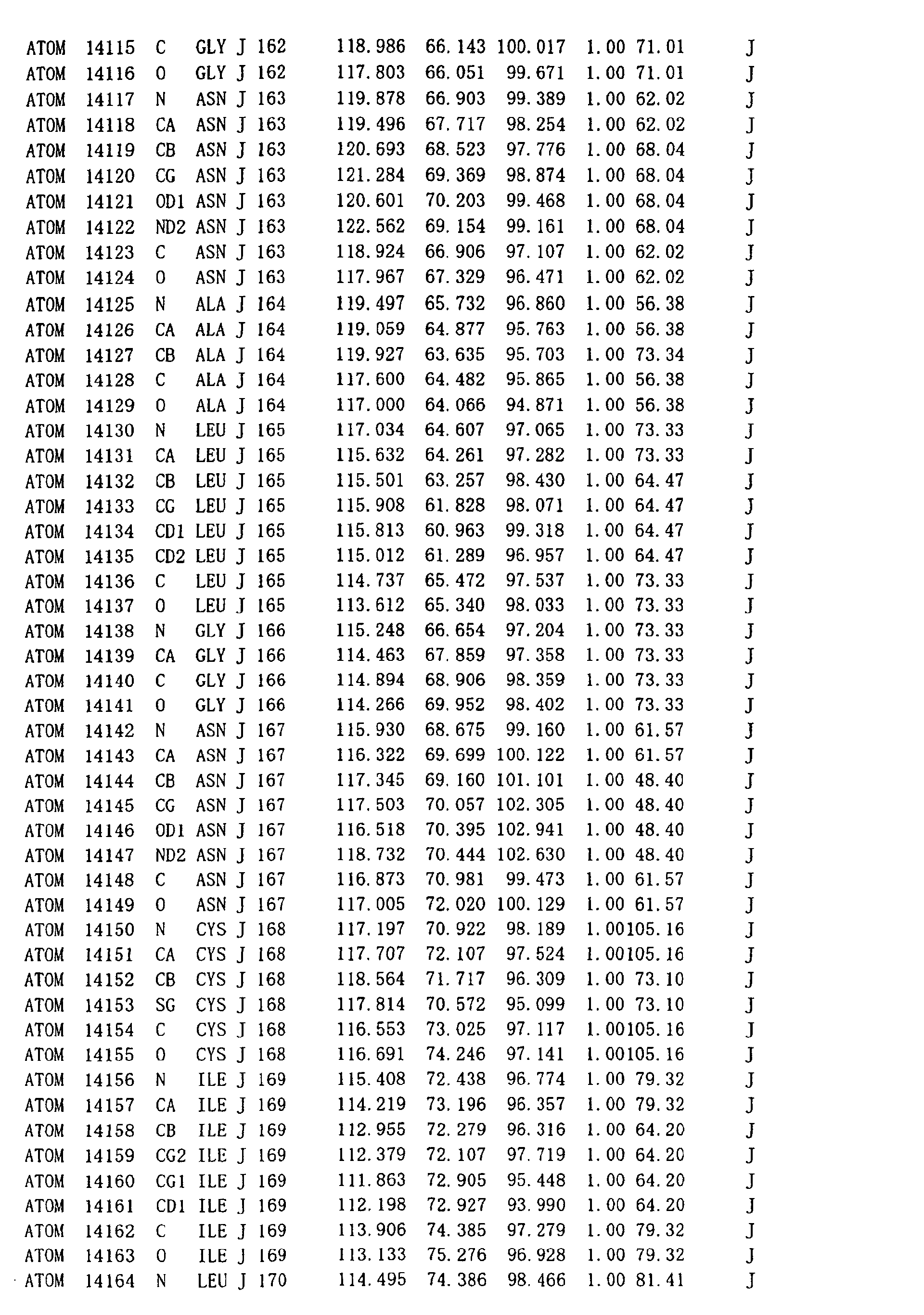

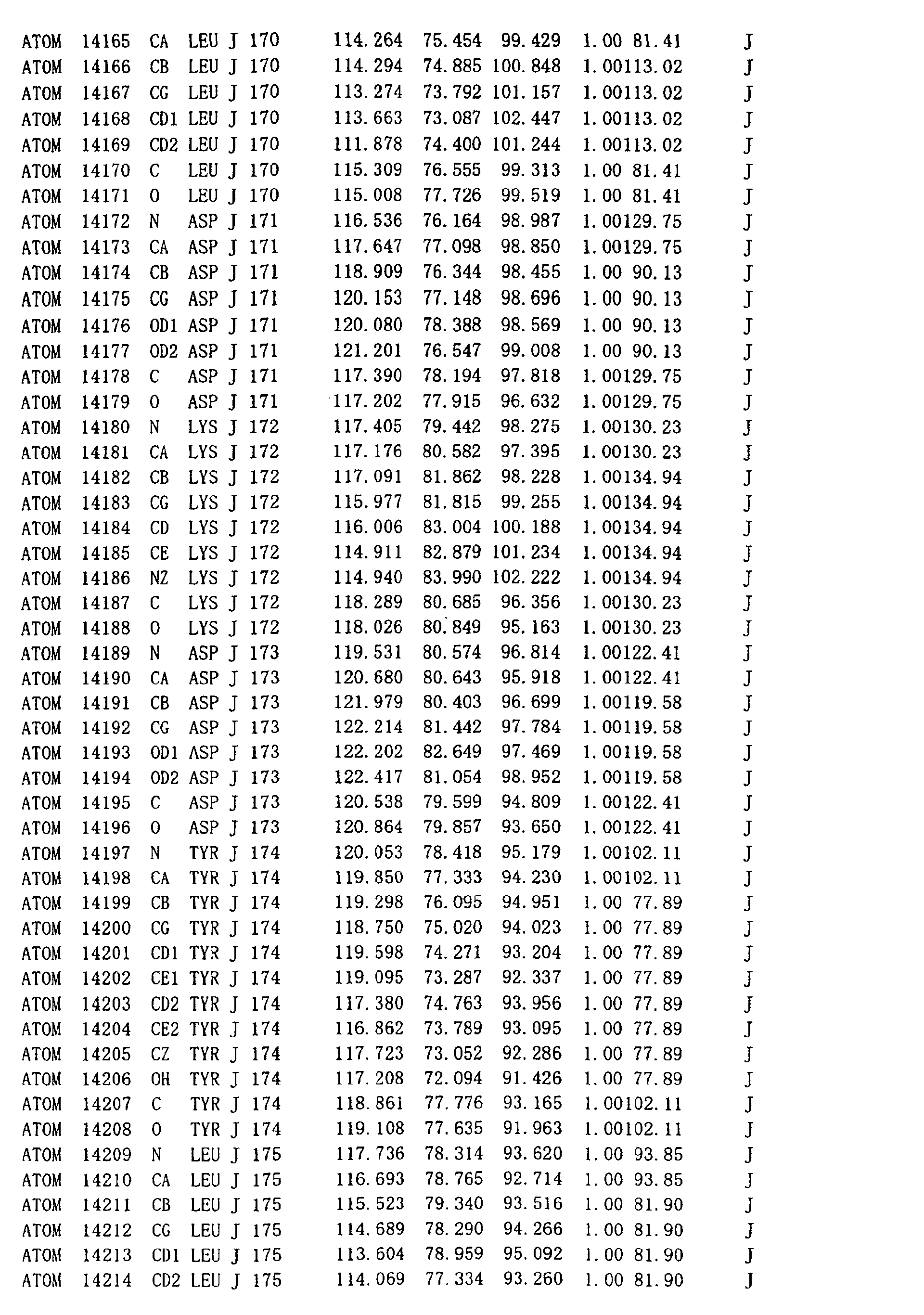

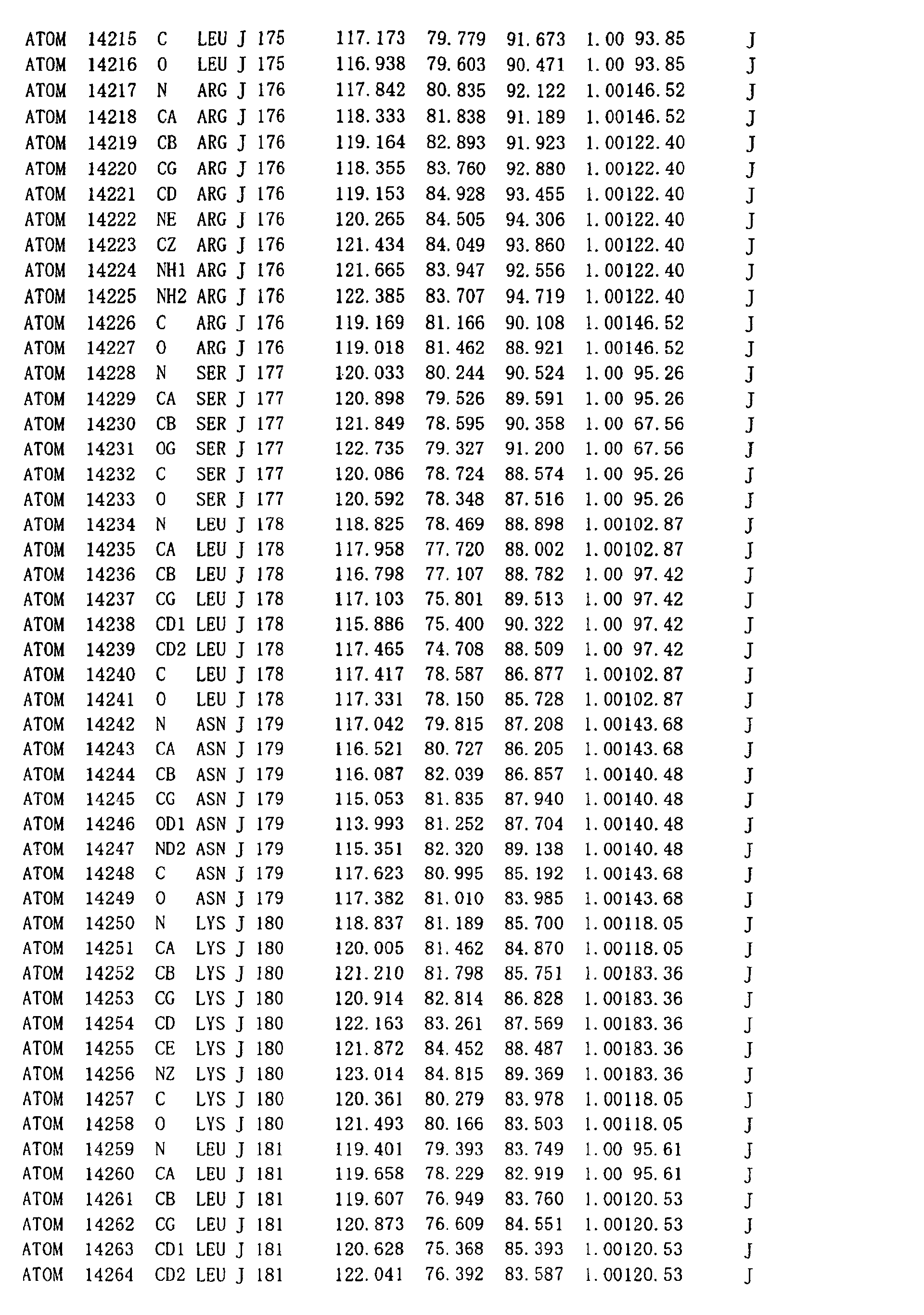

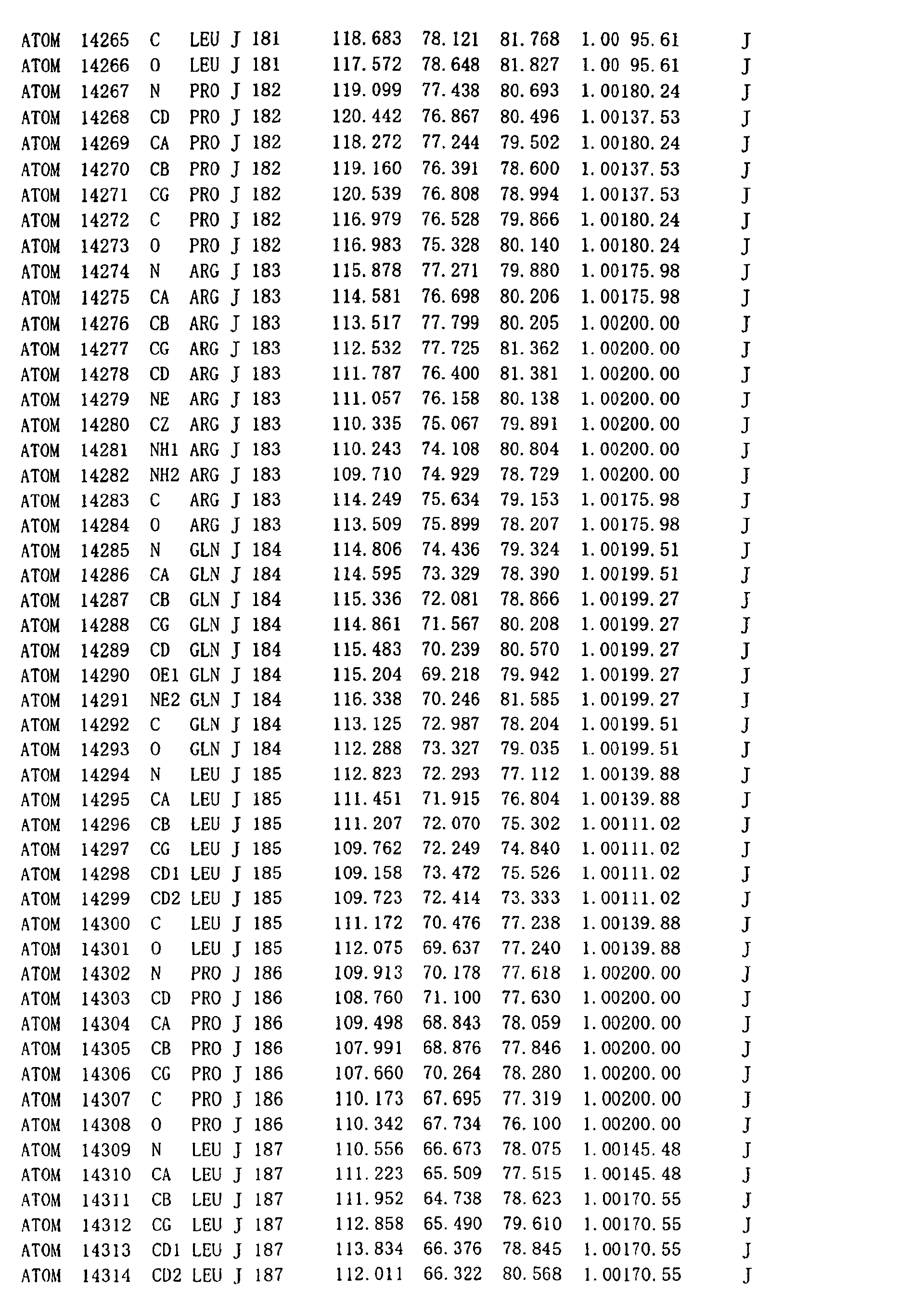

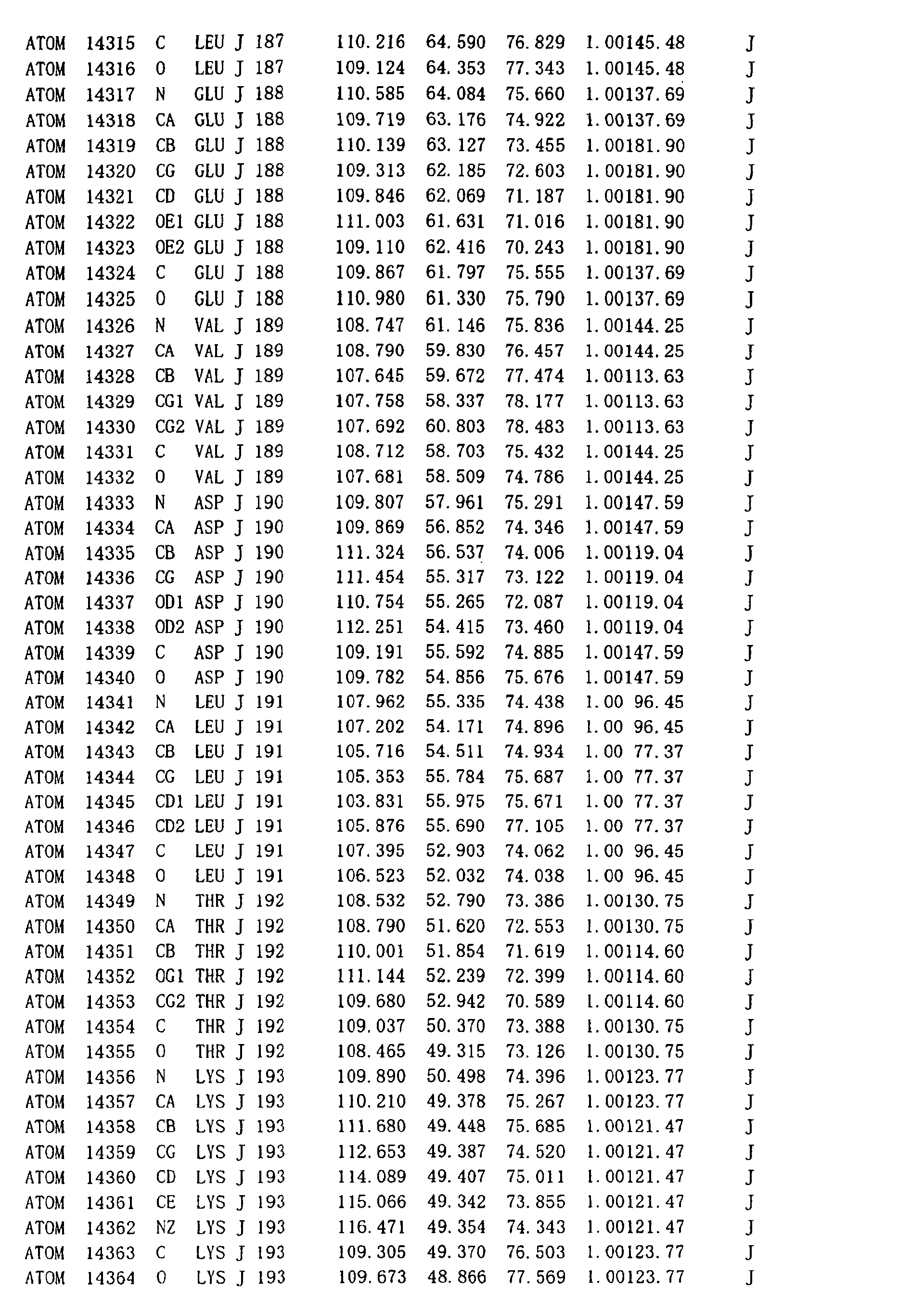

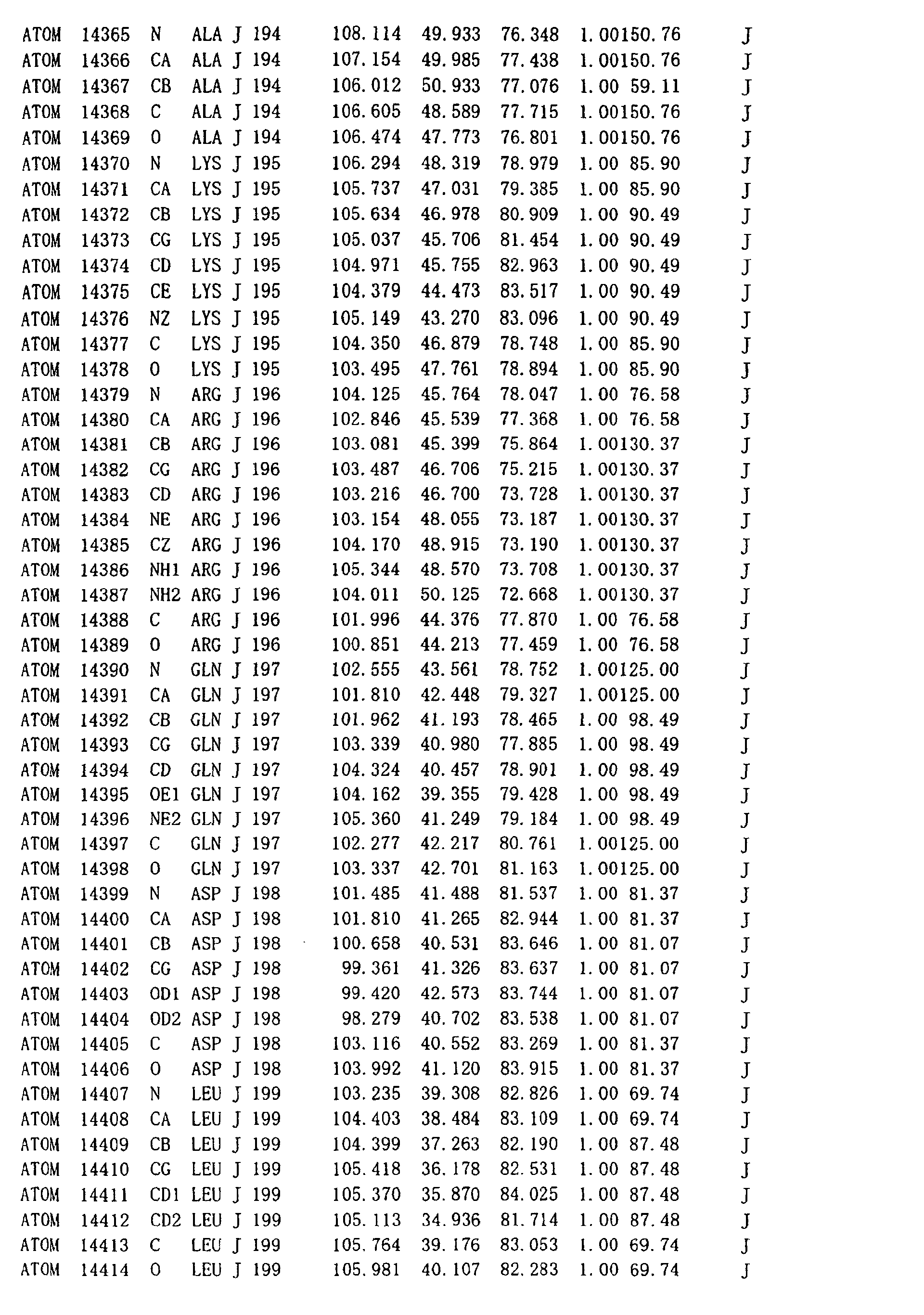

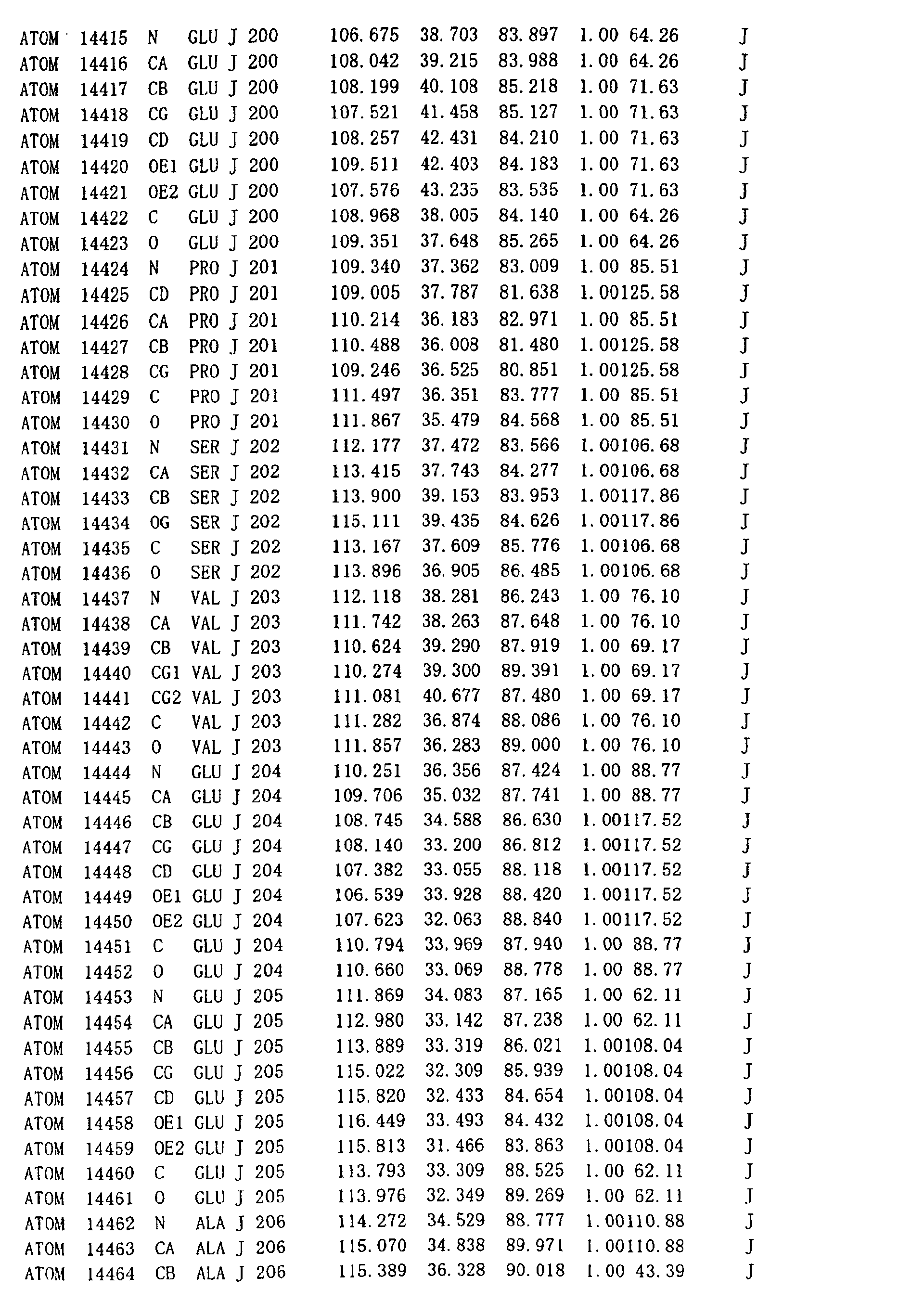

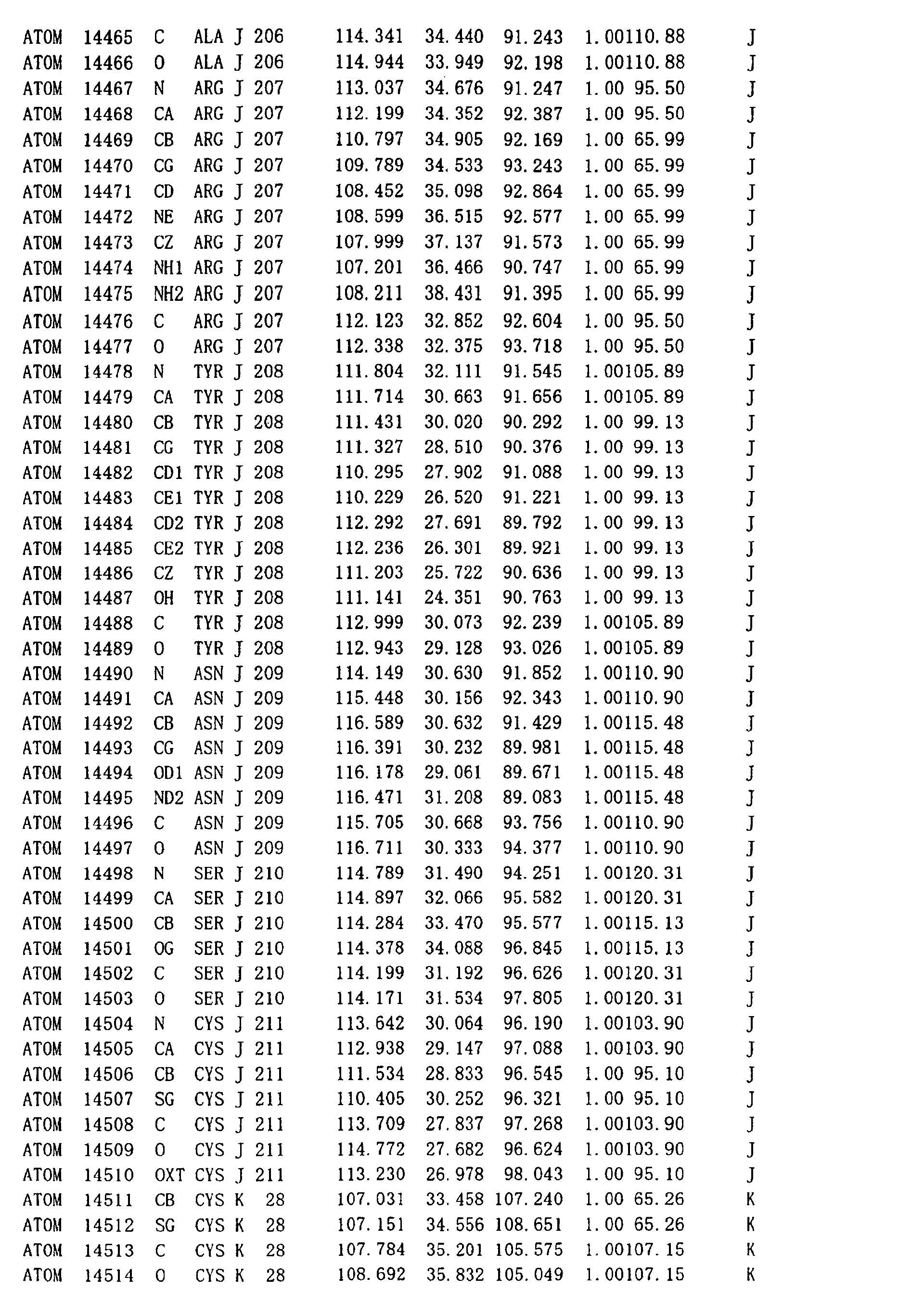

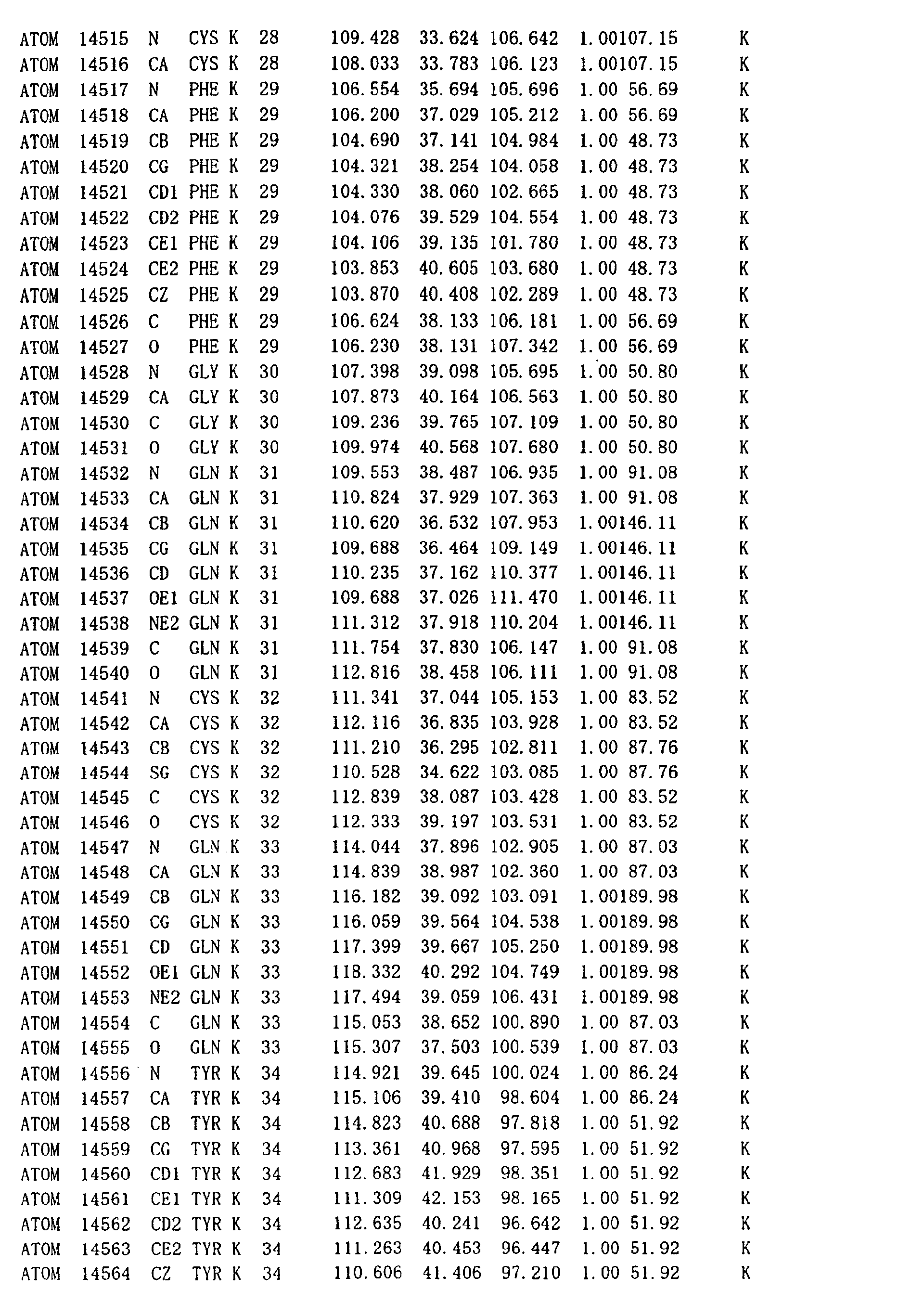

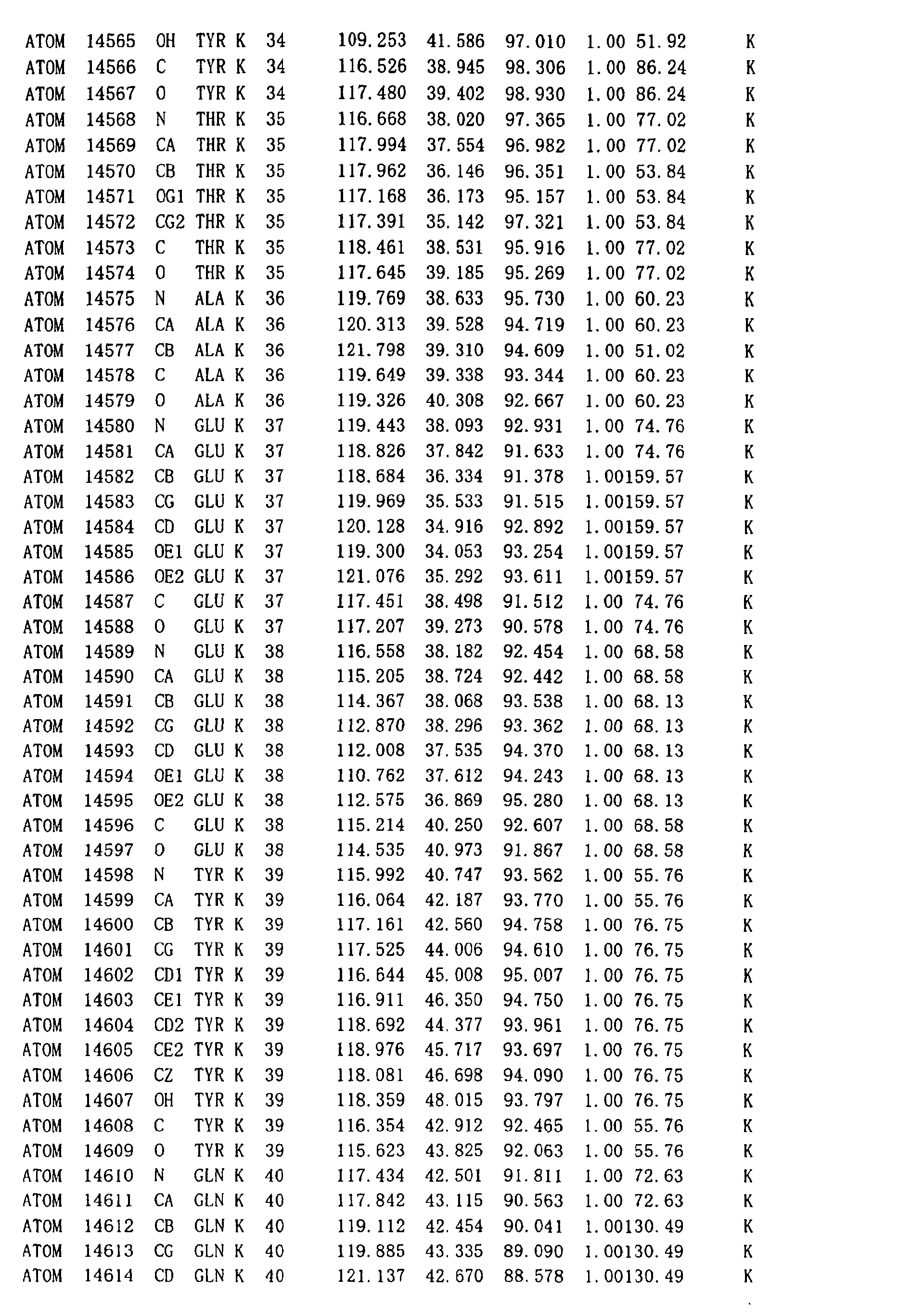

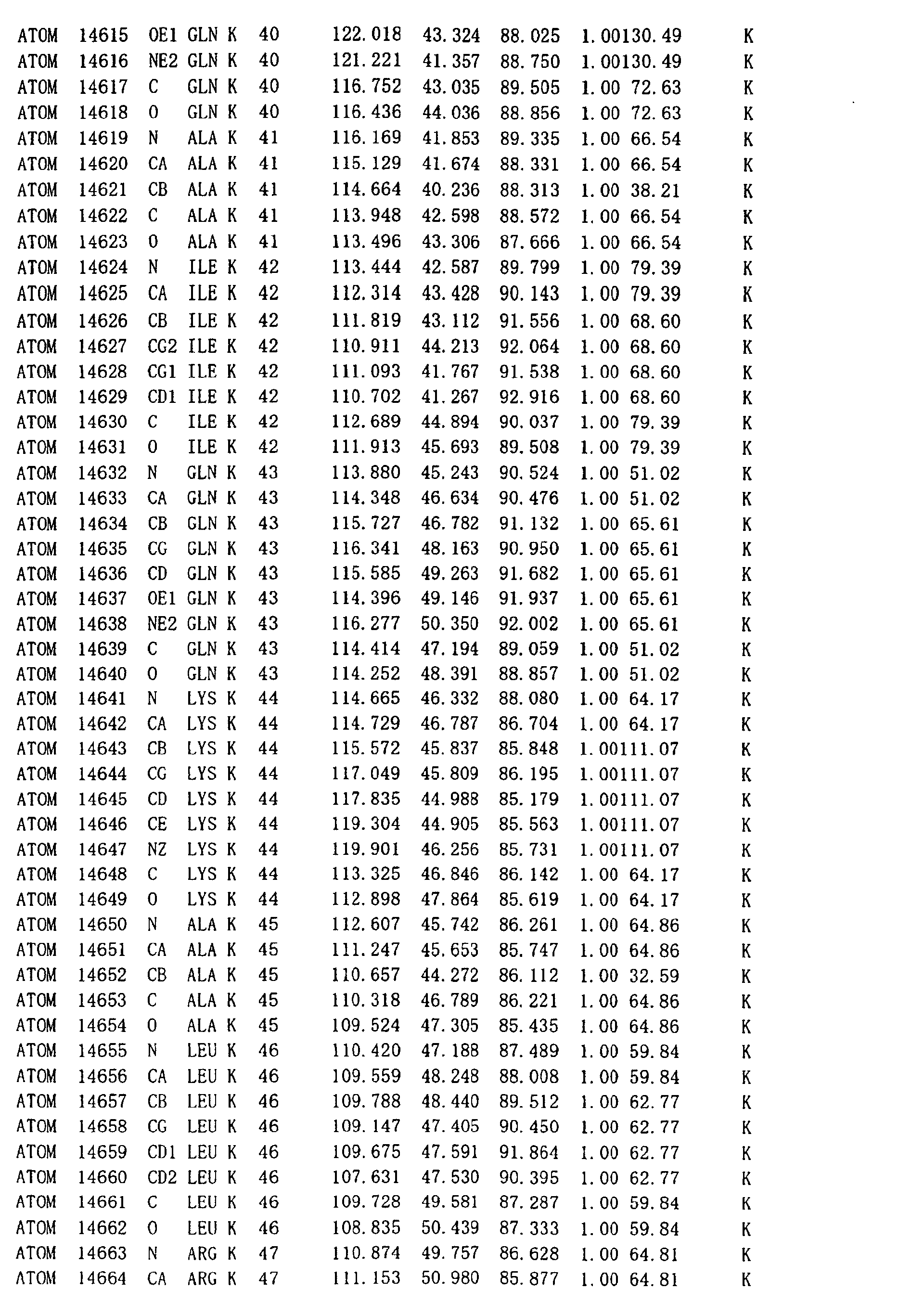

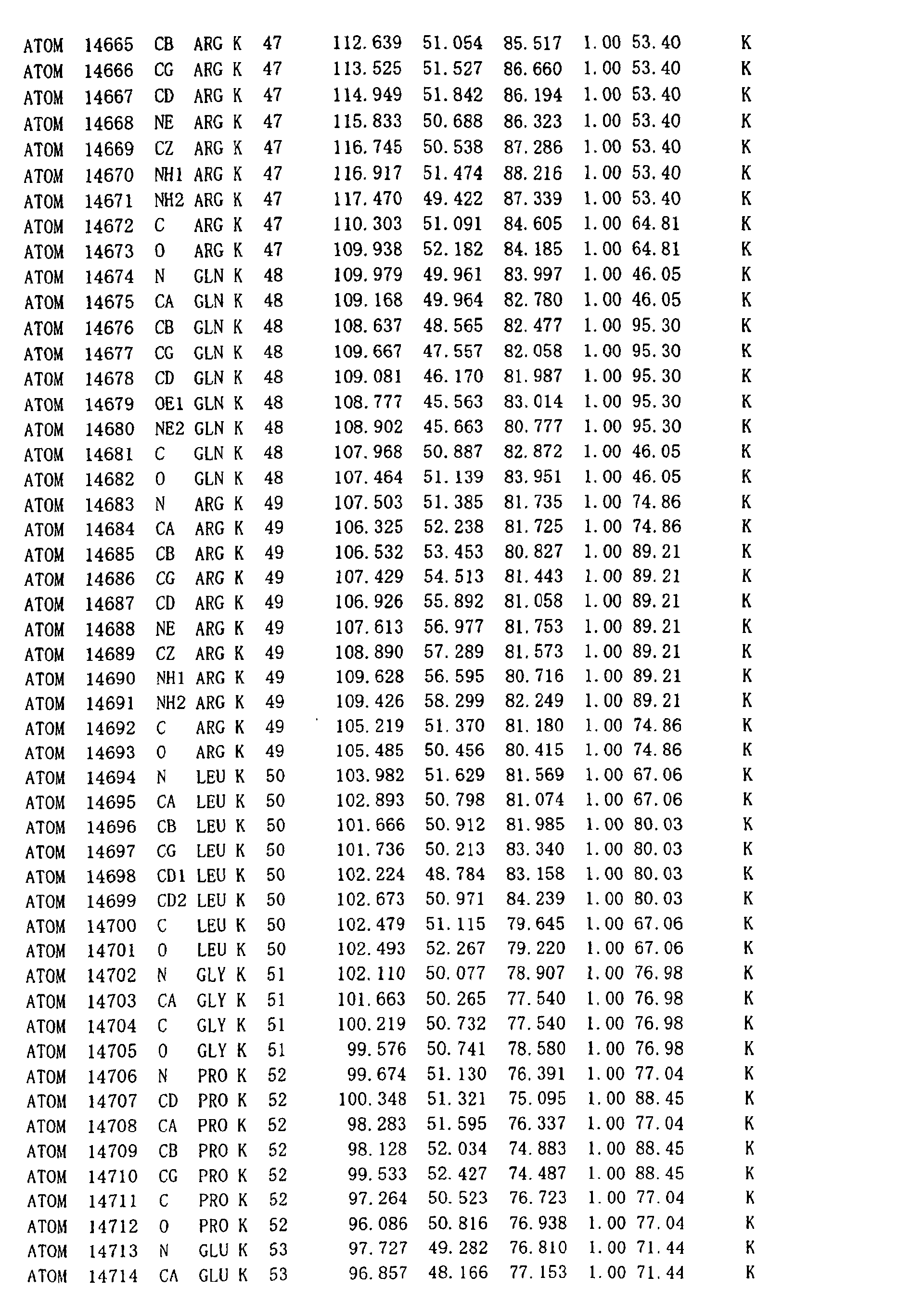

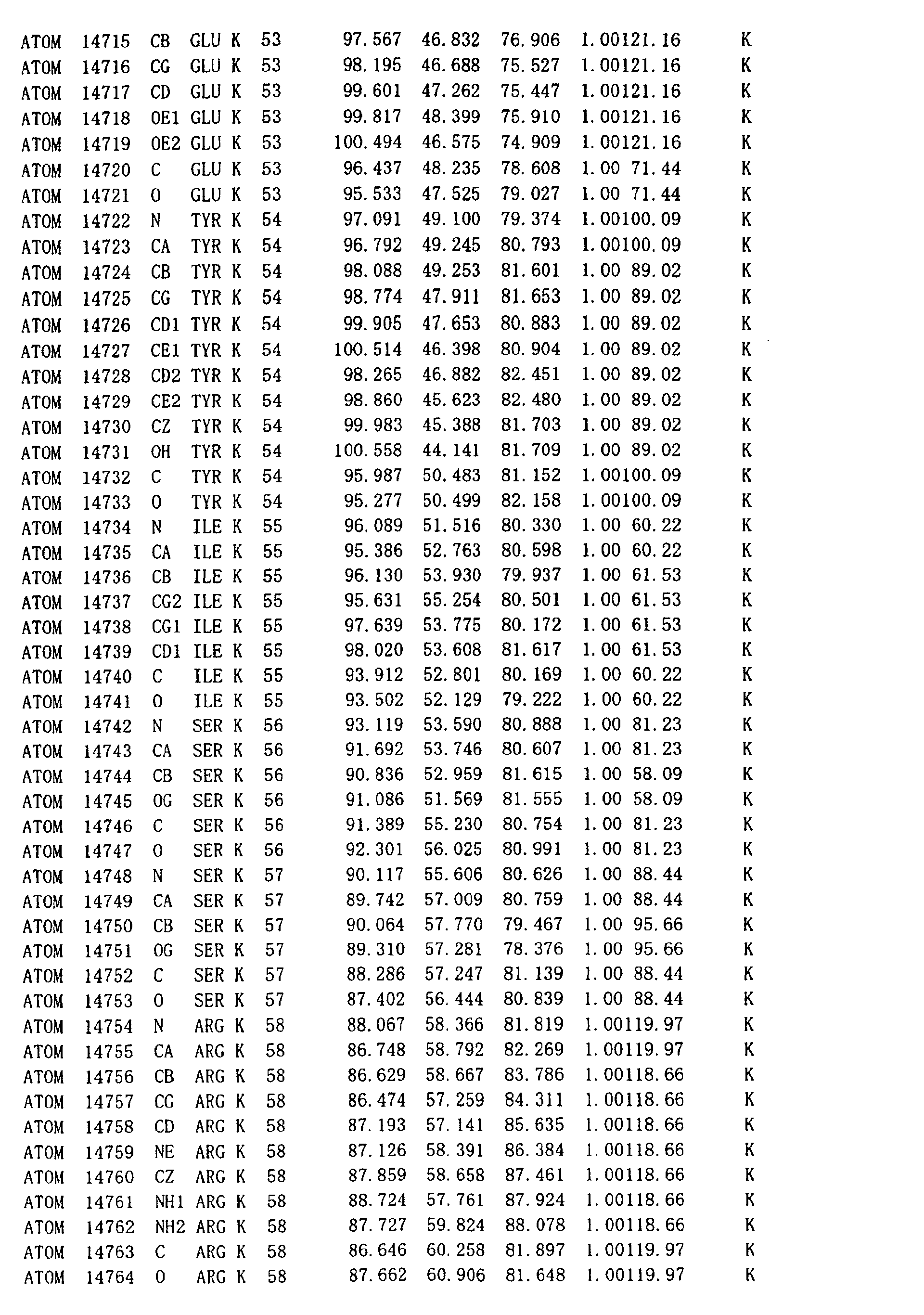

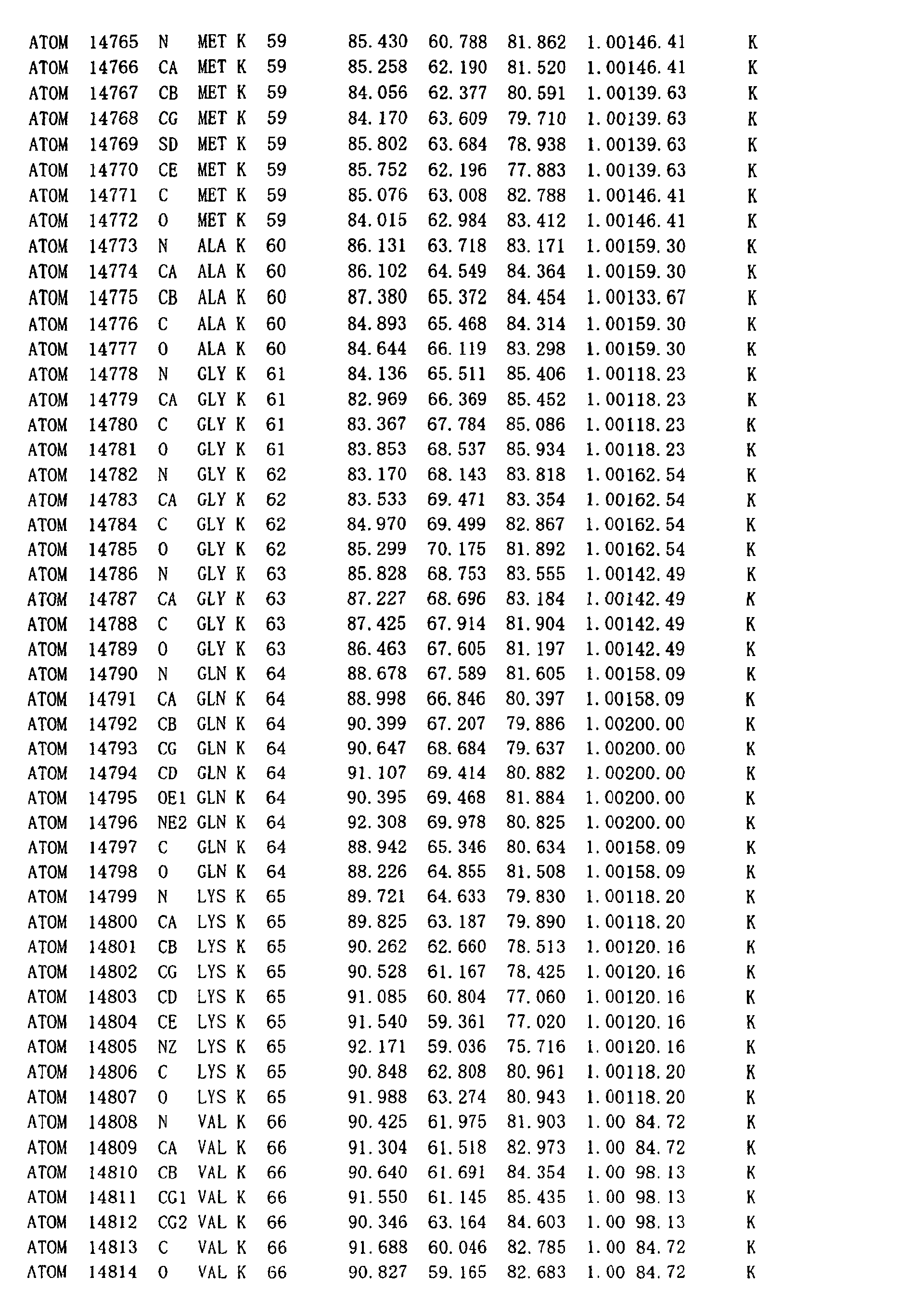

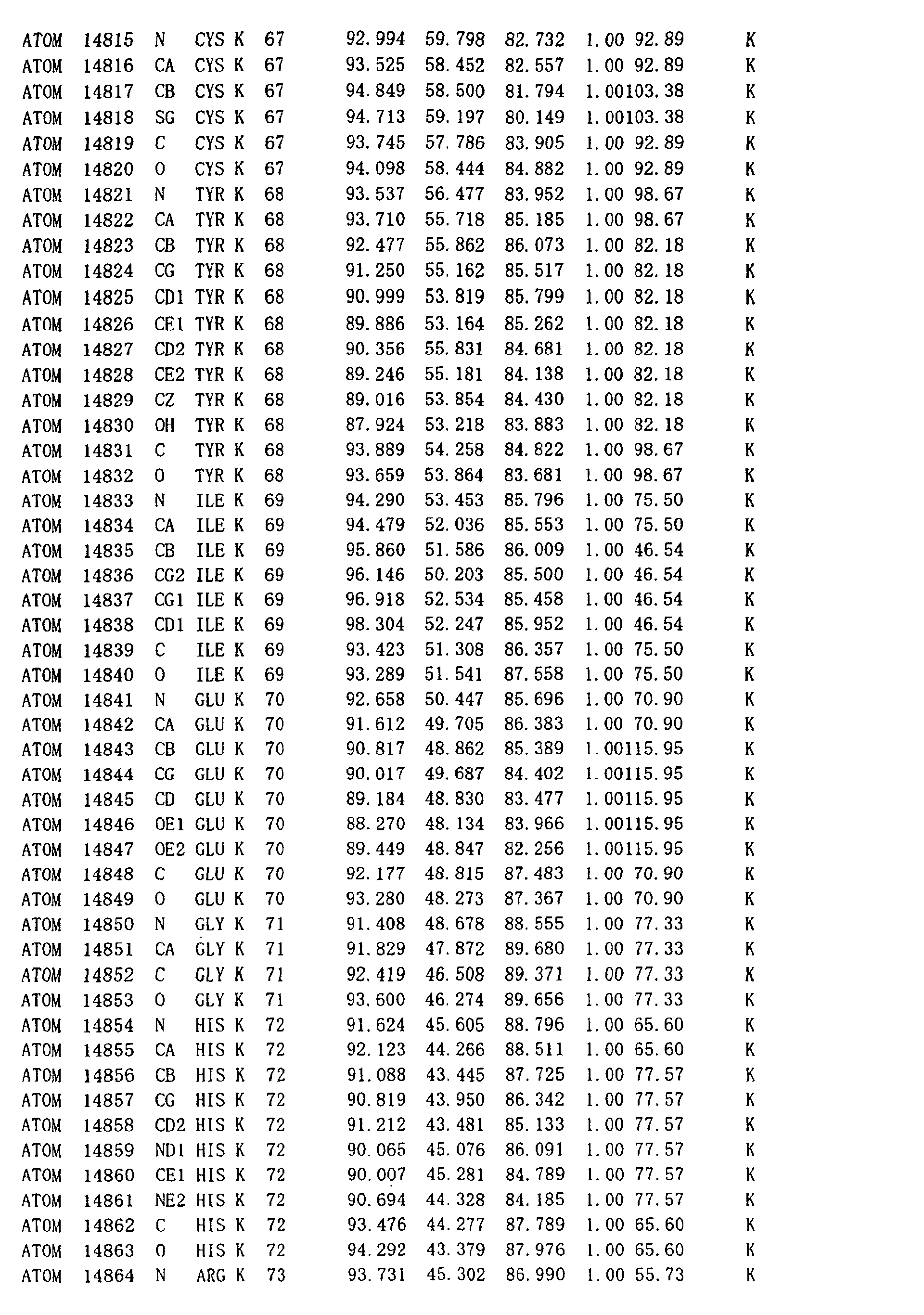

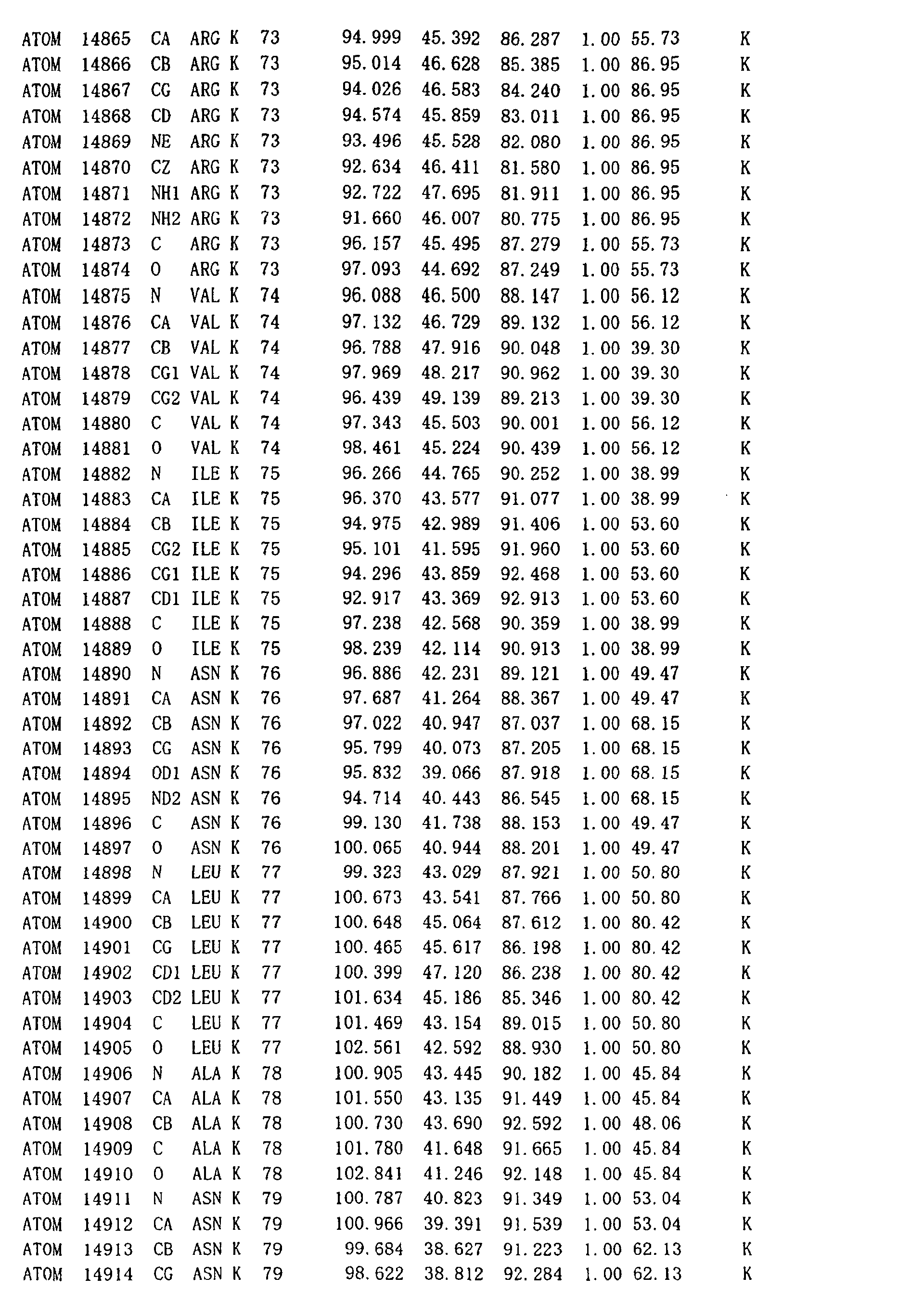

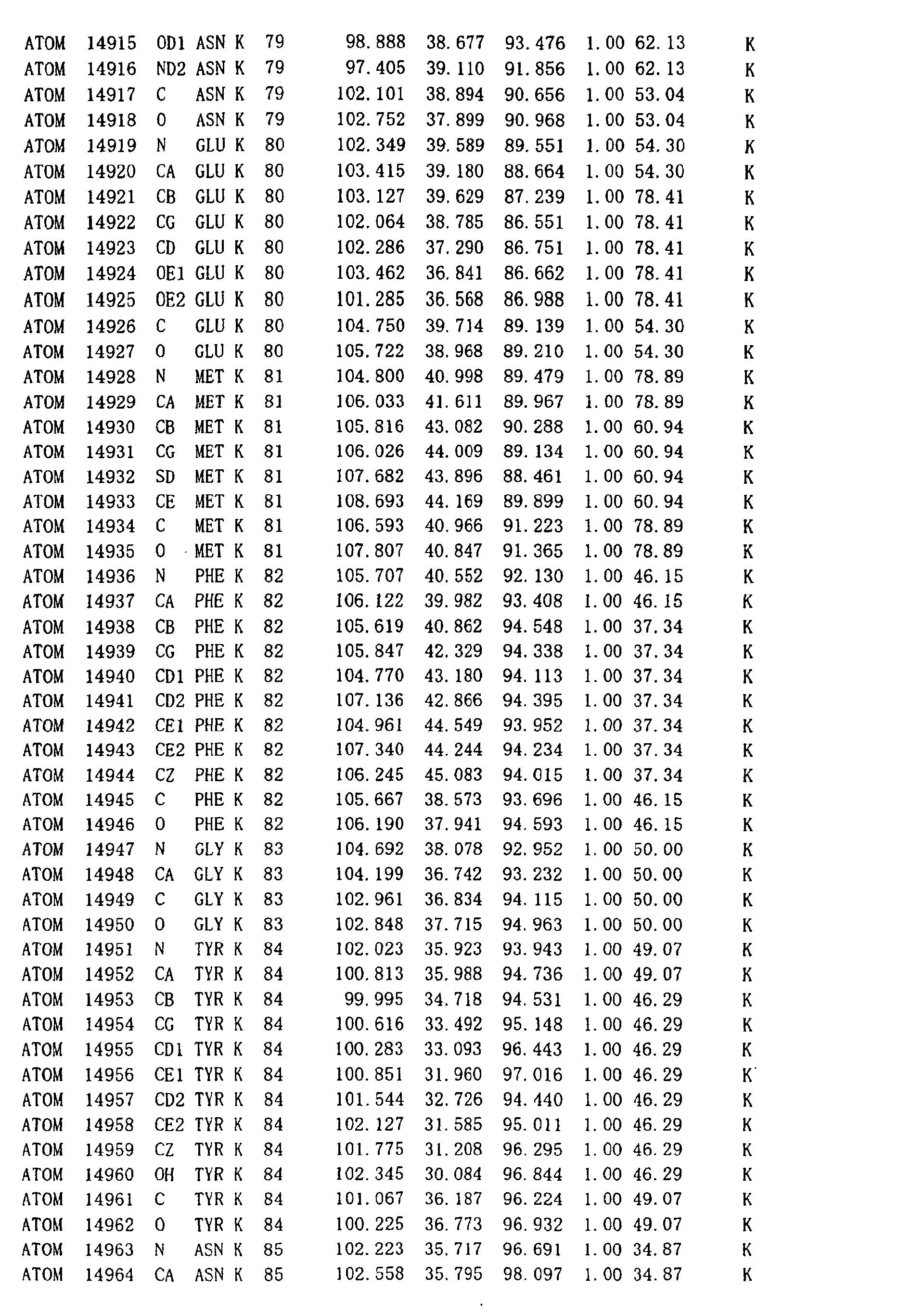

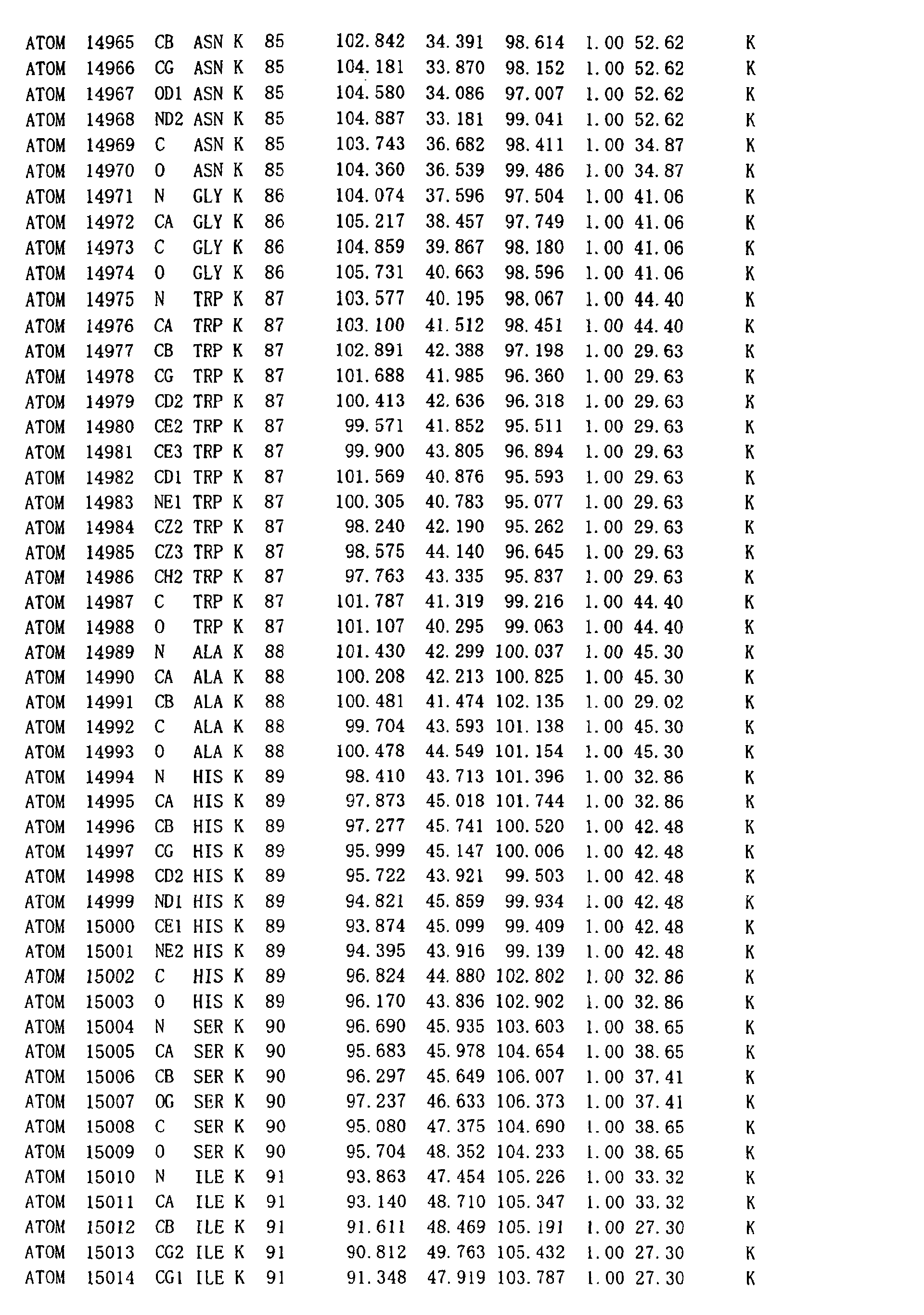

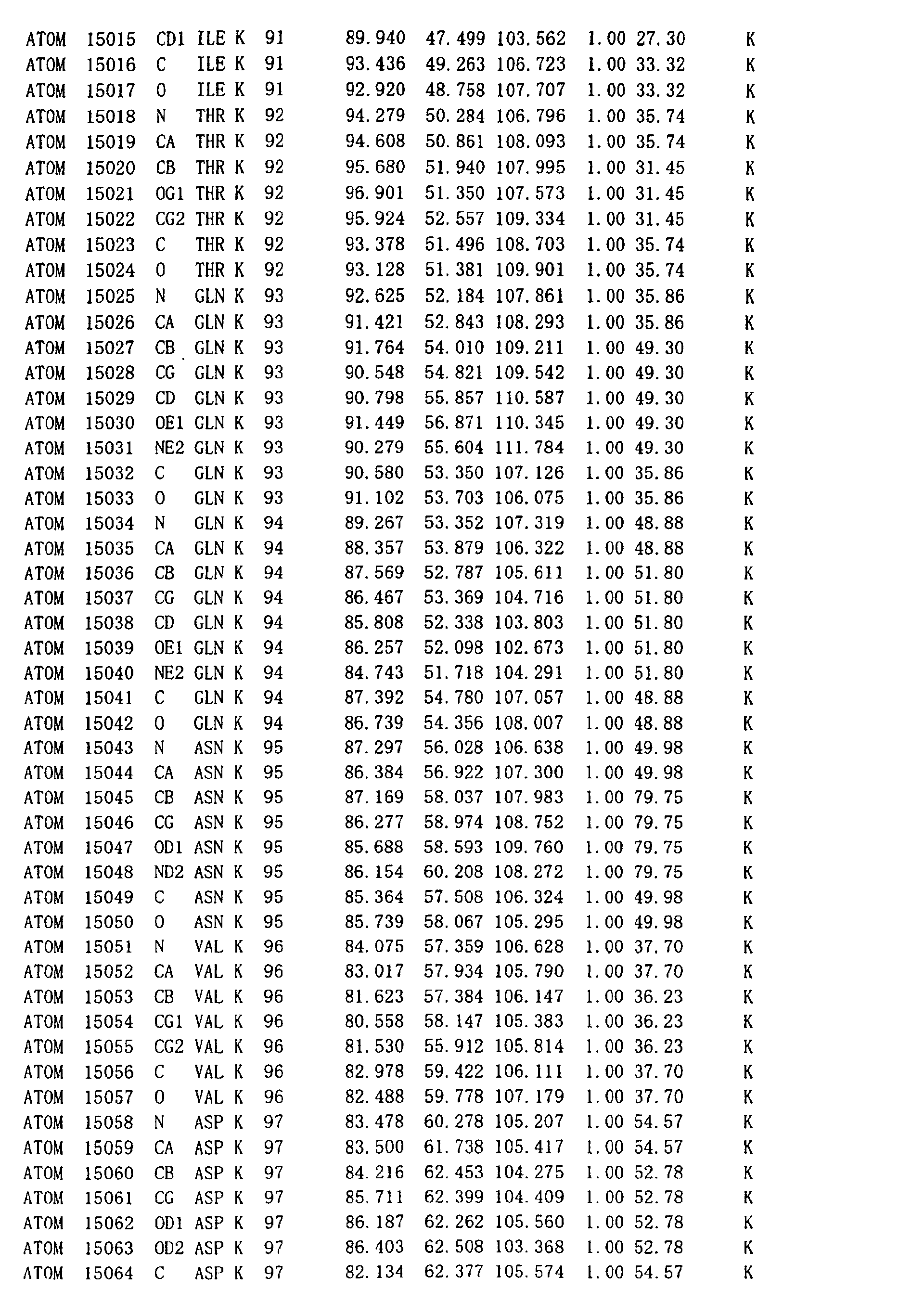

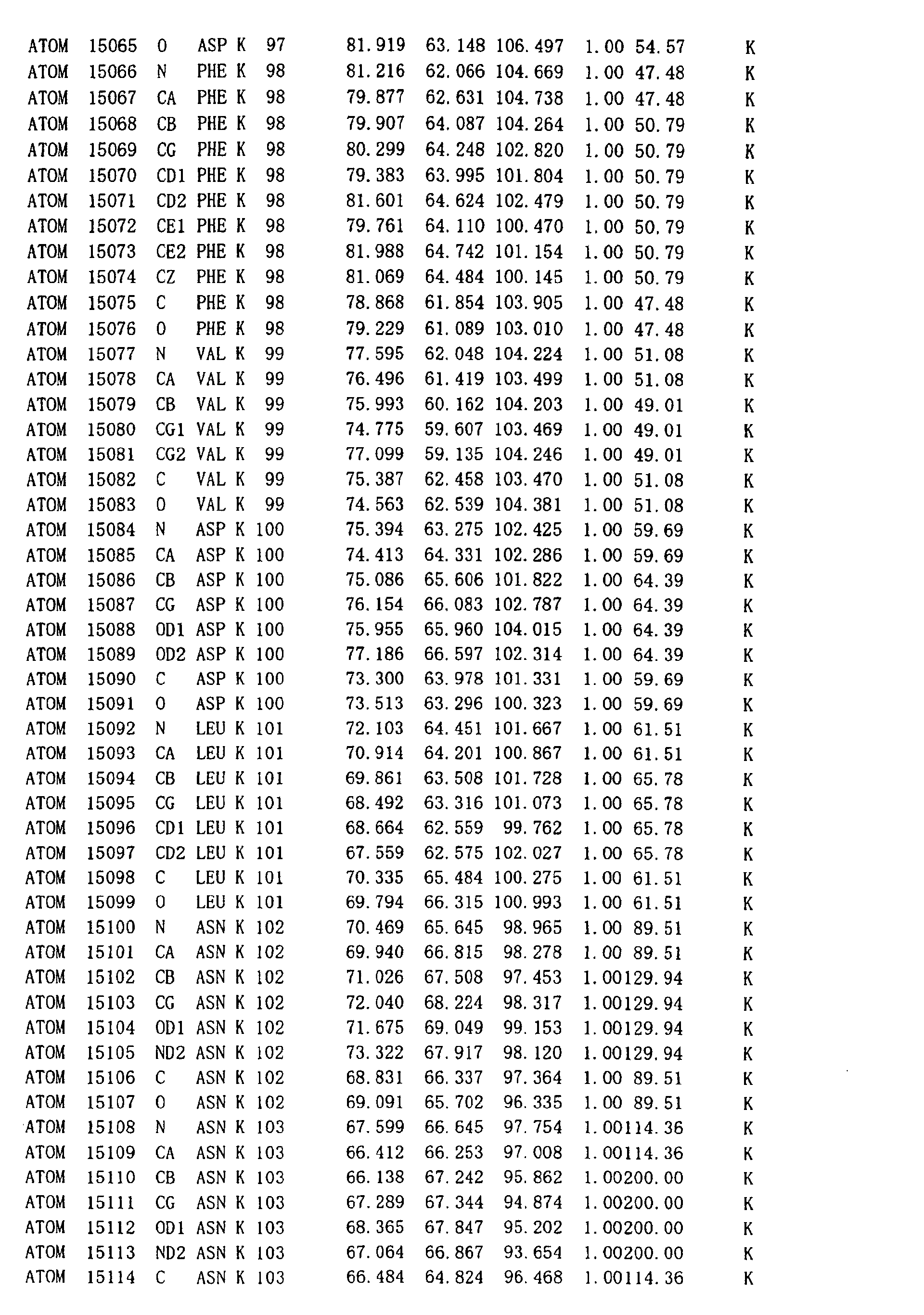

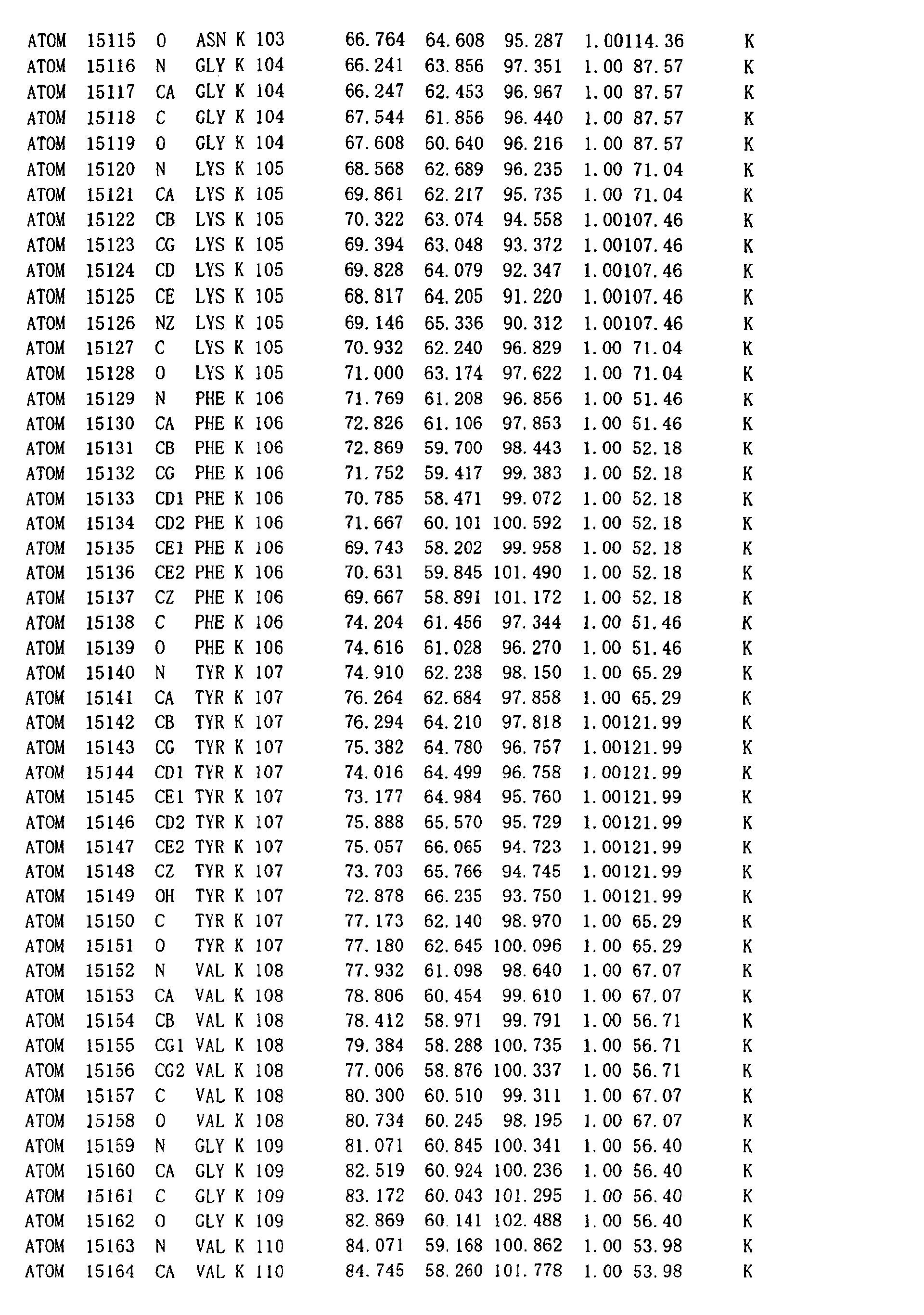

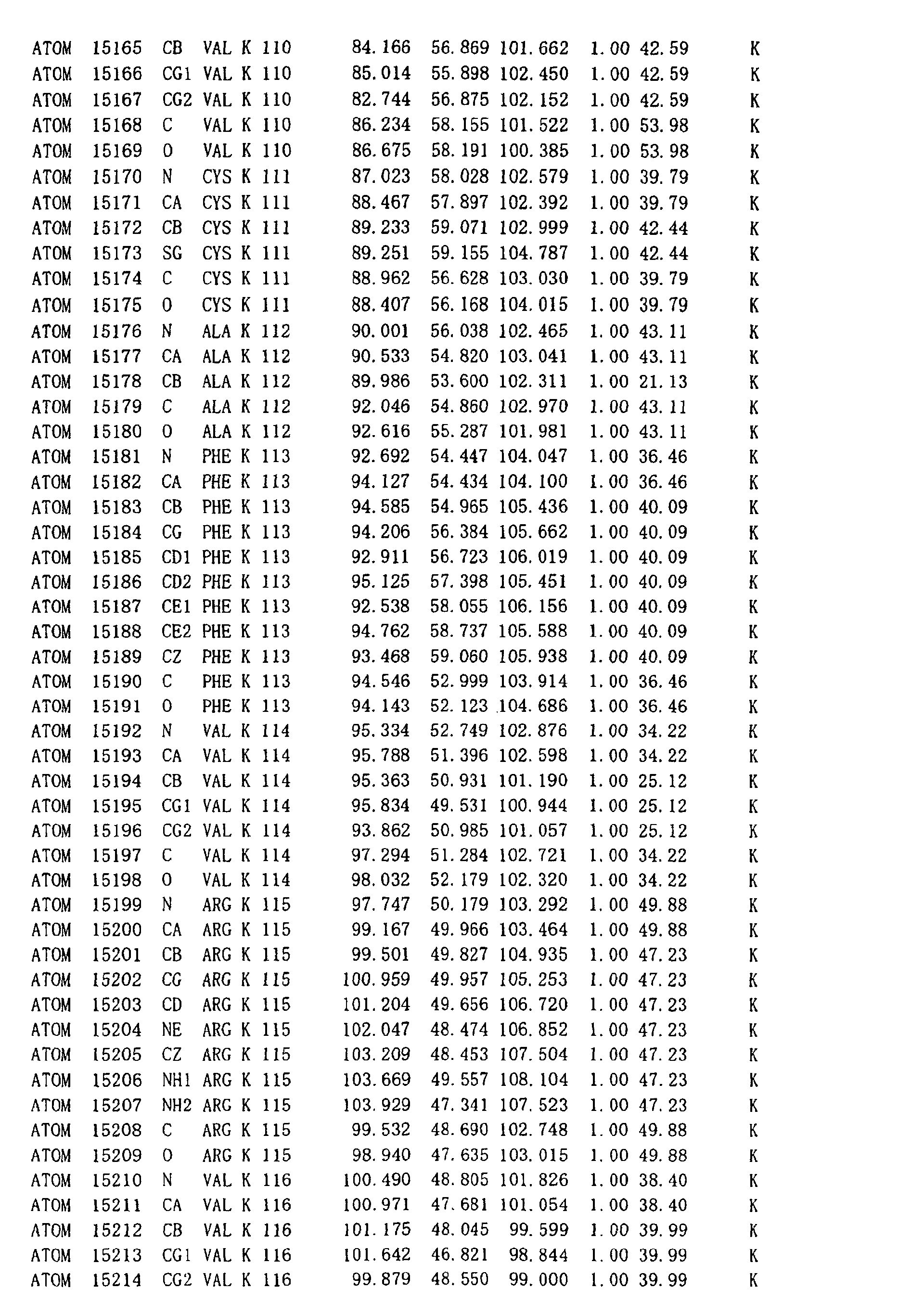

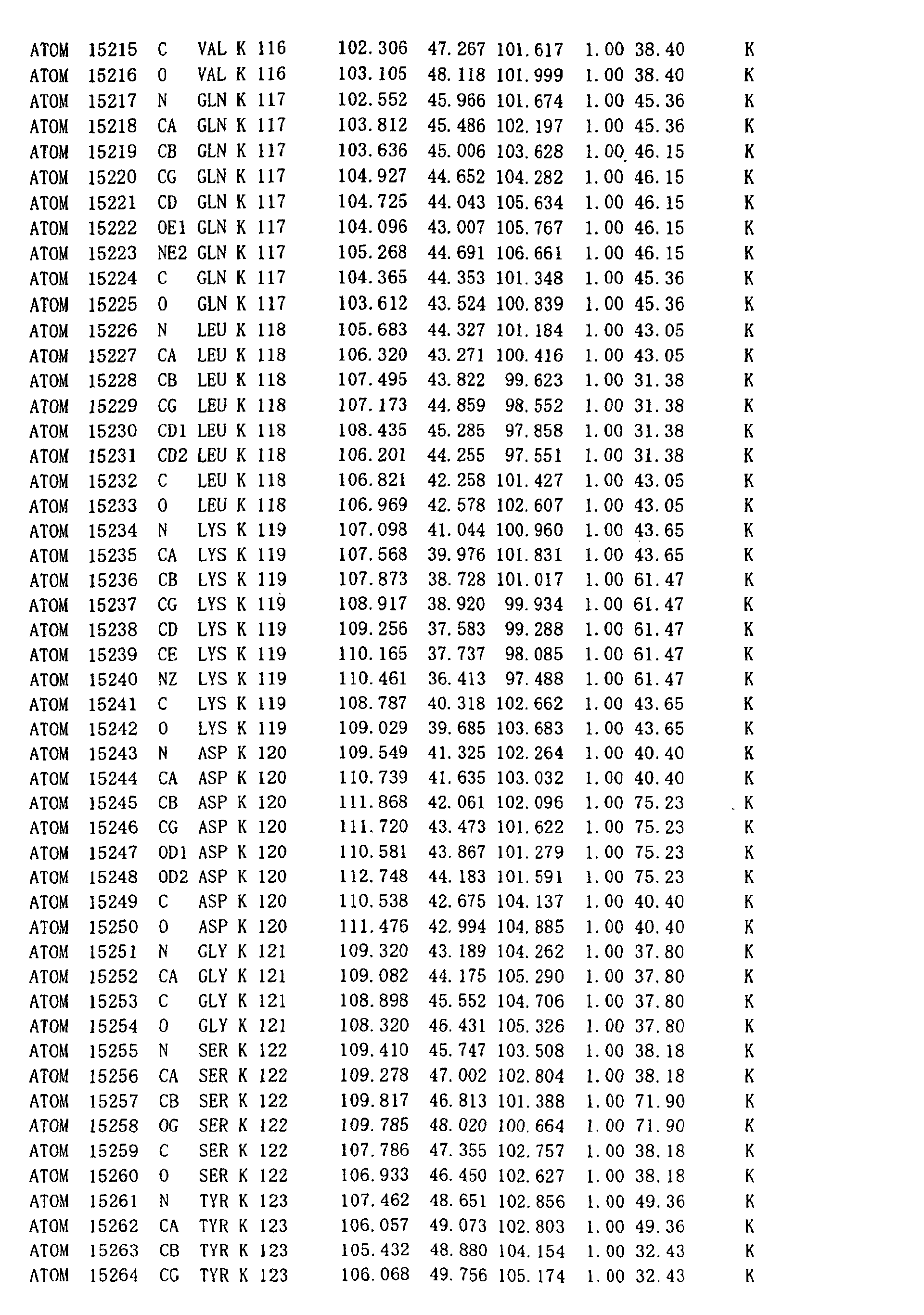

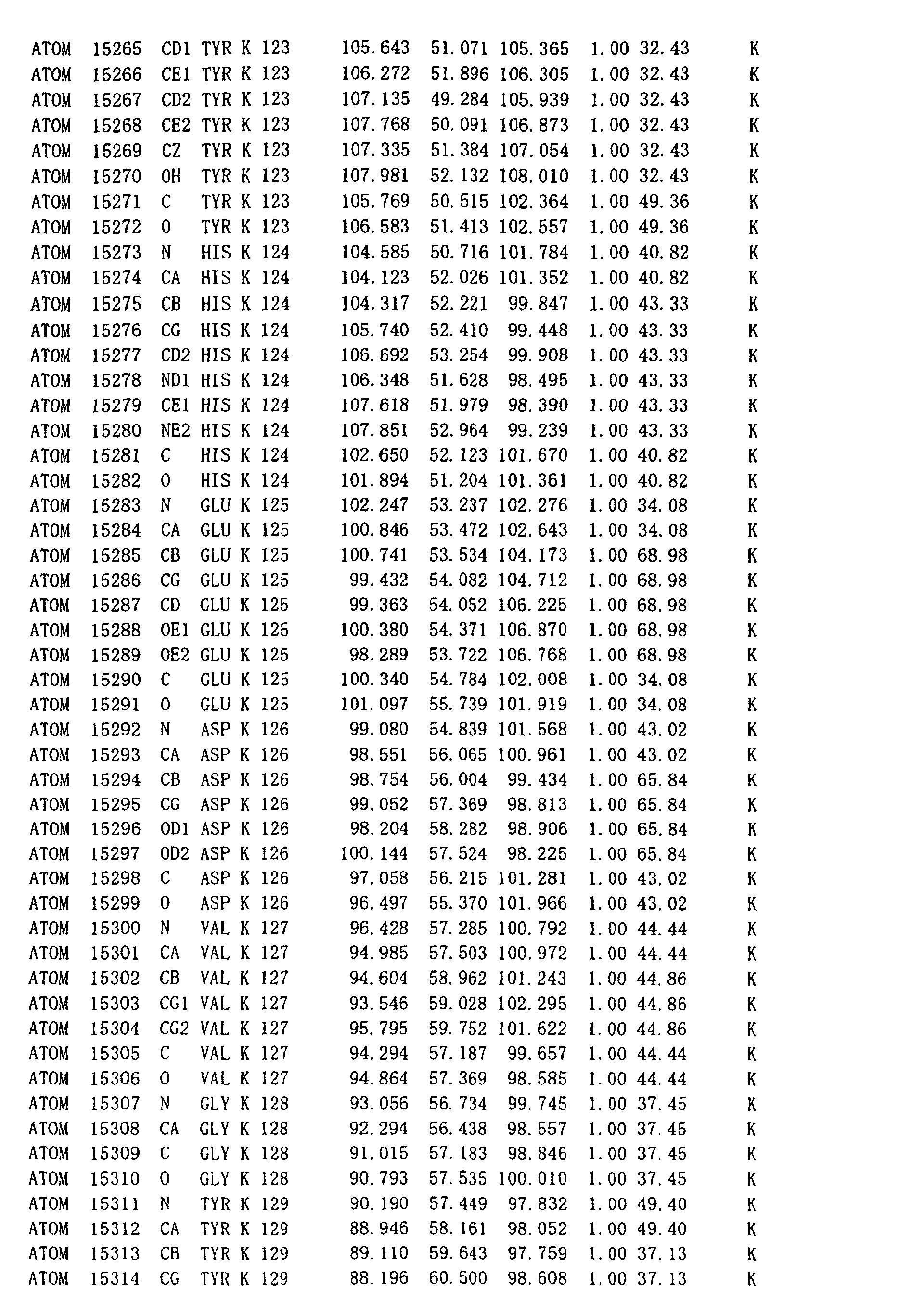

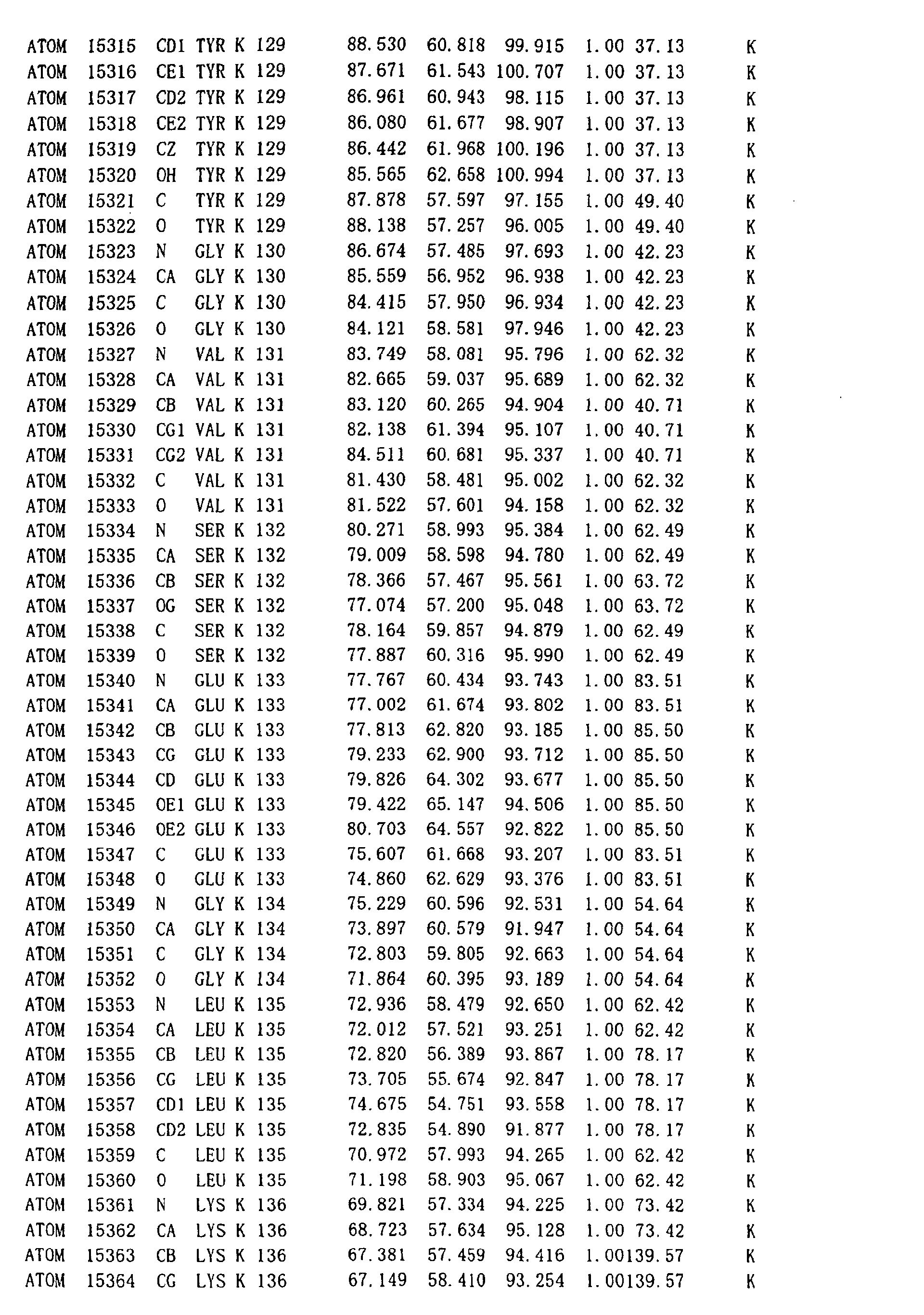

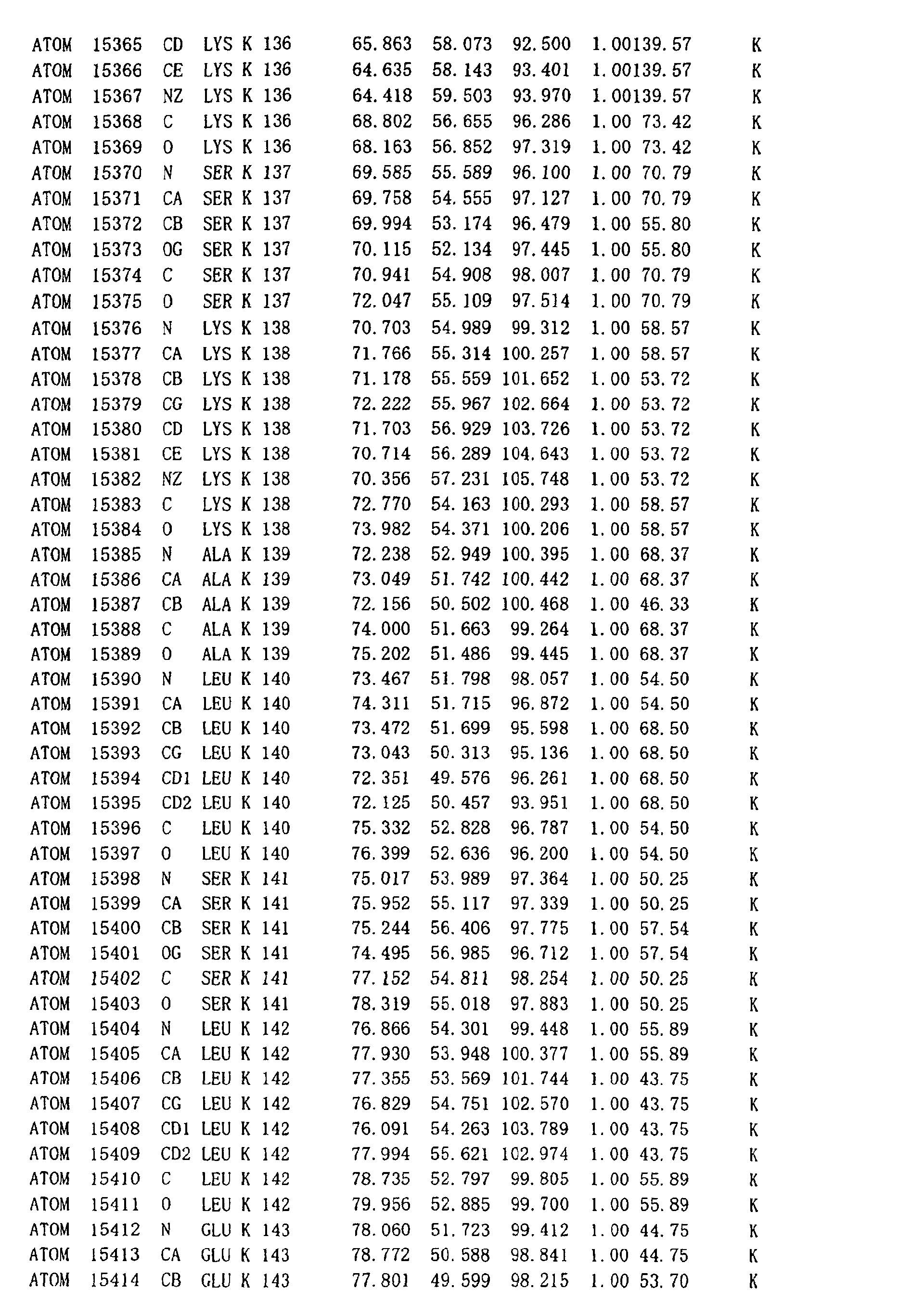

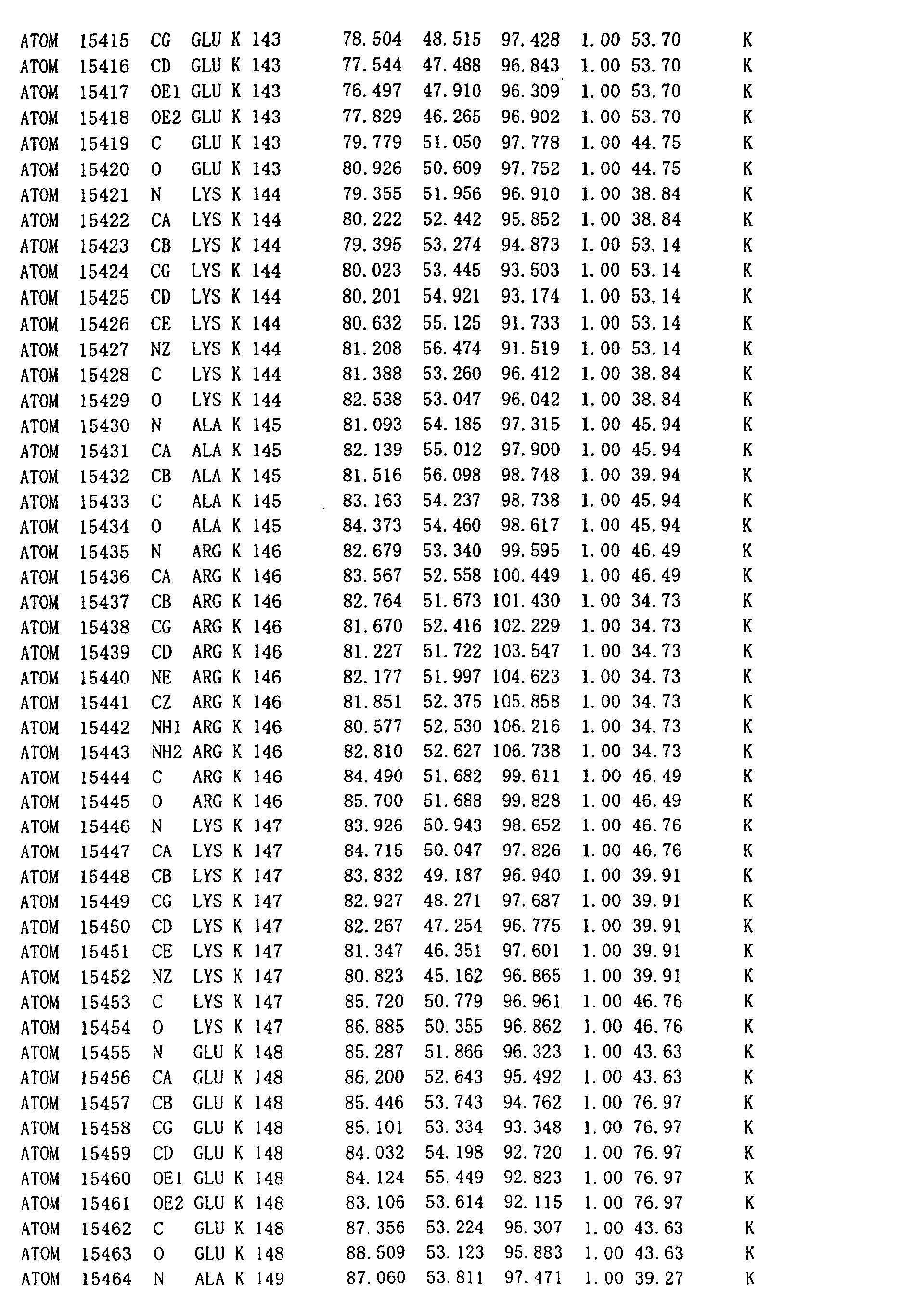

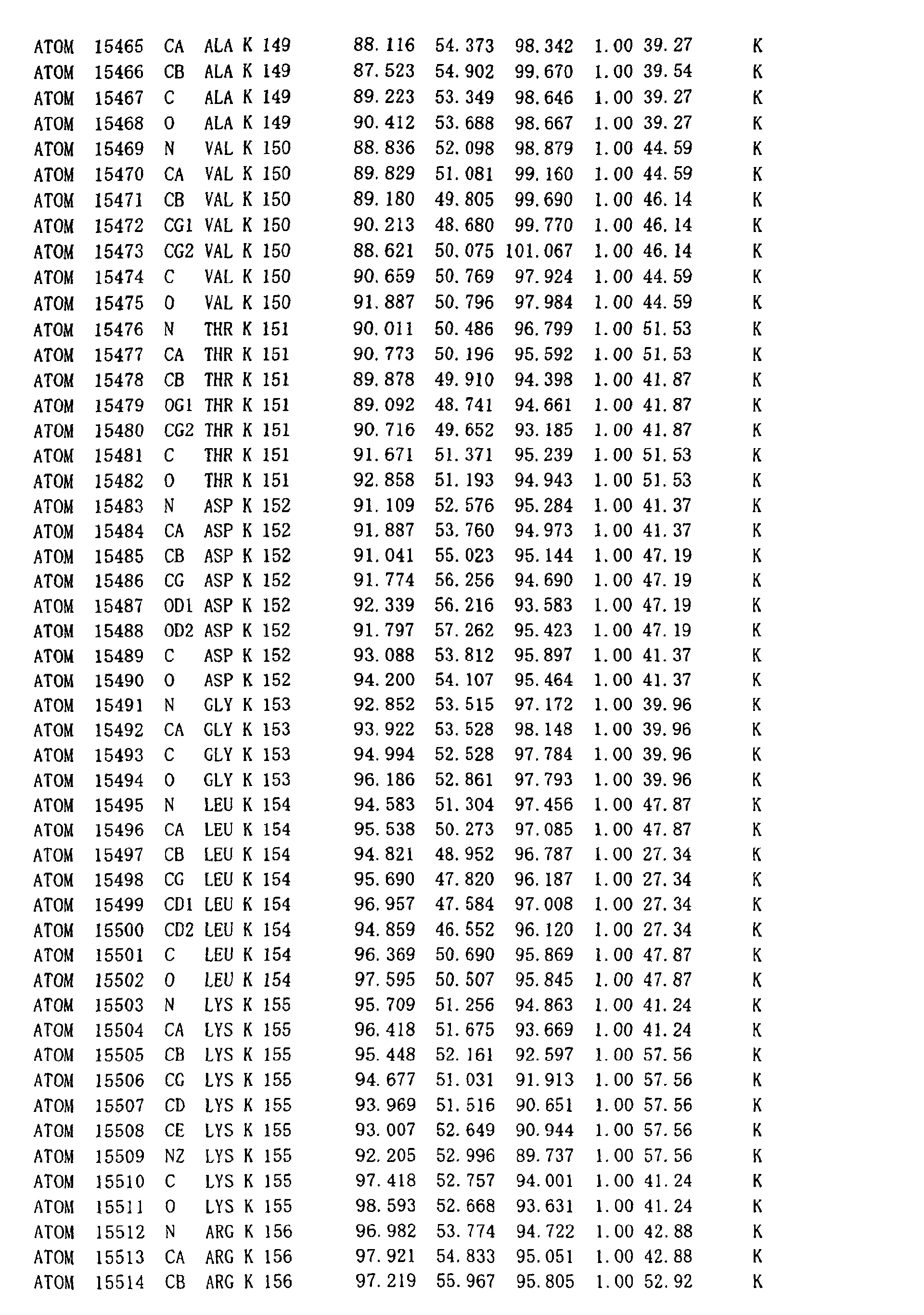

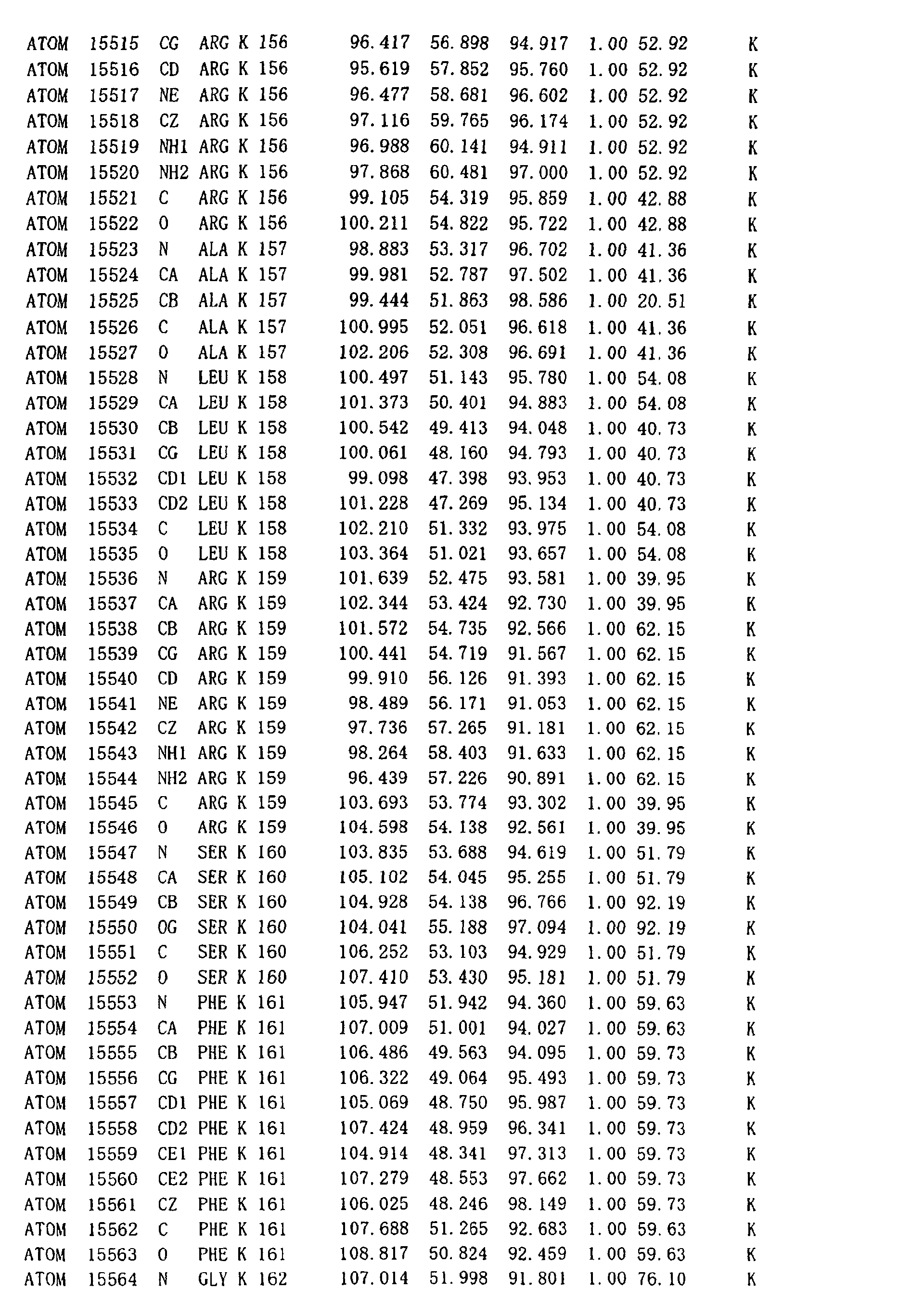

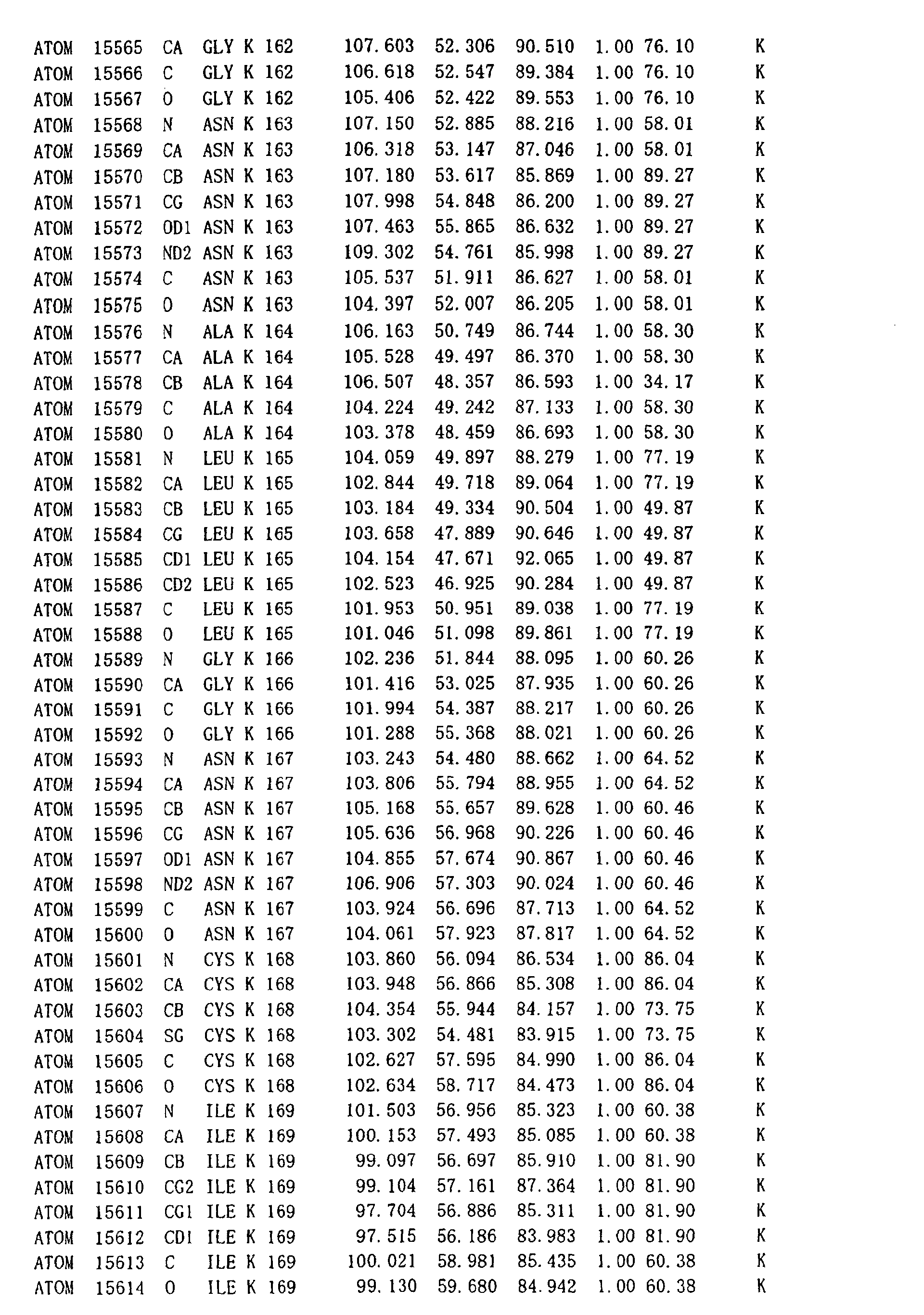

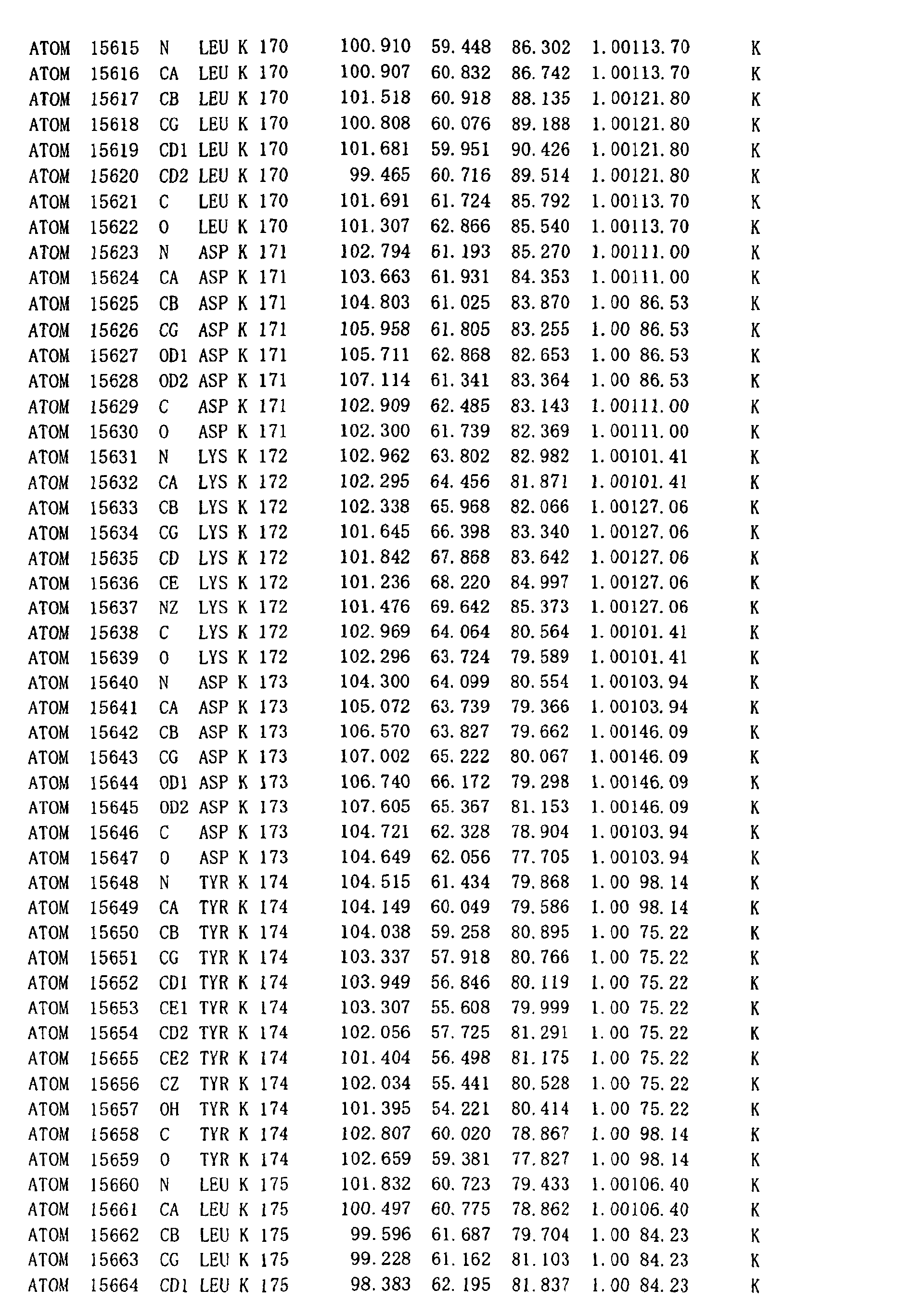

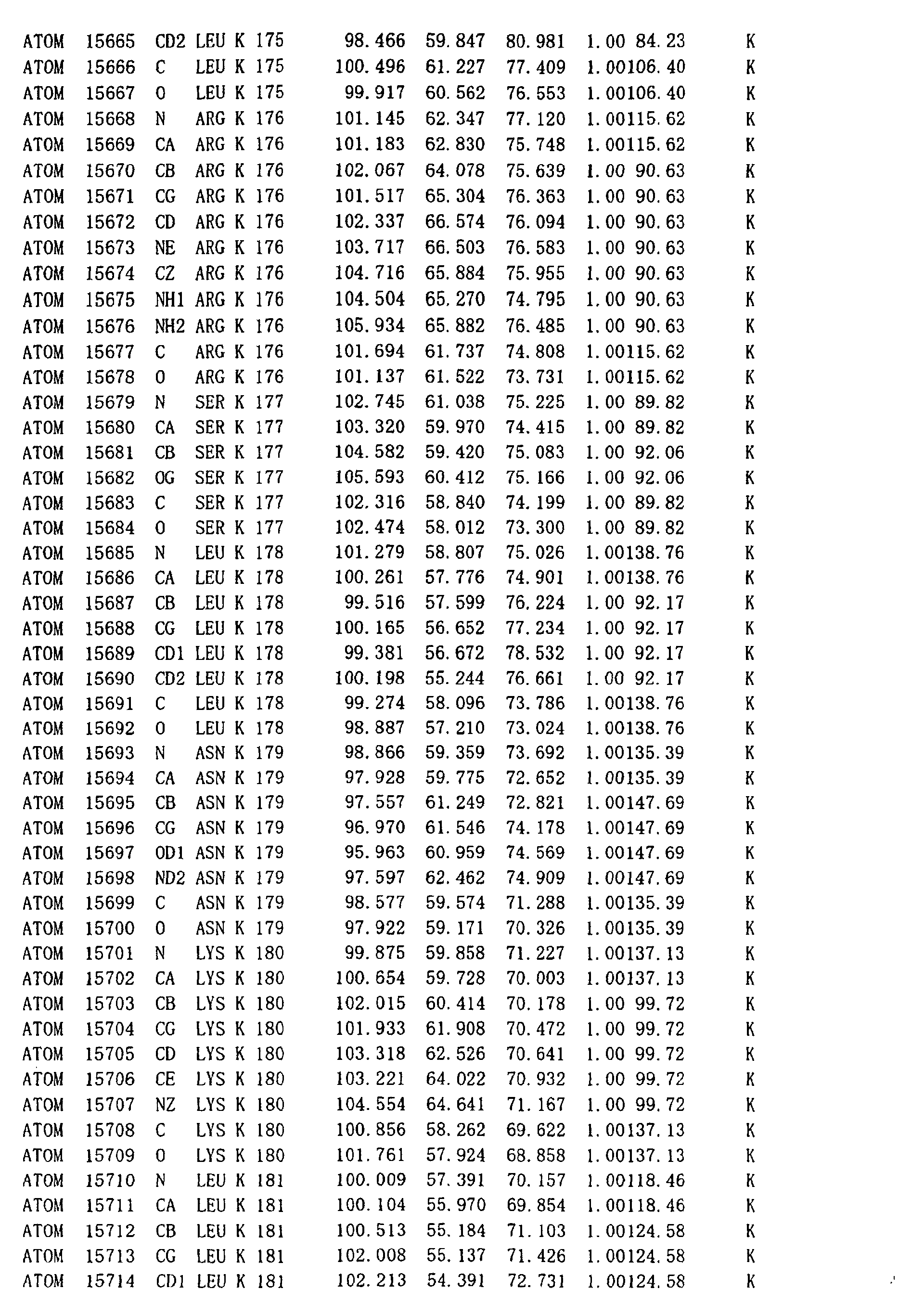

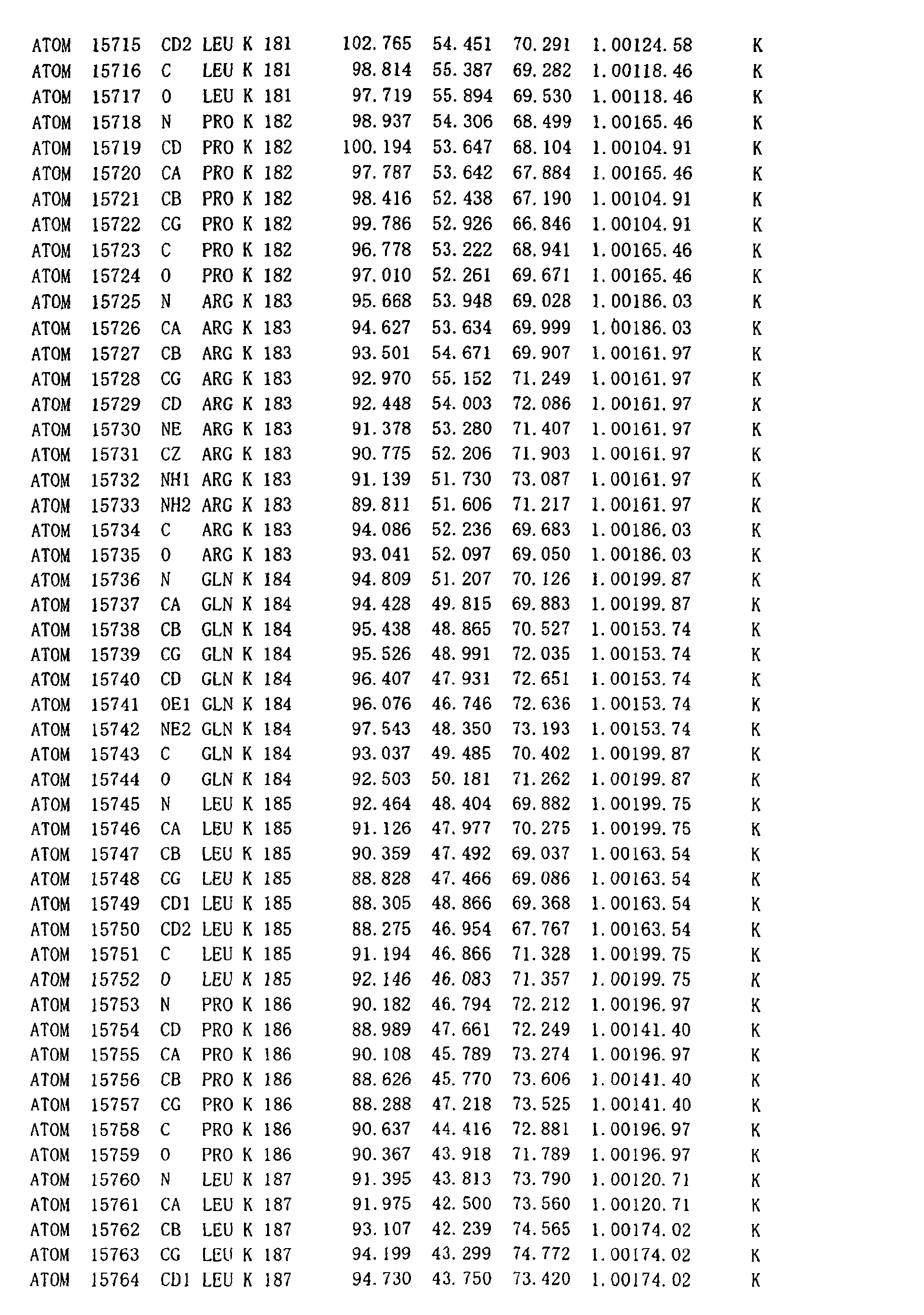

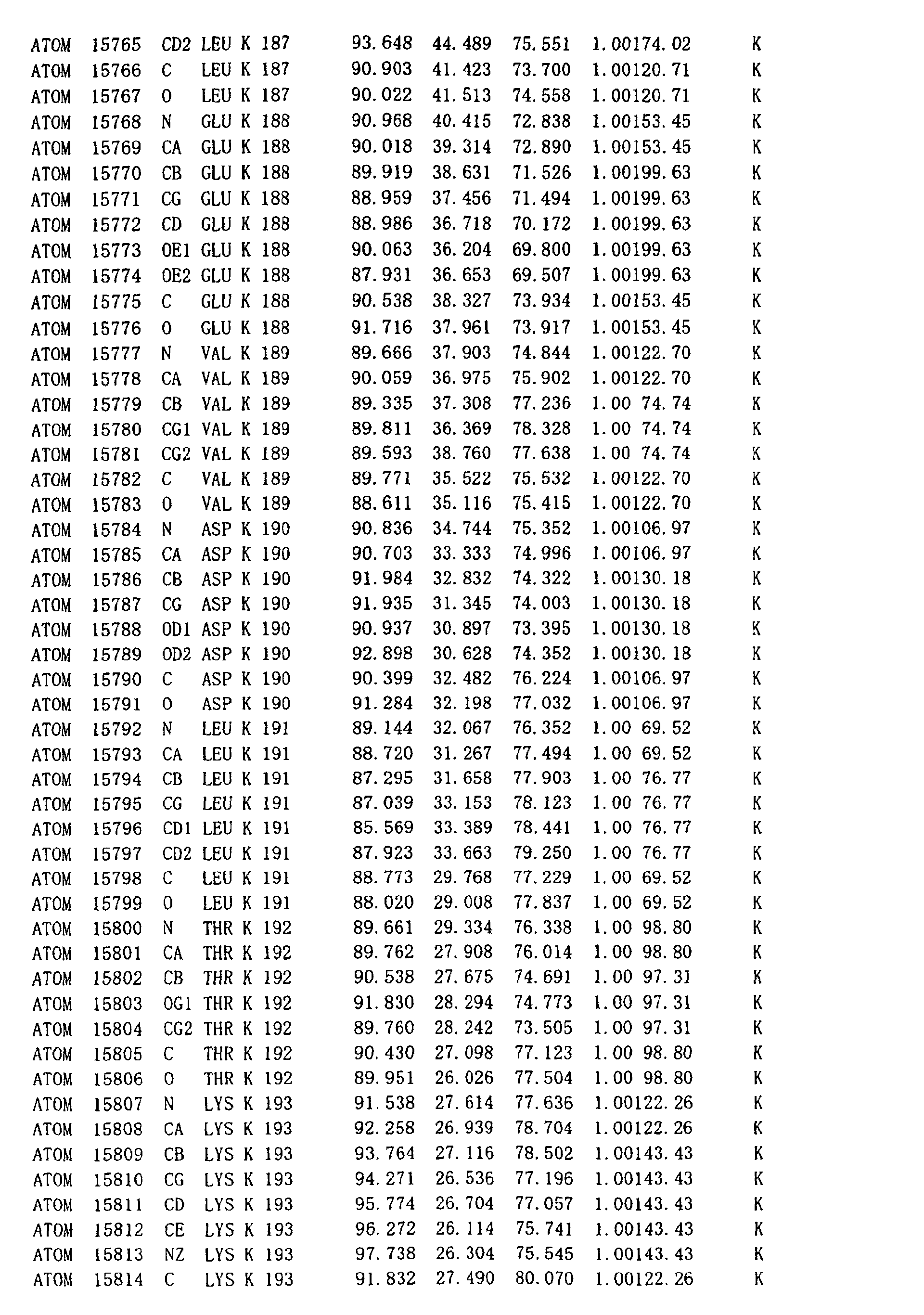

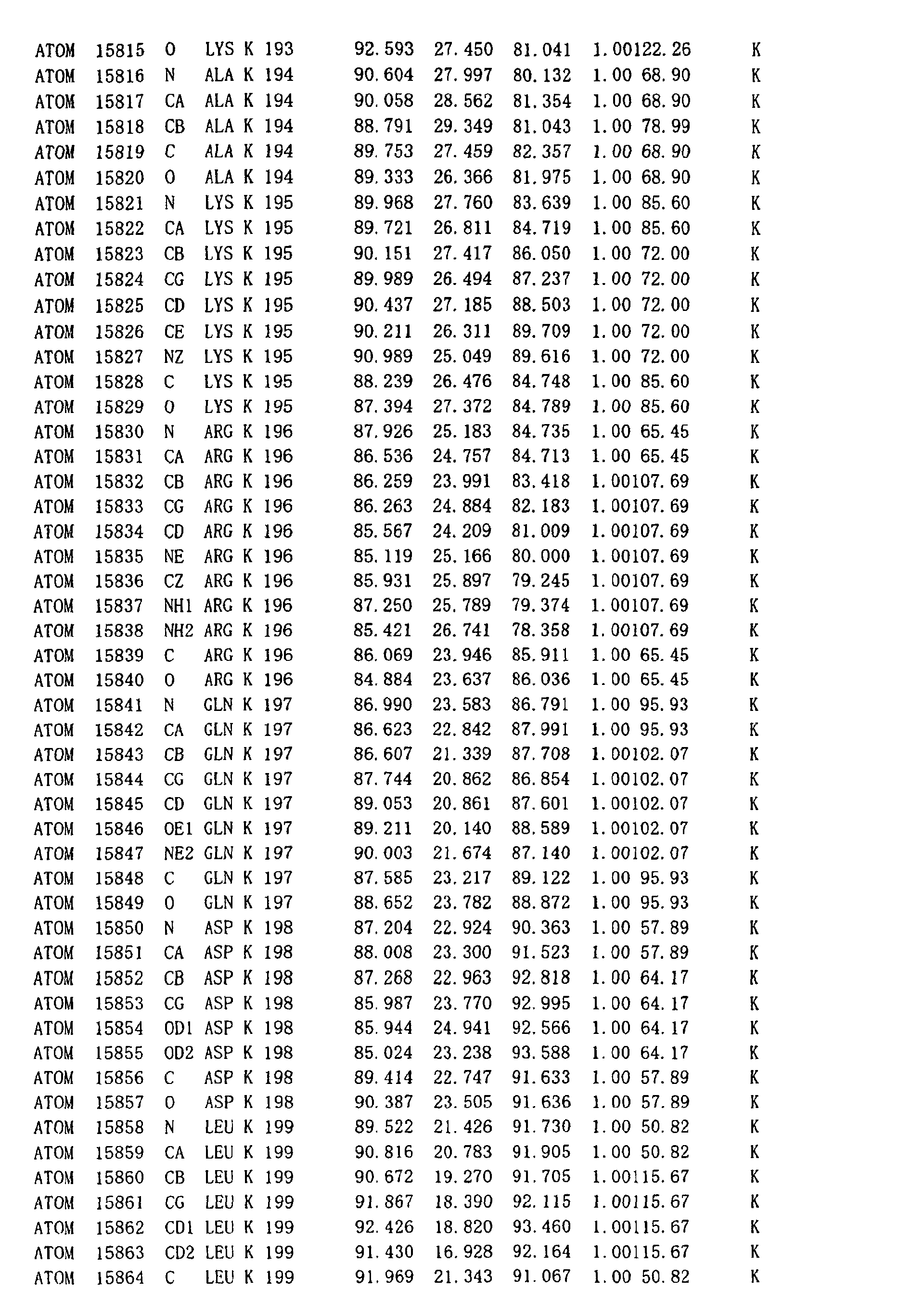

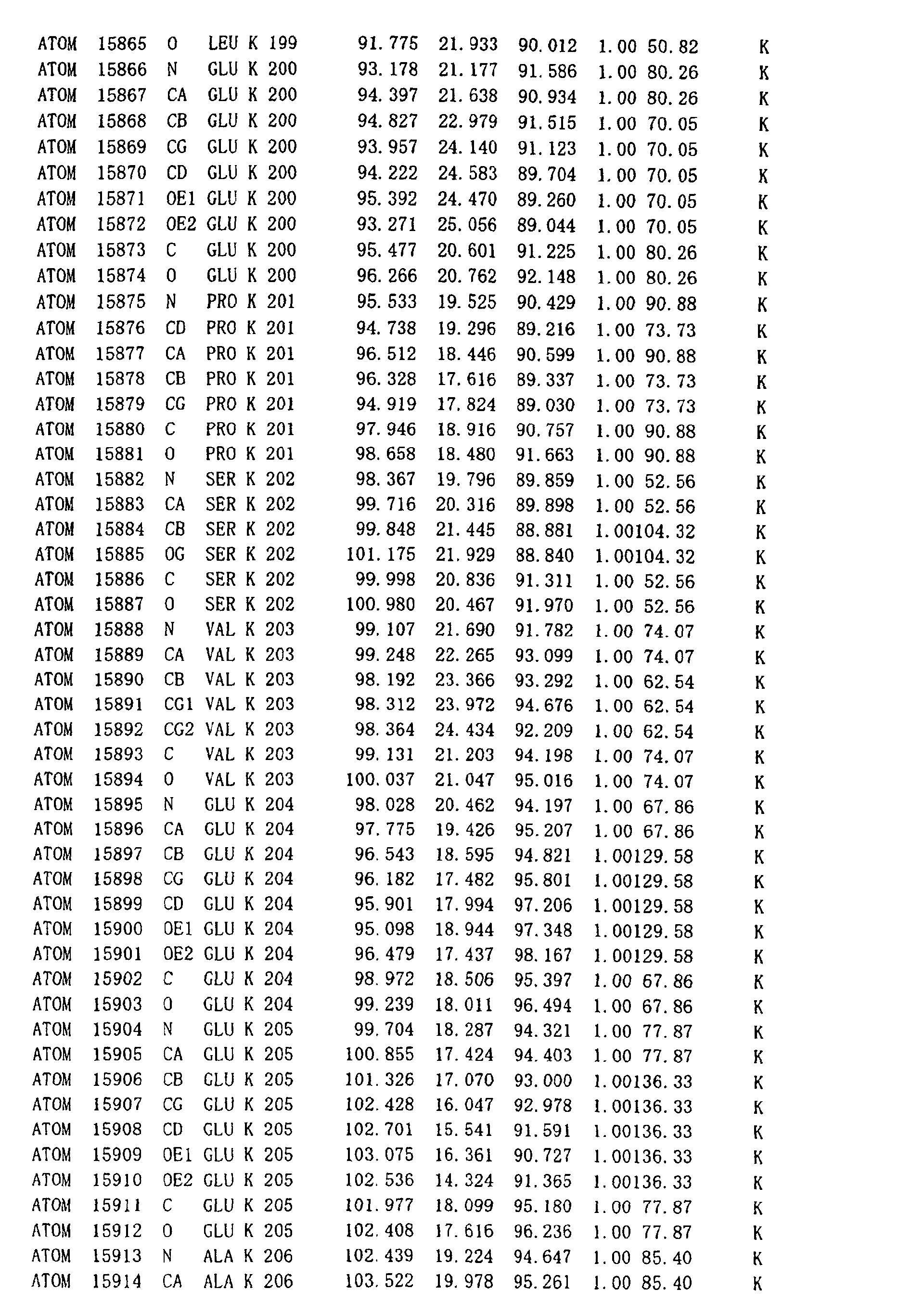

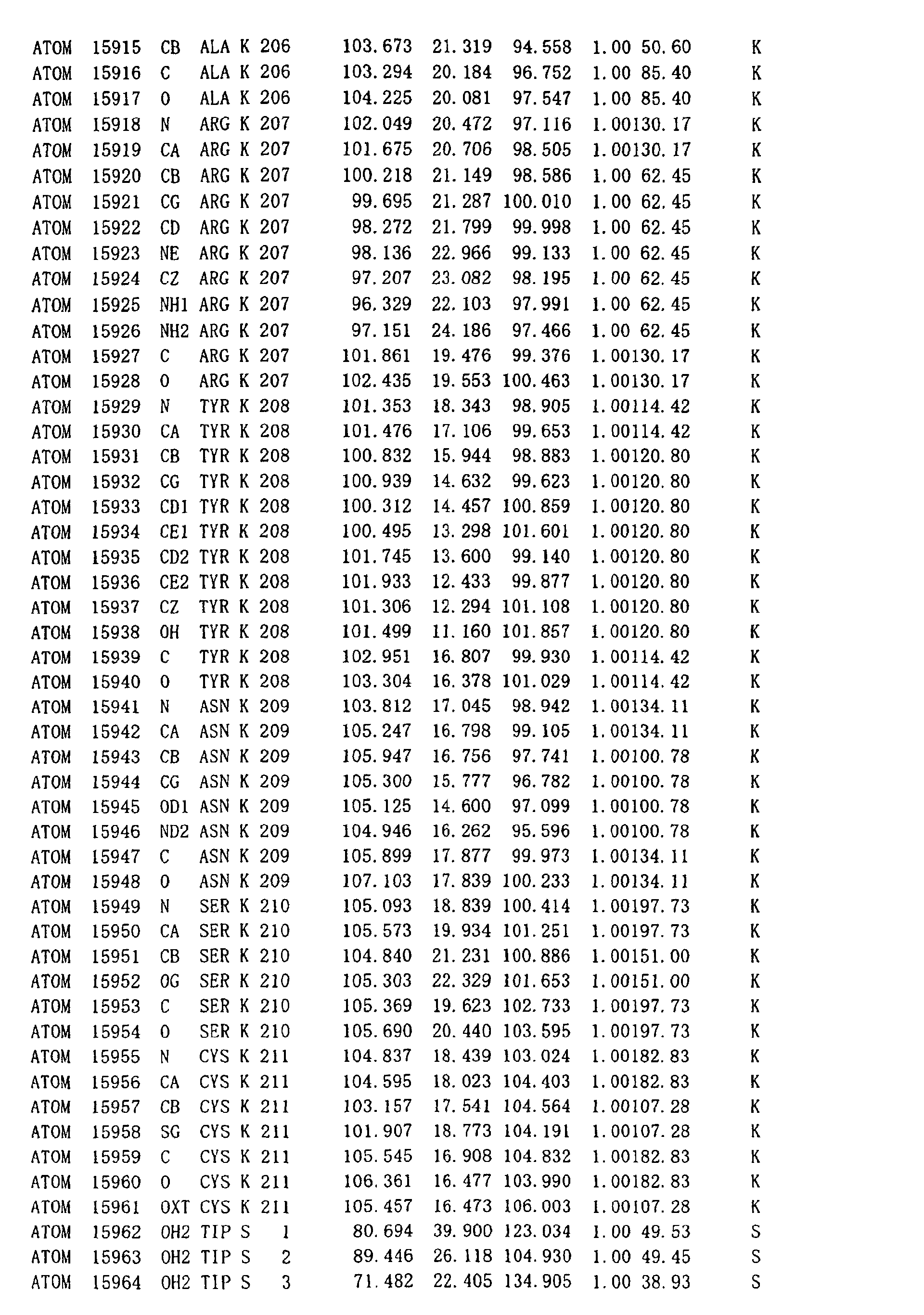

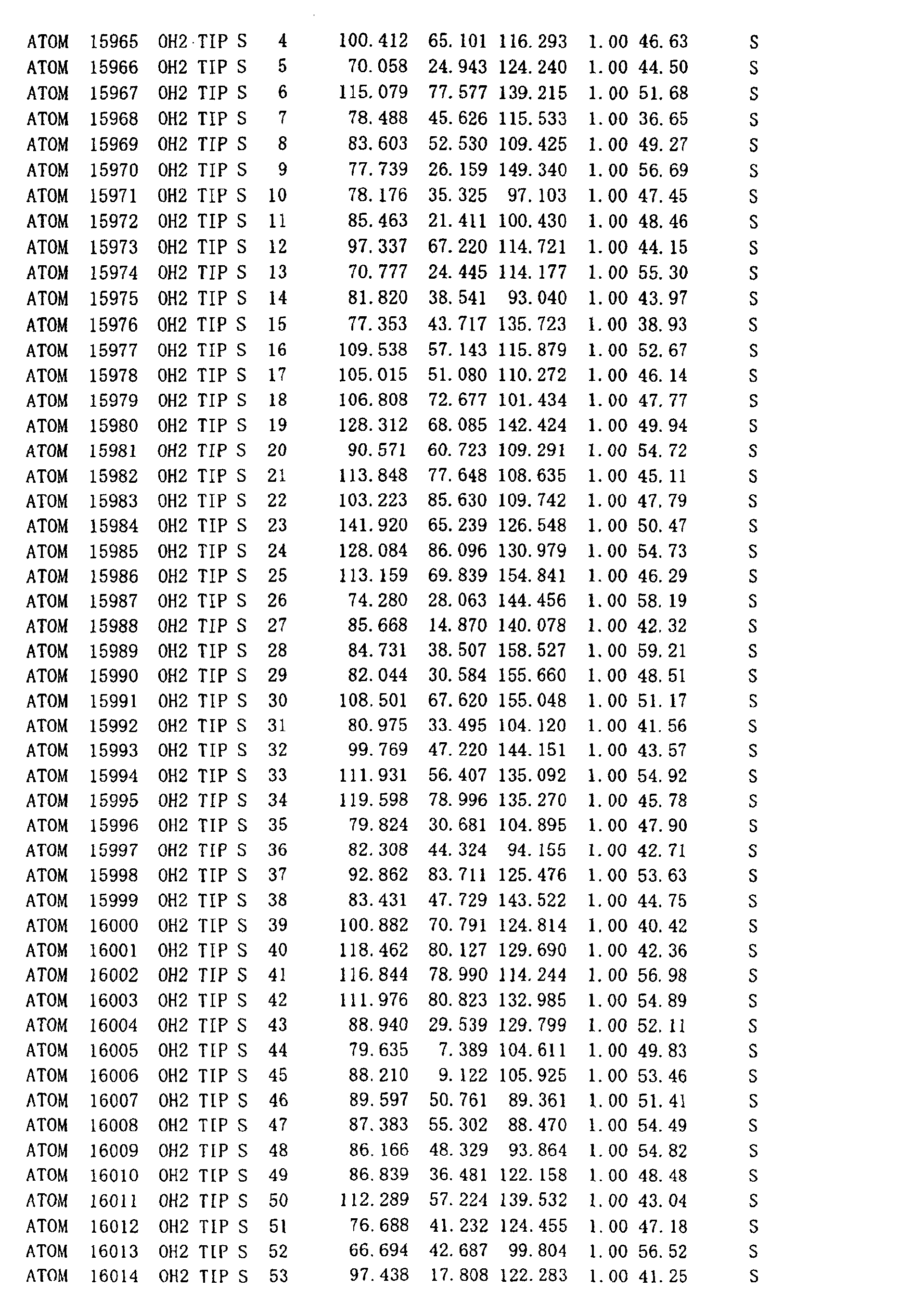

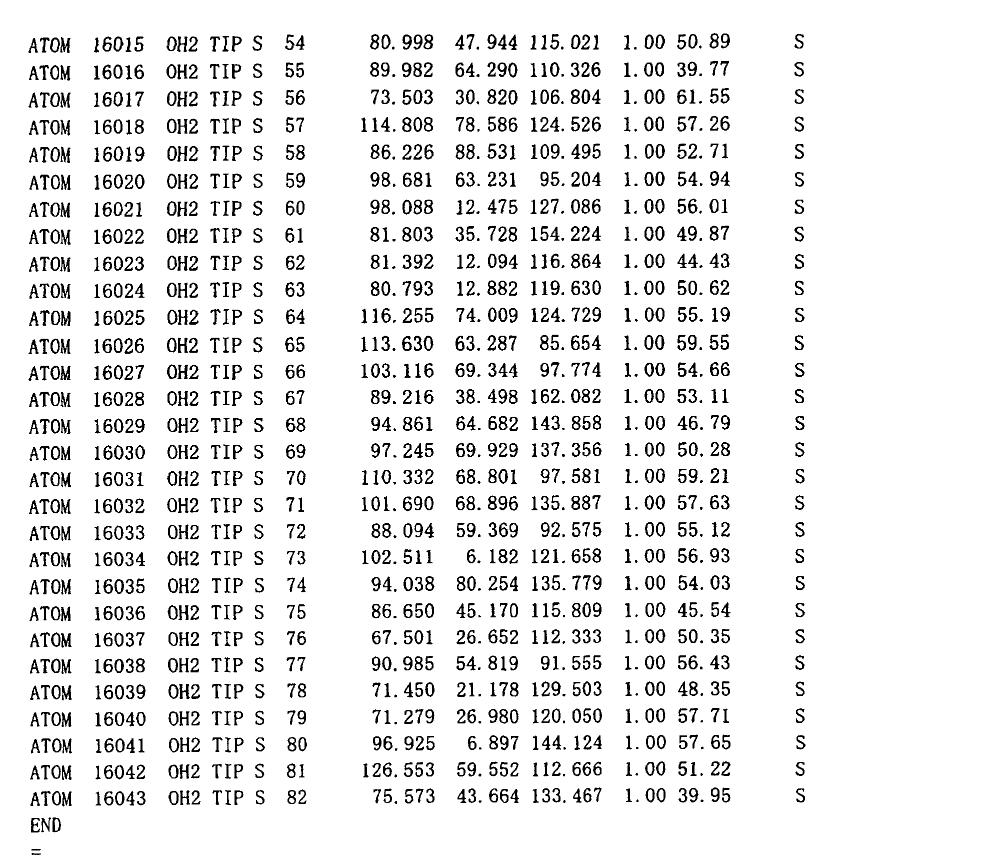

Herein, the atomic coordinates are expressed using the origin of unit lattice in the crystal as the origin in the three-dimensional space. In case that the atomic coordinates of the invention are used for computer calculation, new atomic coordinates obtained as the consequence of mathematic transfer procedures for translation, rotation and symmetry without changing relative allocation of each atom in three-dimensional space are also within the scope of the invention. (Computer system displaying three-dimensional structure model of HsRad52 protein complex)

By inputting the atomic coordinates obtained from the crystal of the DNA recombination/repair protein complex in accordance with the invention into a computer operating a computer program expressing three-dimensional molecule structure or the memory medium of the computer, the three-dimensional structure of the DNA recombination/repair protein complex can be represented in detail.

As the memory medium of computer, any memory medium capable of introducing the atomic coordinates of the DNA recombination/repair protein complex onto the program of the computer is satisfactory, with no specific limitation. The memory medium includes for example electric temporary memory medium called memory and semi-permanent memory media such as flexible disk, hard disk, photodisk, photomagnetic disk and magnetic tape.

A three-dimensional structure model expressing at least a part of the DNA recombination/repair protein complex can be generated by a processor in electric communication with the memory or memory media. As such processor (program), for example, there can be used QUANTA (Molecular Simulations Co. , Ltd.), SYBYL (Molecular Modeling Software, Co., Ltd.), AMBER (Weiner, S. J., etal., (1984) J. Am. Chem. Soc., 106, 765-784) orCHARMM (Brooks, B.R. etal., (1983) J. Comp. Chem. 4, 187-217).

As a program for calculating the coordination energy of the complex and minimizing the energy following the step for generating these molecular models, further, the CHARMM and AMBER and the like are used.

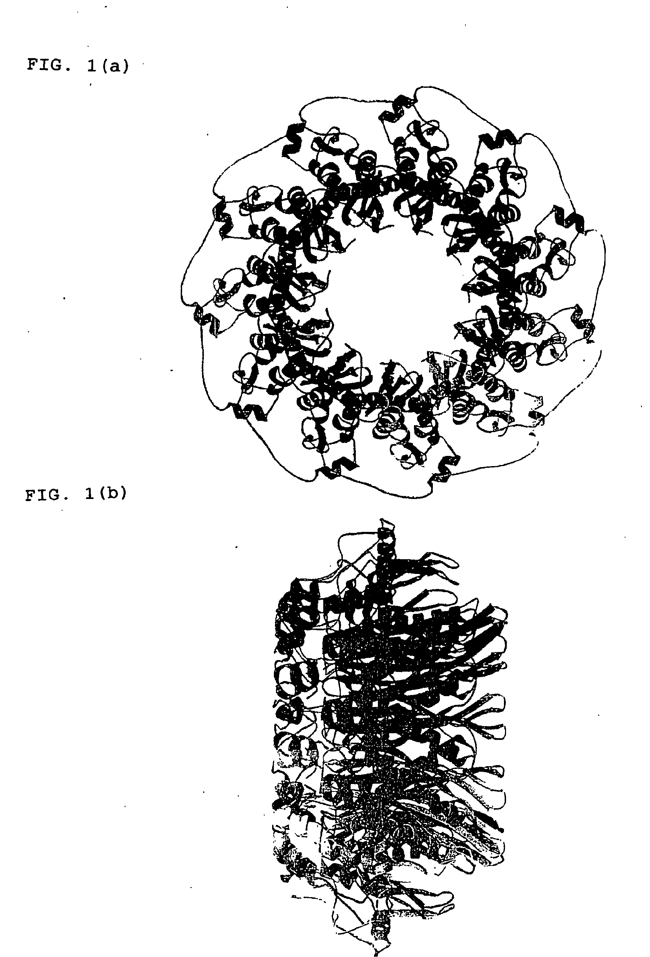

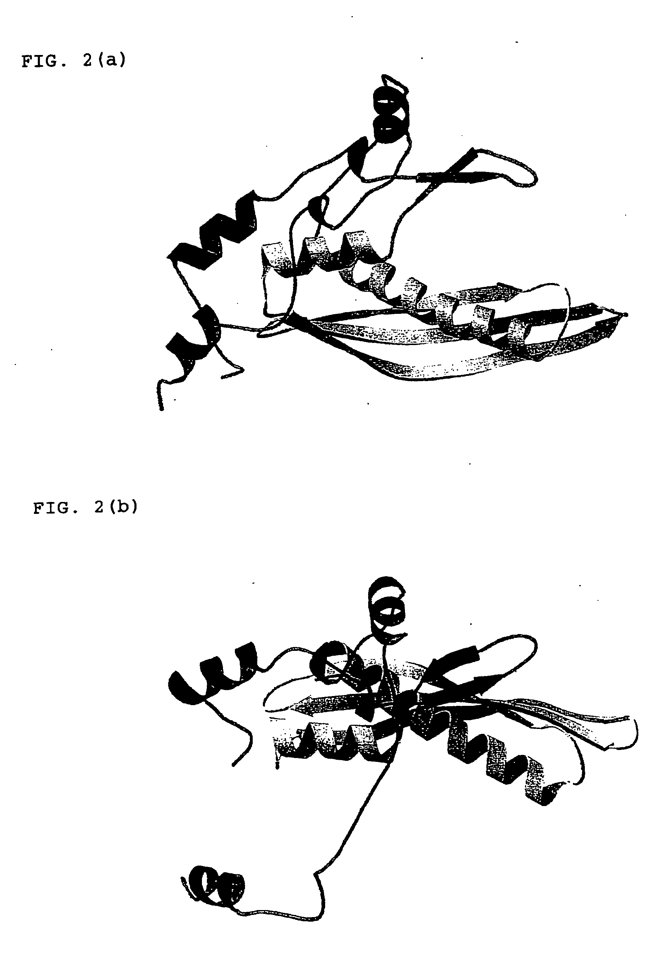

Fig. 1 shows the three-dimensional structure of the DNA recombination/repair protein complex, namely the HsRad52₁₋₂₁₂ complex obtained in such manner at a resolution of 2.8 angstroms in a ribbon model. As apparently shown in Fig. 1, it was found that the crystal of the HsRad52₁₋₂₁₂ complex formed a ring structure through the assembly of the monomer protein of 212 amino acid residues. Because it is supposed that the remaining amino acid residues at the side of C terminus are not directly involved in the formation of the complex, even the full-length HsRad52 in living biological organisms forms the undecameric complex and is therefore encompassed within the scope of the invention. The three-dimensional structure of the monomer composing the complex is shown in Fig. 2. The monomer protein of 212 amino acid residues has five α-helixes and five β-sheet structures.

### (Use of three-dimensional structure of DNA recombination/repair protein complex)

The atomic coordinates obtained from the crystal of the DNA recombination/repair protein complex in accordance with the invention are input in a computer operating a computer program representing three-dimensional molecule structure or the memory medium thereof, to do visual examination and/or calculate the conformational energy, which first enables the rational designing in three-dimensional space of a mutant DNA recombination/repair protein with a modification of a desired characteristic property, for example the improvement of catalytic activity and the improvement of protein stability.

One of typical methods for designing a mutant DNA recombination/repair protein includes a step of inputting the three-dimensional atomic coordinates of the DNA recombination/repair protein complex of the invention in a computer or the memory medium thereof, displaying the three-dimensional structure of the protein on the computer display using an appropriate program and examining the structure visually.

The homologous pairing step as the most important step of the DNA recombinational repairing mechanism can be speculated visually to bind DNA in the periphery of the ring structure, based on the three-dimensional structure of the DNA recombination/repair protein complex shown in Fig. 1. By subsequently calculating for example the surface charge of DNA recombination/repair protein complex, amino acid residues binding to DNA on the surface of the complex can be estimated. Because DNA molecule has a negative charge derived from the phosphate diester bond on the surface, amino acid residues binding to the DNA molecule are highly possibly basic or hydrophobic amino acid residues. By calculating the surface charge of the DNA recombination/repair protein complex, these can be identified. The identified individual amino acid residues are replaced to alanine one by one by a method such as site specific amino acid substitution, to analyze the DNA binding site by alanine scanning and the like for assessing the DNA binding activity. As the consequence of such analysis, amino acid residues speculated as important for DNA binding, or amino acid residues in a region close thereto are substituted with other amino acids or are chemically modified, to prepare mutant enzymes. Herein, the term "region close thereto" means a region involved in the electrostatic interaction, hydrophobic interaction, Van der Waals interaction and hydrogen bonding to the amino acid residues, specifically a region within about 5 angstroms (Å).

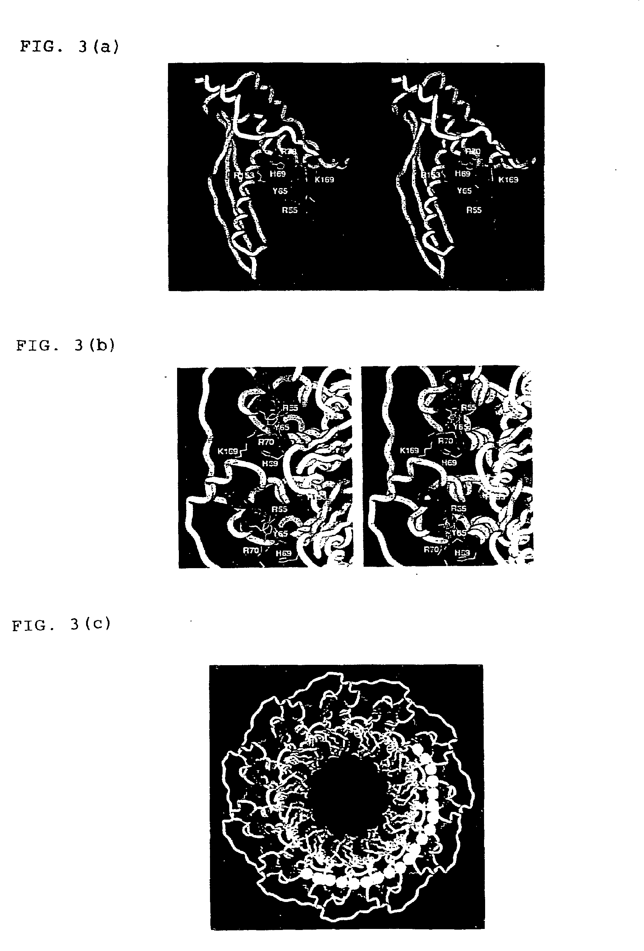

Fig. 3 shows the DNA binding region speculated by such method in the form of three-dimensional structure model. Fig. 3A shows the speculative DNA binding site in the HsRad52 monomer protein. It is shown that the loop region in the vicinity of arginine at position 55 (R55) to arginine at position 70 (R70) and the region in the vicinity of arginine at position 153 to lysine at position 169 are close to each other in terms of the three-dimensional structure within the same molecule (Fig. 3A) and is also close to the adjacent molecules (Fig. 3B) . Because the mutant Rad52 where the any of the basic amino acids is substituted with alanine loses the DNA binding activity according to gel shift assay, it is confirmed that these regions are involved in the DNA binding. Fig. 3C schematically shows how the HsRad52 complex interacts with DNA on the ring-like structure thereof. Therefore,it was shown that in the HsRad52₁₋₂₁₂ complex shown in Fig. 1 or Fig. 3C, the complex was bound to DNA on the lobe hanging out of the ring. The invention includes a polypeptide fragment comprising the amino acid sequence from the position 1 at least up to the position 192 and having a DNA recombinational repairing activity, which can form the DNA binding site.

A mutant enzyme can be designed by deducing the binding between the DNA recombination/repair protein complex and DNA on the basis of the speculation from the coordination energy calculated by a computer. Generally, a three-dimensional protein structure when given enables the calculation of the coordination energy (potential energy). As such energy calculation system, programs such as ECEPP, CHARMM and AMBER can widely be used. As factors composing the coordination energy, for example, binding potential, electrostatic potential, Van der Waals potential, and hydrogen binding potential exist. Once the coordination energy is calculated, the three-dimensional structure can be deduced when a specific amino acid residue is substituted or the like. The optimized structure for these mutant complexes to bind to DNA can be analyzed or deduced.

The mutant designed in accordance with the invention may be prepared by many methods. For example, DNA encoding the mutant of the invention can be prepared by a method known to a person skilled in the art such as site specific mutagenesis, PCR and chemical synthesis or combinations thereof. By introducing DNA encoding these mutant enzymes into an appropriate expression vector and introducing further the expression vector into an appropriate cell to culture the resulting transformant, the mutant enzymes can be expressed.

As such expression vector, various vectors can be used as long as the vectors can be amplified in host cells. When the host is *Escherichia coli,* for example, plasmid vectors with promoter/operator systems such as tac, λPL and T7 having high transcription activity and enabling the induction are used. Specifically, such plasmid vectors include the pKK233-3 (Brosius, J., and Holy, A. (1984) Proc. Natl. Acad. Sci. USA, 81, 6929) and pGEX-1 (Smith, D. B. and Johnson, K. S., (1988) Gene 67, 31-40) with tac promoter and pET-3 (Rosenberg, A. H., *et al.,* (1987) Gene 56, p.125-35) with T7 promoter. In case that the host is bakers' yeast, for example, there is a shuttle vector pAM82 (Miyanohara, A., et al., (1983) Proc. Natl. Acad. Sci. USA, 80,1) usable in both bakers' yeast and *E. coli .* In case that the host is an insect cell, baculovirus vector pBacPAK8/9 (Clontech) and the like are used. In case that the host is an animal cell such as Chinese hamster ovary cell CHO or human HeLa cell, vectors such as pMSG (Amersham Pharmacia) and pcDNA (Invitrogen) are used.

By inputting all or a part of the atomic coordinates of the DNA recombination/repair protein complex provided in accordance with the invention into a computer operating a computer program expressing the three-dimensional molecular structure or the memory medium thereof, a compound binding to the DNA recombination/repair protein complex to modulate the DNA recombinational repairing activity can be identified, screened, assessed or designed. The compound may be any of natural compounds and synthetic compounds or any of high polymer or low molecular compounds.

The method includes a step of displaying a candidate compound and the DNA recombination/repair protein complex according to a computer model and screening a candidate compound (lead compound) in a form or with a chemical structure well suitable for a specific site of the DNA recombination/repair protein complex under visual observation. To prepare or display the molecular model, programs such as QUANTA and SYBYL describedabovecanbeused. Further, various computer programs to estimate the affinity to a specific site of the DNA recombination/repair protein complex, repulsion and steric hindrance and the like can be used. Examples of these programs include GRID (Goodford, P.J., (1985) J. Med. Chem., 28, p. 849-857) to determine a suitable binding site of a biopolymer in terms of energy, MCSS (Miranker, A., etal., (1991) Proteins: Structure, Function and Genetics, 11, p.29-34) to screen functional binding sites, or AUTODOCK (Goodsell, D.S., et al., (1990) PROTEINS: Structure, Function and Genetics, 8, 195-202) to geometrically analyze the interaction between a polymer and a ligand for automatic docking, and DOCK (Kuntz, I.D., et al., (1982) J. Mol. Biol. 161, 269-288). Via calculations using such programs, a region in a specific region of the surface of the DNA recombination/repair protein complex, which can bind to single-stranded or double-stranded DNA in a stable manner in terms of energy, can be deduced, to indicate a candidate compound three-dimensionally complementary to the region.

When such candidate compound (lead compound) is found, continuously, the steric interaction with the candidate compound can be examined visually, for further optimization of a computer display imaging the three-dimensional structure of the DNA recombination/repair protein complex. Using programs such as QUANTA and SYBYL described above, a model for optimizing the candidate compound can be constructed. Besides, various programs useful for the optimization of molecular modeling can be used, including for example CAVEAT (Lauri, G., and Bartlett, P.A., J. (1994) Comput. Aided Mol. Des. 8, 51-66) andHOOK (Eisen, M. B., etal., (1994) Proteins: Struct., Funct., Genet., 19, 199-221).

In addition to the programs, further, a candidate compound can automatically be constructed *de novo* from the three-dimensional structure of the DNA recombination/repair protein complex to output the structure. For them, programs such as LUDI (Bohm, H. J., (1992) J. Comp. Aid. Molec. Design.) and LEGEND (Nishibata, Y., et al. , (1991) Tetrahedron 47, 8985) can be used.

The compounds thus designed or selected are contacted with the DNA recombination/repair protein, to determine whether or not the compound binds to the DNA recombination/repair protein. The candidate compound canbe obtainedusing a chemical synthesis method for general use or from compounds from natural origins. It can be determined by various methods, whether or not the compound binds to the DNA recombination/repair protein. For example, binding to single- stranded DNA or double-stranded DNA, formation of ternary complex with single-stranded DNA or double-stranded DNA, and D-loop formation are observed by gel shift assay. These reactions are essentially based on a reaction of binding of the DNA recombination/repair protein to first single- stranded DNA and annealing with double-stranded DNA containing a nucleotide sequence complementary to the single-stranded DNA. The complex formed in such manner can be detected by various detection methods such as radioactive labeling, using gel electrophoresis.

Otherwise, the interaction of the compound with the DNA recombination/repair protein can be spectrophotometrically detected, using NMR and mass spectrometry. Generally, NMR signal sensitively reflects the local molecular environmental change (ligand binding, structural change and the like). In case that NMR spectrum is measured by mixing a protein interacting with a ligand, therefore, it can be confirmed that the ligand signal gets wider or disappears or the chemical shift values change. In contrast, the structure of protein bound to the ligand can also be detected in a specific manner. When ¹⁵N-¹H HSQC spectrum using a protein uniformly labeled with ¹⁵N, only the NMR signals of the protein can be observed.

The compound modulating the DNA recombinational repairing activity thus obtained may be an inhibitor or activator of the DNA recombinational repairing reaction specific to Rad52. The inhibitor can be used for assaying Rad52-specific activity. For example, mixing the compound with a sample such as cell extract solution to completely inhibit the homologous recombination activity of Rad52, subsequently assaying the DNA recombinational repairing activity, and calculating the difference between the activity and the DNA recombinational repairing activity measured under conditions where the inhibitor is removed, the DNA recombinational repairing activity specific to Rad52 can be calculated.

In case of using the inhibitor as a therapeutic agent of cancer, for example an enhancer for radiotherapy and chemotherapy, the inhibitor is mixed with a carrier for general use, to prepare a pharmaceutical composition. The carrier for pharmaceutical compositions includes for example but is not limited to lactose, glucose, D-mannose, starch, crystal cellulose, calcium carbonate, kaolin, gelatin, hydroxypropyl alcohol, polyvinylpyrrolidone, ethanol, carboxymethyl cellulose, carboxymethyl cellulose sodium salt, magnesium stearate, talc, acetyl cellulose, purified sugar, titanium oxide, benzoic acid, p-oxybenzoate ester, sodium dehydroacetate, gum arabic, tragacanth, methylcellulose, egg yolk, surfactants, simple syrup, citric acid, distilled water, glycerin, propylene glycol, macrogol, phosphate monohydrogen sodium, phosphate dihydrogen sodium, sodium phosphate and sodium chloride. Depending on the intended formulation, for example, injection and tablet, the carrier is mixed with the inhibitor identified by the method of the invention.

### EXAMPLES

The present invention is explained in more detail by reference to the following Examples, however, they do not restrict the scope of the present invention.

### (Example 1)

### Limited proteolysis of HsRad52

5 µl of HsRad52 protein (1 to 3 mg/ml) prepared by a known method (Kagawa *et al.,* J. Biol. Chem., 2001, 276, 35201-8) was mixed with 5 µl of proteinase K (30 µl/ml ) and the mixture was incubated at 25°C for 4 to 6 hours. The proteinase K solution was diluted in 10 mM Tris-HCl solution, pH 7.5. The reaction mixture was fractionated by 12% SDS polyacrylamide gel electrophoresis (SDS-PAGE), and the resulting protein bands were transferred to a PVDF membrane using a semi-wet blotting apparatus (Bio-Rad). Proteolytic bands were visually detected by staining with a Coomassie Brilliant Blue staining solution, followed by destaining of the membrane with methanol. The protein was eluted from a portion of the PVDFmembrane containing the protein bands, to determine the N-terminal amino acid sequence. To measurethe molecular weightby massspectrometry, the reaction mixture containing proteolyzed HsRad52 was treated with 1 µl of 2N HCl to stop further proteolysis by proteinase K. The reaction mixture was diluted with water to 100 fold, for measurement with a mass spectrometry apparatus. As a consequence of them, the region from the N terminus to position 237 of HsRad52, namely HsRad52₁₋₂₃₇ was found resistant to the limited proteolysis by proteinase K.

### (Example 2)

### Analysis of HsRad52₁₋₂₃₇ by deletion

Plural deletion mutants of HsRad52₁₋₂₃₇ as prepared by deleting sequentially every 5 amino acid residues from the C terminus thereof, namely HsRad52₁₋₂₃₂, HsRad52₁₋₂₂₇, HsRad52₁₋₂₂₂, HsRad52₁₋₂₁₇, HsRad52₁₋₂₁₂, HsRad52₁₋₂₀₇, HsRad52₁₋₂₀₂, HsRad52₁₋₁₉₇, HsRad52₁₋₁₉₂ and HsRad52₁₋₁₈₇ were subjected to a combination of both of crystallization and biochemical experiments. HsRad52₁₋₁₉₂ and deletion mutants larger than the HsRad52₁₋₁₉₂ had absolutely the same DNA binding activity as that of HsRad52₁₋₂₃₇. Among all of the deletion mutants, the highest amount of crystal was obtained from HsRad52₁₋₂₁₂ in a system using a screening kit (Crystal Screens 1 and 2 manufactured by Hampton Research Co. , Ltd.). Thus, it was determined from these results that HsRad52₁₋₂₁₂ was the most suitable for structural analysis.

### (Example 3)

### Purification of HsRad52-₁₋₂₁₂

HsRad52₁₋₂₁₂ was purified by three steps of affinity adsorption on Ni-NTA agarose, hexahistidine- tag cleavage and Heparin-Sepharose column chromatography. A DNA encoding HsRad52₁₋₂₁₂ was cloned in pET15-b (Novagen) , an expression vector transcribed by T7 polymerase. The HsRad52₁₋₂₁₂ protein was expressed at a large scale, using *E. coli* strain JM109 (DE3) together with tRNA^{Arg3} recognizing the CGG codon and RNA^{Arg4} recognizing the AGA/AGG codon. In case that the expression plasmid did not contain the tRNA genes, the expression level of the HsRad52₁₋₂₁₂ protein was extremely low. Generally, the HsRad52₁₋₂₁₂ Protein was purified from a culture of one liter. The recombinant *E*. *coli* was cultured at 30°C, to which IPTG was added to a final concentration of 1 mM in the logarithmic growthphase (OD₆₀₀ = 0.6), to induce the expression of the protein. Subsequently, the bacteria were cultured for 4 to 6 hours to collect the bacterial cells, which were disrupted in a buffer solution A (pH 7.8) containing 50 mM Tris-HCl, 0.8 M KCl, 2 mM 2-mercaptoethanol, 10 mM imidazole, 10% glycerol and a protease inhibitor (Complete EDTA-free; manufactured by Boehringer Mannheim Co., Ltd.) on ice by ultrasonication. All the procedures after the collection of the bacterial cells were carried out at 4°C. The disrupted bacterial cells were centrifuged at 27,700 x g for 20 minutes. The resulting supernatant and 4 ml of Ni-NTA agarose bead (Qiagen) were gently mixed together by batch process for one hour. The Ni-NTA agarose bead binding the HsRad52₁₋₂₁₂ protein was charged in Econo-column (Bio-Rad) and rinsed using a buffer solution B (pH 7.8) containing 50 mM Tris-HCl, 0.8 M KCl, 2 mM 2-mercaptoethanol, 50 mM imidazole, and 10% glycerol at a volume 30-fold the volume of the column at a flow rate of about 0.3 ml/minute. The HsRad52₁₋₂₁₂ protein was eluted on a linear concentration gradient of the imidazole solution from 50 mM to 300 mM at a volume 30-fold the volume of the column. The peak fraction eluted with about 0.15 M imidazole solution was recovered, followed by addition of 2 units of thrombin protease (Amersham Pharmacia Biotech) per 1 mg HsRad52₁₋₂₁₂ protein, to remove the hexahistidine-tag sequence. The reaction mixture was immediately dialyzed against a buffer solution C (pH 7.5) containing 50 mM Tris-HCl, 0.2 M KCl, 0.5 mM EDTA, 2 mM 2-mercaptoethanol and 10% glycerol for 12 hours or longer. After the hexahistidine- tag sequence was removed, the HsRad52₁₋₂₁₂ protein was applied to a 4-ml heparin-Sepharose column (Amersham Pharmacia Biotech). The column was rinsed with the buffer solution C of a volume 20-fold that of the column, to elute the protein on a linear KCl concentration gradient of 0.2 M to 1 M and of a volume 20-fold that of the column. The HsRad52₁₋₂₁₂ protein was eluted with a sharp peak at about 0.3 M KCl concentration. The peak fraction of the HsRad52₁₋₂₁₂ protein at 1 to 2 mg/ml concentration was dialyzed against a buffer solution D (pH 7.0) containing 20 mM Hepes-KOH, 0.2 M KCl, 0.5 mM EDTA, 2 mM 2-mercaptoethanol and 50% glycerol and stored at -20°C. Using the protein assay kit of Bio-Rad, the protein was assayed using BSA (Pierce) as a standard substance. The HsRad52₁₋₂₁₂ protein thus purified was used for assaying various enzyme activities such as assaying DNA binding activity and homologous pairing activity.

### (Example 4)

### Purification of protein for crystallization

For crystallization, the HsRad52₁₋₂₁₂ protein was expressed in E.coli at a large scale by the same method as described above and was then purified by the same method as described above, except for the change of only the final buffer solution for use in dialysis to another one. Specifically, the HsRad52₁₋₂₁₂ protein was expressed in *E. coli* JM109 (DE3) using an expression vector pET-15b (Novagen). Then, the HsRad52₁₋₂₁₂ protein with thehistidine tag sequence was purified onNi-NTAagarose (QIAGEN) , fromwhich thehistidine tag sequence was removed by thrombin protease. Subsequently, the protein was purified by heparin-Sepharose column chromatography (Amersham Pharmacia Biotech). The peak fraction eluted from the heparin-Sepharose column was dialyzed against a buffer solution (pH 7 . 0) containing 10 mM Bis-Tris propane, 0.2 M KCl, 0.1 mM EDTA and 2 mM 2 -mercaptoethanol and was then concentrated to 3 to 4 mg/ml, using Centricon-30 (Millipore) . To facillitate the growth of the HsRad52₁₋₂₁₂ crystal, the HsRad52₁₋₂₁₂ protein was mixed with an 8-mer single-stranded DNA (GTTGGTTG, 1.5 to 2 mM) at a molar ratio of 14 :1 to 11: 1 on ice, and was incubated overnight at 4°C. The following day, the HsRad52₁₋₂₁₂ crystal was grown by the hanging drop method at 20°C. The first droplet was prepared by mixing 1 µl of a solution, pH 6.2 containing 12% polyethylene glycol (PEG) 5000 monomethyl ether (MME), 80 mM ammonium sulfate, 20 mM betaine monohydrate or urea, and 40 mM sodium cacodylate, with 2 µl of a mixture of HsRad52₁₋₂₁₂ and the single-stranded DNA. The reservoir solution contained 15% PEG 5000 MME, 0.1 M ammonium sulfate and 50 mM sodium cacodylate (pH6.2). Crystals typically appeared 12 hours or one day and reached the maximum size (0.1 mm × 0.2 mm × 0.3 mm)within 3 days.

### (Example 5)

### Data collection

The crystal of the HsRad52₁₋₂₁₂ protein contains the undecameric HsRad52₁₋₂₁₂ ring as one of asymmetric units. A diffraction data of a 2. 8-angstrom (Å) resolution or more could be obtained. Prior to data collection, the crystal was treated with the reservoir solution diluted to 3 fold with water. Subsequently, the crystal solution was dialyzed against a solution containing 15% ethylene glycol, 5% PEG5000 MME, 33 mM ammonium sulfate and 17 mM sodium cacodylate (pH 6.2)and further dialyzed against a solution of the same composition except for 30% ethylene glycol solution, for cryo-protection. The HsRad52₁₋₂₁₂ crystals were flash frozen in a stream of nitrogen gas.

Data sets of both the native and selenomethionine-substituted crystals were collected at the RIKEN beam line I (BL45XU) at the 8 GeV Super Photon ring (SPring-8) in Harima Institute Japan. The diffraction data were reduced by a data processing program DENZO-SCALEPACK.

### (Example 6)

### Determination and refining of three-dimensional structure

For the three-dimensional structural determination, three data sets from three different crystals (one was native type, and the other two were heavy atom-substituted materials with selenomethionine) were used. The HsRad52₁₋₂₁₂ monomer protein contained four methionine residues. One of the four selenium atoms (methionine residue at position 78) was found by the direct method program, SnB and was continuously used for phase determination. The position of selenium atom was further refined by the SHARP program, so that the initial phase was calculated at 3.5-angstrom resolution. The initial maps were solvent flattened by the program DM. The phases calculated from the two selenomethionine data were combined and enlarged to 2.8 angstroms using the data of the native type. According to the iterative rounds of model building with the Program O and the refining protocol with Program XPLOR, the three-dimensional structure was refined until the complete main chain tracing was possible. All the refining up to this stage contained a 300 weight of NCS restriction. By the final refining including minimization and refining of Group B factor, the NCS weight was reduced step by step from 300 to 50. The final model containing 15841 residues of the HsRad52₁₋₂₁₂ undecamer and 82 water molecules had an R value of 23.5% (28. 5% as free R value) . On the Ramachandran plot of the final structure, 88.6% of the residues existed in the optimal region, while 11.4% of the residues existed in an additional acceptable region. The graphic display of the three-dimensional structure was carried out by using programs MOLSCRIPT and SPOCK.

### (Example 7)

### Introduction of site specific mutagenesis into HsRad52

Based on the three-dimensional structure of HsRad52₁₋₂₁₂ shown in Table 1 and Figs. 1 and 2, basic amino acid residues with possibility of DNA binding in the periphery of the undecameric ring structure were selected, to prepare point mutants of HsRad52₁₋₂₁₂, where these were individually substituted with alanine residue. The selected amino acid residues were arginine at position 55, tyrosine at position 65, histidine at position 69, arginine at position 70, arginine at position 153 and lysine at position 169. Mutants where respective amino acid residues were substituted with alanine are referred to as R55A, Y65A, H69A, R70A, R153A and K169A. The introduction of site specific mutagenesis was done using QuikChange kit (manufactured by Stratagene) according to the instruction manual about the method for use.

The single-stranded and double-stranded DNAs binding activities of the six types of point mutants thus prepared from HsRad52₁₋₂₁₂ were detected by gel shift assay method. The substrate DNA used by the method was prepared as described below. The pGsat4 of double-stranded DNA was prepared by inserting a 198-bp DNA fragment from human α-satellite into a plasmid pGEM-T East vector (manufactured by Promega). The 3.2-kb plasmid DNA was prepared by a gentle lysis method using sarcosyl, without alkali modification of the host *E. coli,* so as to avoid the irreversible denaturation. As the single-stranded DNA, a 50-base oligonucleotide purified by HPLC as shown in SEQ ID NO:2 was purchased from Roche Biochemical. 
SEQ ID NO:2: SAT-1 (50mer

5'-ATTTCATGCTAGACAGAAGAATTCTCAGTAACTTCTTTGTGCTGTGTGTA-3')
 The 5' terminus of the oligonucleotide was labeled with radioactivity in the presence of [γ³²P] ATP using T4 polynucleotide kinase (New England Biolabs) and then purified on a column Chromaspin-10 (manufactured by Clontech).

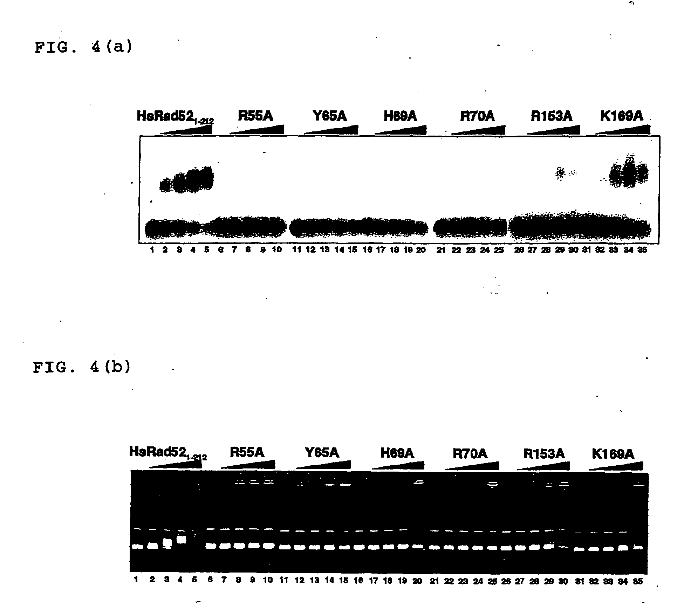

For gel shift assay, a reaction mixtures containing final concentratins of 50 mM Hepes-KOH (pH 7.5), 40 mM KCl, 1 mM MgCl₂, 0.1 mg/ml BSA, 1 mM DTT, 2% glycerol and 0.25 to 2 µM mutants of HsRad52₁₋₂₁₂ was used. For single-stranded DNA binding assay, 1 µl of 1 µM ³²P-labeled SAT-1 single-stranded DNA (nucleotide) was mixed with 9 µl of the reaction mixture, which was incubated at 37°C for 5 minutes. Subsequently, 1 µl of 12% glyceraldehyde solution was added, to immobilize HsRad52 and the single-stranded DNA. After incubating for 20 minutes, a 6-fold volume of staining solution for electrophoresis was added. The reaction mixture was resolved by 1% agarose gel electrophoresis in 0.5 × TBE buffer at 3.3 V/cm and ambient temperature. After electrophoresis, the gel was dried for detection by autoradiography. The amount of the free single-stranded DNA was assayed by an image analyzer BAS2500 manufactured by Fuji Film. The results are shown in Fig. 4A. Single-stranded DNAs that do not bind to HsRad52 remained on the tip of the gel, but as the increase of the protein concentration, the protein bound to the single-stranded DNA shifted toward the side of a higher molecular weight. Compared with the wild type HsRad52₁₋₂₁₂, the single-stranded DNA binding activity of all of the point mutants was lowered. Particularly, the mutants R55A, Y65A, H69A, and R70A lost most of the binding activity to the single-stranded DNA. This indicates that a region close to arginine residue at position 55 to tyrosine residue at position 70 is a DNA binding region.

Next, for the double-stranded DNA binding assay, 1 µl of 15 µM pGsat4 double-stranded DNA was added to 9 µl of the reaction solution, and reacted for 5 minutes, and then electrophoresed on 1% agarose gel in the same manner as described above, except for no immobilization of HsRad52 and the double-stranded DNA. After electrophoresis, the gel was stained with 0.5 µg/ml ethidium bromide for detection. The amount of the free double-stranded DNA was assayed by MacBAS software. The results are shown in Fig. 4B. The double-stranded DNAs that do not bind to HsRad52 remained on the tip of the gel, while as the increase of the protein concentration, the protein eventually bound to the double-stranded DNA and shifted toward the side of a higher molecular weight. Compared with the wild type HsRad52₁₋₂₁₂, the double-stranded DNA binding activity of all of the point mutants was lost. This indicates that two proximate regions, one is a region close to arginine residue at position 55 to tyrosine residue at position 70 and another is a region close to arginine at position 153 and lysine at position 169 are DNA binding regions.

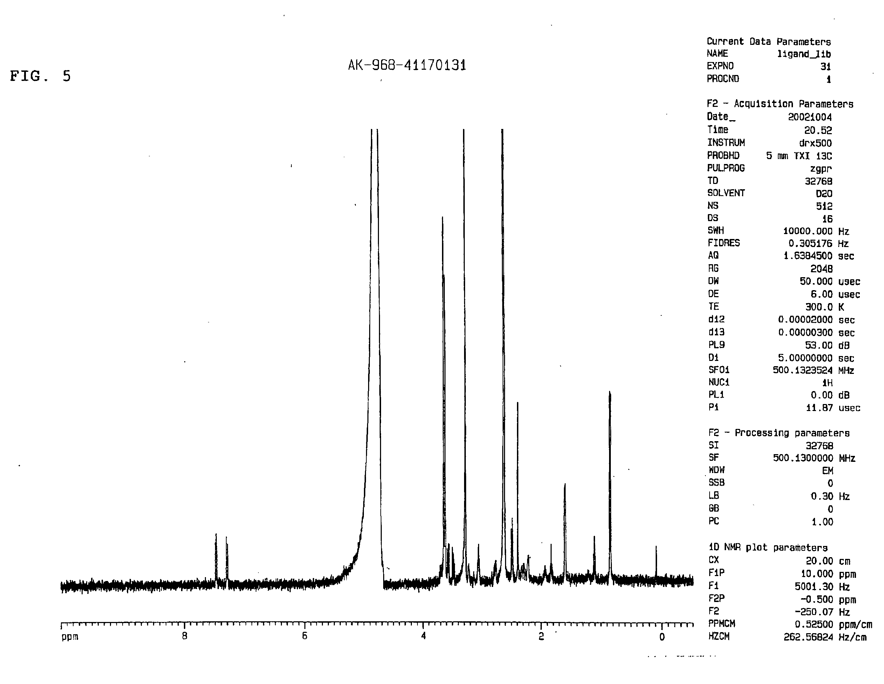

The inventors made an additional analysis of the DNA binding site of HsRad52 in detail using the same method. The results are described by Kagawa W., *et al.,* Molecular Cell, Vol.10, 359-371, August, 2002,which is incorporated by reference to the present specification. In Fig. 5 in the publication, (A) depicts binding to single-stranded DNA; (B) depicts binding to double-stranded DNA; (C) through (F) depict them graphically; (G) depicts the detection of the ternary complex formation of HsRad52, single-stranded DNA and double-stranded DNA; and (H) depicts the D-loop formation with single-stranded DNA and with double-stranded DNA. As apparently shown with reference to these figures, arginine at position 55, tyrosine at position 65, lysine at position 152, arginine at position 153 and arginine at position 156 are all essential for DNA binding. Among them, arginine at position 55 and lysine at position 152 are needed for binding to single-stranded DNA, while tyrosine at position 65, arginine at position 153, and arginine at position 156 are needed for binding to both single-stranded DNA and double-stranded DNA.

### (Example 8)

*In silico* screening for a compound binding to the DNA binding region of HsRad52

### <Data base optimization>

As a comparative data base for low molecular (weight) compound, the low molecular compound catalog data base supplied from SPECS were used. One entry including plural molecules is divided per each molecule, to prepare a library from which overlapping was preliminarily removed, as a population (152323 molecules) for the screening. Based on "Lipinski's Rule of 5", then, target-independent optimization of the low molecular compound data base was carried out. The screening conditions herein used are as follows.
1. Molecular weight of 100 or more to 500 or less
2. Calculated logP value (o/w) of 5 or less (using XLOGP-1 algorithm)
3. Number of hydrogen bond acceptor atoms (number of N atoms and O atoms contained in low molecular compound) of 10 or less
4. Number of hydrogen bond donor atoms (number of NH and OH contained low molecular compound) of 5 or less.

So as to appropriately carry out docking calculation, further, the following molecules were excluded.
1. Molecule with a number of single bonds possibly rotational of 21 or more.
2. Molecule containing radical
3. Molecule containing elements other than H, C, N, O, F, S, P, Cl, Br and I.
The number of molecules after screening was 103773.

### <Speculation of binding site>

A groove comprising a group of residues presumably essentially involved in the binding to both single-stranded DNA and double-stranded DNA in HsRad52₁₋₂₁₂ (arginine at position 55, tyrosine at position 65, lysine at position 152, arginine at position 153 and arginine at position 156 in the amino acid sequence of SEQ ID NO:1) was defined as binding site.

### <Screening>

### (1) Primary screening

Using Dock 4.0, the binding site was docked to the preliminarily optimized low molecular compound library in its entirety. Based on the energy score of Dock, then, lowmolecular compounds were ranked.

### (2) Secondary screening

The 10, 000 molecules highly ranked from the top as obtained as the consequence of the primary screening and similar 178 compounds thereof contained in the data base (Sybyl Finger Print tanimoto similarity of 0.8 or more) were docked in details using AutoDock 3.0.5. Subsequently, compounds within 4 angstroms from the sphere used during the docking were screened, from which molecules with abnormally terminated calculation were excluded. Consequently, the docking structures of 9989 molecules were finally obtained. Among the resulting compounds, the 1000 compounds with highly ranked binding levels to the binding site from the top were defined as ligand candidate compounds.
ΔG (binding free energy change) of the ligand candidate compounds was -9.43 kcal/mol or more.

### (Example 9)

### Screening for HsRad52-binding compounds by NMR

Among the ligand candidate compounds obtained in Example 8, one candidate compound was in view of the binding free energy change (ΔG) obtained by AutoDock and solubility. The compound was dissolved in DMSO-d6 to be adjusted to 455.7 µM. The HsRad52₁₋₂₁₂ protein prepared in Example 3 was dissolved to 152 µM using buffer solution A (dissolved in heavy water to 10 mM Tris-dll, (pH 7.5) /200 mM KCl) . Using them, two types of samples namely the ligand candidate compound alone and the ligand candidate compound to which the HsRad52 protein was added at 1:1 were prepared.

**Table 2**

| Composition of the ligand candidate compound alone in solution | |
|---|---|
| DMSO-d6 solution of candidate compound (455.7 µM) | 20 µl |
| Buffer solution A | 400 µl |
| Total volume | 420 µl |

The final concentration of the ligand candidate compound is 21.7 µM.

**Table 3**

| Composition of mixture solution of the ligand candidate compound and the HsRad52 protein | |
|---|---|
| DMSO-d6 solution of candidate compound (455.7 µM) | 10 µl |
| HsRad52 protein solution (152 µM) | 30 µl |
| Buffer solution A | 170 µl |
| Total volume | 210 µl |

The final concentrations of the ligand candidate compound and the HsRad52 protein are 21.7 µM, respectively.

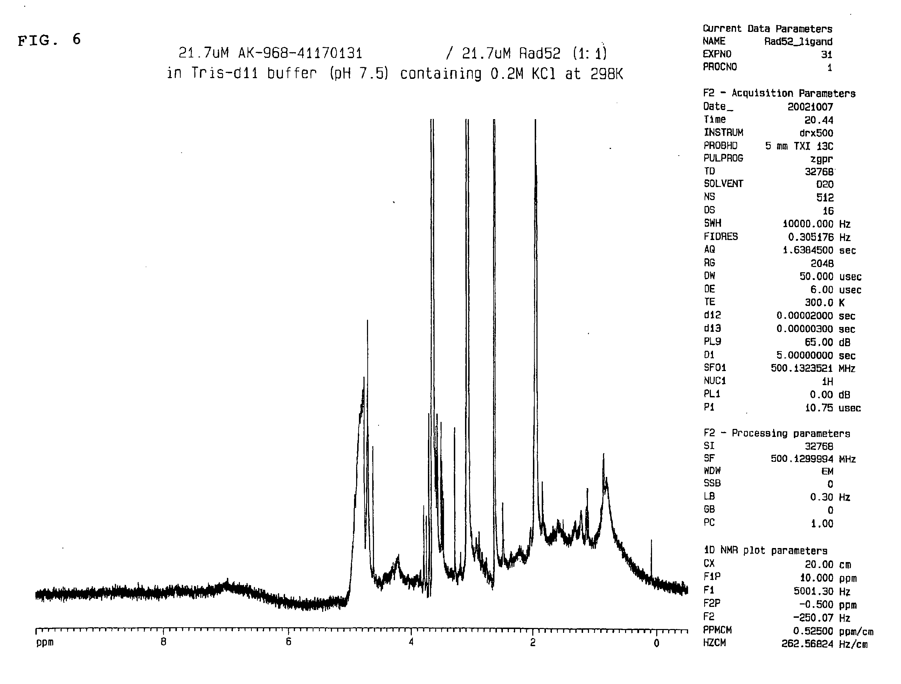

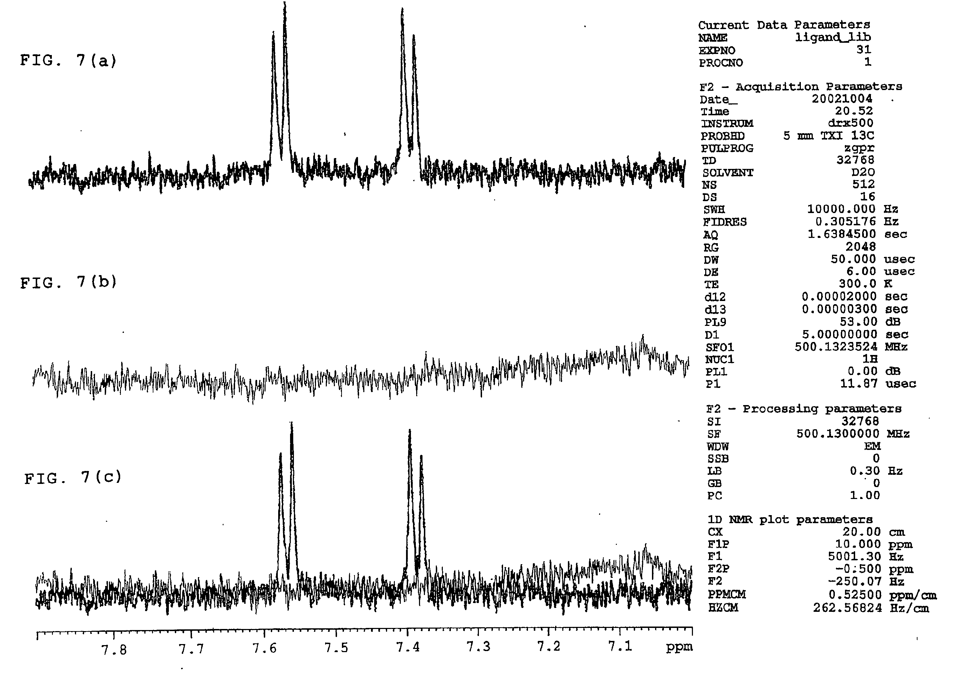

One-dimensional ¹H-NMR of the individual samples was measured, to observe whether or not any change existed between prior to and after the addition of HsRad52 protein. Consequently, the NMR spectrum of a sample of ID AK968-41170131 with a chemical name of cis-6-[4-(4-isobutyl-phenyl)-5-methyl-thiazol-2-ylcarbamoyl]-3,4-dimethyl-cyclohex-3-enec arboxylic acid (Compound A, -11.71 kcal/mol) as such ligand candidate compound is shown in Figs. 5 through 7. Fig. 5 shows the NMR spectrum of Compound A alone; and Fig. 6 shows the NMR spectrum of Compound A mixed with the HsRad52 protein. In Fig. 6, a slight increase of the base line due to the mixing with the protein can be observed, but apparently, Compound A-derived two signals around 7.5 ppm disappeared. The chart enlarging the region is shown in Fig. 7. Fig. 7A shows the case of the Compound A alone; Fig. 7B shows the case of the Compound A mixed with the HsRad52 protein; and Fig. 7C shows the comparison between the two A and B . These results indicate that the signals derived from the four hydrogens in the benzene ring of the Compound A disappeared due to the interaction with HsRad52, and that the Compound A bound to HsRad52.

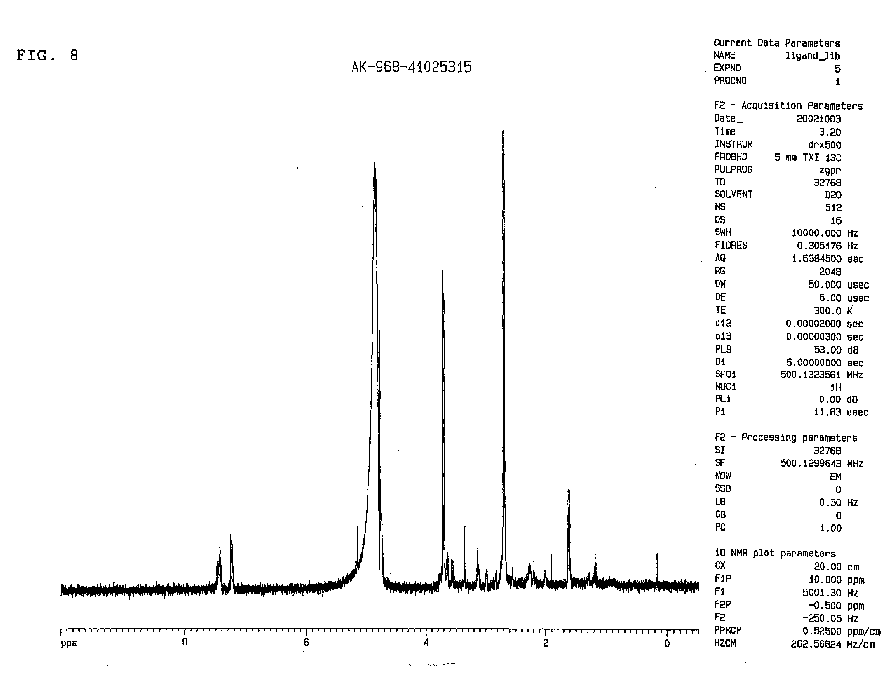

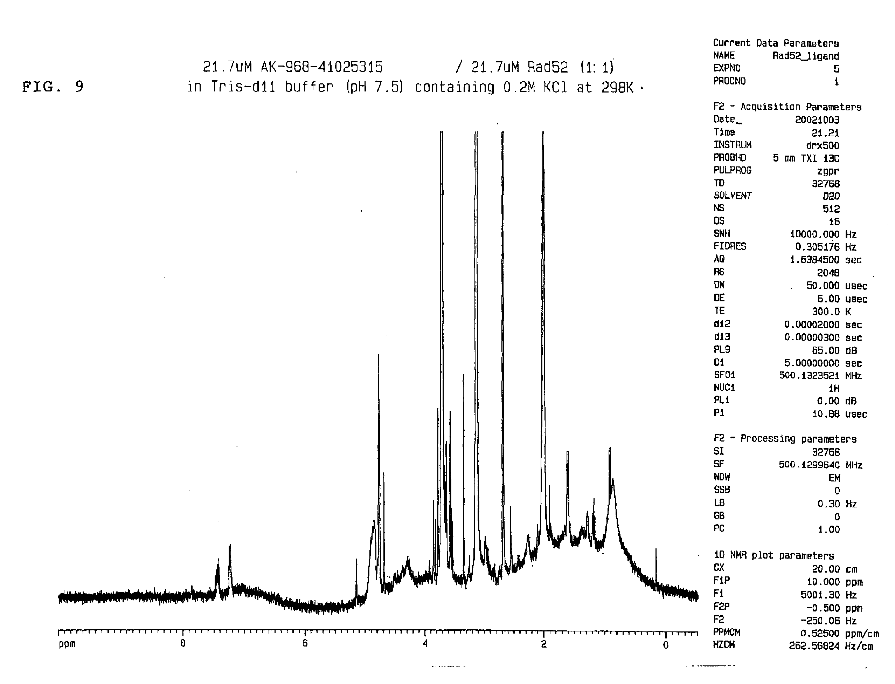

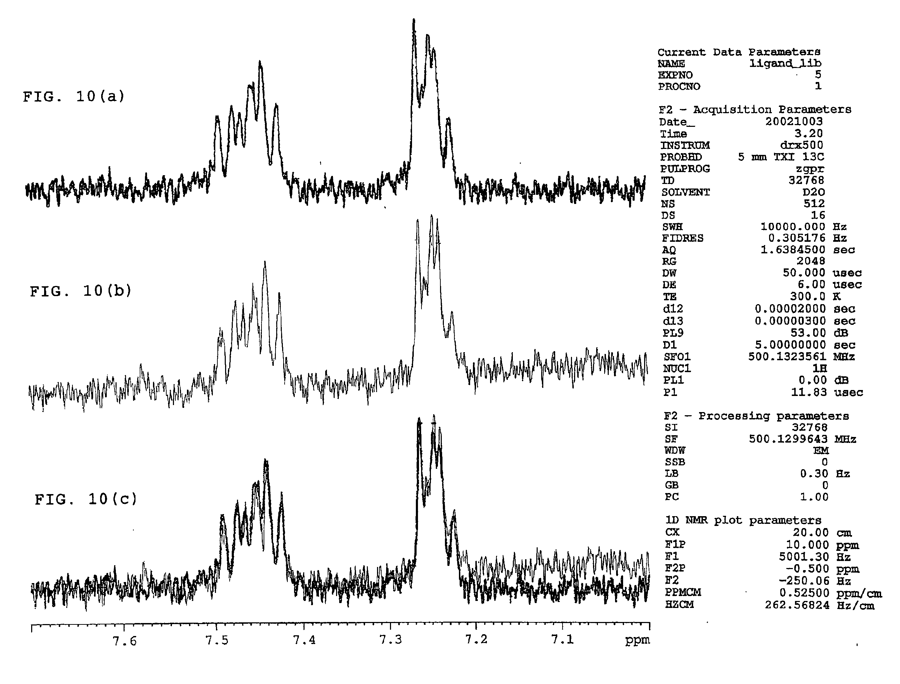

Alternatively, Figs. 8 through 10 show the results of experiments in the same manner using as the ligand candidate compound a sample of ID AK968-41025315 with a chemical name of 3,4-dimethyl-6-[N'-(9H-xanthene-9-carbonyl)-hydrazinocarbonyl]-cyclohex-3-enecarboxylic acid (Compound a, -13.33 kcal/mol). Fig. 8 shows the NMR spectrum of Compound a alone; and Fig. 9 shows the NMR spectrum of a mixture solution of the Compound a and the HsRad52 protein. The chart enlarging the signal around 7.5 ppm is shown in Fig. 10. These figures apparently indicate that the NMR signal of the compound a is never changed even after mixing with HsRad52, possibly indicating that the compound a may not bind to the HsRad52 protein.

Herein, all the NMR spectra were taken in deuterium, using Bruker DRX500 NMR spectrophotometer at 298 K.

Using such method, the plural ligand candidate compounds obtained in Example 8 were adjusted to various concentrations to measure the NMR spectra. Compounds detected with the interaction with HsRad52 are shown below in Table 4. Herein, the strength of a binding force relatively calculated from the signal intensity of the NMR spectrum is shown as relative binding force, provided that the binding force of the Compound A is defined as 1.

**Table 4**

| Ligand binding compounds with suggested binding to HsRad52 by NMR | | | | |
|---|---|---|---|---|
| Compound | Sample ID | AutoDock Energy Score | Compound Name | Relative Binding force |
| A | AK-968-41170131 | -11.71 (kcal/mol) | 6-[4-(4-Isobuty 1-phenyl)-5-methyl-thiazol-2-y Icarbamoyl]-3,4 - dimethyl-cyclo hex-3-enecarbox ylic acid | 1 |
| B | AK-968-41170335 | -11.5 | 6-[4-(4-Isoprop yl-phenyl)-5-me thyl-thiazol-2-ylcarbamoyl]-3, 4-dimethyl-cycl ohex-3-enecarbo xylic acid | 0.5 |
| C | AK-968-41172047 | -11.41 | 3-(4-(4-Butyl-p henyl)-5-methyl - thiazol-2-ylca rbamoyl]-7-oxa-bicyclo[2.2.1]h | 0.5 |
| | | | eptane-2-carbox ylic acid | |
| D | AK-968-41171476 | -11.38 | 6-[4-(2,4-Dimet hyl-phenyl)-5-m ethyl-thiazol-2 - ylcarbamoyl)-3 ,4-dimethyl-cyc lohex-3-enecarb oxylic acid | 0.2 |
| E | AK-968-41171884 | -11.34 | 3,4-Dimethyl-6-[5-methyl-4-(4-propyl-phenyl)-thiazol-2-ylcar bamoyl]-cyclohe x-3-enecarboxyl ic acid | 0.5 |

The results shown in Table 4 suggest that the compounds interactive with HsRad52 may have the structure represented by the following general formula (I).

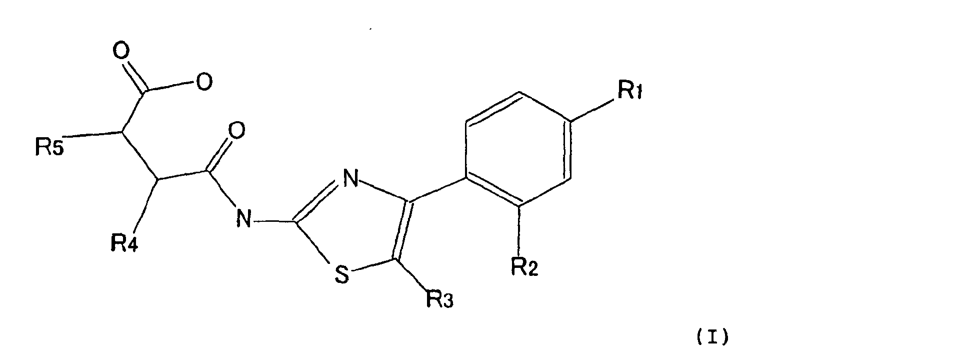

Herein, R₁ represents a straight chain (linear) or branched-chain alkyl group with one to 10 carbon atoms; R₂ and R₃ are independent and represent hydrogen atom or a lower alkyl group such as methyl group; R₄ and R₅ represent an alkyl group or alkenyl group which may or may not have a substituent, where these groups together forma five-membered or six-membered ring.

More specifically, the compounds are cis-6-[4-(4-alkylphenyl)-5-methylthiazol-2-ylcarbamoyl]-3, 4-dimethylcyclohex-3-enecarboxylic acid (herein, the alkyl group is a straight-chain or branched-chain alkyl group with one to 10 carbon atoms) and 3-[4-(4-alkylphenyl)-5-methylthiazol-2-ylcarbamoyl]-7-oxabicyclo[2.2.1]heptane-2-carboxylic acid (herein, the alkyl group means a straight-chain or branched-chain alkyl group with one to 10 carbon atoms). These compounds may be in the form of salts (sodium salt, potassium salt, ammonium salt, etc.). Additionally, these compounds may be in the form of optically active or racemic compound.

### (Example 10)

### Measurement of binding activity of ligand candidate compound to HsRad52 by gel shift assay method

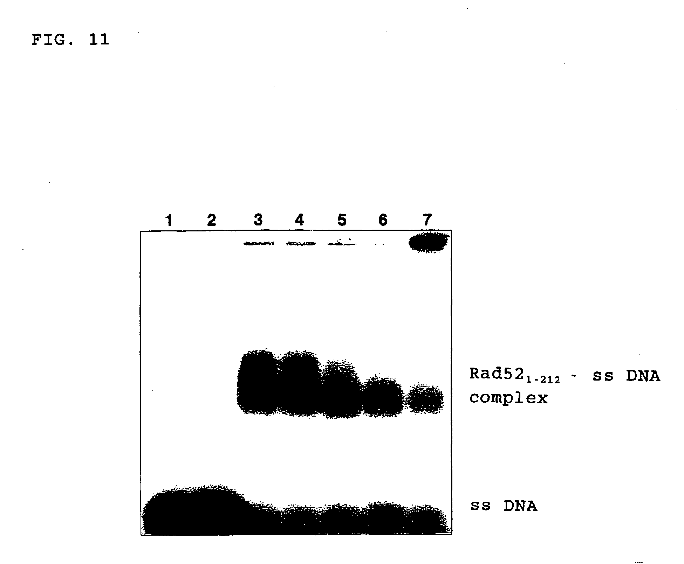

To confirm whether or not the ligand candidate compound AK968-41170131 (Compound A) of which the binding to HsRad52 was suggested in Example 9 actually bound to HsRad52, the activity of Compound A to inhibit the binding between HsRad52 and SAT-1 single-stranded DNA (SEQ ID NO:2; 50-mer) was measured as an indicator by the same method as in Example 7. To a solution containing 50 mM HEPES-KOH (pH 7.5), 40 mM KCl, 1 mM MgCl₂, 0.1 mg/ml BSA, 1 mM DTT, 2% glycerol and 1 µM HsRad52₁₋₂₁₂, where the concentrations were all expressed as final concentrations, were mixed ³²P-labeled SAT-1 single-stranded DNA to a final each base concentration of 1 µM and Compound A at various concentrations at 37°C for 6 minutes. Subsequently, the mixture was fixed with 0.2% glutaraldehyde for 20 minutes and electrophoresed on 1% agarose gel. The gel was dried, for visualization by autoradiography. The results are shown in Fig. 11. In Fig. 11, Lane 1 shows the single-stranded DNA alone; Lane 2 shows the mixture of the single-stranded DNA with Compound A; Lane 3 shows the mixture of the single-stranded DNA with HsRad52₁₋₂₁₂ as samples. Lanes 4 through 7 show the mixture of the single-stranded DNA, HsRad52₁₋₂₁₂ and the respective amounts of Compound A in a final concentration of 0.1, 1, 10 and 100 µM. As apparently shown in Fig. 11, the addition of HsRad52₁₋₂₁₂ shifts the complex with the single-stranded DNA toward the side of higher molecules. While the concentration of the Compound A increases (Lanes 4 through 7), the amount of the complex decreases, i.e. the formation of the complex of the single-stranded DNA with HsRad52₁₋₂₁₂ was inhibited. As speculated from the *in silico* screening (Example 8) and NMR screening (Example 9), thus, it was confirmed that the ligand candidate compound AK968-41170131 (Compound A) bound to the DNA binding site of HsRad52.

### INDUSTRIAL APPLICABILITY

In accordance with the invention, the three-dimensional structure model of the DNA recombination/repair protein complex or a mutant thereof is provided. The three-dimensional structure of the enzyme complex is very important for the understanding of the DNA repair mechanism via homologous recombination. Understanding the reaction mechanism of the enzyme enables the application of the enzyme to the prophylaxis or therapeutic treatment of cancer via DNA repair reaction. As a method for the prophylaxis or therapeutic treatment of cancer, a mutant enzyme wi th improved DNA recombinational repair activity can be designed, along with the screening of compounds capable of binding to the enzyme. The compound obtained by these methods can be used for the diagnosis of diseases caused by DNA damages by measuring the Rad52-specific DNA recombinational repairing activity. Additionally, wide applications such as drug discovery for a target Rad52 and gene transfer into a specific site via homologous recombination in vivo can be expected.

## Claims

1. A polypeptide fragment comprising the amino acid sequence from position 1 to position 192 in the amino acid sequence defined in SEQ ID NO: 1, wherein the amino acid residues at positions 55, 65, 69, 70, 153 and 169 form a DNA binding site.

2. A polypeptide fragment derived from the polypeptide fragment of claim 1, as prepared by deletion, substitution or addition of one or several amino acids, the polypeptide fragment having a DNA binding activity.

3. A DNA recombination/repair protein complex comprising an assembly of the following protein (a) or (b), wherein said complex forms an undecameric ring structure:
(a) a protein consisting of the amino acid sequence of SEQ ID NO:1:
(b) a protein derived from the amino acid sequence of SEQ ID NO:1 as prepared by deletion, addition, insertion or substitution of one or several amino acids, the protein having a DNA recombinational repairing activity.

4. A mutant DNA recombination/repair protein complex comprising an assembly of the protein from posi tion 1 to position 212 in the amino acid sequence of SEQ ID NO:1, wherein the complex forms an undecameric ring structure.

5. The mutant DNA recombination/repair protein complex of claim 4, wherein the complex has a three-dimensional structure substantially defined by the atomic coordinates shown in Table 1.

6. A crystal of an undecameric mutant DNA recombination/repair protein complex comprising an assembly of the protein comprising the amino acid sequence from position 1 to position 212 in the amino acid sequence of SEQ ID NO:1, wherein the crystal has a space group of P4₃2₁2.

7. A crystal of an undecameric mutant DNA recombination/repair protein complex comprising an assembly of the protein comprising the amino acid sequence from position 1 to position 212 in the amino acid sequence of SEQ ID NO:1, wherein the crystal has unit cell dimensions of a=b=145.6 Å, and c=247.5 Å.

8. The crystal of a mutant DNA recombination/repair protein complex of claim 6 or 7, wherein methionine at position 78 in the amino acid sequence of SEQ ID NO:1 is substituted with selenomethionine.

9. A method for preparing a crystal of a DNA recombination/repair protein complex or a mutant thereof, comprising the steps of mixing a DNA recombination/repair protein protein or a mutant thereof at a concentration of 3 to 4 mg/ml and single-stranded DNA at a molar ratio of 14:1 to 11:1, and crystallizing the resulting mixture solution by vapor diffusion method using a buffer solution, pH 6.0 to 8.0 containing 10 to 20% polyethylene glycol (PEG) or polyethylene glycol monomethvl ester (PEGMME) and 0.05 to 0.2 M ammonium sulfate.

10. A method for determining the three-dimensional structure of a DNA recombination/repair protein complex or a mutant thereof, comprising the steps of:
(a) generating a crystal of a protein with DNA recombinational repairing activity,
(b) obtaining the X-ray diffraction data using said crystal by heavy atom isomorphous replacement or multiwavelength anomalous diffraction, and
(c) determining a three-dimensional structural coordinate based on said X-ray diffraction data.

11. A computer system comprising a memory with atomic coordinates defining a DNA recombination/repair protein complex being arranged in the inside thereof and a processor in electric communication with the memory, the processor comprising a process of generating a model with a three-dimensional structure representing at least a part of the DNA recombination/repair protein complex or calculating the conformational energy of the complex.

12. A method for identifying a mutant DNA recombination/repair protein with modulated DNA recombinational repairing activity, comprising the steps of:
(a) constructing the three-dimensional structure representing a DNA recombination/repair protein complex, using a computer model,
(b) identifying a site influencing the DNA recombinational repairing activity on the basis of the three-dimensional structure model,
(c) producing a mutant enzyme by introducing a mutation in said identified site, and
(d) measuring the activity of said mutant enzyme.

13. The method of claim 12, wherein said three-dimensional structure model is substantially defined by the atomic coordinates shown in Table 1.

14. The method of claim 12 or 13, wherein said DNA recombinational repairing activity is DNA binding activity, homologous-pairing activity or multimer-forming activity.

15. A method for identifying a mutant DNA recombination/repair protein with modulated DNA recombinational repairing activity, comprising the steps of:
(a) identifying a DNA binding region by calculating the surface charge of all or a part of the DNA recombination/repair protein complex substantially defined by the atomic coordinates shown in Table 1,
(b) selecting basic and hydrophobic amino acid residues existing in said identified DNA binding region,
(c) preparing a mutant enzyme by carrying out the substitution, deletion, addition or chemical modification of said selected amino acid residues, and
(d) assaying the DNA binding activity and/or homologous pairing activity of said mutant enzyme.

16. The method for identifying a mutant DNA recombination/repair protein of claim 15, wherein the amino acid residues selected at the step (b) are one or two or more amino acid residues selected from arginine at position 55, tyrosine at position 65, histidine at position 69, arginine at position 70, arginine at position 153 and lysine at position 169 in the amino acid sequence of SEQ ID NO:1.

17. A method for producing a mutant DNA recombination/repair protein, comprising the steps of identifying a mutant DNA recombination/repair protein by a method of any one of claims 12 to 16, and expressing said mutant DNA recombination/repair protein using the recombinant DNA encoding said mutant DNA recombination/repair protein.

18. A mutant DNA recombination/repair protein with modulated DNA recombinational repairing activity, as obtained by a method of any one of claims 12 to 16.

19. Amethod for screening a compound capable of modulating the repairing activity of a DNA recombination/repair protein, using a polypeptide fragment or a complex of any one of claims 1 to 5 to design or select a compound binding thereto.

20. Amethod for screening a compound capable of modulating the repairing activity of a DNA recombination/repair protein, using a computer with the memory of the coordinate data of the three-dimensional structure of a polypeptide fragment or a complex of any one of claims 1 through 5 to design or select a compound binding thereto.

21. The method of claim 20, further comprising contacting said designed or selected compound with a DNA recombination/repair protein, and determining whether said compound binds to the DNA recombination/repair protein.

22. A compound modulating the activity of a DNA recombination/repair protein as obtained by a method of any one of claims 19 through 21.

23. The compound according to claim 22, wherein said compound modulating the DNA recombinational repairing activity is a natural compound or a synthetic compound.

24. An inhibitor of DNA recombination/repair protein, consisting essentially of cis-6-[4-(4-alkyl-phenyl)-5-methyl-thiazol-2-ylcarbamoyl]-3,4-dimethyl-3-cyclohexenecarboxylic acid or salt thereof, wherein said alkyl group denotes an alkyl group which has a linear or branched chain of carbon numbers 1 to 10.

25. An inhibitor of DNA recombination/repair protein, consisting essentially of 3-[4-(4-alkyl-phenyl)-5-methyl-thiazol-2- ylcarbamoyl]-7-oxa- bicyclo [2.2.1] heptane-2-carboxylic acid or salt thereof, wherein said alkyl group denotes an alkyl group which has a linear or branched chain of carbon numbers 1 to 10.

26. A method for preparing a pharmaceutical composition containing a compound modulating DNA recombinational repairing activity, comprising a step of mixing a compound obtained by a method of any one of claims 19 through 21 with a pharmaceutically acceptable carrier.
